(19) Europäisches Patentamt
European Patent Office
Office européen des brevets

(11) EP 4 289 845 A1

(12) EUROPEAN PATENT APPLICATION
published in accordance with Art. 153(4) EPC

(43) Date of publication:
**13.12.2023 Bulletin 2023/50**

(21) Application number: **22749028.1**

(22) Date of filing: **27.01.2022**

(51) International Patent Classification (IPC):
***C07D 471/14*** (2006.01) ***C07D 498/14*** (2006.01)
***C07D 513/14*** (2006.01) ***A61K 31/501*** (2006.01)
***A61K 31/4985*** (2006.01) ***A61P 35/00*** (2006.01)

(52) Cooperative Patent Classification (CPC):
**A61K 31/4985; A61K 31/501; A61P 35/00; C07D 471/14; C07D 498/14; C07D 513/14**

(86) International application number:
**PCT/CN2022/074278**

(87) International publication number:
**WO 2022/166749 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.02.2021 CN 202110151579**
**07.07.2021 CN 202110768196**

(71) Applicant: **Shanghai Apeiron Therapeutics Company Limited**
**Shanghai 201210 (CN)**

(72) Inventors:
- **GU, Xiaohui**
  **Shanghai 201210 (CN)**
- **ZHANG, Haoyi**
  **Shanghai 201210 (CN)**

(74) Representative: **Henderson, Helen Lee**
**Withers & Rogers LLP**
**2 London Bridge**
**London SE1 9RA (GB)**

# (54) TRIHETEROCYCLIC COMPOUND, PREPARATION METHOD THEREFOR, AND APPLICATION THEREOF

(57) Disclosed are a triheterocyclic compound, a preparation method therefor, and an application thereof. Specifically, provided is a triheterocyclic compound as represented by formula IA or a pharmaceutically acceptable salt thereof. The triheterocyclic compound has good poly (ADP-ribose) polymerase inhibiting activity, can significantly inhibit the growth of NCI-H1373 human lung adenocarcinoma cells, and can be used for the preparation of drugs for treating and/or preventing abnormal proliferative diseases or other diseases related to targets binding to this series of compounds, such as cancer.

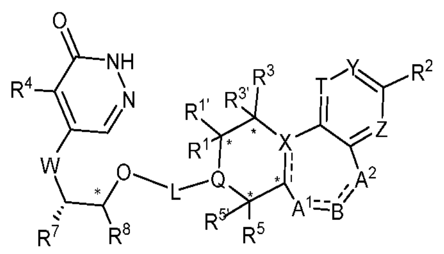

IA

EP 4 289 845 A1

## Description

The present application claims priority of the Chinese Patent Application No. 2021101515791 filed on February 3, 2021, and priority of the Chinese Patent Application No. 2021107681969 filed on July 7, 2021, which are incorporated herein by reference in their entireties.

## TECHNICAL FIELD

The present invention relates to a fused triheterocyclic compound, preparation method therefor and application thereof.

## BACKGROUND

Poly-ADPs (PARPs) are a family of enzymes having 17 members (S. Vyas, M. Chesarone- Cataldo, T. Todorova, Y H. Huang, P. Chang, A systematic analysis of the PARP protein family identifies new functions critical for cell physiology. Nat. Common 4, 2240 (2013)). These enzymes act to catalytically transfer polymeric or monomeric ADP to their target substrate proteins, and have the functions of gene expression, protein degradation and various cell responses to stress (M. S. Cohen, P. Chang, Insights into the biogenesis, function, and regulation of ADP-ribosylation. Nat Chem Biol 14, 236-243 (2018)). The biological functions of some of these enzymes, such as PARP 1 and PARP2, are well recognized; however, the biological functions of other enzymes are not clear at present. There are 17 members in this family, among which 4 (PARP1, PARP2, PARP5A and PARP5B) can be used to synthesize PAR chains. Most other enzymes in the family can only be used to construct a single ADP-ribose unit, so they are deemed as mono(ADP-ribosyl)ases (MARs). The viability of tumor cells under stress is the basic mechanism of tumor occurrence and a new approach for treatment. PARP 1, a member of PARP family, has been proved to be an effective therapeutic target in cancer. Regardless of DNA damage caused by gene mutation or cytotoxic chemotherapy, cell responses to stress are caused. Six drugs are approved in clinic, and several others are in the advanced development stage (A. Ohmoto, S. Yachida, Current status of poly(ADP-ribose)polymerase inhibitors and future directions. Onco Targets Ther 10, 5195-5208(2017).

Aryl hydrocarbon receptor (AHR) is a ligand-activated transcription factor involved in regulating various cellular functions, including pro-inflammatory reaction and biological metabolism (S. Feng, Z. Cao, X. Wang, Role of aryl hydrocarbon receptor in cancer. Biochim Biophys Acta 1836, 197-210 (2013); and B. Stockinger, P. Di Meglio, M. Gialitakis, J. H. Duarte, The aryl hydrocarbon receptor: multitasking in the immune system. Annu Rev Immunol 32, 403-432(2014)). AHR can be activated by a large number of ligands, including endogenous tryptophan produced by metabolism, such as kynurenic acid and some polycyclic aromatic hydrocarbons (C. A. Opitz et al., An endogenous tumor-promoting ligand of the human aryl hydrocarbon receptor. Nature 478, 197-203(2011)). The activation of AHR induces the expression of target genes, including genes involved in metabolism, such as cytochrome P4501A1 and P4501B1. Activation also leads to the increase of TCDD inducible Poly (ADP-Ribose) polymerase (TIPARP). For example, PARP7 acts as a negative regulator of some AHR transcription targets (L. MacPherson et al., Aryl hydrocarbon receptor repressor and TIPARP (ARTD14) use similar, but also distinct mechanisms to repress aryl hydrocarbon receptor signaling. Int J Mot Sci 15, 7939-7957 (2014).

PARP7 is a gene regulated by AHR and an important member of PARP family. PARP7 can only transfer one monomeric ADP- ribose (MAR), and is monoPARP. The PARP catalytic domain of PARP7 contains a zinc finger motif that can bind DNA and a WWE domain that can mediate protein interaction (Ma, Q et al. Biochem, 289,499-506, 2001). Monomeric ADP ribosylation mediated by PARP7 is a reversible post-translational modification involved in many important biological processes, such as immune cell functions, transcription regulation, protein expression and DNA repair. PARP7 is a part of negative feedback loop that regulates AHR activity, and AHR can regulate immune function, inflammation and stem differentiation, and play a role in cancer. PARP7 has been proved to be over-active in tumors and plays a key role in the survival of cancer cells. More importantly, many cancer cells rely on PARP7 to achieve intrinsic cell survival, and studies have shown that PARP7 can make cancer cells "escape" from the immune system. Inhibition of PARP7 can effectively inhibit the growth of cancer cells, restore interferon signal transduction and inhibit the brakes of innate and adaptive immune mechanisms. In several cancer models, PARP7 inhibitors show long-lasting tumor growth inhibition, effective anti-proliferation activity and interferon signal transduction recovery.

PARP7 can also be regulated by other transcription factors and signaling pathways, including androgen receptors (E. C. Bolton et al., Cell- and gene-specific regulation of primary target genes by the androgen receptor. Genes Dev 21, 2005-2017 (2007), platelet-derived growth factor (J. Schmahl, C. S. Raymond, P. Soriano, PDGF signaling specificity is mediated through multiple immediate early genes. Nat Genet 39, 52-60 2007)) and hypoxia-inducible factor 1(N. Hao et al., Xenobiotics and loss of cell adhesion drive distinct transcriptional outcomes by aryl hydrocarbon receptor signaling. Mot Pharmacol 82, 1082-1093(2012)). PARP7 has many cell functions. In the context of AHR signaling, PARP7 acts as a negative feedback mechanism to regulate the expression of P4501A1 and P4501B1 (L. MacPherson et al., Aryl

hydro- carbon receptor repressor and TIPARP (ARTD14) use similar, but also distinct mechanisms to repress aryl hydrocarbon receptor signaling. Int J Mot Sci 15, 7939-7957 (2014), and L. MacPherson et al., 2,3,7,8-Tetrachlorodibenzo-p-dioxin poly(ADP-ribose) polymerase (TIPARP, ARTD14) is a mono-ADP-ribosyltransferase and repressor of aryl hydro- carbon receptor transactivation. Nucleic Acids Res 41, 1604- 1621(2013)). PARP7 is also described to ADP-ribosylate liver X receptor, leading to the regulation of its transcription activity (C. Bindesboll et al., TCDD-inducible poly-ADP-ribose polymerase (TIPARP/PARP7) mono-ADP-ribosylates and co-activates liver X receptors. Biochem J 473, 899-910 (2016)). PARP7 can bind to Sindbis virus (SINV) to promote viral RNA degradation during viral infection ((T. Kozaki et al., Mitochondrial damage elicits a TCDD-inducible poly( ADP-ribose) polymerase-mediated antiviral response. Proc Natl Acad Sci USA 114, 2681-2686(2017)). Also in the case of viral infection, AHR-induced PARP7 can interact with TBK1, which is a major kinase activated during the initiation of pathogen-associated molecular pattern pathway, leading to the activation of type I interferon response and antiviral immunity (T. Yamada et al., Constitutive aryl hydrocarbon receptor signaling constrains Type I interferon-mediated antiviral innate defense Nat Immunol 17 687-604 (2016)). PARP7 has the activity of ADP-ribosylating TBK to inactivate TBK1, thus inhibiting the type I interferon response.

Based on the results of viral infections, it is hypothesized that cancer cells can evade the host immune system through T cell-mediated anti-tumor immunity by inhibiting type I interferon, using abnormally expressed or activated PARP7 as a mechanism. In fact, in a recent study, PARP7 was identified as a possible tumor factor that inhibits T cell activation (D. Pan et al., A major chromatin regulator determines resistance of tumor cells to T cell-mediated killing. Science 359, 770-775 (2018)). In mouse malignant tumor cell lines, PARP7 gene knockout can increase the proliferation and activation of co-cultured T cells, suggesting that PARP7 inhibition may be a feasible strategy to activate T cell-mediated tumor killing.

RBN-2397, developed by Ribon Therapeutics, is currently the first and only PARP7 inhibitor that has entered clinical phase I trial (NCT04053673). The preclinical experimental data show that RBN-2397 has potent anti-tumor growth effect increasing in a dose-dependent manner. More importantly, RBN-2397 induces tumor-specific adaptive immune memory (J. M. Gozgit, et al, PARP7 negatively regulates the type I interferon response in cancer cells and its inhibition triggers antitumor immunity. Cancer Cell 2021, 39, 1214-1226). This indicates that PARP7 inhibitor may be an excellent therapeutic agent against tumors. Therefore, it is of great significance to explore and develop PARP7 inhibitors with different molecular skeletons.

## CONTENT OF THE PRESENT INVENTION

The present invention provides a fused triheterocyclic compound, and a preparation method and use thereof. The fused triheterocyclic compound of the present invention can be used as a poly-ADP-ribose polymerase 7 (PARP 7) inhibitor; and is useful in the treatment of diseases that can be improved by inhibiting poly(ADP- ribose) polymerase 7 and in the preparation of drugs for preventing and/or treating abnormal proliferative diseases or other diseases, for example, cancers, related to the target to which this series of compounds target and bind.

The above technical problems are solved in the present invention through the following technical solutions.

The present invention provides a fused triheterocyclic compound of Formula IA or a pharmaceutically acceptable salt thereof,

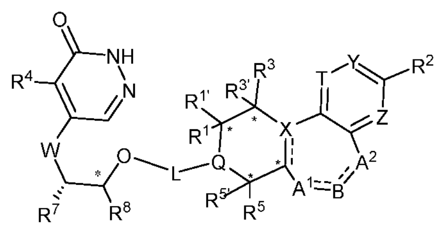

**IA**

where W is NH or O;

L is

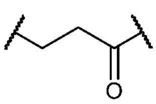, 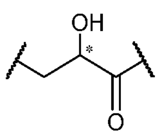, 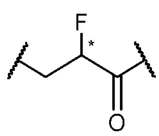, or 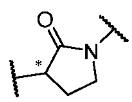;

$R^1$, $R^{1'}$, $R^3$, $R^{3'}$, $R^5$, and $R^{5'}$ are independently H, $C_{1-6}$ alkyl, or $C_{1-6}$ alkyl substituted with one or more $R^{1a}$; or $R^1$ and $R^{1'}$, $R^3$ and $R^{3'}$, and $R^5$ and $R^{5'}$ are independently taken together to form O=, where when more than one substituent is present, they are the same or different, and

$R^{1a}$ is independently halo, CN, or OH;

$R^2$ and $R^4$ are independently halo, CN, $SF_5$, $C_{1-6}$ alkyl, $C_{1-6}$ alkyl substituted with one or more $R^{2a}$, or $C_{1-6}$ alkyl-S-substituted with one or more $R^{2b}$, where when more than one substituent is present, they are the same or different, and $R^{2a}$ and $R^{2b}$ are independently halo;

X is independently $C(R^{3a})$, C or N;

Q is independently $C(R^{3a})$ or N, where when Q is $C(R^{3a})$, the carbon in $C(R^{3a})$ is a chiral carbon atom, and is in R configuration, S configuration or a combination thereof;

T is independently $C(R^{3b})$ or N; Y is independently $C(R^{3c})$ or N; and Z is independently $C(R^{3d})$ or N;

in which $R^{3a}$, $R^{3b}$, $R^{3c}$, and $R^{3d}$ are independently H, halo, CN, OH, $NH_2$, $C_{1-6}$ alkyl, $C_{1-6}$ alkyl substituted with one or more $R^{a4}$, or $C_{1-6}$ alkoxy;

$A^1$ is independently $C(R^{4a})$, $C(R^{4b}R^{4c})$ or C(=O), where $R^{4a}$, $R^{4b}$, and $R^{4c}$ are independently H, $C_{1-6}$ alkyl, or $C_{1-6}$ alkyl substituted with one or more $R^{a1}$, where when more than one substituent is present, they are the same or different;

when B is independently $C(R^{6a})$, or $C(R^{6b}R^{6c})$, $A^2$ is independently $C(R^{5a})$, $C(R^{5b}R^{5c})$, O, S, $N(R^{5d})$, N or a linkage;

when B is independently O, S, S(=O), $S(=O)_2$, N, or $N(R^{6d})$, $A^2$ is independently $C(R^{5a})$, $C(R^{5b}R^{5c})$, or a linkage;

in which $R^{5a}$, $R^{5b}$, $R^{5c}$, and $R^{5d}$ are independently H, $C_{1-6}$ alkyl, or $C_{1-6}$ alkyl substituted with one or more $R^{a2}$, or $R^{5b}$ and $R^{5c}$ are taken together to form =O;

$R^{6a}$, $R^{6b}$, and $R^{6c}$ are independently H, halo, $C_{1-6}$ alkyl, $C_{1-6}$ alkyl substituted with one or more $R^{a3}$, $C_{3-6}$ cycloalkyl, or OH, or $R^{6b}$ and $R^{6c}$ are taken together to form =O;

$R^{6d}$ is independently H, $C_{1-6}$ alkyl, $C_{1-6}$ alkyl substituted with one or more $R^{a3}$, $C_{3-6}$ cycloalkyl or -CN, where when more than one substituent is present, they are the same or different; and

$R^{a1}$, $R^{a2}$, $R^{a3}$, and $R^{a4}$ are independently deuterium, halo or OH;

$R^7$ and $R^8$ are independently H, $C_{1-6}$ alkyl, or $C_{1-6}$ alkyl substituted with $OR^{7a}$, where $R^{7a}$ is selected from H, $C_{1-6}$ alkyl and $C_{1-6}$ haloalkyl; and

---- represents a double bond or a single bond;* indicates a chiral carbon atom that is in R configuration, S configuration or a combination thereof.

In the present invention, in the fused triheterocyclic compound of Formula IA, some substituents can be defined as described below, and substituents not mentioned can be defined as described in any embodiment of the present application (referred to as "in a certain embodiment of the present invention" hereinafter).

In a certain embodiment of the present invention, $R^7$ and $R^8$ are independently $C_{1-6}$ alkyl or $C_{1-6}$ alkyl substituted with $OR^{7a}$, where the $C_{1-6}$ alkyl is independently methyl, ethyl, n-propyl or isopropyl, for example methyl.

In a certain embodiment of the present invention, $R^{7a}$ is selected from $C_{1-6}$ alkyl and $C_{1-6}$ haloalkyl, where the $C_{1-6}$ alkyl is independently methyl, ethyl, n-propyl or isopropyl.

In a certain embodiment of the present invention, $R^{7a}$ is selected from $C_{1-6}$ haloalkyl, where the halo is independently F, Cl, Br or I, for example F.

In a certain embodiment of the present invention, $R^7$ and $R^8$ are independently H or methyl. For example, one of $R^7$ and $R^8$ is H, and the other one is methyl.

In a certain embodiment of the present invention, when the carbon to which $R^8$ is attached is a chiral carbon, it is in S configuration or R configuration,
for example, R configuration.

In a certain embodiment of the present invention, the fused triheterocyclic compound of Formula IA or a pharmaceutically acceptable salt thereof is a fused triheterocyclic compound of Formula I' or a pharmaceutically acceptable salt thereof,

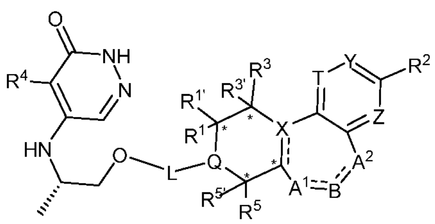

I'

where L is

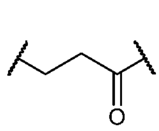, 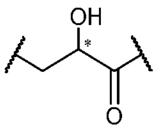, or 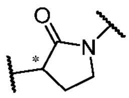;

$R^1$, $R^{1'}$, $R^3$, $R^{3'}$, $R^5$, and $R^{5'}$ are independently H, $C_{1-6}$ alkyl, or $C_{1-6}$ alkyl substituted with one or more $R^{1a}$; or $R^1$ and $R^{1'}$, $R^3$ and $R^{3'}$, and $R^5$ and $R^{5'}$ are independently taken together to form O=, where when more than one substituent is present, they are the same or different, and

$R^{1a}$ is independently halo, CN, or OH;

$R^2$ and $R^4$ are independently halo, CN, $C_{1-6}$ alkyl, $C_{1-6}$ alkyl substituted with one or more $R^{2a}$, or $C_{1-6}$ alkyl-S-substituted with one or more $R^{2b}$, where when more than one substituent is present, they are the same or different, and

$R^{2a}$ and $R^{2b}$ are independently halo;

X is independently $C(R^{3a})$, C or N;

Q is independently $C(R^{3a})$ or N, where

when Q is $C(R^{3a})$, the carbon in $C(R^{3a})$ is a chiral carbon atom, and is in R configuration, S configuration or a combination thereof;

T is independently $C(R^{3b})$ or N; Yis independently $C(R^{3c})$ or N; and Z is independently $C(R^{3d})$ or N;

in which $R^{3a}$, $R^{3b}$, $R^{3c}$, and $R^{3d}$ are independently H, halo, CN, $NH_2$, $C_{1-6}$ alkyl, $C_{1-6}$ alkyl substituted with one or more $R^{a4}$, or $C_{1-6}$ alkoxy;

$A^1$ is independently $C(R^{4a})$, $C(R^{4b}R^{4c})$ or C(=O), where

$R^{4a}$, $R^{4b}$, and $R^{4c}$ are independently H, $C_{1-6}$ alkyl, or $C_{1-6}$ alkyl substituted with one or more $R^{a1}$, where when more than one substituent is present, they are the same or different;

when B is independently $C(R^{6a})$, or $C(R^{6b}R^{6c})$, $A^2$ is independently $C(R^{5a})$, $C(R^{5b}R^{5c})$, O, S, $N(R^{5d})$, N or a linkage;

when B is independently O, S, S(=O), $S(=O)_2$, N, or $N(R^{6d})$, $A^2$ is independently $C(R^{5a})$, $C(R^{5b}R^{5c})$, or a linkage;

in which $R^{5a}$, $R^{5b}$, $R^{5c}$, and $R^{5d}$ are independently H, $C_{1-6}$ alkyl, or $C_{1-6}$ alkyl substituted with one or more $R^{a2}$, or $R^{5b}$ and $R^{5c}$ are taken together to form =O;

$R^{6a}$, $R^{6b}$, and $R^{6c}$ are independently H, halo, $C_{1-6}$ alkyl, $C_{1-6}$ alkyl substituted with one or more $R^{a3}$, $C_{3-6}$ cycloalkyl, or OH, or $R^{6b}$ and $R^{6c}$ are taken together to form =O;

$R^{6d}$ is independently H, $C_{1-6}$ alkyl, $C_{1-6}$ alkyl substituted with one or more $R^{a3}$, $C_{3-6}$ cycloalkyl or -CN, where when more than one substituent is present, they are the same or different; and

$R^{a1}$, $R^{a2}$, $R^{a3}$, and $R^{a4}$ are independently deuterium, halo or OH; and

---- represents a double bond or a single bond;* indicates a chiral carbon atom that is in R configuration, S configuration or a combination thereof.

In a certain embodiment of the present invention, the fused triheterocyclic compound of Formula IA is a fused triheterocyclic compound of Formula I: [0063]

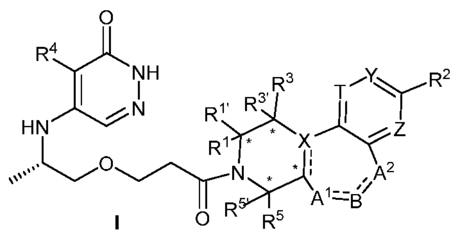

I

where $R^1$, $R^{1'}$, $R^3$, $R^{3'}$, $R^5$, and $R^{5'}$ are independently H, $C_{1-6}$ alkyl or $C_{1-6}$ alkyl substituted with one or more $R^{1a}$; or $R^1$ and $R^{1'}$, $R^3$ and $R^{3'}$, and $R^5$ and $R^{5'}$ are independently taken together to form O=, where when more than one substituent is present, they are the same or different, and

$R^{1a}$ is independently halo, CN, or OH;

$R^2$ and $R^4$ are independently halo, CN, $C_{1-6}$ alkyl, $C_{1-6}$ alkyl substituted with one or more $R^{2a}$, or $C_{1-6}$ alkyl-S-substituted with one or more $R^{2b}$, where when more than one substituent is present, they are the same or different, and

$R^{2a}$ and $R^{2b}$ are independently halo;

X is independently $C(R^{3a})$, C(when ---- is a double bond, that is,

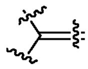)

or N;

T is independently $C(R^{3b})$ or N; Y is independently $C(R^{3c})$ or N; and Z is independently $C(R^{3d})$ or N;

in which $R^{3a}$, $R^{3b}$, $R^{3c}$, and $R^{3d}$ are independently H, halo, CN, $NH_2$ or $C_{1-6}$ alkyl;

$A^1$ is independently $C(R^{4a})$ (when ---- is a double bond), $C(R^{4b}R^{4c})$ (when ---- is a single bond) or C(=O), where $R^{4a}$, $R^{4b}$, and $R^{4c}$ are independently H, $C_{1-6}$ alkyl, or $C_{1-6}$ alkyl substituted with one or more $R^{a1}$, where when more than one substituent is present, they are the same or different;

when B is independently $C(R^{6a})$, or $C(R^{6b}R^{6c})$, $A^2$ is independently $C(R^{5a})$, $C(R^{5b}R^{5c})$, O, S, $N(R^{5d})$, N or a linkage;

when B is independently O, S, N or $N(R^{6d})$, $A^2$ is independently $C(R^{5a})$, $C(R^{5b}R^{5c})$ or a linkage;

in which $R^{5a}$, $R^{5b}$, $R^{5c}$, and $R^{5d}$ are independently H, $C_{1-6}$ alkyl or $C_{1-6}$ alkyl substituted with one or more $R^{a2}$;

$R^{6a}$, $R^{6b}$, and $R^{6c}$ are independently H, halo, $C_{1-6}$ alkyl, $C_{1-6}$ alkyl substituted with one or more $R^{a3}$, or $C_{3-6}$ cycloalkyl;

$R^{6d}$ is independently H, $C_{1-6}$ alkyl, $C_{1-6}$ alkyl substituted with one or more $R^{a3}$, or $C_{3-6}$ cycloalkyl, where when more than one substituent is present, they are the same or different; and

$R^{a1}$, $R^{a2}$, and $R^{a3}$ are independently deuterium, or halo; and

---- represents a double bond or a single bond;* indicates a chiral carbon atom that is in R configuration, S configuration or a combination thereof.

In a certain embodiment of the present invention, one of $R^1$ and $R^{1'}$ is H, and the other one is H, $C_{1-6}$ alkyl, or $C_{1-6}$ alkyl substituted with one or more $R^{1a}$.

For example, one of $R^1$ and $R^{1'}$ is H, and the other one is H or $C_{1-6}$ alkyl.

Alternatively, $R^1$ and $R^{1'}$ are taken together to form O=.

Preferably, $R^1$, and $R^{1'}$ are H.

In a certain embodiment of the present invention, one of $R^3$ and $R^{3'}$ is H, and the other one is H, $C_{1-6}$ alkyl, or $C_{1-6}$ alkyl substituted with one or more $R^{1a}$.

For example, one of $R^3$ and $R^{3'}$ is H, and the other one is H or $C_{1-6}$ alkyl.

Alternatively, $R^3$ and $R^{3'}$ are taken together to form O=.

Preferably, $R^3$, and $R^{3'}$ are H.

In a certain embodiment of the present invention, one of $R^5$ and $R^{5'}$ is H, and the other one is H, $C_{1-6}$ alkyl, or $C_{1-6}$ alkyl substituted with one or more $R^{1a}$,

For example, one of $R^5$ and $R^{5'}$ is H, and the other one is H or $C_{1-6}$ alkyl.

Alternatively, $R^5$ and $R^{5'}$ are taken together to form O=.

Preferably, $R^5$, and $R^{5'}$ are H.

In a certain embodiment of the present invention, one of $R^1$, $R^{1'}$, $R^3$, $R^{3'}$, $R^5$, and $R^{5'}$ is H, $C_{1-6}$ alkyl, or $C_{1-6}$ alkyl substituted with one or more $R^{1a}$, and the others are H.

For example, one of $R^1$, $R^{1'}$, $R^3$, $R^{3'}$, $R^5$, and $R^{5'}$ is H or $C_{1-6}$ alkyl, and the others are H.

Alternatively, one of $R^1$ and $R^{1'}$, $R^3$ and $R^{3'}$, $R^5$ and $R^{5'}$ is O=, and the others are H.

Preferably, $R^1$, $R^{1'}$, $R^3$, $R^{3'}$, $R^5$, and $R^{5'}$ are H.

In a certain embodiment of the present invention, when the carbon to which $R^1$ and $R^{1'}$ is attached is a chiral carbon, it is in S configuration or R configuration,

for example, R configuration.

In a certain embodiment of the present invention, when the carbon to which $R^3$ and $R^{3'}$ is attached is a chiral carbon, it is in S configuration or R configuration.

In a certain embodiment of the present invention, when the carbon to which $R^5$ and $R^{5'}$ is attached is a chiral carbon, it is in S configuration or R configuration.

In a certain embodiment of the present invention, $R^2$ is independently halo, CN, $C_{1-6}$ alkyl substituted with one or more $R^{2a}$, or $C_{1-6}$ alkyl-S- substituted with one or more $R^{2b}$,

for example, halo, or $C_{1-6}$ alkyl substituted with one or more $R^{2a}$, and

for example, $C_{1-6}$ alkyl substituted with one or more halo.

In a certain embodiment of the present invention, $R^4$ is independently halo, CN, or $C_{1-6}$ alkyl substituted with one or more $R^{2a}$,

for example, halo, or $C_{1-6}$ alkyl substituted with one or more $R^{2a}$, and

for example, $C_{1-6}$ alkyl substituted with one or more halo.

In a certain embodiment of the present invention, $R^2$ and $R^4$ are independently $C_{1-6}$ alkyl substituted with one or more $R^{2a}$,
for example, $-CF_3$.

In a certain embodiment of the present invention, X is independently N.

In a certain embodiment of the present invention, T is independently N.

In a certain embodiment of the present invention, $R^{3c}$ is H, CN, $NH_2$ or $C_{1-6}$ alkyl,
for example, H.

In a certain embodiment of the present invention, Y is independently $C(R^{3c})$,
for example, CH.

In a certain embodiment of the present invention, $R^{3d}$ is H, halo, CN or $C_{1-6}$ alkyl,
for example, H.

In a certain embodiment of the present invention, Z is independently $C(R^{3d})$,
for example, CH.

In a certain embodiment of the present invention, $R^{4a}$, $R^{4b}$, and $R^{4c}$ are independently H or $C_{1-6}$ alkyl,

for example, one of $R^{4b}$ and $R^{4c}$ is H, and the other one is H or $C_{1-6}$ alkyl, and
for example, $R^{4b}$ and $R^{4c}$ are H.

In a certain embodiment of the present invention, when $A^1$ is $C(R^{4b}R^{4c})$ and the carbon is a chiral carbon, it is in S configuration, R configuration or a combination thereof.

In a certain embodiment of the present invention, $A^1$ is independently $C(R^{4b}R^{4c})$ or C(=O), for example, $C(R^{4b}R^{4c})$.

In a certain embodiment of the present invention, $R^{5a}$, $R^{5b}$, $R^{5c}$, and $R^{5d}$ are independently H or $C_{1-6}$ alkyl,

for example, one of $R^{5b}$ and $R^{5c}$ is H, and the other one is H or $C_{1-6}$ alkyl, and
for example, $R^{5b}$ and $R^{5c}$ are H.

In a certain embodiment of the present invention, when $A^2$ is $C(R^{5b}R^{5c})$ and the carbon is a chiral carbon, it is in S configuration, R configuration or a combination thereof.

In a certain embodiment of the present invention, $A^2$ is independently $C(R^{5b}R^{5c})$ or a linkage.

In a certain embodiment of the present invention, $R^{6a}$, $R^{6b}$, and $R^{6c}$ are independently H or halo,
for example, $R^{6b}$ and $R^{6c}$ are independently H.

In a certain embodiment of the present invention, $R^{6d}$ is independently H, $C_{1-6}$ alkyl, $C_{1-6}$ alkyl substituted with one or more $R^{a3}$, or $C_{3-6}$ cycloalkyl,
for example, H or $C_{1-6}$ alkyl.

In a certain embodiment of the present invention, B is independently $C(R^{6b}R^{6c})$.

In a certain embodiment of the present invention, B is independently O, S or $N(R^{6d})$,

for example, -O- or -S-,
for example, S.

In a certain embodiment of the present invention,

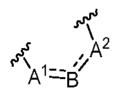

is independently

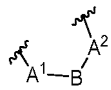,

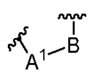 or 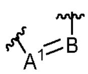.

In a certain embodiment of the present invention, when B is independently O, S, or N($R^{6d}$), $A^2$ is independently C($R^{5b}R^{5c}$) or a linkage.

In a certain embodiment of the present invention, when B is independently C($R^{6b}R^{6c}$), $A^2$ is independently O, N($R^{5d}$) or a linkage.

In a certain embodiment of the present invention, when B is independently N($R^{6d}$) and $R^{6d}$ is independently $C_{1-6}$ alkyl, or $C_{1-6}$ alkyl substituted with one or more $R^{a3}$, $A^1$ is independently C(=O).

In a certain embodiment of the present invention, when T and Z are both N, $A^2$ is independently O, N($R^{5d}$) or C($R^{5b}R^{5c}$).

In a certain embodiment of the present invention,

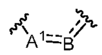

is independently - C($R^{4b}R^{4c}$)-O-, -C($R^{4b}R^{4c}$)-S-, -C($R^{4b}R^{4c}$)-N($R^{6d}$)-, -C($R^{4b}R^{4c}$)-C($R^{6b}R^{6c}$)-, -C(=O)-O-, -C(=O)-N($R^{6d}$)-, -C($R^{4b}R^{4c}$)-S(=O)-, -C($R^{4b}R^{4c}$)-S$(=O)_2$, or -C($R^{4a}$)=C($R^{6a}$)-.

In a certain embodiment of the present invention,

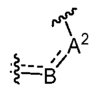

is independently -O-, -S-, - C($R^{6b}R^{6c}$)-, -N($R^{6d}$)-, -O-C($R^{5b}R^{5c}$)-, -S-C($R^{5b}R^{5c}$)-, -N($R^{6d}$)-C($R^{5b}R^{5c}$)-, -C($R^{6b}R^{6c}$)-O-, -C($R^{6b}R^{6c}$)-S-, -C($R^{6b}R^{6c}$)-N($R^{5d}$)-, -S(=O)-C($R^{5b}R^{5c}$)-, -S$(=O)_2$-C($R^{5b}R^{5c}$)-, -O-C($R^{6b}R^{6c}$)-, or =C($R^{6a}$)-.

In a certain embodiment of the present invention, when $R^{3a}$, $R^{3b}$, $R^{3c}$, and $R^{3d}$ are independently $C_{1-6}$ alkyl or $C_{1-6}$ alkyl substituted with one or more $R^{a4}$, the $C_{1-6}$ alkyl or $C_{1-6}$ alkyl in the $C_{1-6}$ alkyl substituted with one or more $R^{a4}$ is methyl, ethyl, n-propyl, isopropyl, n-butyl, isobutyl, s-butyl or t-butyl, for example, methyl. In a certain embodiment of the present invention, when $R^{3a}$, $R^{3b}$, $R^{3c}$, and $R^{3d}$ are independently $C_{1-6}$ alkoxy, the $C_{1-6}$ alkoxy is methoxy, ethoxy, n-propoxy, iso-propoxy, n-butoxy, iso-butoxy, s-butoxy or t-butoxy, for example, methoxy.

In a certain embodiment of the present invention,

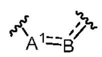

is independently - C($R^{4b}R^{4c}$)-O-, -C($R^{4b}R^{4c}$)-S-, -C($R^{4b}R^{4c}$)-N($R^{6d}$)-, -C($R^{4b}R^{4c}$)-C($R^{6b}R^{6c}$)-, -C(=O)-O-, or-C(=O)-N($R^{6d}$)-;

for example, -$CH_2$-O-, -$CH_2$-S-, -$CH_2$-NH-, -$CH_2$-N($CH_3$)-, -$CH_2$-N($CH_2CF_3$)-, -$CH_2$-$CH_2$-, -$CH_2$-$CF_2$-, -C($CH_3$)-O-, -C($CH_3$)-S-, -C(=O)-NH-, -C(=O)-O-, -C(=O)-N($CD_3$)-, -$CH_2$-N(cyclopropyl)-, or -C(=O)-N(cyclopropyl)-;
for example, -C($R^{4b}R^{4c}$)-O-, -C($R^{4b}R^{4c}$)-S-, -C($R^{4b}R^{4c}$)-N($R^{6d}$)-, or -C(=O)-N($R^{6d}$)-; and
for example, -$CH_2$-O-, or -$CH_2$-S-.

In a certain embodiment of the present invention,

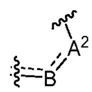

is independently -O-, -S-, - C($R^{6b}R^{6c}$)-, -N($R^{6d}$)-, -O-C($R^{5b}R^{5c}$)-, -S-C($R^{5b}R^{5c}$)-, -N($R^{6d}$)-C($R^{5b}R^{5c}$)-, -C($R^{6b}R^{6c}$)-O-, -C($R^{6b}R^{6c}$)-S-, or -C($R^{6b}R^{6c}$)-N($R^{5d}$)-;

for example, -O-, -O-C($R^{5b}R^{5c}$)-, -S-C($R^{5b}R^{5c}$)-, -N($R^{6d}$)-, or -N($R^{6d}$)-C($R^{5b}R^{5c}$)-; and
for example, -O-, -O-$CH_2$- or -S-$CH_2$-.

In a certain embodiment of the present invention,

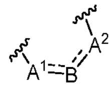

is independently

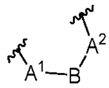

or

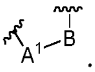

In a certain embodiment of the present invention,

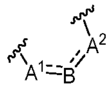

is independently - $C(R^{4b}R^{4c})$-O-, -$C(R^{4b}R^{4c})$-S-, -$C(R^{4b}R^{4c})$-N($R^{6d}$)-, -C(=O)-N($R^{6d}$)-, -$C(R^{4b}R^{4c})$-O-$C(R^{5b}R^{5c})$-,-$C(R^{4b}R^{4c})$-S-$C(R^{5b}R^{5c})$-, -$C(R^{4b}R^{4c})$-N($R^{6d}$)-$C(R^{5b}R^{5c})$-, -$C(R^{4b}R^{4c})$-$C(R^{6b}R^{6c})$-N($R^{5d}$)-, -$C(R^{4b}R^{4c})$-$C(R^{6b}R^{6c})$-O-, -$C(R^{4b}R^{4c})$-$C(R^{6b}R^{6c})$-S-, -C(=O)-N($R^{6d}$)-$C(R^{5b}R^{5c})$-, -C(=O)-O-$C(R^{5b}R^{5c})$-, or -$C(R^{4b}R^{4c})$-$C(R^{6b}R^{6c})$-;

for example, -$C(R^{4b}R^{4c})$-O-, -$C(R^{4b}R^{4c})$-S-, -$C(R^{4b}R^{4c})$-N($R^{6d}$)-, -C(-O)-N($R^{6d}$)-, - $C(R^{4b}R^{4c})$-O-$C(R^{5b}R^{5c})$-, -$C(R^{4b}R^{4c})$-S-$C(R^{5b}R^{5c})$- , -$C(R^{4b}R^{4c})$-N($R^{6d}$)-$C(R^{5b}R^{5c})$-;
for example, -$C(R^{4b}R^{4c})$-O-, -$C(R^{4b}R^{4c})$-S-, -$C(R^{4b}R^{4c})$-O-$C(R^{5b}R^{5c})$-, -$C(R^{4b}R^{4c})$-S-$C(R^{5b}R^{5c})$-; and
for example, -$CH_2$-O-, -$CH_2$-S-, -$CH_2$-O-$CH_2$-, -$CH_2$-S-$CH_2$-.

In a certain embodiment of the present invention,

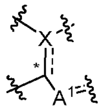

is independently

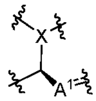

or

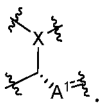

In a certain embodiment of the present invention, when $R^1$, $R^{1'}$, $R^3$, $R^{3'}$, $R^5$, and $R^{5'}$ are independently $C_{1-6}$ alkyl or $C_{1-6}$ alkyl substituted with one or more $R^{1a}$, the $C_{1-6}$ alkyl or $C_{1-6}$ alkyl in the $C_{1-6}$ alkyl substituted with one or more $R^{1a}$ is $C_1$-$C_4$ alkyl,

for example, methyl, ethyl, n-propyl, isopropyl, n-butyl, isobutyl, s-butyl or t-butyl; and

for example, methyl.

In a certain embodiment of the present invention, when $R^{1a}$ is halo, the halo is F, Cl, Br, or I,

for example, F.

In a certain embodiment of the present invention, when $R^2$ and $R^4$ are independently halo, the halo is F, Cl, Br, or I, for example, F.

In a certain embodiment of the present invention, when $R^2$ and $R^4$ are independently $C_{1-6}$ alkyl, $C_{1-6}$ alkyl substituted with one or more $R^{2a}$, or $C_{1-6}$ alkyl-S- substituted with one or more $R^{2b}$, the $C_{1-6}$ alkyl, and $C_{1-6}$ alkyl in the $C_{1-6}$ alkyl substituted with one or more $R^{2a}$ and in the $C_{1-6}$ alkyl-S- substituted with one or more $R^{2b}$ are $C_1$-$C_4$ alkyl,

for example, methyl, ethyl, n-propyl, isopropyl, n-butyl, isobutyl, s-butyl or t-butyl; and
for example, methyl.

In a certain embodiment of the present invention, when $R^2$ and $R^4$ are independently $C_{1-6}$ alkyl substituted with one or more $R^{2a}$, or $C_{1-6}$ alkyl-S- substituted with one or more $R^{2b}$, the number of the substituent is 1, 2, or 3, for example, 3.

In a certain embodiment of the present invention, when $R^{2a}$ and $R^{2b}$ are independently halo, the halo is F, Cl, Br, or I, for example, F.

In a certain embodiment of the present invention, when $R^{3a}$, $R^{3b}$, $R^{3c}$, and $R^{3d}$ are independently halo, the halo is F, Cl, Br, or I, for example, F.

In a certain embodiment of the present invention, when $R^{3a}$, $R^{3b}$, $R^{3c}$, and $R^{3d}$ are independently $C_{1-6}$ alkyl, the $C_{1-6}$ alkyl is $C_1$-$C_4$ alkyl,

for example, methyl, ethyl, n-propyl, isopropyl, n-butyl, isobutyl, s-butyl or t-butyl; and
for example, methyl.

In a certain embodiment of the present invention, when $R^{4a}$, $R^{4b}$, and $R^{4c}$ are independently $C_{1-6}$ alkyl or $C_{1-6}$ alkyl substituted with one or more $R^{a1}$, the $C_{1-6}$ alkyl and $C_{1-6}$ alkyl in the $C_{1-6}$ alkyl substituted with one or more $R^{a1}$ is $C_1$-$C_4$ alkyl,

for example, methyl, ethyl, n-propyl, isopropyl, n-butyl, isobutyl, s-butyl or t-butyl; and
for example, methyl.

In a certain embodiment of the present invention, when $R^{5a}$, $R^{5b}$, $R^{5c}$, and $R^{5d}$ are independently $C_{1-6}$ alkyl or $C_{1-6}$ alkyl substituted with one or more $R^{a2}$, the $C_{1-6}$ alkyl and $C_{1-6}$ alkyl in the $C_{1-6}$ alkyl substituted with one or more $R^{a2}$ is $C_1$-$C_4$ alkyl,

for example, methyl, ethyl, n-propyl, isopropyl, n-butyl, isobutyl, s-butyl or t-butyl; and
for example, methyl.

In a certain embodiment of the present invention, when $R^{6a}$, $R^{6b}$, and $R^{6c}$ are independently halo, the halo is F, Cl, Br, or I, for example, F.

In a certain embodiment of the present invention, when $R^{6a}$, $R^{6b}$, $R^{6c}$, and $R^{6d}$ are independently $C_{1-6}$ alkyl or $C_{1-6}$ alkyl substituted with one or more $R^{a3}$, the $C_{1-6}$ alkyl and $C_{1-6}$ alkyl in the $C_{1-6}$ alkyl substituted with one or more $R^{a3}$ is $C_1$-$C_4$ alkyl,

for example, methyl, ethyl, n-propyl, isopropyl, n-butyl, isobutyl, s-butyl or t-butyl; and

for example, methyl.

In a certain embodiment of the present invention, when $R^{6a}$, $R^{6b}$, $R^{6c}$, and $R^{6d}$ are independently $C_{3-6}$ cycloalkyl, the $C_{3-6}$ cycloalkyl is cyclopropyl, cyclobutyl, cyclopentyl, or cyclohexyl, for example, cyclopropyl.

In a certain embodiment of the present invention, when $R^{a1}$, $R^{a2}$, and $R^{a3}$ are independently halo, the halo is F, Cl, Br, or I, for example, F.

In a certain embodiment of the present invention, $R^1$, $R^{1'}$, $R^3$, $R^{3'}$, $R^5$, and $R^{5'}$ are independently H, methyl, HO-methyl, NC-methyl, $CHF_2$, or $CF_3$; or $R^1$ and $R^{1'}$, $R^3$ and $R^{3'}$, and $R^5$ and $R^{5'}$ are independently taken together to

form O=.

In a certain embodiment of the present invention, $R^2$ and $R^4$ are independently fluoro, bromo, chloro, CN, $CF_3$, or $CF_3$-S-.

In a certain embodiment of the present invention, T is independently N, or CH.

In a certain embodiment of the present invention, Q is independently N or CH.

In a certain embodiment of the present invention, Y is independently CH, N, C(CN), $C(NH_2)$, $C(CHF_2)$, or C(OMe).

In a certain embodiment of the present invention, Z is independently CH, N, C(CN), $C(NH_2)$, $C(CHF_2)$, or $C(CH_3)$.

In a certain embodiment of the present invention, $A^1$ is independently $CH_2$, $C(CH_3)$, C(=O), or CH.

In a certain embodiment of the present invention, B is independently $CH_2$, $CF_2$, O, S, NH, $N(CH_3)$, $N(CD_3)$, N (cyclopropyl), N $(CH_2CF_3)$, $C(CH_3)$, C(=O), N(CN), S(=O), $S(=O)_2$, CH(OH), N $(CH_2CH_3)$, N $(CH_2CH_2OH)$, or CH.

In a certain embodiment of the present invention, $A^2$ is independently $CH_2$, O, S, NH, $N(CH_3)$, N (cyclopropyl), $CH(CH_3)$, a linkage, or C(=O).

In a certain embodiment of the present invention,

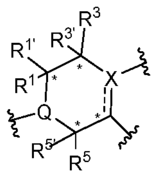

is independently

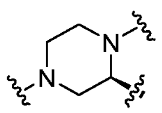 , 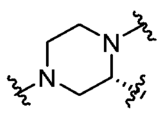 , 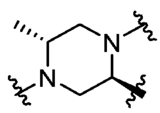 , 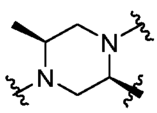 , 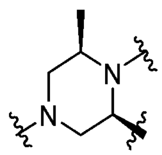 , 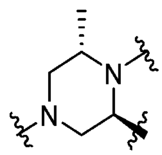 ,

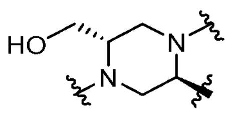 , 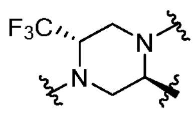 , 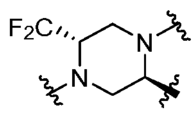 , 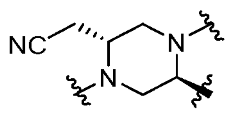 , 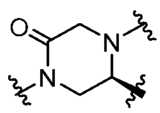 ,

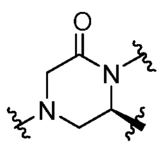 , 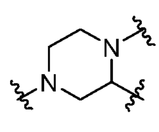 , 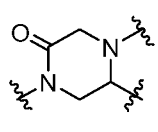 , 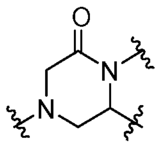 , 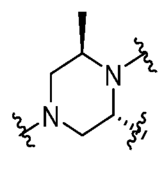 , 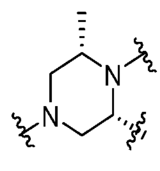 ,

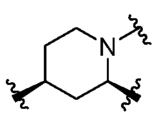 or 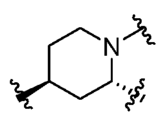 .

In a certain embodiment of the present invention,

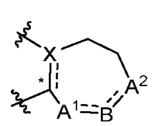

is independently

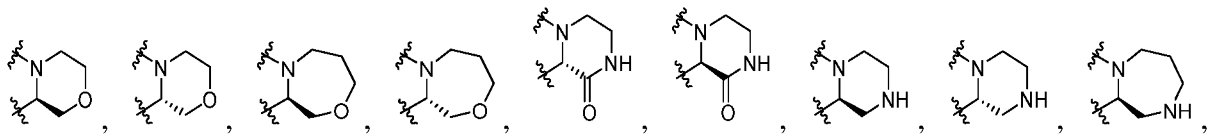
, , , , , , , , ,

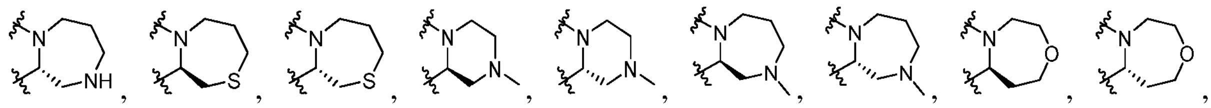
, , , , , , , , ,

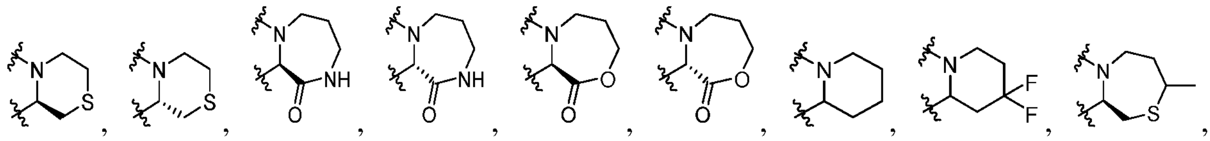
, , , , , , , , ,

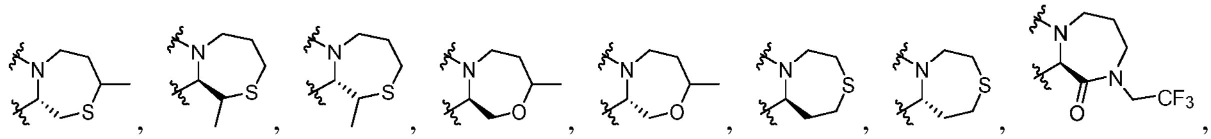
, , , , , , , ,

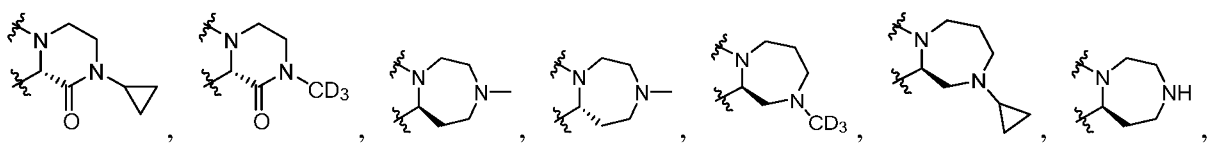
, , , , , , ,

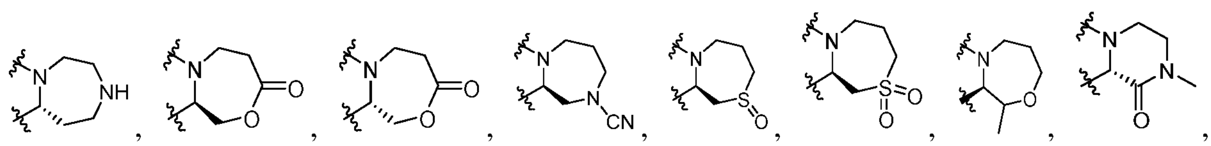
, , , , , , , ,

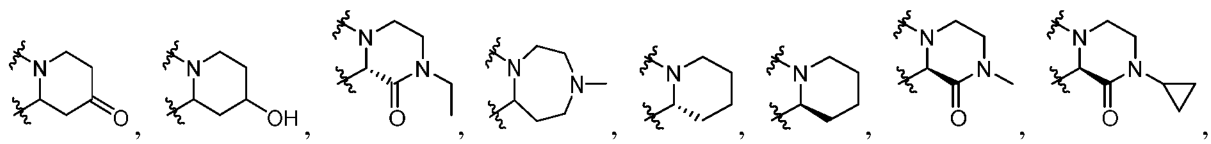
, , , , , , , , ,

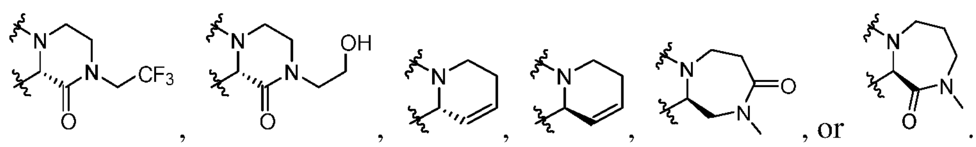
, , , , , or .

In a certain embodiment of the present invention,

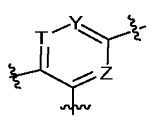

is independently

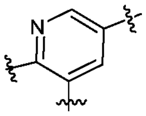, 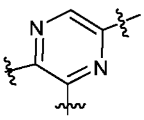, 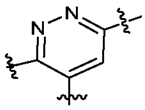, 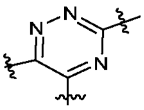 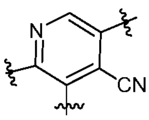, 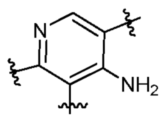, 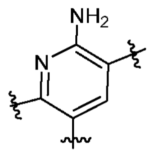,

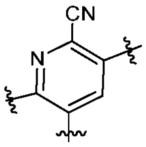, 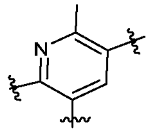, 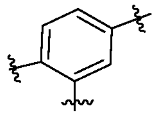, 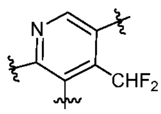, 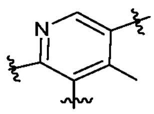, 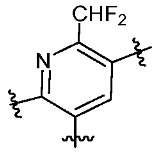 or

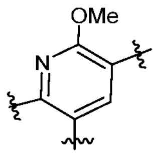.

In a certain embodiment of the present invention, Y is independently CH, N, C(CN), or C($NH_2$), for example, CH, or N.

In a certain embodiment of the present invention, Z is independently CH, N, C(CN), or C($NH_2$), for example, CH or N.

In a certain embodiment of the present invention, $A^1$ is independently $CH_2$, C($CH_3$) or C(=O).

In a certain embodiment of the present invention, B is independently $CH_2$, $CF_2$, O, S, NH, N($CH_3$), N($CD_3$), N (cyclopropyl), N ($CH_2CF_3$), C($CH_3$) or C(=O).

In a certain embodiment of the present invention, $A^2$ is independently $CH_2$, O, S, NH, N($CH_3$), N (cyclopropyl), CH($CH_3$) or a linkage.

In a certain embodiment of the present invention,

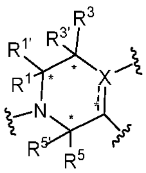

is independently

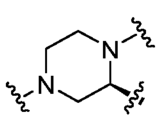, 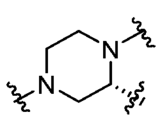, 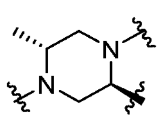, 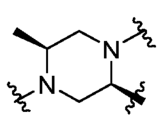, 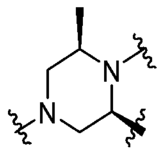, 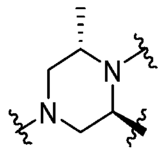,

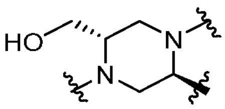, 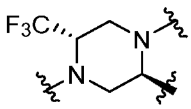, 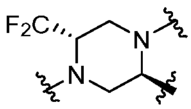, 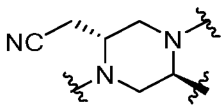, 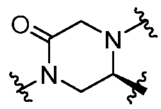,

or

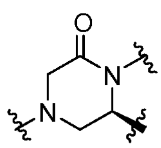,

for example,

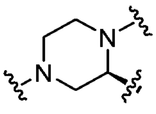, 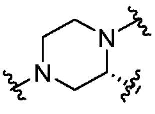, 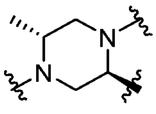, or 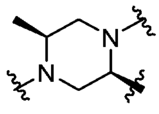.

In a certain embodiment of the present invention,

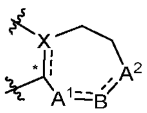

is independently

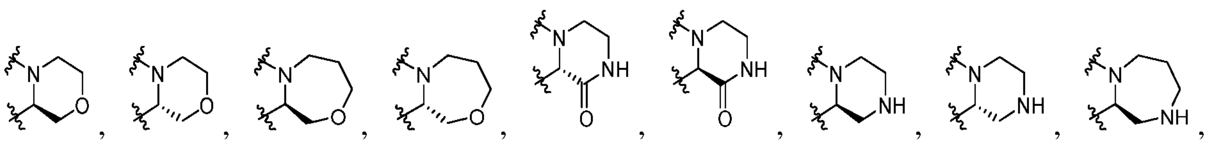

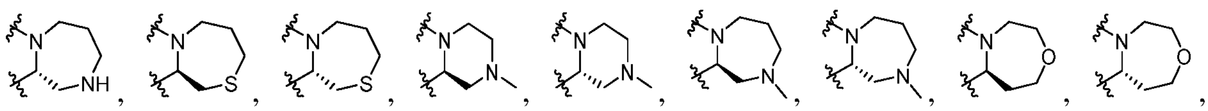

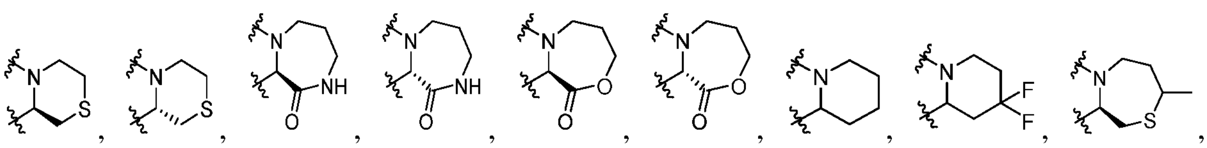

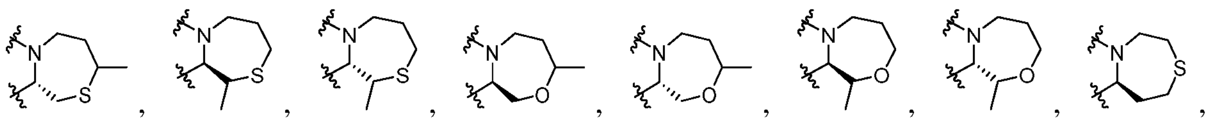

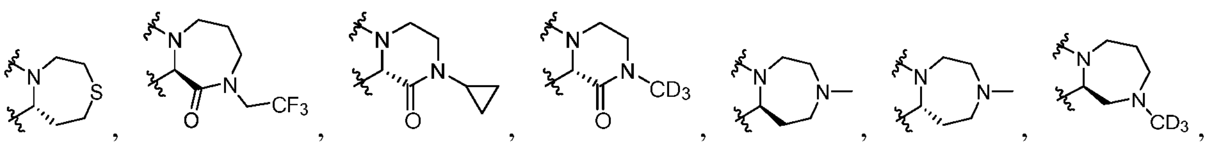

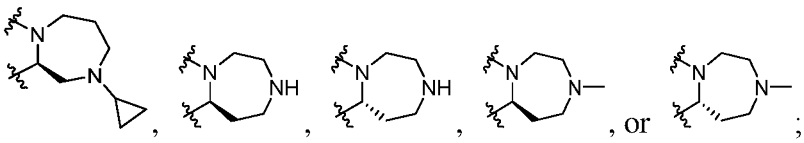

for example,

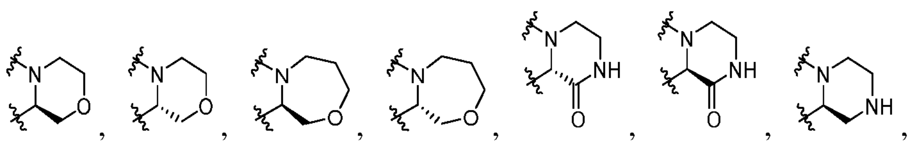

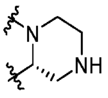, 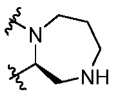, 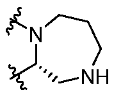, 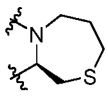, 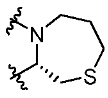, 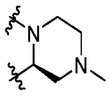, 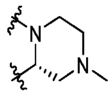, 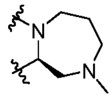, or 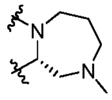;

for example,

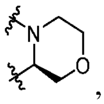, 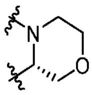, 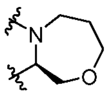, 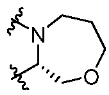, 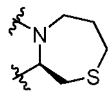, or 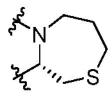.

In a certain embodiment of the present invention,

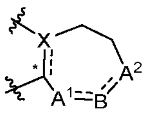

is independently

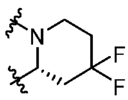, 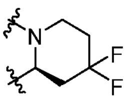, 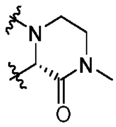, 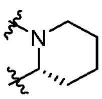, 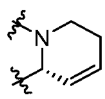, 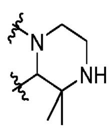, 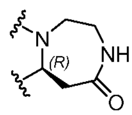, or 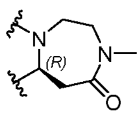.

In a certain embodiment of the present invention,

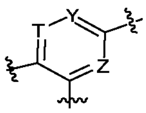

is independently

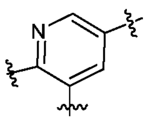, 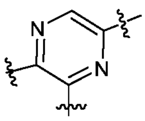, 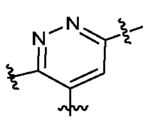, 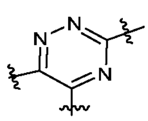, 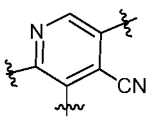, 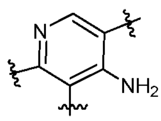, 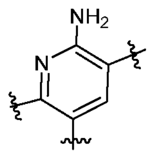,

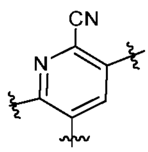, or 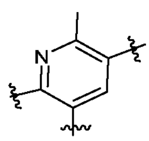;

for example

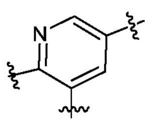 or 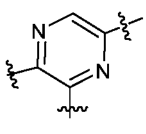;

for example

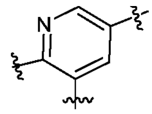

.

In a certain embodiment of the present invention,

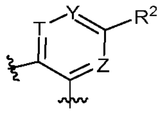

is independently

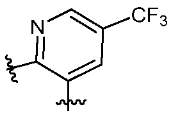

,

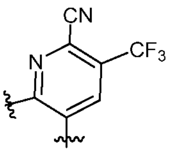

,

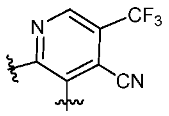

,

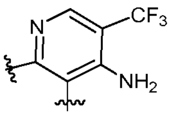

,

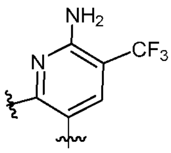

,

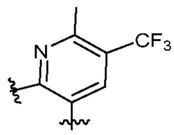

,

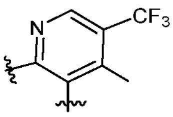

,

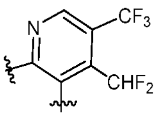

,

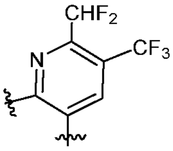

,

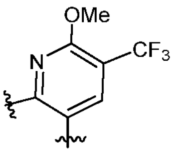

,

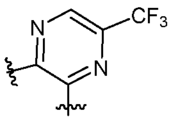

,

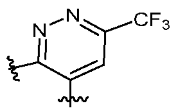

,

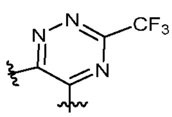

,

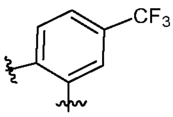

, or

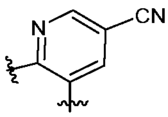

.

In a certain embodiment of the present invention,

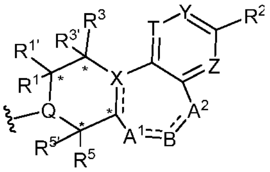

is independently

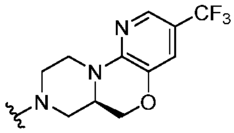

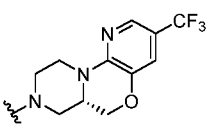

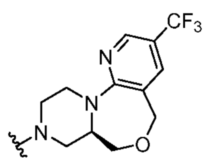

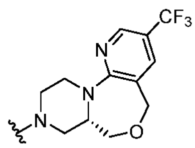

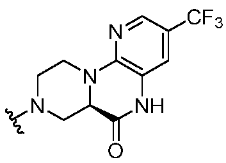

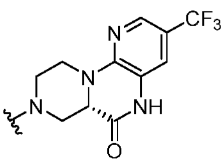

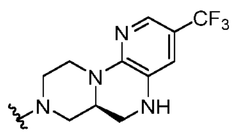

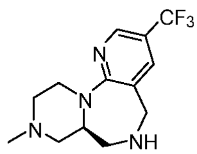

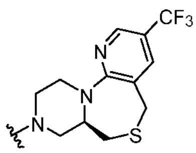

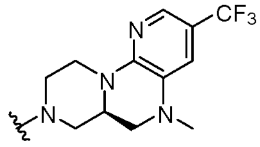
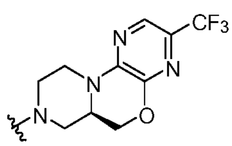
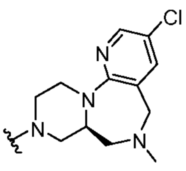
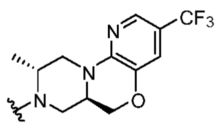
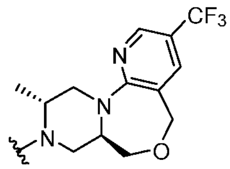

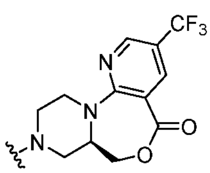
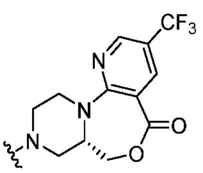
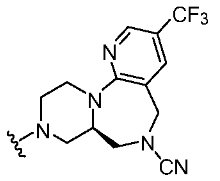
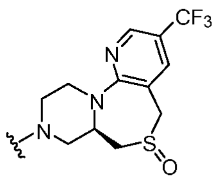
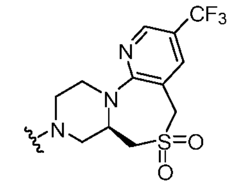

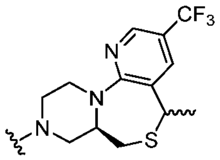
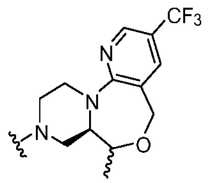
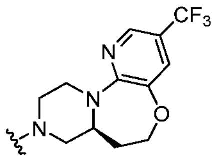
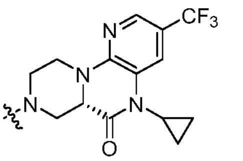
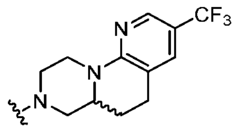

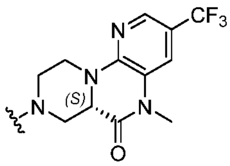
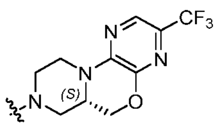
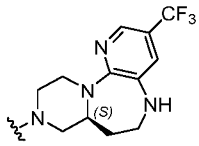
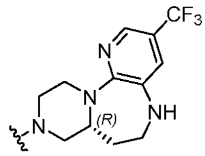
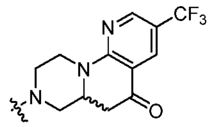

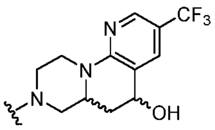
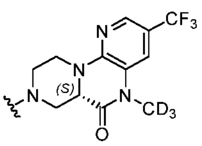
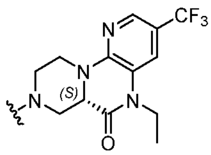
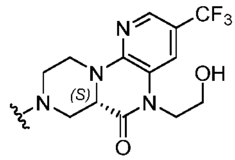
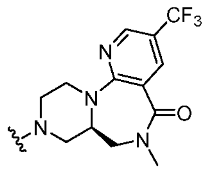

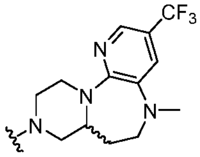
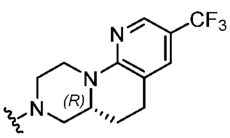
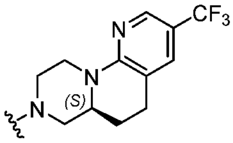
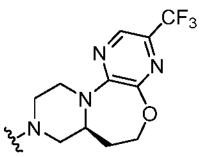
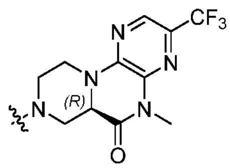

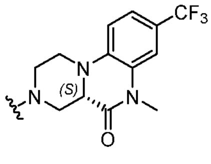
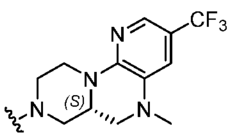
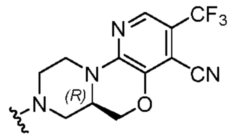
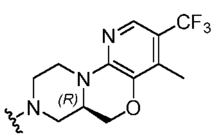
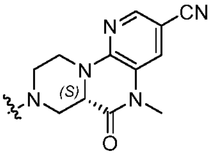

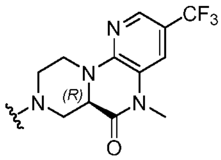
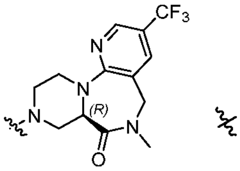
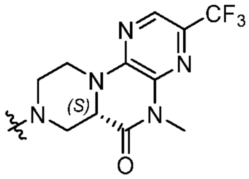
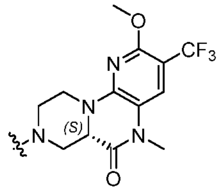
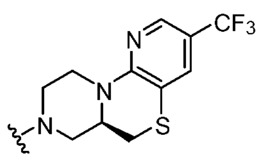

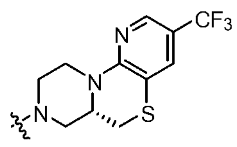 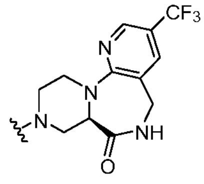 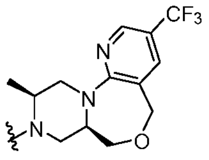 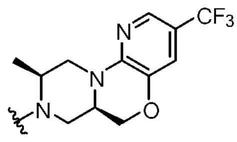 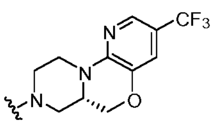

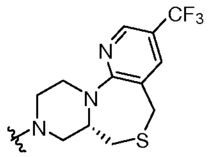 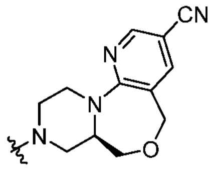 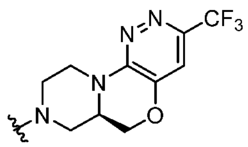 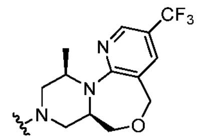 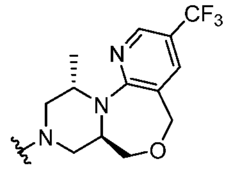

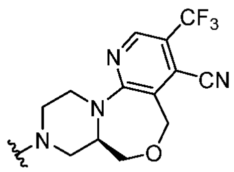 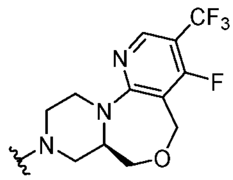 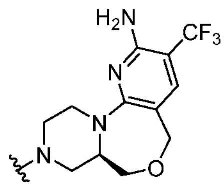 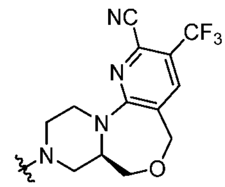 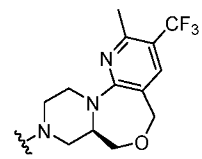

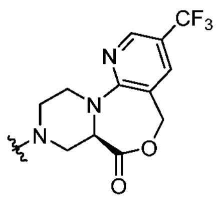 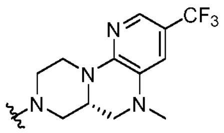 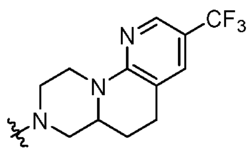 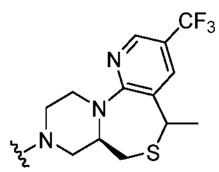 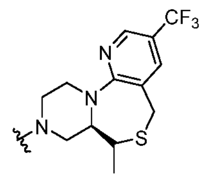

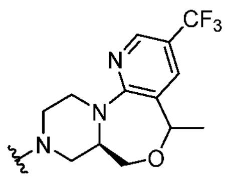 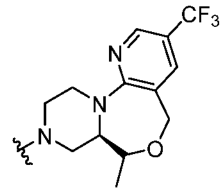 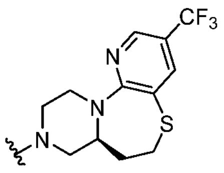 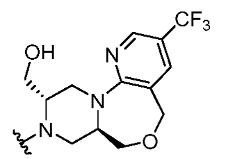 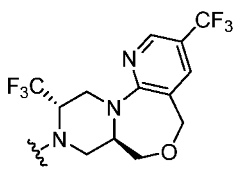

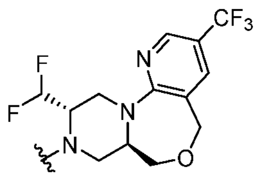 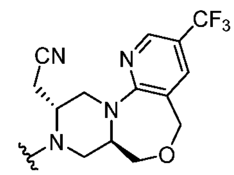 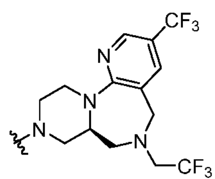 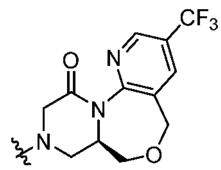 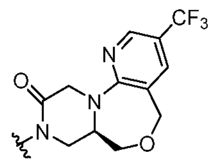

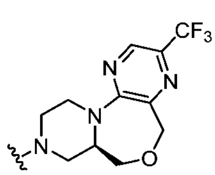 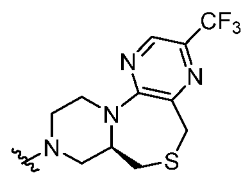 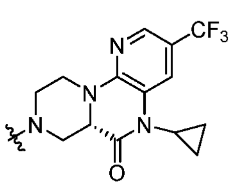 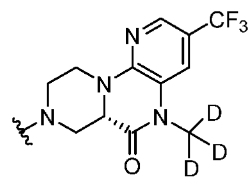

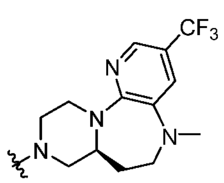 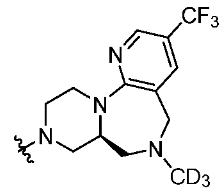 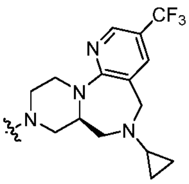 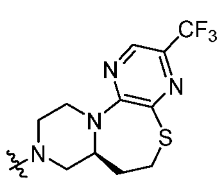 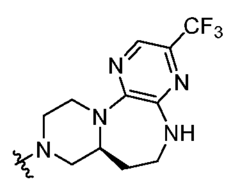

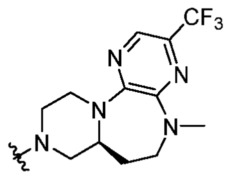
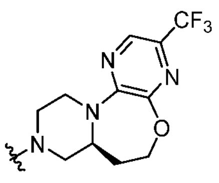
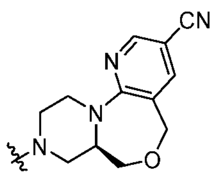
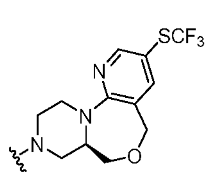
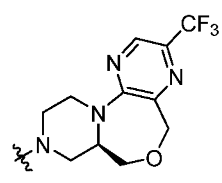

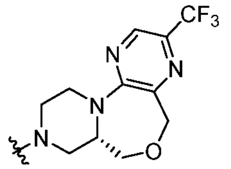
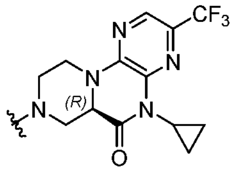
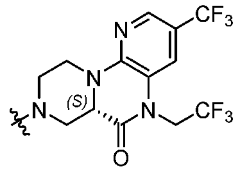
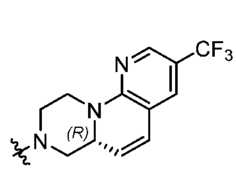

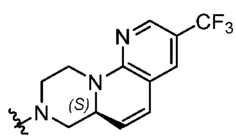
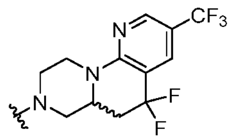
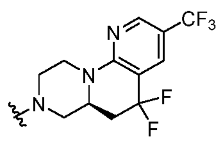
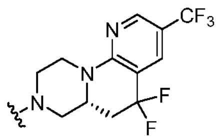
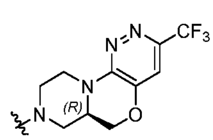

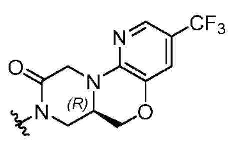
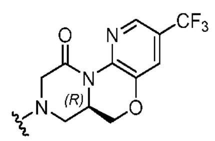
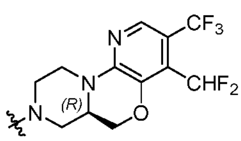
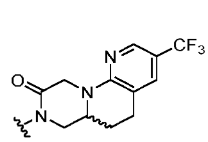
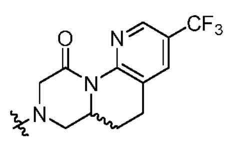

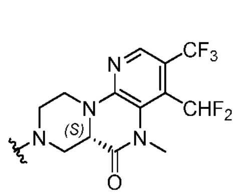
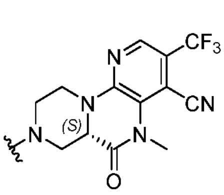
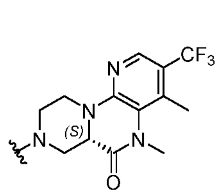
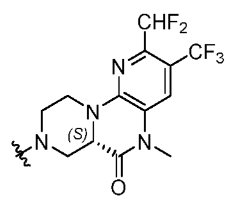
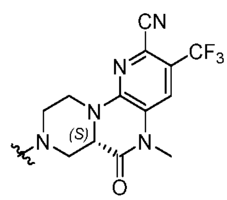

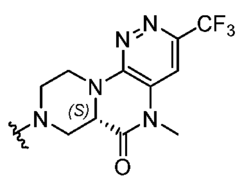
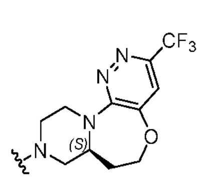
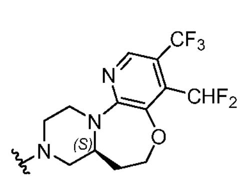
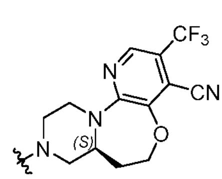
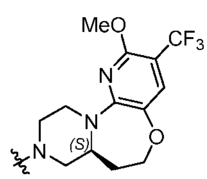

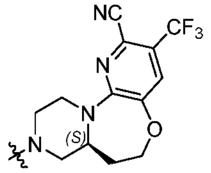
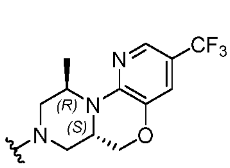
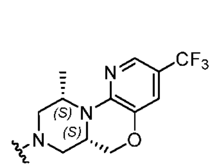
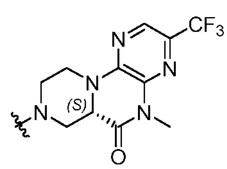
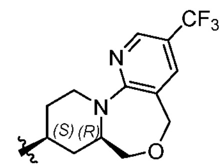

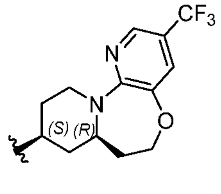
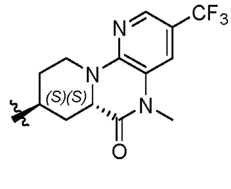
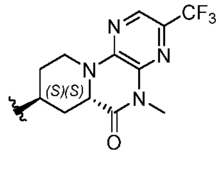
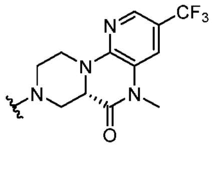
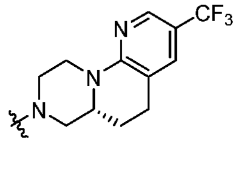

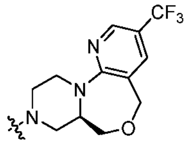 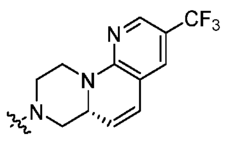 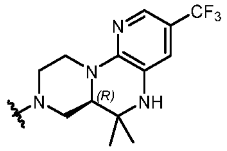 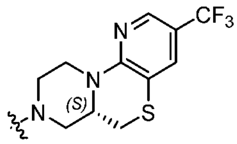 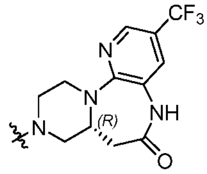

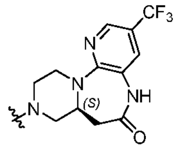 or 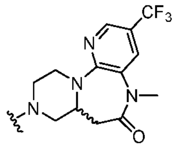 ,

where "∿∿" bond represents a combination of R configuration and S configuration.

In a certain embodiment of the present invention,

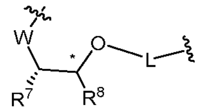

is independently

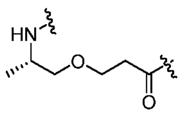 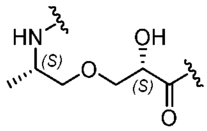 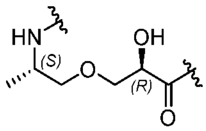 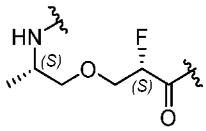 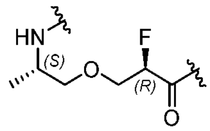

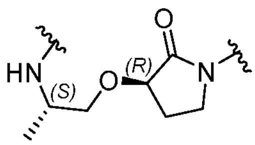 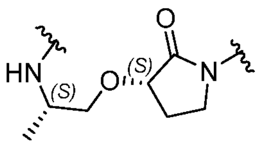 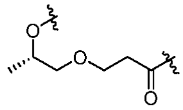 or 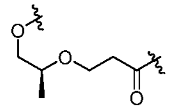 .

In a certain embodiment of the present invention,

$R^1$, and $R^{1'}$ are independently H or $C_{1-6}$ alkyl;
$R^3$, $R^{3'}$, $R^5$, and $R^{5'}$ are independently H;
$R^2$, and $R^4$ are independently $C_{1-6}$ alkyl substituted with one or more halo;
X and T are independently N;
Y is independently $C(R^{3c})$;
Z is independently $C(R^{3d})$ or N;
$A^1$ is independently $C(R^{4b}R^{4c})$ or C(=O);
B is independently O, S, N or $N(R^{6d})$, and $A^2$ is independently $C(R^{5b}R^{5c})$ or a linkage; and
---- represents a single bond.

In a certain embodiment of the present invention,

$R^1$, and $R^{1'}$ are independently H or methyl;
$R^3$, $R^{3'}$, $R^5$, and $R^{5'}$ are independently H;
$R^2$, and $R^4$ are independently $CF_3$;
X and T are independently N;
Y is independently CH;
Z is independently CH or N;
$A^1$ is independently $CH_2$ or C(=O);
B is independently O, S, N or NH, and $A^2$ is independently $CH_2$ or a linkage; and

= represents a single bond.

In a certain embodiment of the present invention,

$R^1$, $R^{1'}$, $R^3$, $R^{3'}$, $R^5$, and $R^{5'}$ are independently H;
$R^2$, and $R^4$ are independently $C_{1-6}$ alkyl substituted with one or more halo;
X and T are independently N;
Y is independently $C(R^{3c})$;
Z is independently $C(R^{3d})$;
$A^1$ is independently $C(R^{4b}R^{4c})$;
B is independently O or S;
$A^2$ is independently $C(R^{5b}R^{5c})$ or a linkage; and
= represents a single bond.

In a certain embodiment of the present invention,

$R^1$, $R^{1'}$, $R^3$, $R^{3'}$, $R^5$, and $R^{5'}$ are independently H;
$R^2$, and $R^4$ are independently $CF_3$;
X and T are independently N;
Y, and Z are independently CH;
$A^1$ is independently $CH_2$;
B is independently O or S;
$A^2$ is independently $CH_2$ or a linkage; and
---- represents a single bond.

In a certain embodiment of the present invention,

Lis

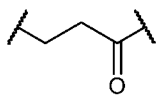 , 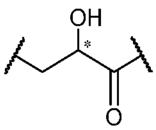 , or 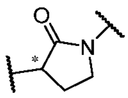 ;

$R^1$, and $R^{1'}$ are independently H, or $R^1$ and $R^{1'}$ are taken together to form O=;
$R^3$, and $R^{3'}$ are independently H, $C_{1-6}$ alkyl, or $R^3$ and $R^{3'}$ are taken together to form O=;
$R^5$, and $R^{5'}$ are independently H;
$R^2$, and $R^4$ are independently halo, CN, or $C_{1-6}$ alkyl substituted with one or more halo;
X is N;
T is $C(R^{3b})$ or N;
$R^{3b}$ is H;
Y is $C(R^{3c})$;
$R^{3c}$ is H, $C_{1-6}$ alkyl substituted with one or more $R^{a4}$, or $C_{1-6}$ alkoxy;
Z is $C(R^{3d})$;
$R^{3d}$ is H, $C_{1-6}$ alkyl or $C_{1-6}$ alkyl substituted with one or more $R^{a4}$;
$A^1$ is independently $C(R^{4a})$, $C(R^{4b}R^{4c})$ or C(=O), where
$R^{4a}$, $R^{4b}$, and $R^{4c}$ are independently H or $C_{1-6}$ alkyl;
when B is independently O, S(=O), $S(=O)_2$, or $N(R^{6d})$, $A^2$ is independently $C(R^{5a})$, $C(R^{5b}R^{5c})$, or a linkage;
when B is independently $C(R^{6a})$, or $C(R^{6b}R^{6c})$, $A^2$ is independently $N(R^{5d})$, or a linkage;
in which $R^{5b}$ and $R^{5c}$ are independently H, or $R^{5b}$ and $R^{5c}$ are taken together to form =O;
$R^{5d}$ is $C_{1-6}$ alkyl;
$R^{6d}$ is $C_{1-6}$ alkyl, $C_{1-6}$ alkyl substituted with one or more $R^{a3}$, $C_{3-6}$ cycloalkyl, or CN;
$R^{6a}$ is H;
$R^{6b}$ and $R^{6c}$ are independently H, -OH, or $R^{6b}$ and $R^{6c}$ are taken together to form =O; and
$R^{a3}$ is halo or -OH; and
---- represents a single bond.

In a certain embodiment of the present invention,

where L is

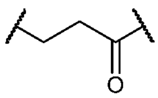 or 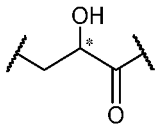 ;

$R^1$, and $R^{1'}$ are independently H or $C_{1-6}$ alkyl;
$R^3$, $R^{3'}$, $R^5$, and $R^{5'}$ are independently H;
$R^2$ is CN, or $C_{1-6}$ alkyl substituted with one or more halo;
$R^4$ is $C_{1-6}$ alkyl substituted with one or more halo;
X is N;
T is $C(R^{3b})$ or N, where $R^{3b}$ is H;
Y is independently $C(R^{3c})$, where $R^{3c}$ is H or $C_{1-6}$ alkyl;
Z is independently $C(R^{3d})$ or N, where $R^{3d}$ is H, CN or $C_{1-6}$ alkyl;
$A^1$ is independently $C(R^{4b}R^{4c})$ or C(=O), where $R^{4b}$ and $R^{4c}$ are independently H;
when B is independently O, S, S(=O), $S(=O)_2$, or $N(R^{6d})$, $A^2$ is independently $C(R^{5b}R^{5c})$ or a linkage;
when B is independently $C(R^{6b}R^{6c})$, $A^2$ is independently O, $N(R^{5d})$ or a linkage;
in which $R^{5b}$ and $R^{5c}$ are independently H, or $C_{1-6}$ alkyl, or $R^{5b}$ and $R^{5c}$ are taken together to form =O;
$R^{6d}$ is H, $C_{1-6}$ alkyl, $C_{1-6}$ alkyl substituted with one or more $R^{a3}$, $C_{3-6}$ cycloalkyl, or CN;
$R^{5d}$ is H, or $C_{1-6}$ alkyl;
$R^{6b}$ and $R^{6c}$ are independently H, -OH, or $R^{6b}$ and $R^{6c}$ are taken together to form =O; and
$R^{a3}$ is deuterium or -OH; and
= represents a single bond.

In a certain embodiment of the present invention,

Lis

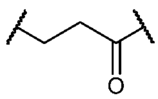 ;

$R^1$, and $R^{1'}$ are independently H or $C_{1-6}$ alkyl;
$R^3$, $R^{3'}$, $R^5$, and $R^{5'}$ are independently H;
$R^2$ is CN, or $C_{1-6}$ alkyl substituted with one or more halo;
$R^4$ is $C_{1-6}$ alkyl substituted with one or more halo;
X and T are independently N;
Y is independently $C(R^{3c})$, where $R^{3c}$ is H;
Z is independently $C(R^{3d})$ or N, where $R^{3d}$ is H;
$A^1$ is independently $C(R^{4b}R^{4c})$ or C(=O), where $R^{4b}$ and $R^{4c}$ are independently H;
when B is independently O, S, or $N(R^{6d})$, $A^2$ is independently $C(R^{5b}R^{5c})$ or a linkage;
when B is independently $C(R^{6b}R^{6c})$, $A^2$ is independently O, $N(R^{5d})$ or a linkage;
when $A^1$ is C(=O) and B is $N(R^{6d})$, $A^2$ is a linkage;
in which $R^{5b}$ and $R^{5c}$ are independently H;
$R^{6d}$ is H, $C_{1-6}$ alkyl, $C_{1-6}$ alkyl substituted with one or more $R^{a3}$, or $C_{3-6}$ cycloalkyl;
$R^{6b}$ and $R^{6c}$ are independently H, or -OH, or $R^{6b}$ and $R^{6c}$ are taken together to form =O;
$R^{5d}$ is H, or $C_{1-6}$ alkyl; and
$R^{a3}$ is deuterium; and
---- represents a single bond.

In a certain embodiment of the present invention,

L is

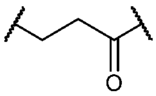 ;

$R^1$, $R^{1'}$, $R^3$, $R^{3'}$, $R^5$, and $R'$ are independently H;
$R^2$, and $R^4$ are independently $C_{1-6}$ alkyl substituted with one or more halo;
X and T are independently N;
Y is independently $C(R^{3c})$, where $R^{3c}$ is H;
Z is independently $C(R^{3d})$ or N, where $R^{3d}$ is H;
$A^1$ is independently $C(R^{4b}R^{4c})$ or C(=O), where $R^{4b}$ and $R^{4c}$ are independently H;
when B is independently O, S, or $N(R^{6d})$, $A^2$ is independently $C(R^{5b}R^{5c})$ or a linkage;
when B is independently $C(R^{6b}R^{6c})$, $A^2$ is independently O, $N(R^{5d})$ or a linkage;
when B is independently $N(R^{6d})$, $A^1$ is independently C(=O);
when $A^1$ is C(=O) and B is $N(R^{6d})$, $A^2$ is a linkage;
when T and Z are both N, $A^2$ is independently O, $N(R^{5d})$ or $C(R^{5b}R^{5c})$;
in which $R^{5b}$ and $R^{5c}$ are independently H;
$R^{6d}$ are independently $C_{1-6}$ alkyl, or $C_{1-6}$ alkyl substituted with one or more $R^{a3}$;
in which $R^{6b}$ and $R^{6c}$ are independently H;
$R^{5d}$ is H; and
$R^{a3}$ is deuterium; and
= represents a single bond.

In a certain embodiment of the present invention, the fused triheterocyclic compound of Formula IA has any of the structures shown below:

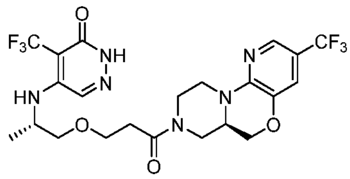 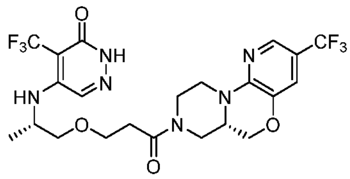 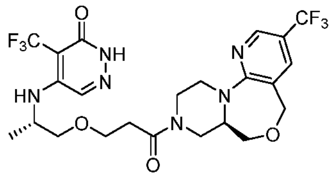

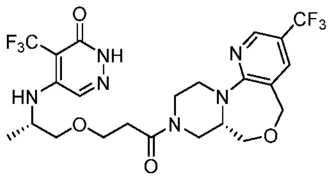 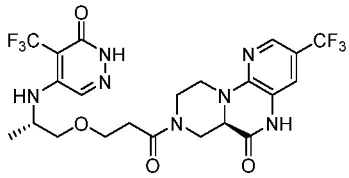 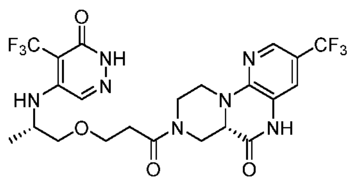

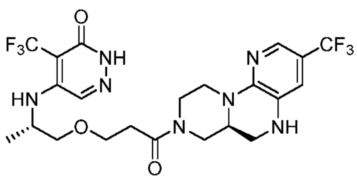 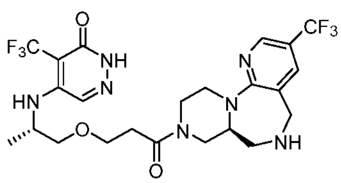 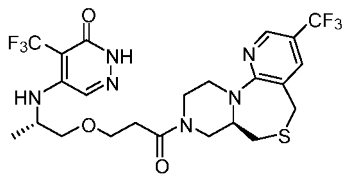

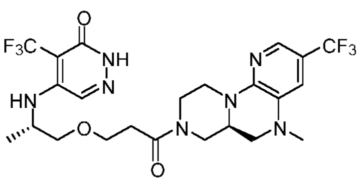 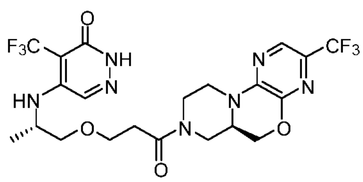 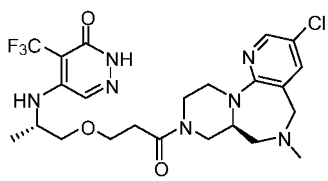

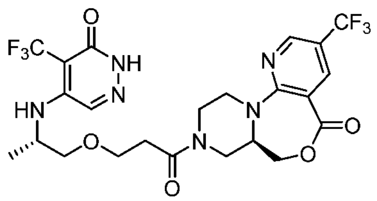
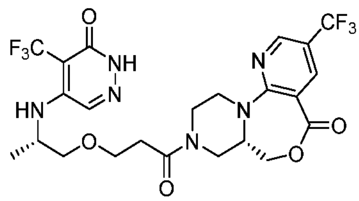
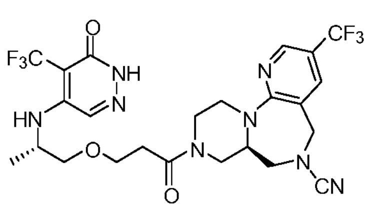
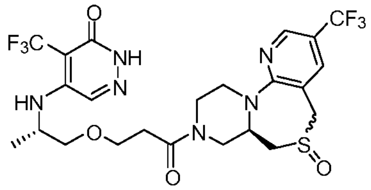
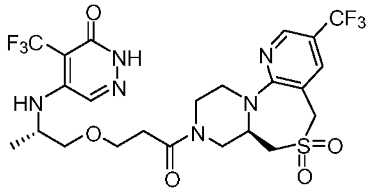
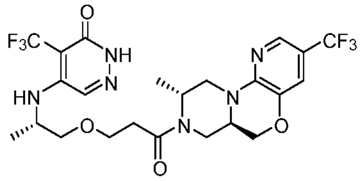
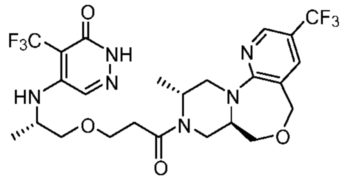
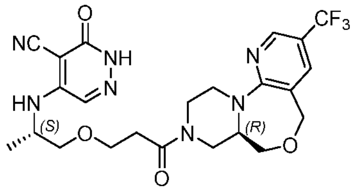
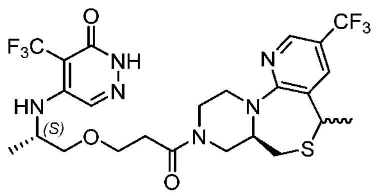
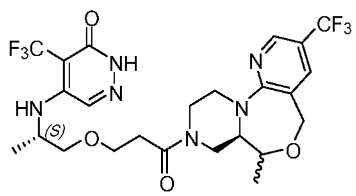
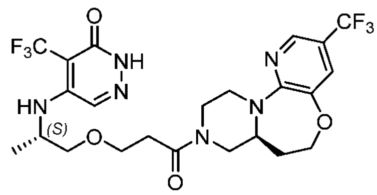
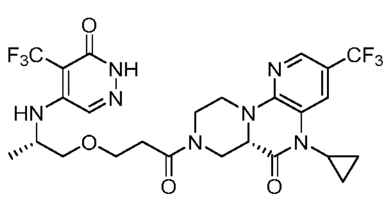
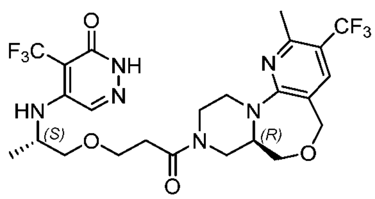
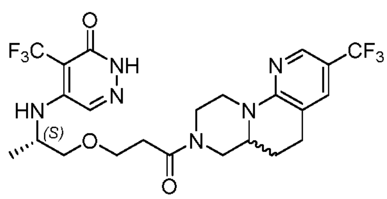
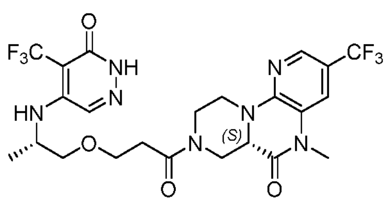
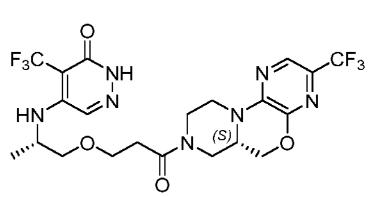
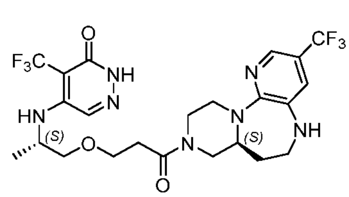
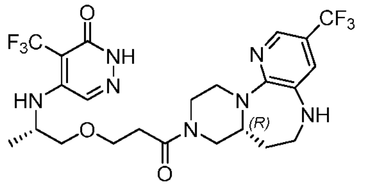
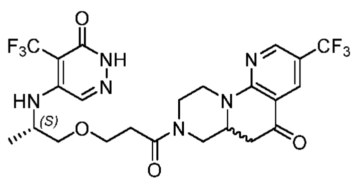
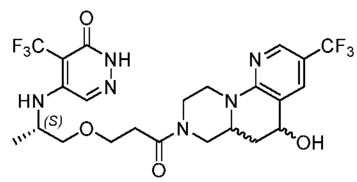

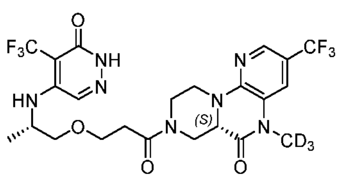
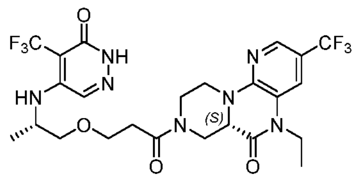
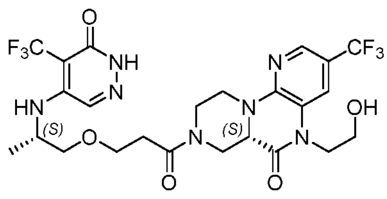

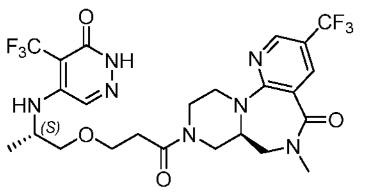
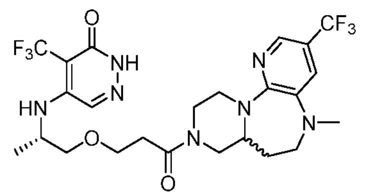
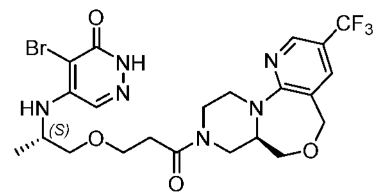

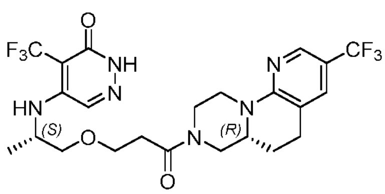
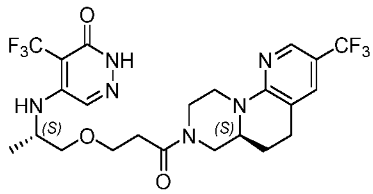
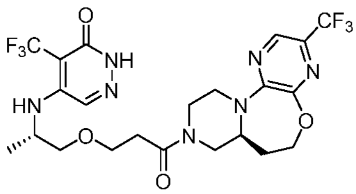

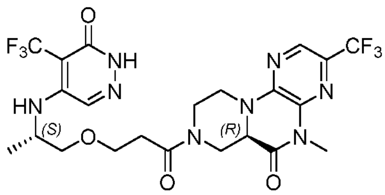
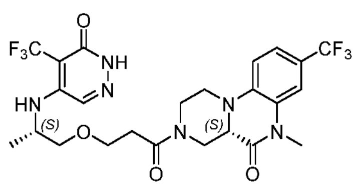
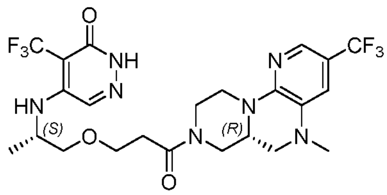

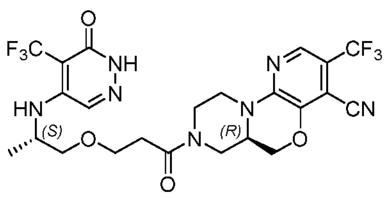
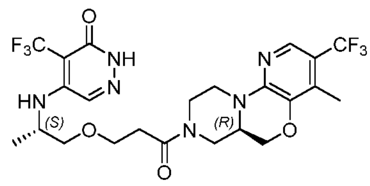
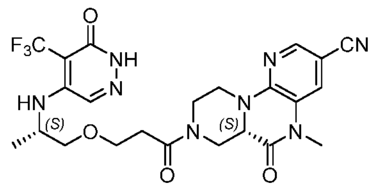

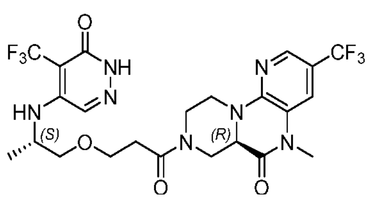
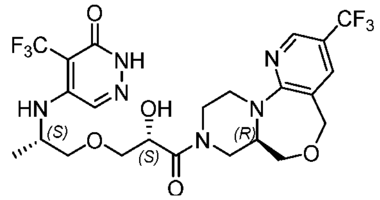
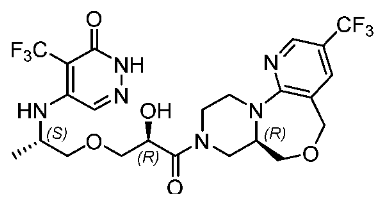

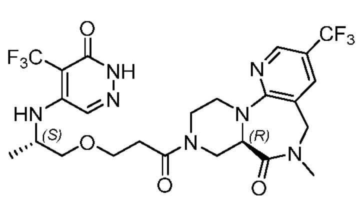
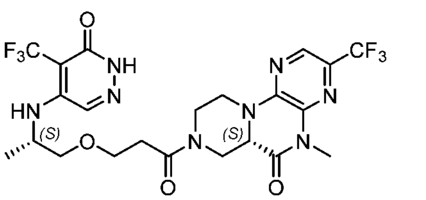
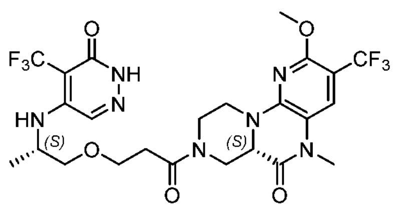

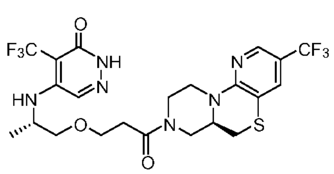
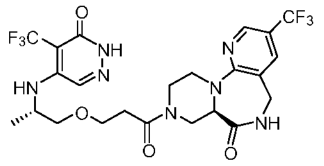
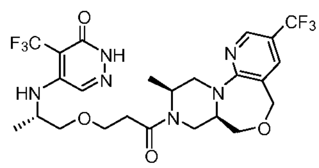

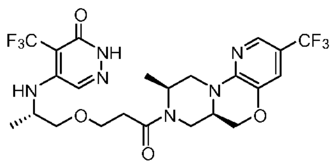
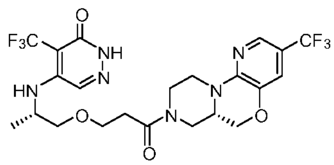

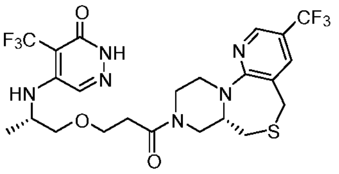
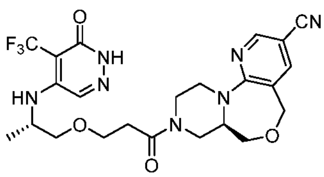
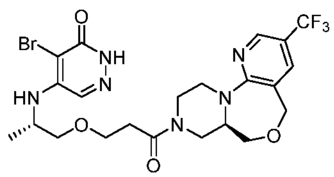

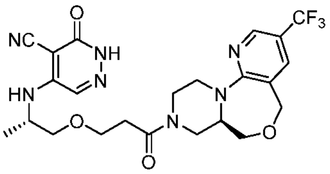
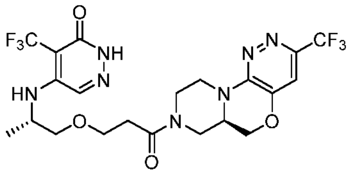
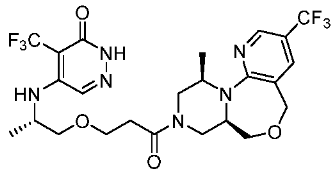

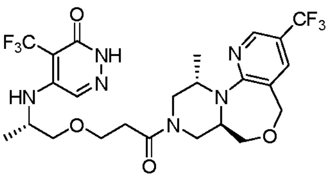
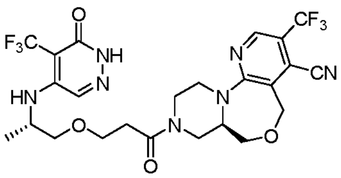
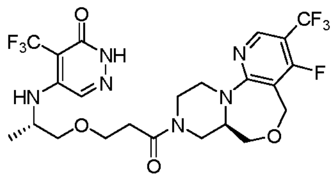

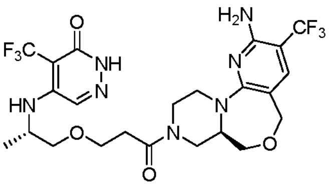
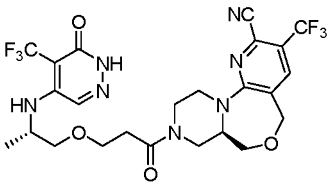
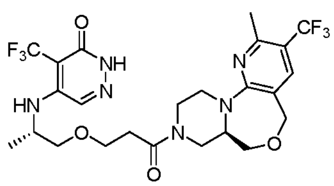

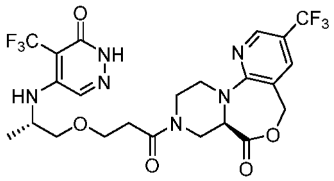
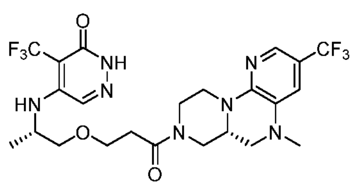

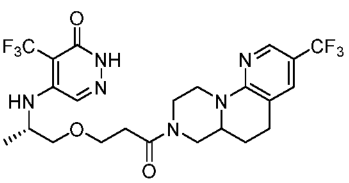
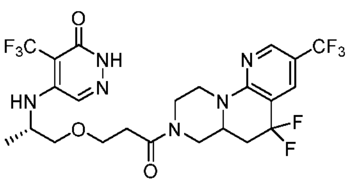
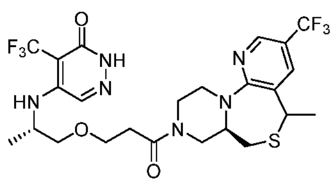

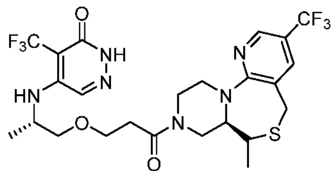
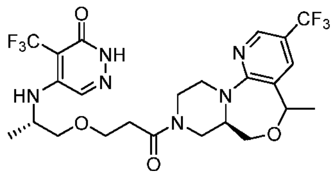
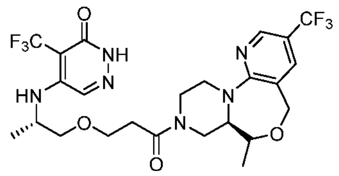

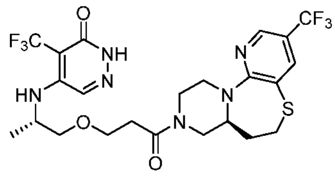
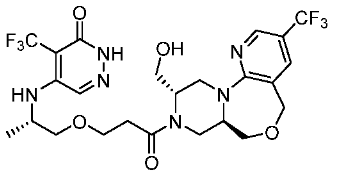
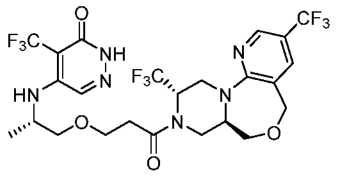

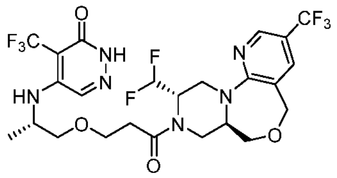
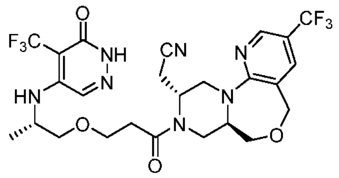
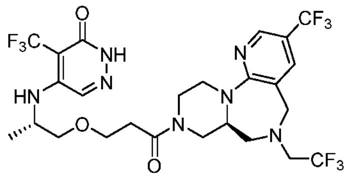

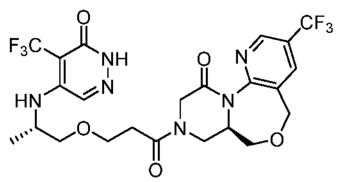
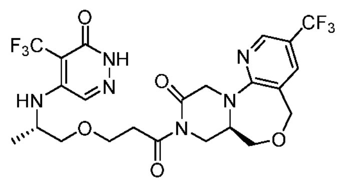
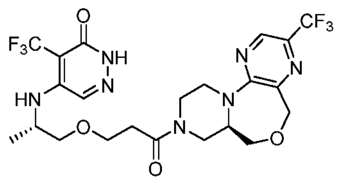

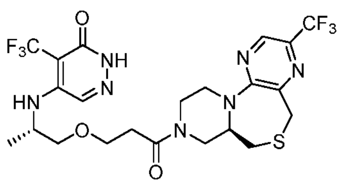
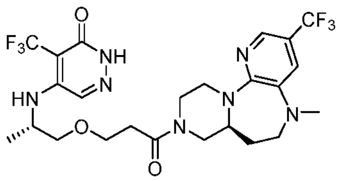
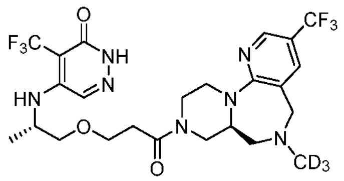

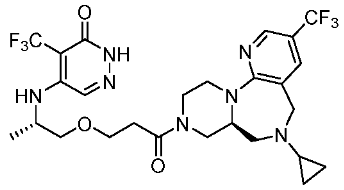

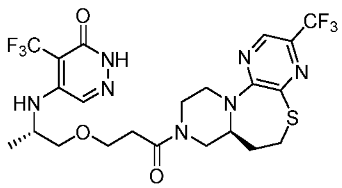
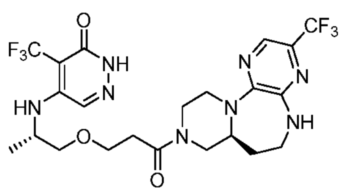
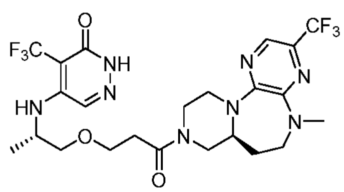

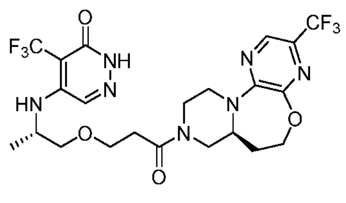
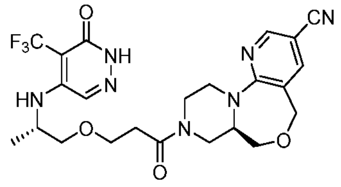
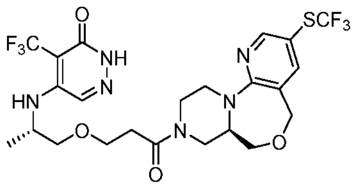

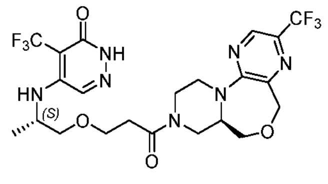
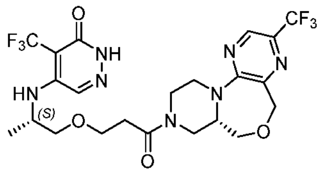
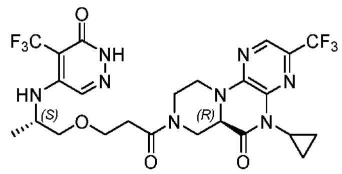

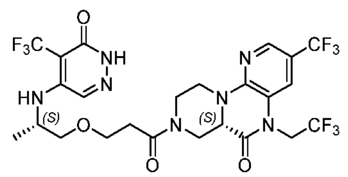
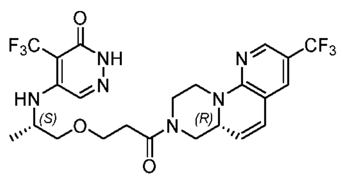
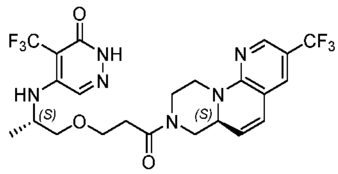

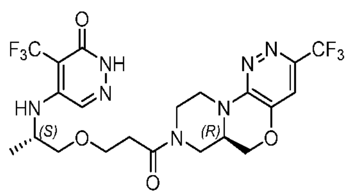
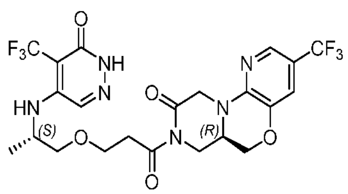
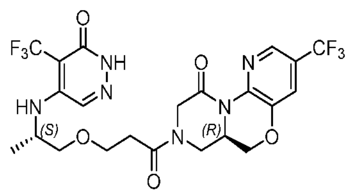

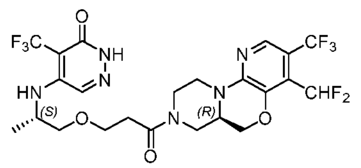
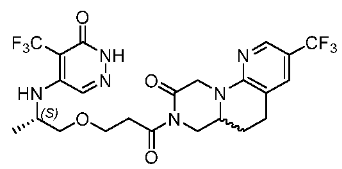

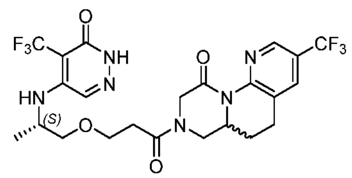
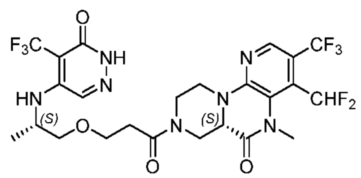
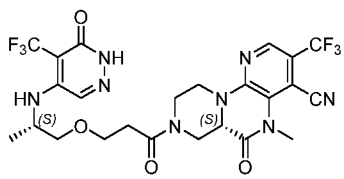

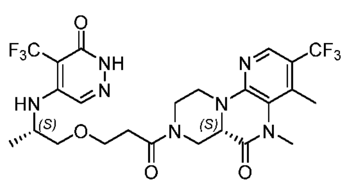
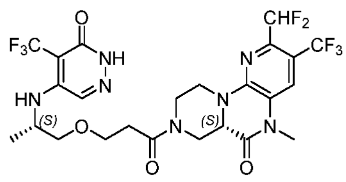

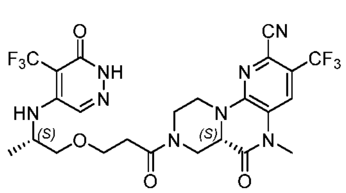
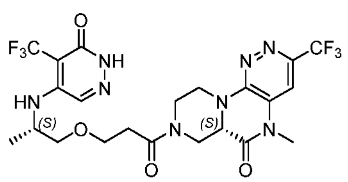
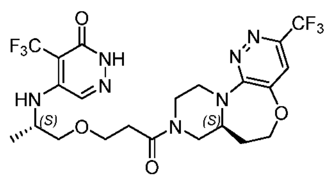

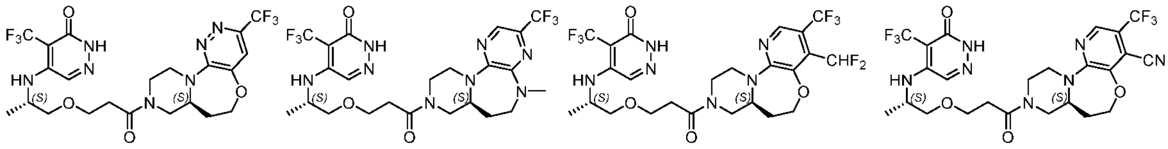

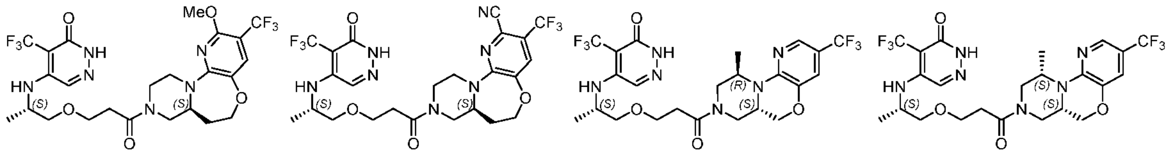

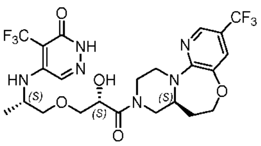

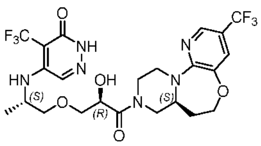

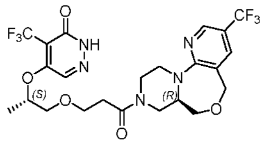

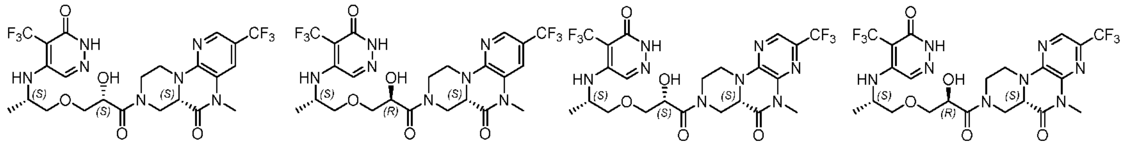

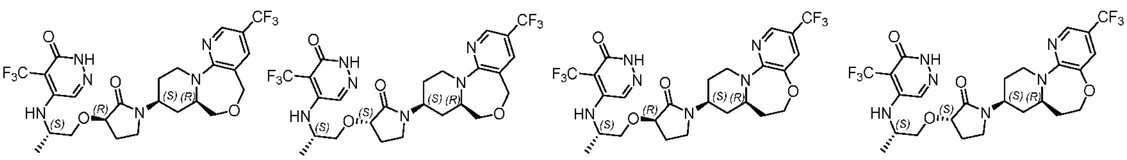

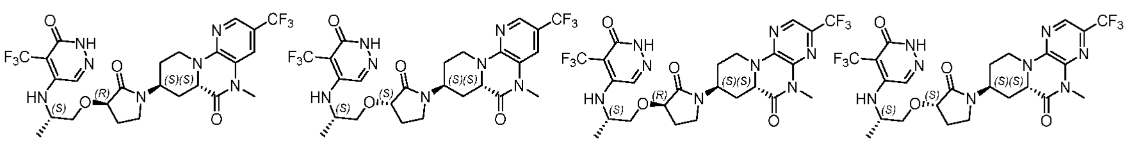

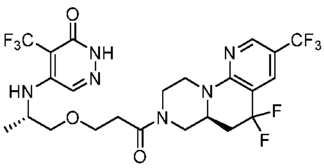

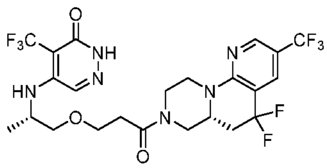

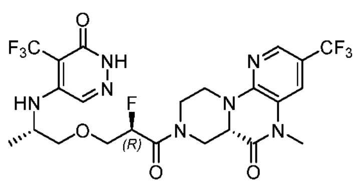

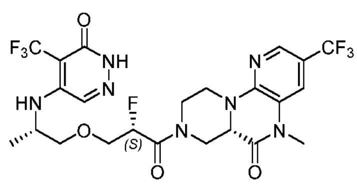

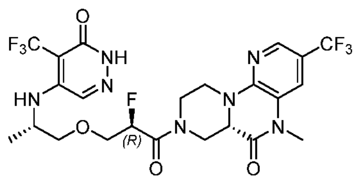

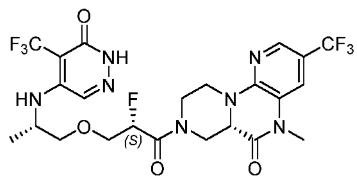

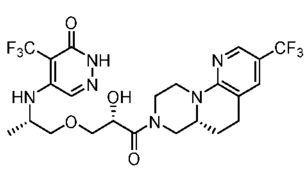
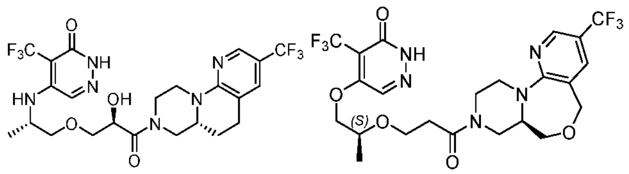

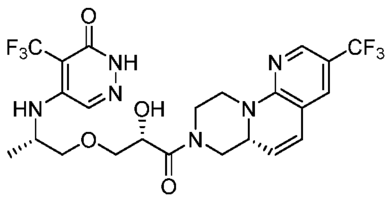
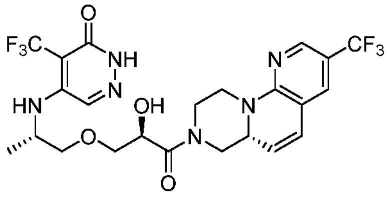
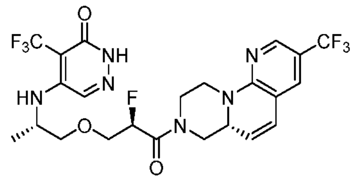

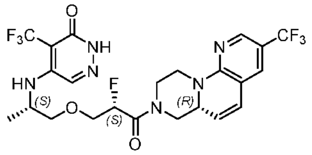
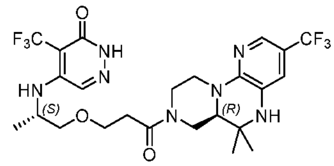

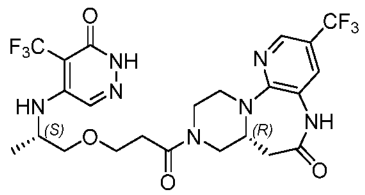
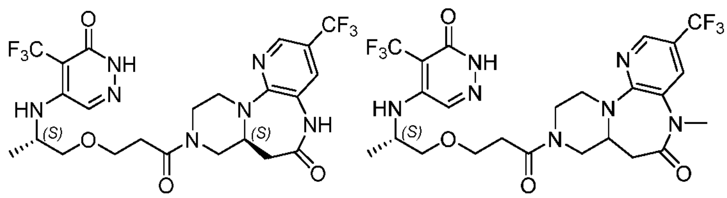
,

in which "~"bond represents a combination of R configuration and S configuration.

Therefore, throughout this specification, the groups and their substituents in the fused triheterocyclic compound of Formula IA or a pharmaceutically acceptable salt thereof can be selected by those skilled in the art, to provide a stable fused triheterocyclic compound of Formula IA or a pharmaceutically acceptable salt thereof, including, but not limited to, the compounds in the examples of the present invention.

The fused triheterocyclic compound of Formula IA or a pharmaceutically acceptable salt thereof of the present invention can be synthesized according to a method similar to those well-known in the chemical field, in which the steps and conditions can refer to those for similar reactions in this field, and particularly according to the description herein. The starting materials are usually commercially available, for example, from Aldrich; or can be easily prepared through methods known to those skilled in the art (obtained from SciFinder, Reaxys online database).

In the present invention, the fused triheterocyclic compound of Formula IA can also be obtained from the prepared fused triheterocyclic compound of Formula I', by side modification using the conventional method in the field to obtain other fused triheterocyclic compound of Formula IA.

Generally, the compound of the present invention can be prepared by the method described in the present invention, where the definitions of substituents are as described for those in Formula I, unless otherwise specified. The following reaction schemes and examples are used to further illustrate the present invention by way of examples.

In the present invention, a method for preparing the fused triheterocyclic compound of Formula IA comprises the following steps:

subjecting Compound of Formula IIA and Compound of Formula IIIA to an amidation reaction in a solvent in the presence of a base and a condensing agent, to obtain a fused triheterocyclic compound of Formula IA:

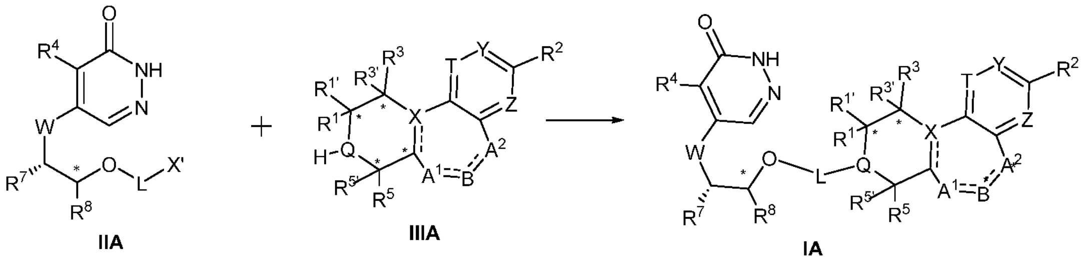

IIA IIIA IA

where W, L, $R^7$, $R^8$, $R^{1'}$, $R^{1'}$, $R^2$, $R^3$, $R^{3'}$, $R^4$, $R^5$, $R^{5'}$, X, T, Y, Z, $A^1$, B, $A^2$, Q, ---- and * are as defined in any of the above embodiments; and
X' is a leaving group, for example, halo (for example, Cl) or OH.

For example, when the fused triheterocyclic compound of Formula IA is a fused triheterocyclic compound of Formula I', the preparation method comprises the following steps:

subjecting Compound of Formula II' and Compound of Formula III' to an amidation reaction in a solvent in the presence of a base and a condensing agent, to obtain a fused triheterocyclic compound of Formula I' :

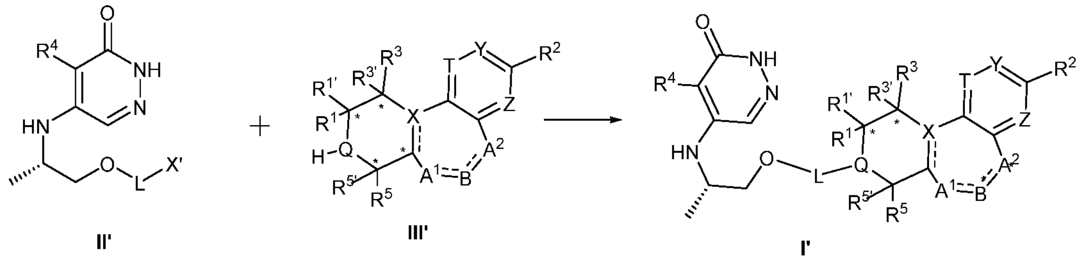

II' III' I'

where L, $R^1$, $R^{1'}$, $R^3$, $R^{3'}$, $R^5$, $R^{5'}$, $R^2$, $R^4$, X, T, Y, Z, $A^1$, B, $A^2$, Q, = and * are as defined above; and X' is a leaving group, for example, halo (for example, Cl) or OH; and
for example,

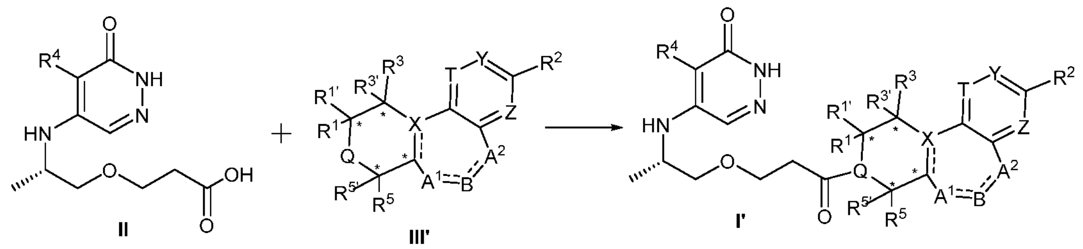

II III' I'

$R^1$, $R^{1'}$, $R^3$, $R^{3'}$, $R^5$, $R^{5'}$, $R^2$, $R^4$, X, T, Y, Z, $A^1$, B, $A^2$, Q, = and * are as defined above.
The conditions and operations of the amidation reaction can be conventional conditions and operations in this type of reactions in the art. In the present invention, preferably:
the solvent is DMF or dichloromethane (DCM). The base is diisopropylethyl amine or $K_2CO_3$. The molar ratio of the base to Compound of Formula II is 1:1. The condensing agent is propylphosphonic anhydride T3P, for example, as a 50% solution in ethyl acetate. The molar ratio of the condensing agent to Compound of Formula II is 1:1.
The molar ratio of Compound of Formula IIA to Compound of Formula IIIA is 1:1. The amidation reaction is carried out under a nitrogen atmosphere. The reaction temperature of the amidation reaction is from 0°C to 60°C.
Essential raw materials or reagents for preparing Compound of Formula IA are commercially available, can be prepared by synthesis methods known in the art. According to the method described in the experimental section below, the compound of the present invention can be prepared in the form of a free base or an acid addition salt thereof. The term pharmaceutically acceptable salt refers to a pharmaceutically acceptable salt defined herein having all pharmaceutical activities of the parent compound. The pharmaceutically acceptable salt can be prepared according to a conventional method by adding a corresponding acid to the organic base in an appropriate organic solvent.
Examples of the acid addition salt include: salts with inorganic acids, for example, hydrochloric acid, hydrobromic

acid, sulfuric acid, nitric acid and phosphoric acid; and salts with organic acids, such as acetic acid, benzenesulfonic acid, benzoic acid, camphorsulfonic acid, citric acid, ethanesulfonic acid, fumaric acid, glucuronic acid, glutamic acid, glycolic acid, hydronaphthoic acid, 2-hydroxyethanesulfonic acid, lactic acid, maleic acid, malic acid, malonic acid, mandelic acid, methanesulfonic acid, 2-naphthalenesulfonic acid, propionic acid, salicylic acid, succinic acid, tartaric acid, p-toluenesulfonic acid or trimethylacetic acid.

The fused triheterocyclic compound of Formula IA may have one or more chiral carbon atoms, so optically pure isomers, for example, pure enantiomer or racemate, or mixed isomers can be separated. Pure individual isomers can be obtained by separation methods known in this art, for example, chiral crystallization into a salt, or separation by a chiral preparative column.

The chemicals used in the synthesis route mentioned in this patent include solvents, reagents, catalysts, protective groups, deprotection groups and protecting groups. The method may additionally include a step before or after the step specifically described herein, and an appropriate protective group may be added or removed to obtain the target compound. In addition, various synthesis steps can be alternately or sequentially carried out to obtain the final target product.

The present invention provides a pharmaceutical composition, which comprises a fused triheterocyclic compound of Formula IA or a pharmaceutically acceptable salt thereof, and (one or more) pharmaceutically acceptable adjuvants. In the pharmaceutical composition, the amount of the fused triheterocyclic compound of Formula IA or a pharmaceutically acceptable salt thereof is a therapeutically effective amount.

The present invention further provides use of the fused triheterocyclic compound of Formula IA or a pharmaceutically acceptable salt thereof in the preparation of PARP inhibitors. The PARP may be PARP7. In the use, the PARP inhibitor can be used in vivo in mammalian, or in vitro, mainly for experimental purposes. For example, it is used as a standard or a reference for comparison, or prepared into a kit according to a conventional method in the art to provide rapid detection for the inhibitory effect on PARP.

The present invention also provides use of the fused triheterocyclic compound of Formula IA or a pharmaceutically acceptable salt thereof in the preparation of drugs for preventing and/or treating PARP-related diseases or PARP-mediated diseases. The PARP may be PARP7. The PARP-related or PARP-mediated diseases may include abnormal proliferative diseases or other diseases related to the target to which this series of compounds target and bind. The abnormal proliferative diseases include, but are not limited to, cancers, such as lung cancer (for example, squamous cell lung tumor, and H1373 lung cancer line), colon cancer, colorectal cancer, pancreatic cancer, renal cancer, gastric cancer, esophageal cancer, ovarian cancer, breast cancer, cervical cancer, head and neck cancer (upper respiratory and digestive tract), bladder cancer, melanoma, fibrosarcoma and glioblastoma. The amount of the fused triheterocyclic compound of Formula IA or a pharmaceutically acceptable salt thereof can be a therapeutically effective amount.

The present invention further provides use of the fused triheterocyclic compound of Formula IA or a pharmaceutically acceptable salt thereof or a pharmaceutical composition comprising the fused triheterocyclic compound of Formula IA or a pharmaceutically acceptable salt thereof in the preparation of drugs for preventing and/or treating abnormal proliferative diseases. The abnormal proliferative diseases include, but are not limited to, cancers, such as lung cancer (for example, squamous cell lung tumor, and H1373 lung cancer line), colon cancer, colorectal cancer, pancreatic cancer, renal cancer, gastric cancer, esophageal cancer, ovarian cancer, breast cancer, cervical cancer, head and neck cancer (upper respiratory and digestive tract), bladder cancer, melanoma, fibrosarcoma and glioblastoma. The amount of the fused triheterocyclic compound of Formula IA or a pharmaceutically acceptable salt thereof can be a therapeutically effective amount.

Another aspect of the present invention relates to a method for preventing and/or treating PARP7-related or PARP-mediated diseases, which comprises: administering, to a patient, a therapeutically effective amount of the fused triheterocyclic compound of Formula IA or a pharmaceutically acceptable salt thereof, or a pharmaceutical composition comprising the fused triheterocyclic compound of Formula IA or a pharmaceutically acceptable salt thereof.

Another aspect of the present invention relates to a method for therapeutically preventing and/or treating abnormal proliferative diseases, which comprises: administering, to a patient, a therapeutically effective amount of the fused triheterocyclic compound of Formula IA or a pharmaceutically acceptable salt thereof, or a pharmaceutical composition comprising the fused triheterocyclic compound of Formula IA or a pharmaceutically acceptable salt thereof. The abnormal proliferative diseases include, but are not limited to, cancers, such as lung cancer (for example, squamous cell lung tumor, and H1373 lung cancer line), colon cancer, colorectal cancer, pancreatic cancer, renal cancer, gastric cancer, esophageal cancer, ovarian cancer, breast cancer, cervical cancer, head and neck cancer (upper respiratory and digestive tract), bladder cancer, melanoma, fibrosarcoma and glioblastoma.

Another aspect of the present invention relates to a drug for inhibiting PARP7, which comprises the fused triheterocyclic compound of Formula IA or a pharmaceutically acceptable salt thereof, or a pharmaceutical composition comprising the fused triheterocyclic compound of Formula IA or a pharmaceutically acceptable salt thereof.

The present invention provides a fused triheterocyclic compound of Formula I or a pharmaceutically acceptable salt thereof,

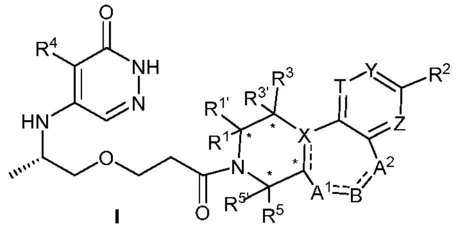

I

where $R^1$, $R^{1'}$, $R^3$, $R^{3'}$, $R^5$, and $R^{5'}$ are independently H, $C_{1-6}$ alkyl or $C_{1-6}$ alkyl substituted with one or more $R^{1a}$; or $R^1$ and $R^{1'}$, $R^3$ and $R^{3'}$, and $R^5$ and $R^{5'}$ are independently taken together to form O=, where when more than one substituent is present, they are the same or different, and
$R^{1a}$ is independently halo, CN, or OH;
$R^2$ and $R^4$ are independently halo, CN, $C_{1-6}$ alkyl, $C_{1-6}$ alkyl substituted with one or more $R^{2a}$, or $C_{1-6}$ alkyl-S-substituted with one or more $R^{2b}$, where when more than one substituent is present, they are the same or different, and
$R^{2a}$ and $R^{2b}$ are independently halo;
X is independently $C(R^{3a})$, C (when - - - - is a double bond, that is,

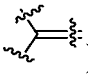

) or N;
T is independently $C(R^{3b})$ or N; Y is independently $C(R^{3c})$ or N; and Z is independently $C(R^{3d})$ or N;
in which $R^{3a}$, $R^{3b}$, $R^{3c}$, and $R^{3d}$ are independently H, halo, CN, $NH_2$ or $C_{1-6}$ alkyl;
$A^1$ is independently $C(R^{4a})$ (when - - - - is a double bond), $C(R^{4b}R^{4c})$ (when - - - - is a single bond) or C(=O), where $R^{4a}$, $R^{4b}$, and $R^{4c}$ are independently H, $C_{1-6}$ alkyl, or $C_{1-6}$ alkyl substituted with one or more $R^{a1}$, where when more than one substituent is present, they are the same or different;
when B is independently $C(R^{6a})$, or $C(R^{6b}R^{6c})$, $A^2$ is independently $C(R^{5a})$, $C(R^{5b}R^{5c})$, O, S, $N(R^{5d})$, N or a linkage;
when B is independently O, S, N or $N(R^{6d})$, $A^2$ is independently $C(R^{5a})$, $C(R^{5b}R^{5c})$ or a linkage;
in which $R^{5a}$, $R^{5b}$, $R^{5c}$, and $R^{5d}$ are independently H, $C_{1-6}$ alkyl or $C_{1-6}$ alkyl substituted with one or more $R^{a2}$;
$R^{6a}$, $R^{6b}$, and $R^{6c}$ are independently H, halo, $C_{1-6}$ alkyl, $C_{1-6}$ alkyl substituted with one or more $R^{a3}$, or $C_{3-6}$ cycloalkyl;
$R^{6d}$ is independently H, $C_{1-6}$ alkyl, $C_{1-6}$ alkyl substituted with one or more $R^{a3}$, or $C_{3-6}$ cycloalkyl, where when more than one substituent is present, they are the same or different; and
$R^{a1}$, $R^{a2}$, and $R^{a3}$ are independently deuterium, or halo; and
- - - - represents a double bond or a single bond;* indicates a chiral carbon atom that is in R configuration, S configuration or a combination thereof.

In the present invention, in the fused triheterocyclic compound of Formula I, some substituents can be defined as described below, and substituents not mentioned can be defined as described in any of the above embodiments.

In a certain embodiment of the present invention, one of $R^1$ and $R^{1'}$ is H, and the other one is H, $C_{1-6}$ alkyl, or $C_{1-6}$ alkyl substituted with one or more $R^{1a}$.

For example, one of $R^1$ and $R^{1'}$ is H, and the other one is H or $C_{1-6}$ alkyl.

Alternatively, $R^1$ and $R^{1'}$ are taken together to form O=.

Preferably, $R^1$, and $R^{1'}$ are H.

In a certain embodiment of the present invention, one of $R^3$ and $R^{3'}$ is H, and the other one is H, $C_{1-6}$ alkyl, or $C_{1-6}$ alkyl substituted with one or more $R^{1a}$.

For example, one of $R^3$ and $R^{3'}$ is H, and the other one is H or $C_{1-6}$ alkyl.

Alternatively, $R^3$ and $R^{3'}$ are taken together to form O=.

Preferably, $R^3$, and $R^{3'}$ are H.

In a certain embodiment of the present invention, one of $R^5$ and $R^{5'}$ is H, and the other one is H, $C_{1-6}$ alkyl, or $C_{1-6}$ alkyl substituted with one or more $R^{1a}$.

For example, one of $R^5$ and $R^{5'}$ is H, and the other one is H or $C_{1-6}$ alkyl.

Alternatively, $R^5$ and $R^{5'}$ are taken together to form O=.

Preferably, $R^5$, and $R^{5'}$ are H.

In a certain embodiment of the present invention, one of $R^1$, $R^{1'}$, $R^3$, $R^{3'}$, $R^5$, and $R^{5'}$ is H, $C_{1-6}$ alkyl, or $C_{1-6}$ alkyl substituted with one or more $R^{1a}$, and the others are H.

For example, one of $R^1$, $R^{1'}$, $R^3$, $R^{3'}$, $R^5$, and $R^{5'}$ is H or $C_{1-6}$ alkyl, and the others are H.

Alternatively, one of $R^1$ and $R^{1'}$, $R^3$ and $R^{3'}$, $R^5$ and $R^{5'}$ is O=, and the others are H.

Preferably, $R^1$, $R^1$, $R^3$, $R^{3'}$, $R^5$, and $R^{5'}$ are H.

In a certain embodiment of the present invention, when the carbon to which $R^1$ and $R^{1'}$ is attached is a chiral carbon, it is in S configuration or R configuration,
for example, R configuration.

In a certain embodiment of the present invention, when the carbon to which $R^3$ and $R^{3'}$ is attached is a chiral carbon, it is in S configuration or R configuration.

In a certain embodiment of the present invention, when the carbon to which $R^5$ and $R^{5'}$ is attached is a chiral carbon, it is in S configuration or R configuration.

In a certain embodiment of the present invention, $R^2$ is independently halo, CN, $C_{1-6}$ alkyl substituted with one or more $R^{2a}$, or $C_{1-6}$ alkyl-S- substituted with one or more $R^{2b}$,

for example, halo, or $C_{1-6}$ alkyl substituted with one or more $R^{2a}$, and
for example, $C_{1-6}$ alkyl substituted with one or more halo.

In a certain embodiment of the present invention, $R^4$ is independently halo, CN, or $C_{1-6}$ alkyl substituted with one or more $R^{2a}$,

for example, halo, or $C_{1-6}$ alkyl substituted with one or more $R^{2a}$, and
for example, $C_{1-6}$ alkyl substituted with one or more halo.

In a certain embodiment of the present invention, $R^2$ and $R^4$ are independently $C_{1-6}$ alkyl substituted with one or more $R^{2a}$,
for example, $-CF_3$.

In a certain embodiment of the present invention, X is independently N.

In a certain embodiment of the present invention, T is independently N.

In a certain embodiment of the present invention, $R^{3c}$ is H, CN, $NH_2$ or $C_{1-6}$ alkyl,
for example, H.

In a certain embodiment of the present invention, Y is independently $C(R^{3c})$,
for example, CH.

In a certain embodiment of the present invention, $R^{3d}$ is H, halo, CN or $C_{1-6}$ alkyl,
for example, H.

In a certain embodiment of the present invention, Z is independently $C(R^{3d})$,
for example, CH.

In a certain embodiment of the present invention, $R^{4a}$, $R^{4b}$, and $R^{4c}$ are independently H or $C_{1-6}$ alkyl,

for example, one of $R^{4b}$ and $R^{4c}$ is H, and the other one is H or $C_{1-6}$ alkyl, and
for example, $R^{4b}$ and $R^{4c}$ are H.

In a certain embodiment of the present invention, when $A^1$ is $C(R^{4b}R^{4c})$ and the carbon is a chiral carbon, it is in S configuration, R configuration or a combination thereof.

In a certain embodiment of the present invention, $A^1$ is independently $C(R^{4b}R^{4c})$ or C(=O), for example, $C(R^{4b}R^{4c})$.

In a certain embodiment of the present invention, $R^{5a}$, $R^{5b}$, $R^{5c}$, and $R^{5d}$ are independently H or $C_{1-6}$ alkyl,

for example, one of $R^{5b}$ and $R^{5c}$ is H, and the other one is H or $C_{1-6}$ alkyl, and
for example, $R^{5b}$ and $R^{5c}$ are H.

In a certain embodiment of the present invention, when $A^2$ is $C(R^{5b}R^{5c})$ and the carbon is a chiral carbon, it is in S configuration, R configuration or a combination thereof.

In a certain embodiment of the present invention, $A^2$ is independently $C(R^{5b}R^{5c})$ or a linkage.

In a certain embodiment of the present invention, $R^{6a}$, $R^{6b}$, and $R^{6c}$ are independently H or halo,
for example, $R^{6b}$ and $R^{6c}$ are independently H.

In a certain embodiment of the present invention, $R^{6d}$ is independently H, $C_{1-6}$ alkyl, $C_{1-6}$ alkyl substituted with one or more $R^3$, or $C_{3-6}$ cycloalkyl,
for example, H or $C_{1-6}$ alkyl.

In a certain embodiment of the present invention, B is independently $C(R^{6b}R^{6c})$.

In a certain embodiment of the present invention, B is independently O, S or $N(R^{6d})$,

for example, -O- or -S-,
for example, S.

In a certain embodiment of the present invention,

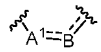

is independently - $C(R^{4b}R^{4c})$-O-, -$C(R^{4b}R^{4c})$-S-, -$C(R^{4b}R^{4c})$-N($R^{6d}$)-, -$C(R^{4b}R^{4c})$-$C(R^{6b}R^{6c})$-, -C(=O)-O-, or-C(=O)-N($R^{6d}$)-;

for example, -$CH_2$-O-, -$CH_2$-S-, -$CH_2$-NH-, -$CH_2$-N($CH_3$)-, -$CH_2$-N($CH_2CF_3$)-, -$CH_2$-$CH_2$-, -$CH_2$-$CF_2$-, -C($CH_3$)-O-, -C($CH_3$)-S-, -C(=O)-NH-, -C(=O)-O-, -C(=O)-N($CD_3$)-, -$CH_2$-N(cyclopropyl)-, or -C(=O)-N(cyclopropyl)-;
for example, -$C(R^{4b}R^{4c})$-O-, -$C(R^{4b}R^{4c})$-S-, -$C(R^{4b}R^{4c})$-N($R^{6d}$)-, or -C(=O)-N($R^{6d}$)-; and
for example, -$CH_2$-O-, or -$CH_2$-S-.

In a certain embodiment of the present invention,

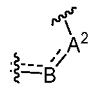

is independently -O-, -S-, - $C(R^{6b}R^{6c})$-, -N($R^{6d}$)-, -O-$C(R^{5b}R^{5c})$-, -S-$C(R^{5b}R^{5c})$-, -N($R^{6d}$)-$C(R^{5b}R^{5c})$-, -$C(R^{6b}R^{6c})$-O-, -$C(R^{6b}R^{6c})$-S-, or -$C(R^{6b}R^{6c})$-N($R^{5d}$)-;

for example, -O-, -O-$C(R^{5b}R^{5c})$-, -S-$C(R^{5b}R^{5c})$-, -N($R^{6d}$)-, or -N($R^{6d}$)-$C(R^{5b}R^{5c})$-; and
for example, -O-, -O-$CH_2$- or -S-$CH_2$-.

In a certain embodiment of the present invention,

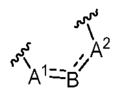

is independently

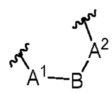

or

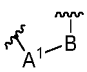
.

In a certain embodiment of the present invention,

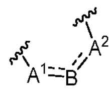

is independently - $C(R^{4b}R^{4c})$-O-, -$C(R^{4b}R^{4c})$-S-, -$C(R^{4b}R^{4c})$-N($R^{6d}$)-, -C(=O)-N($R^{6d}$)-, -$C(R^{4b}R^{4c})$-O-$C(R^{5b}R^{5c})$-,-$C(R^{4b}R^{4c})$-S-$C(R^{5b}R^{5c})$-, -$C(R^{4b}R^{4c})$-N($R^{6d}$)-$C(R^{5b}R^{5c})$-, -$C(R^{4b}R^{4c})$-$C(R^{6b}R^{6c})$-N($R^{5d}$)-, -$C(R^{4b}R^{4c})$-$C(R^{6b}R^{6c})$-O-, -$C(R^{4b}R^{4c})$-$C(R^{6b}R^{6c})$-S-, -C(=O)-N($R^{6d}$)-$C(R^{5b}R^{5c})$-, -C(=O)-O-$C(R^{5b}R^{5c})$-, or -$C(R^{4b}R^{4c})$-$C(R^{6b}R^{6c})$-;

for example, -$C(R^{4b}R^{4c})$-O-, -$C(R^{4b}R^{4c})$-S-, -$C(R^{4b}R^{4c})$-N($R^{6d}$)-, -C(=O)-N($R^{6d}$)-, - $C(R^{4b}R^{4c})$-O-$C(R^{5b}R^{5c})$-,

-C($R^{4b}R^{4c}$)-S-C($R^{5b}R^{5c}$)-, -C($R^{4b}R^{4c}$)-N($R^{6d}$)-C($R^{5b}R^{5c}$)-;
for example, -C($R^{4b}R^{4c}$)-O-, -C($R^{4b}R^{4c}$)-S-, -C($R^{4b}R^{4c}$)-O-C($R^{5b}R^{5c}$)-, -C($R^{4b}R^{4c}$)-S-C($R^{5b}R^{5c}$)-; and
for example, -$CH_2$-O-, -$CH_2$-S-, -$CH_2$-O-$CH_2$-, -$CH_2$-S-$CH_2$-.

In a certain embodiment of the present invention,

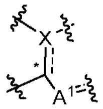

is independently

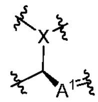

or

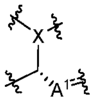.

In a certain embodiment of the present invention, when $R^1$, $R^{1'}$, $R^3$, $R^{3'}$, $R^5$, and $R^{5'}$ are independently $C_{1-6}$ alkyl or $C_{1-6}$ alkyl substituted with one or more $R^{1a}$, the $C_{1-6}$ alkyl or $C_{1-6}$ alkyl in the $C_{1-6}$ alkyl substituted with one or more $R^{1a}$ is $C_1$-$C_4$ alkyl,

for example, methyl, ethyl, n-propyl, isopropyl, n-butyl, isobutyl, s-butyl or t-butyl; and
for example, methyl.

In a certain embodiment of the present invention, when $R^{1a}$ is halo, the halo is F, Cl, Br, or I,
for example, F.
In a certain embodiment of the present invention, when $R^2$ and $R^4$ are independently halo, the halo is F, Cl, Br, or I,
for example, F.
In a certain embodiment of the present invention, when $R^2$ and $R^4$ are independently $C_{1-6}$ alkyl, $C_{1-6}$ alkyl substituted with one or more $R^{2a}$, or $C_{1-6}$ alkyl-S- substituted with one or more $R^{2b}$, the $C_{1-6}$ alkyl, and $C_{1-6}$ alkyl in the $C_{1-6}$ alkyl substituted with one or more $R^{2a}$ and in the $C_{1-6}$ alkyl-S- substituted with one or more $R^{2b}$ are $C_1$-$C_4$ alkyl,

for example, methyl, ethyl, n-propyl, isopropyl, n-butyl, isobutyl, s-butyl or t-butyl; and
for example, methyl.

In a certain embodiment of the present invention, when $R^2$ and $R^4$ are independently $C_{1-6}$ alkyl substituted with one or more $R^{2a}$, or $C_{1-6}$ alkyl-S- substituted with one or more $R^{2b}$, the number of the substituent is 1, 2, or 3,
for example, 3.
In a certain embodiment of the present invention, when $R^{2a}$ and $R^{2b}$ are independently halo, the halo is F, Cl, Br, or I,
for example, F.
In a certain embodiment of the present invention, when $R^{3a}$, $R^{3b}$, $R^{3c}$, and $R^{3d}$ are independently halo, the halo is F, Cl, Br, or I,
for example, F.
In a certain embodiment of the present invention, when $R^{3a}$, $R^{3b}$, $R^{3c}$, and $R^{3d}$ are independently $C_{1-6}$ alkyl, the $C_{1-6}$ alkyl is $C_1$-$C_4$ alkyl,

for example, methyl, ethyl, n-propyl, isopropyl, n-butyl, isobutyl, s-butyl or t-butyl; and
for example, methyl.

In a certain embodiment of the present invention, when $R^{4a}$, $R^{4b}$, and $R^{4c}$ are independently $C_{1-6}$ alkyl or $C_{1-6}$ alkyl substituted with one or more $R^{a1}$, the $C_{1-6}$ alkyl and $C_{1-6}$ alkyl in the $C_{1-6}$ alkyl substituted with one or more $R^{a1}$ is $C_1$-$C_4$ alkyl,

for example, methyl, ethyl, n-propyl, isopropyl, n-butyl, isobutyl, s-butyl or t-butyl; and

for example, methyl.

In a certain embodiment of the present invention, when $R^{5a}$, $R^{5b}$, $R^{5c}$, and $R^{5d}$ are independently $C_{1-6}$ alkyl or $C_{1-6}$ alkyl substituted with one or more $R^{a2}$, the $C_{1-6}$ alkyl and $C_{1-6}$ alkyl in the $C_{1-6}$ alkyl substituted with one or more $R^{a2}$ is $C_1$-$C_4$ alkyl,

for example, methyl, ethyl, n-propyl, isopropyl, n-butyl, isobutyl, s-butyl or t-butyl; and
for example, methyl.

In a certain embodiment of the present invention, when $R^{6a}$, $R^{6b}$, and $R^{6c}$ are independently halo, the halo is F, Cl, Br, or I,
for example, F.

In a certain embodiment of the present invention, when $R^{6a}$, $R^{6b}$, $R^{6c}$, and $R^{6d}$ are independently $C_{1-6}$ alkyl or $C_{1-6}$ alkyl substituted with one or more $R^{a3}$, the $C_{1-6}$ alkyl and $C_{1-6}$ alkyl in the $C_{1-6}$ alkyl substituted with one or more $R^{a3}$ is $C_1$-$C_4$ alkyl,

for example, methyl, ethyl, n-propyl, isopropyl, n-butyl, isobutyl, s-butyl or t-butyl; and
for example, methyl.

In a certain embodiment of the present invention, when $R^{6a}$, $R^{6b}$, $R^{6c}$, and $R^{6d}$ are independently $C_{3-6}$ cycloalkyl, the $C_{3-6}$ cycloalkyl is cyclopropyl, cyclobutyl, cyclopentyl, or cyclohexyl,
for example, cyclopropyl.

In a certain embodiment of the present invention, when $R^{a1}$, $R^{a2}$, and $R^{a3}$ are independently halo, the halo is F, Cl, Br, or I,
for example, F.

In a certain embodiment of the present invention, $R^1$, $R^{1'}$, $R^3$, $R^{3'}$, $R^5$, and $R^{5'}$ are independently H, methyl, HO-methyl, NC-methyl, $CHF_2$, or $CF_3$; or $R^1$ and $R^{1'}$, $R^3$ and $R^{3'}$, and $R^5$ and $R^{5'}$ are independently taken together to form O=.

In a certain embodiment of the present invention, $R^2$ and $R^4$ are independently fluoro, bromo, chloro, CN, $CF_3$, or $CF_3$-S-.

In a certain embodiment of the present invention, Y is independently CH, N, C(CN), or $C(NH_2)$, for example, CH, or N.

In a certain embodiment of the present invention, Z is independently CH, N, C(CN), or $C(NH_2)$, for example, CH or N.

In a certain embodiment of the present invention, $A^1$ is independently $CH_2$, $C(CH_3)$ or C(=O).

In a certain embodiment of the present invention, B is independently $CH_2$, $CF_2$, O, S, NH, $N(CH_3)$, $N(CD_3)$, N (cyclopropyl), N $(CH_2CF_3)$, $C(CH_3)$ or C(=O).

In a certain embodiment of the present invention, $A^2$ is independently $CH_2$, O, S, NH, $N(CH_3)$, N (cyclopropyl), $CH(CH_3)$ or a linkage.

In a certain embodiment of the present invention,

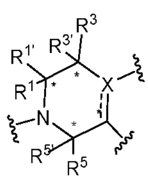

is independently

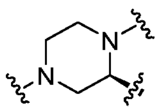, 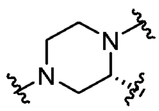, 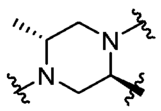, 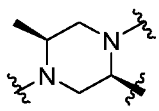, 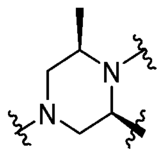, 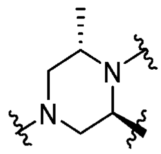,

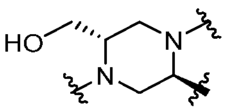, 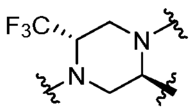, 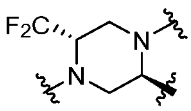, 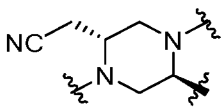, 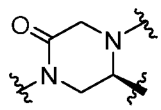,

or

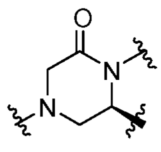,

for example,

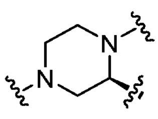, 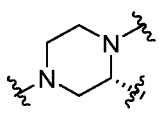, 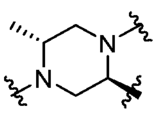, or 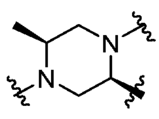.

In a certain embodiment of the present invention,

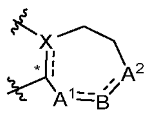

is independently

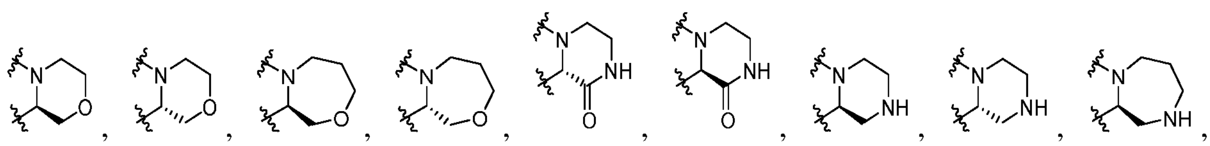
, , , , , , , , ,

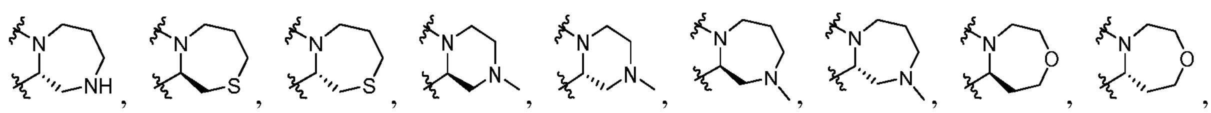
, , , , , , , , ,

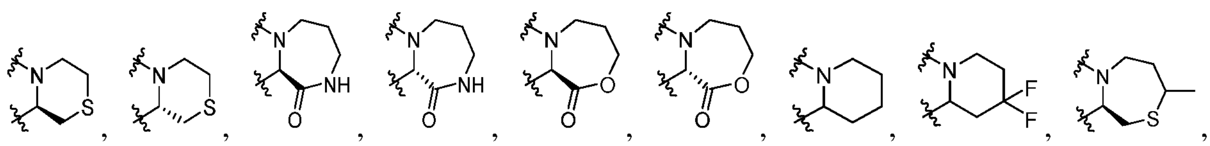
, , , , , , , , ,

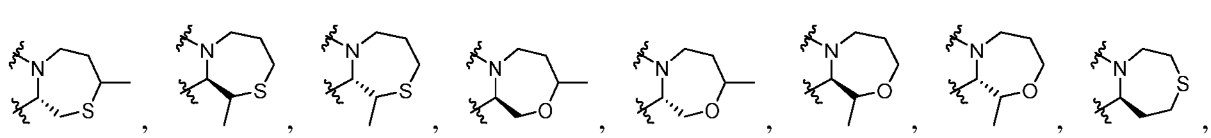
, , , , , , , ,

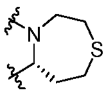, 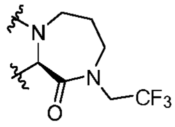, 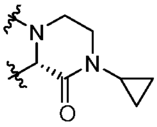, 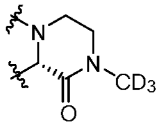, 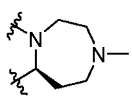, 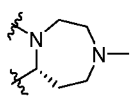, 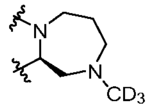,

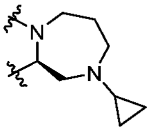, 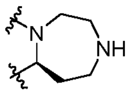, 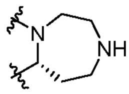, 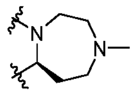, or 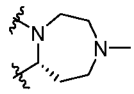;

for example,

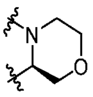, 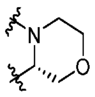, 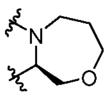, 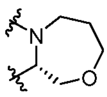, 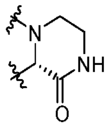, 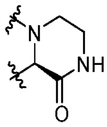, 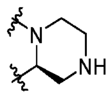,

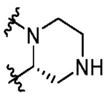, 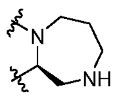, 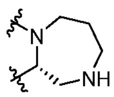, 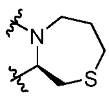, 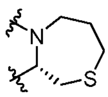, 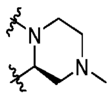, 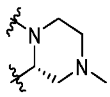, 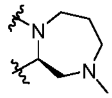, or 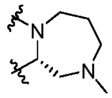;

for example,

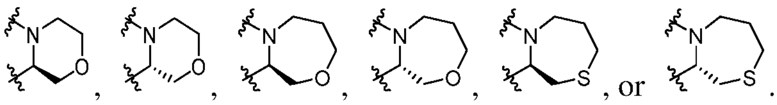
, , , , , or .

In a certain embodiment of the present invention,

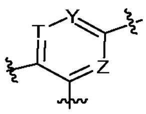

is independently

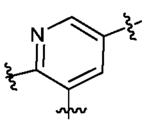, 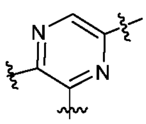, 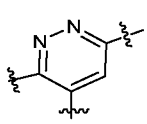, 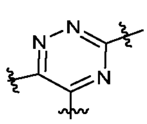 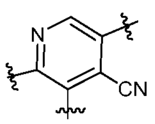, 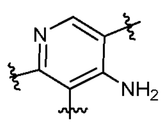, 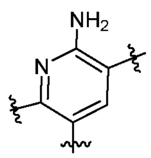,

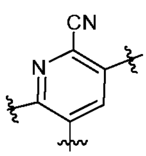, or 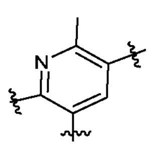;

for example

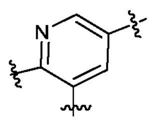 or 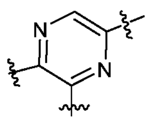 ;

for example

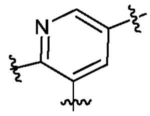 .

In a certain embodiment of the present invention,

$R^1$, and $R^{1'}$ are independently H or $C_{1-6}$ alkyl;
$R^3$, $R^{3'}$, $R^5$, and $R^{5'}$ are independently H;
$R^2$, and $R^4$ are independently $C_{1-6}$ alkyl substituted with one or more halo;
X and T are independently N;
Y is independently $C(R^{3c})$;
Z is independently $C(R^{3d})$ or N;
$A^1$ is independently $C(R^{4b}R^{4c})$ or C(=O);
B is independently O, S, N or $N(R^{6d})$, and $A^2$ is independently $C(R^{5b}R^{5c})$ or a linkage; and
= represents a single bond.

In a certain embodiment of the present invention,

$R^1$, and $R^{1'}$ are independently H or methyl;
$R^3$, $R^{3'}$, $R^5$, and $R^{5'}$ are independently H;
$R^2$, and $R^4$ are independently $CF_3$;
X and T are independently N;
Y is independently CH;
Z is independently CH or N;
$A^1$ is independently $CH_2$ or C(=O);
B is independently O, S, N or NH, and $A^2$ is independently $CH_2$ or a linkage; and
- - - - represents a single bond.

In a certain embodiment of the present invention,

$R^1$, $R^{1'}$, $R^3$, $R^{3'}$, $R^5$, and $R^{5'}$ are independently H;
$R^2$, and $R^4$ are independently $C_{1-6}$ alkyl substituted with one or more halo;
X and T are independently N;
Y is independently $C(R^{3c})$;
Z is independently $C(R^{3d})$;
$A^1$ is independently $C(R^{4b}R^{4c})$;
B is independently O or S;
$A^2$ is independently $C(R^{5b}R^{5c})$ or a linkage; and
= represents a single bond.

In a certain embodiment of the present invention,

$R^1$, $R^{1'}$, $R^3$, $R^{3'}$, $R^5$, and $R^{5'}$ are independently H;
$R^2$, and $R^4$ are independently $CF_3$;
X and T are independently N;
Y, and Z are independently CH;
$A^1$ is independently $CH_2$;
B is independently O or S;
$A^2$ is independently $CH_2$ or a linkage; and

= represents a single bond.

In a certain embodiment of the present invention, the fused triheterocyclic compound of Formula I has any of the structures shown below:

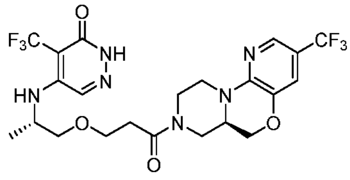
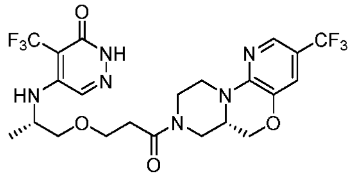
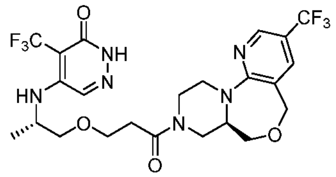
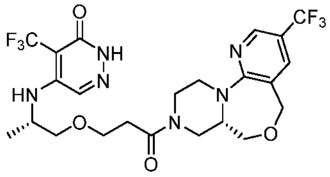
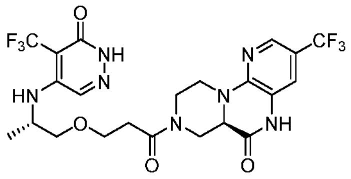
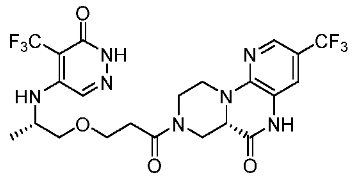
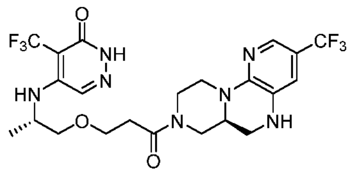
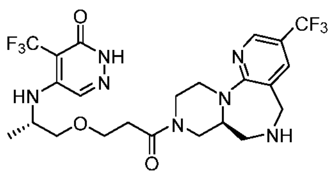
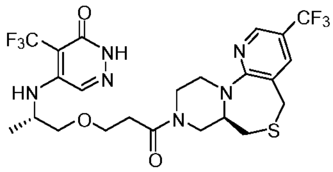
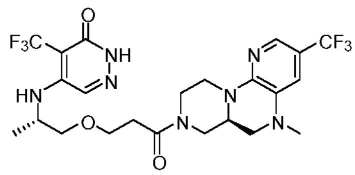
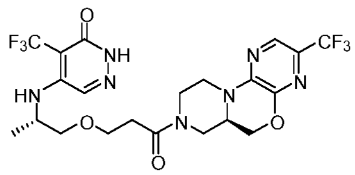
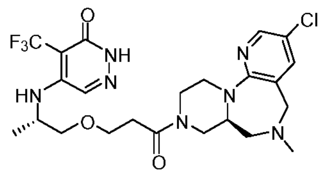
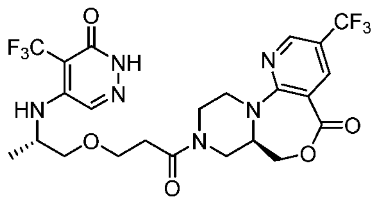
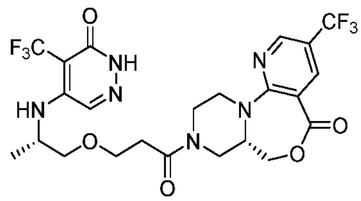
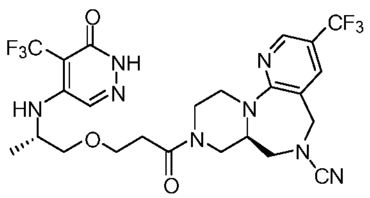
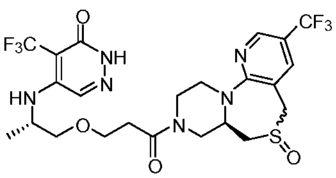
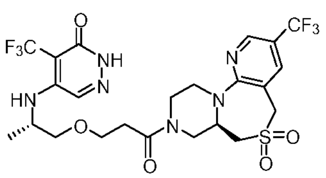
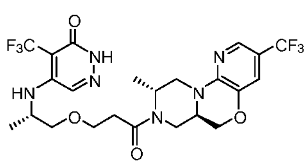
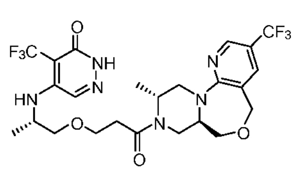
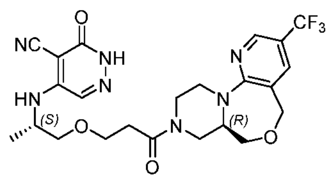

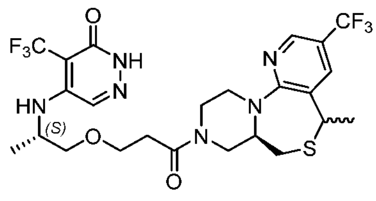
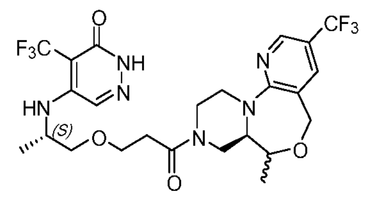
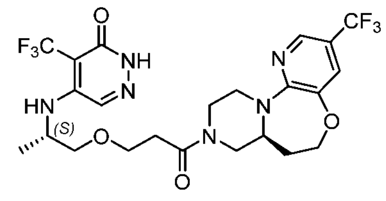

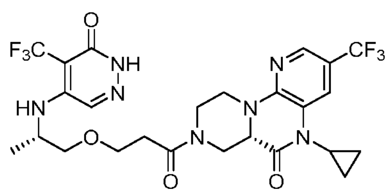
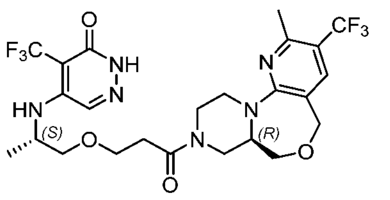
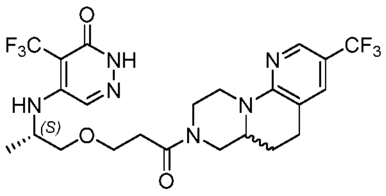

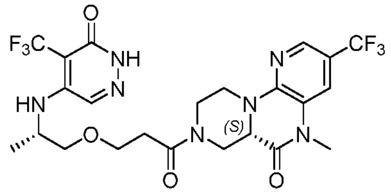
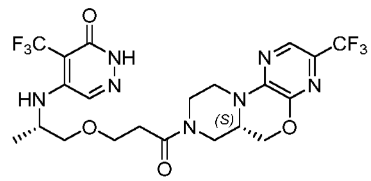
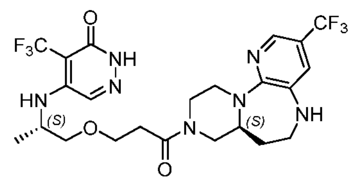

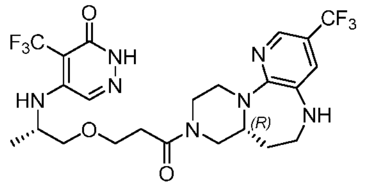
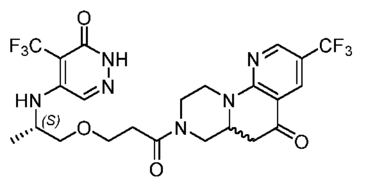
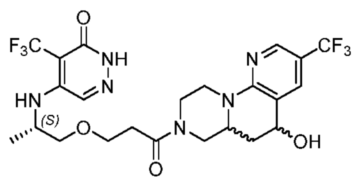

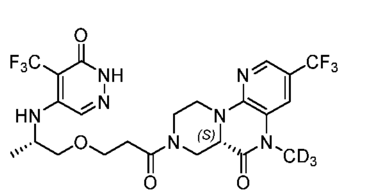
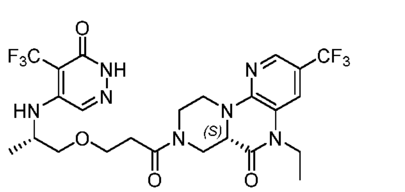
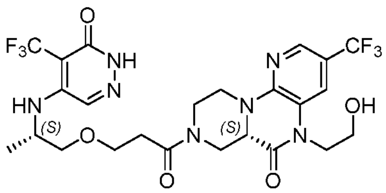

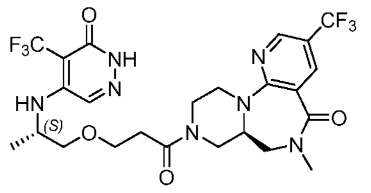
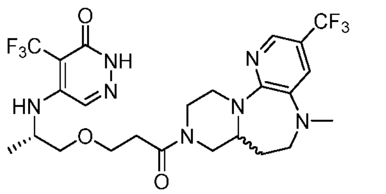
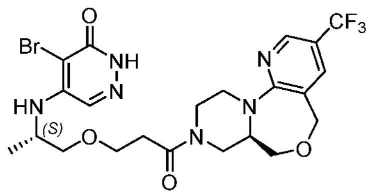

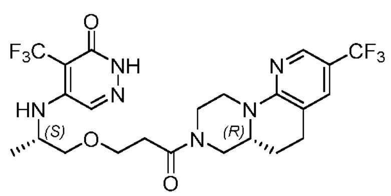
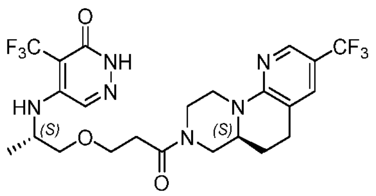
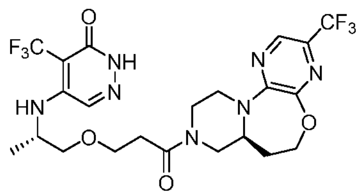

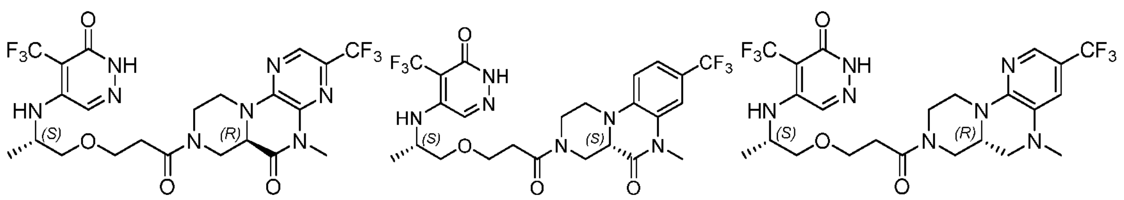

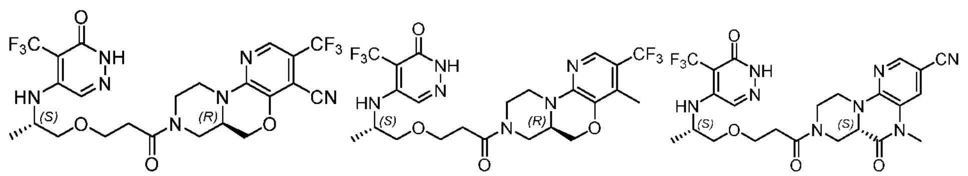

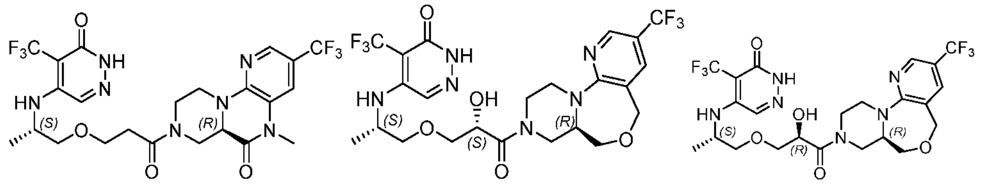

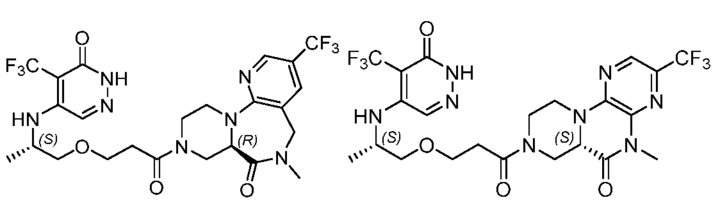

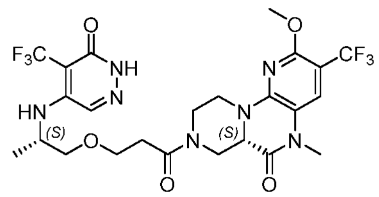

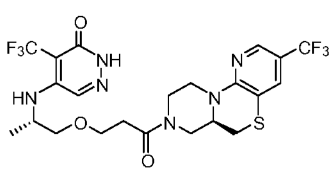

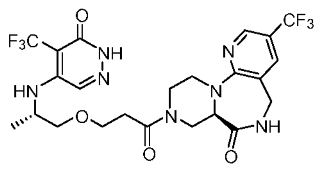

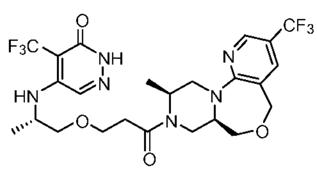

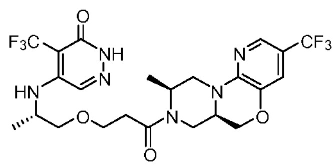

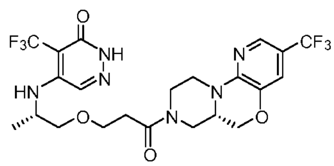

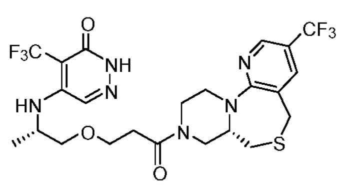

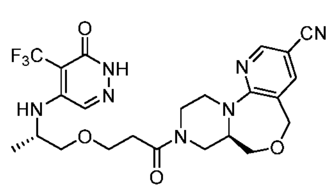

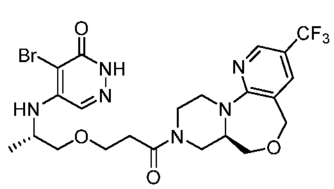

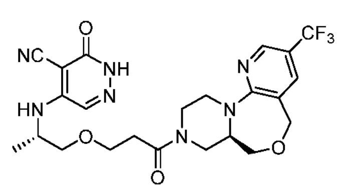

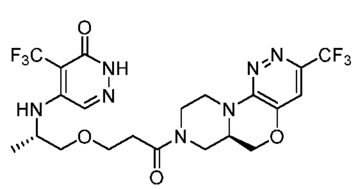

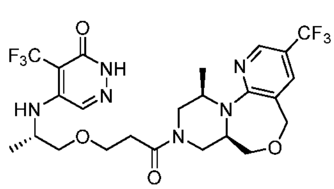

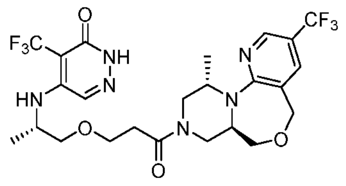
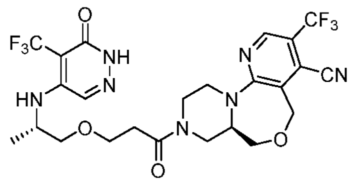
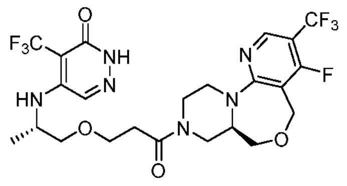

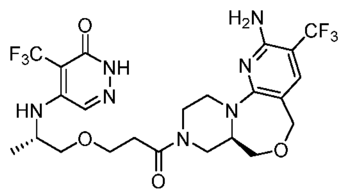
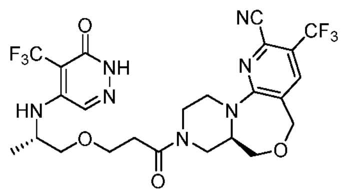
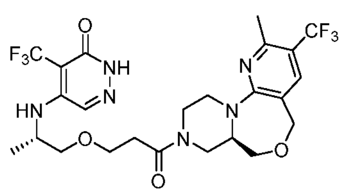

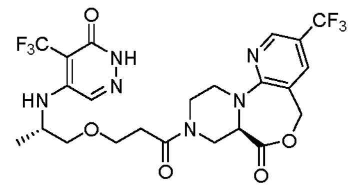
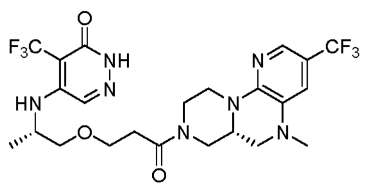

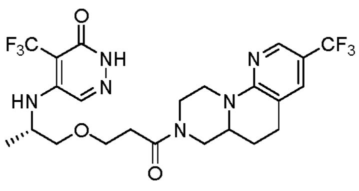
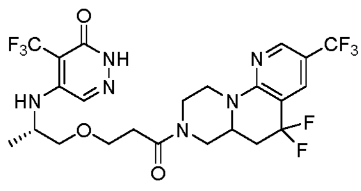
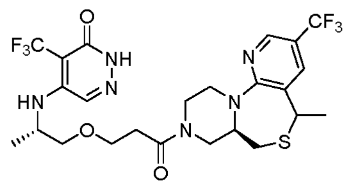

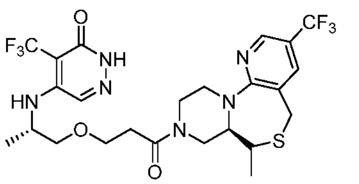
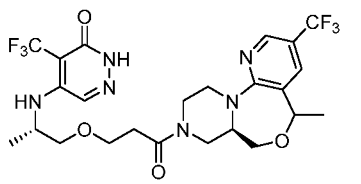
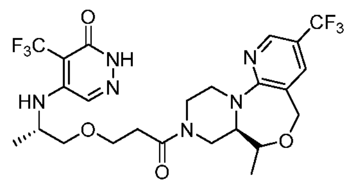

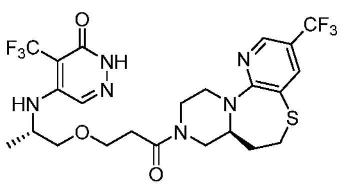
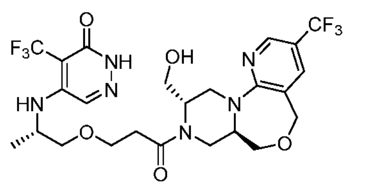
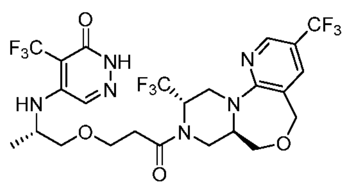

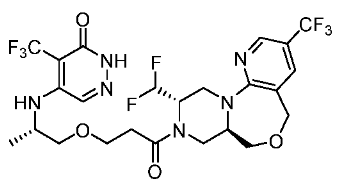
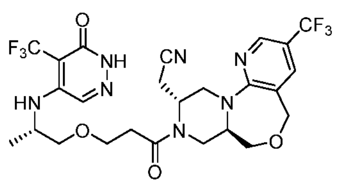
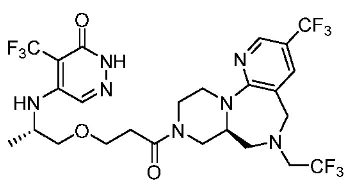

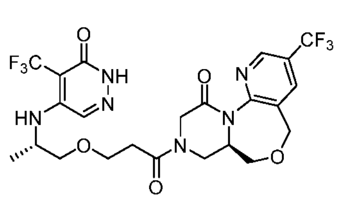
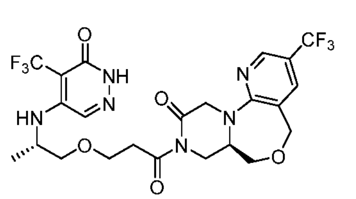
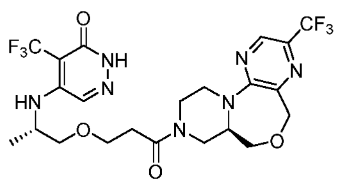

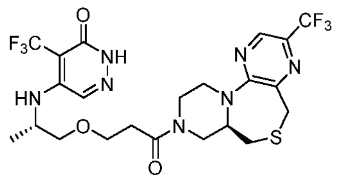
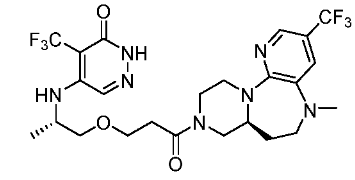
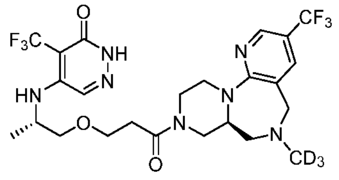

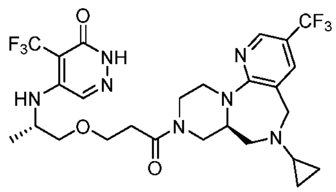

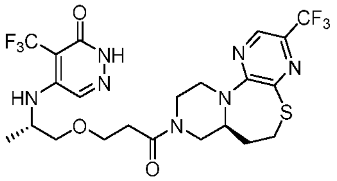
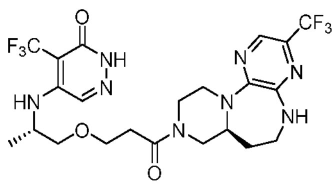
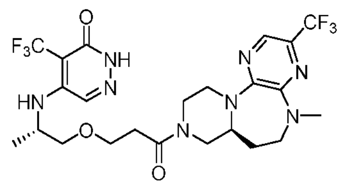

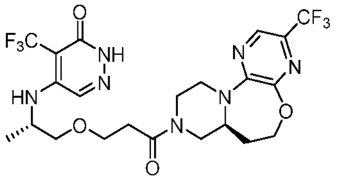
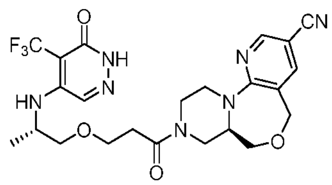
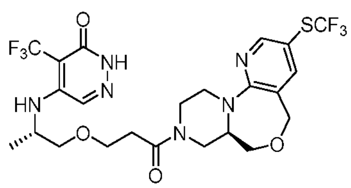

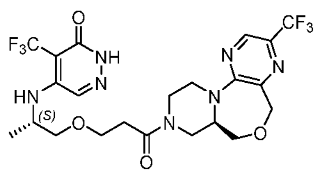
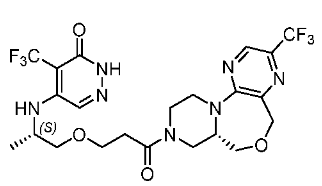
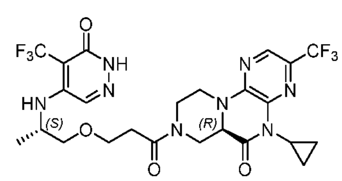

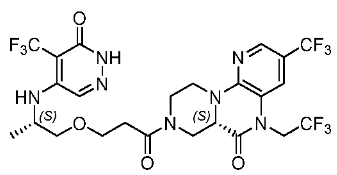
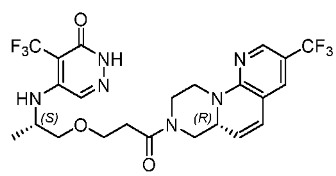
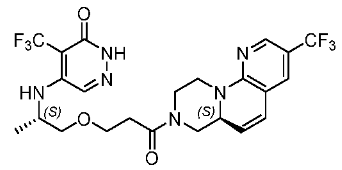

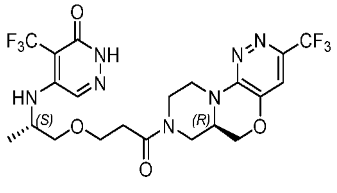
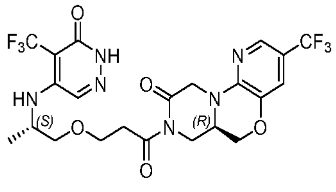
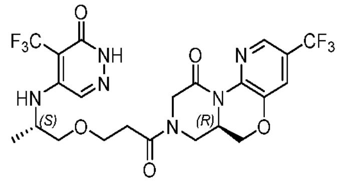

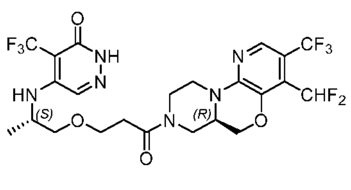
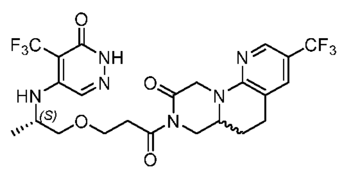

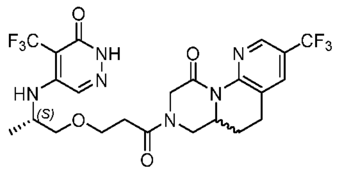
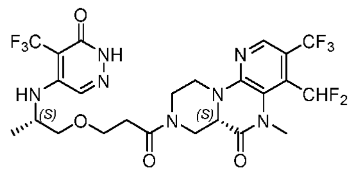
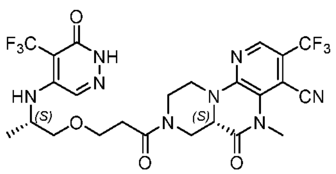
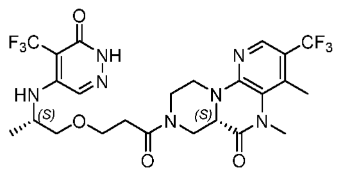
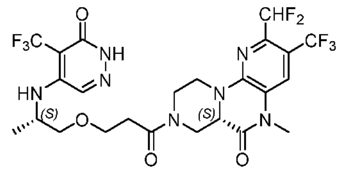
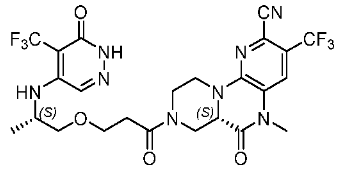
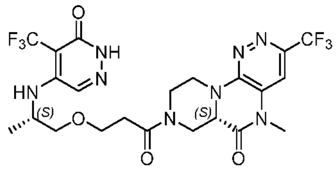
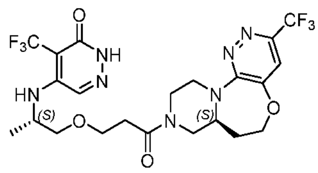
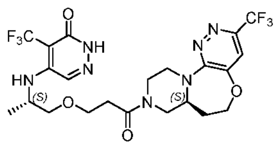
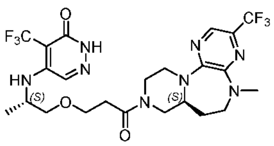
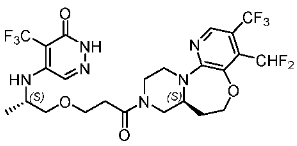
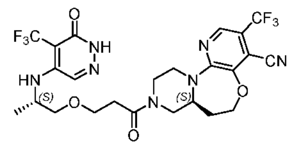
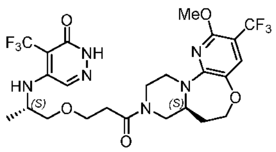
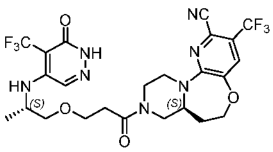
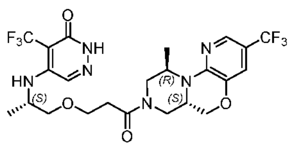
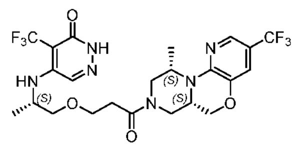
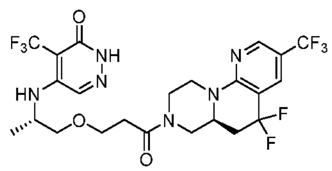
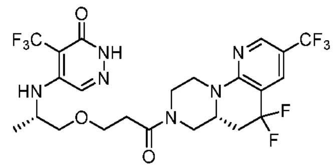
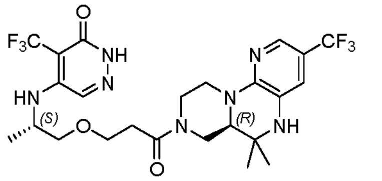
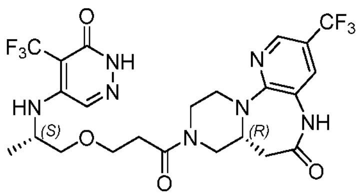
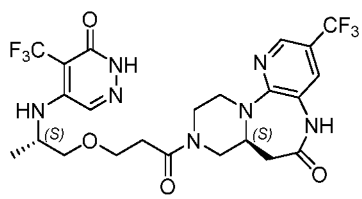
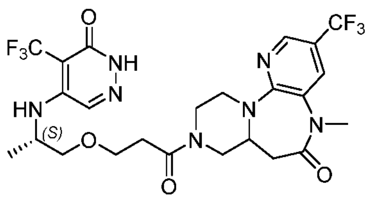

in which "⁓" bond represents a combination of R configuration and S configuration.

Therefore, throughout this specification, the groups and their substituents in the fused heterocyclic compound of Formula I or a pharmaceutically acceptable salt thereof can be selected by those skilled in the art, to provide a stable fused heterocyclic compound of Formula I or a pharmaceutically acceptable salt thereof, including, but not limited to, the compounds in the examples of the present invention.

The fused triheterocyclic compound of Formula I or a pharmaceutically acceptable salt thereof of the present invention can be synthesized according to a method similar to those well-known in the chemical field, in which the steps and conditions can refer to those for similar reactions in this field, and particularly according to the description herein. The starting materials are usually commercially available, for example, from Aldrich; or can be easily prepared through methods known to those skilled in the art (obtained from SciFinder, Reaxys online database).

In the present invention, the fused triheterocyclic compound of Formula I can also be obtained from the prepared fused triheterocyclic compound of Formula I, by side modification using the conventional method in the field to obtain other fused triheterocyclic compound of Formula I.

Generally, the compound of the present invention can be prepared by the method described in the present invention, where the definitions of substituents are as described for those in Formula I, unless otherwise specified. The following reaction schemes and examples are used to further illustrate the present invention by way of examples.

In the present invention, a method for preparing the fused triheterocyclic compound of Formula I comprises the following steps:

subjecting Compound of Formula II and Compound of Formula III to an amidation reaction in a solvent in the presence of a base and a condensing agent, to obtain a fused triheterocyclic compound of Formula I:

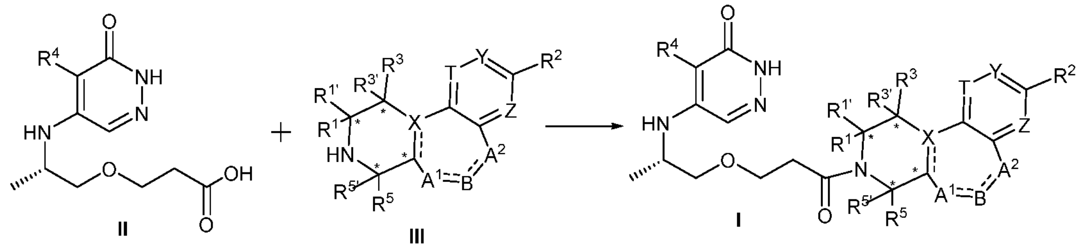

II III I

where $R^1$, $R^{1'}$, $R^3$, $R^{3'}$, $R^5$, $R^{5'}$, $R^2$, $R^4$, X, T, Y, Z, $A^1$, B, $A^2$- - - - and * are as defined above.

The conditions and operations of the amidation reaction can be conventional conditions and operations in this type of reactions in the art. In the present invention, preferably:
the solvent is DMF or dichloromethane (DCM). The base is diisopropylethyl amine or $K_2CO_3$. The molar ratio of the base to Compound of Formula II is 1:1. The condensing agent is propylphosphonic anhydride T3P, for example, as a 50% solution in ethyl acetate. The molar ratio of the condensing agent to Compound of Formula II is 1:1.

The molar ratio of Compound of Formula II to Compound of Formula III is 1:1. The amidation reaction is carried out under a nitrogen atmosphere. The reaction temperature of the amidation reaction is from 0°C to 60°C.

Essential raw materials or reagents for preparing Compound of Formula I are commercially available, can be prepared by synthesis methods known in the art. According to the method described in the experimental section below, the compound of the present invention can be prepared in the form of a free base or an acid addition salt thereof. The term pharmaceutically acceptable salt refers to a pharmaceutically acceptable salt defined herein having all pharmaceutical activities of the parent compound. The pharmaceutically acceptable salt can be prepared according to a conventional method by adding a corresponding acid to the organic base in an appropriate organic solvent.

Examples of the acid addition salt include: salts with inorganic acids, for example, hydrochloric acid, hydrobromic acid, sulfuric acid, nitric acid and phosphoric acid; and salts with organic acids, such as acetic acid, benzenesulfonic acid, benzoic acid, camphorsulfonic acid, citric acid, ethanesulfonic acid, fumaric acid, glucuronic acid, glutamic acid, glycolic acid, hydronaphthoic acid, 2-hydroxyethanesulfonic acid, lactic acid, maleic acid, malic acid, malonic acid, mandelic acid, methanesulfonic acid, 2-naphthalenesulfonic acid, propionic acid, salicylic acid, succinic acid, tartaric acid, p-toluenesulfonic acid or trimethylacetic acid.

The fused triheterocyclic compound of Formula I may have one or more chiral carbon atoms, so optically pure isomers, for example, pure enantiomer or racemate, or mixed isomers can be separated. Pure individual isomers can be obtained by separation methods known in this art, for example, chiral crystallization into a salt, or separation by a chiral preparative column.

The chemicals used in the synthesis route mentioned in this patent include solvents, reagents, catalysts, protective groups, deprotection groups and protecting groups. The method may additionally include a step before or after the step specifically described herein, and an appropriate protective group may be added or removed to obtain the target compound. In addition, various synthesis steps can be alternately or sequentially carried out to obtain the final target product.

The present invention provides a pharmaceutical composition, which comprises a fused triheterocyclic compound of Formula I or a pharmaceutically acceptable salt thereof, and (one or more) pharmaceutically acceptable adjuvants. In the pharmaceutical composition, the amount of the fused triheterocyclic compound of Formula I or a pharmaceutically acceptable salt thereof is a therapeutically effective amount.

The present invention further provides use of the fused triheterocyclic compound of Formula I or a pharmaceutically acceptable salt thereof in the preparation of PARP inhibitors. The PARP may be PARP7. In the use, the PARP inhibitor can be used in vivo in mammalian, or in vitro, mainly for experimental purposes. For example, it is used as a

standard or a reference to provide comparison, or prepared into a kit according to a conventional method in the art to provide rapid detection for the inhibitory effect on PARP.

The present invention also provides use of the fused triheterocyclic compound of Formula I or a pharmaceutically acceptable salt thereof in the preparation of drugs for preventing and/or treating PARP-related or PARP-mediated diseases. The PARP may be PARP7. The PARP-related or PARP-mediated diseases may include abnormal proliferative diseases or other diseases related to the target to which this series of compounds target and bind. The abnormal proliferative diseases include, but are not limited to, cancers, such as lung cancer (for example, squamous cell lung tumor, and H1373 lung cancer line), colon cancer, colorectal cancer, pancreatic cancer, renal cancer, gastric cancer, esophageal cancer, ovarian cancer, breast cancer, cervical cancer, head and neck cancer (upper respiratory and digestive tract), bladder cancer, melanoma, fibrosarcoma and glioblastoma. The amount of the fused triheterocyclic compound of Formula I or a pharmaceutically acceptable salt thereof can be a therapeutically effective amount.

The present invention further provides use of the fused triheterocyclic compound of Formula I or a pharmaceutically acceptable salt thereof or a pharmaceutical composition comprising the fused triheterocyclic compound of Formula I or a pharmaceutically acceptable salt thereof in the preparation of drugs for preventing and/or treating abnormal proliferative diseases. The abnormal proliferative diseases include, but are not limited to, cancers, such as lung cancer (for example, squamous cell lung tumor, and H1373 lung cancer line), colon cancer, colorectal cancer, pancreatic cancer, renal cancer, gastric cancer, esophageal cancer, ovarian cancer, breast cancer, cervical cancer, head and neck cancer (upper respiratory and digestive tract), bladder cancer, melanoma, fibrosarcoma and glioblastoma. The amount of the fused triheterocyclic compound of Formula I or a pharmaceutically acceptable salt thereof can be a therapeutically effective amount.

Another aspect of the present invention relates to a method for preventing and/or treating PARP7-related or PARP-mediated diseases, which comprises: administering, to a patient, a therapeutically effective amount of the fused triheterocyclic compound of Formula I or a pharmaceutically acceptable salt thereof, or a pharmaceutical composition comprising the fused triheterocyclic compound of Formula I or a pharmaceutically acceptable salt thereof.

Another aspect of the present invention relates to a method for therapeutically preventing and/or treating abnormal proliferative diseases, which comprises: administering, to a patient, a therapeutically effective amount of the fused triheterocyclic compound of Formula I or a pharmaceutically acceptable salt thereof, or a pharmaceutical composition comprising the fused triheterocyclic compound of Formula I or a pharmaceutically acceptable salt thereof. The abnormal proliferative diseases include, but are not limited to, cancers, such as lung cancer (for example, squamous cell lung tumor, and H1373 lung cancer line), colon cancer, colorectal cancer, pancreatic cancer, renal cancer, gastric cancer, esophageal cancer, ovarian cancer, breast cancer, cervical cancer, head and neck cancer (upper respiratory and digestive tract), bladder cancer, melanoma, fibrosarcoma and glioblastoma.

Another aspect of the present invention relates to a drug for inhibiting PARP7, which comprises the fused triheterocyclic compound of Formula I or a pharmaceutically acceptable salt thereof, or a pharmaceutical composition comprising the fused triheterocyclic compound of Formula I or a pharmaceutically acceptable salt thereof.

The compound of the present invention can be administered locally or systemically, for example, by enteral administration, such as rectal or oral administration, or parenteral administration to mammals (especially humans). The compound of the present invention can also be administered parenterally, for example, by inhalation, injection or infusion, such as intravenous, intra-arterial, intraosseous, intramuscular, intracerebral, extra-ventricular, intrasynovial, intrasternal, intrathecal, intra-lesion, intracranial, intra-tumor, intradermal and subcutaneous injection or infusion.

The effective amount of the compound, pharmaceutical composition or drug in the present invention depends on the species, weight, age, individual condition, and individual pharmacokinetic parameters of the mammal, diseases to be treated and the mode of administration.

The effective amount of the compound, pharmaceutical composition or drug in the present invention can be easily determined by routine experiments, and the most effective and convenient route of administration and the optimum preparation can also be determined by routine experiments.

The pharmaceutically acceptable adjuvant can be those widely used in the art of drug production. The adjuvant is mainly used to provide a safe, stable and functional pharmaceutical composition, and can also provide a way of making the active ingredients dissolve out at an expected rate after the subjects receive the composition administered, or promoting the effective absorption of the active ingredients after the subjects receive the composition administered. The pharmaceutically acceptable adjuvant may be an inert filler, or provide a certain function, for example, to stabilize the overall pH value of the composition or to prevent the degradation of the active ingredient in the composition. The pharmaceutically acceptable adjuvant may include one or more of a binder, a suspending agent, an emulsifier, a diluent, a filler, a granulating agent, an adhesive, a disintegrant, a lubricant, an anti-sticking agent, a glidant, a wetting agent, a gelling agent, an absorption retarder, a dissolution inhibitor, an enhancer, an adsorbent, a buffer, a chelating agent, a preservative, a colorant, a correctant and a sweetener.

Materials useful as pharmaceutically acceptable adjuvants include, but are not limited to, ion exchangers, alumina, aluminum stearate, lecithin, serum proteins (such as human serum albumin), buffer substances (such as

phosphate, glycine, sorbic acid or potassium sorbate), partial glyceride mixtures of saturated vegetable fatty acids, water, salts or electrolytes (such as protamine sulfate, disodium hydrogen phosphate, potassium hydrogen phosphate, sodium chloride or zinc salt), silica gel, magnesium trisilicate, polyvinylpyrrolidone, polyacrylate, wax, polyethylene-polypropylene oxide block copolymer, lanolin, sugars (such as lactose, glucose and sucrose), starch (such as corn starch and potato starch), cellulose and its derivatives (such as sodium carboxymethylcellulose, ethyl cellulose and cellulose acetate), powdered gums, malt, gels, talc, excipients (such as cocoa butter and suppository wax), oils (such as peanut oil, cottonseed oil, safflower oil, sesame oil, olive oil, corn oil and soybean oil), diol compounds (such as propylene glycol or polyethylene glycol), esters (such as ethyl oleate and ethyl laurate), agar, buffers (such as magnesium hydroxide and aluminum hydroxide), alginic acid, pyrogen free water, isotonic brine, Ringer's solution, ethanol, phosphate buffer, other non-toxic suitable lubricants (such as sodium lauryl sulfate and magnesium stearate), and colorants, releasing agents, coating agents, sweeteners, flavoring agents, flavorants, preservers and antioxidants.

The pharmaceutical composition of the present invention can be prepared by any method known to those skilled in the art according to the disclosure. For example, the conventional mixing, dissolution, granulation, emulsification, grinding, encapsulation, embedding or freeze-drying process can be used.

The pharmaceutical dosage form of the compound of the present invention can be provided in the form of immediate-release, controlled-release, sustained-release or targeted drug release systems. For example, the commonly used dosage forms include solutions and suspensions, (micro)emulsions, ointments, gels and patches, liposomes, tablets, dragees, soft-shell or hard-shell capsules, suppositories, ovules, implants, amorphous or crystalline powders, aerosols and freeze-dried preparations. Depending on the route of administration used, special devices may be needed to administer or give the drug, for example, syringes and needles, inhalators, pumps, injection pens, applicators or special flasks. The pharmaceutical dosage form often consist of a drug, an excipient and a container/sealing system. One or more excipients (also called inactive ingredients) can be added to the compound of the present invention to improve or promote the manufacture, stability, administration and safety of the drug, and provide an approach to the required drug release curve. Therefore, the types of excipients added to the drug can depend on various factors, for example, physical and chemical properties, route of administration, and preparation steps of the drug. Pharmaceutically acceptable excipients existing in the art, including those listed in various pharmacopoeias can be used. (see U.S. Pharmacopeia, USP, Japanese Pharmacopoeia, JP, European Pharmacopoeia, EP, and British pharmacopoeia, BP; publications of the Center for Drug Evaluation and Research (CEDR) of the U.S. Food and Drug Administration, www.fda.gov, for example, Inactive Ingredient Guide (1996); and Ash and Ash eds. Hand book of Pharmaceutical Additives, 2002, Synapse Information Resources, Inc., Endicott NY; etc.).

The pharmaceutical dosage form of the compound of the present invention can be manufactured by any method well known in the art, for example, by conventional mixing, sieving, dissolving, melting, granulating, coating, tableting, suspending, extruding, spray drying, grinding, emulsifying, (nano/micron-scale) encapsulation, embedding or freeze-drying process. As mentioned above, the composition of the present invention may include one or more physiologically acceptable inactive ingredients, which will promote the processing of the active molecules into preparations for pharmaceutical purpose.

The pharmaceutical composition of the present invention can be administered locally or systemically, for example, by enteral administration, such as rectal or oral administration, or parenteral administration to mammals (especially humans), and comprises a therapeutically effective amount of the compound or a pharmaceutically acceptable salt thereof according to the present invention as an active ingredient, together with a pharmaceutically acceptable excipient, such as a pharmaceutically acceptable carrier. The therapeutically effective amount of the active ingredient is defined in the context, and depends on the species, weight, age, individual condition, individual pharmacokinetic parameters, diseases to be treated and the mode of administration. For enteral administration, such as oral administration, the compound of the present invention can be formulated into a wide variety of dosage forms.

The pharmaceutical compositions and dosage forms may contain one or more compounds of the present invention, or one or more pharmaceutically acceptable salts thereof as the active ingredient. The pharmaceutically acceptable carrier can be solid or liquid. Solid preparations include powders, tablets, pills, lozenges, capsules, suppositories and dispersible granules. The solid carrier can also be one or more substances useful as a diluent, a flavoring agent, a solubilizing agent, a lubricant, a suspending agent, a binder, a preservative, a tablet disintegrant or an encapsulating material. In the powder, the carrier is usually a finely divided solid, which is in a mixture with the finely divided active component. In the tablet, the active component is generally mixed with a carrier with essential binding ability in an appropriate proportion and compacted according to the required shape and size. Suitable carriers include, but are not limited to, magnesium carbonate, magnesium stearate, talc, sugar, lactose, pectin, dextrin, starch, gelatin, methyl cellulose, sodium carboxymethyl cellulose, low-melting wax, and cocoa butter, etc. The preparation containing the active compound may include an encapsulating material as a carrier to provide a capsule, in which the active component with or without a carrier is surrounded by the carrier in association with it.

Other forms suitable for oral administration include liquid preparations, including emulsions, syrups, elixirs, aqueous solutions, and aqueous suspensions, or solid preparations intended to be converted into liquid preparations

immediately before use. The emulsion can be prepared in a solution, for example, a propylene glycol/water solution, or it can contain an emulsifier, such as lecithin, sorbitan monooleate or Arabic gum. The aqueous solution can be prepared by dissolving the active ingredient in water and adding an appropriate colorant, flavorant, stabilizer and thickener. The aqueous suspension can be prepared by dispersing the active ingredient in the form of fine particles in water with a binder such as natural or synthetic gum, resin, methyl cellulose, or carboxymethyl cellulose and other commonly used suspending agents. The solid preparations include solutions, suspensions and emulsions. Besides the active component, it can also contain a colorant, a flavorant, a stabilizer, a buffer, an artificial or natural sweetener, a dispersant, a thickener and a solubilizer.

Exemplary compositions for rectal administration include suppositories, which may contain, for example, a suitable non-irritating excipient, for example, cocoa butter, synthetic glycerides or polyethylene glycol, which is solid at normal temperature, but melt and/or dissolve in the rectal lumen to release the drug.

The compound of the present invention can also be administered parenterally, for example, by inhalation, injection or infusion, such as intravenous, intra-arterial, intraosseous, intramuscular, intracerebral, extra-ventricular, intrasynovial, intrasternal, intrathecal, intra-lesion, intracranial, intra-tumor, intradermal and subcutaneous injection or infusion.

Therefore, for parenteral administration, the pharmaceutical composition of the present invention can be in the form of sterile injectable or infusible preparations, for example, a sterile aqueous or oily suspension. The suspension can be prepared according to a known technology in the art using a suitable dispersing or wetting agent (for example, Tween 80) and suspending agent. The sterile injectable or infusible preparation can also be a sterile injectable or infusible solution or suspension in a parenterally acceptable nontoxic diluent or solvent. For example, the pharmaceutical composition can be a solution in 1,3- butanediol. Other examples of acceptable vehicles and solvents that can be used in the pharmaceutical composition of the present invention include, but are not limited to, mannitol, water, Ringer's solution and isotonic sodium chloride solution. In addition, sterile nonvolatile oils are often used as a solvent or suspending medium. Any mild non-volatile oils can be used for this purpose, including synthetic monoglycerides or diglycerides. Fatty acids such as oleic acid and their glyceride derivatives, as well as pharmaceutically acceptable natural oils, for example, olive oil or castor oil, especially in polyoxyethylated forms, can be used to prepare injections. These oily solutions or suspensions may also contain a long-chain alcohol diluent or a dispersant. Solutions for parenteral use may also include a suitable stabilizer and, if necessary, a buffer substance. Suitable stabilizers include antioxidants, for example, sodium bisulfate, sodium sulfite, ascorbic acid, citric acid, or salts thereof, and EDTA sodium salt, alone or in combination. The parenteral solution may also contain a preservative, such as benzalkonium chloride, p-hydroxybenzoic acid or propyl p-hydroxybenzoate and chlorobutanol.

For inhalation or nasal administration, suitable pharmaceutical preparations include particles, aerosols, powders, mists or droplets, having, for example, an average particle size of about 10 microns or less. For example, a composition for inhalation in the form of a solution can be prepared in brine by using benzyl alcohol or other suitable preservatives, an absorption enhancer for improving the bioavailability, fluorocarbon and/or other solubilizers or dispersants known in the art.

The pharmaceutical composition of the present invention can also be locally administered to the skin or mucosa. For topical application, the pharmaceutical composition can be, for example, a lotion, a gel, a paste, a tincture, a transdermal patch, and a gel for transmucosal delivery.

The pharmaceutical composition can be an appropriate ointment preparation containing the active ingredient suspended or dissolved in a carrier. Carriers for topical administration of the compound of the present invention include, but are not limited to, mineral oil, liquid petroleum, white petroleum, propylene glycol, polyoxyethylene, polyoxypropylene, emulsified wax and water. Alternatively, the pharmaceutical composition can be prepared into a suitable lotion or emulsion containing the active compound suspended or dissolved in a carrier. Suitable carriers include, but are not limited to, mineral oil, sorbitan monostearate, polysorbate 60, cetyl alcohol, 2-octyldodecanol, benzyl alcohol and water. The pharmaceutical composition of the present invention can also be locally applied to the lower intestinal tract through a rectal suppository preparation or a suitable enema preparation. Suitable pharmaceutical excipients (such as carriers) and methods for preparing pharmaceutical dosage forms are described in standard reference textbooks in the field of pharmaceutical preparations (Remington's Pharmaceutical Sciences, Mack Publishing Company).

The therapeutically effective dose can be estimated using various methods well known in the art. The initial dose for animal research can be based on the effective concentration established in cell culture assays. The dosage range suitable for human individuals can be determined, for example, from the data obtained from animal research and cell culture assays. In some embodiments, the compound of the present invention can be prepared into a drug for oral administration.

The effective amount or therapeutically effective amount or dose of a drug (for example, the compound of the present invention) refers to an amount of the drug or compound that causes the improvement of symptoms or the extension of survival time of the individuals. The toxicity and therapeutic efficacy of the molecule can be determined by a standard medical procedure in cell culture or experimental animals, for example, for example, by determining $LD_{50}$ (a

dose causing 50% mortality of the population) and $ED_{50}$ (a dose that is therapeutically effective for 50% of the population). A ratio of the dose that produces toxicity to the dose that produces a therapeutic effect is the therapeutic index, which can be expressed as $LD_{50}/ED_{50}$. An agent showing high therapeutic index is preferred.

An effective amount or a therapeutically effective amount is an amount of a compound or pharmaceutical composition that will trigger a biological or medical response of tissues, systems, animals or humans sought by researchers, veterinarians, doctors or other clinicians. The dosage is preferably within the range of circulation concentration including the minimal toxicity or non-toxic $ED_{50}$. The dosage can vary within this range, depending on the dosage form used and/or the route of administration used. According to the methods known in the art, an appropriate preparation, route of administration, dosage, and dosing interval can be selected by taking specific individual conditions into account.

The dosage and interval can be adjusted individually to provide the plasma level of the active ingredient sufficient to obtain the desired effect, that is, minimum effective concentration (MEC). MEC of each compound will be different, and can be estimated from in-vitro data and animal experiments. The dosage required to obtain MEC depends on the individual features and route of administration. In the case of local administration or selective uptake, the effective local concentration of the drug may be independent of the plasma concentration.

The amount of drug or composition administered depends on various factors, including the sex, age and weight of the treated individual, the severity of the disease, the mode of administration and the judgment of the prescriber.

If necessary, the composition of the present invention can be provided by a package or dispensing device containing one or more unit dosage forms (containing the active ingredient). For example, the package or device may include a metal or plastic foil (such as foamed packaging) or a glass and rubber stopper, for example, in a vial. The package or dispensing device can be provided with instructions for use. It is also possible to prepare a composition containing the compound of the present invention formulated in a compatible pharmaceutical carrier, which is accommodated in an appropriate container with a label indicating specific conditions to be treated.

Unless otherwise specified, all technical and scientific terms used herein have the standard meanings in the art to which the claimed subject belongs. If there are multiple definitions for a term, the definition herein shall prevail.

**Definitions of groups**

Unless otherwise specified, the following definitions used herein shall apply. For the purpose of the present invention, the chemical elements are consistent with the periodic table, CAS version, and the Handbook of Chemistry and Physics, 75th edition, 1994. In addition, that general principle of organic chemistry can refer to the description in "Organic Chemistry", Thomas Sorrell, University Science Books, Sausalito: 1999, and "March's Advanced Organic Chemistry" by Michael B. Smith and Jerry March, John Wiley & Sons, New York: 2007, which are incorporated herein by reference in their entireties.

In this specification, the groups and their substituents can be selected by those skilled in the art to provide stable structural moieties and compounds. When a substituent is described by a conventional chemical formula written from left to right, the substituent also includes a chemically equivalent substituent obtained by writing the structural formula from right to left.

Some chemical groups defined herein are preceded by simplified symbols to indicate the total number of carbon atoms in the group. For example, $C_1$-$C_6$ alkyl refers to an alkyl group defined below, having 1, 2, 3, 4, 5 or 6 carbon atoms in total. The total number of carbon atoms indicated by the simplified symbol does not include carbon that may exist in a substituent to the group.

Herein, numerical ranges defined for the substituents such as 0 to 4, 1 to 4, and 1 to 3, indicate integers in this range, for example, 1 to 6 indicates 1, 2, 3, 4, 5, and 6.

Besides, when used in the description and claims of the present application, the following terms have the following meanings unless otherwise specified.

The term "includes" is an open expression, that is, it includes the contents indicated in the present invention, but does not exclude other contents.

The term "substituted" refers to the substitution of any one or more hydrogen atoms on a specific atom with a substituent(s), including heavy hydrogen and hydrogen variants, as long as the valence of the specific atom is normal and the substituted compound is stable.

Generally, the term "optionally", the term "substituted" means that one or more hydrogen atoms in a given structure are replaced by a specific substituent(s). Further, when a group is substituted with more than one substituent, the substituents are independent of each other, that is, the more than one substituent may be different from each other or the same. Unless otherwise indicated, the substitution with a substituent can occur at various substitutable positions of the substituted group. When more than one position in a given structural formula can be substituted with one or more substituents selected from specific groups, the substitution with the substituents can occur at the same or different positions.

In various sections of the specification, the substituents of the compound disclosed in the present invention

are disclosed according to the group type or range. In particular, the present invention includes each independent sub-combination of various members in the type or group of groups. For example, the term "$C_1$-$C_6$ alkyl" or "$C_{1-6}$ alkyl group" particularly refers to independently disclosed methyl, ethyl, $C_3$ alkyl, $C_4$ alkyl, $C_5$ alkyl and $C_6$ alkyl; and "$C_{1-4}$ alkyl" particularly refers to independently disclosed methyl, ethyl, $C_3$ alkyl (that is, propyl, including n-propyl and isopropyl), and $C_4$ alkyl (that is, butyl, including n-butyl, isobutyl, s-butyl and t-butyl).

The term "halo" is selected from F, Cl, Br or I, and particularly refers to F or Cl.

In the present application, the term "alkyl", as a group or a part of other groups, refers to a saturated aliphatic hydrocarbon group, which is a linear or branched group containing 1 to 12 carbon atoms, preferably a linear or branched alkyl group containing 1 to 6 carbon atoms, and represented by general formula $C_nH_{2n+1}$. The term "$C_1$-$C_6$ alkyl" refers to an alkyl moiety comprising 1, 2, 3, 4, 5 or 6 carbon atoms. In a certain embodiment, the "alkyl" refers to $C_1$-$C_6$ alkyl. In a certain embodiment, the "alkyl" refers to $C_1$-$C_4$ alkyl.

Non-limiting examples of lower alkyl groups containing 1 to 6 carbon atoms include methyl, ethyl, n-propyl, isopropyl, n-butyl, isobutyl, t-butyl, s-butyl, n-pentyl, 1, 1-dimethylpropyl, 1, 2-dimethylpropyl, 2, 2-dimethylpropyl, 1-ethylpropyl, 2-methylbutyl, 3-methylbutyl, n-hexyl, 1-ethyl-2- methylpropyl, 1, 1, 2-trimethylpropyl, 1, 1-dimethylbutyl, 1, 2-dimethylbutyl, 2, 2-dimethylbutyl, 1, 3-dimethylbutyl, 2-ethylbutyl, 2- methylpentyl, 3-methylpentyl, 4-methylpentyl, and 2,3-dimethylbutyl.

In the present application, unless otherwise specified, the term "cycloalkyl" as a group or a part of other groups refers to a saturated monocyclic, polycyclic or bridged carbocyclic substituent composed exclusively of carbon atoms and hydrogen atoms, which can be attached to the rest of a molecule by a single bond via any suitable carbon atom. When it is polycyclic, it can be a bridged ring system or a spiro ring system with bridge ring connection or spiro ring connection (that is, two geminal hydrogen atoms on a carbon atom are replaced by an alkylene group). A cycloalkyl substituent can be attached to the central molecule through any suitable carbon atom. In some embodiments, $C_3$-$C_6$ cycloalkyl includes cyclopropyl ($C_3$), cyclobutyl ($C_4$), cyclopentyl ($C_5$), and cyclohexyl ($C_6$). In some embodiments, examples of $C_3$-$C_{10}$ cycloalkyl include $C_3$-$C_6$ cycloalkyl, and cycloheptyl ($C_7$), cyclooctyl ($C_8$), cyclononyl ($C_9$) and cyclodecyl ($C_{10}$).

As used herein, the terms "moiety", "structural moiety", "chemical moiety", "group" and "chemical group" refer to a specific fragment or functional group in a molecule. The chemical moiety is generally considered as a chemical entity embedded or attached to a molecule.

When the atom via which the listed substituent is attached to the compound contemplated by the chemical general formula but not specifically given is not specified, such a substituent can be bonded via any atom. The combination of substituents and/or their variants is permitted only if such a combination will produce a stable compound.

In various sections of the specification, linking substituents are described. When a structure clearly requires a linking group, the Markush variable given for the group should be understood as a linking group. For example, if a structure requires a linking group, and the Markush group definition for this variable gives "alkyl" or "aryl", it should be understood that the "alkyl" or "aryl" respectively represents an alkylene or arylene linking group.

In some specific structures, when the alkyl group is explicitly expressed as a linking group, the alkyl group represents a linking alkyl group, for example, $C_1$- $C_6$ alkyl in the group "halo-$C_1$-$C_6$ alkyl" is understood as $C_1$-$C_6$ alkylene.

The term "alkylene" refers to a saturated divalent hydrocarbyl group obtained by removing two hydrogen atoms from a linear or branched saturated hydrocarbyl group. Examples of alkylene group includes methylene ($-CH_2-$), ethylene {including $-CH_2CH_2-$ or $-CH(CH_3)-$}, and isopropylene {including $-CH(CH_3)CH_2-$ or $-C(CH_3)_2-$} etc.

Unless otherwise specified, all technical and scientific terms used herein have the standard meanings in the art to which the claimed subject belongs. If there are multiple definitions for a term, the definition herein shall prevail.

It should be understood that the singular form used in the present invention, such as "an", includes plural reference, unless otherwise specified. In addition, the term "includes" is an open-ended, but not closed-ended definition, and it includes the contents indicated in the present invention, but does not exclude other contents.

Unless otherwise specified, the traditional methods such as mass spectrometry and elemental analysis are adopted in the present invention, and the steps and conditions can refer to the conventional operation steps and conditions in this art.

Unless otherwise specified, standard nomenclature and standard laboratory procedures and techniques for analytical chemistry, organic synthetic chemistry and optics are adopted in the present invention. In some cases, standard technologies are used in chemical synthesis, chemical analysis and performance tests by light-emitting devices.

In addition, it should be noted that unless otherwise explicitly indicated, the description "... are independently" used in the present invention should be understood in a broad sense, and means that specific individuals described are independent of each other and can be the same or different. In further detail, the description "... are independently" used in the present invention means that specific options expressed by the same symbols in different groups do not affect each other, or that specific options expressed by the same symbols in the same group do not affect each other.

It can be understood by those skilled in the art that according to the conventions used in this art, "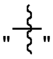" used in the structural formula of a group described in the present application means that a corresponding group is attached to other fragments and groups in the compound through this site.

"Pharmaceutically acceptable" refers to a situation that can be used to prepare a pharmaceutical composition, which is generally safe and non-toxic, and desirable biologically and otherwise, including acceptable situations for veterinary and human uses.

The term "excipient" refers to a pharmaceutically acceptable chemical substance, for example, a reagent known to those skilled in the art of pharmaceutical science to facilitate the administration. It is a compound that can be used to prepare a pharmaceutical composition, and is generally safe, non-toxic and desirable biologically or otherwise, including excipients acceptable for veterinary and human uses. Common excipients include binders, surfactants, diluents, disintegrants and lubricants.

The term "therapeutically effective amount" refers to an amount of a compound used that is sufficient to achieve the treatment of a disease state when administered to a subject to treat the disease state. The "therapeutically effective amount" will vary depending on the compound, the disease state treated, the severity of the disease treated, the age and relative health of the subject, the route and mode of administration, and the judgment of the attending physician or veterinarian.

As used herein, "treatment" or "treating" is to obtain beneficial or expected results, including clinical results. The beneficial or expected clinical results include, but are not limited to, alleviation or improvement of one or more symptoms or disorders, reduction of severity, stabilization of disease state (for example, it is not exacerbated), prevention of spread, delay or slowdown of disease development, improvement or remission of disease state, and partial or total amelioration, whether it is detectable or undetectable. The term can also refer to prolonged survival time compared with the expected survival time without treatment.

The term mammal refers to human or any mammal, such as primates, farm animals, pet animals or laboratory animals. Examples of these animals include monkeys, cows, sheep, horses, pigs, dogs, cats, rabbits, mice and rats. The mammals are preferably humans.

Within the common knowledge in the art, the above-mentioned preferred conditions can be arbitrarily combined to obtain the preferred embodiments of the present invention.

The reagents and raw materials used in the present invention are all commercially available.

The present invention has the following positive and beneficial effects. The fused triheterocyclic compound can be used as a poly-ADP-ribose polymerase 7 (PARP 7) inhibitor; and is useful in the treatment of diseases that can be improved by inhibiting poly(ADP- ribose) polymerase and in the preparation of drugs for preventing and/or treating abnormal proliferative diseases or other diseases related to the target to which this series of compounds target and bind.

## DETAILED DESCRIPTION OF THE PREFERRED EMBODIMENT

The present invention will be further described by way of examples below; however, the present invention is not limited thereto. In examples below where no specific conditions are given in the experimental methods, the experimental conditions are selected according to conventional methods and conditions, or according to the product instructions.

PMB: 4-methoxybenzyl; T3P: propylphosphonic anhydride.

### Synthesis of Intermediates

**Intermediate1:** Synthesis of (S) -3-(2-((6-oxo-5-(trifluoromethyl)-1,6-dihydropyridazin-4-yl)amino)propoxy)propionic acid

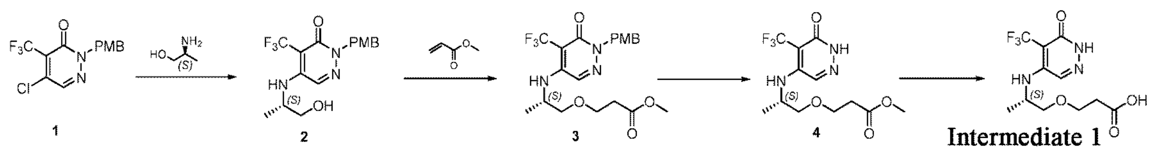

1 2 3 4 Intermediate 1

Step I: Synthesis of 5-{[(2S)-1-hydroxypropyl-2-yl]amino}-2-[(4-methoxyphenyl)methyl]-4-(trifluoromethyl)-2,3-dihydropyridin-3-one **(2)**

(2S)-2-aminoprop-1-ol (0.236 g,3.14 mmol) and 5-chloro-2-[(4-methoxyphenyl)methyl]-4-(trifluoromethyl)-2,3-dihydropyridazin-3-one (1 g,3.14 mmol) were dissolved in ethanol (10 mL), and then triethyl amine (0.952 g,9.42 mmol) was added. The reaction mixture was reacted with stirring at 60°C for 12 hrs. The reaction solution was cooled and then concentrated to dryness. The crude product was purified by column chromatography on silica gel (petroleum ether: ethyl acetate =40:60) to obtain the target product **(2)** as a yellow oil: 5-{[(2S)-1-hydroxypropyl-2-yl]amino}-2-[(4-methoxyphenyl)methyl]-4-(trifluoromethyl)-2,3-dihydropyridazin-3-one **(2)** (0.8 g, 2.239 mmol, yield: 71%), LCMS: MS(ESI)m/z: 358[M+H]$^+$.

Step II: Synthesis of methyl 3-[(2S)-2-({1-[(4-methoxyphenyl)methyl]-6-oxo-5-(trifluoromethyl)-1,6-dihydropyridazin-4-yl}amino)propoxy]propionate **(3)**

The compound 5-{[(2S)-1-hydroxypropyl-2-yl]amino}-2-[(4-methoxyphenyl)methyl]-4-(trifluoromethyl)-2,3-dihydropyridazin-3-one (0.63 g, 1.4 mmol) and methyl acrylate (0.362 g, 4.2 mmol) were dissolved in acetonitrile (5 mL), and then cesium carbonate (1.37 g, 4.2 mmol) was added. The mixture was reacted with stirring at room temperature for 3 hrs. The solid was filtered off, and the filtrate was concentrated to obtain a crude product. The crude product was purified by column chromatography on silica gel (petroleum ether: ethyl acetate =70:30) to obtain the target product as a yellow oil: methyl 3-[(2S)-2-({ 1-[(4-methoxyphenyl)methyl]-6-oxo-5-(trifluoromethyl)-1,6-dihydropyridazin-4-yl}amino)propoxy]propionate **(3)** (400 mg, 0.902 mmol, yield: 64%), LCMS: MS(ESI)m/z: 444 [M+H]$^+$.

Step III: Synthesis of methyl 3-[(2S)-2-{[6-oxo-5-(trifluoromethyl)-1,6-dihydropyridazin-4-yl] amino }propoxy]propionate **(4)**

The compound methyl 3-[(2S)-2-({ 1-[(4-methoxyphenyl)methyl]-6-oxo-5-(trifluoromethyl)-1,6-dihydropyridazin-4-yl}amino)propoxy]propionate (0.5 g, 1.128 mmol) was dissolved in dichloromethane (5 mL), and then trifluoroacetic acid (0.2 mL) and trifluoromethanesulfonic acid (0.1 mL) were added. The reaction mixture was reacted with stirring at room temperature for 2 hrs. After complete reaction, a saturated sodium bicarbonate solution was added to the ice-cold reaction solution to adjust the pH to 8. The solution was extracted with ethyl acetate (20 mL). The extract was dried over anhydrous sodium sulfate, and then filtered to remove anhydrous sodium sulfate. The crude product was purified by column chromatography on silica gel(petroleum ether: ethyl acetate =20:80) to obtain the target product as a white oil: methyl 3-[(2S)-2-{[6-oxo-5-(trifluoromethyl)-1,6-dihydropyridazin-4-yl]amino}propoxy]propionate **(4)** (0.2 g, 0.693 mmol, yield: 83%). LCMS: MS(ESI)m/z: 324 [M+H]$^+$.

Step IV: Synthesis of 3-[(2S)-2-{[6-oxo-5-(trifluoromethyl)-1,6-dihydropyridazin-4-yl]amino}propoxy]propionic acid **(Intermediate 1)**

Methyl 3-[(2S)-2-{ [6-oxo-5-(trifluoromethyl)-1,6-dihydropyridazin-4-yl]amino}propoxy]propionate (1 g, 3.093 mmol) was dissolved in methanol (5 mL) and water (5 mL), and then lithium hydroxide (0.15 g, 3.712 mmol) was added. The reaction mixture was stirred at room temperature until the reaction was completed. Then the reaction solution was poured into iced water (50 mL), adjusted to pH 3, and extracted with ethyl acetate (20 ml). The crude product was purified by column chromatography on silica gel (petroleum ether: ethyl acetate =10:90) to obtain the target product as a white oil: 3-[(2S)-2-{[6-oxo-5-(trifluoromethyl)-1,6-dihydropyridazin-4-yl]amino}propoxy]propionic acid **(Intermediate 1)** (0.8 g, 2.587 mmol, yield: 83%), LCMS: MS(ESI)m/z: 310 [M+H]$^+$.

**Intermediate 2:** Synthesis of 2-hydroxy-3-((S)-2-((6-oxo-5-(trifluoromethyl)-1-((2-(trimethylsilyl)ethoxy)methyl)-1, 6-dihydropyridazin-4-yl)amino)propoxy)propionic acid

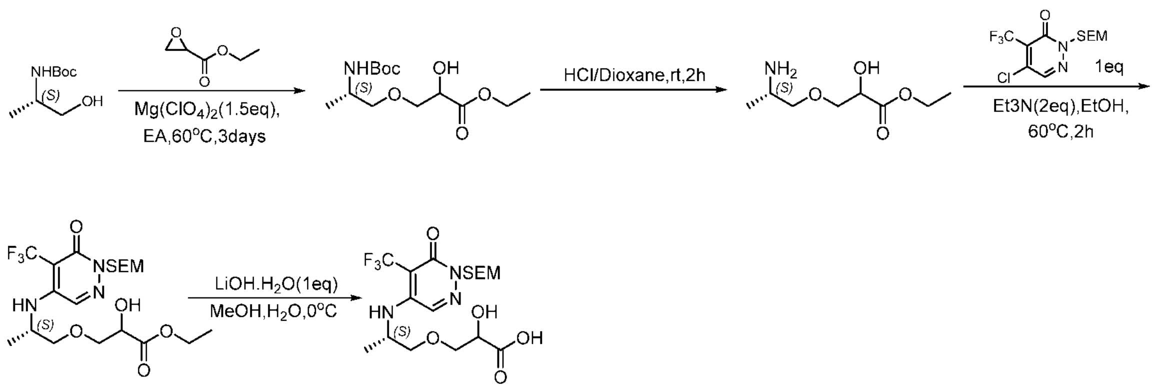


Intermediate 2

Step I: Synthesis of ethyl 3-((S)-2-((t-butoxycarbonyl)amino)propoxy)-2-hydroxypropionate

(S)-2-(t-butoxycarbonylamino)-1-propanol (10.00 g, 57.1 mmol) and ethyl oxirane-2-carboxylate (6.62 g, 57.1 mmol) were dissolved in ethyl acetate (200 ml), and then magnesium perchlorate (19.12 g, 85.65 mmol) was added, and reacted at 60°C for 3 days. After complete reaction, the reaction solution was cooled to room temperature, poured into water (200 ml), and extracted with ethyl acetate (50 ml × 3). The organic phases were combined, washed with saturated brine, dried over anhydrous sodium sulfate, and filtered. The filtrate was concentrated. The crude product was purified by column chromatography on silica gel (ethyl acetate:petroleum ether=80: 20) to obtain ethyl 3-((S)-2-((t-butoxycarbonyl)amino)propoxy)-2-hydroxypropionate as a yellow oil (9.98 g, 34.2 mmol, yield 59%). LCMS (ESI) m/z: 292 $[M+H]^+$

Step II: Synthesis of ethyl 3-((S)-2-aminopropoxy)-2-hydroxypropionate

Ethyl 3-((S)-2-((t-butoxycarbonyl)amino)propoxy)-2-hydroxypropionate (9.98 g, 34.2 mmol) was dissolved in ice-cold solution (0°C) of hydrogen chloride in 1,4-dioxane (4 mol/l, 30 ml), slowly heated to room temperature, and reacted with stirring for 2 hrs. After complete reaction, the reaction solution was concentrated under reduced pressure. The crude product was diluted with water (20 ml), adjusted to pH 8-9 with sodium bicarbonate, and then extracted with dichloromethane (20 ml × 3). The organic phases were combined, washed with saturated brine, dried over anhydrous sodium sulfate, and filtered. The filtrate was concentrated. The crude product was purified by column chromatography on silica gel (dichloromethane: methanol=80: 20) to obtain ethyl 3-((S)-2-aminopropoxy)-2-hydroxypropionate as a yellow solid (4.99 g, 26.0 mmol, yield 76%). LCMS (ESI) m/z: 192 $[M+H]^+$

Step III: Synthesis of ethyl 2-hydroxy-3-((S)-2-((6-oxo-5-(trifluoromethyl)-1-(2-(trimethylsilyl)ethoxy)methyl)-1, 6-dihydropyridazin-4-yl)amino)propoxy)propionic acid

Ethyl 3-((S)-2-aminopropoxy)-2-hydroxypropionate (4.99 g, 26.0 mmol) and 5-chloro-4-(trifluoromethyl)-2-(2-(trimethylsilyl)ethoxy)methyl)pyridazin-3(2H)-one (8.53 g, 26.0 mmol) were dissolved in ethanol (20 ml), and then triethyl amine (5.25 g, 52.0 mmol) was added and reacted at 60°C for 2 hrs. After complete reaction, the reaction solution was cooled to room temperature, poured into water (50 ml), and extracted with ethyl acetate (30 ml × 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (dichloromethane: methanol=70: 30) to obtain ethyl 2-hydroxy-3-((S)-2-((6-oxo-5-(trifluoromethyl)-1-(2-(trimethylsilyl)ethoxy)methyl)-1, 6-dihydropyridazin-4-yl)amino)propoxy)propionate as a yellow oil (1.40 g, 2.9 mmol, yield 11%). LCMS (ESI) m/z: 484 $[M+H]^+$

Step IV: Synthesis of 2-hydroxy-3-((S)-2-((6-oxo-5-(trifluoromethyl)-1-((2-(trimethylsilyl)ethoxy)methyl)-1, 6-dihydropyridazin-4-yl)amino)propoxy)propionic acid

To a mixed solvent of methanol (5.0 ml) and water (1.0 ml), ethyl 2-hydroxy-3-((S)-2-((6-oxo-5-(trifluoromethyl)-1-(2-(trimethylsilyl)ethoxy)methyl)-1, 6-dihydropyridazin-4-yl)amino)propoxy)propionate (1.40 g, 2.9 mmol) and lithium hydroxide monohydrate (121 mg, 2.9mmol) were added, and stirred at 0°C for 3 hrs. After complete reaction, the reaction solution was adjusted with 1 mol/l hydrochloric acid to pH 6, and extracted with ethyl acetate (20 ml*2). The organic phases were washed with saturated brine, dried over anhydrous sodium sulfate, and filtered. The filtrate was concentrated.

The crude product was purified by column chromatography on silica gel (dichloromethane : methanol=60: 40) to obtain 2-hydroxy-3-((S)-2-((6-oxo-5-(trifluoromethyl)-1-((2-(trimethylsilyl)ethoxy)methyl)-1, 6-dihydropyridazin-4-yl)amino)propoxy)propionic acid as a yellow oil (400 mg, 0.88 mmol, yield: 30%). LCMS (ESI) m/z: 456[M+H]$^+$

**Intermediate 3, Intermediate3-100, and Intermediate 3-200:** Synthesis of ethyl 2-hydroxy-3-((S)-2-((1-(4-methoxybenzyl)-6-oxo-5-(trifluoromethyl)-1, 6-dihydropyridazin-4-yl)amino)propoxy) propionate**(Intermediate 3),** ethyl (S)-2-hydroxy-3-((S)-2-((1-(4-methoxybenzyl)-6-oxo-5-(trifluoromethyl)-1, 6-dihydropyridazin-4-yl)amino)propoxy)propionate**(Intermediate 3-100)**, and ethyl (R)-2-hydroxy-3-((S)-2-((1-(4-methoxybenzyl)-6-oxo-5-(trifluoromethyl)-1, 6-dihydropyridazin-4-yl)amino)propoxy)propionate **(Intermediate 3-200)**

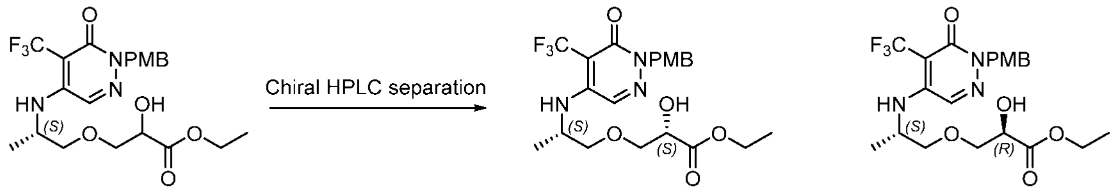


Intermediate 3 Intermediate 3-100 Intermediate 3-200

The synthesis of ethyl 2-hydroxy-3-((S)-2-((1-(4-methoxybenzyl)-6-oxo-5-(trifluoromethyl)-1, 6-dihydropyridazin-4-yl)amino)propoxy)propionate **(Intermediate 3)** was similar to that of **Intermediate 2.** Intermediate 3 (2.00 g, 4.23 mmol) was resolved by chiral high-performance liquid chromatography (chromatographic column: CHIRAL PAK IC Cellulose-SB, 2*25 cm, 5 μm, mobile phase A: methyl t-butyl ether (0.1% diethyl amine), mobile phase B: ethanol, flow rate: 20 ml/min, elution gradient: mobile phase B from 0% to 20% over 25 min, detection wavelength: 220 nm) to obtain ethyl (S)-2-hydroxy-3-((S)-2-((1-(4-methoxybenzyl)-6-oxo-5-(trifluoromethyl)-1, 6-dihydropyridazin-4-yl)amino)propoxy)propionate as a pale yellow oil (400 mg, 1.072 mmol, yield20%), and ethyl (R)-2-hydroxy-3-((S)-2-((1-(4-methoxybenzyl)-6-oxo-5-(trifluoromethyl)-1, 6-dihydropyridazin-4-yl)amino)propoxy)propionate as a pale yellow oil (300 mg, 0.804 mmol, yield 15%).

First peak **(Intermediate 3-100):** ethyl (S)-2-hydroxy-3-((S)-2-((1-(4-methoxybenzyl)-6-oxo-5-(trifluoromethyl)-1, 6-dihydropyridazin-4-yl)amino)propoxy)propionate

LCMS (ESI): 421 [M+H]$^+$

Chiral chromatography: chromatographic column: CHIRALPAK IC-3 4.6*50 mm,3 um; mobile phase A: n-hexane (0.1% diethyl amine), mobile phase B: ethanol; mobile phase A: mobile phase B=80:20; flow rate: 1.0 ml/min; temperature: 25°C; wavelength: 220/254 nm; retention time: 4.65 min.

Second peak **(Intermediate 3-200):** ethyl (R)-2-hydroxy-3-((S)-2-((1-(4-methoxybenzyl)-6-oxo-5-(trifluoromethyl)-1, 6-dihydropyridazin-4-yl)amino)propoxy)propionate

LCMS (ESI): 421 [M+H]$^+$

Chiral chromatography: chromatographic column: CHIRALPAK IC-3 4.6*50 mm, 3 um; mobile phase A: n-hexane (0.1% diethyl amine), mobile phase B: ethanol; mobile phase A: mobile phase B=80:20; flow rate: 1.0 ml/min; temperature: 25°C; wavelength: 220/254 nm; retention time: 5.76 min.

The stereochemistry of alcohols in the first and second peaks is arbitrary, that is,

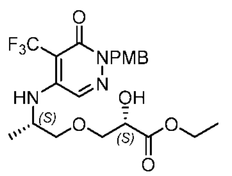 or 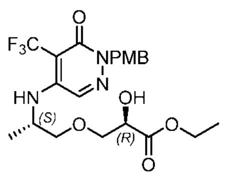

Intermediate 3-100

, or

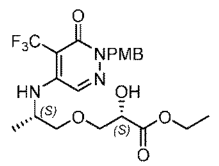 or 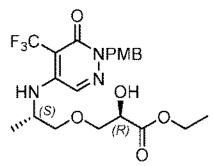

Intermediate 3-200

;

and this is also applicable to other similar situations.

**Intermediate 4:** Synthesis of 2-fluoro-3-((S)-2-((1-(4-methoxybenzyl)-6-oxo-5-(trifluoromethyl)-1, 6-dihydropyridazin-4-yl)amino)propoxy)propionic acid

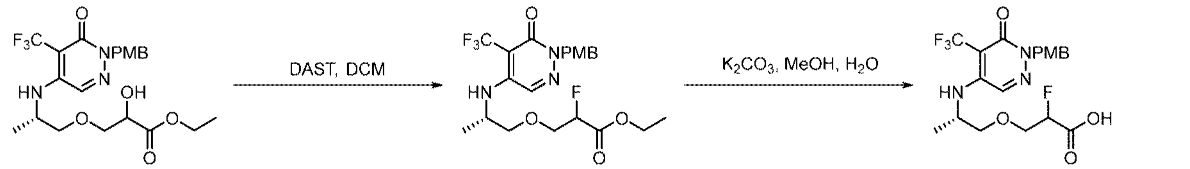


Intermediate 3 1 Intermediate 4

Step I: Synthesis of ethyl 2-fluoro-3-((S)-2-((1-(4-methoxybenzyl)-6-oxo-5-(trifluoromethyl)-1, 6-dihydropyridazin-4-yl)amino)propoxy)propionate

Ethyl 2-hydroxy-3-((S)-2-((1-(4-methoxybenzyl)-6-oxo-5-(trifluoromethyl)-1, 6-dihydropyridazin-4-yl)amino)propoxy) propionate **(Intermediate 3,** 1.02 g, 2.11 mmol) was dissolved in dichloromethane (30 ml). The system was cooled to -40°C, and then diethylaminosulfur trifluoride (1.71 g, 10.55 mmol) was diluted in dichloromethane (10 ml) and added dropwise to the reaction solution. The mixed solution was slowly warmed to room temperature and reacted continuously for 12 hrs. After the reaction was completed, the mixed solution was slowly poured into a saturated sodium bicarbonate solution, and extracted with dichloromethane (30 ml × 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (dichloromethane: methanol=80: 20) to obtain ethyl 2-fluoro-3-((S)-2-((1-(4-methoxybenzyl)-6-oxo-5-(trifluoromethyl)-1, 6-dihydropyridazin-4-yl)amino)propoxy)propionate as a yellow oil (300 mg, 0.63 mmol, yield 30%). LCMS (ESI): 476 $[M+H]^+$

Step II: Synthesis of 2-fluoro-3-((S)-2-((1-(4-methoxybenzyl)-6-oxo-5-(trifluoromethyl)-1, 6-dihydropyridazin-4-yl)amino)propoxy)propionic acid

Ethyl 2-fluoro-3-((S)-2-((1-(4-methoxybenzyl)-6-oxo-5-(trifluoromethyl)-1, 6-dihydropyridazin-4-yl)amino)propoxy)propionate (300 mg, 0.63 mmol) was dissolved in a mixed solvent of methanol (5ml) and water (1 ml), and then potassium carbonate (174 mg, 1.26 mol) was added. The mixed solution was reacted at room temperature for 3 hrs. After complete reaction, the reaction solution was adjusted with 1 mol/l dilute hydrochloric acid to pH 5, and extracted with ethyl acetate (20 ml × 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (dichloromethane: methanol=30: 70) to obtain 2-fluoro-3-((S)-2-((1-(4-methoxybenzyl)-6-oxo-5-(trifluoromethyl)-1, 6-dihydropyridazin-4-yl)amino)propoxy)propionic acid as ayellow oil (200 mg, 0.45 mmol, yield 71%). LCMS (ESI): 448 $[M+H]^+$

**Intermediate 5:** Synthesis of 8-benzyl-3-(trifluoromethyl)-6,6a,7,8,9,10-hexahydro-*5H*-pyrazino[1,2-a][1,8]naphthyridin-5-one

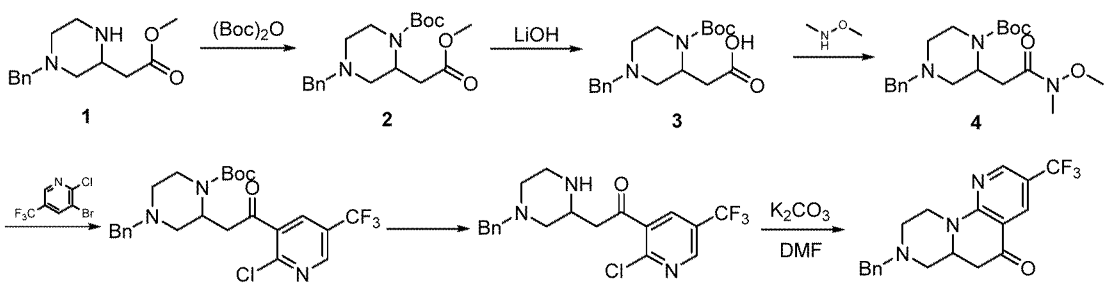


5 6 Intermediate 5

Step I: Synthesis of t-butyl 4-benzyl-2-(2-methoxy-2-oxyethyl)piperazin-1-carboxylate

Methyl 2-(4-benzylpiperazin-2-yl)acetate (5.00 g, 20.14 mmol) and sodium bicarbonate (3.38 g, 40.27 mmol) in a mixed solvent of dichloromethane (50 mL) and water (10 mL) were added to di-t-butyl dicarbonate (6.77 g, 30.20 mmol), and stirred at room temperature for 2 hrs. Then the product was poured into water (10 mL), extracted with dichloromethane (10 mL × 2), dried over anhydrous sodium sulfate, filtered under suction, rotary dried, and purified by column chromatography (petroleum ether/ethyl acetate =80: 20) to obtain the target product as a pale yellow oil (3.01 g, 32 mmol, yield: 60%). LCMS: MS(ESI)m/z: 349 $[M+H]^+$.

Step II: Synthesis of 2-(4-benzyl-1-(t-butoxycarbonyl)piperazin-2-yl)acetic acid

A mixture of t-butyl 4-benzyl-2-(2-methoxy-2-oxoethyl)piperazin-1-carboxylate (1.00g, 2.87mmol) and lithium hydroxide (0.23 g, 5.74 mmol) in tetrahydrofuran (10 mL) and water (2 mL) was stirred at room temperature for 2 hrs, then poured into water (10 mL), adjusted with formic acid to pH 3, and extracted with ethyl acetate (20 mL × 2). The organic phase was dried over anhydrous sodium sulfate, filtered under suction and rotary dried to obtain the target product as a white solid (0.60 g, 3.61 mmol, yield: 63%). LCMS: MS(ESI)m/z: 335 [M+H]+.

Step III: Synthesis of t-butyl 4-benzyl-2-(2-(methoxy(methyl)amino)-2-oxoethyl)piperazin-1-carboxylate

A solution of 2-(4-benzyl-1-(t-butoxycarbonyl)piperazin-2-yl)acetic acid (1.00 g, 2.99 mmol), N,O-dimethylhydroxylamine hydrochloride (0.29 g, 2.99 mmol), 1-ethyl-3(3-dimethylpropylamine)carbodiimide (1.14 g, 5.98 mmol), 1-hydroxybenzotriazole (1.90 g, 8.97 mmol) and NN-diisopropylethylene diamine (1.93 g, 14.95 mmol) in dichloromethane (10 mL) was stirred at room temperature for 12 hrs, then poured into water (10 mL), and extracted with dichloromethane (10 mL). The organic phase was dried over anhydrous sodium sulfate, filtered under suction, concentrated, and purified by column chromatography (petroleum ether: ethyl acetate =50: 50) to obtain the target product as a white oil (0.80 g, 2.1 mmol, yield: 71%). LCMS: MS(ESI)m/z: 378 $[M+H]^+$.

Step IV: Synthesis of t-butyl 4-benzyl-2-(2-(2-chloro-5-(trifluoromethyl)pyridin-3-yl)-2-oxyethyl)piperazin-1-carboxylate

To a mixture of t-butyl 4-benzyl-2-(2-(methoxy(methyl)amino)-2-oxoethyl)piperazin-1-carboxylate (1.00 g, 2.65 mmol) in diethyl ether (50 mL), n-butyl lithium (2.65 mL, 5.30 mmol, 2.5 mol/L solution in n-hexane) was added at -78°C, and then 3-bromo-2-chloro-5-(trifluoromethyl)pyridine (0.69 g, 2.65 mmol) was added. The mixture was stirred for 1 hr, then poured into saturated ammonium chloride (100 mL), and extracted with ethyl acetate (100 mL). The organic phase was dried over anhydrous sodium sulfate, filtered under suction, rotary dried, and purified by column chromatography (petroleum ether: ethyl acetate =75: 15) to obtain the target product as a red oil (0.80 g, 1.61 mmol, yield: 61%). LCMS: MS(ESI)m/z: 498 $[M+H]^+$.

Step V: Synthesis of 2-(4-benzylpiperazin-2-yl)-1-(2-chloro-5-(trifluoromethyl)pyridin-3-yl)ethyl-1-one

A mixture of t-butyl 4-benzyl-2-(2-(2-chloro-5-(trifluoromethyl)pyridin-3-yl)-2-oxoethyl)piperazin-1-carboxylate (3.00 g, 6.02 mmol) in hydrochloric acid/dioxane (10 mL) was stirred at room temperature for 2 hrs. The mixture was concentrated, to obtain the target product as a yellow oil (1.80 g, 4.51 mmol, yield: 75%), LCMS: MS(ESI)m/z: 398 $[M+H]^+$.

Step VI: Synthesis of 8-benzyl-3-(trifluoromethyl)-6,6a,7,8,9,10-hexahydro-*5H*-pyrazino[1,2-a][1,8]naphthyridin-5-one

A mixture of 2-(4-benzylpiperazin-2-yl)-1-(2-chloro-5-(trifluoromethyl)pyridin-3-yl)ethyl-1-one (0.50 g, 1.26 mmol) and potassium carbonate (0.35 g, 2.51 mmol) in DMF (5 mL) was stirred at 80°C for 2 hrs, then poured into water (10 mL), extracted with ethyl acetate (10 mL × 2), dried over anhydrous sodium sulfate, filtered under suction, concentrated, and purified by column chromatography (petroleum ether: ethyl acetate =70: 30) to obtain the target product as a yellow oil (0.22 g, 1.5 mmol, yield: 60%). LCMS: MS(ESI)m/z: 362 $[M+H]^+$.

**Intermediate 6 and Intermediate 7:** Synthesis of 3-((S)-1-hydroxyprop-2-yl)oxy)-1-((R)-3-(trifluoromethyl)-7a, 8, 10, 11-tetrahydro-5H-pyrazino[2, 1-c]pyrido[2, 3-e][1, 4]oxazepane-9(7H)-yl)prop-1-one **(Intermediate 6)** and 3-((S)-2-hydroxypropoxy)-1-((R)-3-(trifluoromethyl)-7a, 8, 10, 11-tetrahydro-5H-pyrazino[2, 1-c]pyrido[2, 3-e][1, 4]oxazepane-9(7H)-yl)prop-1-one **(Intermediate 7)**

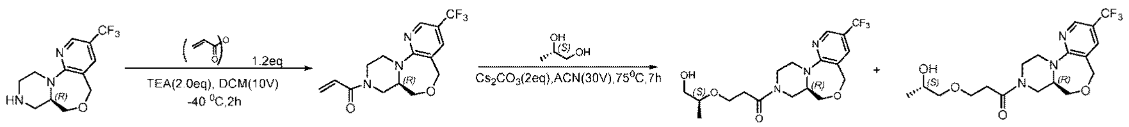


1 2 Intermediate 6 Intermediate 7

Step I: Synthesis of (R)-1-(3-(trifluoromethyl)-7a, 8, 10, 11-tetrahydro-5H-pyrazino[2, 1-c]pyrido[2, 3-e][1, 4]oxazepane-9(7H)-yl)prop-2-en-1-one

(R)-3-(trifluoromethyl)-7, 7a, 8, 9, 10, 11-hexahydro-5H-pyrazino[2, 1-c]pyrido[2, 3-e][1, 4]oxazepane (synthesized as described in **Example 5,** 3.50 g, 12.8 mmol) was added to dichloromethane (50 ml). Triethyl amine (3.33 g, 25.6 mmol) and acrylic anhydride (1.92 g, 15.36 mmol) were added to the mixture, and the system was stirred at -40°C for 2 hrs. After the reaction was completed, the mixture was poured into water (50 ml), and extracted with dichloromethane (25 ml*2). The organic phase was washed with saturated brine, dried over anhydrous sodium sulfate, and filtered. The filtrate was concentrated, and the obtained crude product was purified by column chromatography on silica gel (petroleum ether:ethyl acetate =40: 60) to obtain (R)-1-(3-(trifluoromethyl)-7a, 8, 10, 11-tetrahydro-5H-pyrazino[2, 1-c]pyrido[2, 3-e][1, 4]oxazepane-9(7H)-yl)prop-2-en-1-one as a yellow oil (1.71 g, 5.2 mmol, yield 41%). LCMS (ESI): 328 [M+H]+

Step II: Synthesis of 3-((S)-1-hydroxyprop-2-yl)oxy)-1-((R)-3-(trifluoromethyl)-7a, 8, 10, 11-tetrahydro-5H-pyrazino[2, 1-c]pyrido[2, 3-e][1, 4]oxazepane-9(7H)-yl)prop-1-one **(Intermediate 6)** and 3-((S)-2-hydroxypropoxy)-1-((R)-3-(trifluoromethyl)-7a, 8, 10, 11-tetrahydro-5H-pyrazino[2, 1-c]pyrido[2, 3-e][1, 4]oxazepane-9(7H)-yl)prop-1-one **(Intermediate 7)**

(R)-1-(3-(trifluoromethyl)-7a, 8, 10, 11-tetrahydro-5H-pyrazino[2, 1-c]pyrido[2, 3-e][1, 4]oxazepane-9(7H)-yl)prop-2-en-1-one (1.01 g, 3.05 mmol) and cesium carbonate (1.98 g, 6.1 mmol) were added to acetonitrile (30.00 ml). The mixture was stirred at 75°C for 7 hrs. After complete reaction, the mixture was concentrated under reduced pressure, then added to water (20 ml), and then extracted with ethyl acetate (15 ml*2). The organic phase was washed with saturated brine, dried over anhydrous sodium sulfate, and filtered. The filtrate was concentrated, and the obtained crude product was purified by reversed phase chromatography (acetonitrile: water =50: 50) to obtain 3-((S)-1-hydroxyprop-2-yl)oxy)-1-((R)-3-(trifluoromethyl)-7a, 8, 10, 11-tetrahydro-5H-pyrazino[2, 1-c]pyrido[2, 3-e][1, 4]oxazepane-9(7H)-yl)prop-1-one as a yellow oil (250 mg, 0.62 mmol, yield 20%) and 3-((S)-2-hydroxypropoxy)-1-((R)-3-(trifluoromethyl)-7a, 8, 10, 11-tetrahydro-5H-pyrazino[2, 1-c]pyrido[2, 3-e][1, 4]oxazepane-9(7H)-yl)prop-1-one as a yellow oil (500 mg, 1.24 mmol, yield 40%).

First peak **(Intermediate 6):** 3-((S)-1-hydroxyprop-2-yl)oxy)-1-((R)-3-(trifluoromethyl)-7a, 8, 10, 11-tetrahydro-5H-pyrazino[2, 1-c]pyrido[2, 3-e][1, 4]oxazepane-9(7H)-yl)prop-1-one

LCMS (ESI): 404 [M+H]+

HNMR (400 MHz, DMSO-d6): δ ppm 8.49-8.31 (m, 1H), 7.81-7.71(m, 1H), 4.92-4.75(m,1H), 4.61-4.41(m, 2H), 4.23-3.81(m, 2H), 3.92-3.83(m, 2H), 3.81-3.72 (m,2H), 3.70-3.62(m, 2H),3.62-3.33(m, 3H), 3.22-3.14(m, 3H), 2.73-2.55 (m, 2H),1.11-0.92 (m, 3H).

Second peak **(Intermediate 7):** 3-((S)-2-hydroxypropoxy)-1-((R)-3-(trifluoromethyl)-7a, 8, 10, 11-tetrahydro-5H-pyrazino[2, 1-c]pyrido[2, 3-e][1, 4]oxazepane-9(7H)-yl)prop-1-one

LCMS (ESI): 404 [M+H]+

HNMR (400 MHz, DMSO-d6): δ ppm 8.49-8.31 (m, 1H), 7.81-7.65(m, 1H), 4.92-4.81(m,1H), 4.61-4.41(m, 2H), 4.15-4.01(m, 2H), 4.01-3.92(m, 2H), 3.81-3.72 (m,2H), 3.70-3.62(m, 2H),3.62-3.33(m, 3H), 3.22-3.14(m, 3H), 2.73-2.55 (m, 2H),1.11-0.92 (m, 3H).

**Intermediates 8 and 9:** Synthesis of (S)-5,5-difluoro-3-(trifluoromethyl)-6,6a,7,8,9,10-hexahydro-5H-pyrazino[1,2-a][1,8]naphthyridine **(Intermediate 8)** and (R)-5,5-difluoro-3-(trifluoromethyl)-6,6a,7,8,9,10-hexahydro-5H-pyrazino[1,2-a][1,8]naphthyridine

## (Intermediate 9)

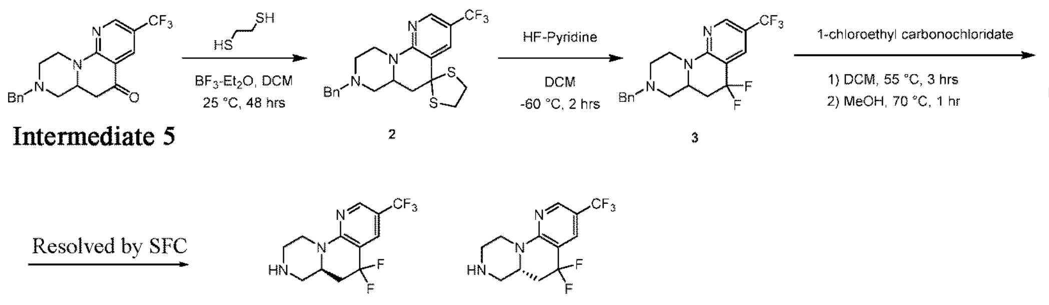


Intermediate 5 2 3 4

Intermediate 8 Intermediate 9

Step 1: Synthesis of 8-benzyl-3-(trifluoromethyl)-6,6a, 7,8,9,10-hexahydrospiro[pyrazino[1,2-a][1,8]naphthyridin-5,2'-[1,3]dithiolane]

Compound 8-benzyl-3-(trifluoromethyl)-6,6a, 7,8,9,10-hexahydro-5H-pyrazino[l,2-a][1,8]naphthyridin-5-one **(Intermediate 5,** 2.80 g, 7.75 mmol) was dissolved in dichloromethane (40 ml), and then ethanedithiol (2.19 g, 23.25 mmol, 1.96 mL) and boron trifluoride diethyl etherate (3.30 g, 23.3 mmol) were added. The reaction solution was reacted at 25 °C for 24 hrs. LC-MS showed that the raw materials were not completely consumed. Ethanedithiol (2.19 g, 23.25 mmol, 1.96 mL) and boron trifluoride diethyl etherate (3.30 g, 23.3 mmol) were added to the reaction solution, and the reaction solution was continuously reacted at 25°C for 24 hrs. LC-MS showed that the raw materials were completely consumed. Dichloromethane (40 ml) and 15% aqueous sodium hydroxide solution (80 ml) were added to the reaction solution. After extraction, the organic phase was collected, washed with saturated brine, dried over anhydrous sodium sulfate, and filtered. The filtrate was concentrated to obtain a crude product. The crude product was purified by column chromatography (petroleum ether/ethyl acetate =20/1) to obtain the product 8-benzyl-3-(trifluoromethyl)-6,6a, 7,8,9,10-hexahydrospiro[pyrazino[1,2-a][1,8]naphthyridin-5,2'-[1,3]dithiolane] as a yellow solid (2.30 g, yield: 67.8%). MS (ESI): m/z = 438.1[M+H]+

Step 2: Synthesis of 8-benzyl-5,5-difluoro-3-(trifluoromethyl)-6,6a,7,8,9,10-hexahydro-5H-pyrazino[1,2-a][1,8]naphthyridine

N-iodosuccinimide (2.37 g, 10.5 mmol) was suspended in dichloromethane (20 ml), and cooled to -60 °C. Under nitrogen atmosphere, hydrogen fluoride-pyridine complex (2.98 g, 21.0 mmol, 70%) was added, and reacted at -60 °C for 1 hr. The compound 8-benzyl-3-(trifluoromethyl)-6,6a, 7,8,9,10-hexahydrospiro[pyrazino[1,2-a][1,8]naphthyridin-5,2'-[1,3]dithiolane (2.30 g, 5.26 mmol) was dissolved in dichloromethane (20 ml), added to the reaction solution at -60 °C, and reacted at -60°C for 1 hr. LC-MS showed that the raw materials were completely consumed, and the product was produced. The reaction solution was added with dichloromethane (150 ml), and washed with saturated sodium sulfite solution and sodium carbonate solution. The organic phase was collected, dried over anhydrous sodium sulfate, and filtered. The filtrate was concentrated to obtain a crude product. The crude product was purified by column chromatography (petroleum ether/ethyl acetate = 20/1) to obtain the product 8-benzyl-5,5-difluoro-3-(trifluoromethyl)-6,6a,7,8,9,10-hexahydro-5H-pyrazino[1,2-a][1,8]naphthyridine as a pale yellow solid (900.0 mg, yield : 44.7%). MS (ESI): m/z = 384.1[M+H]+

Step 3: Synthesis of (S)-5,5-difluoro-3-(trifluoromethyl)-6,6a,7,8,9,10-hexahydro-SH-pyrazino[1,2-a][1,8]naphthyridine and (R)-5,5-difluoro-3-(trifluoromethyl)-6,6a,7,8,9,10-hexahydro-5H-pyrazino[1,2-a][1,8]naphthyridine

The compound 8-benzyl-5,5-difluoro-3-(trifluoromethyl)-6,6a,7,8,9,10-hexahydro-SH-pyrazino[1,2-a][1,8]naphthyridine (900.0 mg, 2.35 mmol) was dissolved in dichloromethane (10 ml), and cooled to 0°C. Under nitrogen atmosphere, chloroethyl 1-chloroformate (503.5 mg, 3.52 mmol) was added. The reaction was continued at 55 °C for 3 hrs, until TLC showed that most raw materials were reacted and a new product was produced. The reaction solution was concentrated to obtain a yellow oil, which was dissolved in methanol (8 ml), and reacted at 70°C for 1 hr. The product was produced as detected by LC-MS. The reaction solution was concentrated to obtain a crude product. The crude product was purified by column chromatography (dichloromethane /methanol = 20/1) to obtain the product (235.0 mg). The combined product (500.9 mg in total) was chirally resolved (chiral chromatographic column: DAICEL CHIRALPAK AD (250 mm * 30 mm, 10 um); mobile phase A: $CO_2$, mobile phase B: ethanol (0.1% $NH_3H_2O$); mobile phase A:mobile phase B = 20%; flow rate: 80 ml/min; temperature: 38°C; wavelength: 220/254 nm) to obtain the product (S)-5,5-difluoro-3-(trifluoromethyl)-6,6a,7,8,9,10-hexahydro-5H-pyrazino[1,2-a][1,8]naphthyridine as a pale yellow solid (194.2 mg), and the product (R)-5,5-difluoro-3-(trifluoromethyl)-6,6a,7,8,9,10-hexahydro-5H-pyrazino[1,2-a][1,8]naphthyridine as a pale yellow solid (196.6 mg).

MS (ESI): m/z = 294.1 $[M+H]^+$.

$^1H$ NMR (400MHz, CHLOROFORM-d) δ 8.46 (s, 1H), 7.94 (s, 1H), 4.83 - 4.76 (m, 1H), 3.48 (br t, J=11.4 Hz, 1H), 3.23 - 3.14 (m, 2H), 2.90 - 2.75 (m, 2H), 2.67 (t, J=10.9 Hz, 1H), 2.46 - 2.35 (m, 1H), 2.17 - 2.01 (m, 1H).

First peak **(Intermediate 8):** Chiral chromatography: chromatographic column: ChiralPak AD-3, 150 × 4.6 mm I.D., 3 μm; mobile phase A: $CO_2$, mobile phase B: ethanol (0.05% DEA); mobile phase A: mobile phase B= 5% to 40% over 4.5 min; and then maintained at 5% B over 1.5 min, flow rate: 2.5 ml/min; temperature: 40°C; wavelength: 220/254 nm; retention time: 1.94 min.

Second peak **(Intermediate 9):** Chiral chromatography: chromatographic column: ChiralPak AD-3, 150 × 4.6 mm I.D., 3 μm; mobile phase A: $CO_2$, mobile phase B: ethanol (0.05% DEA); mobile phase A: mobile phase B = 5% to 40% over 4.5 min; and then maintained at 5% B over 1.5 min, flow rate: 2.5 ml/ min; temperature: 40°C; wavelength: 220/254 nm; retention time: 2.61 min.

The stereochemistry in the first and second peaks is arbitrary, that is

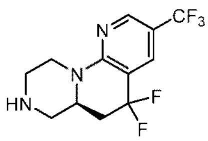 or 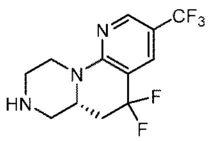

Intermediate 8

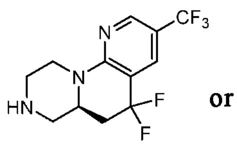 or 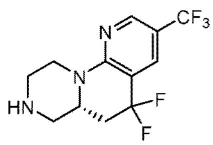

Intermediate 9

.

**Intermediate 10:** Synthesis of t-butyl (R)-3-(acetyloxymethyl)-4-(4-iodo-5-(trifluoromethyl)pyridin-2-yl)piperazin-1-carboxylate

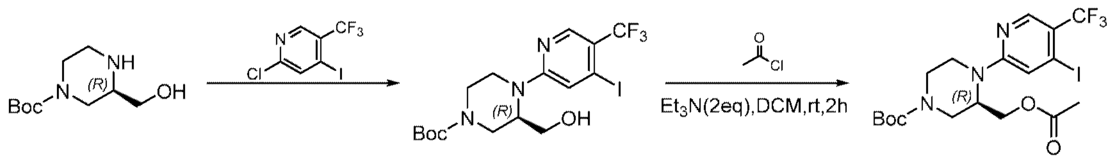


1

Intermediate 10

Step I: Synthesis of t-butyl (R)-3-(hydroxymethyl)-4-(4-iodo-5-(trifluoromethyl)pyridin-2-yl)piperazin-1-carboxylate

t-butyl 3-(hydroxymethyl)piperazin-1-carboxylate (13.00 g, 60.11 mmol) was added to N,N-dimethylformamide (260.00 ml). Then, 2-chloro-4-iodo-5-(trifluoromethyl)pyridine (22.18 g, 72.13 mmol) was added, followed by potassium carbonate (16.61 g, 120.22 mmol). The mixture was stirred at 100°C for 4 hrs. After the reaction was completed, the system was cooled to room temperature. The reaction solution was poured into water (500 ml), and then extracted with ethyl acetate (100 ml*3). The organic phases were combined, washed with saturated brine, dried over anhydrous sodium sulfate, and filtered. The filtrate was concentrated. The crude product was purified by column chromatography (ethyl acetate:petroleum ether = 40:60) to obtain t-butyl (R)-3-(hydroxymethyl)-4-(4-iodo-5-(trifluoromethyl)pyridin-2-yl)piperazin-1-carboxylate as a yellow solid (6.50 g, 13.34 mmol, yield 22%). LCMS (ESI) m/z: 488$[M+H]^+$.

Step II: Synthesis of t-butyl (R)-3-(acetyloxymethyl)-4-(4-iodo-5-(trifluoromethyl)pyridin-2-yl)piperazin-1-carboxylate

t-butyl (R)-3-(hydroxymethyl)-4-(4-iodo-5-(trifluoromethyl)pyridin-2-yl)piperazin-1-carboxylate (6.00 g, 12.31 mmol) was added to dichloromethane (120.00 ml), and then triethyl amine (1.93g, 24.63mmol) was added. The mixed solution was cooled to 0°C in an ice-water bath, and then acetyl chloride (1.50 g, 14.78 mmol) was slowly added dropwise. Subsequently, the mixture was stirred at room temperature for 2 hrs. After the reaction was completed, the reaction solution was quenched by pouring into ice water (100 ml), and then extracted with dichloromethane (100 ml*2). The organic phases were combined, washed with saturated brine, dried over anhydrous sodium sulfate, and filtered. The filtrate was concentrated. The crude product was purified by column chromatography (ethyl acetate:petroleum ether = 40:60) to obtain t-butyl (R)-3-(acetyloxymethyl)-4-(4-iodo-5-(trifluoromethyl)pyridin-2-yl)piperazin-1-carboxylate as a yellow solid (4.00 g, 7.56 mmol, yield 61%). LCMS (ESI) m/z: 530[M+H]+.

**Specific Examples**

**Example 1:** Synthesis of 5-(((S)-1-(3-oxo-3-((R)-3-(trifluoromethyl)-6a,7,9,10-tetrahydropyrazino[1,2-d]pyrido[3,2-b][1,4]oxazin-8(6H)-yl)propoxy)prop-2-yl)amino)-4-(trifluoromethyl)pyridazin-3(2H)-one **(Compound 1)**

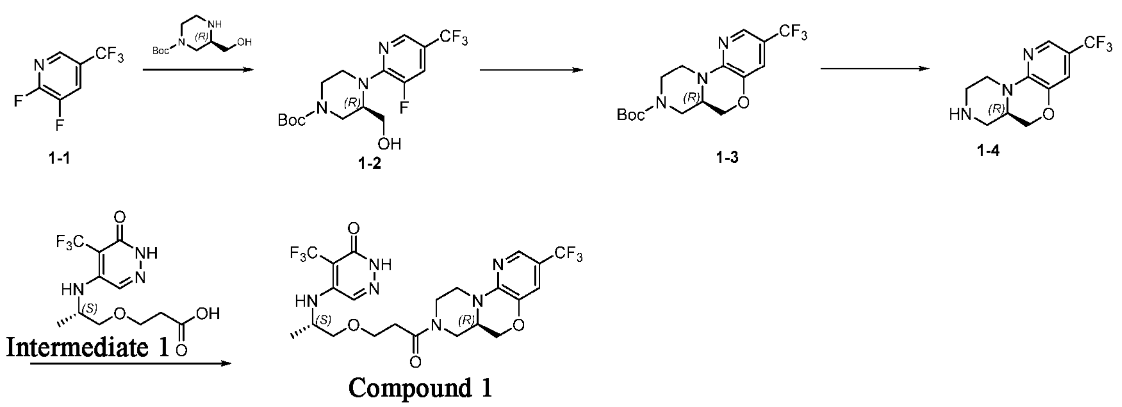

1-1 1-2 1-3 1-4

Compound 1

Step I: Synthesis of t-butyl (3R)-4-[3-fluoro-5-(trifluoromethyl)pyridin-2-yl]-3-(hydroxymethyl)piperazin-1-carboxylate **(1-2)**

A mixture of t-butyl (3R)-3-(hydroxymethyl)piperazin-1-carboxylate (1.0 g, 4.624 mmol) and 2,3-difluoro-5-(trifluoromethyl)pyridine (0.85 g, 4.624 mmol) was dissolved in DMF (2 mL), and then $K_2CO_3$ (0.263 g, 4.624 mmol) was added. The mixture was heated to 80°C under nitrogen atmosphere, and reacted for 12 hrs with stirring. The reaction mixture was cooled and then filtered. The filtrate was concentrated under reduced pressure, added with water (30 mL), extracted with ethyl acetate, and dried over anhydrous sodium sulfate. Anhydrous sodium sulfate was removed by filtration, and the filtrate was rotary dried. The crude product was purified by column chromatography on silica gel (petroleum ether: ethyl acetate =85:15) to obtain the target product as a white solid (600 mg, 0.319 mmol, yield: 70%). LCMS: MS(ESI) m/z: 380 $[M+H]^+$ .

Step II: Synthesis of t-butyl (10R)-3-(trifluoromethyl)-6a,7,9,10-tetrahydropyrazino[1,2-d]pyrido[3,2-b][1,4]oxazin-8(6H)-carboxylate **(1-3)**

t-butyl (3R)-4-[3-fluoro-5-(trifluoromethyl)pyridin-2-yl]-3-(hydroxymethyl)piperazin-1-carboxylate (500 mg, 1.318 mmol) was dissolved in DMF (5 mL), and then potassium tert-butoxide (443 mg, 3.95 mmol) was added. The mixture was heated to 80°C under nitrogen atmosphere, and reacted for 12 hrs with stirring. The reaction solution was cooled, added with ice water (10 mL), and extracted with ethyl acetate (10 mL × 3). The organic phase was dried over anhydrous sodium sulfate. Anhydrous sodium sulfate was removed by filtration, and rotary dried. The crude product was purified by column chromatography on silica gel (petroleum ether: ethyl acetate =75:25) to obtain the target product as a white solid (400 mg, 1.113 mmol, 84.45%). LCMS: MS(ESI) m/z: 360 $[M+H]^+$.

Step III: Synthesis of (R)-3-(trifluoromethyl)-6,6a,7,8,9,10-hexahydropyrazino[1,2-d]pyrido[3,2-b][1,4]oxazine **(1-4)**

t-butyl (10R)-3-(trifluoromethyl)-6a,7,9,10-tetrahydropyrazino[1,2-d]pyrido[3,2-b][1,4]oxazin-8(6H)-carboxylate (500 mg, 1.391 mmol) was dissolved in dichloromethane (5 mL), and then trifluoroacetic acid (3 mL) was added. The reaction mixture was reacted for 2 hrs at room temperature with stirring. After complete reaction, the reaction solution was concentrated under reduced pressure. The crude product was diluted in water (30 mL), adjusted to pH 8-9 with sodium bicarbonate, and extracted with dichloromethane (20 mL × 3). The crude product was purified by column chromatography on silica gel (petroleum ether: ethyl acetate =15:85) to obtain the target product as a white solid (300 mg, 1.157 mmol, yield: 83%), LCMS: MS(ESI)m/z: 260 $[M+H]^+$.

Step IV: Synthesis of 5-(((S)-1-(3-oxo-3-((R)-3-(trifluoromethyl)-6a,7,9,10-tetrahydropyrazino[1,2-d]pyrido[3,2-b][1,4]oxazin-8(6H)-yl)propoxy)prop-2-yl)amino)-4-(trifluoromethyl)pyridazin-3(2H)-one **(Compound 1)**

3-[(2S)-2-{[6-oxo-5-(trifluoromethyl)-1,6-dihydropyridazin-4-yl]amino}propoxy]propionic acid (100 mg, 0.323 mmol) **(Intermediate 1)** and (R)-3-(trifluoromethyl)-6,6a,7,8,9,10-hexahydropyrazino[1,2-d]pyrido[3,2-b][1,4]oxazine (83.83 mg, 0.323 mmol) **(1-4)** were dissolved in dichloromethane (1.0 mL), and then diisopropylethyl amine (0.053 mL, 0.323 mmol) and T3P ( 102.89 mg, 0.323 mmol, 50% solution in ethyl acetate) were added. The reaction mixture was stirred at room temperature for 2 hrs. The reaction solution was poured into ice water (20 mL), and the aqueous phase was then extracted with ethyl acetate (10 mL × 3). The organic phases were combined and dried over anhydrous sodium sulfate. Anhydrous sodium sulfate was removed by filtration, and the filtrate was rotary dried to obtain a crude product. The crude product was purified by preparative liquid-phase chromatography (column: XBridge Prep OBD C18, 30 × 150 mm 5 um, mobile phase A: water (containing 10 mol/l ammonium bicarbonate), mobile phase B: acetonitrile, flow rate: 60 ml/ min, elution gradient: mobile phase B from 20% to 52% over 8 min, detection wavelength: 220 nm, retention time: 7.28 min) to obtain the target product as a white solid (50 mg, 0.091 mmol, yield: 28.09%).

$^1$H NMR (400 MHz, DMSO-$d_6$) $\delta$ (ppm): 12.45 (s, 1H), 8.07 (s, 1H), 7.91 (s, 1H), 7.29 (d, *J*= 2.2 Hz, 1H), 6.27-6.26 (m, 1H), 4.48-4.38 (m, 3H), 4.17-4.13 (m, 1H), 4.05-3.95 (m, 2H), 3.69-3.67 (m, 2H), 3.51-3.48 (d, *J* = 5.5 Hz, 2H), 3.20-3.09(m,1H), 2.91-2.84 (m, 1H), 2.81- 2.69 (m, 1H), 2.68-2.57 (m, 3H), 1.16-1.14 (d, *J*= 6.5 Hz, 3H). LCMS: MS(ESI)m/z: 551.00 $[M+H]^+$.

**Example 2:** Synthesis of 5-(((S)-1-(3-oxo-3-((S)-3-(trifluoromethyl)-6a,7,9,10-tetrahydropyrazino[1,2-d]pyrido[3,2-b][1,4]oxazin-8(6H)-yl)propoxy)prop-2-yl)amino)-4-(trifluoromethyl)pyridazin-3(2H)-one **(Compound 2)**

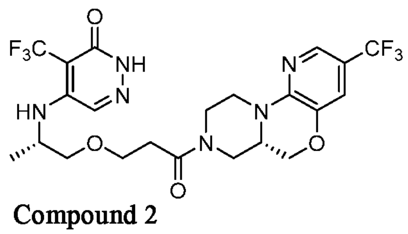

Compound 2

The synthesis method was the same as that in **Example 1,** except that t-butyl (3S)-3-(hydroxymethyl)piperazin-1-carboxylate was used in place of the reaction raw material t-butyl (3R)-3-(hydroxymethyl)piperazin-1-carboxylate in Step I for reaction. **Compound 2** as a crude product was purified by preparative liquid-phase chromatography (column: XBridge Prep OBD C18, 30 × 150 mm 5 um, mobile phase A: water (containing 10 mol/l ammonium bicarbonate), mobile phase B: acetonitrile, flow rate: 60 ml/ min, elution gradient: mobile phase B from 20% to 52% over 8 min, detection wavelength: 220 nm, retention time: 7.28 min) to obtain the target product as a white solid (50 mg, 0.091 mmol, yield: 23.00%).

$^1$HNMR(400 MHz, DMSO-$d_6$) $\delta$ (ppm): 12.46 (s, 1H), 8.07 (s,1H), 7.91 (s, 1H), 7.29-7.28 (d, *J*= 2.1 Hz, 1H), 6.28-6.25 (m, 1H), 4.51-4.38 (m, 3H), 4.18-4.13 (m, 1H), 4.05-3.95 (m, 2H), 3.73-3.64 (m, 2H), 3.49-3.48 (d, *J* = 5.5 Hz, 2H), 3.17-3.11 (m, 1H), 3.01-2.85 (m,1H), 2.79-2.71 (m, 1H), 2.68-2.62 (m, 2H), 2.45-2.41 (m, 1H), 1.16-1.14 (d, *J*= 6.5 Hz, 3H). LCMS: MS(ESI)m/z: 551.00 $[M+H]^+$.

**Example 3:** Synthesis of (R) -9-(3-((S)-2-((6-oxo-5-(trifluoromethyl)-1,6-dihydropyridazin-4-yl)amino)propoxy)propionyl)-3-(trifluoromethyl)-7, 7a, 8,9,10,11-hexahydro-5H-pyrazino[2,1-c]pyrido[2,3-e][1,4]oxazin-5-one **(Compound 3)**

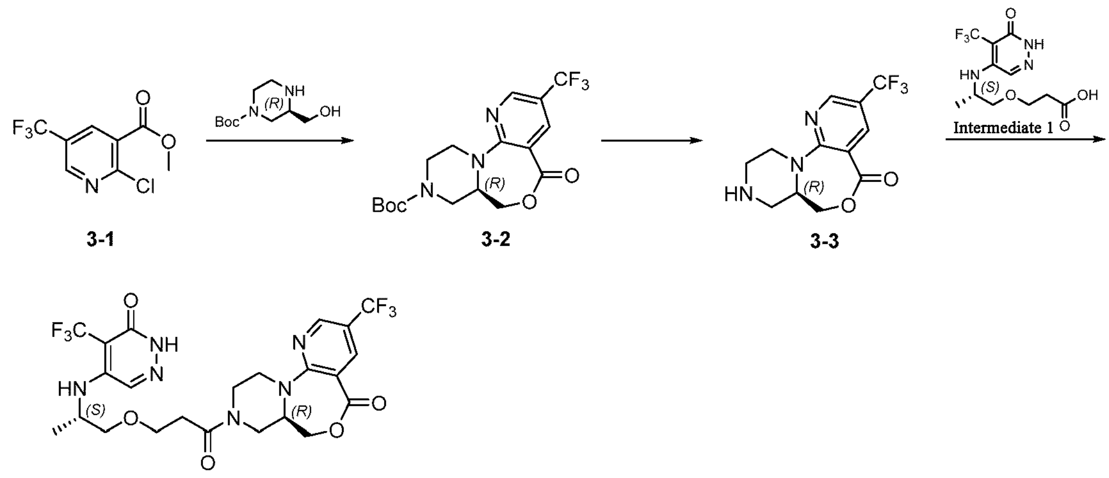


3-1 3-2 3-3

Compound 3

Step I: Synthesis of t-butyl (R)-5-oxo-3-(trifluoromethyl)-7a,8, 10, 11-tetrahydro-5H-pyrazino[2,1-c]pyrido[2,3-e][1,4]oxazolin-9(7H)-carboxylate **(3-2)**

Methyl 2-chloro-5-trifluoromethylpyridin-3-carboxylate (100 mg, 0.417 mmol) **(3-1)** and t-butyl (3R)-3-hydroxymethyl-piperidin-1-carboxylate (91 mg, 0.417 mmol) were dissolved in DMF (2 mL), and then potassium carbonate (115 mg, 0.84 mmol) was added. The reaction mixture was reacted for 12 hrs at 80°C with stirring. After cooling, the solid was filtered off. The filtrate was concentrated to dryness, and the crude product was added to water (50 mL), and extracted with ethyl acetate (20 mL × 3). The organic phases were combined, and dried over anhydrous sodium sulfate. Anhydrous sodium sulfate was removed by filtration, and the filtrate was rotary dried. The crude product was purified by column chromatography on silica gel (petroleum ether:ethyl acetate =54:46) to obtain the target product as a white solid (120 mg, 0.310 mmol, yield: 74%). LCMS: MS(ESI)m/z: 388 $[M+H]^+$.

Step II: Synthesis of (R) -3-(trifluoromethyl)-7,7a,8,9, 10, 11-hexahydro-5H-pyrazino[2,1-c]pyrido[2,3-e][1,4]Oxazepane-5-one **(3-3)**

t-butyl (R)-5-oxo-3-(trifluoromethyl)-7a,8,10,11-tetrahydro-5H-pyrazino[2,1-c]pyrido[2,3-e][1,4]oxazolin-9(7H)-carboxylate **(3-2)** (500 mg, 1.29 mmol) was dissolved in ice-cold (0°C) hydrogen chloride solution (4 M) (5 mL) in 1,4-dioxane. The reaction mixture was slowly warmed to room temperature and reacted for 2 hrs with stirring. After complete reaction, the reaction solution was concentrated under reduced pressure. The crude product was diluted in water (30 mL), adjusted to pH 8-9 with sodium bicarbonate, and extracted with dichloromethane (20 mL × 3). The crude product was purified by column chromatography on silica gel (petroleum ether: ethyl acetate =15:85) to obtain the target product as a white solid (250 mg, 0.87 mmol, yield: 67%), LCMS: MS(ESI)m/z: 288 $[M+H]^+$.

Step III: Synthesis of (R) -9-(3-((S)-2-((6-oxo-5-(trifluoromethyl)-1,6-dihydropyridazin-4-yl)amino)propoxy)propionyl)-3-(trifluoromethyl)-7,7a,8,9,10,11-hexahydro-5H-pyrazino[2,1-c]pyrido[2,3-e][1,4]oxazin-5-one **(Compound 3)**

3-[(2S)-2-{[6-oxo-5-(trifluoromethyl)-1,6-dihydropyridazin-4-yl]amino}propoxy]propionic acid (108 mg, 0.348 mmol) **(Intermediate 1)** and the compound (S)-3-(trifluoromethyl)-6,6a,7,8,9,10-hexahydropyrazino[1,2-d]pyrido[3,2-b][1,4]oxazine (100 mg, 0.348 mmol) **(3-3)** was dissolved in dichloromethane (2 .0 mL), and then diisopropylethyl amine (90 mg, 0.696 mmol) and T3P (112 mg, 0.418 mmol, 50% solution in ethyl acetate) were added. The reaction mixture was stirred at room temperature for 2 hrs. The reaction solution was poured into ice water (20 mL), and the aqueous phase was then extracted with ethyl acetate (10 mL × 3). The organic phases were combined and dried over anhydrous sodium sulfate. Anhydrous sodium sulfate was removed by filtration, and the filtrate was rotary dried to obtain a crude product. The crude product was purified by preparative liquid-phase chromatography (column: XBridge Prep OBD C18, 30 × 150 mm 5 um, mobile phase A: water (containing 10 mol/l ammonium bicarbonate), mobile phase B: acetonitrile, flow rate: 60 ml/ min, elution gradient: mobile phase B from 20% to 52% over 8 min, detection wavelength: 220 nm, retention time: 6.95 min) to obtain the target product as a white solid (52 mg, 0.090 mmol, yield: 25.01%).

$^1H$ NMR (400 MHz, DMSO-$d_6$) δ (ppm): 12.45 (s, 1H), 8.75 (s, 1H), 8.14 (d, *J*= 2.2 Hz, 1H), 7.92 (s, 1H), 6.28-6.26 (m, 1H), 4.67-4.63 (m, 1H), 4.54-4.48 (m, 1H), 4.47-4.33 (m, 2H), 4.19-4.11 (m, 1H), 4.04-3.91(m,1H), 3.78-3.65(m, 3H), 3.50-3.49 (d, *J* = 2.2 Hz, 2H), 3.25-3.16(m,1H),3.13-2.95(m,1H), 2.83 -2.56 (m, 3H), 1.15 (d, *J* = 6.5

Hz, 3H). LCMS: MS(ESI)m/z: **579.05** $[M+H]^+$.

**Example 4:** Synthesis of (S) -9-(3-((S)-2-((6-oxo-5-(trifluoromethyl)-1,6-dihydropyridazin-4-yl)amino)propoxy)propionyl)-3-(trifluoromethyl)-7, 7a, 8,9,10,11-hexahydro-5H-pyrazino[2,1-c]pyrido[2,3-e][1,4]oxazin-5-one **(Compound 4)**

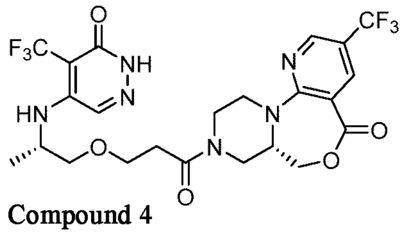

Compound 4

The synthesis method was the same as that in **Example 3,** except that t-butyl (3S)-3-(hydroxymethyl)piperazin-1-carboxylate was used in place of the reaction raw material t-butyl (3R)-3-(hydroxymethyl)piperazin-1-carboxylate in Step I for reaction. **Compound 4** as a crude product was purified by high performance liquid chromatography (column: XBridge Prep OBD C18, 30 × 150mm 5um, mobile phase A: water (containing 10 mol/l ammonium bicarbonate), mobile phase B: acetonitrile, flow rate: 60 ml/ min, elution gradient: mobile phase B from 20% to 52% over 8 min, detection wavelength: 220nm, retention time: 7.13 min) to obtain the target product as a white solid (20 mg, 0.035 mmol, yield: 53%).

$^1H$ NMR (400 MHz, DMSO-$d_6$) δ (ppm): 12.45 (s, 1H), 8.75 (s, 1H), 8.14 (d, *J* = 2.5 Hz, 1H), 7.92 (s, 1H), 6.34-6.20 (m, 1H), 4.67-4.63 (m, 1H), 4.53-4.50 (m, 1H), 4.43 - 4.33 (m, 2H), 4.17-4.13 (m, 1H), 4.04-3.91 (m, 1H), 3.80-3.67 (m, 3H), 3.50 - 3.49 (m, 2H), 3.22-3.16 (m, 1H), 3.08-2.95 (m, 1H), 2.81 - 2.62 (m, 3H), 1.16 (d, *J* = 6.4 Hz, 3H). LCMS: MS(ESI)m/z: **579.15** $[M+H]^+$.

**Example 5:** Synthesis of 5-(((S)-1-(3-oxo-3-((R)-3-(trifluoromethyl)-7a,8,10,11-tetrahydro-5H-pyrazino[2,1-c]pyrido[2,3-e][1,4]Oxazepan-9(7H)-yl)propoxy)prop-2-yl)amino)-4-(trifluoromethyl)pyridazin-3(2H)-one **(Compound 5)**

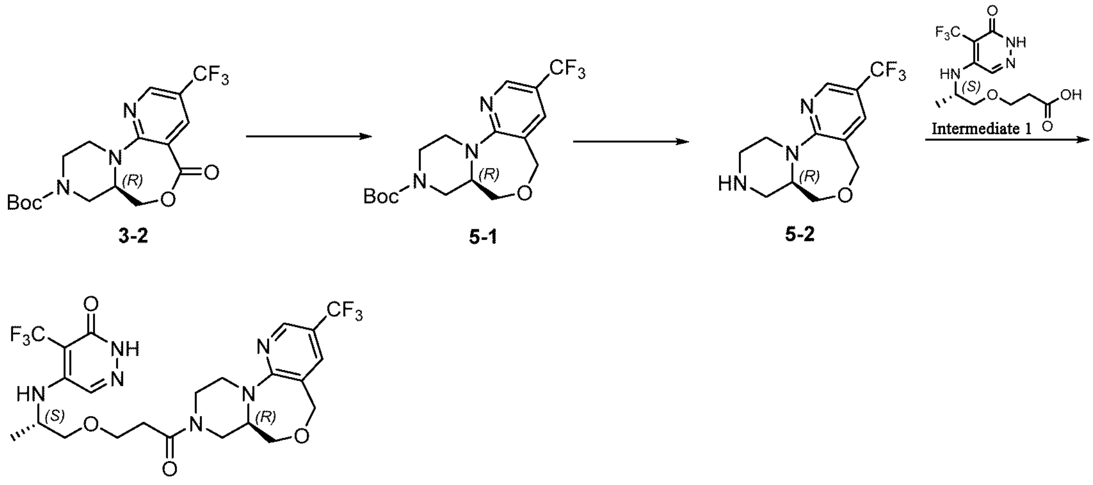


3-2

5-1

5-2

Compound 5

Step I: Synthesis of t-butyl (R)-3-(trifluoromethyl)-7a,8, 10, 11-tetrahydro-5H-pyrazino[2,1-c]pyrido[2,3-e][1,4]oxazolin-9(7H)-carboxylate (5-1)

t-butyl (R)-5-oxo-3-(trifluoromethyl)-7a,8,10,11-tetrahydro-5H-pyrazino[2,1-c]pyrido[2,3-e][1,4]oxazolin-9(7H)-carboxylate **(3-2)**(500 mg, 1.29 mmol) wad dissolved in tetrahydrofuran (5 mL), and cooled to 0°C. Then solid sodium borohydride (50 mg, 1.295 mmol) was added, and then boron trifluoride-diethyl etherate (185 mg, 1.292 mmol) was added dropwise with stirring. After that, the reaction mixture was reacted with stirring at room temperature for 2 hrs. The reaction solution was poured into ice water (10 ml), and the aqueous phase was extracted with dichloromethane

(20 ml × 3). The organic phases were combined, and dried over anhydrous sodium sulfate. Anhydrous sodium sulfate was removed by filtration, and the crude product was purified by column chromatography on silica gel (petroleum ether: ethyl acetate =25:75) to obtain the target product as a white solid (120 mg, 0.321 mmol, yield: 24%). LCMS: MS(ESI)m/z: 374 $[M+H]^+$.

Step II: Synthesis of (R) -3-(trifluoromethyl)-7,7a,8,9,10,11-hexahydro-5H-pyrazino[2,1-c]pyrido[2,3-e][1,4]Oxazepane **(5-2)**

t-butyl (R)-3-(trifluoromethyl)-7a,8,10,11-tetrahydro-5H-pyrazino[2,1-c]pyrido[2,3-e][1,4]oxazolin-9(7H)-carboxylate **(5-1)** (100 mg, 0.268 mmol) was dissolved in ice-cold (0°C) hydrogen chloride solution (4 M, 5 mL) in 1,4-dioxane, slowly warmed to room temperature, and reacted with stirring for 2 hrs. After complete reaction, the reaction solution was concentrated under reduced pressure. The crude product was diluted in water (30 mL), adjusted to pH 8-9 with sodium bicarbonate, and extracted with dichloromethane (20 mL × 3). The crude product was purified by column chromatography on silica gel (petroleum ether: ethyl acetate =34:66) to obtain the target product as a white solid (70 mg, 0.256 mmol, yield: 95%). LCMS: MS (ESI) m/z: 274$[M+H]^+$.

Step III: Synthesis of 5-(((S)-1-(3-oxo-3-((R)-3-(trifluoromethyl)-7a,8,10,11-tetrahydro-5H-pyrazino[2,1-c]pyrido[2,3-e][1,4]Oxazepan-9(7H)-yl)propoxy)prop-2-yl)amino)-4-(trifluoromethyl)pyridazin-3(2H)-one **(Compound 5)**

3-[(2S)-2-{[6-oxo-5-(trifluoromethyl)-1,6-dihydropyridazin-4-yl]amino}propoxy]propionic acid (10 mg, 0.032 mmol) **(Intermediate 1)** and the compound (R)-3-(trifluoromethyl)-7,7a,8,9,10,11-hexahydro-5H-pyrazino[2,1-c]-pyrido[2,3-e][1,4]Oxazepane **(5-2)** (8.84 mg, 0.032 mmol) were dissolved in dichloromethane (2 mL). Then diisopropylethyl amine (10 mg, 0.064 mmol) and T3P (21 mg, 0.064 mmol) were sequentially added, and the reaction solution was reacted at room temperature with stirring for 2 hrs. The reaction solution was concentrated under reduced pressure, and the crude product was purified by high performance liquid chromatography (column: XBridge Prep OBD C18, 30 × 150 mm, 5 um; mobile phase A: water (10 mmol/L sodium bicarbonate), mobile phase B: acetonitrile; flow rate: 60 mL/min; gradient: mobile phase B from 20% to 52% over 8 min, detection wavelength: 220 nm; retention time: 6.85 min) to obtain the target product as a white solid (11 mg, 0.019 mmol, yield: 59%).

$^1H$ NMR (400 MHz, DMSO-$d_6$) δ (ppm): 12.45 (s, 1H), 8.42-8.38 (m, 1H), 7.91-7.90 (d, *J* = 5.1 Hz, 1H), 7.81-7.77 (m , 1H), 6.26-6.25 (m, 1H), 4.94-4.86 (m, 1H),4.55-4.49 (m, 1H), 4.14 (brs, 1H), 4.02 - 3.77 (m, 5H), 3.75 - 3.57 (m, 5H), 3.56 - 3.41 (m, 3H), 2.62 - 2.54 (m, 2H), 1.16-1.13 (t, *J*= 6.3 Hz, 3H). LCMS: MS (ESI) m/z: **565.10** $[M+H]^+$.

**Example 6:** Synthesis of 5-(((S)-1-(3-oxo-3-((S)-3-(trifluoromethyl)-7a,8,10,11-tetrahydro-5H-pyrazino[2,1-c]pyrido[2,3-e][1,4]Oxapzepan-9(7H)-yl)propoxy)prop-2-yl)amino)-4-(trifluoromethyl)pyridazin-3(2H)-one **(Compound 6)**

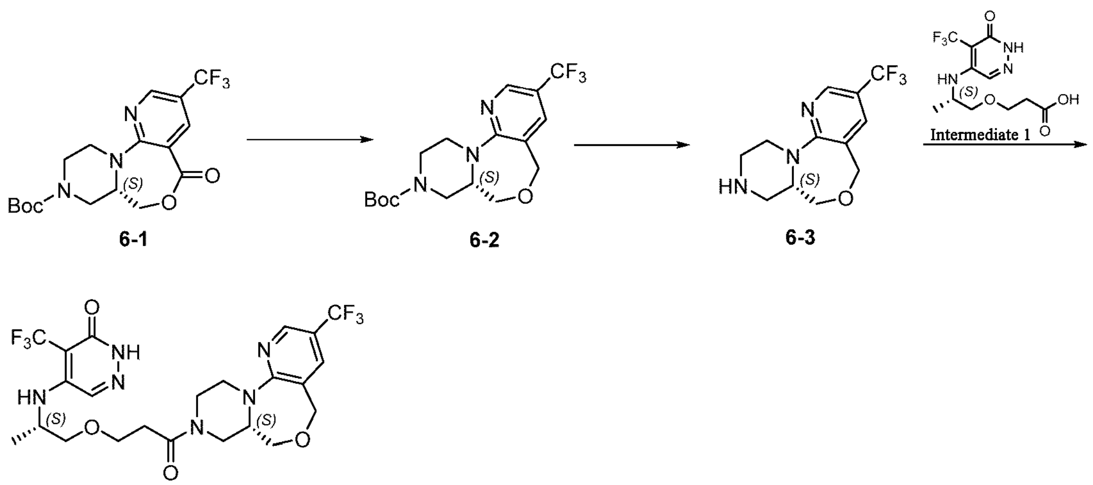


6-1

6-2

6-3

Compound 6

The synthesis route was the same as that in **Example 5,** except that t-butyl (S)-5-oxo-3-(trifluoromethyl)-7a,8,10,11-tetrahydro-5H-pyrazino[2,1-c]pyrido[2,3-e][1,4]oxazolin-9(7H)-carboxylate **(6-1)** was used in place of the reaction raw material t-butyl (R)-5-oxo-3-(trifluoromethyl)-7a,8,10,11-tetrahydro-5H-pyrazino[2,1-c]pyrido[2,3-e][1,4]oxazolin-9(7H)-carboxylate **(3-2)** in Step I for reaction. **Compound 6** as a crude product was purified by high performance liquid chromatography (column: XBridge Prep OBD C18, 30 × 150 mm, 5 um; mobile phase A: water (10 mmol/L sodium bicarbonate), mobile phase B: acetonitrile; flow rate: 60 mL/min; gradient: mobile phase B from 20% to 52% over 8 min, detection wavelength: 220 nm; retention time: 6.90 min) to obtain the target product as a white solid (11 mg, 0.019 mmol, yield: 59%).

$^1H$ NMR (400 MHz, DMSO-$d_6$) δ (ppm): 12.45 (s, 1H), 8.42-8.39 (m, 1H), 7.91 (s, 1H), 7.81-7.77 (m, 1H), 6.28-6.25 (m,1H), 4.94-4.86(m, 1H), 4.54-4.49 (m, 1H), 4.19-4.11 (m, 1H), 4.05-3.64 (m, 10H), 3.56 - 3.41 (m, 3H), 2.56 - 2.52 (m, 2H), 1.16-1.14 (m, 3H). LCMS: MS (ESI) m/z: **565.00** $[M+H]^+$.

**Example 7:** Synthesis of (R) -8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-5H-pyrazino[1,2-a]pyrido[3,2-e]pyrazin-6(6aH)-one **(Compound 7)**

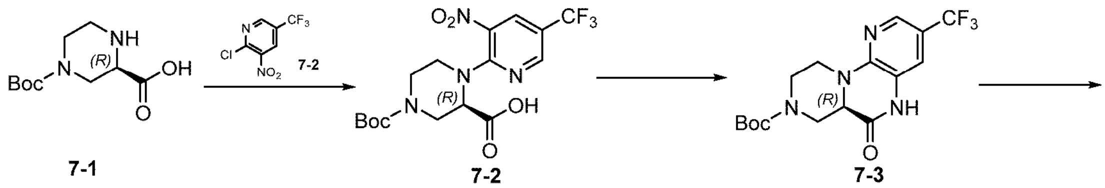


**7-1** **7-2** **7-3**

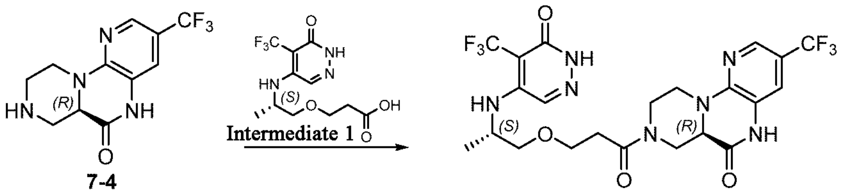


**7-4**

**Compound 7**

Step I: Synthesis of (R) -4-(t-butoxycarbonyl)-1-(3-nitro-5-(trifluoromethyl)pyridin-2-yl)piperazin-2-carboxylic acid **(7-2)**

(R)-4-(t-butoxycarbonyl)piperazin-2-carboxylic acid **(7-1)** (0.50 g, 2.17 mmol) and 2-chloro-3-nitro-5-(trifluoromethyl)pyridine **(7-2)** (0.49 g, 2.17 mmol) were dissolved in methanol (5 mL), and then diisopropylethyl amine (0.65 g, 4.34 mmol) was added. The reaction was stirred overnight at room temperature. The reaction solution was concentrated, diluted in water and then extracted with ethyl acetate (20 mL × 3). The organic phase was dried over anhydrous sodium sulfate. Anhydrous sodium sulfate was removed by filtration. The filtrate was concentrated to obtain a crude product, which was purified by column chromatography on silica gel (petroleum ether: ethyl acetate =35:65) to obtain the target product as a yellow solid (0.90 g, 2.17mmoL, yield: 99%). LCMS: MS (ESI) m/z: 421$[M+H]^+$.

Step II: Synthesis of t-butyl (R)-6-oxo-3-(trifluoromethyl)-5,6,6a,7,9,10-hexahydro-8H-pyrazino[1,2-a]pyrido[3,2-e]pyrazin-8-carboxylate **(7-3)**

(R) -4-(t-butoxycarbonyl)-1-(3-nitro-5-(trifluoromethyl)pyridin-2-yl)piperazin-2-carboxylic acid **(7-2)** (0.20 g, 0.48 mmol) was dissolved in ethanol (10.00 mL), and the solid ammonium chloride (0.25 g, 4.76 mmol) and iron powder (0.27 g, 4.76 mmol) were added. The reaction mixture was heated to 70°C and reacted for 3 hrs with stirring. The solid was removed by filtration, and the filtrate was concentrated under reduced pressure and rotary dried to obtain a black crude product. The crude product was purified by column chromatography on silica gel (petroleum ether: ethyl acetate =65:35) to obtain the target product as a yellow solid (80 mg, 0.215mmol, yield: 42%). LCMS: MS (ESI) m/z: 373$[M+H]^+$.

Step III: Synthesis of (R) -3-(trifluoromethyl)-7,8,9,10-tetrahydro-5H-pyrazino[1,2-a]pyrido[3,2-e]pyrazin-6(6aH)-one **(7-4)**

t-butyl (R)-6-oxo-3-(trifluoromethyl)-5,6,6a,7,9,10-hexahydro-8H-pyrazino[1,2-a]pyrido[3,2-e]pyrazin-8-carboxylate **(7-3)** (0.08 g, 0.215 mmol) was dissolved in a solution of hydrogen chloride in 1,4-dioxane (2.00 mL, 4M). The reaction mixture was stirred at room temperature for 0.5 hr, until the reaction was completed. The reaction solution was concentrated under reduced pressure. The crude product was added with water (10 mL), adjusted to pH 8-9 with a saturated sodium bicarbonate aqueous solution, and extracted with ethyl acetate (10 mL × 3). The organic phase was dried over anhydrous sodium sulfate. Anhydrous sodium sulfate was removed by filtration, and the filtrate was concentrated under reduced pressure to obtain a crude product. The crude product was purified by column chromatography on silica gel (petroleum ether: ethyl acetate =10:90) to obtain a yellow target product (51mg, 0.18 mmol, yield: 84%). LCMS: MS (ESI) m/z: 273 [M+H]$^+$.

Step IV: Synthesis of (R) -8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-5H-pyrazino[1,2-a]pyrido[3,2-e]pyrazin-6(6aH)-one **(Compound 7)**

(R) -3-(trifluoromethyl)-7,8,9,10-tetrahydro-SH-pyrazino[1,2-a]pyrido[3,2-e]pyrazin-6(6aH)-one **(7-4)** (146.72 mg, 0.54 mmol) and (S) -3-(2-((6-oxo-5-(trifluoromethyl)-1,6-dihydropyridazin-4-yl)amino)propoxy)propionic acid **(Intermediate 1)** (200.00 mg, 0.65 mmol) were dissolved in dichloromethane (10.00 mL). Diisopropylethyl amine (348.27 mg, 2.69 mmol) and T3P(1028.91 mg, 1.62 mmol) were added, and the reaction solution was reacted at room temperature for 1 hr with stirring. The reaction solution was diluted in dichloromethane (100 mL), and the organic phase was extracted with water (30 mL × 3), and then washed with saturated brine. The organic phase was dried over anhydrous sodium sulfate. Anhydrous sodium sulfate was removed by filtration, and the filtrate was concentrated under reduced pressure to obtain a crude product, which was purified by high performance liquid chromatography (column: YMC-Actus Triart C18, 30 mm X 150 mm, 5 um, mobile phase A: water (containing 0.05% trifluoroacetic acid), mobile phase B: acetonitrile, flow rate: 60 ml/ min, elution gradient: mobile phase B from 5% to 45% over 8 min, detection wavelength: 220 nm, retention time: 7.05 min) to obtain the target product as a white solid (59.61 mg, yield: 19%). $^1$H NMR (400 MHz, DMSO-$d_6$) δ (ppm): 12.44 (s, 1H), 10.99-10.93 (m, 1H), 8.13 (s, 1H), 7.91 (s, 1H), 7.16 (d, $J$ = 2.2 Hz, 1H),6.28-6.27(m,1H), 4.83-4.47(m, 2H), 4.24 - 4.14 (m, 2H), 4.09 - 3.97 (m, 1H), 3.75-3.68 (m, 2H), 3.52-3.45 (m, 2H), 3.25-3.06 (m, 1H), 2.82 - 2.74 (m, 1H), 2.68-2.60 (m, 2H), 1.16-1.15 (d, $J$ = 6.5 Hz, 3H). LCMS: MS (ESI) m/z: 564.10 [M+H]$^+$.

**Example 8:** Synthesis of (S) -8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-SH-pyrazino[1,2-a]pyrido[3,2-e]pyrazin-6(6aH)-one **(Compound 8)**

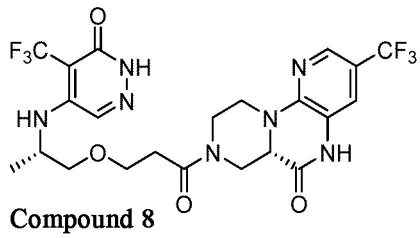

Compound 8

The synthesis steps were the same as those for **Compound 7,** except that (S) -4-(t-butoxycarbonyl)piperazin-2-carboxylic acid was used in place of (R) -4-(t-butoxycarbonyl)piperazin-2-carboxylic acid **(7-1)** for reaction. **Compound 8** as a crude product was purified by high performance liquid chromatography (column: YMC-Actus Triart C18, 30 mm X 150 mm, 5 um, mobile phase A: water (containing 0.05% trifluoroacetic acid), mobile phase B: acetonitrile, flow rate: 60 ml/ min, elution gradient: mobile phase B from 5% to 45% over 8 min, detection wavelength: 220 nm, retention time: 7.05 min) to obtain the target product as a white solid (75.78 mg, yield: 24%).

$^1$H NMR (400 MHz, DMSO-$d_6$) δ (ppm): 12.45-12.43 (d, $J$ = 7.4 Hz, 1H), 10.98-10.92 (m, 1H), 8.13 (s, 1H), 7.91-7.89 (m, 1H), 7.16 (s, 1H), 6.27-6.26 (m, 1H), 4.83-4.49 (m, 2H), 4.25-4.13 (m, 2H), 4.07-3.99 (m,1H), 3.73-3.68 (m, 2H), 3.50-3.49 (d, $J$ = 5.5 Hz, 2H), 3.26-3.07 (m, 1H), 2.85-2.76 (m, 1H), 2.72-2.60 (m, 3H), 1.16-1.15 (d, $J$ = 6.5 Hz, 3H). LCMS: MS(ESI)m/z: 564.10 [M+H]$^+$.

**Example 9:** Synthesis of 5-(((S)-1-(3-oxo-3-((S)-3-(trifluoromethyl)-5,6,6a,7,9,10-hexahydro-8H-pyrazino[1,2-a]pyrido[3,2-e]pyrazin-8-yl)propoxy)prop-2-yl)amino)-4-(trifluoromethyl)pyridazin-3(2H)-one **(Compound 9)**

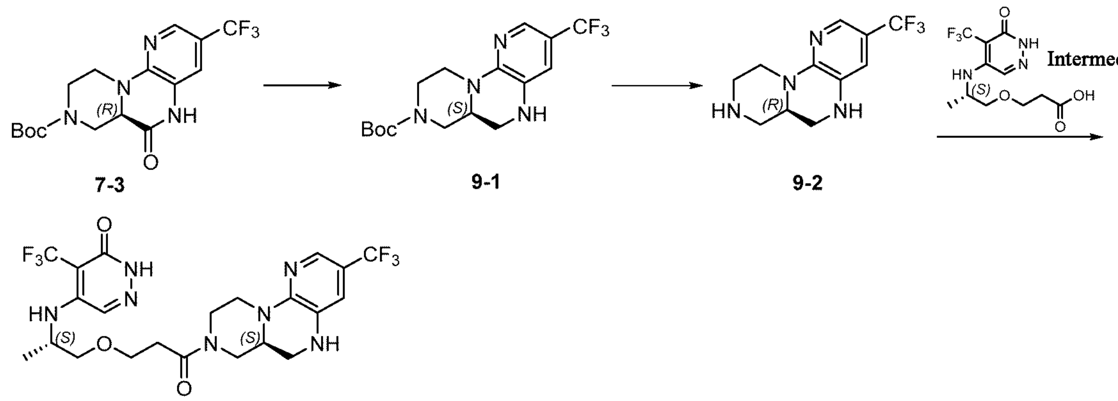


7-3 9-1 9-2

Compound 9

Step I: Synthesis of t-butyl (S)-3-(trifluoromethyl)-5,6,6a,7,9,10-hexahydro-8H-pyrazino[1,2-a]pyrido[3,2-e]pyrazin-8-carboxylate **(9-1)**

t-butyl (R)-6-oxo-3-(trifluoromethyl)-5,6,6a,7,9,10-hexahydro-8H-pyrazino[1,2-a]pyrido[3,2-e]pyrazin-8-carboxylate (0.12g, 0.32mmol) was dissolved in tetrahydrofuran (1ml), and then boron trifluoride-diethyl etherate (0.07 g, 0.48 mmol) and sodium borohydride (0.02 g, 0.48 mmol) were added. The reaction solution was stirred at room temperature for 0.5 hr. After the reaction was completed, the reaction solution was quenched by adding dropwise to a saturated sodium bicarbonate aqueous solution cooled to 0°C with stirring. The reaction solution was extracted three times with ethyl acetate and water, and washed with a saturated sodium chloride aqueous solution. The organic phase was dried over anhydrous sodium sulfate, and rotary dried under a low pressure to obtain the target product as a yellow solid (0.12 g, yield: 100%). LCMS: MS (ESI) m/z: 359 $[M+H]^+$.

Step II: Synthesis of (R)-3-(trifluoromethyl)-6,6a,7,8,9,10-hexahydro-5H-pyrazino[1,2-a]pyrido[3,2-e]pyrazine **(9-2)**

(S)-3-(trifluoromethyl)-5,6,6a,7,9,10-hexahydro-8H-pyrazino[1,2-a]pyrido[3,2-elpyrazin-8-t-butyl carboxylate (0.15 g, 0.42 mmol) was dissolved in 1,4-dioxane (2 ml), and then a solution of hydrochloric acid in dioxane (4 mol/l, 2 ml) was added. The reaction solution was stirred at room temperature for 0.5 hr. After the reaction was completed, dioxane was removed by rotary drying the solution under a low pressure, to obtain the target product as a yellow solid (0.22 g, yield: 204%). LCMS: MS(ESI) m/z: 259 $[M+H]^+$.

Step III: Synthesis of 5-(((S)-1-(3-oxo-3-((S)-3-(trifluoromethyl)-5,6,6a,7,9,10-hexahydro-8H-pyrazino[1,2-a]pyrido[3,2-e]pyrazin-8-yl)propoxy)prop-2-yl)amino)-4-(trifluoromethyl)pyridazin-3(2H)-one **(Compound 9)**

(R)-3-(trifluoromethyl)-6,6a,7,8,9,10-hexahydro-5H-pyrazino[1,2-a]pyrido[3,2-e]pyrazine **(9-2)** (0.08 g, 0.32 mmol) was dissolved in dichloromethane (10 ml), and then N,N-diisopropylethyl amine (0.42 g, 3.23 mmol), ethyl (S)-3-(2-((6-oxo-5-(trifluoromethyl)-1,6-dihydropyrazin-4-)amino)propoxy) propionate **(Intermediate 1)** (0.10 g, 0.32 mmol) and 1-propylphosphonic anhydride (0.51g, 1.62 mmol) were added. The reaction solution was stirred at room temperature for 1 hr. After the reaction was completed, the reaction solution was extracted three times with water and ethyl acetate. The organic phase was washed once with saturated sodium chloride solution, and dried over anhydrous sodium sulfate. The crude product was purified by high performance liquid chromatography (column: Xselect CSH OBD Column, 30*150 mm 5 um, mobile phase A: water (containing 10 mol/l ammonium bicarbonate), mobile phase B: acetonitrile, flow rate: 60 ml/ min, elution gradient: mobile phase B from 5% to 50% over 8 min, detection wavelength: 220 nm, retention time: 8.07 min) to obtain the target product as a white solid (0.05 g, yield: 27%). $^1H$ NMR (400 MHz, DMSO-$d_6$) $\delta$ (ppm): 12.46 (s, 1H), 7.92 (s, 1H), 7.71 (s, 1H), 6.80 (d, J = 2.1 Hz, 1H), 6.32-6.18 (m, , 2H), 4.55 - 4.43 (m, 2H), 4.15 (t, J = 7.2 Hz, 1H), 4.09-3.90(m, 1H), 3.73-3.64 (m, 2H), 3.49 (d, J = 5.6 Hz, 3H), 3.32-3.21 (m, 1H), 3.15-3.02(m,1H), 2.84-2.77 (m, 1H), 2.68-2.60 (m, 3H), 2.48-2.36 (m, 1H), 1.16 (d, J = 6.5 Hz, 3H). LCMS: MS(ESI)m/z: 550.10 $[M+H]^+$.

**Example 10:** Synthesis of 5-(((S)-1-(3-oxo-3-((S)-3-(trifluoromethyl)-6,7,7a,8,10,11-hexahydropyrazino[1,2-a]pyrido[3,2-f][1,4]diazepin-9(5H)-yl)propoxy)propanol-2-yl)amino)-4-(trifluoromethyl)piperazin-3(2H)-one (**Compound 10**)

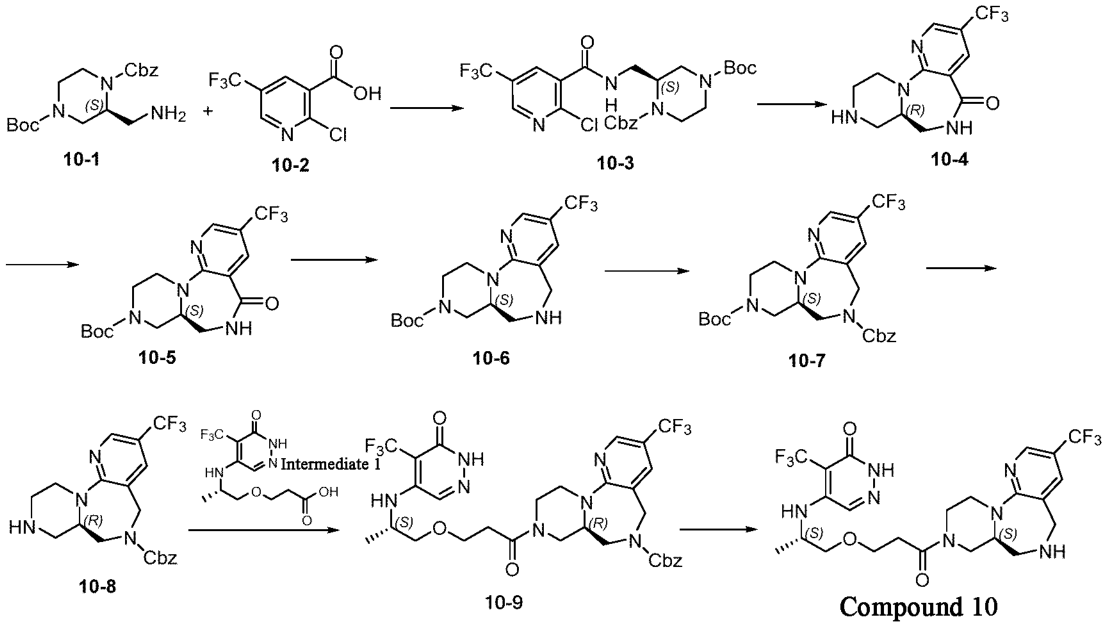


10-1 10-2 10-3 10-4 10-5 10-6 10-7 10-8 10-9 Compound 10

Step **I:** Synthesis of 1-phenyl 4-(t-butoxycarbonyl) (S)-2-((2-chloro -5-(trifluoromethyl)nicotinamidoisophthalic acid)methyl)piperazin-1,4-dicarboxylate (**10-3**)

phenyl- 4-(t-butyl) (R)-2-(aminomethyl)piperazin-1,4-dicarboxylate (10-1) (1 g, 2.85 mmol) and 2-chloro-5-trifluoromethylpyridin-3-carboxylic acid (**10-2**) (0.624 g, 2.85 mmol) were dissolved in dichloromethane (10 mL), and then diisopropylethyl amine (0.735 g, 5.7 mmol) and $T_3P$ (3.648 g, 5.7 mmol, 50% solution in ethyl acetate) were added. The reaction mixture was stirred at room temperature for 2 hrs. The reaction solution was poured into ice water (20 mL), and the aqueous phase was then extracted with ethyl acetate (10 mL × 3). The organic phases were combined and dried over anhydrous sodium sulfate. Anhydrous sodium sulfate was removed by filtration, and the filtrate was rotary dried to obtain a crude product. The crude product was purified by column chromatography to obtain the target product as a white solid (2.224 g, 4 mmol, yield: 70%), LCMS: MS(ESI) m/z: 557 $[M+H]^+$.

Step II: Synthesis of (R)-3-(trifluoromethyl)-7,7a,8,9,10,11-hexahydropyrazino[1,2-a]pyrido[3,2-f] [1,4]diazepine -5(6H)-one (**10-4**)

phenyl 4-(t-butoxycarbonyl) (S)-2-((2-chloro -5-(trifluoromethyl)nicotinamidoisophthalic acid)methyl)piperazin-1,4-dicarboxylate (**10-3**)(2.224 g, 4 mmol) was dissolved in concentrated hydrochloric acid (10 mL). The reaction mixture was stirred at 50 $^0$C for 2 hrs, and the reaction solution was poured into sodium carbonate/ice water (20 mL). The aqueous phase was extracted with ethyl acetate (10 mL × 3). The organic phases were combined and dried over anhydrous sodium sulfate. Anhydrous sodium sulfate was removed by filtration, and the filtrate was rotary dried to obtain a crude product, which was purified by column chromatography on silica gel to obtain the target product as a white solid (0.803 g, 2.8 mmol, yield: 70%), LCMS: MS(ESI) m/z: 287 $[M+H]^+$.

Step III: Synthesis of t-butoxycarbonyl-(R)-3-(trifluoromethyl)-7,7a,8,9,10,11-hexahydropyrazino[1,2-a]pyrido[3,2-f][1,4]diazepine -5(6H)-one (**10-5**)

(R)-3-(trifluoromethyl)-7,7a,8,9,10,11-hexahydropyrazino[1,2-a]pyrido[3,2-fl[1,4]diazepine -5(6H)-one (**10-4**)(1.00 g, 3.4 mmol) was dissolved in dichloromethane (10 mL), and then triethyl amine (0.68 g, 6.8 mmol) and $(Boc)_2O$ (1.5 g, 6.8 mmol) were added to the reaction system. The reaction mixture was stirred at room temperature for 2 hrs, and the reaction solution was poured into water (20 mL). The aqueous phase was extracted with ethyl acetate (10 mL × 3). The organic phases were combined, and dried over anhydrous sodium sulfate. Anhydrous sodium sulfate was removed by filtration, and the filtrate was rotary dried to obtain a crude product. The crude product was purified by column chromatography on silica gel to obtain the target product as a white solid (0.921g, 2.38 mmol, yield: 70%), LCMS: MS(ESI) m/z: 387 $[M+H]^+$.

Step IV: Synthesis of (S)-3-(trifluoromethyl)-6,7,7a,8,10,11-hexahydropyrazino[1,2-a]pyrido[3,2-f] [1,4]diazepine -9(5H)-t-butyl carboxylate (**10-6**)

(t-butoxycarbonyl-(R)-3-(trifluoromethyl)-7,7a,8,9,10,11-hexahydropyrazino[1,2-a]pyrido[3,2-f] [1,4]diazepine -5(6H)-one (**10-5**) (500 mg, 1.29 mmol) was dissolved in tetrahydrofuran (5 mL), and cooled to 0°C. Then, solid sodium borohydride (50 mg, 1.295 mmol) was added, and boron trifluoride-diethyl etherate (185 mg, 1.292 mmol) was added dropwise with stirring. After that, the reaction mixture was reacted with stirring at room temperature for 2 hrs. The reaction solution was poured into ice water (10 ml), and the aqueous phase was extracted with dichloromethane (20 ml × 3). The organic phases were combined, and dried over anhydrous sodium sulfate. Anhydrous sodium sulfate was removed by filtration. The crude product was purified by column chromatography on silica gel to obtain the target product as a white solid (120 mg, 0.321 mmol, yield: 24%). LCMS: MS(ESI) m/z: 373 $[M+H]^+$.

Step V: Synthesis of benzyl t-butyl (S)-3-(trifluoromethyl)-7a,8,10,11-hexahydropyrazino[1,2-a]pyrido[3,2-f][1,4]piperazin-6,9(*5H,7H*)-dicarboxylate (**10-7**)

A mixture of t-butyl (S)-3-(trifluoromethyl)-6,7,7a,8,10,11-hexahydropyrazino[1,2-a]pyrido [3,2-f] [1,4] diazepine -9(5H)-carboxylate (**10-6**) (0.5 g, 1.31mmol) and benzoxycarbonyl chloride (0.24 g, 1.31 mmol) were dissolved in dichloromethane (10 mL), and then triethyl amine (0.262 g, 2.62 mmol) was added. The reaction mixture was reacted for 2 hrs at room temperature with stirring. The reaction solution was cooled and concentrated to dryness. The crude product was purified by column chromatography on silica gel to obtain the target product as a yellow oil (0.532 g, 1.05 mmol, yield: 80%), LCMS: MS(ESI) m/z: 507 $[M+H]^+$.

Step VI: Synthesis of benzyl (R)-3-(trifluoromethyl)-7,7a,8,9,10,11-hexahydropyrazino[1,2-a]pyrido[3,2-f][1,4]piperazin-6(5H)-carboxylate (**10-8**)

6-phenyl 9-(t-butyl) (S)-3-(trifluoromethyl)-7a,8,10,11-hexahydropyrazino[1,2-a]pyrido[3,2-f][1,4]piperazin-6,9(5H,7H)-dicarboxylate (**10-7**)(500 mg, 0.986 mmol) was dissolved in dichloromethane (5 mL), and then trifluoroacetic acid (3 mL) was added. The reaction mixture was reacted at room temperature for 2 hrs with stirring. After complete reaction, the reaction solution was concentrated under reduced pressure. The crude product was diluted in water (30 mL), adjusted to pH 8-9 with sodium bicarbonate, and extracted with dichloromethane (20 mL × 3). The organic extracts were combined and rotary dried under reduced pressure to obtain a crude product. The crude product was purified by column chromatography on silica gel to obtain the target product as a white solid (300 mg, 1.157 mmol, yield: 83%), LCMS: MS(ESI)m/z: 407 $[M+H]^+$.

Step VII: Synthesis of benzyl (R)-9-(3-((S)-2-((6-oxo-5-(trifluoromethyl)-1,6-hexahydropyrazin-4-yl)amino)propoxy)propanol)-3-(trifluoromethyl)-7,7a, 8,9,10,11-hexahydropyrazino[1,2-a]pyrido[3,2-f][1,4]piperazin-6(5H)-carboxylate (**10-9**)

**Intermediate 1** 3-[(2S)-2-{ [6-oxo-5-(trifluoromethyl)-1,6-dihydropyridazin-4-yl]amino}propoxy]propionic acid (100 mg, 0.323 mmol) and benzyl (R)-3-(trifluoromethyl)-7,7a,8,9,10,11-hexahydropyrazino[1,2-a]pyrido[3,2-f][1,4]piperazin-6(5H)-carboxylate (83.83 mg, 0.323 mmol) were dissolved in dichloromethane (1.0 mL). Then, diisopropylethyl amine (0.053 mL, 0.323 mmol) and T3P (102.89 mg, 0.323 mmol, 50% solution in ethyl acetate) were added. The reaction mixture was stirred at room temperature for 2 hrs. The reaction solution was poured into ice water (20 mL), and the aqueous phase was then extracted with ethyl acetate (10 mL × 3). The organic phases were combined, and dried over anhydrous sodium sulfate. Anhydrous sodium sulfate was removed by filtration, and the filtrate was rotary dried to obtain a crude product, which was purified by column chromatography on silica gel to obtain the target product (80 mg, 1.157 mmol, yield: 70%), LCMS: MS(ESI)m/z: 697 $[M+H]^+$ .

Step VIII: Synthesis of 5-(((S)-1-(3-oxo-3-((S)-3-(trifluoromethyl)-6,7,7a,8,10,11-hexahydropyrazino[1,2-a]pyrido[3,2-f][1,4]diazepine -9(5H)-yl)propoxy)propanol-2-yl)amino)-4-(trifluoromethyl)piperazin-3(2H)-one (**Compound 10**)

Benzyl (R)-9-(3-((S)-2-((6-oxo-5-(trifluoromethyl)-1,6-hexahydropyrazin-4-yl)amino)propoxy)propanol)-3-(trifluoromethyl)-7,7a,8,9,10,11-hexahydropyrazino[1,2-a]pyrido[3,2-f] [1,4]piperazin-6(5H)-carboxylate (**10-9**) (80 mg, 1.157 mmol) was dissolved in dichloromethane (1 .0 mL), and then palladium dichloride (0.5mg, 0.01 mmol), triethyl amine (2231.4 mg, 2.314mmol) and triethylsilane (268.21 mg, 2.314 mmol) were added. The reaction mixture was stirred at room temperature for 2 hrs. The reaction solution was poured into ice water (20 mL), and the aqueous phase was then extracted with ethyl acetate (10 mL × 3). The organic phases were combined and dried over anhydrous sodium sulfate. Anhydrous sodium sulfate was removed by filtration, and the filtrate was rotary dried to obtain a crude product. The crude product was purified by preparative liquid-phase chromatography (column: XBridge Prep OBD C18, 30 ×

150 mm 5 um, mobile phase A: water (containing 10 mol/l ammonium bicarbonate), mobile phase B: acetonitrile, flow rate: 60 ml/ min, elution gradient: mobile phase B from 20% to 52% over 8 min, detection wavelength: 220 nm, retention time: 7.12 min) to obtain the target product as a white solid (50 mg, 0.091 mmol, yield: 28.09%).

$^1H$ NMR (400 MHz, DMSO-$d_6$) δ (ppm): 12.45 (s, 1H), 8.31-8.28 (m, 1H), 7.91 (s, 1H), 7.61 (s, 1H), 6.27-6.26 (m, 1H), 4.12-4.06 (m, 2H), 4.01-3.91 (m, 1H), 3.88-3.80 (m, 2H), 3.79-3.67 (m, 6H), 3.63-3.51 (m, 2H), 3.50-3.44(m,2H), 2.99-2.91 (m, 1H), 2.89-2.81 (m, 2H), 1.18-1.10 (m, 3H). LCMS: MS(ESI)m/z: 564.15 $[M+H]^+$.

**Example 11:** Synthesis of (S)-9-(3-((S)-2-((6-oxo-5-(trifluoromethyl)-1,6-dihydropyridin-4-yl)amino)propoxy)propanol)-3-(trifluoromethyl)-7,7a,8,9,10,11-hexahydropyrazino[1,2-a]pyrido[3,2-f][1,4]diazepine-6(5H)-carbonitrile (**Compound 11**)

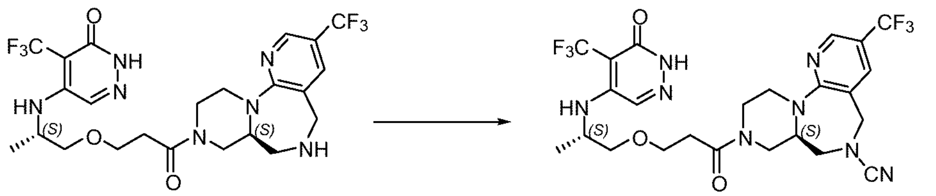

Compound 10 Compound 11

**Compound 10** 5-(((S)-1-(3-oxo-3-((S)-3-(trifluoromethyl)-6,7,7a,8,10,11-hexahydropyrazino[1,2-a]pyrido[3,2-f][1,4]diazepine -9(5H)-yl)propoxy)propanol-2-yl)amino)-4-(trifluoromethyl)piperazin-3(2H)-one (80 mg,1.157 mmol) was dissolved in N,N-dimethylformamide (1.0 mL), and then potassium carbonate (321 mg,2.314 mmol) and cyanogen bromide (115.7 mg,1.157 mmol) were added. The reaction mixture was stirred at room temperature for 2 hrs. The reaction solution was poured into ice water (20 mL), and the aqueous phase was then extracted with ethyl acetate (10 mL × 3). The organic phases were combined, and dried over anhydrous sodium sulfate. Anhydrous sodium sulfate was removed by filtration, and the filtrate was rotary dried to obtain a crude product. The crude product was purified by reverse phase preparative liquid chromatography (column: XBridge Prep OBD C18, 30 × 150 mm 5 um, mobile phase A: water (containing 10 mol/l ammonium bicarbonate), mobile phase B: acetonitrile, flow rate: 60 ml/ min, elution gradient: mobile phase B from 20% to 52% over 8 min, detection wavelength: 220 nm, retention time: 7.09 min) to obtain the target product as a white solid (50 mg, 0.091 mmol, yield: 28.09%). $^1H$ NMR (400 MHz, DMSO-$d_6$) δ (ppm): 12.46 (s, 1H), 8.44-8.41 (m, 1H), 7.91-7.90 (m, 1H), 7.86-7.83(m, 1H), 6.28-6.26 (m, 1H), 4.76-4.67 (m, 1H), 4.40-4.35 (m, 1H), 4.32-4.22 (m, 1H), 4.20-4.05 (m, 1H), 3.93-3.78 (m, 3H), 3.75-3.62(m,5H), 3.58-3.51 (m, 1H), 3.50- 3.45 (m, 2H), 3.44-3.36(m,2H),2.57-2.54(m,1H),1.16-1.12 (m, 3H). LCMS: MS(ESI)m/z: 589.25 $[M+H]^+$.

**Example 12:** Synthesis of 5-(((S)-1-(3-oxo-3-((R)-3-(trifluoromethyl)-7a,8,10,11-tetrahydro-5H-pyrazino[2,1-c]pyrido[2,3-e][1,4]thiazin-9(7H)-yl)propoxy)prop-2-yl)amino)-4-(trifluoromethyl)pyridazin-3(2H)-one (**Compound 12**)

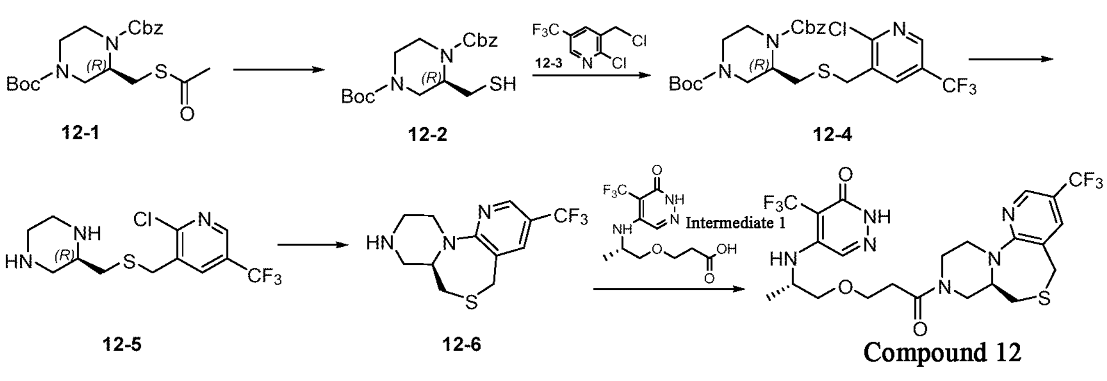


12-1 12-2 12-4 12-5 12-6 Compound 12

Step I: Synthesis of 1-benzyl 4-(t-butyl) (R)-2-(sulfydrylmethyl)piperazin-1,4-dicarboxylate (**12-2**)

benzyl 4-(t-butyl) (R)-2-(acetylthio)methyl)piperazin-1,4-dicarboxylate (**1**)(700.00 mg, 1.71 mmol) was dissolved

in ethanol (1.00 mL), and then a 4 mol/l sodium hydroxide aqueous solution (685.42 mg, 17.14 mmol) was added. The reaction was continued at room temperature for 2 hrs. After the reaction was completed, the reaction solution was rotary dried under a low pressure to remove ethanol, added with water, and extracted with three times with ethyl acetate. The organic phase was washed with saturated brine, dried over anhydrous sodium sulfate, and rotary dried to obtain a crude product as a yellow solid. The crude product was purified by column chromatography on silica gel to obtain the target product (400.00 mg, yield: 64%). LCMS: MS(ESI) m/z: 367 $[M+H]^+$.

Step II: Synthesis of 1-benzyl 4-(t-butyl) (R)-2-((((2-chloro-5-(trifluoromethyl)pyridin-3-yl)methyl)thio)methyl)piperazin-1,4-dicarboxylate (**12-4**)

Benzyl 4-(t-butyl) (R)-2-(sulfydrylmethyl)piperazin-1,4-dicarboxylate (400.00 mg, 1.09 mmol) was dissolved in ethanol (10.00 mL), and then 2-chloro-3-(chloromethyl)-5-(trifluoromethyl)pyridine (426.80 mg, 1.86 mmol) was added. The reaction was stirred at room temperature for 2 hrs. After the reaction was completed, the reaction solution was rotary evaporated under reduced pressure to remove ethanol, diluted in water, and extracted three times with ethyl acetate. The organic phase was washed with saturated brine and dried over anhydrous sodium sulfate. Anhydrous sodium sulfate was removed by filtration, and the filtrate was rotary evaporated under reduced pressure to remove the solvent, so as to obtain a crude product. The crude product was purified by column chromatography on silica gel to obtain the target product as a yellow solid (256.00 mg, yield: 42%). LCMS: MS(ESI) m/z: 560 $[M+H]^+$.

Step III: Synthesis of (R)-2-((((2-chloro-5-(trifluoromethyl)pyridin-3-yl)methyl)thio)methyl)piperazine (**12-5**)

benzyl 4-(t-butyl) (R)-2-((((2-chloro-5-(trifluoromethyl)pyridin-3-yl)methyl)thio)methyl)piperazin-1,4-dicarboxylate (190.00 mg, 0.34 mmol) was dissolved in hydrochloric acid (4.00 mL). The reaction was stirred at room temperature for 0.5 hr. After the reaction was completed, the reaction solution was rotary evaporated to dryness under reduced pressure. The crude product was purified by column chromatography on silica gel to obtain the target product as a yellow solid (220.00 mg, yield: 200%). LCMS: MS (ESI) m/z: 326 $[M+H]^+$.

Step IV: Synthesis of (R)-3-(trifluoromethyl)-7,7a,8,9,10,11-hexahydro-5H-pyrazino[2,1-c]pyrido[2,3-e][1,4]thiazoline (**12-6**)

(R)-2-((((2-chloro-5-(trifluoromethyl)pyridin-3-yl)methyl)thio)methyl)piperazine (210.00 mg, 0.64 mmol) was dissolved in DMF (4.00 mL), and potassium carbonate (178.18 mg, 1.29 mmol) was added. The reaction was stirred at 100°C for 2 hrs. After the reaction was completed, the reaction solution was diluted in water, and the aqueous phase was extracted three times with ethyl acetate. The extracts were combined, washed with saturated brine, and dried over anhydrous sodium sulfate. Anhydrous sodium sulfate was removed by filtration, and the filtrate was rotary evaporated under reduced pressure, to obtain a crude product. The crude product was purified by column chromatography on silica gel to obtain the target product as a yellow solid (160.00 mg, yield: 86%) . LCMS: MS(ESI) m/z: 290 $[M+H]^+$.

Step V: Synthesis of 5-(((S)-1-(3-oxo-3-((R)-3-(trifluoromethyl)-7a,8,10,11-tetrahydro-5H-pyrazino[2,1-c]pyrido[2,3-e][1,4]thiazin-9(7H)-yl)propoxy)prop-2-yl)amino)-4-(trifluoromethyl)pyridazin-3(2H)-one (**Compound 12**)

(R)-3-(trifluoromethyl)-7,7a,8,9,10,11-hexahydro-SH-pyrazino[2,1-c]pyrido[2,3-e][1,4]thiazoline (**12-6**) (0.07 g, 0.22 mmol) was dissolved in dichloromethane (2.00 ml). N,N-diisopropylethyl amine (0.29 g, 2.25 mmol), **Intermediate1** (S)-3-(2-((6-oxo-5-(trifluoromethyl)-1,6-dihydropyridazin-4-yl)amino)propoxy)propionic acid (0.07 g, 0.22 mmol) and 1-propylphosphonic anhydride (0.71 g, 1.12 mmol) were sequentially added. The reaction was stirred at room temperature for 1 hr. After the reaction was completed, the reaction was quenched with water, and the reaction solution was extracted three times with dichloromethane. The organic phase was washed with saturated brine, and dried over anhydrous sodium sulfate. Anhydrous sodium sulfate was removed by filtration, and then the solvent was removed by rotary evaporation, to obtain a crude product. The crude product was purified by preparative liquid chromatography (column: Xselect CSH OBD Column, 30*150 mm 5 um, mobile phase A: water (containing 10 mol/l ammonium bicarbonate), mobile phase B: acetonitrile, flow rate: 60 ml/ min, elution gradient: mobile phase B from 5% to 42% over 8 min, detection wavelength: 220 nm, retention time: 7.78 min) to obtain the target product as a white solid (10 mg, yield: 5%).

$^1$H NMR (400 MHz, DMSO-$d_6$) $\delta$ (ppm): 12.45 (s, 1H),8.35-8.30 (m, 1H), 7.91 (s, 1H), 7.75-7.70 (m, 1H), 6.30-6.25 (m, 1H), 4.36-4.23 (m, 1H),4.20-4.09 (m, 1H),3.95-3.85 (m, 1H), 3.83-3.76 (m, 1H), 3.72-3.61 (m, 6H),3.50-3.41 (m, 2H), 3.01-2.90 (m, 2H), 2.87-2.78 (m,2H),2.59-2.56 (m, 2H), 1.19-1.12 (m, 3H). LCMS: MS(ESI)m/z: 581.10 $[M+H]^+$.

**Example 13:** Synthesis of 5-(((S)-1-(3-(S)-5-methyl-3-(trifluoromethyl)-5,6,6a,7,9,10-hexahydro-8H-pyrazino[1,2-a]pyrido[3,2-e]pyrazin-8-yl)-3-oxypropoxy)prop-2-yl)amino)-4-(trifluoromethyl)pyridazin-3(2H)-one (**Compound 13**)

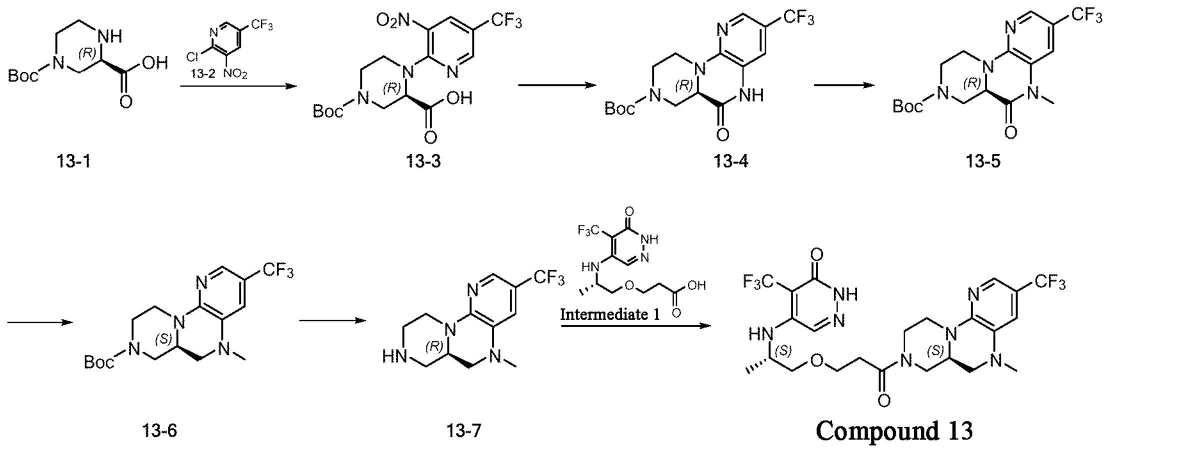


13-1 13-3 13-4 13-5

13-6 13-7 Compound 13

Step I: Synthesis of (R)-4-(t-butoxycarbonyl)-1-(3-nitro-5-(trifluoromethyl)pyridin-2-yl)piperazin-2-carboxylic acid (**13-3**)

(R)-4-(t-butoxycarbonyl)piperazin-2-carboxylic acid (1.00 g, 4.34 mmol) was dissolved in methanol (10.00 mL), and then triethyl amine (1.30 g, 8.69 mmol) and 2-chloro-3-nitro-5-(trifluoromethyl)pyridine (1.18 g, 5.21 mmol) were added. The reaction was stirred overnight at room temperature. After the reaction was completed, the reaction solution was extracted three times with ethyl acetate and water. The organic phase was washed with saturated brine, dried over anhydrous sodium sulfate, and rotary dried under a low pressure to obtain a crude product. The crude product was purified by medium-pressure column chromatography to obtain the target product as a white solid (0.96 g, yield: 53%). LCMS: MS(ESI) m/z: 421 $[M+H]^+$.

Step II: Synthesis of t-butyl (R)-6-oxo-3-(trifluoromethyl)-5,6,6a,7,9,10-hexahydro-8H-pyrazino[1,2-a]pyrido[3,2-e]pyrazin-8-carboxylate (**13-4**)

(R)-4-(t-butoxycarbonyl)-1-(3-nitro-5-(trifluoromethyl)pyridin-2-yl)piperazin-2-carboxylic acid (0.96 g, 2.28 mmol) was dissolved in ethanol (50.00 mL), and then ammonium chloride (1.22 g, 22.84 mmol) and iron powder (1.28 g, 22.84 mmol) were added. The reaction was stirred at 70°C for 2 hrs. After the reaction was completed, the reaction solution was quenched with water, and extracted three times with dichloromethane. The crude product was purified by column chromatography on silica gel to obtain the target product as a yellow solid (0.30 g, yield: 35%). LCMS: MS(ESI) m/z: 373 $[M+H]^+$.

Step III: Synthesis of t-butyl (R)-5-methyl-6-oxo-3-(trifluoromethyl)-5,6,6a,7,9,10-hexahydro-8H-pyrazino[1,2-a]pyrido[3,2-e]pyrazin-8-carboxylate (**13-5**)

t-butyl (R)-6-oxo-3-(trifluoromethyl)-5,6,6a,7,9,10-hexahydro-8H-pyrazino[1,2-a]pyrido[3,2-e]pyrazin-8-carboxylate (0.20 g, 0.54 mmol) was dissolved in tetrahydrofuran (20.00 mL), and then cesium carbonate (0.26 g, 0.81 mmol) and iodomethane (0.11 g, 0.81 mmol) were added. The reaction was stirred at room temperature for 2 hrs. The reaction solution was quenched with water and extracted three times with ethyl acetate. The organic phase was dried and rotary dried under reduced pressure to obtain a crude product as a brown oil (0.40 g, yield: 193%). LCMS: MS(ESI) m/z: 387 $[M+H]^+$.

Step IV: Synthesis of t-butyl (S)-5-methyl-3-(trifluoromethyl)-5,6,6a,7,9,10-hexahydro-8H-pyrazino[1,2-a]pyrido[3,2-e]pyrazin-8-carboxylate (**13-6**)

t-butyl (R)-5-methyl-6-oxo-3-(trifluoromethyl)-5,6,6a,7,9,10-hexahydro-8H-pyrazino[1,2-a]pyrido[3,2-e]pyrazin-8-carboxylate (0.40 g, 1.04 mmol) was dissolved in tetrahydrofuran (20.00 mL), and cooled to 0°C. Boron trifluoride-diethyl etherate (1.67 mL, 0.00 mmol) and sodium borohydride (0.40 g, 0.00 mmol) were added to the solution, and the reaction was stirred at room temperature for 0.5 hr. The reaction solution was quenched with water and extracted

three times with ethyl acetate. The organic phase was washed with saturated brine, dried over anhydrous sodium sulfate, and concentrated to dryness by rotary evaporation under reduced pressure to obtain a crude product as a yellow oil (0.30 g, yield: 78%). LCMS: MS(ESI) m/z: 373 $[M+H]^+$.

Step V: Synthesis of (R)-5-methyl-3-(trifluoromethyl)-6,6a,7,8,9,10-hexahydro-5H-pyrazino[1,2-a]pyrido[3,2-e]pyrazine (**13-7**)

t-butyl (S)-5-methyl-3-(trifluoromethyl)-5,6,6a,7,9,10-hexahydro-8H-pyrazino[1,2-a]pyrido[3,2-e]pyrazin-8-carboxylate (0.30 g, 0.81 mmol) was dissolved in dioxane (5.00 mL), and then a 4 mol/l hydrochloric acid solution in dioxane (5.00 mL, 20.00 mmol) was added to the solution. The reaction stirred at room temperature for 0.5 hr. The reaction solution was rotary dried under reduced pressure to remove dioxane, and a crude product as a rose-red solid (0.38 g, yield: 173%) was obtained. LCMS: MS(ESI) m/z: 273 $[M+H]^+$.

Step VI: Synthesis of 5-(((S)-1-(3-(S)-5-methyl-3-(trifluoromethyl)-5,6,6a,7,9,10-hexahydro-8H-pyrazino[1,2-a]pyrido[3,2-e]pyrazin-8-yl)-3-oxypropoxy)prop-2-yl)amino)-4-(trifluoromethyl)pyridazin-3(2H)-one (**Compound 13**)

(R)-5-methyl-3-(trifluoromethyl)-6,6a,7,8,9,10-hexahydro-5H-pyrazino[1,2-a]pyrido[3,2-e]pyridazine (**13-7**) (73.37 mg, 0.27 mmol) was dissolved in dichloromethane (7 mL). DIEA (348.27 mg, 2.69 mmol), (S)-3-(2-((6-oxo-5-(trifluoromethyl)-1,6-dihydropyridazin-4-yl)amino)propoxy)propionic acid (**Intermediate1**) (100.00 mg, 0.32 mmol) and 1-propylphosphonic anhydride (428.71 mg, 1.35 mmol) were then sequentially added. The reaction solution was stirred at room temperature for 0.5 hr. After the reaction was completed, the reaction solution was quenched with water and extracted three times with dichloromethane. The organic phases were combined, washed with saturated brine, and dried over anhydrous sodium sulfate. After filtration, the filtrate was rotary dried under reduced pressure to obtain a crude product. The crude product was purified by high-performance reverse preparative chromatography (column: XBridge Prep OBD C18, 30 × 150 mm 5 um, mobile phase A: water (containing 10 mol/l ammonium bicarbonate), mobile phase B: acetonitrile, flow rate: 60 ml/ min, elution gradient: mobile phase B from 20% to 60% over 8 min, detection wavelength: 220 nm, retention time: 6.4 min) to obtain the target product as a white solid (32.06 mg, yield: 21%).

$^1H$ NMR (400 MHz, DMSO-$d_6$) δ (ppm): 12.45 (s, 1H), 7.91 (s, 1H), 7.77 (s, 1H), 6.75 (s, 1H), 6.32-6.22 (m, 1H), 4.59-4.56 (m, 1H), 4.44-4.42 (m, 1H), 4.20 - 4.11 (m, 1H), 4.03-3.96 (m, 1H), 3.73-3.65 (m, 2H), 3.53-3.48 (m,2H), 3.42-3.37 (m,1H), 3.17 - 3.00 (m, 2H), 2.91-2.72(m, 4H), 2.71-2.65(m, 1H), 2.62-2.58 (m, 2H), 2.438-2.42(m, 1H), 1.16 (d, J = 6.5 Hz, 3H). LCMS: MS(ESI)m/z: 564.10 $[M+H]^+$.

**Example 14:** Synthesis of 5-(((S)-1-(3-oxo-3-((R)-3-(trifluoromethyl)-6a,7,9,10-tetrahydropyrazino[1,2-d]pyrido[3,2-b][1,4]oxazin-8(6H)-yl)propoxy)prop-2-yl)amino)-4-(trifluoromethyl)pyridazin-3(2H)-one (**Compound 14**)

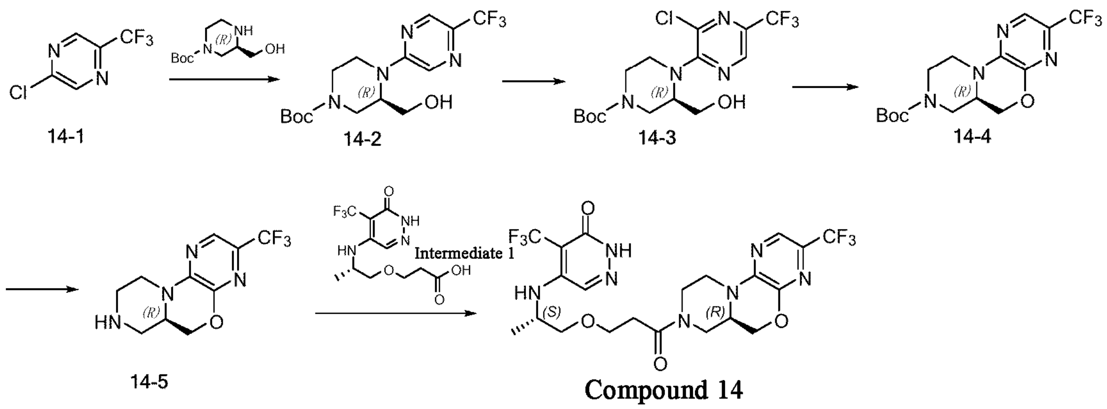


14-1 14-2 14-3 14-4

14-5 Compound 14

Step I: Synthesis of t-butyl (3R)-4-5-(trifluoromethyl)pyridin-2-yl]-3-(hydroxymethyl)pyrazin-1-carboxylate (**14-2**)

A mixture of (3R)-3-(hydroxymethyl)piperazin-1-t-butyl carboxylate (1.0 g,4.624 mmol) and 2-dichloro-5-(trifluoromethyl)pyrazine (**14-1**) (0.85 g,4.624 mmol) were dissolved in DMF (20 mL), and then potassium carbonate (0.263 g, 4.624 mmol) was added, and reacted for 12 hrs at 60°C with stirring under nitrogen atmosphere. The reaction mixture was cooled and then filtered. The filtrate was concentrated under reduced pressure, added with water (30 mL), extracted with ethyl acetate, and dried over anhydrous sodium sulfate. Anhydrous sodium sulfate was removed by filtration, and

the filtrate was rotary dried under reduced pressure. The crude product was purified by column chromatography on silica gel to obtain the target product as a white solid (800 mg, 3.25 mmol, yield: 70%). LCMS: MS(ESI) m/z: 363 $[M+H]^+$.

Step II: Synthesis of t-butyl 3R-4-[3-chloro-5-(trifluoromethyl)pyrazin-2-yl]-3-(hydroxymethyl)piperazin-1-carboxylate (**14-3**)

The compound 1-t-butyl (3R)-4-5-(trifluoromethyl)pyrazin-2-yl]-3-(hydroxymethyl)piperazin-carboxylate (**14-2**) (0.6 g, 1.65 mmol) and N-chlorosuccinimide (0.447 g,2.4 mmol) were dissolved in N,N-dimethylformamide (5 mL). The reaction mixture was heated to $60^0$C and reacted for 3 hrs with stirring. After complete reaction, the solid was removed by filtration. The filtrate was concentrated to obtain a crude product, which was purified by column chromatography on silica gel to obtain the target product as a yellow oil (260 mg, 0.962 mmol, yield: 64%). LCMS: MS(ESI)m/z: 397 $[M+H]^+$.

Step III: Synthesis of (10R)-3-(trifluoromethyl)-6a,7,9,10-tetrahydropyrazino[1,2-d]piperazino[3,2-b][1,4]oxazin-8(6H)-t-butyl carboxylate (**14-4**)

t-butyl (3R-4-[3-chloro-5-(trifluoromethyl)pyrazin-2-yl]-3-(hydroxymethyl)piperazin-1-carboxylate (**14-3**)(500 mg,1.318 mmol) was dissolved in DMF (5 mL), and then potassium carbonate (443 mg, 3.95 mmol) was added. The mixture was heated to 80°C under nitrogen atmosphere, and reacted for 12 hrs with stirring. The reaction solution was cooled, added with ice water (10 mL), and extracted with ethyl acetate (10 mL × 3). The organic phase was dried over anhydrous sodium sulfate. Anhydrous sodium sulfate was removed by filtration, and the filtrate was rotary dried. The crude product was purified by column chromatography on silica gel to obtain the target product as a white solid (300 mg, 1.113 mmol, 60%). LCMS: MS(ESI) m/z: 361 $[M+H]^+$.

Step IV: Synthesis of (10R)-3-(trifluoromethyl)-6a,7,9,10-tetrahydropyrazino[1,2-d]piperazino[3,2-b][1,4]oxazin-8(6H)-carboxylic acid (**14-5**)

(10R)-3-(trifluoromethyl)-6a,7,9,10-tetrahydropyrazino[1,2-d]piperazino[3,2-b][1,4]oxazin-8(6H)-t-butyl carboxylate (**14-5**)(500 mg, 1.391 mmol) was dissolved in hydrochloric acid in dioxane (6 mL). The reaction mixture was reacted for 2 hrs at room temperature with stirring. After complete reaction, the reaction solution was concentrated under reduced pressure. The crude product was diluted in water (30 mL), adjusted to pH 8-9 with sodium bicarbonate, and extracted with dichloromethane (20 mL × 3). The crude product was purified by column chromatography on silica gel to obtain the target product as a white solid (300 mg, 1.157 mmol, yield: 83%), LCMS: MS (ESI) m/z: 261 $[M+H]^+$.

Step V: Synthesis of 5-(((S)-1-(3-oxo-3-((R)-3-(trifluoromethyl)-6a,7,9,10-tetrahydropyrazino[1,2-d]pyrido[3,2-b][1,4]oxazin-8(6H)-yl)propoxy)prop-2-yl)amino)-4-(trifluoromethyl)pyridazin-3(2H)-one (**Compound 14**)

3-[(2S)-2-{[6-oxo-5-(trifluoromethyl)-1,6-dihydropyridazin-4-yl]amino}propoxy]propionic acid (**Intermediate1**) (120 mg,0.387 mmol) and (R)-3-(trifluoromethyl)-6,6a,7,8,9,10-hexahydropyrazino[1,2-d]piperazino[3,2-b][1,4]oxazine (**14-5**) (95 mg, 0.387 mmol) were dissolved in DCM (3 .0 mL). Diisopropylethyl amine (0.053 mL,0.387 mmol) and T3P (102.89 mg, 0.387 mmol, 50% solution in ethyl acetate) were then added. The reaction mixture was stirred at room temperature for 2 hrs. The reaction solution was poured into ice water (20 mL), and the aqueous phase was then extracted with ethyl acetate (10 mL × 3). The organic phases were combined, and dried over anhydrous sodium sulfate. Anhydrous sodium sulfate was removed by filtration, and the filtrate was rotary dried to obtain a crude product. The crude product was purified by preparative liquid chromatography (column: XBridge Prep OBD C18, 30 × 150 mm 5 um, mobile phase A: water (containing 10 mol/l ammonium bicarbonate), mobile phase B: acetonitrile, flow rate: 60 ml/ min, elution gradient: mobile phase B from 20% to 52% over 8 min, detection wavelength: 220 nm, retention time: 7.03 min) to obtain the target product as a white solid (50 mg, 0.091 mmol, yield: 28.09%).

$^1$H NMR (400 MHz, DMSO-$d_6$) δ (ppm): 12.45 (s, 1H), 8.17 (s, 1H), 7.91 (s, 1H), 6.28-6.265(m, 1H), 4.63-4.56 (m, 1H), 4.51-4.46 (m, 1H), 4.45-4.35 (m, 1H), 4.24-4.12 (m, 2H),4.11-4.01(m,1H), 3.75-3.61 (m,3H), 3.49-3.48(m,2H), 3.01- 2.89 (m, 2H), 2.77- 2.68 (m, 1H), 2.65-2.60 (m, 2H), 1.62-1.43 (m, 1H), 1.16-1.14 (d, *J* = 6.5 Hz, 3H), 0.96-0.92 (m, 1H). LCMS: MS(ESI)m/z: 552.15 $[M+H]^+$.

**Example 15:** Synthesis of 5-(((2S)-1-(3-((7aR)-6-oxo-3-(trifluoromethyl)-7a,8,10,11-tetrahydro-5H-pyrazino[2,1-c]pyrido[2,3-e][1,4]thiazin-9(7H)-yl)-3-oxopropoxy)prop-2-yl)amino)-4-(trifluoromethyl)pyridazin-3(2H)-one (**Compound 15**)

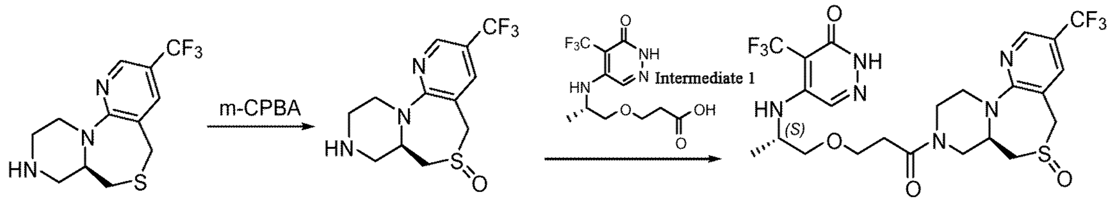


12-6 15-1 Compound 15

Step I: Synthesis of (7aR)-3-(trifluoromethyl)-7,7a,8,9,10,11-hexahydro-6-oxo5H-pyrazino[2,1-c]pyrido[2,3-e][1,4] thiazoline (**15-1**)

(R)-3-(trifluoromethyl)-7,7a,8,9,10,11-hexahydro-SH-pyrazino[2,1-c]pyrido[2,3-e][1,4]thiazoline (**12-6**) (0.08 g, 0.28 mmol) was dissolved in dichloromethane (2 mL), cooled to 0°C, and then added with m-chloroperoxybenzoic acid (0.05 g, 0.28 mmol). The reaction was stirred at 0°C for 0.5 hr. After the reaction was completed, the reaction solution was quenched with water and extracted three times with dichloromethane. The organic phase was washed with saturated brine, and dried over anhydrous sodium sulfate. Anhydrous sodium sulfate was removed by filtration, and the filtrate was rotary dried under reduced pressure to obtain a crude product as a yellow solid (0.07 g, yield: 78%). LCMS: MS(ESI) m/z: 306 $[M+H]^+$.

Step II: Synthesis of 5-(((2S)-1-(3-((7aR)-6-oxo-3-(trifluoromethyl)-7a,8,10,11-tetrahydro-5H-pyrazino[2,1-c]pyrido[2,3-e][1,4]thiazin-9(7H)-yl)-3-oxopropoxy)prop-2-yl)amino)-4-(trifluoromethyl)pyridazin-3(2H)-one (**Compound 15**)

(R)-3-(trifluoromethyl)-7,7a,8,9,10,11-hexahydro-5H-pyrazino[2,1-c]pyrido[2,3-e][1,4]thiazoline -5-one (**15-1**) (0.06 g, 0.20 mmol) and (S)-3-(2-((6-oxo-5-(trifluoromethyl)-1,6-dihydropyridazin-4-yl)amino)propoxy)propionic acid (**Intermediate1**) (0.06 g, 0.20 mmol) were dissolved in dichloromethane (2.00 mL). DIEA(0.25 g, 1.97 mmol) and 1-propylphosphonic anhydride (0.31 g, 0.98 mmol) were then sequentially added. The reaction mixture was stirred at room temperature for 0.5 hr. After the reaction was completed, the reaction solution was quenched with water and extracted three times with dichloromethane. The organic phase was washed with saturated brine and dried over anhydrous sodium sulfate. Anhydrous sodium sulfate was removed by filtration, and the filtrate was rotary dried under reduced pressure to obtain a solid crude product. The crude product was purified by reverse high-performance preparative liquid chromatography (column: Xselect CSH OBD Column, 30*150 mm 5 um, mobile phase A: water (containing 10 mol/l ammonium bicarbonate), mobile phase B: acetonitrile, flow rate: 60 ml/ min, elution gradient: mobile phase B from 5% to 42% over 8 min, detection wavelength: 220 nm, retention time: 7.78 min) to obtain the target product as a white solid (0.01 g, yield: 5%).

$^1H$ NMR (400 MHz, DMSO-$d_6$) $\delta$ (ppm): 12.44(s, 1H), 8.55-8.50 (m, 1H),8.05-7.95 (m, 1H), 7.92 (s, 1H), 6.35-6.25 (m, 1H), 4.39-4.32 (m, 1H),4.26-4.20 (m, 1H),4.18-4.11 (m, 2H), 4.11-4.05 (m, 1H), 3.98-3.92 (m, 1H), 3.91-3.84 (m, 1H), 3.77-3.74 (m, 1H), 3.71-3.65 (m, 3H), 3.62-3.58 (m, 1H), 3.50-3.47 (m, 2H), 2.92-2.81 (m, 2H), 2.60-2.58 (m, 2H), 1.19-1.10 (m, 3H). LCMS: MS(ESI)m/z: 597.10 $[M+H]^+$.

**Example 16:** Synthesis of (R)-8-(3-((S)-2-((6-oxo-5-(trifluoromethyl)-1,6-dihydropyridazin-4-yl)amino)propoxy)propionyl)-3-(trifluoromethyl)-6,6a,7,8,9,10-hexahydro-12H-pyrazino[2,1-c]pyrido[2,3-f][1,4]oxazepin-12-one (**Compound 16**)

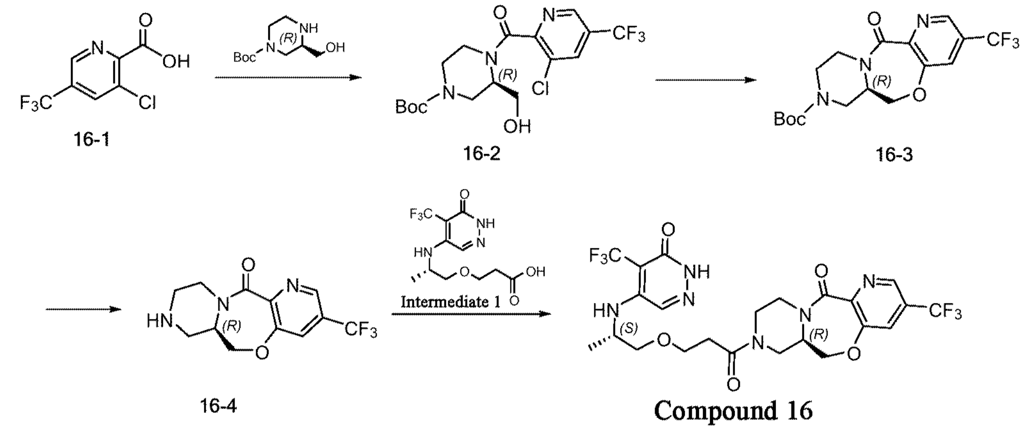


16-1 16-2 16-3 16-4 Compound 16

Step I: Synthesis of t-butyl (R)-4-(3-chloro-5-(trifluoromethyl)methylpyridinyl)-3-(hydroxymethyl)piperazin-1-carboxylate (**16-2**)

Chloro-5-(trifluoromethyl)pyridincarboxylic acid (**16-1**) (1.56 g, 6.94 mmol) and t-butyl (R)-3-(hydroxymethyl)piperazin-1-carboxylate (1.00 g, 4.62 mmol) were dissolved in dichloromethane (20 mL). DIEA (1.79 g, 13.87 mmol), 1-hydroxybenzotriazole (0.94 g, 6.94 mmol) and 1-(3-dimethylaminopropyl)-3-ethylcarbodiimide hydrochloride (1.33 g, 6.94 mmol) were sequentially added. The reaction mixture was stirred at room temperature for 1 hr. After the reaction was completed, the reaction solution was quenched with water and extracted three times with dichloromethane. The organic phase was washed with saturated brine, and dried over anhydrous sodium sulfate. Anhydrous sodium sulfate was removed by filtration, and the filtrate was rotary dried under reduced pressure to obtain a crude product as a white solid (1.20 g, yield: 61%). LCMS: MS(ESI) m/z: 424 $[M+H]^+$.

Step II: t-butyl (R)-12-oxo-3-(trifluoromethyl)-6a,7,9,10-tetrahydro-12H-pyrazino[2,1-c]pyrido[2,3-f][1,4]Oxazepane-8(6H)-carboxylate (**16-3**)

t-butyl (R)-4-(3-chloro-5-(trifluoromethyl)methylpyridinyl)-3-(hydroxymethyl)piperazin-1-carboxylate (**16-2**) (1.00 g, 2.36 mmol) was dissolved in dimethyl sulfoxide (15 mL), and then potassium carbonate (0.98 g, 7.08 mmol) was added. The reaction mixture was stirred for 2 hrs at 100°C. After the reaction was completed, the reaction solution was cooled, quenched with water, and extracted three times with ethyl acetate. The organic phase was washed with saturated brine, and dried over anhydrous sodium sulfate. Anhydrous sodium sulfate was removed by filtration, and the filtrate was rotary dried under reduced pressure to obtain a crude product as a yellow solid (0.42 g, yield: 46%). LCMS: MS(ESI) m/z: 388 $[M+H]^+$.

Step III: Synthesis of (R)-3-(trifluoromethyl)-6,6a,7,8,9,10-hexahydro-12H-pyrazino[2,1-c]pyrido[2,3-f][1,4]oxazepin-12-one (**16-4**)

t-butyl (R)-12-oxo-3-(trifluoromethyl)-6a,7,9,10-tetrahydro-12H-pyrazino[2,1-c]pyrido[2,3-f][1,4]Oxazepane-8(6H)-carboxylate (**16-3**) (0.15 g, 0.39 mmol) was dissolved in 1,4-dioxane (1.5 mL), and then a hydrochloric acid solution in dioxane (4 M,1.50 mL, 6 mmol) was added. The reaction was stirred at room temperature for 0.5 hr. After the reaction was completed, the reaction solution was rotary dried under reduced pressure to obtain a crude product as a yellow solid (0.23 g, yield: 207%). LCMS: MS(ESI) m/z: 288 $[M+H]^+$.

Step IV: Synthesis of (R)-8-(3-((S)-2-((6-oxo-5-(trifluoromethyl)-1,6-dihydropyridazin-4-yl)amino)propoxy)propionyl)-3-(trifluoromethyl)-6,6a,7,8,9,10-hexahydro-12H-pyrazino[2,1-c]pyrido[2,3-f][1,4]oxazepin-12-one **(Compound 16)**

(R)-3-(trifluoromethyl)-6,6a,7,8,9,10-hexahydro-12H-pyrazino[2,1-c]pyrido[2,3-f][1,4]oxazepin-12-one (**16-4**) (0.08 g, 0.27 mmol) were dissolved in dichloromethane (7.00 mL), and then DIEA (0.35 g, 2.69 mmol), (S)-3-(2-((6-oxo-5-(trifluoromethyl)-1,6-dihydropyridazin-4-yl)amino)propoxy)propionic acid (0.10 g, 0.32 mmol) and 1-propylphosphonic anhydride (0.43 g, 1.35 mmol) were sequentially added. The reaction mixture was stirred at room temperature for 0.5 hr. After the reaction was completed, the reaction solution was quenched with water and extracted three times with dichloromethane. The organic phases were combined, washed with saturated brine and dried over anhydrous sodium sulfate. Anhydrous sodium sulfate was removed by filtration, and the filtrate was rotary dried under reduced pressure to obtain a crude product. The crude product was purified by reverse high-performance preparative liquid chromatography

(column: YMC-Actus Triart C18, 30 mm × 150 mm, 5 um, mobile phase A: water (containing 10 mol/l ammonium bicarbonate), mobile phase B: acetonitrile, flow rate: 60 ml/ min, elution gradient: mobile phase B from 10% to 52% over 8 min, detection wavelength: 220 nm, retention time: 7.02 min) to obtain the target product as a white solid (0.05 g, yield: 32%).

$^1$H NMR (400 MHz, DMSO-$d_6$) δ (ppm): 12.43 (s, 1H), 8.88 (s, 1H), 8.07 (s, 1H), 7.91-7.90 (m, 1H), 6.30-6.20 (m, 1H), 4.43 - 4.20 (m, 2H), 4.20-4.04(m, 2H), 4.04 - 3.73 (m, 4H), 3.73 - 3.60 (m, 3H), 3.58-3.52 (m, 1H), 3.15 - 3.46 (m, 2H), 2.63-2.55 (m, 2H), 1.21-1.09 (m, 3H). LCMS: MS(ESI)m/z: 579.10 [M+H].

**Example 17:** Synthesis of (S)-8-(3-((S)-2-((6-oxo-5-(trifluoromethyl)-1,6-dihydropyridazin-4-yl)amino)propoxy)propionyl)-3-(trifluoromethyl)-6,6a,7,8,9,10-hexahydro-12H-pyrazino[2,1-c]pyrido[2,3-f][1,4]oxazepin-12-one (**Compound 17**)

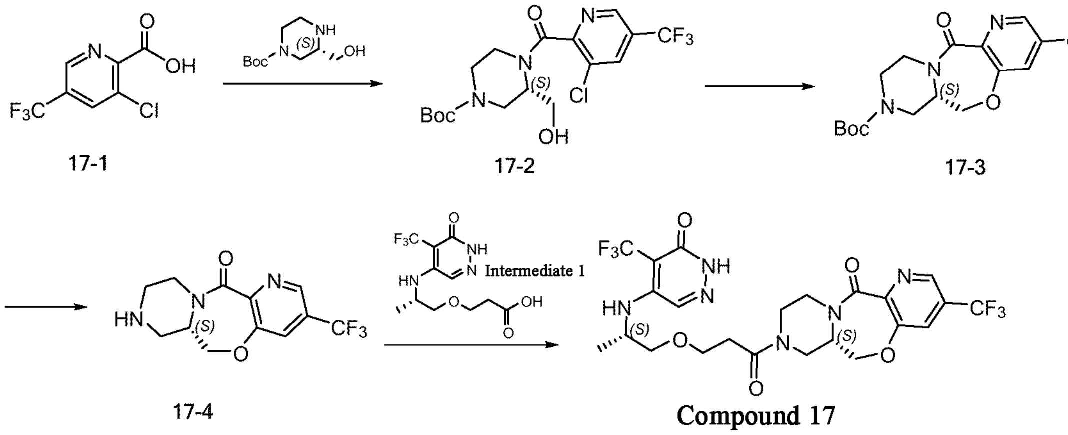


17-1 17-2 17-3 17-4 Compound 17

The synthesis steps were the same as those in the synthesis method for **Compound 16,** except that t-butyl (S)-3-(hydroxymethyl)piperazin-1-carboxylate was used in place of the reaction raw material t-butyl (R)-3-(hydroxymethyl)piperazin-1-carboxylate in Step I for reaction.

The crude product was purified by reverse high-performance preparative liquid chromatography (column: YMC-Actus Triart C18, 30 mm × 150 mm, 5 um, mobile phase A: water (containing 10 mol/l ammonium bicarbonate), mobile phase B: acetonitrile, flow rate: 60 ml/ min, elution gradient: mobile phase B from 10% to 52% over 8 min, detection wavelength: 220 nm, retention time: 7.02 min) to obtain the target product as a white solid (0.03 g, yield: 29%).

$^1$H NMR (400 MHz, DMSO-$d_6$) δ (ppm): 12.46 (s, 1H), 8.88 (s, 1H), 8.08-8.06 (m, 1H), 7.92-7.90 (m, 1H), 6.30-6.27 (m, 1H), 4.42 - 4.18 (m, 2H), 4.18-4.02 (m, 2H), 4.01 - 3.73 (m, 4H), 3.73 - 3.60 (m, 3H), 3.58-3.52 (m, 1H), 3.15 - 3.46 (m, 2H), 2.63-2.55 (m, 2H), 1.21-1.09 (m, 3H). LCMS: MS(ESI)m/z: 579.05 $[M+H]^+$.

**Example 18:** Synthesis of 5-(((S)-1-(3-oxo-3-((R)-3-(trifluoromethyl)-6a,7,9,10-tetrahydro-12H-pyrazino[2,1-c]pyrido[2,3-f][1,4]oxazepin-8(6H)-yl)propoxy)prop-2-yl)amino)-4-(trifluoromethyl)pyridazin-3(2H)-one (**Compound 18**)

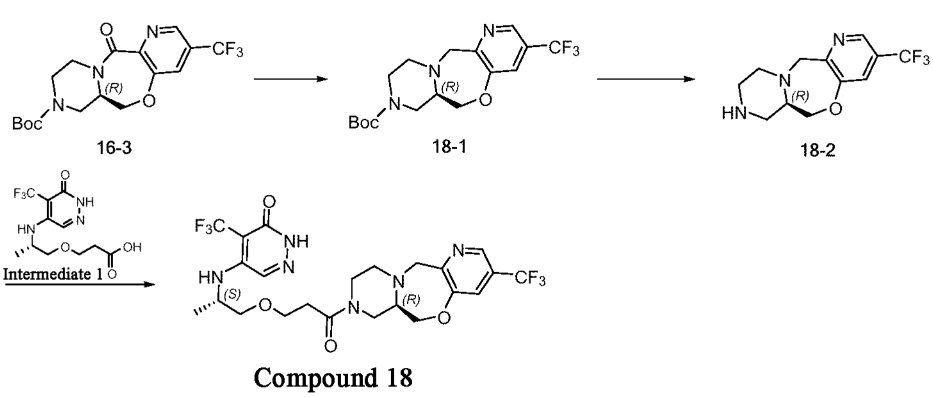


16-3 18-1 18-2 Compound 18

Step I: Synthesis of t-butyl (R)-3-(trifluoromethyl)-6a,7,9,10-tetrahydro-12H-pyrazino[2,1-c]pyrido[2,3-f][1,4]Oxazepane-8(6H)-carboxylate (**18-1**)

(R)-12-oxo-3-(trifluoromethyl)-6a,7,9,10-tetrahydro-12H-pyrazino[2,1-c]pyrido[2,3-f][1,4]Oxazepane-8(6H)-t-butyl carboxylate (**16-3**)(0.15 g, 0.39 mmol) was dissolved in tetrahydrofuran (3.00 mL), and cooled to 0°C. Boron trifluoride-diethyl etherate (1.65 g, 11.62 mmol) and sodium borohydride (0.37 g, 9.68 mmol) were added, and the reaction was stirred at 0°C for 2 hrs. After the reaction was completed, the reaction solution was quenched with water, and extracted three times with ethyl acetate. The organic phases were combined, washed with saturated brine, and dried over anhydrous sodium sulfate. Anhydrous sodium sulfate was removed by filtration, and the filtrate was rotary dried under reduced pressure to obtain a crude product as a white solid (0.23 g, yield: 157%). LCMS: MS(ESI) m/z: 374 $[M+H]^+$.

Step II: Synthesis of (R)-3-(trifluoromethyl)-6,6a,7,8,9,10-hexahydro-12H-pyrazino[2,1-c]pyrido[2,3-f][1,4]oxazepin-12-one (**18-2**)

(R)-3-(trifluoromethyl)-6a,7,9,10-tetrahydro-12H-pyrazino[2,1-c]pyrido[2,3-f][1,4]Oxazepane-8(6H)-t-butyl carboxylate (**18-1**) (0.23 g, 0.61 mmol) was dissolved in dioxane (5.00 mL), and then a hydrochloric acid solution in dioxane (4 M, 5.00 mL, 20.00 mmol) was added. The reaction mixture was stirred at room temperature for 0.5 hr. After the reaction was completed, the filtrate was rotary dried under reduced pressure to obtain a crude product as a yellow solid (0.25 g, yield: 150%). LCMS: MS(ESI) m/z: **274** $[M+H]^+$.

Step III: Synthesis of 5-(((S)-1-(3-oxo-3-((R)-3-(trifluoromethyl)-6a,7,9,10-tetrahydro-12H-pyrazino[2,1-c]pyrido[2,3-f][1,4]oxazepin-8(6H)-yl)propoxy)prop-2-yl)amino)-4-(trifluoromethyl)pyridazin-3(2H)-one (**Compound 18**)

(R)-3-(trifluoromethyl)-6,6a,7,8,9,10-hexahydro-12H-pyrazino[2,1-c]pyrido[2,3-f][1,4]oxazepin-12-one **(18-2)** (0.1 g,1 eq) was dissolved in dichloromethane (1 ml), and then N,N-diisopropylethyl amine (0.24 g, 1.83 mmol), (S)-3-(2-((6-oxo-5-(trifluoromethyl)-1,6-dihydropyridazin-4-yl)amino)propoxy)propionic acid (**Intermediate 1**) (0.113 g, 1.0 eq) and 1-propylphosphonic anhydride (0.23 g, 0.73 mmol) were added. The reaction was stirred at room temperature for 0.5 hr. After the reaction was completed, the reaction solution was quenched with water and extracted three times with dichloromethane. The organic phase was washed with saturated brine, dried over anhydrous sodium sulfate, and rotary dried under a low pressure. The crude product was purified by preparative liquid-phase chromatography (column: YMC-Actus Triart C18, 30 mm × 150 mm, 5 um, mobile phase A: water (containing 10 mol/l ammonium bicarbonate), mobile phase B: acetonitrile, flow rate: 60 ml/min, elution gradient: mobile phase B from 20% to 75% over 8 min, detection wavelength: 220 nm, retention time: 7.58 min) to obtain the target product 5-(((S)-1-(3-oxo-3-((R)-3-(trifluoromethyl)-6a,7,9,10-tetrahydro-12H-pyrazino[2,1-c]pyrido[2,3-f][1,4]oxazepin-8(6H)-yl)propoxy)prop-2-yl)amino)-4-(trifluoromethyl)pyridazin-3(2H)-one as a white solid (0.03 g, yield: 12%). $^1H$ NMR (400 MHz, DMSO-$d_6$) δ (ppm): 12.46 (s, 1H), 8.57 (s, 1H), 7.91 (d, $J$ = 4.4 Hz, 1H), 7.77 (s, 1H), 6.30-6.20 (m, 1H), 4.46-4.31 (m, 1H), 4.22-3.95 (m, 3H), 3.91-3.78 (m, 2H),3.78-3.60 (m, 3H), 3.52-3.43 (m, 2H), 3.24-2.97 (m, 1H), 2.92-2.77 (m, 2H), 2.74-2.63 (m, 2H), 2.59-2.57 (m, 1H), 2.44-2.39 (m, 1H),1.21-1.10 (m, 3H). LCMS: MS(ESI)m/z: 565.10 $[M+H]^+$.

**Example 19:** Synthesis of 5-(((S)-1-(3-oxo-3-((S)-3-(trifluoromethyl)-6a,7,9,10-tetrahydro-12H-pyrazino[2,1-c]pyrido[2,3-f][1,4]oxazepin-8(6H)-yl)propoxy)prop-2-yl)amino)-4-(trifluoromethyl)pyridazin-3(2H)-one (**Compound 19**)

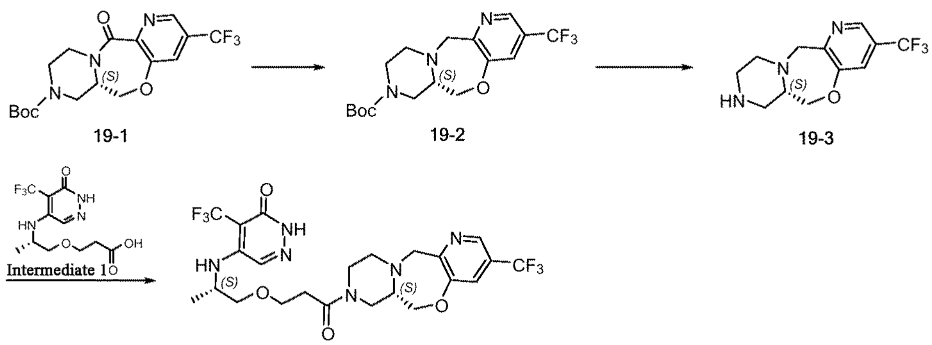

19-1 19-2 19-3

Intermediate 1

Compound 19

The synthesis steps were the same as those in the synthesis method for **Compound 18**, except that t-butyl (S)-12-oxo-3-(trifluoromethyl)-6a,7,9,10-tetrahydro-12H-pyrazino[2,1-c]pyrido[2,3-f][1,4]Oxazepane-8(6H)-carboxylate was used in place of the reaction raw material t-butyl (R)-12-oxo-3-(trifluoromethyl)-6a,7,9,10-tetrahydro-12H-pyrazino[2,1-c]pyrido[2,3-f][1,4]Oxazepane-8(6H)-carboxylate in Step I for reaction. The crude product was purified by reverse high preformance liquid preparaptive chromatography (column: YMC-Actus Triart C18, 30 mm X 150 mm, 5 um, mobile phase A: water (containing 10 mol/l ammonium bicarbonate), mobile phase B: acetonitrile, flow rate: 60 ml/ min, elution gradient: mobile phase B from 20% to 75% over 8 min, detection wavelenght: 220 nm, retention time: 7.58 min) to obtain the target product as a white solid (0.02 g, yield: 18%).

$^1$H NMR (400 MHz, DMSO-$d_6$) $\delta$(ppm): 12.47 (s, 1H), 8.57 (s, 1H), 7.91 (d, *J*=4.4 Hz, 1H), 7.77 (s, 1H), 6.30-6.20 (m, 1H), 4.46-4.31 (m, 1H), 4.18-4.02 (m, 3H), 3.99-3.75 (m, 4H), 3.72-3.60 (m, 3H), 3.52-3.43 (m, 2H), 3.05-2.97 (m, 1H), 2.92-2.77 (m, 3H), 2.74-2.69 (m, 1H), 1.21-1.10 (m, 3H). LCMS: MS(ESI)*m/z*: 565.10 [M+H]$^+$.

**Example 20:** Synthesis of (R) -9-(3((S)-2-((6-oxo-5-(trifluoromethyl)-1,6-dihydropyridazin-4-yl)amino)propoxy)propionyl)-3-fluoro-7,7a,8,9,10,11-hexahydro-5H-pyrazino[2,1-c]pyrido[2,3-e][1,4]oxazin-5-one (**Compound 20**)

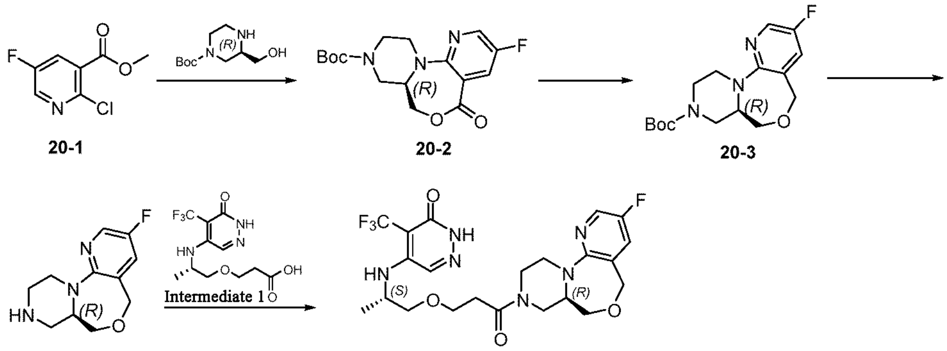


20-1 20-2 20-3

20-4 Compound 20

Step I: Synthesis of t-butyl (R)-5-oxo-3-fluoro-7a,8,10,11-tetrahydro-5H-pyrazino[2,1-c]pyrido[2,3-e][1,4]oxazolin-9(7H)-carboxylate (**20-2**)

t-butyl (3R)-3-hydroxymethyl-piperidin-1-carboxylate (900 mg,4.17 mmol) and methyl 2-fluoro-5-chloropyridin-3-carboxylate (1 g, 4.17 mmol)(**20-1**) were dissolved in DMF (20 mL), and then potassium carbonate (1.5 g, 8.4 mmol) was added. The reaction mixture was reacted for 16 hrs at 80°C with stirring. After cooling, the solid was filtered off. The filtrate was concentrated to dryness, and the crude product was added to water (100 mL), and extracted with ethyl acetate (100 mL × 3). The organic phases were combined, and dried over anhydrous sodium sulfate. Anhydrous sodium sulfate was removed by filtration, and the filtrate was rotary dried. The crude product was purified by column chromatography on silica gel to obtain the target product as a white solid (1.2 g, 3.1 mmol, yield: 60%). LCMS: MS(ESI) m/z: 338 [M+H]$^+$.

Step II: Synthesis of t-butyl (R)-3-fluoro-7a,8,10,11-tetrahydro-5H-pyrazino[2,1-c]pyrido[2,3-e][1,4]oxazolin-9(7H)-carboxylate (**20-3**)

t-butyl (R)-5-oxo-3-fluoro-7a,8,10,11-tetrahydro-5H-pyrazino[2,1-c]pyrido[2,3-e][1,4]oxazolin-9(7H)-carboxylate (**20-2**)(1 g, 2.6 mmol) was dissolved in tetrahydrofuran (8 mL), and cooled to 0°C. Solid sodium borohydride (100 mg, 2.6 mmol) was added, and boron trifluoride-diethyl etherate (370 mg, 2.6 mmol) was added dropwise with stirring. After that, the reaction mixture was reacted with stirring at room temperature for 2 hrs. The reaction solution was poured into ice water (10 ml), and the aqueous phase was extracted with dichloromethane (20 ml × 3). The organic phases were combined, and dried over anhydrous sodium sulfate. Anhydrous sodium sulfate was removed by filtration. The crude product was purified by column chromatography on silica gel to obtain the target product as a white solid (480 mg, 0.64 mmol, yield: 50%). LCMS: MS(ESI) m/z: 324[M+H]$^+$.

Step III: Synthesis of (R)-3-fluoro-7,7a,8,9,10,11-hexahydro-5H-pyrazino[2,1-c]pyrido[2,3-e][1,4]Oxazepane-5-one (**20-4**)

t-butyl (R)-5-oxo-3-fluoro-7a,8,10,11-tetrahydro-5H-pyrazino[2,1-c]pyrido[2,3-e][1,4]oxazolin-9(7H)-carboxylate (**20-3**) (1 g, 2.6 mmol) was dissolved in a hydrogen chloride solution in 1,4-dioxane (4 M, 5 mL), and reacted for 2 hrs at room temperature with stirring. After complete reaction, the reaction solution was concentrated under reduced pressure. The crude product was diluted in water (30 mL), adjusted to pH 8-9 with sodium bicarbonate, and extracted with dichloromethane (20 mL × 3). The crude product was purified by column chromatography on silica gel to obtain the target product as a white solid (500mg, 1.80 mmol, yield: 60%), LCMS: MS (ESI) m/z: 224 $[M+H]^+$.

Step IV: Synthesis of (R) -9-(3-((S)-2-((6-oxo-5-(trifluoromethyl)-1,6-dihydropyridazin-4-yl)amino)propoxy)propionyl)-3-fluoro-7,7a,8,9,10,11-hexahydro-5H-pyrazino[2,1-c]pyrido[2,3-e][1,4]oxazin-5-one (**Compound 20**)

3-[(2S)-2-{[6-oxo-5-(trifluoromethyl)-1,6-dihydropyridazin-4-yl]amino}propoxy]propionic acid (**Intermediate1**) (100 mg, 0.348 mmol) and (S)-3-fluoro-6,6a,7,8,9,10-hexahydropyrazino[1,2-d]pyrido[3,2-b][1,4] oxazine (**20-4**) (100 mg, 0.348 mmol) were dissolved in dichloromethane (2 .0 mL), and then diisopropylethyl amine (100 mg, 0.696 mmol) and 1-propylphosphonic anhydride (120 mg, 0.418 mmol, 50% solution in ethyl acetate) were added. The reaction mixture was stirred at room temperature for 2 hrs. The reaction solution was poured into ice water (20 mL), and the aqueous phase was then extracted with ethyl acetate (10 mL × 3). The organic phases were combined, and dried over anhydrous sodium sulfate. Anhydrous sodium sulfate was removed by filtration, and the filtrate was rotary dried to obtain a crude product. The crude product was purified by reverse preparative liquid chromatography (column: XBridge Prep OBD C18, 30 × 150 mm 5 um, mobile phase A: water (containing 10 mol/l ammonium bicarbonate), mobile phase B: acetonitrile, flow rate: 60 ml/ min, elution gradient: mobile phase B from 20% to 52% over 8 min, detection wavelength: 220 nm, retention time: 6.21 min) to obtain the target product as a white solid (52 mg, 0.090 mmol, yield: 25.01%).

$^1H$ NMR (400 MHz, DMSO-$d_6$) δ (ppm): 12.41 (s, 1H), 8.12 (s, 1H),8.07 (d, *J* = 2.2 Hz, 1H), 7.90 (d, *J* = 2.2 Hz, 1H), 7.72-7.70 (m, 1H), 6.28-6.23 (m, 1H), 4.38-4.22 (m, 1H), 4.19-3.89 (m, 2H), 3.80-3.62 (m, 6H), 3.53-3.45 (m, 2H), 3.25-3.12(m,1H),2.95-2.83 (m, 1H), 2.83-2.70 (m, 2H),2.65-2.57(m,2H).2.38-2.26(m,1H), 1.20-1.09 (m , 3H). LCMS: MS(ESI)m/z: 515.15 $[M+H]^+$.

**Example 21:** Synthesis of (R) -9-(3-((S)-2-((6-oxo-5-(trifluoromethyl)-1,6-dihydropyridazin-4-yl)amino)propoxy)propionyl)-3-chloro-7,7a,8,9,10,11-hexahydro-5H-pyrazino[2,1-c]pyrido[2,3-e][1,4]oxazin-5-one (**Compound 21**)

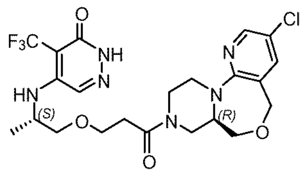

Compound 21

The synthesis method was the same as that in **Example 20.** The crude product was purified by reverse preparative liquid chromatography (column: XBridge Prep OBD C18, 30 × 150 mm 5 um, mobile phase A: water (containing 10 mol/l ammonium bicarbonate), mobile phase B: acetonitrile, flow rate: 60 ml/ min, elution gradient: mobile phase B from 20% to 52% over 8 min, detection wavelength: 220 nm, retention time: 6.10 min) to obtain the target product (R) -9-(3-((S)-2-((6-oxo-5-(trifluoromethyl)-1,6-dihydropyridazin-4-yl)amino)propoxy)propionyl)-3-chloro-7,7a,8,9,10,11-hexahydro-5H-pyrazino[2,1-c]pyrido[2,3-e][1,4]oxazin-5-one (**Compound 21**) as a white solid (52 mg, 0.090 mmol, yield: 25.01%).

$^1H$ NMR (400 MHz, DMSO-$d_6$) δ (ppm): 12.41 (brs, 1H), 8.12 (s, 1H), 7.90 (d, *J* = 2.2 Hz, 1H), 7.86 (s, 1H), 6.28-6.22 (m, 1H), 4.42-4.27 (m, 1H), 4.19-4.11 (m, 1H), 4.11-3.95 (m, 1H), 3.80-3.68 (m, 4H), 3.68-3.63 (m, 2H), 3.50-3.46 (m, 2H), 3.25-3.16(m, 1H), 2.95-2.82 (m,1H), 2.81-2.79 (m, 1H), 2.78-2.70 (m, 1H), 2.62-2.58(m,2H). 2.31-2.22(m,1H), 1.18-1.13 (m, 3H). LCMS: MS(ESI)m/z: 531.10 $[M+H]^+$.

**Example 22:** Synthesis of 5-(((S)-1-(3-((R)-6,6-dioxa-3-(trifluoromethyl)-7a,8,10,11-tetrahydro-5H-pyrazino[2,1-c]pyrido[2,3-e][1,4]]thiazin-9(7H)-yl)-3-oxypropoxy)prop-2-yl)amino)-4-(trifluoromethyl)pyridazin-3(2H)-one (**Compound 22**)

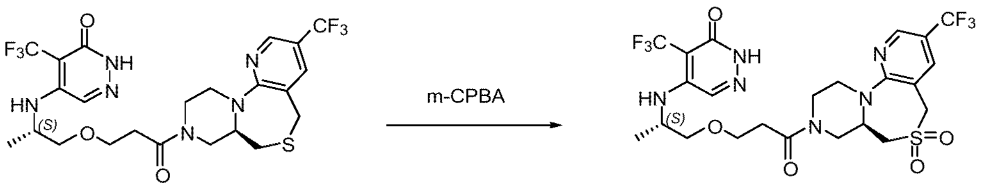


Compound 12 Compound 22

5-(((S)-1 -(3 -oxo-3 -((R)-3 -(trifluoromethyl)-7 a, 8,10,11-tetrahydro-5H-pyrazino[2,1-c]pyrido[2,3-e][1,4]]thiazin-9(7H)-yl)propoxy)prop-2-yl)amino)-4-(trifluoromethyl)pyridazin-3(2H)-one **(Compound 12)** (125.00 mg, 0.22 mmol) was dissolved in methanol (25.00 mL), cooled 0°C, and then added with m-chloroperoxybenzoic acid (74.31 mg, 0.43 mmol). The reaction was stirred at 0°C for 1 hr. After the reaction was completed, methanol was removed by rotary evaporation under reduced pressure. The crude product was purified by reverse high-performance preparative liquid chromatography (column: Xselect CSH OBD Column 30*150 mm 5 um, mobile phase A: mobile phase A: water (containing 10 mol/l ammonium bicarbonate), mobile phase B: acetonitrile, flow rate: 60 ml/ min, elution gradient: mobile phase B from 10% to 55% over 8 min, detection wavelength: 220 nm, retention time: 7.27 min) to obtain the target product as a white solid (1.22 mg, yield: 1%).

$^1$H NMR (400 MHz, DMSO-$d_6$) δ (ppm): 12.46 (s, 1H), 8.55-8.50 (m, 1H),8.05-7.95 (m, 1H), 7.92 (s, 1H), 6.35-6.25 (m, 1H), 5.17-5.03 (m, 1H), 4.57-4.48 (m, 1H),4.36-4.28 (m, 1H), 4.18-4.09 (m, 1H), 4.01-3.84 (m, 2H), 3.75-3.55 (m, 8H), 3.52-3.44 (m, 3H), 2.60-2.58 (m, 1H),1.18-1.12 (m, 3H). LCMS: MS(ESI)m/z: 613.15 $[M+H]^+$.

**Example 23:** Synthesis of 5-(((S)-1-(3-((S)-6-methyl-3-(trifluoromethyl)-6,7,7a,8,10,11-octahydropyrazino[1,2-a] pyrido[3,2-f][1,4]Diazepin-9(5H)-yl)-3-propoxy)prop-2-yl)amino)-4-(trifluoromethyl) pyridazin-3(2H)-one (**Compound 23**)

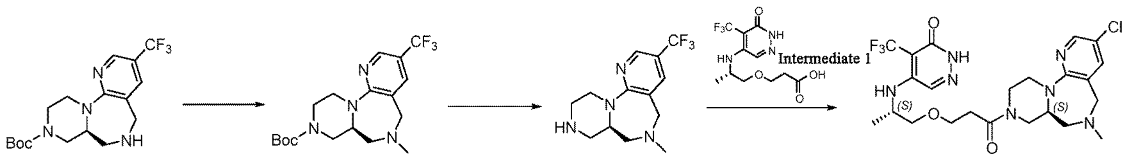


10-6 23-1 23-2 Compound 23

Step I: Synthesis of (S)-6-methyl-3-(trifluoromethyl)-6,7,7a,8,9,10,11-octahydropyrazino[1,2-a]pyrido[3,2-f][1,4]Diazepin-9(5H)-t-butyl carboxylate (**23-1**)

t-butyl (S)-3-(trifluoromethyl)-6,7,7a,8,10,11-octahydropyrazino[1,2-a]pyrido[3,2-fl[1,4]Diazepin-9(5H)-carboxylate (**10-6**) (100 mg,0.27 mmol) was dissolved in methanol (2 mL), cooled to 0°C, and added with solid sodium borohydride (114 mg, 0.54 mmol). A 30% formaldehyde aqueous solution (135 mg, 1.35 mmol) was added dropwise with stirring. After that, the reaction mixture was reacted with stirring at room temperature for 2 hrs. The reaction solution was poured into ice water (10 ml), and the aqueous phase was extracted with dichloromethane (20 ml × 3). The organic phases were combined, and dried over anhydrous sodium sulfate. Anhydrous sodium sulfate was removed by filtration, and the filtrate was concentrated under reduced pressure to obtain a crude product. The crude product was purified by column chromatography on silica gel target product as a white solid (65 mg,0.321 mmol, yield: 58%). LCMS: MS(ESI) m/z: 387 $[M+H]^+$.

Step II: Synthesis of (R)-6-methyl-3-(trifluoromethyl)-5,6,7,7a,8,9,10,11-octahydropyrazino[1,2-a]pyrido[3,2-f] [1,4]diazepine (**23-2**)

t-butyl (S)-6-methyl-3-(trifluoromethyl)-6,7,7a,8,9,10,11-octahydropyrazino[1,2-a]pyrido[3,2-f][1,4]Diazepin-9(5H)-carboxylate (**23-1**)(65 mg,0.168 mmol) was dissolved in ice-cold (0°C) hydrogen chloride-1,4-dioxane solution (4 M,5 mL), slowly warmed to room temperature and reacted for 2 hrs with stirring. After complete reaction, the reaction solution was concentrated under reduced pressure. The crude product was diluted in water (30 mL), adjusted to pH 8-9 with sodium bicarbonate, and extracted with dichloromethane (20 mL × 3). The crude product was purified by column chromatography on silica gel to obtain the target product as a white solid (40 mg,0.139 mmol, yield: 89%). LCMS: MS(ESI) m/z: 287 $[M+H]^+$.

Step III: Synthesis of 5-(((S)-1-(3-((S)-6-methyl-3-(trifluoromethyl)-6,7,7a,8,10,11-octahydropyrazino[1,2-a] pyrido[3,2-f][1,4] Diazepin-9(5H)-yl)-3-propoxy)prop-2-yl)amino)-4-(trifluoromethyl) pyridazin-3(2H)-one (**Compound 23**)

3-[(2S)-2- {[6-oxo-5-(trifluoromethyl)-1,6-dihydropyridazin-4-yl]amino}propoxy]propionic acid (**intermediate 1**) (43 mg, 0.139 mmol) and (R)-6-methyl-3-(trifluoromethyl)-5,6,7,7a,8,9,10,11-octahydropyrazino[1,2-a]pyrido[3,2-f][1,4]diazepine (**23-2**) (40 mg, 0.139 mmol) were dissolved in dichloromethane (1.0 mL), and then diisopropylethyl amine (0.023 mL, 0.139 mmol) and T3P (44.20 mg, 0.139 mmol, 50% solution in ethyl acetate) were added. The reaction mixture was stirred at room temperature for 2 hrs. The reaction solution was poured into ice water (20 mL), and then the aqueous phase was extracted with ethyl acetate (10 mL × 3). The organic phases were combined, and dried over anhydrous sodium sulfate. Anhydrous sodium sulfate was removed by filtration, and the filtrate was rotary dried to obtain a crude product. The crude product was purified by reverse high performance preparative liquid chromatography (column: XBridge Prep OBD C18, 30 × 150 mm 5 um, mobile phase A: water (containing 10 mol/l ammonium bicarbonate), mobile phase B: acetonitrile, flow rate: 60 ml/ min, elution gradient: mobile phase B from 20% to 52% over 8 min, detection wavelength: 220 nm, retention time: 7.28 min) to obtain the target product as a white solid (1.2 mg, 0.002 mmol, yield: 1.5%). $^1$H NMR (400 MHz, DMSO-$d_6$) $\delta$ (ppm): 12.47 (s, 1H), 8.36 (d, $J$ = 8.0 Hz, 1H), 7.91 (d, $J$ = 4.0 Hz, 1H), 7.73 (s, 1H), 6.34-6.29 (m, 1H), 4.19-4.11 (m, 1H), 3.97-3.81 (m, 4H), 3.77-3.67 (m, 4H), 3.61-3.53 (m, 2H), 3.49 (d, $J$ = 4.0 Hz, 2H), 2.77-2.74 (m, 2H), 2.68-2.55 (m, 3H), 2.32 (s, 3H), 1.16-1.13 (m, 3H). LCMS: MS(ESI)m/z: 578.20 [M+H]$^+$.

**Example 24:** Synthesis of 5-(((S)-1-(3-((6aR,9R)-9-methyl-3-(trifluoromethyl)-6a,7,9,10-tetrahydropyrazino[1,2-d]pyrido[3,2-b][1,4]oxazin-8(6H)-yl)-3-oxypropoxy)prop-2-yl)amino)-4-(trifluoromethyl)pyridazin-3(2H)-one (**Compound 24**)

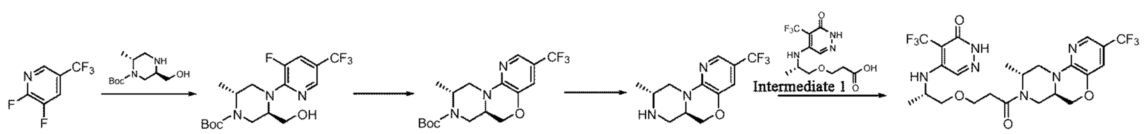


24-1 24-2 24-3 24-4 Compound 24

Step I: Synthesis of t-butyl (2R,5R)-4-(3-fluoro-5-(trifluoromethyl)pyridin-2-yl)-5-(hydroxymethyl)-2-methylpiperazin-1-carboxylate (**24-2**)

2,3-Difluoro-5-(trifluoromethyl)pyridine (**24-1**) (0.40 g, 2.17 mmol) and (2R,5R)-5-(hydroxymethyl)-2-methylpiperazin-1-t-butyl carboxylate (0.50 g, 2.17 mmol) were dissolved in DMF (25.00 mL), and then DIEA (0.56 g, 4.34 mmol). The reaction mixture was stirred at 50°C for 3 hrs. After the reaction was completed, the solution was quenched with water and extracted three times with ethyl acetate. The organic phases were combined, washed with saturated brine, and dried over anhydrous sodium sulfate. Anhydrous sodium sulfate was removed by filtration, and the filtrate was concentrated under reduced pressure to obtain a crude product as a yellow oil (0.72 g, yield: 84%). LCMS: MS(ESI) m/z: 394 [M+H]$^+$.

Step II: Synthesis of t-butyl (6aR,9R)-9-methyl-3-(trifluoromethyl)-6a,7,9,10-tetrahydropyrazino[1,2-d]pyrido[3,2-b][1,4]oxazin-8(6H)-carboxylate (**24-3**)

t-butyl (2R,5R)-4-(3-fluoro-5-(trifluoromethyl)pyridin-2-yl)-5-(hydroxymethyl)-2-methylpiperazin-1-carboxylate (**24-2**) (0.50 g, 1.27 mmol) was dissolved in DMF (10.00 mL), and then potassium tert-butoxide (0.29 g, 2.54 mmol) was added. The reaction was stirred overnight at room temperature. After the reaction was completed, the solution was quenched with water and extracted three times with ethyl acetate. The organic phase was washed with saturated brine, and dried over anhydrous sodium sulfate. Anhydrous sodium sulfate was removed by filtration, and the filtrate was concentrated under reduced pressure to obtain the target product as a yellow oil (0.48 g, yield: 101%). LCMS: MS(ESI) m/z: 374 [M+H]$^+$.

Step III: Synthesis of (6aR,9R)-9-methyl-3-(trifluoromethyl)-6,6a,7,8,9,10-hexahydropyrazino[1,2-d]pyrido[3,2-b][1,4]oxazine (**24-4**)

t-butyl (6aR,9R)-9-methyl-3-(trifluoromethyl)-6a,7,9,10-tetrahydropyrazino[1,2-d]pyrido[3,2-b][1,4]oxazin-8(6H)-carboxylate (**24-3**) (0.34 g, 0.91 mmol) was dissolved in dioxane (5.00 mL), and then a 4 mol/l hydrogen chloride solution in dioxane (5.00 mL, 20.00 mmol) was added. The reaction was stirred at room temperature for half an hour. After the reaction was completed, the reaction solution was concentrated under reduced pressure to obtain the target

product as a yellow solid (0.35 g, yield: 141%). LCMS: MS(ESI) m/z: 274 [M+H]+.

Step IV: Synthesis of 5-(((S)-1-(3-((6aR,9R)-9-methyl-3-(trifluoromethyl)-6a,7,9,10-tetrahydropyrazino[1,2-d]pyrido[3,2-b][1,4]oxazin-8(6H)-yl)-3-oxypropoxy)prop-2-yl)amino)-4-(trifluoromethyl)pyridazin-3(2H)-one (**Compound 22**)

(6aR,9R)-9-methyl-3-(trifluoromethyl)-6,6a,7,8,9,10-hexahydropyrazino[1,2-d]pyrido[3,2-b][1,4]oxazine (**24-4**) ((100.00 mg, 0.32 mmol) and (S)-3-(2-((6-oxo-5-(trifluoromethyl)-1,6-dihydropyridazin-4-yl)amino)propoxy)propionic acid (**intermediate 1**) (88.37 mg, 0.32 mmol) were dissolved in dichloromethane (10.00 mL). DIEA (417.93 mg, 3.23 mmol) and 1-propylphosphonic anhydride (T3P, 50% solution in ethyl acetate,1028.91 mg, 1.62 mmol) were added. The reaction mixture was stirred at room temperature for 0.5 hr. After the reaction was completed, the reaction solution was quenched with water and extracted three times with dichloromethane. The organic phase was washed with saturated brine and dried over anhydrous sodium sulfate. Anhydrous sodium sulfate was removed by filtration, and the filtrate was concentrated to obtain a crude product. The crude product was purified by reverse high-performance preparative liquid chromatography (column: XBridge Prep OBD C18 Column, 30 * 150 mm , 5 μm, mobile phase A: water (containing 10 mol/l ammonium bicarbonate), mobile phase B: acetonitrile, flow rate: 60 ml/ min, elution gradient: mobile phase B from 20% to 60% over 8 min, detection wavelength: 220 nm, retention time: 7.48 min) to obtain the target product as a white solid (31.98 mg, yield: 18%).

$^1$H NMR (400 MHz, DMSO-$d_6$) δ (ppm): 12.46 (s, 1H), 8.09-7.96 (m, 1H), 7.96-7.83 (m, 1H), 7.30-7.16 (m, 1H), 6.26-6.19 (m, 1H), 4.48-4.17 (m, 3H), 4.17-3.92 (m, 2H), 3.87-3.47 (m, 6H), 3.47-3.37 (m, 2H ), 2.62-2.55 (m, 1H), 2.46-2.38 (m, 1H), 1.23-1.02 (m, 6H). LCMS: MS(ESI)m/z: 565.15 [M+H]+.

**Example 25:** Synthesis of 5-(((S)-1-(3-((6aR,9R)-9-methyl-3-(trifluoromethyl)-6a,7,9,10-tetrahydropyrazino[1,2-d]pyrido[3,2-b][1,4]oxazin-8(6H)-yl)-3-oxypropoxy)prop-2-yl)amino)-4-(trifluoromethyl)pyridazin-3(2H)-one (**Compound 25**)

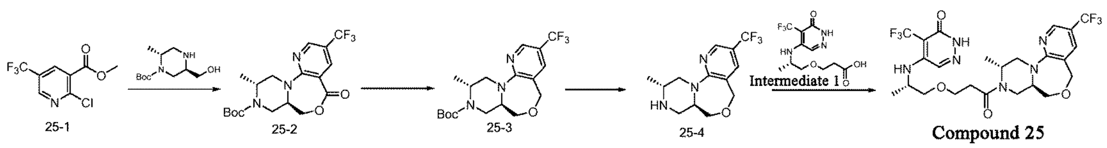


25-1 25-2 25-3 25-4 Compound 25

The synthesis method was the same as that in **Example 20.** Compound **25** as a crude product was purified by reverse high performance preparative liquid chromatography (column: XBridge Prep OBD C18 Column, 30 * 150 mm , 5 μm, mobile phase A: water (containing 10 mol/l ammonium bicarbonate), mobile phase B: acetonitrile, flow rate: 60 ml/ min, elution gradient: mobile phase B from 20% to 60% over 8 min, detection wavelength: 220 nm, retention time: 7.48 min) to obtain the target product as a white solid (0.06 g, yield: 31%).

$^1$H NMR (400 MHz, DMSO-$d_6$) δ (ppm): 12.46 (m, 1H), 8.43 (s, 1H), 8.00-7.73 (m, 2H), 6.28 (s, 1H), 5.00-4.89 (m, 1H), 4.39-4.28 (m, 1H), 4.26-3.94 (m, 4H), 3.94-3.77 (m, 2H), 3.77-3.62 (m, 3H), 3.62-3.55 (m, 1H),3.52-3.41 (m, 3H), 2.69-2.59 (m, 1H ), 2.48-2.40 (m, 1H), 1.26-1.04 (m, 6H). LCMS: MS(ESI)m/z: 579.15 [M+H]+.

**Example 26:** Synthesis of 3-oxo-5-((S)-1-(3-oxo-3-((R)-3-(trifluoromethyl)-7a,8,10,11-tetrahydro-*5H*-pyrazino[2,1-c]pyrido[2,3-e][1,4]oxazepan-9(*7H*)-yl)propoxy)prop-2-yl)amino)-2,3-dihydropyridazin-4-carbonitrile (**Compound 26**)

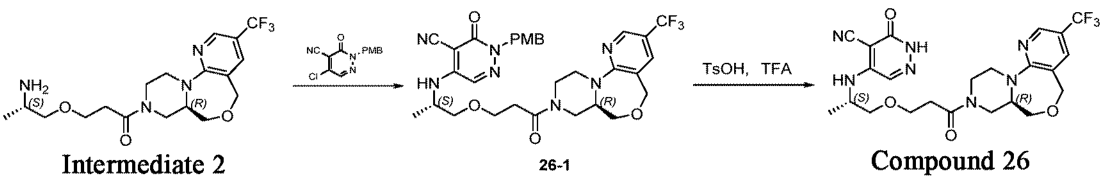


Intermediate 2 26-1 Compound 26

Step I: Synthesis of 2-(4-methoxybenzyl)-3-oxo-5-(((S)-1-(3-oxo-3-((R)-3-(trifluoromethyl)-7a,8,10,11-tetrahydro-*5H*-pyrazino[2,1-c]pyrido[2,3-e][1,4]oxazepan-9(*7H*)-yl)propoxy)prop-2-yl)amino)-2,3-dihydropyridazin-4-carbonitrile **(26-1)**

To a solution ( 5mL) of the mixture of 3-((S)-2-aminopropoxy)-1-((R)-3-(trifluoromethyl)-7a,8,10,11-tetrahydro-5*H*-pyrazino[2,1-c]pyrido[2,3-e][1,4]oxazepan-9(7*H*)-yl)prop-1-one (77 mg, 0.19 mmol) and potassium carbonate (52 mg, 0.38 mmol) in DMF, 5-chloro-2-(4-methoxybenzyl)-3-oxo-2,3-dihydropyridazin-4-carbonitrile (52mg, 0.19mmol) was added, and stirred at 80°C for 2 hrs. The reaction solution was then extracted with ethyl acetate (10 mL × 2) and water (10 mL). The organic phases were combined, washed with saturated brine (10 mL × 2), dried over anhydrous sodium sulfate, and filtered. The filtrate was concentrated under reduced pressure to obtain the crude target product 2-(4-methoxybenzyl)-3-oxo-5-(((S)-1-(3-oxo-3-((R)-3-(trifluoromethyl)-7a,8,10,11-tetrahydro-5*H*-pyrazino[2,1-c]pyrido[2,3-e][1,4]oxazepan-9(7*H*)-yl)propoxy)prop-2-yl)amino)-2,3-dihydropyridazin-4-carbonitrile as a yellow solid (15 mg, 0.02 mmol, yield: 18%). The crude product was used directly in the next reaction without purification. LCMS: MS(ESI)m/z: 642 $[M+H]^+$.

Step II: Synthesis of 3-oxo-5-((S)-1-(3-oxo-3-((R)-3-(trifluoromethyl)-7a,8,10,11-tetrahydro-5*H*-pyrazino[2,1-c]pyrido[2,3-e][1,4]oxazepan-9(7*H*)-yl)propoxy)prop-2-yl)amino)-2,3-dihydropyridazin-4-carbonitrile (**Compound 26**)

A solution of 2-(4-methoxybenzyl)-3-oxo-5-(((S)-1-(3-oxo-3-((R)-3-(trifluoromethyl)-7a,8,10,11-tetrahydro-5*H*-pyrazino[2,1-c]pyrido[2,3-e][1,4]oxazepan-9(7*H*)-yl)propoxy)prop-2-yl)amino)-2,3-dihydropyridazin-4-carbonitrile (15 mg, 0.02 mmol) in dichloromethane (1 mL) was added with p-toluenesulfonic acid (12 mg, 0.06 mmol) and trifluoroacetic acid (8 mg, 0.06 mmol) and stirred at room temperature for 10 min. The reaction solution was extracted with dichloromethane (10 mL × 2) and water (10 mL), washed with saturated brine (10 mL × 2), dried over anhydrous sodium sulfate, and filtered. The filtrate was concentrated under reduced pressure, and purified by high-performance preparative liquid chromatography (column: XBridge Prep OBD C18, 30 × 150 mm 5 um, mobile phase A: water (containing 10 moL/L ammonium bicarbonate), mobile phase B: acetonitrile, flow rate: 60 mL/min, elution gradient: mobile phase B from 10% to 50% over 8 min, detection wavelength: 220 nm, retention time: 8.6 min) to obtain the target product as a white solid (6.41 mg, 0.01 mmol, yield: 52%).

$^1$H NMR(400 MHz, DMSO-$d_6$) δ(ppm): 12.46(brs, 1H), 8.50-8.40(m, 1H), 8.35-7.10(m, 3H), 4.95-4.85(m, 1H), 4.58-4.48(m, 1H), 4.48-4.08(m, 1H), 4.08-3.91(m, 2H), 3.91-3.58(m, *7H*), 3.55-3.40(m, 3H), 3.32-3.22(m, 1H), 2.62-2.56(m, 2H), 1.17(d, *J* = 4.2Hz, 3H). LCMS: MS(ESI)m/z: 522.30$[M+H]^+$.

**Example 27:** Synthesis of (7aR)-5-methyl-3-(trifluoromethyl)-7,7a,8,9,10,11-hexahydro-5*H*-pyrazino[2,1-c]pyrido[2,3-e][1,4]thiazoline **(Compound 27)**

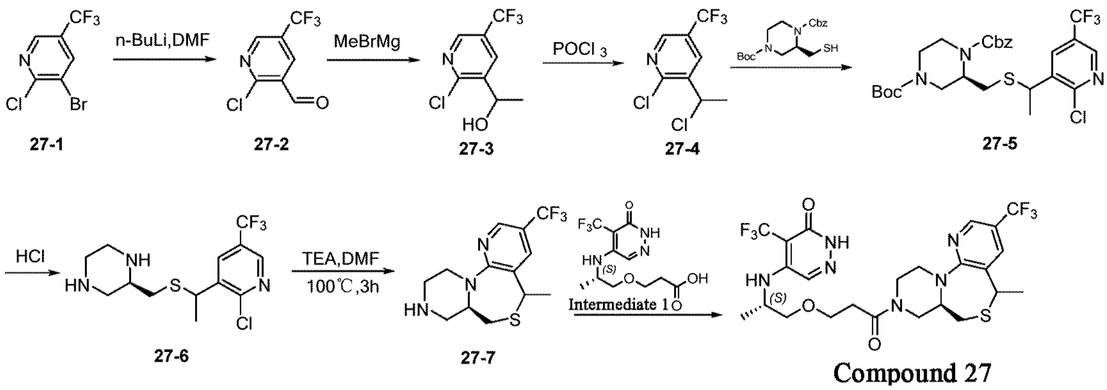


Step I: Synthesis of 2-chloro-5-(trifluoromethyl)nicotinaldehyde **(27-2)**

bromo-2-chloro-5-(trifluoromethyl)pyridine (9 g, 35 mmol) was dissolved in tetrahydrofuran (50 mL), and n-butyl lithium (15.4 mL, 38.5 mmol, 2.5 mol/L solution in n-hexane) was added dropwise to the mixture at -65°C. The mixture was reacted for 1 hr with stirring under nitrogen atmosphere at -65°C, and then DMF (5.1 g, 70 mmol) was added dropwise at -65°C and stirred for 1 hr at this temperature. After complete reaction, the mixture was quenched with a saturated ammonium chloride solution (100 mL), and then extracted with ethyl acetate (50 mL × 2). The organic phases were combined and dried over anhydrous sodium sulfate. Anhydrous sodium sulfate was removed by filtration, and the filtrate was rotary dried. The crude product was purified by column chromatography on silica gel (dichloromethane: methanol=10: 90) to obtain the target product as a brown oil (6 g, 29 mmol, yield: 80%), LCMS (ESI) m/z: 210 $[M+H]^+$.

Step II: Synthesis of 1-(2-chloro-5-(trifluoromethyl)pyridin-3-yl)ethyl-1-ol (**27-3**)

chloro-5-(trifluoromethyl)nicotinaldehyde (6 g, 29 mmol) were dissolved in tetrahydrofuran (70 mL), and methylmagnesium bromide (29 mL, 29 mmol, 1 mol/L solution in diethyl ether) was added dropwise to the mixture. The mixture was reacted for 1 hr with stirring at 0°C under nitrogen atmosphere. After the reaction was completed, the mixture was added with brine (100 mL) and extracted with ethyl acetate (50 mL × 2). The organic phases were combined and dried over anhydrous sodium sulfate. Anhydrous sodium sulfate was removed by filtration, and the filtrate was rotary dried. The crude product was purified by column chromatography on silica gel (ethyl acetate: petroleum ether=40: 60) to obtain the target product as a yellow oil (4g, 18mmol, yield : 62%), LCMS (ESI) m/z: 226[M+H]$^+$.

Step III: Synthesis of 2-chloro-3-(1-chloroethyl)-5-(trifluoromethyl)pyridine (**27-4**)

1-(2-chloro-5-(trifluoromethyl)pyridin-3-yl)ethyl-1-ol (1.3 g, 5.8 mmol) was dissolved in phosphorus oxychloride (100mL), and the mixture was heated to 60°C under nitrogen atmosphere, and reacted overnight with stirring. The reaction mixture was cooled and concentrated under reduced pressure. The residue was then dissolved in ethyl acetate (50 mL), and adjusted to pH 8 with a sodium bicarbonate solution. The aqueous phase was separated and then extracted with ethyl acetate (30 mLx 2). The organic phases were combined and dried over anhydrous sodium sulfate. Anhydrous sodium sulfate was removed by filtration, and the filtrate was rotary dried. The crude product was purified by column chromatography on silica gel (ethyl acetate: petroleum ether=50: 50) to obtain the target product as a yellow solid (0.9 g, 3.7 mmol, yield: 63%), LCMS (ESI) m/z: 244 [M+H]$^+$.

Step IV: Synthesis of t-butyl 1-benzyl 4-(2R)-2-((1-(2-chloro-5-(trifluoromethyl)pyridin-3-yl)ethyl)thio)methyl)piperazin-1,4-dicarboxylate (**27-5**)

chloro-3-(1-chloroethyl)-5-(trifluoromethyl)pyridine (0.9 g, 3.7 mmol) and 1-benzyl 4-(t-butyl) (R)- 2-(sulfydrylmethyl)piperazin-1,4-dicarboxylate (1.35 g, 3.9 mmol) were dissolved in DMF(20 mL), and then potassium carbonate (1.16 g, 8 mmol) was added. The mixture was reacted for 1 hr with stirring at room temperature under nitrogen atmosphere. After the reaction was completed, the mixture was filtered, added with water (40 mL), and extracted with ethyl acetate (30 mL × 2). The organic phases were combined, and dried over anhydrous sodium sulfate. Anhydrous sodium sulfate was removed by filtration, and the filtrate was rotary dried. The crude product was purified by column chromatography on silica gel (ethyl acetate: petroleum ether=40: 60) to obtain the target product as a yellow oil (820 mg, 1.4 mmol, yield: 37%), LCMS (ESI) m/z: 574[M+H]$^+$.

Step V: Synthesis of (2R)-2-((1-(2-chloro-5-(trifluoromethyl)pyridin-3-yl)ethyl)thio)methyl)piperazine (**27-6**)

t-butyl 1-benzyl 4-(2R)-2-((1-(2-chloro-5-(trifluoromethyl)pyridin-3-yl)ethyl)thio)methyl)piperazin-1,4-dicarboxylate (820 mg, 1.4 mmol) was dissolved in concentrated hydrochloric acid (10 mL). The mixture was heated to 50°C under nitrogen atmosphere, and reacted for 3 hrs with stirring. After complete reaction, the mixture was rotary dried, and the resulting product was purified by column chromatography (acetonitrile: water =50: 50) to obtain the target product as a yellow oil (300mg, 0.88mmol, yield: 62%) , LCMS: (ESI) m/z: 340 [M+H]$^+$.

Step VI: Synthesis of (7aR)-5-methyl-3-(trifluoromethyl)-7,7a,8,9,10,11-hexahydro-*5H*-pyrazino[2,1-c]pyrido[2,3-e][1,4]thiazoline (**27-7**)

(2R)-2-((1-(2-chloro-5-(trifluoromethyl)pyridin-3-yl)ethyl)thio)methyl)piperazine (600mg, 0.88mmol) and triethyl amine (100 mg, 1.76 mmol) were dissolved in DMF (10 mL), and the mixture was heated to 100°C under nitrogen atmosphere, and reacted for 3 hrs with stirring. The reaction mixture was cooled, added with water (30 mL), and quenched with ethyl acetate (30 mL × 3). The organic phases were combined, and dried over anhydrous sodium sulfate. Anhydrous sodium sulfate was removed by filtration, and the filtrate was rotary dried. The crude product was purified by column chromatography on silica gel (ethyl acetate: petroleum ether=50: 50) to obtain the target product as a yellow oil (50 mg, 0.16 mmol, yield: 18%), LCMS: (ESI)m/z: 304[M+H]$^+$.

Step VII: Synthesis of 5-((S)-1-(3-oxo-3-((S)-3-(trifluoromethyl)-6a,7,9,10-tetrahydropyrazino[1,2-d]pyrido[3,2-b][1,4]thiazin-8(6H)-yl)propoxy)prop-2-yl)amino)-4-(trifluoromethyl)pyridazin-3(*2H*)-one (**Compound 27**)

3-[(2S)-2- {[6-oxo-5-(trifluoromethyl)-1,6-dihydropyridazin-4-yl]amino}propoxy]propionic acid (50 mg, 0.16 mmol) and (7aR)-5-methyl-3-(trifluoromethyl)-7,7a,8,9,10,11-hexahydro-*5H*-pyrazino[2,1-c]pyrido[2,3-e][1,4]thiazoline (50 mg, 0.16 mmol) were dissolved in dichloromethane (5 .0mL), and then diisopropylethyl amine (61 mg, 0.48 mmol)

and $T_3P$ (153 mg, 0.24 mmol, 50% solution in ethyl acetate) were added. The reaction mixture was stirred at room temperature for 2 hrs. The reaction solution was poured into ice water (20mL), and the aqueous phase was then extracted with ethyl acetate (10 mL × 3). The organic phases were combined and dried over anhydrous sodium sulfate. Anhydrous sodium sulfate was removed by filtration, and the filtrate was rotary dried to obtain a crude product. The crude product was purified by preparative liquid-phase chromatography (column: YMC-Actus Triart C18, 30 × 150 mm, 5 μm; mobile phase A: water (containing 10 mol/L ammonium bicarbonate), mobile phase B: acetonitrile, flow rate: 60 mL/min, elution gradient: mobile phase B from 25% to 60% over 8 min, detection wavelength: 220 nm, retention time: 9.12 min) to obtain the target product as a white solid (2.29 mg, 0.004 mmol, yield: 2.5%).

$^1$H NMR(400 MHz, DMSO-$d_6$,) δ(ppm): 12.32(brs, 1H), 8.62-8.49(m, 1H), 7.96-7.80(m, 2H), 6.29-6.27(m, 1H), 4.56-4.47(m, 1H), 4.40-4.21(m, 1H), 4.15-4.07(m, 1H), 4.01-3.89(m, 1H), 3.82-3.64(m, 3H), 3.62-3.53(m, 1H), 3.52-3.50(m, 2H), 3.45-3.40(m, 1H), 3.26-3.23(m, 1H), 3.17-3.12(m, 1H), 3.07-3.02(m, 1H), 2.86-2.78(m, 1H), 2.63-2.57(m, 2H), 1.57(d, J = 6.0 Hz, 3H), 1.16(d, J = 6.4 Hz, 3H). LCMS: MS(ESI) m/z: 595.25[M+H]$^+$.

**Example 28:** S-(((S)-1-(3-((7R,7 AR)-7-methyl-3-(trifluoromethyl)-7a,8, 10, 11-tetrahydro-*5H*-pyrazino[2,1-c]pyrido[2,3-e][1,4]oxazepan-9(*7H*-yl)-3-oxypropoxy)prop-2-yl)amino)-4-(trifluoromethyl)pyridazin-3(*2H*)-one **(Compound 28)**

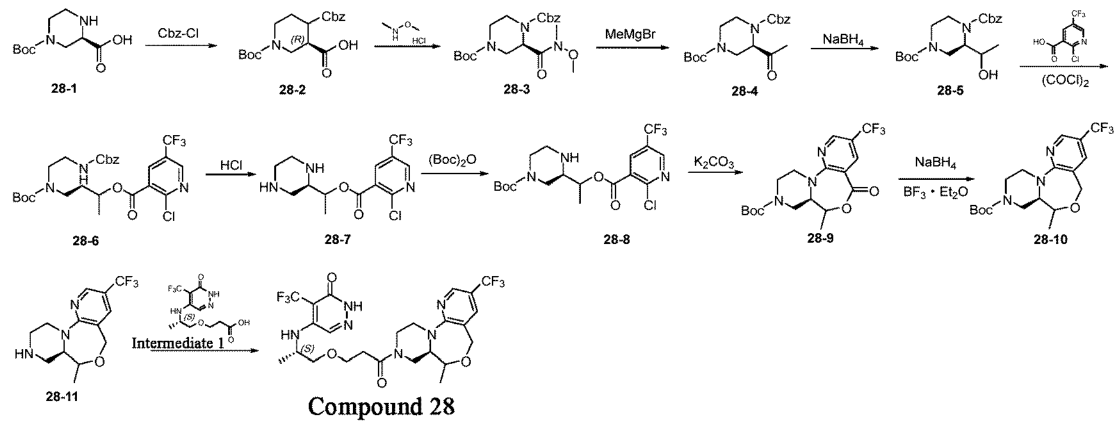


Step I: Synthesis of (R)-1-(benzoxy)carbonyl)-4-(t-butoxycarbonyl)piperazin-2-carboxylic acid **(28-2)**

(R)-4-(t-butoxycarbonyl)piperazin-2-carboxylic acid (5 g, 22 mmol) and sodium bicarbonate (3.7g, 44 mmol) were dissolved in acetone (50 mL) and water (50 mL), and then benzyl chloroformate (4.5 g, 26.4 mmol) was added dropwise to the mixed solution. The mixture was reacted for 3 hrs with stirring at room temperature under nitrogen atmosphere. After the reaction was completed, the reaction solution was added with water (30mL), and then extracted with ethyl acetate (30 mL × 3). The organic phases were combined, and dried over anhydrous sodium sulfate. Anhydrous sodium sulfate was removed by filtration, and the filtrate was rotary dried. The crude product was purified by column chromatography on silica gel (ethyl acetate : petroleum ether=50: 50) to obtain the target product as a yellow oil (4.5 g, 12.4 mmol, yield: 56%), LCMS: (ESI) m/z: 365[M+H]$^+$.

Step II: Synthesis of 1-benzyl4-(t-butyl) (R)-2-(methoxy(methyl)carbamoyl)piperazin-1,4-dicarboxylate (**28-3**)

(R)-1-(benzoxy)carbonyl)-4-(t-butoxycarbonyl)piperazin-2-carboxylic acid (4.5 g, 12.4mmol) were dissolved in dichloromethane (70 mL), and then 1-(3-dimethylaminopropyl)-3-ethylcarbodiimide hydrochloride (3.6 g, 18.6 mmol), N,N-diisopropylethyl amine (4.8 g, 37.2 mmol), 1-hydroxybenzotriazole (2.5 g, 18.6mmol), and dimethylhydroxylamine hydrochloride (2.4 g, 24.8 mmol) were added to the mixed solution. The mixture was reacted for 3 hrs with stirring at room temperature under nitrogen atmosphere. After the reaction was completed, the reaction solution was added with water (70 mL), and then extracted with dichloromethane (30 mL × 3). The organic phases were combined, and dried over anhydrous sodium sulfate. Anhydrous sodium sulfate was removed by filtration, and the filtrate was rotary dried. The crude product was purified by column chromatography on silica gel (ethyl acetate: petroleum ether=50: 50) to obtain the target product as a yellow oil (3g, 7.4mmol, yield: 59%), LCMS: (ESI) m/z: 408[M+H]$^+$.

Step III: Synthesis of 1-benzyl 4-(t-butyl) (R)-2-acetylpiperazin-1,4-dicarboxylate **(28-4)**

Benzyl 4-(t-butyl) (R)-2-(methoxy(methyl)carbamoyl)piperazin-1,4-dicarboxylate (3 g, 7.4 mmol) was dissolved in tetrahydrofuran (30 mL), and then methylmagnesium bromide (7.4mL, 14.8mmol, 2 mol/L solution in diethyl ether) was added dropwise to the mixed solution. The mixture was reacted for 1 hr with stirring at 0°C under nitrogen atmosphere. After the reaction was completed, the reaction solution was added with brine (30mL), and then extracted with ethyl acetate (30 mL × 3). The organic phases were combined, and dried over anhydrous sodium sulfate. Anhydrous sodium sulfate was removed by filtration, and the filtrate was rotary dried. The crude product was purified by column chromatography on silica gel (ethyl acetate : petroleum ether=40: 60) to obtain the target product as a yellow oil (1.8g, 5mmol, yield: 67%), LCMS: (ESI) m/z: 363$[M+H]^+$.

Step IV: Synthesis of 1-benzyl 4-(t-butyl) (R)-2-(S)-1-hydroxyethyl)piperazin-1,4-dicarboxylate **(28-5)**

Benzyl 4-(t-butyl) (R)-2-(S)-1-hydroxyethyl)piperazin-1,4-dicarboxylate (1.8 g, 5 mmol) were dissolved in tetrahydrofuran (20 mL) and ethanol (20 mL), and then solid sodium borohydride (570 mg, 15 mmol) was added to the mixed solution. The mixture was reacted for 3 hr with stirring at 0°C under nitrogen atmosphere. After the reaction was completed, the reaction solution was added with brine (30mL), and then extracted with ethyl acetate (30 mL × 3). The organic phases were combined, and dried over anhydrous sodium sulfate. Anhydrous sodium sulfate was removed by filtration, and the filtrate was rotary dried. The crude product was purified by column chromatography on silica gel (ethyl acetate : petroleum ether=40: 60) to obtain the target product as a yellow oil (1.4g, 3.8mmol, yield: 75%), LCMS:(ESI)m/z: 365$[M+H]^+$.

Step V: Synthesis of 1-benzyl 4-(t-butyl)(R)-2-(R)-1-(2-chloro-5-(trifluoromethyl)pyridin-3-yl)methoxy)ethyl)piperazin-1,4-dicarboxylate **(28-6)**

Chloro-5-(trifluoromethyl)nicotinic acid (1.7g, 7.6mmol) was dissolved in dichloromethane (20 mL), and then oxalyl chloride (1.4 g, 11.4 mmol) was added dropwise to the mixed solution. The mixture was reacted for 1 hr with stirring at room temperature under nitrogen atmosphere. After the reaction was completed, the mixed solution was directly rotary dried, then dissolved in dichloromethane (1 mL), added dropwise to a solution of 1-benzyl 4-(t-butyl)(R)-2-(S)-1-hydroxyethyl)piperazin-1,4-dicarboxylate (1.4 g, 3.8 mmol) and triethyl amine (1.9 g, 19 mmol) in dichloromethane (20 mL), and reacted for 2 hrs at room temperature. After the reaction was completed, the reaction solution was added with brine (30 mL), and then extracted with dichloromethane (30 mL × 3). The organic phases were combined, and dried over anhydrous sodium sulfate. Anhydrous sodium sulfate was removed by filtration, and the filtrate was rotary dried. The crude product was purified by column chromatography on silica gel (ethyl acetate: petroleum ether=50: 50) to obtain the target product as a yellow oil (1.1g, 2mmol, yield: 50%). LCMS:(ESI)m/z: 572$[M+H]^+$.

Step VI: Synthesis of (R)-2-(R)-1-(2-chloro-5-(trifluoromethyl)pyridin-3-yl)methoxy)ethyl)piperazine **(28-7)**

t-butyl 1-benzyl 4-(R)-2-(R)-1-(2-chloro-5-(trifluoromethyl)pyridin-3-yl)methoxy)ethyl)piperazin-1,4-dicarboxylate (1.1 g, 2 mmol) was dissolved in concentrated hydrochloric acid (38%, 10 mL), slowly warmed to 50°C, and reacted for 3 hrs with stirring. After complete reaction, the reaction solution was concentrated under reduced pressure. The crude product was diluted in water (30 mL), adjusted to pH 8-9 with sodium bicarbonate, and extracted three times with dichloromethane (20 mL × 3). The organic phase were combined, and dried over anhydrous sodium sulfate. Anhydrous sodium sulfate was removed by filtration, and the filtrate was rotary dried. The crude product was purified by column chromatography on silica gel (ethyl acetate : petroleum ether=50: 50) to obtain the target product as a yellow solid (500 mg, 1.5 mmol, yield: 77%), LCMS: (ESI) m/z: 338$[M+H]^+$.

Step VII: Synthesis of t-butyl (R)-3-((R)-1-((2-chloro-5-(trifluoromethyl)nicotinoyl)oxy)ethyl)piperazin-1-carboxylate **(28-8)**

(R)-2-(R)-1-(2-chloro-5-(trifluoromethyl)pyridin-3-yl)methoxy)ethyl)piperazine (500 mg, 1.5 mmol) and triethyl amine (300 mg, 3 mmol) were dissolved in dichloromethane (10 mL), and then di-t-butyl dicarbonate (492 mg, 1.5 mmol) were added dropwise to the mixed solution. The mixture was reacted for 3 hr with stirring at room temperature under nitrogen atmosphere. After the reaction was completed, the reaction solution was added with brine (30mL), and then extracted with ethyl acetate (30 mL × 3). The organic phases were combined, and dried over anhydrous sodium sulfate. Anhydrous sodium sulfate was removed by filtration, and the filtrate was rotary dried. The crude product was purified by column chromatography on silica gel (ethyl acetate : petroleum ether=50: 50) to obtain the target product as a yellow oil (430 mg, 0.98 mmol, yield: 65%), LCMS: (ESI) m/z: 438$[M+H]^+$.

Step VIII: Synthesis of t-butyl (7R,7AR)-7-methyl-5-oxo-3-(trifluoromethyl)-7a,8,10,11-tetrahydro-*5H*-pyrazino[2,1-c]pyrido[2,3-e][1,4]Oxazepane-9(*7H*)-carboxylate **(28-9)**

t-butyl (R)-3-((R)-1-((2-chloro-5-(trifluoromethyl)nicotinoyl)oxy)ethyl)piperazin-1-carboxylate (430mg, 0.98mmol) was dissolved in DMF (5 mL), and then potassium carbonate(270 mg, 1.96 mmol) was added. The mixture was heated to 100°C under nitrogen atmosphere, and reacted for 12 hrs with stirring. The reaction solution was cooled, added with ice water (30 mL), and extracted with ethyl acetate (30 mL × 3). The organic phase was dried over anhydrous sodium sulfate. Anhydrous sodium sulfate was removed by filtration, and rotary dried. The crude product was purified by column chromatography on silica gel (ethyl acetate: petroleum ether=70: 30) to obtain the target product as a yellow oil (200mg, 0.5mmol, yield: 51%), LCMS: (ESI)m/z: 402[M+H]$^+$.

Step IX: Synthesis of t-butyl (7R,7AR)-7-methyl-3-(trifluoromethyl)-7a,8,10,11-tetrahydro-*5H*-pyrazino[2,1-c]pyrido[2,3-e][1,4]Oxazepane-9(*7H*)-carboxylate **(28-10)**

t-butyl (7R,7AR)-7-methyl-5-oxo-3-(trifluoromethyl)-7a,8,10,11-tetrahydro-*5H*-pyrazino[2,1-c]pyrido[2,3-e][1,4]Oxazepane-9(*7H*)-carboxylate (200 mg, 0.5 mmol) was dissolved in tetrahydrofuran (10 mL), and then cooled to 0°C. Solid sodium borohydride (38 mg, 1 mmol) and boron trifluoride-diethyl etherate (0.4 mL) were added, and stirred for 1 hr. After the reaction was completed, the reaction solution was poured into ice water (10 mL), and the aqueous phase was then extracted with ethyl acetate (15 mL × 3). The organic phases were combined, and dried over anhydrous sodium sulfate. Anhydrous sodium sulfate was removed by filtration, and rotary dried. The crude product was purified by column chromatography on silica gel (ethyl acetate: petroleum ether=50: 50) to obtain the target product as a yellow oil (40mg, 0.1mmol, yield: 20%), LCMS: (ESI)m/z: 388[M+H]$^+$.

Step X: Synthesis of (7R,7aR)-7-methyl-3-(trifluoromethyl)-7,7a,8,9,10,11-hexahydro-*5H*-pyrazino[2,1-c]pyrido[2,3-e][1,4]Oxazepane-5-one **(28-11)**

T-butyl (7R,7AR)-7-methyl-3-(trifluoromethyl)-7a,8,10,11-tetrahydro-*5H*-pyrazino[2,1-c]pyrido[2,3-e][1,4]Oxazepane-9(*7H*)-carboxylate (40 mg, 0.1 mmol) was dissolved in ice-cold (0°C) hydrogen chloride-1,4-dioxane solution (4 M, 1 mL), slowly warmed to room temperature and reacted for 4 hrs with stirring. After complete reaction, the reaction solution was concentrated under reduced pressure. The crude product was diluted in water (30 mL), adjusted to pH 8-9 with sodium bicarbonate, and extracted with dichloromethane (20 mL × 3). The organic phases were combined, and dried over anhydrous sodium sulfate. Anhydrous sodium sulfate was removed by filtration, and rotary dried. The crude product was purified by column chromatography on silica gel (ethyl acetate: petroleum ether=40: 60) to obtain the target product as a yellow solid (20 mg, 0.069 mmol, yield: 66%), LCMS: (ESI) m/z: 288[M+H]$^+$.

Step XI: Synthesis of 5-(((S)-1-(3-((7R,7AR)-7-methyl-3-(trifluoromethyl)-7a,8,10,11-tetrahydro-*5H*-pyrazino[2,1-c]pyrido[2,3-e][1,4]oxazepan-9(*7H*)-yl)-3-oxypropoxy)prop-2-yl)amino)-4-(trifluoromethyl)pyridazin-3(*2H*)-one (**Compound 28**)

(7R,7aR)-7-methyl-3-(trifluoromethyl)-7,7a,8,9,10,11-hexahydro-*5H*-pyrazino[2,1-c]pyrido[2,3-e][1,4]oxazepane (20 mg, 0.069 mmol) and (S)-3-(2-((6-oxo-5-(trifluoromethyl)-1,6-dihydropyridazin-4-yl)amino)propoxy)propionic acid (21 mg, 0.069 mmol) were dissolved in dichloromethane (1.0 mL), and then diisopropylethyl amine (27 mg, 0.207 mmol) and a propylphosphonic anhydride solution (66 mg, 0.104 mmol, 50% solution in ethyl acetate) were added. The reaction mixture was stirred at room temperature for 2 hrs. The reaction solution was poured into ice water (20mL), and the aqueous phase was then extracted with ethyl acetate (10 mL × 3). The organic phases were combined and dried over anhydrous sodium sulfate. Anhydrous sodium sulfate was removed by filtration, and the filtrate was rotary dried to obtain a crude product. The crude product was purified by preparative liquid-phase chromatography (column: XBridge Prep OBD C18, 30 × 150 mm 5 um, mobile phase A: water (containing 10 mol/L ammonium bicarbonate), mobile phase B: acetonitrile, flow rate: 60 mL/min, elution gradient: mobile phase B from 10% to 50% over 8 min, detection wavelength: 220 nm, retention time: 7.53 min) to obtain the target product as a white solid (1.28 mg, 0.002 mmol, yield 1.25%).

$^1$HNMR(400 MHz, DMSO-$d_6$) δ(ppm): 12.45(brs, 1H), 8.50(s, 1H), 8.18-8.10(m, 1H), 7.92-7.84(m, 1H), 6.31-6.21(m, 1H), 4.11-4.04(m, 3H), 4.01-3.88(m, 2H), 3.74-3.59(m, 3H), 3.49-3.40(m, 4H), 3.14-2.92(m, 1H), 2.84-2.60(m, 4H), 1.42(d, *J* = 6.4 Hz, 3H), 1.16-1.12(m, 3H). LCMS: MS(ESI)m/z: 579.35 [M+H]$^+$.

**Example 29:** Synthesis of 5-(((S)-1-(3-oxo-3-((S)-3-(trifluoromethyl)-6,7,7a,8,10,11-hexahydro-9H-pyrazino[1,2-d]pyrido[3,2-b][1,4]oxazepan-9-yl)propoxy)prop-2-yl)amino)-4-(trifluoromethyl)pyridazin-3(*2H*)-one (**Compound 29**)

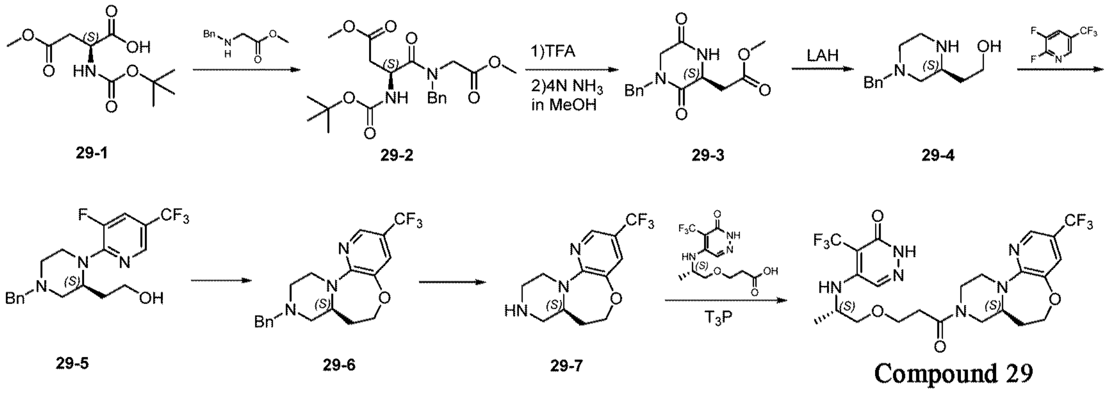


29-1 29-2 29-3 29-4

29-5 29-6 29-7 Compound 29

Step I: Synthesis of methyl (S)-4-(benzyl(2-methoxy-2-oxyethyl)amino)-3-(t-butoxycarbonyl)amino)-4-oxobutyrate **(29-2)**

(S)-2-(t-butoxycarbonyl)amino)-4-methoxy-4-oxobutyric acid (10 g, 0.04 mol) and methyl benzyl glycinate (10.8 g, 0.06 mol) were dissolved in dichloromethane (200 mL) in a flask, and 1-(3-dimethylaminopropyl)-3-ethylcarbodiimide hydrochloride (11.5 g, 0.06 mol), 1-hydroxybenzotriazole (8.1 g, 0.06 mol) and N,N-diisopropylethyl amine (15.5 g, 012 mol) were then added, and reacted for 2 hrs at room temperature. After the reaction was completed, the reaction was quenched with water (200 mL). The aqueous phase was separated , and the aqueous phase was extracted with dichloromethane (100 ML × 2). The organic phases were combined, washed with saturated brine, dried over anhydrous sodium sulfate, and filtered. The filtrate was concentrated. The resulting crude product was purified by column chromatography on silica gel (dichloromethane: methanol=10: 90) to obtain the target product as a yellow oil (8 g, 0.02 mol, yield: 72%). LCMS: MS(ESI)m/z: 409[M+H]$^+$.

Step II: Synthesis of methyl (S)-2-(4-benzyl-3,6-dioxopiperazin-2-yl)acetate **(29-3)**

Methyl (S)-4-(benzyl(2-methoxy-2-oxyethyl)amino)-3-(t-butoxycarbonyl)amino)-4-oxobutyrate (8 g, 0.02 mol) was dissolved in trifluoroacetic acid, and the mixture was stirred for 1 hr at room temperature. The mixture was concentrated under reduced pressure, and then aminomethanol (50 mL) was added, stirred at room temperature for 3 hrs, and then concentrated to obtain the target product as a yellow oil (4.1 g, 0.014 mol, yield: 80%). LCMS: MS(ESI)m/z: 277[M+H]$^+$.

Step III: Synthesis of (S)-2-(4-benzylpiperazin-2-yl)ethyl-1-ol **(29-4)**

Methyl (S)-2-(4-benzyl-3,6-dioxopiperazin-2-yl)acetate (4.1 g, 0.014 mol) was added to a 50 mL round-bottom flask, and dissolved in tetrahydrofuran (100mL). Lithium aluminum hydride (14 mL, 0.028 mol, 2 mol/L solution in tetrahydrofuran) was added at 0°C, stirred overnight at room temperature, added with sodium sulfate decahydrate, and stirred for 30 min. After filtration, the filtrate was rotary dried to obtain a crude product as a yellow solid, which was purified by high performance liquid chromatography (column: XBridge Prep OBD C18, 30 × 150 mm 5 um, mobile phase A: water (containing 10 moL/L ammonium bicarbonate), mobile phase B: acetonitrile, flow rate: 60 mL/min, elution gradient: mobile phase B from 5% to 30% over 8 min, detection wavelength: 220 nm, retention time: 7.37 min) to obtain the target product as a yellow solid (1.2 g, 5.4 mmol, yield: 36%). LCMS: MS(ESI)m/z: 221[M+H]$^+$.

Step IV: Synthesis of (S)-2-(4-benzyl-1-(3-fluoro-5-(trifluoromethyl)pyridin-2-yl)piperazin-2-yl)ethyl-1-ol **(29-5)**

(S)-2-(4-benzylpiperazin-2-yl)ethyl-1-ol (600 mg, 2.5 mmol) was dissolved in DMF (12 mL) in a reaction flask, and then 2,3-difluoro-5-(trifluoromethyl)pyridine (915 mg, 5 mmol) and potassium carbonate(690 mg, 5 mmol) were added. The mixture was stirred overnight at 80°C, cooled and then extracted with ethyl acetate (50 mL × 2) and water (25mL). The organic phase was washed with saturated brine, dried over anhydrous sodium sulfate, and filtered. The filtrate was concentrated under reduced pressure. The resulting crude product was purified by column chromatography on silica gel (ethyl acetate : petroleum ether=3: 1) to obtain the target product as a yellow oil (300 mg, 0.78 mmol, yield:

34%). LCMS: MS(ESI)m/z: 384[M+H]+.

Step V: Synthesis of (S)-9-benzyl-3-(trifluoromethyl)-7,7a,8,9,10,11-hexahydro-6H-pyrazino[1,2-d]pyrido[3,2-b][1,4]oxazepane **(29-6)**

(S)-2-(4-benzyl-1-(3-fluoro-5-(trifluoromethyl)pyridin-2-yl)piperazin-2-yl)ethyl-1-ol (300 mg, 0.78 mmol) was dissolved in DMF (6 mL), and then potassium tert-butoxide (175 mg, 1.56 mmol) was added. The mixture was stirred for 3 hrs at 100°C, cooled and then extracted with ethyl acetate (20 mL × 2) and water (10 mL). The organic layers were combined, washed with saturated brine, dried over anhydrous sodium sulfate, and filtered. The filtrate was rotary dried and then purified by column chromatography on silica gel (petroleum ether: ethyl acetate =1: 1) to obtain the target product as a yellow oil (260 mg, 0.71 mmol, yield: 91%). LCMS: MS(ESI)m/z: 364[M+H]+.

Step VI: Synthesis of (S)-3-(trifluoromethyl)-7,7a,8,9,10,11-hexahydro-6H-pyrazino[1,2-d]pyrido[3,2-b][1,4]oxazepane **(29-7)**

(S)-9-benzyl-3-(trifluoromethyl)-7,7a,8,9,10,11-hexahydro-6H-pyrazino[1,2-d]pyrido[3,2-b][1,4]oxazepane (260 mg, 0.72 mmol) was dissolved in acetic acid (20 mL), and added with anhydrous palladium on carbon (50 mg). The mixture was stirred for 2 hrs at room temperature under hydrogen atmosphere. After the reaction was completed, the reaction solution was filtered, and the filtrate was rotary dried to obtain a crude product, which was purified by high performance liquid chromatography (column: XBridge Prep OBD C18, 30 × 150 mm 5 um, mobile phase A: water (containing 10 moL/L ammonium bicarbonate), mobile phase B: acetonitrile, flow rate: 60 mL/min, elution gradient: mobile phase B from 5% to 40% over 8 min, detection wavelength: 220 nm, retention time: 8.27 min) to obtain the target product as a colorless oil (110 mg, 0.4 mmol, yield: 56%) LCMS: MS(ESI)m/z: 274[M+H]+.

Step VII: Synthesis of 5-(((S)-1-(3-oxo-3-((S)-3-(trifluoromethyl)-6,7,7a,8,10,11-hexahydro-9H-pyrazino[1,2-d]pyrido[3,2-b] [1,4]oxazepan-9-yl)propoxy)prop-2-yl)amino)-4-(trifluoromethyl)pyridazin-3(2H)-one **(Compound 29)**

(S)-3-(trifluoromethyl)-7,7a,8,9,10,11-hexahydro-6H-pyrazino[1,2-d]pyrido[3,2-b][1,4]oxazepane (55 mg, 0.2 mmol) was dissolved in dichloromethane (2 mL), and then a solution of 50% propylphosphonic anhydride (190.8 mg, 0.3 mmol) in ethyl acetate, N,N-diisopropylethyl amine (77.4 mg, 0.6 mmol) and (S)-3-(2-((6-oxo-5-(trifluoromethyl)-1,6-dihydropyridazin-4-yl)amino)propoxy)propionic acid (62 mg, 0.2 mmol) were added. The mixture was stirred at room temperature for 1 hr, and then extracted with dichloromethane (10 mL × 2) and water (5 mL). The organic phases were combined, washed with saturated brine, dried over anhydrous sodium sulfate, and filtered. The filtrate was rotary dried and purified by high performance liquid chromatography (column: XBridge Prep OBD C18, 30 × 150 mm 5 um, mobile phase A: water (containing 10 moL/L ammonium bicarbonate), mobile phase B: acetonitrile, flow rate: 60 mL/min, elution gradient: mobile phase B from 20% to 58% over 8 min, detection wavelength: 220 nm, retention time: 7.57 min) to obtain the target product as a white solid (26.82 mg, 0.047 mmol, yield: 23%).

$^1$H NMR(400MHz, DMSO-$d_6$) δ(ppm): 12.45(brs,1H), 8.23-8.08(m, 1H), 8.02-7.83(m, 1H), 7.39-7.32(m, 1H), 6.32-6.22(m, 1H), 4.36-4.20(m, 2H), 4.19-4.07(m, 1H), 4.07-3.95(m, 1H), 3.90-3.76(m, 2H), 3.75-3.62(m,4H), 3.62-3.37(m, 4H), 2.59-2.53(m, 2H), 2.18-2.01(m, 1H),1.97-1.83(m, 1H), 1.20-1.03(m, 3H). LCMS: MS(ESI)m/z: 565.20[M+H]+.

**Example 30:** Synthesis of (s)-5-cyclopropyl-8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-*5H*-pyrazino[1,2-a]pyrido[3,2-e]pyrazin-6(*6aH*)-one **(Compound 30)**

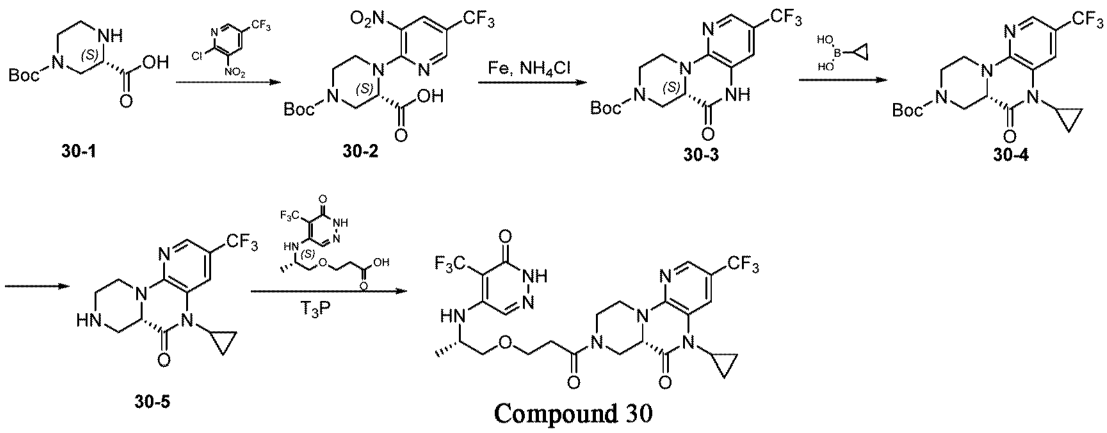


30-1 30-2 30-3 30-4

30-5

Compound 30

Step I: Synthesis of (S)-4-(t-butoxycarbonyl)-1-(3-nitro-5-(trifluoromethyl)pyridin-2-yl)piperazin-2-carboxylic acid **(30-2)**

(S)-4-(t-butoxycarbonyl)piperazin-2-carboxylic acid (2.00 g, 8.69 mmol) was added to methanol (20 mL), and then 2-chloro-3-nitro-5-(trifluoromethyl)pyridine (1.96 g, 8.69 mmol) and TEA (1.75 g, 17.38 mmol) were added. The mixture was stirred at room temperature for 16 hrs. The resulting mixture was concentrated under reduced pressure to obtain a crude target product (S)-4-(t-butoxycarbonyl)-1-(3-nitro-5-(trifluoromethyl)pyridin-2-yl)piperazin-2-carboxylic acid as a brown solid (4.00 g). The crude product was used directly in the next reaction without further purification. LCMS: MS(ESI)m/z: $421[M+H]^+$.

Step II: Synthesis of t-butyl (S)-6-oxo-3-(trifluoromethyl)-5,6,6a,7,9,10-hexahydro-8H-pyrazino[1,2]pyrido[3,2]pyrazin-8-carboxylate **(30-3)**

(S)-4-(t-butoxycarbonyl)-1-(3-nitro-5-(trifluoromethyl)pyridin-2-yl)piperazin-2-carboxylate (2.00 g, 4.76 mmol) was added to ethanol (40 mL), and then iron powder (2.67 g, 47.60 mmol) and ammonium chloride (2.52 g, 47.60 mmol) were added. The mixture was stirred at 75°C for 2 hrs. The mixture was filtered, and the filtrate was extracted with water (100 mL) and ethyl acetate (50 mL × 2). The organic phases were combined, washed with saturated sodium chloride solution, dried over anhydrous sodium sulfate, filtered and concentrated to obtain a crude product. The crude product was purified by column chromatography on silica gel (petroleum ether: ethyl acetate =30: 70) to obtain the target product t-butyl (S)-6-oxo-3-(trifluoromethyl)-5,6,6a,7,9,10-hexahydro-8H-pyrazino[1,2]pyrido[3,2]pyrazin-8-carboxylate (0.40 g, 1.08 mmol, yield: 22.6%). LCMS: MS(ESI)m/z: $373[M+H]^+$.

Step III: Synthesis of t-butyl (S)-5-cyclopropyl-6-oxo-3-(trifluoromethyl)-5,6,6a,7,9,10-hexahydro-*8H*-pyrazino[1,2-a]pyrido[3,2-e]pyrazin-8-carboxylate **(30-4)**

T-butyl (S)-6-oxo-3-(trifluoromethyl)-5,6,6a,7,9,10-hexahydro-*8H*-pyrazino[1,2-a]pyrido[3,2-e]pyrazin-8-carboxylate (300 mg, 0.80 mmol) was added to dimethylformamide (20 mL), and then cyclopropylboric acid (138mg, 1.60mmol) and $Cu(OAc)_2$(9.9mg, 0.05mmol) were added. The mixture was reacted for 120 hrs with stirring at 60°C. The mixture was added with water (40 mL), and extracted with ethyl acetate (20 mL × 3). The organic layer was collected and dried over anhydrous sodium sulfate. Sodium sulfate was filtered off and the filtrate was concentrated to obtain a crude product. The crude product was purified by column chromatography on silica gel (petroleum ether: ethyl acetate =30: 70) to obtain the target product as a white solid (149mg, 0.36mmol, yield: 45%). LCMS: MS(ESI)m/z: $413[M+H]^+$.

Step IV: Synthesis of (S)-5-cyclopropyl-3-(trifluoromethyl)-7,8,9,10-tetrahydro-*5H*-pyrazino[1,2-a]pyrido[3,2-e]pyrazin-6(*6aH*)-one **(30-5)**

T-butyl (S)-5-cyclopropyl-6-oxo-3-(trifluoromethyl)-5,6,6a,7,9,10-hexahydro-*8H*-pyrazino[1,2-a]pyrido[3,2-e]pyrazin-8-carboxylate (150 mg, 0.36 mmol) was added to a mixed solution of trifluoroacetic acid (2 mL) and dichloromethane (5 mL). The mixture was reacted for 2 hrs with stirring at room temperature. The reaction solution was concentrated under reduced pressure. The residue was added with water (20 mL), and adjusted to pH 8-9 with a saturated sodium bicarbonate aqueous solution. The aqueous phase was then extracted with ethyl acetate (10 mL × 3). The organic layer was dried over anhydrous $Na_2SO_4$, and filtered to remove $Na_2SO_4$. The filtrate was concentrated to obtain a crude product. The crude product was purified by column chromatography on silica gel (petroleum ether: ethyl acetate =30: 70) to obtain the target product as a burgundy solid (100 mg, 0.32 mmol, yield: 88%). LCMS: MS(ESI)m/z: $313[M+H]^+$.

Step V: Synthesis of (S)-5-cyclopropyl-8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-*5H*-pyrazino[1,2-a]pyrido[3,2-e]pyrazin-6(*6aH*)-one **(Compound 30)**

(S)-5-cyclopropyl-3-(trifluoromethyl)-7,8,9,10-tetrahydro-*5H*-pyrazino[1,2-a]pyrido[3,2-e]pyrazin-6(*6aH*)-one (35 mg, 0.11 mmol) was dissolved in dichloromethane (2 mL) in a sample flask. Then, (S)-3-(2-((6-oxo-5-(trifluoromethyl)-1,6-dihydropyridazin-4-yl)amino)propoxy)propionic acid (35 mg, 0.11 mmol), propylphosphonic anhydride (214.90 mg, 0.33 mmol), and N,N-diisopropylethyl amine (72.10 mg, 0.56 mmol) were added. The mixture was stirred at room temperature for 1 hr, then added with water (5 mL), and extracted with dichloromethane (5 mL × 3). The organic layer was collected, dried over sodium sulfate and concentrated to obtain the crude product. The crude product was purified by high performance liquid chromatography (chromatographic column: YMC-Actus Triart C18, 30 × 150 mm, 5 μm; mobile phase A: water (containing 10 mmol/L ammonium bicarbonate), mobile phase B: acetonitrile; flow rate: 60mL/min; gradient: 25% B to 60% B in 8 min; wavelength: 220 nm; retention time: 7.85 min) to obtain the target product as a white solid (7.75 mg, 0.0132 mmol, yield: 12%).

$^1H$ NMR(400 MHz, DMSO-$d_6$) δ(ppm): 12.53(s, 1H), 8.22(s, 1H), 7.99-7.82(m, 1H), 7.66(s, 1H), 6.40-6.18(m, 1H), 4.90-4.77(m, 1H), 4.57-4.22(m, 2H), 4.19-3.99(m, 2H), 3.73-3.64(m, 2H), 3.52-3.48(m, 2H), 3.21-3.08(m, 1H), 2.85-2.74(m, 2H), 2.69-2.62(m, 3H), 1.29-1.14(m, 4H), 1.14-1.05(m, 1H) , 0.86-0.72(m, 1H) ,0.55-0.40(m, 1H). LCMS: MS(ESI)m/z: 604.05 $[M+H]^+$.

**Example 31** Synthesis of 5-(((S)-1-(3-(R)-2-methyl-3-(trifluoromethyl)-7a,8,10,11-tetrahydro-*5H*-pyrazino[2,1-c]pyrido[2,3-e][1,4]oxazepan-9(*7H*)-yl)-3-oxypropoxy)prop-2-yl)amino)-4-(trifluoromethyl)pyridazin-3(2*H*)-one **(Compound 31)**

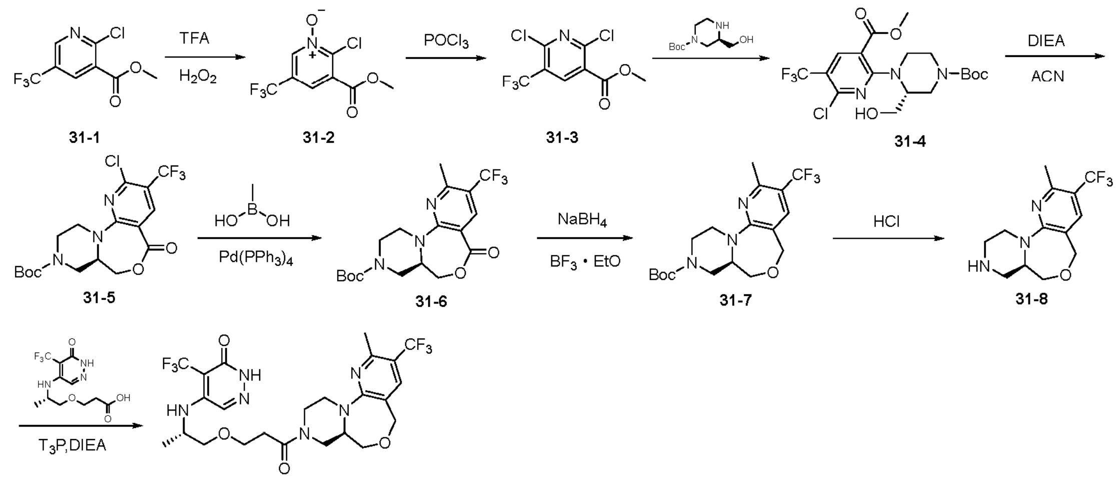


Compound 31

Step I: Synthesis of 2-chloro-3-(methoxycarbonyl)-5-(trifluoromethyl)pyridin-1-oxide **(31-2)**

To a solution of methyl 2-chloro-5-(trifluoromethyl)nicotinate (5 g, 20 mmol) in TFA (10 mL), a hydrogen peroxide solution (5 mL) was added. The mixture was stirred for 1 hrs at 70°C, then poured into water (5 mL), and then extracted with ethyl acetate (20 mL × 3). The organic phase was dried over anhydrous sodium sulfate, and filtered to remove sodium sulfate. The filtrate was concentrated under reduced pressure, and the remaining crude product was purified by column chromatography on silica gel (petroleum ether: ethyl acetate =80: 20) to obtain the target product as a white solid (2.5 g, 1.2 mmol, yield: 60%), LCMS: (ESI) m/z: 256 $[M+H]^+$.

Step II: Synthesis of methyl 2, 6-dichloro-5-(trifluoromethyl)nicotinate **(31-3)**

A mixture of 2-chloro-3-(methoxycarbonyl)-5-(trifluoromethyl)pyridine 1-oxide (2.5 g, 20 mmol) in phosphorus oxychloride (10 mL) was stirred for 2 hrs at 100°C. The reaction solution was concentrated under reduced pressure to remove excessive phosphorus oxychloride. The residue was dissolved in dry ethyl acetate, mixed well with silica gel (6 g), and concentrated under reduced pressure. The sample was dry loaded and purified by column chromatography on

silica gel (petroleum ether: ethyl acetate =70: 30) to obtain the target product as a yellow oil (1.5 g, 12 mmol, yield: 60%), LCMS: (ESI)m/z: 274 [M+H]$^+$.

Step III: Synthesis of t-butyl (R)-4-(6-chloro-3-(methoxycarbonyl)-5-(trifluoromethyl)pyridin-2-yl)-3-(hydroxymethyl)piperazin-1 -carboxylate **(31-4)**

To a mixture of methyl 2, 6-dichloro-5-(trifluoromethyl)nicotinate (1 g, 3.64 mol) and t-butyl (R)-3-(hydroxymethyl)piperazin-1-carboxylate (0.78 g, 3.6 mmol) in acetonitrile (10 mL), N,N-diisopropylethyl amine (0.5 g, 7.2 mmol) was added, and the mixture was stirred at room temperature for 12 hrs. The reaction solution was poured into water (20 mL), and extracted with ethyl acetate (10 mL × 2). The organic phases were combined, and dried over anhydrous sodium sulfate. Sodium sulfate was filtered off, and the filtrate was concentrated under reduced pressure, and then purified by column chromatography on silica gel (petroleum ether: ethyl acetate =30: 70) to obtain the target product as a white solid (1g, 3.64 mmol, yield: 70%). LCMS: (ESI)m/z: 454[M+H]$^+$.

Step IV: Synthesis of t-butyl (R)-2-chloro-5-oxo-3-(trifluoromethyl)-7a,8,10,11-tetrahydro-*5H*-pyrazino[2,1-c]pyrido[2,3-e][1, 4]Oxazepane-9(*7H*)-carboxylate **(31-5)**

A mixture of t-butyl (R)-4-(6-chloro-3-(methoxycarbonyl)-5-(trifluoromethyl)pyridin-2-yl)-3-(hydroxymethyl)piperazin-1-carboxylate (1 g, 2.2 mmol) and N,N-diisopropylethyl amine (0.5 g, 4.4 mmol) in acetonitrile (10 mL) was stirred for 12 hrs at 80°C, then poured into water (10 mL), and extracted with ethyl acetate (10 mL × 2). The organic phase was dried over anhydrous sodium sulfate, filtered under suction, and rotary dried. The residue was purified by column chromatography (petroleum ether: ethyl acetate =74: 26) to obtain the target product as a white solid (0.75g, 1.8mmol, yield: 80%). LCMS:(ESI)m/z: 422[M+H]$^+$.

Step V: Synthesis of t-butyl (R)-2-methyl-5-oxo-3-(trifluoromethyl)-7a,8,10,11-tetrahydro-*5H*-pyrazino[2,1-c]pyrido[2,3-e][1,4]Oxazepane-9(*7H*)-carboxylate **(31-6)**

T-butyl (R)-2-chloro-5-oxo-3-(trifluoromethyl)-7a,8,10,11-tetrahydro-*5H*-pyrazino[2,1-c]pyrido[2,3-e][1,4]Oxazepane-9(*7H*)-carboxylate (0.1 g, 0.23 mmol) and methylboric acid (0.02 g, 0.35 mmol) were added to dioxane (1 mL), and then tetrakis (triphenylphosphine)palladium (0.054 g, 0.023 mmol) and cesium carbonate (0.154 g, 0.46 mmol) were added. The mixture was stirred for 2 hrs at 100°C, and filtered under suction. The filtrate was poured into water (2 mL), and then extracted with ethyl acetate (10 mL × 2). The organic phase was dried over anhydrous sodium sulfate, and filtered to remove sodium sulfate. The filtrate was concentrated, and the remaining crude product was purified by column chromatography on silica gel (petroleum ether: ethyl acetate =50: 50) to obtain the target product (0.08 g, 0.23 mmol, yield: 100%). LCMS:(ESI)m/z: 557[M+H]$^+$.

Step VI: Synthesis of t-butyl (R)-2-methyl-3-(trifluoromethyl)-7a,8,10,11-tetrahydro-*5H*-pyrazino[2,1-c]pyrido[2,3-e][1,4]oxazepan-9(*7H*)-carboxylate **(31-7)**

To a solution of t-butyl (R)-2-methyl-5-oxo-3-(trifluoromethyl)-7a,8,10,11-tetrahydro-*5H*-pyrazino[2,1-c]pyrido[2,3-e][1,4]Oxazepane-9(*7H*)-carboxylate (0.08 g, 0.2 mol) in tetrahydrofuran (5 mL), boron trifluoride-diethyl etherate (2 mL) and sodium borohydride (0.036 g, 1 mmol) were added. The mixture was stirred at room temperature for 2 hrs, poured into water (20 mL), and then extracted with ethyl acetate (20 mL × 2). The organic phase was dried over anhydrous sodium sulfate, filtered under suction, and rotary dried, to obtain a crude product. The crude product was purified by column chromatography on silica gel to obtain the target product (0.077 g, 0.2 mmol, yield: 40%). LCMS: (ESI)m/z: 287[M+H]$^+$.

Step VII: Synthesis of (R)-2-methyl-3-(trifluoromethyl)-7,7a,8,9,10,11-hexahydro-*5H*-pyrazino[2,1-c]pyrido[2,3-e][1,4]Oxazepane **(31-8)**

T-butyl (R)-2-methyl-3-(trifluoromethyl)-7a,8,10,11-tetrahydro-*5H*-pyrazino[2,1-c]pyrido[2,3-e][1,4]Oxazepane-9(*7H*)-carboxylate (0.8 g, 2.06 mmol) was added to hydrochloric acid/dioxane (2 mL). The mixture was stirred at room temperature for 2 hrs, and then concentrated to obtain the target product as a white solid (0.5 g, 2.06 mmol, yield: 70%), LCMS: (ESI)m/z: 387[M+H]$^+$.

Step VIII: Synthesis of 5-(((S)-1-(3-oxy-3-((S)-3-(trifluoromethyl)-6,7,7a,8,10,11-hexahydropyrazino[1,2-a]pyrido[3,2-f][1,4]diazepine -9(*5H*)-yl)propoxy)propanol-2-yl)amino)-4-(trifluoromethyl)piperazin-3(*2H*)-one **(Compound 31)**

A solution of (R)-2-methyl-3-(trifluoromethyl)-7,7a,8,9,10,11-hexahydro-*5H*-pyrazino[2,1-c]pyrido[2,3-e][1,4]oxazepane (50 mg, 0.13 mmol) and (S)-3-(2-((6-oxo-5-(trifluoromethyl)-1,6-dihydropyridazin-4-yl)amino)propoxy)propionic acid (53 mg, 0.13 mmol) in DCM (2 mL) was added with propylphosphonic anhydride (158.64 mg, 0.65 mmol) and NN-diisopropylethyl amine (67.3 mg, 0.52 mmol). The mixture was stirred at room temperature for 2 hrs. The mixture was poured into water (5 mL), and extracted with dichloromethane (5 mL × 2). The organic phase was dried over anhydrous sodium sulfate, filtered under suction, concentrated, and separated by preparative high performance liquid chromatography (column: XBridge Prep OBD C18, 30 × 150 mm 5 um, mobile phase A: water (containing 10 mol/L ammonium bicarbonate), mobile phase B: acetonitrile, flow rate: 60 mL/min, elution gradient: mobile phase B from 20% to 52% over 8 min, detection wavelength: 220 nm, retention time: 7.67min) to obtain the target product as a white solid (2.32 mg, 0.091 mmol, yield: 30%).

$^1$H NMR(400 MHz, DMSO-$d_6$) δ(ppm): 12.45(s, 1H), 7.95-7.90(m, 1H), 7.70-7.68(m, 1H), 6.28(s, 1H), 4.90-4.80(m, 1H), 4.52-4.42(m, 1H), 4.18-4.11(m, 1H), 4.08-3.93(m, 2H), 3.93-3.80(m, 3H), 3.80-3.52(m, *8H*), 3.49-3.45(m, 2H), 2.46-2.45(m, 3H), 1.18-1.12(m, 3H). LCMS: MS(ESI)m/z: 579.25[M+H]$^+$.

**Example 32:** Synthesis of 5-(((2S)-1-(3-oxo-3-(3-(trifluoromethyl)-5,6,6a,7,9,10-hexahydro-*8H*-pyrazino[1,2-a][1,8]naphthyridin-8-yl)propoxy)prop-2-yl)amino)-4-(trifluoromethyl)pyridazin-3(*2H*)-one **(Compound 32)**

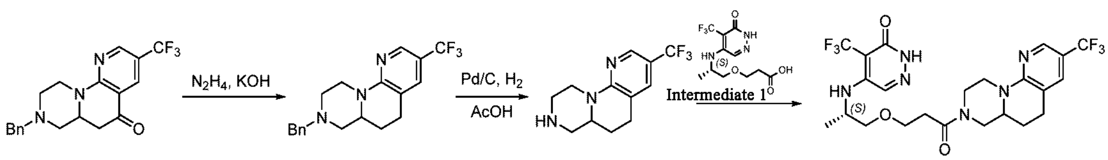


Intermediate 5 32-1 32-2 Compound 32

Step I: Synthesis of 8-benzyl-3-(trifluoromethyl)-6,6a,7,8,9,10-hexahydro-*5H*-pyrazino[1,2-a][1,8]naphthyridine **(32-1)**

8-benzyl-3-(trifluoromethyl)-6,6a,7,8,9,10-hexahydro-*5H*-pyrazino[1,2-a][1,8]naphthyridin-5-one **Intermediate 5** (0.10 g, 0.28 mmol), hydrazine hydrate (0.02 g, 0.55 mmol), and potassium hydroxide (0.03 g, 0.55 mmol) were added to diethylene glycol (5 mL). The mixture was stirred at 160°C for 2 hrs, then poured into water (2 mL), and then extracted with ethyl acetate (3 mL × 2). The organic phase was dried over anhydrous sodium sulfate, and filtered to remove sodium sulfate. The filtrate was concentrated under reduced pressure to obtain the target product as a yellow oil (0.05 g, 0.14 mmol, yield: 52%). LCMS: MS(ESI)m/z: 358[M+H]$^+$.

Step II: Synthesis of 3-(trifluoromethyl)-6,6a,7,8,9,10-hexahydro-*5H*-pyrazino[1,2-a][1,8]naphthyridine **(32-2)**

A mixture of 8-benzyl-3-(trifluoromethyl)-6,6a,7,8,9,10-hexahydro-*5H*-pyrazino[1,2-a][1,8]naphthyridine (0.12 g, 0.08 mmol) and palladium/carbon (0.01 g, 0.02 mmol) in acetic acid (2 mL) was stirred for 2 hrs under hydrogen atmosphere at room temperature, filtered and concentrated. The residue was purified by column chromatography (acetonitrile: water =30: 70) to obtain the target product as a yellow oil (0.05 g, 0.05 mmol, yield: 68%). LCMS: MS(ESI)m/z: 258[M+H]$^+$.

Step III: Synthesis of 5-(((2S)-1-(3-oxo-3-(3-(trifluoromethyl)-5,6,6a,7,9,10-hexahydro-*8H*-pyrazino[1,2-a][1,8]naphthyridin-8-yl)propoxy)prop-2-yl)amino)-4-(trifluoromethyl)pyridazin-3(*2H*)-one **(Compound 32)**

3-(trifluoromethyl)-6,6a,7,8,9,10-hexahydro-*5H*-pyrazino[1,2-a][1,8]naphthyridine (50 mg, 0.19 mmol) and (S)-3-(2-(6-oxo-5-(trifluoromethyl)-1,6-dihydropyridazin-4-yl)amino)propoxy)propionic acid **Intermediate1** (60.10 mg, 0.19 mmol) were added to dichloromethane (2 mL), and then propylphosphonic anhydride (127.50 mg, 0.39 mmol) and N,N-diisopropyldiamine (75.22 mg, 0.58 mmol) were added. The mixture was stirred at room temperature for 2 hrs. Then, the mixture was poured into water (2 mL), and then extracted with ethyl acetate (3 mL × 2). The organic phase was dried over anhydrous sodium sulfate, filtered under suction, concentrated, and purified by preparative high performance liquid chromatography (column: XBridge preparative OBD C18 column, 30 × 150 mm, 5 μm; mobile phase A: water (10 mmol/L $NH_4HCO_3$), mobile phase B: acetonitrile; flow rate: 60 mL/min; gradient: 30% B to 60% B in 8 min, 60% B;

wavelength: 220 nm; retention time: 6.45 min) to obtain the target product as a white solid (50 mg, 0.08 mmol, yield: 47%).

$^1H$ NMR(400 MHz, DMSO-$d_6$) δ(ppm): 12.45(s, 1H), 8.24(s, 1H), 7.91(s, 1H), 7.53(s, 1H), 6.27(s, 1H), 4.71-4.68(m, 1H), 4.43-4.36(m, 1H),4.16-4.10(m, 1H), 4.00-3.95(m, 1H), 3.71-3.63(m, 2H), 3.52-3.49(m, 2H), 3.15-3.09(m, 1H), 2.93-2.84(m, 1H), 2.82-2.74(m, 4H), 2.62-2.58(m, 2H), 2.11-2.06(m, 1H), 1.69-1.52(m, 1H), 1.22-1.11(m, 3H). LCMS: MS(ESI)m/z: 549.20 $[M+H]^+$.

**Example 33:** Synthesis of (S)-5-cyclopropyl-8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-5H-pyrazino[1,2-a]pyrido[3,2-e]pyrazin-6(*6aH*)-one **(Compound 33)**

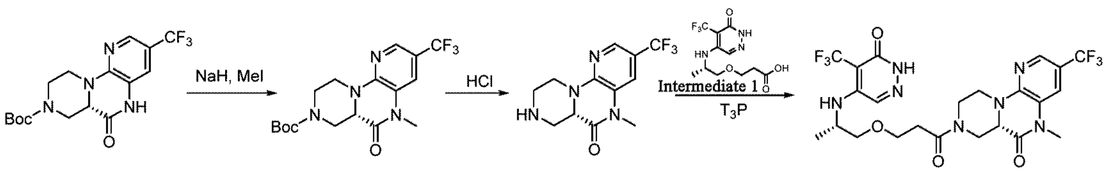


30-3 33-1 33-2 Compound 33

Step I: Synthesis of t-butyl (S)-5-methyl-6-oxo-3-(trifluoromethyl)-5,6,6a,7,9,10-hexahydro-*8H*-pyrazino[1,2-a]pyrido[3,2-e]pyrazin-8-carboxylate **(33-1)**

t-butyl (s)-6-oxo-3-(trifluoromethyl)-5,6,6a,7,9,10-hexahydro-*8H*-pyrazino[1,2-a]-pyrido[3,2-e]pyrazin-8-carboxylate (200 mg, 0.54 mmol) was added to tetrahydrofuran (5 mL), and cooled to 0°C. Then sodium hydride (26 mg, 0.64 mmol) was added. The mixture was stirred at room temperature for 1 hr, and then cooled to 0°C. The mixture was slowly added with iodomethane (113.60 mg, 0.80 mmol), reacted for 1 hr at room temperature, and then quenched with ice water (10 mL). The reaction solution was extracted with EA (10 mL × 3). The organic layer was collected, and dried over anhydrous sodium sulfate. The filtrate was concentrated. The crude product was purified by column chromatography on silica gel to obtain the target product as a colorless oil (100 mg, 0.26 mmol, yield: 48%). LCMS: MS(ESI)m/z: 387 $[M+H]^+$.

Step II: Synthesis of (S)-5-methyl-3-(trifluoromethyl)-7,8,9,10-tetrahydro-*5H*-pyrazino[1,2-a]pyrido[3,2-e]pyrazin-6(*6aH*)-one **(33-2)**

t-butyl (s)-5-methyl-6-oxo-3-(trifluoromethyl)-5,6,6a,7,9,10-hexahydro-8H-pyrazino[1,2-a]pyrido[3,2-e]pyrazin-8-carboxylate (90 mg, 0.23 mmol) was added to a hydrochloric acid/dioxane solution (5 mL). The mixture was stirred at room temperature for 1 hr, dried and concentrated under reduced pressure, to obtain a crude product as an orange solid (80 mg, yield: 100%). The crude product was used directly in the next reaction without purification. LCMS: MS(ESI)m/z: 287$[M+H]^+$.

Step III: Synthesis of (S)-5-methyl-8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-*5H*-pyrazino[1,2-a]pyrido[3,2-e]pyrazin-6(*6aH*)-one **(Compound 33)**

(S)-5-methyl-3-(trifluoromethyl)-7,8,9,10-tetrahydro-5*H*-pyrazino[1,2-a]pyrido[3,2-e]pyrazin-6(*6aH*)-one (80mg, 0.27mmol) was dissolved in dichloromethane (1 mL), and then (S)-3-(2-((6-oxo-5-(trifluoromethyl)-1,6-dihydropyridazin-4-yl)amino)propoxy)propionic acid (85.84 mg, 0.27 mmol), propylphosphonic anhydride (355.84 mg, 0.54 mmol) and N,N-diisopropylethyl amine (180.40 mg, 1.36 mmol) were added. The mixture was stirred at room temperature for 1 hr. Then the reaction solution was added with water (5 mL), and extracted with dichloromethane (5 mL × 3). Then the organic layer was collected, dried over sodium sulfate, and filtered to remove sodium sulfate. The filtrate was concentrated to obtain a crude product, which was purified by high performance liquid chromatography (chromatographic column: Xsele CSH C18 OBD chromatographic column 30 × 150 mm 5 μm; mobile phase A: water (0.05% FA), mobile phase B: ACN; flow rate: 60 mL/min; gradient: 15% B to 55% B over 8min, 55% B; wavelength: 220 nm; RT1 (minimum): 7.82) to obtain the target product as a yellow solid (22.90 mg, 0.039 mmol, yield: 14.4%).

$^1H$ NMR(400 MHz, DMSO-$d_6$) δ(ppm): 12.434(s, 1H), 8.206(s, 1H), 7.99-7.80(m, 1H), 7.56-7.39(m, 1H), 6.39-6.21(m, 1H), 4.59-4.43(m, 2H), 4.33-3.98(m, 3H), 3.78-.3.63(m, 2H), 3.57-3.46(m, 3H), 3.32-3.31(m, 3H),3.19-3.05(m, 1H), 2.89-2.68(m, 2H),1.21-1.12(m, 3H). LCMS: MS(ESI)m/z: 578.05$[M+H]^+$.

**Example 34** Synthesis of propionamide 5-(((S)-1-(3-oxo-3-((S)-3-(trifluoromethyl)-6a,7,9,10-tetrahydrobipyrazino[2,3-b: 1[1,4]oxazin-8(*6H*)-yl)propoxy)prop-2-yl)amino)-4-(trifluoromethyl)pyridazin-3(*2H*)-one **(Compound 34)**

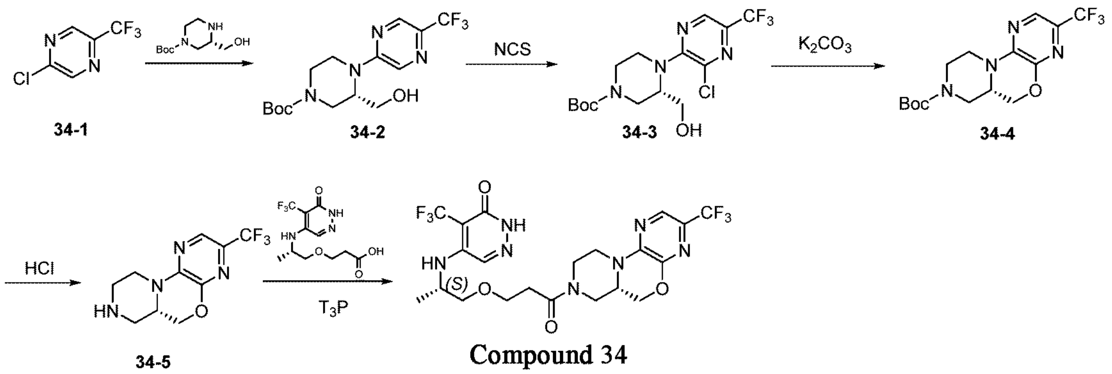

34-1 34-2 34-3 34-4 34-5 Compound 34

Step I: Synthesis of t-butyl (S)-3-(hydroxymethyl)-4-(5-(trifluoromethyl)pyrazin-2-yl)piperazin-1-carboxylate **(34-2)**

Chloro-5-(trifluoromethyl)pyrazine (1.00 g, 5.50 mmol) was dissolved in dimethylformamide (20 mL), and then t-butyl (S)-3-(hydroxymethyl)piperazin-1-carboxylate (1.42 g, 6.59 mmol) and potassium carbonate (1.52 g, 10.99 mmol) were added. The mixture was stirred for 12 hrs at 60°C. The reaction solution was added with water (50 mL), and then extracted with ethyl acetate (50 mL × 3). The organic layer was collected, dried over anhydrous sodium sulfate, and filtered. The filtrate was concentrated, and the resulting crude product was purified by column chromatography on silica gel (petroleum ether: ethyl acetate =60: 40) to obtain the target product as an orange oil (0.47 g, 1.29 mmol, yield: 23%). LCMS: MS(ESI)m/z: 363 $[M+H]^+$.

Step II: Synthesis of t-butyl (S)-4-(3-chloro-5-(trifluoromethyl)pyrazin-2-yl)-3-(hydroxymethyl)piperazin-1-carboxylate **(34-3)**

t-butyl (S)-4-(3-chloro-5-(trifluoromethyl)pyrazin-2-yl)-3-(hydroxymethyl)piperazin-1-carboxylate (460 mg, 1.27 mmol) was added to dimethylformamide (10 mL), and then N-chlorosuccinimide (254 mg, 1.90 mmol) was added. The mixture was stirred for 16 hrs at 50°C. After the reaction was completed, the reaction solution was added with water (100 mL), and then extracted with ethyl acetate (50 mL × 3). The organic layer was then collected, dried over anhydrous sodium sulfate, and filtered. The filtrate was concentrated to obtain a crude product. The obtained crude product was purified by column chromatography (petroleum ether: ethyl acetate =30: 70) to obtain the target product as a yellow oil (102 mg, 0.26 mmol, yield: 20%). LCMS: MS(ESI)m/z: 397$[M+H]^+$.

Step III: Synthesis of t-butyl (S)-3-(trifluoromethyl)-6a,7,9,10-tetrahydrobipyrazino[2,3-b: 1',2'-d][1,4]oxazin-8(*6H*)-carboxylate **(34-4)**

t-butyl (S)-4-(3-chloro-5-(trifluoromethyl)pyrazin-2-yl)-3-(hydroxymethyl)piperazin-1-carboxylate (100 mg, 0.25 mmol) was added to dimethylformamide (2 mL), and then potassium carbonate (70 mg, 0.50 mmol) was added. The mixture was stirred at room temperature for 2 hrs. The reaction solution was added with water (5 mL) and then extracted with ethyl acetate (5 mL × 3). The organic layer was collected, dried over anhydrous sodium sulfate, and filtered. The filtrate was concentrated to obtain a crude product, which was purified by column chromatography on silica gel (petroleum ether: ethyl acetate =65: 35) to obtain the target product t-butyl (S)-3-(trifluoromethyl)-6a,7,9,10-tetrahydrobipyrazino[2,3-b: 1',2'-d][1,4]oxazin-8(6H)-carboxylate as a colorless oil (60 mg, 0.17 mmol, yield: 66%). LCMS: MS(ESI)m/z: 361$[M+H]^+$.

Step IV: Synthesis of (S)-3-(trifluoromethyl)-6,6a,7,8,9,10-hexahydrobipyrazino[2,3-b: 1',2'-D] [1,4]oxazine **(34-5)**

t-butyl (S)-3-(trifluoromethyl)-6a,7,9,10-tetrahydrobipyrazino[2,3-b: 1',2'-d][1,4]oxazin-8(6H)-carboxylate (50 mg, 0.13 mmol) was dissolved in hydrochloric acid/dioxane solution (5 mL). The mixture was stirred at room temperature for 2 hrs. The mixture was quenched with water (10 mL), and then extracted with ethyl acetate (20 mL × 2). The organic layer was washed with saturated sodium chloride solution, dried over anhydrous sodium sulfate, filtered, and concentrated to obtain a crude target product as a colorless oil (50 mg, yield: 100%). The crude product was used directly in the next reaction without further purification. LCMS: MS(ESI)m/z: 261$[M+H]^+$.

Step V: Synthesis of propionamide 5-(((S)-1-(3-oxo-3-((S)-3-(trifluoromethyl)-6a,7,9,10-tetrahydrobipyrazino[2,3-b: 1',2'-d][1,4]oxazin-8(6H)-yl)propoxy)prop-2-yl)amino)-4-(trifluoromethyl)pyridazin-3(2*H*)-one **(Compound 34)**

(S)-3-(trifluoromethyl)-6,6a7,8,9,10-hexahydrobipyrazino[2,3-b: 1',2'-D][1,4]oxazine (50 mg, 0.19 mmol) was added to dichloromethane (5 mL), and then (S)-3-(2-((6-oxo-5-(trifluoromethyl)-1,6-dihydropyridazin-4-yl)amino)propoxy)propionic acid (59 mg, 0.19 mmol), propylphosphonic anhydride (351.12 mg, 0.57 mmol), and N,N-diisopropylethyl amine (72.96 mg, 0.57 mmol) were added. The mixture was stirred at room temperature for 2 hrs. Then, the reaction solution was added with water (10 mL), and extracted with dichloromethane (5 mL × 3). The organic layer was collected, dried over anhydrous sodium sulfate and filtered. The filtrate was concentrated, and the obtained crude product was purified by high-performance liquid chromatography (Xsele CSH OBD column 30 × 150 mm, 5 um; mobile phase A: water (10 mmol/L ammonium bicarbonate), mobile phase B: acetonitrile; flow rate: 60 mL/min; gradient: 10% B to 60% B in 8 min; wavelength: 220 nm; retention time: 7.17 min) to obtain the target product as a white solid (6.38 mg, 0.01 mmol, yield: 6%).

$^1$H NMR(400 MHz, DMSO-*d*$_6$) δ(ppm): 12.53(s, 1H), 8.17(s, 1H), 8.01-7.78(m, 1H), 6.41-6.12(m, 1H), 4.69-4.55(m, 1H), 4.55-4.45(m, 1H), 4.45-4.33(m, 1H),4.28-4.13(m, 2H),4.13-4.01(m, 1H), 3.73-3.65(m, 2H), 3.58-3.46(m, 3H), 3.25-3.12(m, 1H),2.98-2.82(m, 2H), 2.65-2.61(m, 2H),1.22-1.09(m, 3H). LCMS: MS(ESI)m/z: 552.05 [M+H]$^+$.

**Example 35:** 5-(((S)-1-(3-oxo-3-((S)-3-(trifluoromethyl)-6,7,7a,8,10,11-hexahydropyrazino[1,2-d]pyrido[3,2-b][1,4]diazepine -9(*5H*)-yl)propoxy)prop-2-yl)amino)-4-(trifluoromethyl)pyridazin-3(*2H*)-one **(Compound 35)**

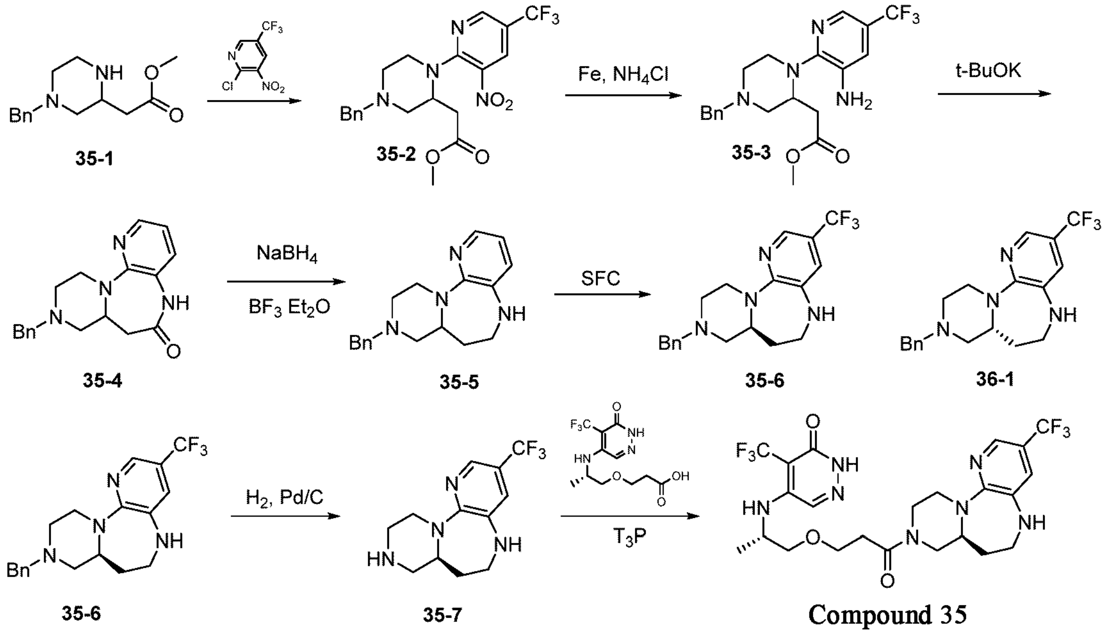


35-1 35-2 35-3

35-4 35-5 35-6 36-1

35-6 35-7 Compound 35

Step I: Synthesis of methyl 2-(4-benzyl-1-(3-nitro-5-(trifluoromethyl)pyridin-2-yl)piperazin-2-yl)acetate **(35-2)**

Methyl 2-(4-benzylpiperazin-2-yl)acetate (3.00 g, 12.09 mmol) was added to dimethylformamide (60 mL). Then 2-chloro-3-nitro-5-(trifluoromethyl)pyridine (2.72 g, 12.09 mmol) and potassium carbonate (3.33 g, 24.18 mmol) were added. The mixture was stirred at 60°C for 13 hrs. After the reaction was completed, the reaction solution was added with water (100 mL), and then extracted with ethyl acetate (50 mL × 3). The organic layer was collected, dried over anhydrous sodium sulfate, and filtered. The filtrate was concentrated, to obtain a crude product. The crude product was purified by column chromatography on silica gel (EA: PE=30: 60) to obtain the target product as a yellow oil (4.00 g, 9.13 mmol, yield: 75.51%). LCMS: MS(ESI)m/z: 438[M+H]$^+$.

Step II: Synthesis of methyl 2-(1-(3-amino-5-(trifluoromethyl)pyridin-2-yl)-4-benzylpiperazin-2-yl)acetate **(35-3)**

Methyl 2-(4-benzyl-1-(3-nitro-5-(trifluoromethyl)pyridin-2-yl)piperazin-2-yl)acetate (3.80 g, 8.67 mmol) was added to ethanol (80 mL), and then iron power (7.28 g, 130.05 mmol) and ammonium chloride (6.89 g, 130.05 mmol) was added to the solution. The mixture was stirred for 2 hrs at 80°C. The reaction solution was filtered through a Buchner funnel. The filtrate was dried under reduced pressure, then dissolved in water (100 mL), and then extracted with ethyl acetate (50 mL × 3). The organic layer was collected, dried over anhydrous sodium sulfate, and filtered. The filtrate was concentrated, to obtain a crude product. The crude product was purified by column chromatography on silica gel (EA: PE=20: 80) to obtain the target product as a yellow oil (2.40 g, 5.86 mmol, yield: 69%). LCMS: MS(ESI)m/z: 409[M+H]$^+$.

Step III: Synthesis of 9-benzyl-3-(trifluoromethyl)-7,7a,8,9,10,11-hexahydropyrazino[1,2-d]pyrido[3,2-b][1,4]diazepan-6(*5H*)-one **(35-4)**

Methyl 2-(1-(3-amino-5-(trifluoromethyl)pyridin-2-yl)-4-benzylpiperazin-2-yl)acetate (2.40 g, 5.88 mmol) was added to tetrahydrofuran (80 mL). Then, potassium tert-butoxide (1.31g, 11.76 mmol) was added to the reaction solution. The mixture was stirred at room temperature for 2 hrs. After the reaction was completed, the reaction solution was added with water (100mL), and then extracted with ethyl acetate (50 mL × 3). The organic layer was collected, dried over anhydrous sodium sulfate, and filtered. The filtrate was concentrated, to obtain a crude product. The crude product was purified by column chromatography on silica gel (EA: PE=30: 70) to obtain the target product as a white solid (1.50 g, 4.8 mmol, yield: 68%). LCMS: MS(ESI)m/z: 309[M+H]$^+$.

Step IV: Synthesis of (S)-5-cyclopropyl-3-(trifluoromethyl)-7,8,9,10-tetrahydro-*5H*-pyrazino[1,2-a]pyrido[3,2-e]pyrazin-6(*6aH*)-one **(35-5)**

9-benzyl-3(trifluoromethyl)-7,7a,8,9,10,11-hexahydropyrazino[1,2-d]pyrido[3,2-b][1,4]diazepan-6(*5H*)-one (1.20 g, 3.19 mmol) was added to tetrahydrofuran (20 mL). Then boron trifluoride (2 mL) and sodium borohydride (1.20 g, 31.90 mmol) were added. The mixture was stirred at room temperature for 1 hr. After the reaction was completed, the reaction solution was quenched with ice water (40 mL), and then extracted with ethyl acetate (20 mL × 3). The organic layer was collected, dried over anhydrous sodium sulfate, and filtered. The filtrate was concentrated, to obtain a crude product. The crude product was purified by column chromatography on silica gel (EA: PE=30: 70) to obtain the target product as a yellow solid (0.30 g, 1.02 mmol, yield: 26%). LCMS: MS(ESI)m/z: 295[M+H]$^+$.

Step V: Synthesis of (S)-9-benzyl-3-(trifluoromethyl)-5,6,7,7a,8,9,10,11-octahydropyrazino[1,2-d]pyrido[3,2-b][1,4]diazepine **(35-6)**

9-benzyl-3(trifluoromethyl)-5,6,7,7a,8,9,10,11-octahydropyrido[1,2-d]pyrido[3,2-b][1,4]diazepine (0.30 g, 1.02 mmol) was chirally resolved by high performance liquid chromatography (chromatographic column: CHIRALPAK IG-3, 4.6 × 50 mm 3 um; mobile phase A: n-hexane (0.1% diethyl amine), mobile phase B: ethanol; flow rate: 20 ml/ min; gradient: 10% B to 10% B over 12.5 min; wavelength: 220/254 nm; retention time 1: 9.1 min, retention time 2: 11.3 min) to obtain (S)-9-benzyl-3-(trifluoromethyl)-5,6,7,7a,8,9,10,11-octahydropyrazino[1,2-d]pyrido[3,2-b][1,4]diazepine as a white solid (**35-6**, 120 mg, 0.40 mmol, yield: 40%), and (R)-9-benzyl-3-(trifluoromethyl)-5,6,7,7a,8,9,10,11-octahydropyrazino[1,2-d]pyrido[3,2-b][1,4]diazepine as a white solid (**36-1**, 120mg, 0.40mmol, yield: 40%).

First peak: (S)-9-benzyl-3-(trifluoromethyl)-5,6,7,7a,8,9,10,11-octahydropyrazino[1,2-d]pyrido[3,2-b][1,4]diazepine

LCMS: MS(ESI)m/z: 363[M+H]$^+$.
Chiral HPLC: CHIRALPAK IG-3 4.6*50 mm, 3 um; mobile phase A: (0.1% ethylene diamine) n-hexane, mobile phase B: ethanol; mobile phase A: mobile phase B=90:10; flow rate: 1ml/min; temperature: 25°C; retention time: 2.17 min.

Second peak: (R)-9-benzyl-3-(trifluoromethyl)-5,6,7,7a,8,9,10,11-octahydropyrazino[1,2-d]pyrido[3,2-b][1,4]diazepine

LCMS: MS(ESI)m/z: 363[M+H]$^+$.
Chiral HPLC: CHIRALPAK IG-3 4.6*50 mm, 3 um; mobile phase A: (0.1% ethylene diamine) n-hexane, mobile phase B: ethanol; mobile phase A: mobile phase B=90:10; flow rate: 1ml/min; temperature: 25°C; retention time: 2.51 min.

Step VI: Synthesis of (S)-3-(trifluoromethyl)-5,6,7,7a,8,9,10,11-octahydropyrazino[1,2-d]pyrido[3,2-b][1,4]diazepine **(35-7)**

To a solution of (S)-9-benzyl-3-(trifluoromethyl)-5,6,7,7a,8,9,10,11-octahydropyrazino[1,2-d]pyrido[3,2-b][1,4]diazepine (110 mg, 0.33 mmol) in glacial acetic acid (5 mL), palladium/carbon (11mg) was added. The mixture was stirred for 16 hrs under H2 atmosphere at room temperature. The reaction solution was filtered through a Buchner

funnel. The filter cake was washed with methanol (10 mL × 2), and the filtrate was concentrated under reduced pressure to obtain a crude product. The crude product was purified by column chromatography on silica gel (EA: PE=20: 80) to obtain the target product as a yellow oil (40 mg, 0.15 mmol, yield: 49%). LCMS: MS(ESI)m/z: 273[M+H]$^+$.

Step VII: Synthesis of 5-(((S)-1-(3-oxo-3-((S)-3-(trifluoromethyl)-6,7,7a,8,10,11-hexahydropyrazino[1,2-d]pyrido[3,2-b][1,4]diazepine -9(*5H*)-yl)propoxy)prop-2-yl)amino)-4-(trifluoromethyl)pyridazin-3(*2H*)-one **(Compound 35)**

(S)-3-(trifluoromethyl)-5,6,7,7a,8,9,10,11-octahydropyrazino[1,2-d]pyrido[3,2-b][1,4]diazepine (38 mg, 0.14 mmol) was added to dimethylformamide (1 mL, 12.86 mmol), and then (S)-3-(2-((6-oxo-5-(trifluoromethyl)-1,6-dihydropyridazin-4-yl)amino)propoxy) (38 mg, 0.12 mmol), propylphosphonic anhydride (200 mg, 1.20 mmol), and N,N-diisopropylethyl amine (0.27 mg, 1.55 mmol) were added. The mixture was stirred at room temperature for 1 hr. After the reaction was completed, the reaction solution was added with water (10 mL). The mixture was extracted with ethyl acetate (5 mL × 2). The organic layer was washed with saturated sodium chloride solution, dried over anhydrous sodium sulfate, and filtered. The filtrate was concentrated, to obtain a crude product. The crude product was purified by preparative high performance liquid chromatography (XBridge preparative OBD C18 column, 30 × 150 mm, 5 μm; mobile phase A: water (10 mmol/L ammonium bicarbonate), mobile phase B: acetonitrile; flow rate: 60 mL/min; gradient: 10% B to 54% B in 10 min; wavelength: 220 nm; retention time: 9.22min) to obtain the target product as a white solid (5mg, 0.01 mmol, yield: 6%).

LCMS: MS(ESI)m/z: 564.10 [M+H]$^+$. $^1$H NMR(400 MHz, DMSO-$d_6$) δ(ppm): 12.44(s, 1H), 7.89(s, 1H), 7.80-7.77(m, 1H), 6.98(s, 1H), 6.27-6.23(s, 1H), 5.77-5.75(m, 1H), 4.19-4.09(m, 1H), 3.90-3.82(m, 1H), 3.77-3.64(m, 4H), 3.60-3.53(m, 2H), 3.50-3.41(m, 5H), 3.20-3.15(m, 1H), 2.58-2.54(m, 2H), 1.94-1.79(m, 1H), 1.72-1.61(m, 1H), 1.25-1.09(m, 3H).

**Example 36** 5-(((R)-1-(3-oxo-3-((S)-3-(trifluoromethyl)-6,7,7a,8,10,11-hexahydropyrazino[1,2-d]pyrido[3,2-b][1,4]diazepine -9(*5H*)-yl)propoxy)prop-2-yl)amino)-4-(trifluoromethyl)pyridazin-3(*2H*)-one **(Compound 36)**

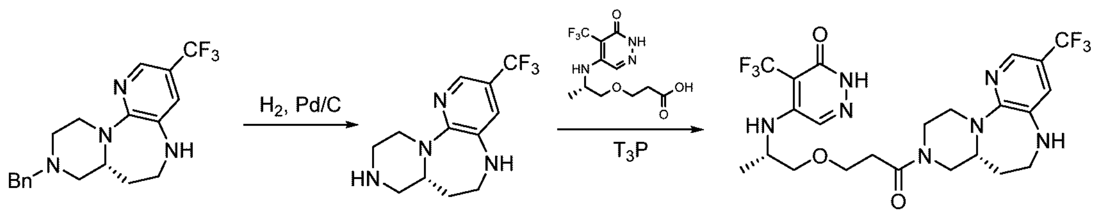


36-1 36-2 Compound·36

Step I: Synthesis of (R)-3-(trifluoromethyl)-5,6,7,7a,8,9,10,11-octahydropyrazino[1,2-d]pyrido[3,2-b][1,4]diazepine **(36-2)**

To a solution of (R)-9-benzyl-3-(trifluoromethyl)-5,6,7,7a,8,9,10,11-octahydropyrazino[1,2-d]pyrido[3,2-b][1,4]diazepine **36-1** (110 mg, 0.33 mmol) in glacial acetic acid (5 mL), palladium/carbon was added (11 mg). The mixture was stirred for 16 hrs under hydrogen atmosphere at room temperature. The reaction solution was filtered through a Buchner funnel. The filter cake was washed with methanol (5 mL × 2), and the filtrate was concentrated under reduced pressure to obtain a crude product. The crude product was purified by column chromatography on silica gel (ethyl acetate: petroleum ether=20: 80) to obtain the target product as a yellow oil (80 mg, 0.29 mmol, yield: 49%). LCMS: MS(ESI)m/z: 273[M+H]$^+$.

Step II: Synthesis of 5-(((R)-1-(3-oxo-3-((S)-3-(trifluoromethyl)-6,7,7a,8,10,11-hexahydropyrazino[1,2-d]pyrido[3,2-b][1,4]diazepine -9(*5H*)-yl)propoxy)prop-2-yl)amino)-4-(trifluoromethyl)pyridazin-3(*2H*)-one **(Compound 36)**

(R)-3-(trifluoromethyl)-5,6,7,7a,8,9,10,11-octahydropyrazino[1,2-d]pyrido[3,2-b][1,4]diazepine (78 mg, 0.28 mmol) was added to N,N-dimethylformamide (5 mL), and then (S)-3-(2-((6-oxo-5-(trifluoromethyl)-1,6-dihydropyridazin-4-yl)amino)propoxy) **(Intermediate 1,** 88.60 mg, 0.28 mmol), propylphosphonic anhydride (363 mg, 2.8 mmol) and N,N-diisopropylethyl amine (184 mg, 1.40 mmol) were added. The mixture was stirred at room temperature for 1 hr. After the reaction was completed, the mixed solution was added with water (5 mL), and then extracted with ethyl acetate (5 mL × 2). The organic layer was washed with saturated sodium chloride solution, dried over anhydrous sodium sulfate, and filtered. The filtrate was concentrated, to obtain a crude product. The crude product was purified by preparative high

performance liquid chromatography (XBridge preparative OBD C18 column, 30 × 150 mm, 5 μm; mobile phase A: water (containing 10 mmol/L ammonium bicarbonate), mobile phase B: acetonitrile; flow rate: 60 mL/min; gradient: 10% B to 54% B in 10 min; wavelength: 220 nm; retention time: 9.22 min) to obtain the target product as a white solid (7.79 mg, 0.01 mmol, yield: 9.29%).

LCMS: MS(ESI)m/z: 564.10 $[M+H]^+$. $^1H$ NMR(400 MHz, DMSO-$d_6$) δ(ppm): 12.54(s, 1H), 8.02-7.85(m, 1H), 7.790(s, 1H), 6.39-6.12(m, 1H), 5.88-5.62(m, 1H), 4.28-3.99(m, 1H), 3.91-3.83(m, 1H), 3.82-3.62(m, 4H), 3.61-3.51(m, 2H), 3.51-3.45(m, 3H), 3.45-3.37(m, 1H), 3.30-3.12(m, 2H), 2.58-2.52(m, 2H), 2.06-1.74(m, 1H) , 1.74-1.51(m, 1H), 1.28-1.10(m, 3H).

**Example 37:** Synthesis of 8-(3-((S)-2-((6-oxo-5-(trifluoromethyl)-1,6-dihydropyridazin-4-yl)amino)propoxy)propionyl)-3-(trifluoromethyl)-6,6a,7,8,9,10-hexahydro-*5H*-pyrazino[1,2-a][1,8]naphthyridin-5-one **(Compound 37)**

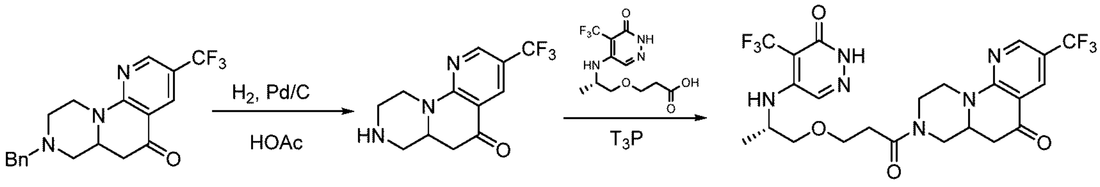


Intermediate 5 37-1 Compound 37

Step I: Synthesis of 3-(trifluoromethyl)-6,6a,7,8,9,10-hexahydro-*5H*-pyrazino[1,2-a][1,8]naphthyridin-5-one (**37-1**)

A mixture of 8-benzyl-3-(trifluoromethyl)-6,6a,7,8,9,10-hexahydro-*5H*-pyrazino[1,2-a][1,8]naphthyridin-5-imine **Intermediate 5** (1.00 g, 2.77 mmol) and palladium/carbon (0.10 g) in acetic acid (10 mL) was stirred for 2 hrs under hydrogen atmosphere at room temperature, then filtered and concentrated. The residue was purified by column chromatography (acetonitrile: water =30: 70) to obtain the target product as a yellow oil (0.52 g, 1.91 mmol, yield: 69%). LCMS: MS(ESI)m/z: 272 $[M+H]^+$.

Step II: Synthesis of 8-(3-((S)-2-((6-oxo-5-(trifluoromethyl)-1,6-dihydropyridazin-4-yl)amino)propoxy)propionyl)-3-(trifluoromethyl)-6,6a,7,8,9,10-hexahydro-5*H*-pyrazino[1,2-a][1,8]naphthyridin-5-one (**Compound 37**)

A solution of 3-(trifluoromethyl)-6,6a,7,8,9,10-hexahydro-5*H*-pyrazino[1,2-a][1,8]naphthyridin-5-one (0.05 g, 0.18 mmol) and (S)-3-(2-((6-oxo-5-(trifluoromethyl)-1,6-dihydropyridazin-4-yl)amino)propoxy)propionic acid (**Intermediate1**, 0.06 g, 0.18 mmol) in dichloromethane (3mL) was added with propylphosphonic anhydride (0.28 g, 0.92 mmol) and N,N-diisopropylethylene diamine (0.12 g, 0.92 mmol). The mixture was stirred at room temperature for 2 hrs, then poured into water (10 mL), extracted with dichloromethane (10 mL), dried over anhydrous sodium sulfate, filtered under suction, rotary dried, and purified by preparative high performance liquid chromatography (chromatographic column: XBridge Prep OBD C18 chromatographic column, 30 x 150 mm, 5 μm; mobile phase A: water (10 mmol/L ammonium bicarbonate), mobile phase B: acetonitrile; flow rate: 60 mL/min; gradient: 25% B to 55% B in 8 min; wavelength: 220 nm; retention time: 6.73 min) to obtain the target product as a white solid (8.12 mg, 0.01 mmol, yield 8%).

LCMS: MS(ESI)m/z: 563.05$[M+H]^+$. $^1H$ NMR (400 MHz, DMSO-$d_6$) δ(ppm): 12.46(s, 1H), 8.75(s, 1H), 8.13-8.12(d, J = 2.6 Hz, 1H), 7.91(s, 1H), 6.28(s, 1H), 4.68-4.58(m, 1H), 4.54-4.47(m, 1H), 4.19-4.04(m, 2H), 3.78-3.65(m, 3H), 3.48(d, J = 5.6 Hz, 2H), 3.26-3.21(m, 1H), 3.13-3.05(m, 1H), 2.96-2.90(m, 1H), 2.85-2.82(m, 2H), 2.64-2.62(m, 2H), 1.15(d, 3H, J = 6.4 Hz).

**Example 38:** Synthesis of 5-(((2S)-1-(3-(5-hydroxy-3-(trifluoromethyl)-5,6,6a,7,9,10-hexahydro-8*H*-pyrazino[1,2-a][1,8]naphthyridin-8-yl)-3-oxopropoxy)prop-2-yl)amino)-4-(trifluoromethyl)pyridazin-3(2*H*)-one (**Compound 38**)

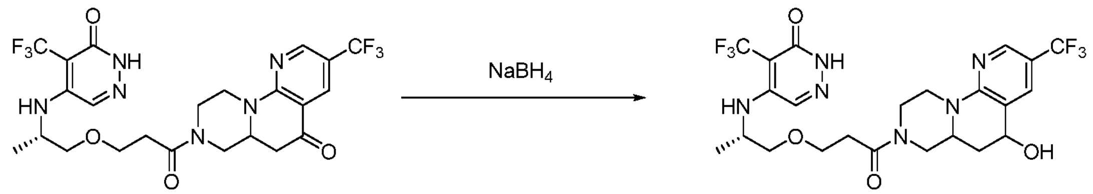


A mixture of 8-(3-((S)-2-((6-oxo-5-(trifluoromethyl)-1, 6-dihydropyridazin-4-yl)amino)propoxy)propionyl)-3-(trifluoromethyl)-6,6a,7,8,9,10-hexahydro-5*H*-pyrazino[1,2-a][1,8]naphthyridin-5-one (**Compound 37**, 50 mg, 0.09 mmol) in methanol (1mL) was added with sodium borohydride (7.11 mg, 0.18 mmol). The mixture was stirred at room temperature for 2 hrs, then poured into water (10 mL), extracted with dichloromethane (10 mL), dried over anhydrous sodium sulfate, and filtered under suction. The filtrate was concentrated, and the residue was purified by preparative high performance liquid chromatography (column: XBridge preparative OBD C18 column, 30 x 150 mm, 5 um; mobile phase A: water (10mol/L ammonium bicarbonate), mobile phase B: acetonitrile; flow rate: 60 mL/min; gradient: 10% B to 35% B in 10 min; 220 nm; retention time: 9.10 min) to obtain the target product as a white solid (4.55 mg, 0.008 mmol, yield: 8%).

LCMS: MS(ESI)m/z: 565.20 $[M+H]^+$. $^1H$ NMR(400 MHz, DMSO-$d_6$) δ(ppm): 12.45(s, 1H), 8.29(s, 1H), 7.94-7.89(m, 1H), 7.71-7.69(m, 1H), 6.28-6.27(m, 1H), 5.80-5.78(m, 1H), 4.71-4.63(m, 2H), 4.49-4.42(m, 1H), 4.19-4.11(m, 1H), 4.09-3.99(m, 1H), 3.72-3.68(m, 2H), 3.52-3.49(m, 2H),3.21- 3.11(m, 1H), 3.01-2.87(m, 1H), 2.88-2.79(m,1H), 2.76-2.71(m, 1H), 2.63-2.61(m, 2H), 2.32-2.18(m, 1H), 1.62-1.46(m, 1H), 1.17-1.12(m, 3H).

**Example 39:** Synthesis of (s)-5-(methyl-D3)-8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-*5H*-pyrazino[1,2-a]pyrido[3,2-e]pyrazino-6(6*aH*)-one **(Compound 39)**

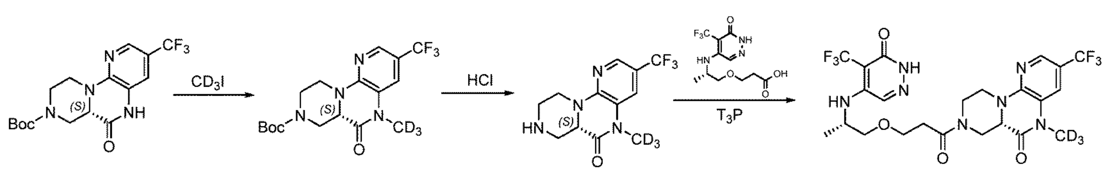


30-3 39-1 39-2 Compound 39

Step I: Synthesis of t-butyl (S)-5-(methyl-D3)-6-oxo-3-(trifluoromethyl)-5,6,6a,7,9,10-hexahydro-8*H*-pyrazino[1,2-a]pyrido[3,2-e]pyrazin-8-carboxylate **(39-1)**

t-butyl (S)-6-oxo-3-(trifluoromethyl)-5,6,6a,7,9,10-hexahydro-8H-pyrazino[1,2-a]pyrido[3,2-e]pyrazin-8-carboxylate **30-3** (500 mg, 1.3 mmol) was dissolved in tetrahydrofuran (10 mL), and sodium hydride (60%, 100 mg, 2.6 mmol) was added at 0°C. The mixture was stirred at room temperature for 1 hr, and then deuterated iodomethane (0.16 mL, 2.6 mmol) was added, and reacted for 2 hrs. The reaction solution was quenched with an ammonium chloride aqueous solution (20 mL), and extracted with ethyl acetate (40 mL x 2). The organic phases were combined, washed with saturated brine, dried over anhydrous sodium sulfate, and then filtered. The filtrate was rotary dried and then purified by column chromatography on silica gel (petroleum ether: ethyl acetate =70: 30) to obtain the target product as a yellow solid (170 mg, 0.43 mmol, yield: 32.5%). LCMS: MS(ESI)m/z: 390$[M+H]^+$.

Step II: Synthesis of (S)-5-(methyl-d3)-3-(trifluoromethyl)-7,8,9,10-tetrahydro-*5H*-pyrazino[1,2-a]pyrido[3,2-e]pyrazin-6(6*aH*)-one (**39-2**)

t-butyl (S)-5-(methyl-D3)-6-oxo-3-(trifluoromethyl)-5,6,6a,7,9,10-hexahydro-8*H*-pyrazino[1,2-a]pyrido[3,2-e]pyrazin-8-carboxylate (170 mg, 0.43 mmol) was added to a reaction flask, and then a hydrogen chloride/dioxane solution (5 mL) was added. The mixture was stirred at room temperature for 1 hr, and then concentrated under reduced pressure to obtain a crude product as a yellow solid (150 mg, 0.51 mmol). The crude product was used directly in the next reaction without purification. LCMS: MS(ESI)m/z: 290$[M+H]^+$.

Step III: Synthesis of (S)-5-(methyl-D3)-8-(3-((S)-2-((6-oxo-5-(trifluoromethyl)-1, 6-dihydropyridazin-4-yl)amino)propoxy)propionyl)-3-(trifluoromethyl)-7,8,9,10-tetrahydro-*5H*-pyrazino[1,2-a]pyrido[3,2-e]pyrazino-6(6*aH*)-one (**Compound 39**)

(S)-5-(methyl-D3)-3-(trifluoromethyl)-7,8,9,10-tetrahydro-*5H*-pyrazino[1,2-a]pyrido[3,2-e]pyrazin-6(6*aH*)-one (50 mg, 0.17 mmol) and (S)-3-(2-((6-oxo-5-(trifluoromethyl)-1,6-dihydropyridazin-4-yl)amino)propoxy)propionic acid (53 mg, 0.17 mmol) were dissolved in dichloromethane (5 mL), and then a solution of 1-propylphosphonic anhydride (162mg, 0.255mmol) in ethyl acetate and N,N-diisopropylethyl amine (65 mg, 0.34 mmol) were added. The mixture was stirred at room temperature for 2 hrs. After the reaction was completed, the mixture was poured into water (10 mL), and then extracted with dichloromethane (10 mL x 2). The organic phases were combined, washed with saturated brine, dried over anhydrous sodium sulfate, and filtration. The filtrate was rotary dried, and then purified by high performance liquid chromatography (column: XBridge Prep OBD C18, 30 x 150 mm 5 um, mobile phase A: water (containing 10 moL/L ammonium bicarbonate), mobile phase B: acetonitrile, flow rate: 60 mL/min, elution gradient: mobile phase B from 5% to 30% over 8 min, detection wavelength: 220 nm, retention time: 7.37 min) to obtain the target product as a white solid (42.38 mg, 0.07 mmol, yield: 38.48%).

LCMS: MS(ESI)m/z: 581.10[M+H]$^+$. $^1$H NMR (400 MHz, DMSO-$d_6$) δ(ppm): 8.19(s, 2H), 7.91(s, 1H), 7.47(s, 1H), 6.40-6.05(m, 1H), 4.94-4.24(m, 2H), 4.24-4.11(m, 1H), 4.11-3.97(m, 1H), 3.76-3.61(m, 2H), 3.56-3.44(m, 2H), 3.27-3.17(m, 1H), 3.15-3.02(m, 1H), 2.84-2.62(m, 4H), 1.22-1.08(m, 3H).

**Example 40:** Synthesis of (7aR)-5-methyl-3-(trifluoromethyl)-7,7a,8,9,10,11-hexahydro-*5H*-pyrazino[2,1-c]pyrido[2,3-e][1,4]thiazole **(Compound 40)**

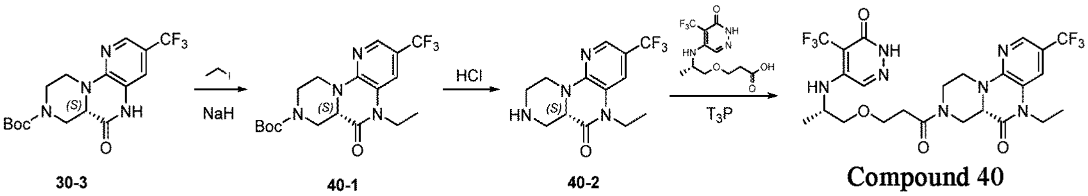


30-3 40-1 40-2 Compound 40

Step I: Synthesis of t-butyl (S)-5-ethyl-6-oxo-3-(trifluoromethyl)-5,6,6a,7,9,10-hexahydro-*8H*-pyrazino[1,2-a]pyrido[3,2-e]pyrazin-8-carboxylate **(40-1)**

t-butyl (S)-6-oxo-3-(trifluoromethyl)-5,6,6a,7,9,10-hexahydro-8*H*-pyrazino[1,2-a]pyrido[3,2-e]pyrazin-8-carboxylate **30-3** (500 mg, 1.3 mmol) was dissolved in DMF (10 mL), and then cooled to 0°C. Then, solid sodium hydride (60%, 100 mg, 2.6 mmol) was added, and stirred for 1 hr. Next, iodoethane (300 mg, 1.95 mmol) was added, and continuously reacted overnight at room temperature. After the reaction was completed, the reaction solution was poured into ice water (30 mL), and the aqueous phase was then extracted with ethyl acetate (30 mL x 3). The organic phases were combined, and dried over anhydrous sodium sulfate. Anhydrous sodium sulfate was removed by filtration, and then concentrated. The crude product was purified by column chromatography on silica gel (ethyl acetate : petroleum ether=50: 50) to obtain the target product as a yellow oil (200mg, 0.5mmol, yield : 37%), LCMS:(ESI) m/z: 401 [M+H]$^+$.

Step II: Synthesis of (S)-5-ethyl-3-(trifluoromethyl)-7,8,9,10-tetrahydro-*5H*-pyrazino[1,2-a]pyrido[3,2-e]pyrazin-6(6*aH*)-one **(40-2)**

t-butyl (S)-S-ethyl-6-oxo-3-(trifluoromethyl)-5,6,6a,7,9,10-hexahydro-*8H*-pyrazino[1,2-a]pyrido[3,2-e]pyrazin-8-carboxylate (200 mg, 0.5 mmol) was dissolved in ice-cold (0°C) hydrogen chloride -1,4-dioxane solution (4 M, 5 mL), slowly warmed to room temperature and reacted for 4 hrs with stirring. After complete reaction, the reaction solution was concentrated under reduced pressure. The crude product was diluted in water (30 mL), adjusted to pH 8-9 with sodium bicarbonate, and extracted with dichloromethane (30 mL x 3). The organic phases were combined, and dried over anhydrous sodium sulfate. Anhydrous sodium sulfate was removed by filtration, and then concentrated. The crude product was purified by column chromatography on silica gel (ethyl acetate : petroleum ether=50: 50) to obtain the target product as a yellow solid (100 mg, 0.33 mmol, yield: 66%), LCMS:(ESI) m/z: 301[M+H]$^+$.

Step III: Synthesis of (S)-5-ethyl-8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-*5H*-pyrazino[1,2-a]pyrido[3,2-e]pyrazin-6(*6aH*)-one **(Compound 40)**

(S)-5-ethyl-3-(trifluoromethyl)-7,8,9,10-tetrahydro-*5H*-pyrazino[1,2-a]pyrido[3,2-e]pyrazin-6(6*aH*)-one (50mg, 0.16mmol) and Intermediate 1(S)-3-(2-((6-oxo-5-(trifluoromethyl)-1,6-dihydropyridazin-4-yl)amino)propoxy)propionic acid (49 mg, 0.16 mmol) were dissolved in dichloromethane (1.0 mL), and then diisopropylethyl amine (124 mg, 0.96 mmol) and a propylphosphonic anhydride solution (305 mg, 0.48 mmol, 50% solution in ethyl acetate) were added. The reaction mixture was stirred at room temperature for 2 hrs. The reaction solution was poured into ice water (20mL), and the aqueous phase was then extracted with ethyl acetate (10 mL x 3). The organic phases were combined and dried over anhydrous sodium sulfate. Anhydrous sodium sulfate was removed by filtration, and the filtrate was rotary dried to obtain a crude product. The crude product was purified by preparative liquid-phase chromatography (column: XBridge Prep OBD C18, 30 x 150 mm 5 um, mobile phase A: water (containing 10 mol/L ammonium bicarbonate), mobile phase B: acetonitrile, flow rate: 60 mL/min, elution gradient: mobile phase B from 10% to 50% over 8 min, detection wavelength: 220 nm, retention time: 7.53 min) to obtain the target product as a white solid (1.18mg, 0.002mmol, yield: 1.25%).

LCMS: MS(ESI)m/z: 592.15$[M+H]^+$. $^1H$ NMR(400 MHz, DMSO-$d_6$) δ(ppm): 12.44(s, 1H), 8.20(s, 1H), 7.91-7.89(m, 1H), 7.53(s, 1H), 6.28(s, 1H), 4.86-4.45(m, 2H), 4.29-3.96(m, *5H),* 3.70-3.67(m, 2H), 3.49-3.48(m, 2H), 3.14-3.07(m, 1H), 2.81-2.73(m, 1H), 2.70-2.64(m, 3H), 1.16-1.09(m, 6H).

**Example 41:** Synthesis of (S)-5-(2-hydroxyethyl)-8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-5H-pyrazino[1,2-a]pyrido[3,2-e]pyrazino-6(6aH)-one **(Compound 41)**

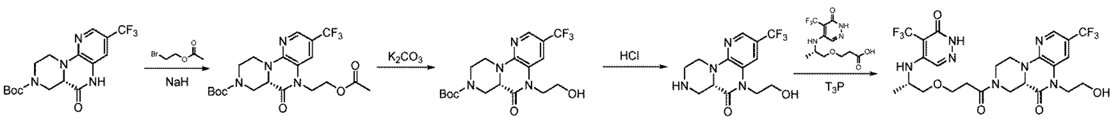


30-3 41-1 41-2 41-3 Compound 41

Step I: Synthesis of t-butyl (S)-5-(2-acetyloxyethyl)-6-oxo-3-(trifluoromethyl)-5,6,6a,7,9,10-hexahydro-*8H*-pyrazino[1,2-a]pyrido[3,2-e]pyrazin-8-carboxylate **(41-1)**

t-butyl (S)-6-oxo-3-(trifluoromethyl)-5,6,6a,7,9,10-hexahydro-*8H*-pyrazino[1,2-a]pyrido[3,2-e]pyrazin-8-carboxylate **30-3** (500 mg, 1.34 mmol) was dissolved in N,N-dimethylformamide (10 mL), and cooled to 0°C. Then solid sodium hydride (60%, 107 mg, 2.68 mmol) was added, and stirred for 1 hr at 0°C. Ethyl 2-bromoethylacetate (448 mg, 2.68 mmol) was then added, and further reacted overnight with stirring at room temperature. After the reaction was completed, the reaction solution was poured into ice water (20 mL), and then extracted with ethyl acetate (20 mL x 3). The organic phases were combined, and dried over anhydrous sodium sulfate. Anhydrous sodium sulfate was removed by filtration, and the filtrate was concentrated. The resulting crude product was purified by column chromatography on silica gel(ethyl acetate :petroleum ether=50: 50) to obtain the target product as a yellow oil (280 mg, 0.61 mmol, yield : 45%), LCMS (ESI): 459 $[M+H]^+$.

Step II: Synthesis of t-butyl (S)-5-(2-hydroxyethyl)-6-oxo-3-(trifluoromethyl)-5,6,6a,7,9,10-hexahydro-*8H*-pyrazino[1,2-a]pyrido[3,2-e]pyrazin-8-carboxylate **(41-2)**

t-butyl (S)-5-(2-acetyloxyethyl)-6-oxo-3-(trifluoromethyl)-5,6,6a,7,9,10-hexahydro-*8H*-pyrazino[1,2-a]pyrido[3,2-e]pyrazin-8-carboxylate (280 mg, 0.61 mmol) was dissolved in methanol (5 mL) and water (5 mL), and then solid potassium carbonate (168 mg, 1.22 mmol) was added, and reacted for 6 hrs at room temperature. After the reaction was completed, the reaction solution was poured into water (20 mL), and then extracted with ethyl acetate (20 mL x 3). The organic phases were combined, and dried over anhydrous sodium sulfate. Anhydrous sodium sulfate was removed by filtration, and the filtrate was concentrated under reduced pressure to obtain a crude product. The crude product was purified by column chromatography on silica gel to obtain the target product as a yellow oil (160 mg, yield: 63%), LCMS (ESI)m/z: 417$[M+H]^+$.

Step III: Synthesis of (S)-5-(2-hydroxyethyl)-3-(trifluoromethyl)-7,8,9,10-tetrahydro-5H-pyrazino[1,2-a]pyrido[3,2-e]pyrazin-6(*6aH*)-one **(41-3)**

The crude product t-butyl (S)-5-(2-hydroxyethyl)-6-oxo-3-(trifluoromethyl)-5,6,6a,7,9,10-hexahydro-*8H*-pyrazino[1,2-a]pyrido[3,2-e]pyrazin-8-carboxylate (160 mg) was dissolved in ice-cold (0°C) hydrogen chloride -1,4-dioxane solution (4 M, 8 mL), slowly warmed to room temperature, and reacted for 4 hrs with stirring. After complete reaction, the reaction solution was concentrated under reduced pressure. The crude product was diluted in water (20 mL), adjusted to pH 8-9 with sodium bicarbonate, and then extracted with dichloromethane (20 mL x 3). The organic phases were combined, and dried over anhydrous sodium sulfate. Anhydrous sodium sulfate was removed by filtration, and the filtrate was rotary dried. The crude product was purified by column chromatography on silica gel (ethyl acetate :petroleum ether=50: 50) to obtain the target product as a yellow solid (100 mg, 0.32 mmol, yield: 81.5%), LCMS (ESI)m/z: 317 [M+H]$^+$.

Step IV: Synthesis of (S)-5-(2-hydroxyethyl)-8-(3-((S)-2-((6-oxo-5-(trifluoromethyl)-1,6-dihydropyridazin-4-yl)amino)propoxy)propionyl)-3-(trifluoromethyl)-7,8,9,10-tetrahydro-*5H*-pyrazino[1,2-a]pyrido[3,2-e]pyrazino-6(6*aH*)-one **(Compound 41)**

(S)-5-(2-hydorxyethyl)-3-(trifluoromethyl)-7,8,9,10-tetrahydro-5H-pyrazino[1,2-a]pyrido[3,2-e]pyrazin-6(6aH)-one (100 mg, 0.32 mmol) and (S)-3-(2-((6-oxo-5-(trifluoromethyl)-1,6-dihydropyridazin-4-yl)amino)propoxy)propionic acid (99 mg, 0.32 mmol) were dissolved in dichloromethane (5.0 mL), and then diisopropylethyl amine (206 mg, 1.6 mmol) and a propylphosphonic anhydride solution (610 mg, 0.96 mmol, 50% solution in ethyl acetate) were added. The reaction mixture was stirred at room temperature for 2 hrs. Then the reaction solution was poured into ice water (20 mL), and the aqueous phase was extracted with dichloromethane (20 mL x 3). The organic phases were combined, and dried over anhydrous sodium sulfate. Anhydrous sodium sulfate was removed by filtration, and the filtrate was rotary dried to obtain a crude product. The crude product was purified by preparative liquid-phase chromatography (column: XBridge Prep OBD C18, 30 x 150 mm 5 um, mobile phase A: water (containing 10 mol/l ammonium bicarbonate), mobile phase B: acetonitrile, flow rate: 60 mL/ min, elution gradient: mobile phase B from 10% to 50% over 8 min, detection wavelength: 220 nm, retention time: 7.53 min) to obtain the target product as a white solid (19.41mg, 0.032mmol, yield: 10%).

LCMS: MS(ESI)m/z: 608.15 [M+H]$^+$. $^1$H NMR (400 MHz, DMSO-$d_6$) δ(ppm): 12.38 (s, 1H), 8.19 (s, 1H), 7.91-7.89 (m, 1H), 7.68 (s, 1H), 6.26 (s, 1H), 4.91-4.44 (m, 3H), 4.26-3.93 (m, 5H), 3.72-3.68 (m, 2H), 3.62-3.57 (m, 2H), 3.50-3.48 (m, 2H), 3.25-3.08 (m, 1H), 2.80-2.67 (m, 1H), 2.65-2.58 (m, 3H), 1.15 (d, J = 6.4 Hz, 3H).

**Example 42:** Synthesis of (S)-6-methyl-9-(3-((S)-2-((6-oxo-5-(trifluoromethyl)-1,6-dihydropyridazin-4-yl)amino)propoxy)propionyl)-3-(trifluoromethyl)-7,7a,8,9,10,11-hexahydropyrazino[1,2-a]pyrido[3,2-f][1,4]diazepan-5(*6H*)-one **(Compound 42)**

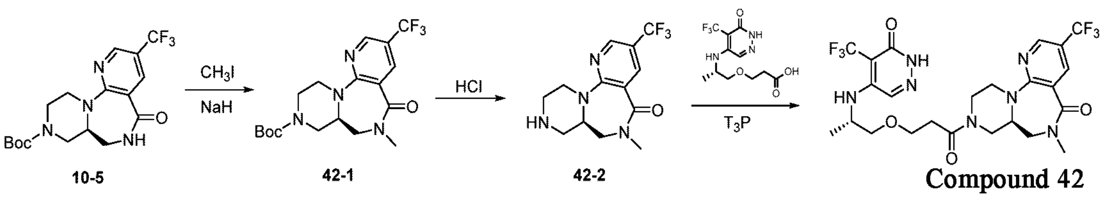


10-5 42-1 42-2 Compound 42

Step I: Synthesis of t-butyl (S)-6-methyl-5-oxo-3-(trifluoromethyl)-6,7,7a,8,10,11-hexahydropyrazino[1,2-a]pyrido[3,2-f][1,4]diazepan-9(*5H*)-carboxylate **(42-1)**

t-butyl (S)-5-oxo-3-(trifluoromethyl)-6,7,7a,8,10,11-hexahydropyrazino[1,2-a]pyrido[3,2-f][1,4]diazepan-9(*5H*)-carboxylate **10-5** (300 mg, 0.78 mmol) was dissolved in tetrahydrofuran (10 mL), and cooled to 0°C. Then solid sodium hydride (60%, 94 mg, 2.34 mmol) was added, and stirred for 1 hr at 0°C. Next, iodomethane (222 mg, 1.56 mmol) was added, and further reacted for 3 hrs at room temperature. After the reaction was completed, the reaction solution was poured into ice water (20 mL), and the aqueous phase was then extracted with ethyl acetate (20 mL x 3). The organic phases were combined, and dried over anhydrous sodium sulfate. Anhydrous sodium sulfate was removed by filtration, and the filtrate was rotary dried. The crude product was purified by column chromatography on silica gel (ethyl acetate :petroleum ether=40: 60) to obtain the target product as a yellow oil (240mg, 0.60mmol, yield : 77%), LCMS: (ESI)m/z: 401 [M+H]$^+$.

Step II: Synthesis of (R)-6-methyl-3-(trifluoromethyl)-7,7a,8,9,10,11-hexahydropyrazino[1,2-a]pyrido[3,2-f][1,4]diazepan-5(6*H*)-one **(42-2)**

t-butyl (S)-6-methyl-5-oxo-3-(trifluoromethyl)-6,7,7a,8,10,11-hexahydropyrazino[1,2-a]pyrido[3,2-f][1,4]diazepan-9(*5H*)-carboxylate (240 mg, 0.60 mmol) was dissolved in ice-cold (0°C) hydrogen chloride-1,4-dioxane solution (4 M, 8 mL), slowly warmed to room temperature and reacted for 4 hrs with stirring. After complete reaction, the reaction solution was concentrated under reduced pressure. The crude product was diluted in water (20 mL), adjusted to pH 8-9 with sodium bicarbonate, and extracted with dichloromethane (20 mL x 3). The organic phase was dried over anhydrous sodium sulfate, and filtered. The filtrate was concentrated. The crude product was purified by column chromatography on silica gel (ethyl acetate :petroleum ether=50: 50) to obtain the target product as a yellow solid (100mg, 0 .33mmol, yield : 55%), LCMS: (ESI)m/z: 301[M+H]$^+$.

Step III: Synthesis of (S)-6-methyl-9-(3-((S)-2-((6-oxo-5-(trifluoromethyl)-1,6-dihydropyridazin-4-yl)amino)propoxy)propionyl)-3-(trifluoromethyl)-7,7a, 8,9,10,11-hexahydropyrazino[1,2-a]pyrido[3,2-f][1,4]diazepan-5(*6H*)-one **(Compound 42)**

(R)-6-methyl-3-(trifluoromethyl)-7,7a,8,9,10,11-hexahydropyrazino[1,2-a]pyrido[3,2-f][1,4]diazepan-5(*6H*)-one (100 mg, 0.33 mmol) and (S)-3-(2-((6-oxo-5-(trifluoromethyl)-1,6-dihydropyridazin-4-yl)amino)propoxy)propionic acid (102 mg, 0.33 mmol) were dissolved in dichloromethane (10.0 mL), and then diisopropylethyl amine (255 mg, 1.98 mmol) and a propylphosphonic anhydride solution (630 mg, 0.99 mmol, 50% solution in ethyl acetate) were added. The reaction mixture was stirred at room temperature for 1 hr. The reaction solution was poured into ice water (20 mL), and the aqueous phase was extracted with dichloromethane (20 mL x 3). The organic phases were combined, and dried over anhydrous sodium sulfate. Anhydrous sodium sulfate was removed by filtration, and the filtrate was rotary dried to obtain a crude product. The crude product was purified by preparative liquid-phase chromatography (column: XBridge Prep OBD C18, 30 x 150 mm 5 um, mobile phase A: 10 mol/L ammonium bicarbonate aqueous solution, mobile phase B: acetonitrile, flow rate: 60 mL/ min, elution gradient: mobile phase B from 10% to 46% over 8 min, detection wavelength: 220 nm, retention time: 8.03 min) to obtain the target product as a white solid (8.05mg, 0.014mmol, yield: 4.2%).

LCMS: MS(ESI)m/z: 592.15 [M+H]$^+$. $^1$H NMR (400 MHz, DMSO-$d_6$) δ(ppm): 8.66 (s, 1H), 8.04-8.03 (m, 1H), 7.91 (s, 1H), 6.27 (s, 1H), 4.44-4.35 (m, 1H), 4.23-4.14 (m, 2H), 4.03-3.90 (m, 1H), 3.72-3.64 (m, 3H), 3.67-3.53 (m, 2H), 3.50-3.45 (m, 2H), 3.25-3.15 (m, 1H), 3.07-3.03 (m, 3H), 3.04-2.87 (m, 1H), 2.80-2.67 (m, 1H), 2.66-2.54 (m, 2H), 1.16 (d, J = 8.0 Hz, 3H).

**Example 43:** Synthesis of 5-((((2S)-1-(3-(5-methyl-3-(trifluoromethyl))-6,7,7a, 8, 10, 11-hexahydropyrazino[1,2-d]pyrido[3,2- b] [1,4] diazepine -9(5H)-yl)-3-oxopropoxy)prop-2-yl)amino)-4-(trifluoromethyl)pyridazin-3(2H)-one **(Compound 43)**

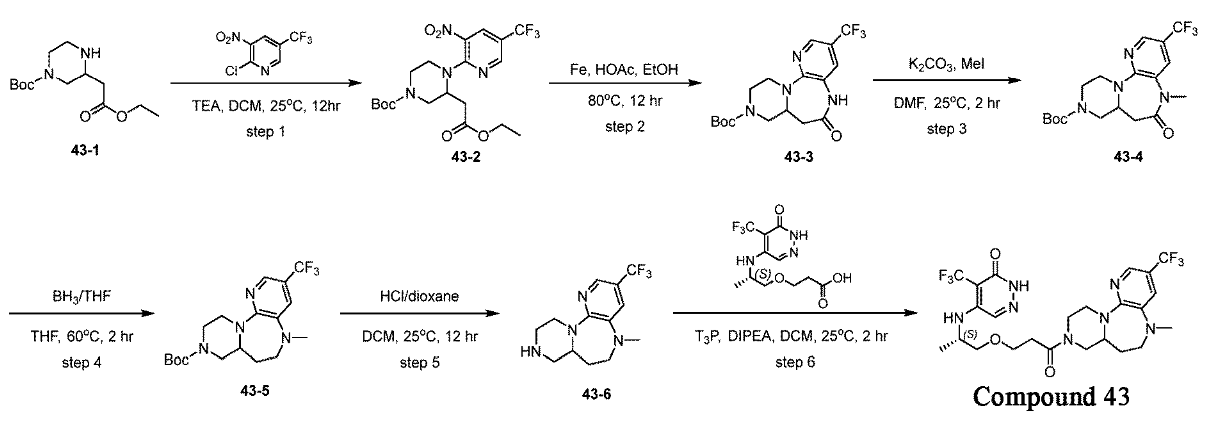


Step I: Synthesis of 3-(2-ethoxy-2-oxyethyl)-4-(3-nitro-5-(trifluoromethyl)pyridin-2-yl)piperazin-1-t-butyl carboxylate **(43-2)**

To a solution of 2-chloro-3-nitro-5-(trifluoromethyl)pyridine (3.50 g, 15.45 mmol) and triethyl amine (6.45 mL, 46.35 mmol) in dichloromethane (52 mL), t-butyl 3-(2-ethoxy-2-oxyethyl)piperazin-1-carboxylate was added (4.63 g,

16.99 mmol). The reaction system was stirred for 12 hrs under nitrogen atmosphere at 25°C. Then water (30 mL) was added, extracted three times with dichloromethane (30 mL), and dried over anhydrous sodium sulfate. The filtrate was rotary dried. The crude product was purified by column chromatography on silica gel (80 g silica gel, eluant: 0-50% ethyl acetate /petroleum ether), to obtain t-butyl 3-(2-ethoxy-2-oxyethyl)-4-(3-nitro-5-(trifluoromethyl)pyridin-2-yl)piperazin-1-carboxylate as a white solid (7 g, 15.1 mmol, 98% yield). LCMS (ESI): $[M-^tBu+H]^+$=407.1;

Step II: Synthesis of t-butyl 6-oxo-3-(trifluoromethyl)-6,7,7a, 8,10,11-hexahydropyrazino[1,2-d]pyrido[3,2-b][1,4] diazepine -9(5H)-carboxylate **(43-3)**

To a solution of t-butyl 3-(2-ethoxy-2-oxyethyl)-4-(3-nitro-5-(trifluoromethyl)pyridin-2-yl)piperazin-1-carboxylate (7 g, 15.1mmol ) and acetic acid (17.31 mL, 302.74 mmol) in ethanol (140 mL), reduced iron powder (8.45 g, 151.38 mmol) was added. The reaction system was stirred for 12 hrs under nitrogen atmosphere at 80°C. The reaction solution was filtered, and rotary dried to half of the volume. The reaction solution was added with water (200 mL), and extracted three times with dichloromethane (200 mL). The organic phase was washed with saturated sodium bicarbonate (200 mL) and saturated brine (200 mL). The organic phase was dried over anhydrous sodium sulfate, and the filtrate was rotary dried. t-butyl 6-oxo-3-(trifluoromethyl)-6,7,7a, 8,10,11-hexahydropyrazino[1,2-d]pyrido[3,2-b][1,4] diazepine -9(5H)-carboxylate as a white solid (5.90 g, 15.1 mmol, 76% yield) was obtained. LCMS (ESI): $[M-^tBu+H]^+$=331.1;

Step III: Synthesis of t-butyl 5-methyl-6-oxo-3-(trifluoromethyl)-6,7,7a, 8,10,11-hexahydropyrazino[1,2-d]pyrido[3,2-b] [1,4] diazepine -9(5H)-carboxylate **(43-4)**

To a solution of t-butyl 6-oxo-3-(trifluoromethyl)-6,7,7a, 8,10,11-hexahydropyrazino[1,2-d]pyrido[3,2-b] [1,4] diazepine -9(5H)-carboxylate (600 mg, 1.55 mmol) in DMF (12 mL), potassium carbonate (428.6 mg, 3.11 mmol) was added at 0°C. Then, iodomethane (440.8 mg, 3.11 mmol) was added dropwise at 0°C. The reaction system was stirred for 2 hrs under nitrogen atmosphere at 25°C. The reaction mixture was filtered, added with water (36 mL), and extracted three times with ethyl acetate (40 mL). The organic phases were combined, washed with brine (30 mL), and dried over anhydrous sodium sulfate. The filtrate was rotary dried. t-butyl 5-methyl-6-oxo-3-(trifluoromethyl)-6,7,7a, 8,10,11-hexahydropyrazino[1,2-d]pyrido[3,2-b] [1,4] diazepine -9(5H)-carboxylate as a white solid (610 mg, 1.53 mmol, 98% yield) was obtained. LCMS (ESI): $[M-tBu+H]^+$=345.1;

Step IV: Synthesis of t-butyl 5-methyl-3-(trifluoromethyl)-6,7,7a, 8,10,11-hexahydropyrazino[1,2-d]pyrido[3,2-b] [1,4] diazepine -9(5H)-carboxylate **(43-5)**

To a solution of t-butyl 5-methyl-6-oxo-3-(trifluoromethyl)-6,7,7a, 8,10,11-hexahydropyrazino[1,2-d]pyrido[3,2-b] [1,4] diazepine -9(5H)-carboxylate (350 mg, 0.87 mmol) in tetrahydrofuran (10 mL), 1 M borane/tetrahydrofuran solution (1.75 mL, 1.75 mmol) was added dropwise at 0°C. The reaction mixture was stirred for 2 hrs under nitrogen atmosphere at 60°C. Methanol (6 mL) was then added dropwise at 0°C. The reaction solution was stirred for 2 hrs under nitrogen atmosphere at 25°C, and then rotary dried to obtain t-butyl 5-methyl-3-(trifluoromethyl)-6,7,7a, 8,10,11-hexahydropyrazino[1,2-d]pyrido[3,2-b] [1,4] diazepine -9(5H)-carboxylate as a white solid (340 mg, 0.87mmol, 99% yield). LCMS (ESI): $[M+H]^+$=387.3;

Step V: Synthesis of 5-methyl-3-(trifluoromethyl)-5,6,7,7a, 8,9,10,11-octahydropyrazino[1,2-d]pyrido[3,2-b] [1,4] diazepine (**43-6**)

To a solution of t-butyl 5-methyl-3-(trifluoromethyl)-6,7,7a, 8,10,11-hexahydropyrazino[1,2-d]pyrido[3,2-b] [1,4] diazepine -9(5H)-carboxylate (340 mg, 0.87 mmol) in dichloromethane (2 mL), 4M hydrochloric acid/dioxane solution (2.2 mL, 8.8 mmol) was added at 0°C. The reaction system was stirred for 12 hrs at 25°C, and rotary dried to obtain 5-methyl-3-(trifluoromethyl)-5,6,7,7a, 8,9,10,11-octahydropyrazino[1,2-d]pyrido[3,2-b] [1,4] diazepine as a yellow solid (340 mg, 0.87 mmol, 99% yield). LCMS (ESI): $[M+H]^+$=287.1;

Step VI: Synthesis of 5-((((2S)-1-(3-(5-methyl-3-(trifluoromethyl))-6,7,7a, 8,10,11-hexahydropyrazino[1,2-d]pyrido[3,2-b] [1,4] diazepine -9(SH)-yl)-3-oxopropoxy)prop-2-yl)amino)-4-(trifluoromethyl)pyridazin-3(2H)-one **(Compound 43)**

To a solution of (S)-3-(2-((6-oxo-5-(trifluoromethyl)-1,6-dihydropyridazin-4-yl)amino)propoxy)propionic acid (100 mg, 0.32 mmol), diisopropylethyl amine (320 uL, 1.94 mmol) and 5-methyl-3-(trifluoromethyl)-5,6,7,7a, 8,9,10, 11-octahydropyrazino[1,2-d]pyrido[3,2-b] [1,4] diazepine (139 mg, 0.49mmol) in dichloromethane (2 mL), 50% $T_3P$ (1029 mg, 1.62 mmol) was added, and the reaction system was stirred at 25°C for 2 hrs. The reaction solution was added with water (5 mL), and extracted three times with dichloromethane (5 mL). The organic phase was dried over anhydrous

sodium sulfate, filtered, and rotary dried. The residue was purified by preparative liquid chromatography (column: YMC-Actus Triart C18 150*30 mm*5 um; mobile phase: water (0.225% FA)-ACN; B%: 38%-58%.), to obtain the compound 5-((((2S)-1-(3-(5-methyl-3-(trifluoromethyl))-6,7,7a, 8,10,11-hexahydropyrazino[1,2-d]pyrido[3,2- b] [1,4] diazepine -9(SH)-yl)-3-oxopropoxy)prop-2-yl)amino)-4-(trifluoromethyl)pyridazin-3(2H)-one (50 mg, 0.08 mmol, 27% yield).

LCMS (ESI): $[M+H]^+$=578.3. $^1H$ NMR (400MHz, deuterated methanol-$d_4$) $\delta$ ppm 8.04 - 7.84 (m, 2H), 7.07 (d, *J*=2.3 Hz, 1H), 6.24 (br s, 1H), 4.26 - 4.01 (m, 2H), 3.95 - 3.69 (m, 5H), 3.67 - 3.41 (m, 5H), 3.22 - 3.11 (m, 1H), 2.86 (s, 3H), 2.78 - 2.60 (m, 2H), 2.05 - 1.93 (m, 1H), 1.82 - 1.69 (m, 1H), 1.31 - 1.23 (m, 3H).

**Example 44:** Synthesis of 4-bromo-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)pyridazin-3(2H)-one **(Compound 44)**

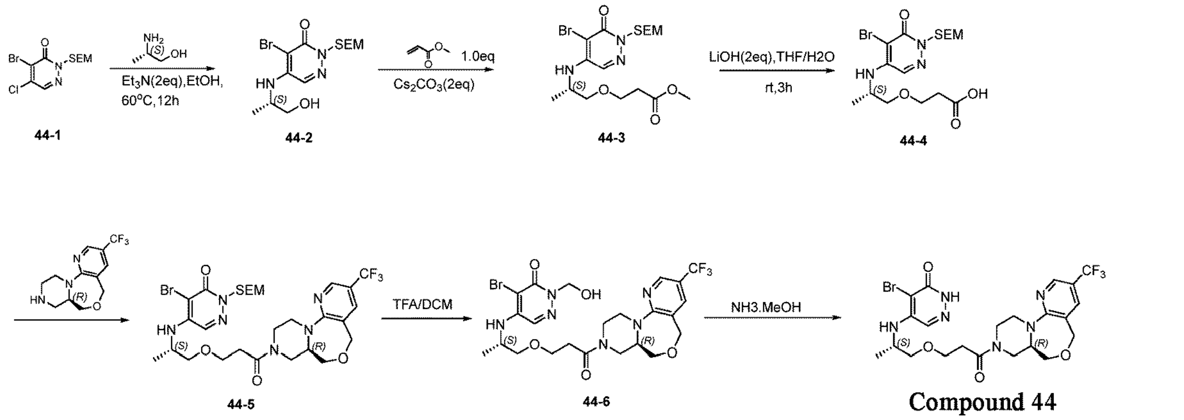


Step I: Synthesis of (S)-4-bromo-5-((1-hydroxypropyl-2-yl)amino)-2-((2-(trimethylsilyl)ethoxy)methyl)pyridazin-3(2H)-one **(44-2)**

bromo-5-chloro-2-(2-(trimethylsilyl)ethoxy)methyl)pyridazin-3(2H)-one (500.00 mg, 1.47 mmol), (S)-2-aminoprop-1-ol (110.25 mg, 1.47 mmol) and triethyl amine (294.39 mg, 2.94 mmol) were added to ethanol (5.00 ml). The mixture was stirred for 12 hrs at 60°C. Then, the mixture was concentrated under reduced pressure, then added to water (2 ml), and extracted with ethyl acetate (5 ml*2). The organic phase was washed with saturated brine, dried over anhydrous sodium sulfate, and filtered. The filtrate was concentrated, and the obtained crude product was purified by column chromatography on silica gel (petroleum ether:ethyl acetate =30: 70) to obtain (S)-4-bromo-5-((1-hydroxypropyl-2-yl)amino)-2-((2-(trimethylsilyl)ethoxy)methyl)pyridazin-3(2H)-one as a yellow oil (256.36 mg, 0.68 mmol, yield: 46%). LCMS (ESI) m/z: 378[M+H]+

Step II: Synthesis of methyl (S)-3-(2-((5-bromo-6-oxo-1-((2-(trimethylsilyl)ethoxy)methyl)-1, 6-dihydropyridazin-4-yl)amino)propoxy)propionate **(44-3)**

At room temperature, (S)-4-bromo-5-((1-hydroxypropyl-2-yl)amino)-2-(2-(trimethylsilyl)ethoxy)methyl)pyridazin-3(2H)-one (500.00 mg, 1.32 mmol), methyl acrylate (113.52 mg, 1.32 mmol) and cesium carbonate (866.95 mg, 2.64 mmol) were added to acetonitrile (10.00 ml), stirred for 2 hrs, then filtered and concentrated. The mixture was added with water (10 ml), and then extracted with ethyl acetate (10 ml * 2). The organic phase was washed with saturated brine, dried over anhydrous sodium sulfate, and filtered. The filtrate was concentrated. The obtained crude product was purified by column chromatography on silica gel (petroleum ether:ethyl acetate =80:20) to obtain methyl (S)-3-(2-((5-bromo-6-oxo-1-((2-(trimethylsilyl)ethoxy)methyl)-1, 6-dihydropyridazin-4-yl)amino)propoxy)propionate as a yellow oil (259.84 mg, 0.56 mmol, yield: 42%). LCMS (ESI) m/z: 464[M+H]+

Step III: Synthesis of (S)-3-(2-((5-bromo-6-oxo-1-((2-(trimethylsilyl)ethoxy)methyl)-1, 6-dihydropyridazin-4-yl)amino)propoxy)propionic acid **(44-4)**

To a mixed solution of tetrahydrofuran (1.5 ml) and water (1.5 ml), methyl (S)-3-(2-((5-bromo-6-oxo-1-((2-(trimethylsilyl)ethoxy)methyl)-1, 6-dihydropyridazin-4-yl)amino)propoxy)propionate (0.30 g, 0.65 mmol) and lithium hydroxide monohydrate (0.05 g, 1.3 mmol) were added. The mixture was stirred at room temperature for 3 hrs. After complete

reaction, the reaction solution was adjusted to pH 6 with formic acid, and then extracted with ethyl acetate (5 ml * 2). The organic phase was washed with saturated brine, dried over anhydrous sodium sulfate, and filtered. The filtrate was concentrated, to obtain crude (S)-3-(2-((5-bromo-6-oxo-1-((2-(trimethylsilyl)ethoxy)methyl)-1, 6-dihydropyridazin-4-yl)amino)propoxy)propionic acid as a yellow oil (0.15 g, 0.33 mmol, yield: 50%). LCMS (ESI) m/z: 450[M+H]+

Step IV: Synthesis of 4-bromo-5-((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-2-(2-(trimethylsilyl)ethoxy)methyl)pyridazin-3(2H)-one **(44-5)**

(S)-3-(2-((5-bromo-6-oxo-1-((2-(trimethylsilyl)ethoxy)methyl)-1, 6-dihydropyridazin-4-yl)amino)propoxy)propionic acid (0.30 g, 0.67 mmol), (R)-3-(trifluoromethyl)-7, 7a, 8, 9, 10, 11-hexahydro-5H-pyrazino[2, 1-c]pyrido[2, 3-e][1, 4]oxazepane (0.2 g, 0.44 mmol ), propylphosphonic anhydride (0.84g, 1.33mmol, 50 wt% solution in ethyl acetate) and diisopropylethyl amine (0.26 g, 2.00 mmol) were dissolved in dichloromethane (5.00 ml), stirred at room temperature for 5 hrs, then poured into water (10 ml), and extracted with dichloromethane (10 ml*2). The organic phase was washed with saturated brine, dried over anhydrous sodium sulfate, and filtered. The filtrate was concentrated. The obtained crude product was purified by column chromatography on silica gel (petroleum ether:ethyl acetate =30: 70) to obtain 4-bromo-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-2-((2-(trimethylsilyl)ethoxy)methyl)pyridazin-3(2H)-one as a yellow solid (0.20g, 0.28mmol, yield: 64%). LCMS (ESI) m/z: 705[M+H]+

Step V: Synthesis of 4-bromo-2-(hydroxymethyl)-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepanyl-9(7H)-yl)propoxy)prop-2-yl)amino)pyridazin-3(2H)-one **(44-6)**

bromo-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-2-((2-(trimethylsilyl)ethoxy)methyl)pyridazin-3(2H)-one (0.30 g, 0.28 mmol) was added to a mixed solution (5 ml, 1:1) of trifluoroacetic acid and dichloromethane, stirred at room temperature for 2 hrs, and then concentrated under reduced pressure to obtain crude 4-bromo-2-(hydroxymethyl)-5-(((S)-1-(3-oxo-3-((R)-3-(trifluoromethyl)-7a, 8, 10, 11-tetrahydro-5H-pyrazino[2, 1-c]pyrido[2, 3-e][1, 4]Oxazepanyl-9(7H)-yl)propoxy)prop-2-yl)amino)pyridazin-3(2H)-one as a yellow oil (120 mg,0.2 mmol, yield: 71%). LCMS (ESI) m/z: 605[M+H]+

Step VI: Synthesis of 4-bromo-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)pyridazin-3(2H)-one **(Compound 44)**

bromo-2-(hydroxymethyl)-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)pyridazin-3(2H)-one (50.00 mg, 0.08 mmol) was added to a 7 mol/l aminomethanol solution, and reacted for two hours at room temperature. After complete reaction, the reaction solution was concentrated under reduced pressure. The resulting crude product was purified by preparative chromatography (chromatographic column: Xsele CSH C18 OBD column 30 * 150 mm 5 μm; mobile phase A: 0.05% formic acid aqueous solution, mobile phase B: acetonitrile; flow rate: 60ml/ min; gradient: 3% B to 3% B over 2 min, 3% B to 33% B over 8 min; wavelength: 220 nm; retention time: 6.63 min) to obtain 4-bromo-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)pyridazin-3(2H)-one as a pale yellow semi-solid (2.05 mg, 0.0035 mmol, yield 4%).

LCMS(ESI)m/z: 574.95 [M+H]+, $^1$H NMR (400 MHz, DMSO-$d_6$) δ ppm 12.55 (s, 1H), 8.89 (s, 1H), 8.49 (s, 1H), 7.79 (s, 1H), 6.58-6.06 (m, 1H), 5.78 (t, *J* = 8.6 Hz, 1H), 4.90-4.82 (m, 1H), 4.81-4.71 (m, 1H), 4.71-4.61 (m, 2H), 4.47 - 4.38(m, 2H), 4.35-4.26 (m, 2H), 4.11 - 4.03 (m, 2H), 3.72 - 3.66 (m, 2H), 3.51-3.49 (m, 2H), 2.88-2.78 (m, 1H), 2.72-2.62(m, 2H), 1.31-1.05 (m, 3H).

**Example 45:** Synthesis of 5-(((S)-1-(3-oxo-3-((S)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one **(Compound 45)** and 5-(((S)-1-(3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one **(Compound 46)**

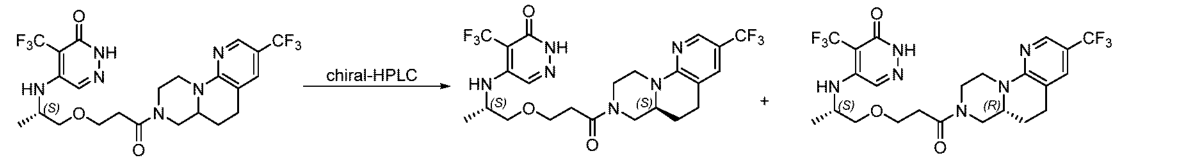


Compound 45 Compound 46

5-(((2S)-1-(3-oxo-3-(3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one (**Compound 32,** 50.00 mg, 0.091 mmol) was resolved by chiral preparative chromatography (chromatographic column: CHIRAL ART Cellulose-SB, 2*25 cm, 5 μm, mobile phase A: methyl t-butyl ether (containing 0.1% diethyl amine), mobile phase B: methanol, flow rate: 20 ml/ min, elution gradient: mobile phase B from 8% to 8% over 8 min, detection wavelength: 220 nm) to obtain the first peak 5-(((S)-1-(3-oxo-3-((S)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one as a white solid **(Compound 45)** (10.08 mg, 0.018 mmol, yield 20%), and the second peak 5-(((S)-1-(3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one as a white solid **(Compound 46)** (11.52 mg, 0.021 mmol, yield 23%).

First peak **(Compound 45):** 5-(((S)-1-(3-oxo-3-((S)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one. LCMS(ESI)m/z: 549.15[M+H]$^+$ . HNMR (400 MHz, DMSO-$d_6$): δ ppm 12.45(s,1H), 8.23(s,1H), 7.90(s,1H), 7.52(s,1H), 6.34-6.21(m,1H), 4.75-4.63(m,1H), 4.47-4.35(m,1H), 4.21-4.11(m,1H), 4.05-3.95(m,1H), 3.77-3.61(m,2H), 3.54-3.44(m,2H), 3.39-3.35(m,1H), 3.23-3.04(m,1H), 2.96-2.84(m,1H), 2.83-2.69(m,3H), 2.64-2.58(m,2H), 2.12-1.99(m,1H), 1.65-1.54(m,1H), 1.21-1.10(m,3H). Chiral HPLC: chromatographic column CHIRAL ART Cellulose-SB 4.6*100 mm, 3 um; mobile phase A: methyl t-butyl ether (containing 0.1% diethyl amine), mobile phase B: methanol, mobile phase A: mobile phase B=90:10; flow rate: 1.0ml/ min; temperature: 25°C; retention time: 3.16 min

Second peak **(Compound 46):** 5-(((S)-1-(3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one. LCMS (ESI)m/z: 549.15[M+H]$^+$. HNMR (400 MHz, DMSO-$d_6$): δ ppm 12.46(s,1H), 8.23(s,1H), 7.90(s,1H), 7.52(s,1H), 6.32-6.20(m,1H), 4.78-4.62(m,1H), 4.48-4.33(m,1H), 4.24-4.08(m,1H), 4.05-3.89(m,1H), 3.83-3.57(m,2H), 3.54-3.44(m,2H), 3.43-3.36(m,1H), 3.25-3.00(m,1H), 2.96-2.84(m,1H), 2.82-2.69(m,3H), 2.64-2.58(m,2H), 2.11-1.99(m,1H), 1.75-1.43(m,1H), 1.22-1.07(m,3H). Chiral HPLC: chromatographic column CHIRAL ART Cellulose-SB 4.6*100 mm, 3 um; mobile phase A: methyl t-butyl ether (containing 0.1% diethyl amine), mobile phase B: methanol, mobile phase A: mobile phase B=90:10; flow rate: 1.0 ml/ min; temperature: 25°C; retention time: 3.61 min.

**Example 46:** Synthesis of 5-(((S)-1-(3-oxo-3-((S)-3-(trifluoromethyl)-6, 7, 7a, 8, 10, 11-hexahydro-9H-bipyrazino[2, 3-b: 1 ', 2'-d][1, 4]oxazepane-9-yl)propoxy)prop-2-yl)amino)-4-(trifluoromethyl)pyridazin-3(2H)-one **(Compound 47)**

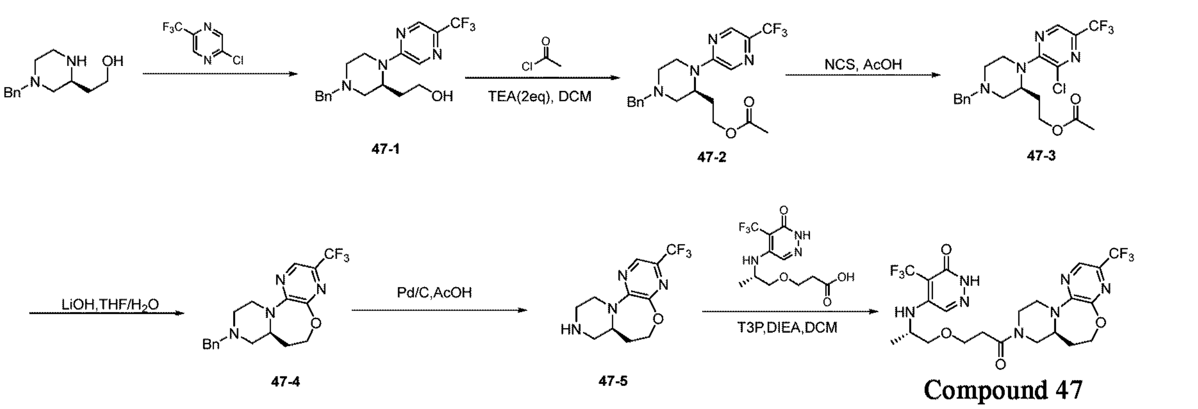


47-1 47-2 47-3 47-4 47-5 Compound 47

Step I: Synthesis of (S)-2-(4-benzyl-1-(5-(trifluoromethyl)pyrazin-2-yl)piperazin-2-yl)ethyl-1-ol (**47-1**)

(S)-2-(4-benzylpiperazin-2-yl)ethyl-1-ol (2.00 g, 9.08 mmol) was added to N,N-dimethylformamide (40.00 ml). Then 2-chloro-5-(trifluoromethyl)pyrazine (1.98 g, 10.89 mmol) was added, and N,N-diisopropylethyl amine (2.34 g,

18.16 mmol) was slowly added. The mixture was stirred at 80°C for 4 hrs. After the reaction was completed, the system was cooled to room temperature. The reaction solution was poured into water (20 ml), and then extracted with ethyl acetate (20 ml*3). The organic phases were combined, washed with saturated brine, dried over anhydrous sodium sulfate, and filtered. The filtrate was concentrated. The crude product was purified by column chromatography (ethyl acetate :petroleum ether=30:70) to obtain (S)-2-(4-benzyl-1-(5-(trifluoromethyl)pyrazin-2-yl)piperazin-2-yl)ethyl-1-ol as a yellow oil (1.50 g, 4.09 mmol, yield 45%). LCMS (ESI) m/z: 367$[M+H]^+$

Step I: Synthesis of (S)-2-(4-benzyl-1-(5-(trifluoromethyl)pyrazin-2-yl)piperazin-2-yl)ethyl acetate (**47-2**)

(S)-2-(4-benzyl-1-(5-(trifluoromethyl)pyrazin-2-yl)piperazin-2-yl)ethyl-1-ol (600.00 mg, 1.64 mmol) was dissolved in dichloromethane (60.00 ml), and then acetyl chloride (192.00 mg, 2.46 mmol) and triethyl amine (336.00 mg, 3.33 mmol) were added. The mixture was stirred at room temperature for 2 hrs. The reaction solution was added with water (100 ml), and then extracted with dichloromethane (50 ml*2). The organic layer was collected, washed with saturated brine, dried over anhydrous sodium sulfate, and filtered. The filtrate was concentrated, and the resulting crude product was purified by column chromatography on silica gel (petroleum ether: ethyl acetate =80:20) to obtain (S)-2-(4-benzyl-1-(5-(trifluoromethyl)pyrazin-2-yl)piperazin-2-yl)ethyl acetate (550.00mg, 1.35 mmol, yield:79%), as a yellow oil. LCMS (ESI) m/z: 409[M+H]+

Step II: Synthesis of (S)-2-(4-benzyl-1-(3-chloro-5-(trifluoromethyl)pyrazin-2-yl)piperazin-2-yl)ethyl acetate (**47-3**)

(S)-2-(4-benzyl-1-(5-(trifluoromethyl)pyrazin-2-yl)piperazin-2-yl)ethyl acetate (550.00 mg, 1.35 mmol) was added to acetic acid (55.00 ml), and then N-chlorosuccinimide (537.90 mg, 4.04 mmol) was added. The mixture was stirred for 3 hrs at 80°C. The reaction solution was cooled, rotary dried to remove the solvent, then diluted in water (20ml), and extracted with ethyl acetate (10 ml * 3). The organic layer was collected, washed with saturated brine, dried over anhydrous sodium sulfate, and filtered. The filtrate was concentrated to obtain a crude product, and the resulting crude product was purified by column chromatography on silica gel (petroleum ether: ethyl acetate =60:40) to obtain (S)-2-(4-benzyl-1-(3-chloro-5-(trifluoromethyl)pyrazin-2-yl)piperazin-2-yl)ethyl acetate as a colorless oil (110.00 mg, 0.25 mmol, yield 18%). LCMS (ESI) m/z: 443[M+H]+

Step III: Synthesis of (S)-9-benzyl-3-(trifluoromethyl)-7, 7a, 8, 9, 10, 11-hexahydro-6H-bipyrazino[2, 3-b:1 ', 2'-d][1, 4]oxazepane(**47-4**)

(S)-2-(4-benzyl-1-(3-chloro-5-(trifluoromethyl)pyrazin-2-yl)piperazin-2-yl)ethyl acetate (110.00 mg, 0.25 mmol) was added to tetrahydrofuran (5.00 ml) and water (5.00 ml), and then lithium hydroxide monohydrate (104.90 mg, 2.5 mmol) was added to the mixed solution and reacted at room temperature for 3 hrs. After the reaction was completed, the reaction solution was added with water (10ml), and then extracted with ethyl acetate (10ml*3). The organic layer was collected, dried over anhydrous sodium sulfate, and filtered. The filtrate was concentrated, and the resulting crude product was purified by column chromatography on silica gel (petroleum ether: ethyl acetate =80:20) to obtain (S)-9-benzyl-3-(trifluoromethyl)-7, 7a, 8, 9, 10, 11-hexahydro-6H-bipyrazino[2, 3-b:1 ', 2'-d][1, 4]oxazepane as a yellow solid (90.00 mg, 0.24 mmol, yield: 96%). LCMS (ESI) m/z: 365[M+H]+

Step IV: Synthesis of (S)-3-(trifluoromethyl)-7, 7a, 8, 9, 10, 11-hexahydro-6H-bipyrazino[2, 3-b:1', 2'-d][1, 4]oxazepane (**47-5**)

(S)-9-benzyl-3-(trifluoromethyl)-7, 7a, 8, 9, 10, 11-hexahydro-6H-bipyrazino[2, 3-b:1 ', 2'-d][1, 4]oxazepane (85.00 mg, 0.23 mmol) were dissolved in glacial acetic acid (10.00 ml), and then aqueous palladium on carbon (8.50 mg, 10%) was added to the mixed solution. The mixture was stirred for 2 hrs under nitrogen atmosphere at room temperature. The reaction solution was filtered through a Buchner funnel. The filter cake was washed with methanol (10 ml * 2), and the filtrate was concentrated, to obtain crude (S)-3-(trifluoromethyl)-7, 7a, 8, 9, 10, 11-hexahydro-6H-bipyrazino[2, 3-b: 1', 2'-d][1, 4]oxazepane as a yellow oil (30.00 mg). The crude product was directly used in the next reaction without purification. LCMS (ESI) m/z: 275[M+H]+

Step V: Synthesis of 5-(((S)-1-(3-oxo-3-((S)-3-(trifluoromethyl)-6, 7, 7a, 8, 10, 11-hexahydro-9H-bipyrazino[2, 3-b:1 ', 2'-d][1, 4]oxazepane-9-yl)propoxy)prop-2-yl)amino)-4-(trifluoromethyl)pyridazin-3(2H)-one (**Compound 47**)

(S)-3-(trifluoromethyl)-7, 7a, 8, 9, 10, 11-hexahydro-6H-bipyrazino[2, 3-b: 1', 2'-d][1, 4]oxazepane (28.00 mg, 0.10 mmol) was added to dichloromethane (5.00 ml), and then (S)-3-(2-((6-oxo-5-(trifluoromethyl)-1, 6-dihydropyridazin-4-yl)amino)propoxy)propionic acid (31.50 mg, 0.10 mmol), propylphosphonic anhydride (194.61 mg, 0.31 mmol) and

N,N-diisopropylethyl amine (65.79 mg, 0.51 mmol) were added. The mixture was stirred at room temperature for 2 hrs. Then the reaction solution was added with water (10 ml), and extracted with dichloromethane (5 ml x 3). The organic layer was collected, washed with saturated brine, dried over anhydrous sodium sulfate, and filtered. The filtrate was concentrated, and the resulting crude product was purified by preparative chromatography (chromatographic column: CSH C18 OBD column 30 x 150 mm 5 μm, n; mobile phase A: 10 mmol/l ammonium bicarbonate aqueous solution, mobile phase B: acetonitrile; flow rate: 60 ml/ min; gradient: 10% B to 56% B over 8 min; wavelength: 220 nm; retention time: 7.43 min) to obtain 5-(((S)-1-(3-oxo-3-((S)-3-(trifluoromethyl)-6, 7, 7a, 8, 10, 11-hexahydro-9H-bipyrazino[2, 3-b: 1 ', 2'-d][1, 4]oxazepane-9-yl)propoxy)prop-2-yl)amino)-4-(trifluoromethyl)pyridazin-3(2H)-one as a white solid (4.41 mg, 0.01 mmol, yield: 8%).

LCMS (ESI) m/z: 566.00 [M+H]+. HNMR (400 MHz, DMSO-d6): δ ppm 12.45 (s, 1H), 8.22-8.09(m, 1H), 7.92-7.79(m, 1H), 6.32-6.15 (m, 1H), 4.57-4.31(m, 2H), 4.28-4.00(m, 2H), 3.93-3.84 (m, 1H),3.81-3.55(m, 6H),3.51-3.44 (m, 2H), 3.39-3.37 (m, 1H), 2.58-2.53 (m, 2H), 2.29-2.09 (m, 1H),1.93-1.82(m,1H), 1.27-0.95(m, 3H).

**Example 47:** Synthesis of (R)-5-methyl-8-(3-((S)-2-((6-oxo-5-(trifluoromethyl)-1, 6-dihydropyridazin-4-yl)amino)propoxy)propionyl)-3-(trifluoromethyl)-7, 8, 9, 10-tetrahydro-5H-bipyrazino[1, 2-a:2 ', 3 '-e]pyrazin-6(6aH)-one **(Compound 48)**

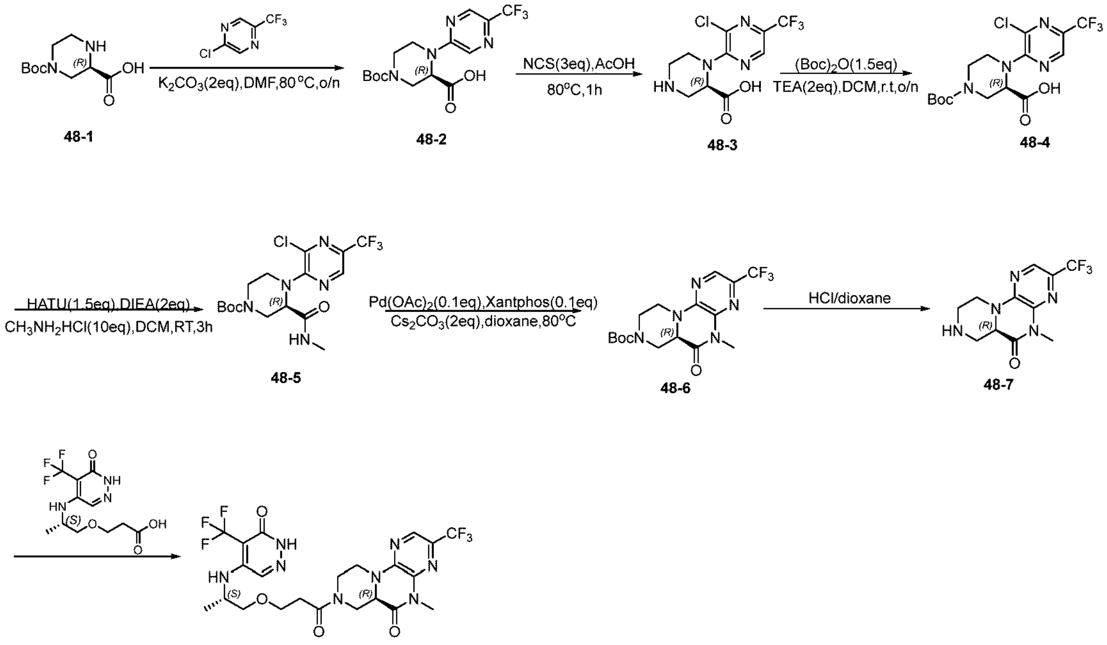


Compound 48

Step I: Synthesis of (R)-4-(t-butoxycarbonyl)-1-(5-(trifluoromethyl)pyrazin-2-yl)piperazin-2-carboxylic acid

(R)-4-(t-butoxycarbonyl)piperazin-2-carboxylic acid (2 g, 8.70 mmol) and 2-chloro-5-(trifluoromethyl)pyrazine (1.57 g, 8.70 mmol) were dissolved in N,N-dimethylformamide (20 ml), and then potassium carbonate (2.40 g, 17.40 mmol) was added, and reacted overnight at 80°C. After complete reaction, the reaction solution was cooled to room temperature, poured into water (50 ml), adjusted to pH 6 with formic acid, and extracted with ethyl acetate (20 ml x 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (dichloromethane : methanol=70: 30) to obtain (R)-4-(t-butoxycarbonyl)-1-(5-(trifluoromethyl)pyrazin-2-yl)piperazin-2-carboxylic acid as a yellow oil (980 mg, 2.61 mmol, yield 30%). LCMS (ESI): 377 [M+H]+

Step II: Synthesis of (R)-1-(3-chloro-5-(trifluoromethyl)pyrazin-2-yl)piperazin-2-carboxylic acid

(R)-4-(t-butoxycarbonyl)-1-(5-(trifluoromethyl)pyrazin-2-yl)piperazin-2-carboxylic acid (980 mg, 2.61 mmol) was dissolved in acetic acid (10 ml), and then N-chlorosuccinimide (1.04 mg, 7.83 mmol) was added, and reacted at 80°C for 1 hr. After complete reaction, the reaction solution was cooled to room temperature, and poured into water (30

ml). The aqueous phase was then extracted with ethyl acetate (20 ml x 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (dichloromethane : methanol=70: 30) to obtain (R)-1-(3-chloro-5-(trifluoromethyl)pyrazin-2-yl)piperazin-2-carboxylic acid as a yellow oil (428 mg, 1.38 mmol, yield 52%). LCMS (ESI): 311 [M+H]+

Step III: Synthesis of (R)-4-(t-butoxycarbonyl)-1-(3-chloro-5-(trifluoromethyl)pyrazin-2-yl)piperazin-2-carboxylic acid

(R)-1-(3-chloro-5-(trifluoromethyl)pyrazin-2-yl)piperazin-2-carboxylic acid (428 mg, 1.38 mmol) and triethyl amine (279 mg, 2.76 mmol) were dissolved in dichloromethane (5 ml), and then di-t-butyl dicarbonate (451 mg, 2.07 mmol) was added and reacted overnight at room temperature. After the reaction was completed, the reaction solution was poured into water (20 ml), adjusted to pH 6 with formic acid, and extracted with ethyl acetate (20 ml x 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (dichloromethane : methanol=80: 20) to obtain (R)-4-(t-butoxycarbonyl)-1-(3-chloro-5-(trifluoromethyl)pyrazin-2-yl)piperazin-2-carboxylic acid as a yellow oil (340 mg, 0.83 mmol, yield 60%). LCMS (ESI): 411 [M+H]+

Step IV: Synthesis of t-butyl (R)-4-(3-chloro-5-(trifluoromethyl)pyrazin-2-yl)-3-(methylaminoformyl)piperazin-1 -carboxylate

(R)-4-(t-butoxycarbonyl)-1-(3-chloro-5-(trifluoromethyl)pyrazin-2-yl)piperazin-2-carboxylic acid (340 mg, 0.83 mmol), methylamine hydrochloride (556 mg, 8.30 mmol), and N,N,N',N'-tetramethyl-O-(7-azabenzotriazole -1-yl)uronium hexafluorophosphate (475 mg, 1.25 mmol) were dissolved in dichloromethane (5 ml), and then diisopropylethyl amine (214 mg, 1.66 mmol) was added, and reacted for 3 hrs at room temperature. After the reaction was completed, the reaction solution was poured into water (20 ml), and the aqueous phase was extracted with ethyl acetate (20 ml x 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate :petroleum ether=50: 50) to obtain t-butyl (R)-4-(3-chloro-5-(trifluoromethyl)pyrazin-2-yl)-3-(methylaminoformyl)piperazin-1-carboxylate as a yellow oil (210 mg, 0.49 mmol, yield 59%). LCMS (ESI): 424 [M+H]+

Step V: Synthesis of t-butyl (R)-5-methyl-6-oxo-3-(trifluoromethyl)-5, 6, 6a, 7, 9, 10-hexahydro-8H-bipyrazino[1, 2-a:2 ', 3 '-e]pyrazin-8-carboxylate

T-butyl (R)-4-(3-chloro-5-(trifluoromethyl)pyrazin-2-yl)-3-(methylaminoformyl)piperazin-1-carboxylate (210 mg, 0.49 mmol), cesium carbonate (319 mg, 0.98 mmol), and 4,5-bis(diphenylphosphine)-9,9-dimethylxanthene (28 mg, 0.049 mmol) were dissolved in 1,4-dioxane (5 ml), and then palladium acetate (11 mg, 0.049 mmol) was added. The system was purged three time with nitrogen, and reacted for 2 hrs at 80°C. After complete reaction, the reaction solution was cooled to room temperature, poured into water (20 ml), and the aqueous phase was extracted with ethyl acetate (20 ml x 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate :petroleum ether=60: 40) to obtain t-butyl (R)-5-methyl-6-oxo-3-(trifluoromethyl)-5, 6, 6a, 7, 9, 10-hexahydro-8H-bipyrazino[1, 2-a:2 ', 3 '-e]pyrazin-8-carboxylate as a yellow oil (120 mg, 0.31 mmol, yield 63%). LCMS (ESI): 388 [M+H]+

Step VI: Synthesis of (R)-5-methyl-3-(trifluoromethyl)-7, 8, 9, 10-tetrahydro-5H-bipyrazino[1, 2-a:2 ', 3 '-e]pyrazin-6(6aH)-one

T-butyl (R)-5-methyl-6-oxo-3-(trifluoromethyl)-5, 6, 6a, 7, 9, 10-hexahydro-8H-bipyrazino[1, 2-a:2 ', 3 '-e]pyrazin-8-carboxylate (120 mg, 0.31 mmol) was dissolved in ice-cold (0°C) hydrogen chloride/1, 4-dioxane solution (4 mol/l, 8 ml), slowly warmed to room temperature and reacted for 4 hrs with stirring. After complete reaction, the reaction solution was concentrated under reduced pressure. The crude product was diluted in water (20 ml), adjusted to pH 8-9 with sodium bicarbonate, and then extracted with dichloromethane (20 mL x 3). The organic phases were combined, washed with saturated brine, dried over anhydrous sodium sulfate, and filtered. The filtrate was concentrated. The crude product was purified by column chromatography on silica gel(ethyl acetate :petroleum ether=70: 30) to obtain (R)-5-methyl-3-(trifluoromethyl)-7, 8, 9, 10-tetrahydro-5H-bipyrazino[1, 2-a:2 ', 3 '-e]pyrazin-6(6aH)-one as a yellow solid (80 mg, 0 .27 mmol, yield 87%). LCMS (ESI): 288 [M+H]+

Step VII: Synthesis of (R)-5-methyl-8-(3-((S)-2-((6-oxo-5-(trifluoromethyl)-1, 6-dihydropyridazin-4-yl)amino)propoxy)propionyl)-3-(trifluoromethyl)-7, 8, 9, 10-tetrahydro-5H-bipyrazino[1, 2-a:2 ', 3 '-e]pyrazin-6(6aH)-one

(R)-5-methyl-3-(trifluoromethyl)-7, 8, 9, 10-tetrahydro-5H-bipyrazino[1, 2-a:2 ', 3 '-e]pyrazin-6(6aH)-one (80 mg, 0 .27 mmol) and (S)-3-(2-((6-oxo-5-(trifluoromethyl)-1, 6-dihydropyridazin-4-yl)amino)propoxy)propionic acid (83.43 mg, 0.27 mmol) were dissolved in dichloromethane (2 ml), and then diisopropylethyl amine (209 mg, 1.62 mmol) and propylphosphonic anhydride solution (515 mg, 0.81 mmol, 50% solution in ethyl acetate) were added. The reaction mixture was stirred at room temperature for 1 hr. After the reaction was completed, the reaction solution was poured into ice water (10 ml), and the aqueous phase was extracted with dichloromethane (10 ml x 3). The organic phases were combined, washed with saturated brine, dried over anhydrous sodium sulfate, and filtered. The filtrate was concentrated, and the crude product was purified by preparative liquid-phase chromatography (chromatographic column: XBridge Prep OBD C18, 30 x 150 mm 5 um, mobile phase A: 10 mmol/l ammonium bicarbonate aqueous solution, mobile phase B: acetonitrile, flow rate: 60 ml/ min, elution gradient: mobile phase B from 25% to 63% in 8 min, detection wavelength: 220 nm, retention time: 7.45 min) to obtain (R)-5-methyl-8-(3-((S)-2-((6-oxo-5-(trifluoromethyl)-1, 6-dihydropyridazin-4-yl)amino)propoxy)propionyl)-3-(trifluoromethyl)-7, 8, 9, 10-tetrahydro-5H-bipyrazino[1, 2-a:2 ', 3 '-e]pyrazin-6(6aH)-one as a white solid (27.13 mg, 0.046 mmol, yield 17%).

LCMS (ESI) m/z: 579.10 $[M+H]^+$. HNMR (400 MHz, DMSO-d6): δ ppm 12.10 (brs, 1H), 8.19 (s, 1H), 7.91 (s, 1H), 6.32-6.22 (m, 1H), 4.86-4.42 (m, 2H), 4.46-3.94 (m, 3H), 3.76-3.62 (m, 2H), 3.53-3.43 (m, 2H), 3.32-3.29 (m, 3H), 3.18-2.80 (m, 2H), 2.79-2.58 (m, 3H), 1.15 (d, J = 6.4 Hz, 3H).

**Example 48:** Synthesis of (S)-6-methyl-3-(3-(S)-2-(6-oxo-5-(trifluoromethyl)-1, 6-dihydropyridazin-4-yl)amino)propoxy)propionyl)-8-(trifluoromethyl)-2, 3, 4, 4a-tetrahydro-1H-pyrazino[1, 2-a]quinoxalin-5(6H)-one (**Compound 49**)

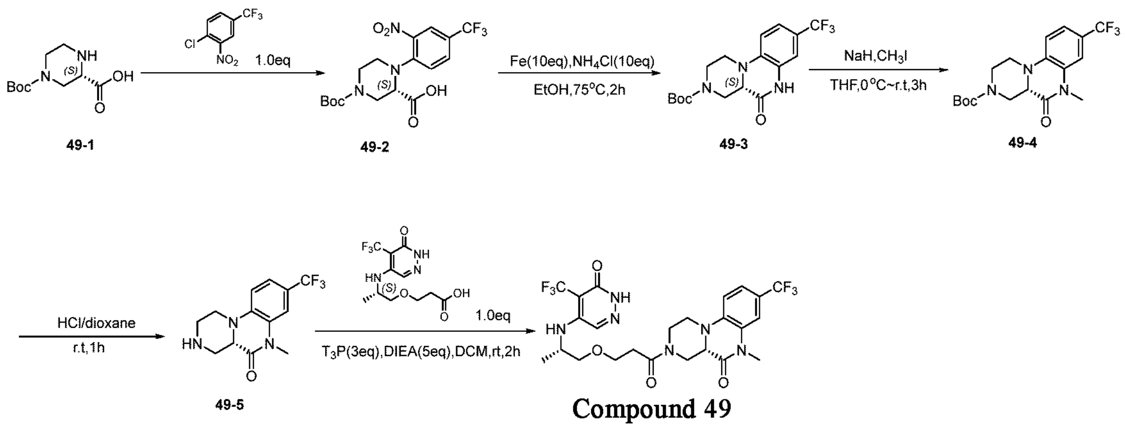

49-1
49-2
49-3
49-4
49-5
Compound 49

Step I: Synthesis of (S)-4-(t-butoxycarbonyl)-1-(2-nitro-4-(trifluoromethyl)phenyl)piperazin-2-carboxylic acid

(S)-4-(t-butoxycarbonyl)piperazin-2-carboxylic acid (2.00 g, 8.69 mmol) was dissolved in N,N-dimethylformamide (40.00 ml), and then 1-chloro-2-nitro-4-(trifluoromethyl)benzene (1.94 g, 8.62 mmol) and potassium carbonate (2.39 g, 17.32 mmol) were added. The mixture was stirred for 2 hrs at 80°C. The reaction solution was added with water (80 ml), adjusted to pH 6 with formic acid, and then extracted with ethyl acetate (40 ml*3). The organic layer was collected, washed with saturated brine, dried over anhydrous sodium sulfate, and filtered. The filtrate was concentrated, and the resulting crude product was purified by column chromatography on silica gel (dichloromethane : methanol=80:20) to obtain (S)-4-(t-butoxycarbonyl)-1-(2-nitro-4-(trifluoromethyl)phenyl)piperazin-2-carboxylic acid as a yellow solid (1.80 g, 4.29 mmol, yield:49%). LCMS(ESI)m/z: 420[M+H]+

Step II: Synthesis of t-butyl (S)-5-oxo-8-(trifluoromethyl)-1, 2, 4, 4a, 5, 6-hexahydro-3H-pyrazino[1, 2-a]quinoxalin-3-carboxylate

(S)-4-(t-butoxycarbonyl)-1-(2-nitro-4-(trifluoromethyl)phenyl)piperazin-2-carboxylic acid (1.5 g, 3.57 mmol) was added to ethanol (30.00 ml), and then iron powder (1.99 g, 35.53 mmol) and ammonium chloride (1.91 g, 36.03 mmol) were added. The mixture was stirred for 2 hrs at 75°C. After complete reaction, the reaction solution was cooled to room temperature, and filtered through diatomaceous earth. The filter cake was washed with ethanol (20 ml*2). The filtrate

was concentrated under reduced pressure, diluted in water (50 ml), and then extracted with ethyl acetate (30 ml*3). The organic layer was collected, washed with saturated brine, dried over anhydrous sodium sulfate, and filtered. The filtrate was concentrated to obtain a crude product, and the resulting crude product was purified by column chromatography on silica gel (petroleum ether: ethyl acetate =25:75) to obtain t-butyl (S)-5-oxo-8-(trifluoromethyl)-1, 2, 4, 4a, 5, 6-hexahydro-3H-pyrazino[1, 2-a]quinoxalin-3-carboxylate as a white solid (0.90 g, 2.42 mmol, yield 68%). LCMS(ESI)m/z: 372[M+H]+

Step III: Synthesis of t-butyl (S)-6-methyl-5-oxo-8-(trifluoromethyl)-1, 2, 4, 4a, 5, 6-hexahydro-3H-pyrazino[1, 2-a]quinoxalin-3-carboxylate

T-butyl (S)-5-oxo-8-(trifluoromethyl)-1, 2, 4, 4a, 5, 6-hexahydro-3H-pyrazino[1, 2-a]quinoxalin-3-carboxylate (200.00 mg, 0.54 mmol) was added to tetrahydrofuran (20.00 ml). The mixture was cooled to 0°C, and then added with sodium hydride (36.00 mg, 0.90 mmol, 60%). The reaction system was warmed to room temperature and stirred for one hour. Then the mixture was cooled to 0°C , added with iodomethane (114.20 mg, 0.81 mmol), and further reacted for 2 hrs. After the reaction was completed, the reaction solution was added with ice water (40ml), and then extracted with ethyl acetate (20 ml*3). The organic layer was collected, dried over anhydrous sodium sulfate, and filtered. The filtrate was concentrated to obtain a crude product, which was purified by column chromatography on silica gel (petroleum ether: ethyl acetate =80:20) to obtain t-butyl (S)-6-methyl-5-oxo-8-(trifluoromethyl)-1, 2, 4, 4a, 5, 6-hexahydro-3H-pyrazino[1, 2-a]quinoxalin-3-carboxylate as a yellow solid (150.00 mg, 0.39 mmol, yield: 72%). LCMS(ESI)m/z: 386[M+H]+

Step IV: Synthesis of (S)-6-methyl-8-(trifluoromethyl)-2, 3, 4, 4a-tetrahydro-1H-pyrazino[1, 2-a]quinoxalin-5(6H)-one

T-butyl (S)-6-methyl-5-oxo-8-(trifluoromethyl)-1, 2, 4, 4a, 5, 6-hexahydro-3H-pyrazino[1, 2-a]quinoxalin-3-carboxylate (140.00 mg, 0.36 mmol) was dissolved in hydrogen chloride/dioxane solution (4 mol/l, 5.00 ml). The mixture was stirred at room temperature for 1 hrs. After complete reaction, the reaction solution was concentrated under reduced pressure to obtain crude (S)-6-methyl-8-(trifluoromethyl)-2, 3, 4, 4a-tetrahydro-1H-pyrazino[1, 2-a]quinoxalin-5(6H)-one as a green solid (120.00 mg). The crude product was used in the next reaction without purification. LCMS(ESI)m/z: 286[M+H]+

Step V: Synthesis of (S)-6-methyl-3-(3-(S)-2-(6-oxo-5-(trifluoromethyl)-1, 6-dihydropyridazin-4-yl)amino)propoxy)propionyl)-8-(trifluoromethyl)-2, 3, 4, 4a-tetrahydro-1H-pyrazino[1, 2-a]quinoxalin-5(6H)-one

(S)-6-methyl-8-(trifluoromethyl)-2, 3, 4, 4a-tetrahydro-1H-pyrazino[1, 2-a]quinoxalin-5(6H)-one (50.00 mg, 0.18 mmol) was added to dichloromethane (5.00 ml), and then (S)-3-(2-((6-oxo-5-(trifluoromethyl)-1, 6-dihydropyridazin-4-yl)amino)propoxy)propionic acid (54.08 mg, 0.18 mmol), 1-propylphosphonic anhydride (333.90 mg, 0.54 mmol, 50% solution in ethyl acetate) and N,N-diisopropylethyl amine (112.88 mg, 0.88 mmol) were added. The mixture was stirred at room temperature for 2 hrs. Then the reaction solution was added with water (10 ml), and extracted with dichloromethane (5 ml x 2). The organic layer was collected, washed with saturated brine, dried over anhydrous sodium sulfate, and filtered. The filtrate was concentrated, and the resulting crude product was purified by high performance liquid chromatography (chromatographic column Xsele CSH C18 OBD column, 30 x 150 mm 5 μm; mobile phase A: 0.05% formic acid aqueous solution, mobile phase B: acetonitrile; flow rate: 60 ml/ min; gradient: 10% B to 65% B over 8 min; wavelength: 220 nm; retention time: 7.50 min) to obtain (S)-6-methyl-3-(3-(S)-2-(6-oxo-5-(trifluoromethyl)-1, 6-dihydropyridazin-4-yl)amino)propoxy)propionyl)-8-(trifluoromethyl)-2, 3, 4, 4a-tetrahydro-1H-pyrazino[1, 2-a]quinoxalin-5(6H)-one as a white solid (14.81 mg, 0.03 mmol, yield: 15%).

LCMS(ESI)m/z: 577.10 [M+H]+. HNMR (400 MHz, DMSO-d6): δ ppm 12.52-12.42 (m, 1H), 8.04-7.78(m, 1H), 7.51-7.15(m, 2H), 7.15-6.87 (m, 1H), 6.40-6.10(m, 1H), 4.92-4.43(m, 1H), 4.36-3.98 (m, 2H),3.78-3.65(m, 3H),3.65-3.40 (m, 3H), 3.36-3.33 (m, 3H), 3.25-3.15 (m, 1H), 2.81-2.70 (m, 1H), 2.68-2.59(m,3H), 1.26-0.92(m, 3H).

**Example 49:** Synthesis of 5-(((S)-1-(3-((R)-5-methyl-3-(trifluoromethyl)-5, 6, 6a, 7, 9, 10-hexahydro-8H-pyrazino[1, 2-a]pyrido[3, 2-e]pyrazin-8-yl)-3-oxypropoxy)prop-2-yl)amino)-4-(trifluoromethyl)pyridazin-3(2H)-one **(Compound 50)**

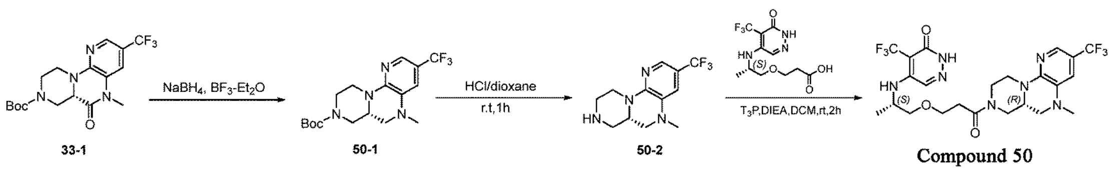


33-1 50-1 50-2 Compound 50

Step I: Synthesis of t-butyl (R)-5-methyl-3-(trifluoromethyl)-5, 6, 6a, 7, 9, 10-hexahydro-8H-pyrazino[1, 2-a]pyrido[3, 2-e]pyrazin-8-carboxylate

T-butyl (S)-5-methyl-6-oxo-3-(trifluoromethyl)-5, 6, 6a, 7, 9, 10-hexahydro-8H-pyrazino[1, 2-a]pyrido[3, 2-e]pyrazin-8-carboxylate (200 mg, 0.52 mmol) was dissolved in tetrahydrofuran (10.00 ml). Sodium borohydride (200.00 mg, 5.20 mmol) was added to the mixture, and then a boron trifluoride-diethyl etherate solution (2.00 ml) was added dropwise. The mixture was stirred at room temperature for 2 hrs. After the reaction was completed, the reaction solution was quenched with a sodium bicarbonate aqueous solution (30 ml), and then extracted with ethyl acetate (20 ml*3). The organic layer was collected, washed with saturated brine, dried over anhydrous sodium sulfate, and filtered. The filtrate was concentrated to obtain crude t-butyl (R)-5-methyl-3-(trifluoromethyl)-5, 6, 6a, 7, 9, 10-hexahydro-8H-pyrazino[1, 2-a]pyrido[3, 2-e]pyrazin-8-carboxylate as a yellow oil (300.00 mg). LCMS (ESI)m/z: 372[M+H]+

Step II: Synthesis of (S)-5-methyl-3-(trifluoromethyl)-6, 6a, 7, 8, 9, 10-hexahydro-5H-pyrazino[1, 2-a]pyrido[3, 2-e]pyrazine

T-butyl (R)-5-methyl-3-(trifluoromethyl)-5, 6, 6a, 7, 9, 10-hexahydro-8H-pyrazino[1, 2-a]pyrido[3, 2-e]pyrazin-8-carboxylate (200.00 mg, 0.53 mmol) was dissolved in hydrogen chloride/dioxane solution (4 mol/l, 5.00 ml). The mixture was stirred at room temperature for 1 hrs. After complete reaction, the reaction solution was concentrated under reduced pressure to obtain crude (S)-5-methyl-3-(trifluoromethyl)-6, 6a, 7, 8, 9, 10-hexahydro-5H-pyrazino[1, 2-a]pyrido[3, 2-e]pyrazine as a yellow oil (200.00 mg). The crude product was used in the next reaction without purification. LC-MS(ESI)m/z: 272[M+H]+

Step III: Synthesis of 5-(((S)-1-(3-(R)-5-methyl-3-(trifluoromethyl)-5, 6, 6a, 7, 9, 10-hexahydro-8H-pyrazino[1, 2-a]pyrido[3, 2-e]pyrazin-8-yl)-3-oxypropoxy)prop-2-yl)amino)-4-(trifluoromethyl)pyridazin-3(2H)-one

(S)-5-methyl-3-(trifluoromethyl)-6, 6a, 7, 8, 9, 10-hexahydro-5H-pyrazino[1, 2-a]pyrido[3, 2-e]pyrazine (200.00 mg, 0.73 mmol) was added to dichloromethane (10.00 ml), and then (S)-3-(2-((6-oxo-5-(trifluoromethyl)-1, 6-dihydropyridazin-4-yl)amino)propoxy)propionic acid (40.00 mg, 0.13 mmol), 1-propylphosphonic anhydride (1400.00 mg, 2.20 mmol, 50% solution in ethyl acetate) and N,N-diisopropylethyl amine (474.00 mg, 3.67 mmol) were added. The mixture was stirred at room temperature for 2 hrs. Then, the reaction solution was added with water (20 ml), and extracted with dichloromethane (10 ml x 3). The organic layer was collected, washed with saturated brine, dried over anhydrous sodium sulfate, and filtered. The filtrate was concentrated, and the resulting crude product was purified by high performance liquid chromatography (Xsele CSH C18 OBD column, 30 x 150 mm 5 μm; mobile phase A: 0.05% formic acid aqueous solution, mobile phase B: acetonitrile; flow rate: 60 ml/ min; gradient: 5% B to 85% B over 8 min; wavelength: 220 nm; retention time: 6.33 min) to obtain 5-(((S)-1-(3-(R)-5-methyl-3-(trifluoromethyl)-5, 6, 6a, 7, 9, 10-hexahydro-8H-pyrazino[1, 2-a]pyrido[3, 2-e]pyrazin-8-yl)-3-oxypropoxy)prop-2-yl)amino)-4-(trifluoromethyl)pyridazin-3(2H)-one as a white solid (7.59mg, 0.013mmol, yield: 4%).

LCMS(ESI)m/z: 564.05 [M+H]+. HNMR (400 MHz, DMSO-d6): δ ppm 12.46 (s, 1H), 7.91(s, 1H),7.76(s, 1H), 6.75(s, 1H), 6.41-6.13 (m, 1H),4.71-4.50(m, 1H), 4.50-4.29(m, 1H), 4.29-4.05 (m, 1H),4.05-3.86(m, 1H),3.83-3.59 (m, 2H), 3.51-3.46 (m, 2H), 3.44-3.35 (m, 2H), 3.09-3.00 (m, 1H),2.91-2.79(m,4H), 2.77-2.56(m, 3H), 2.48-2.37 (m, 1H) ,1.29-1.00(m, 3H).

**Example 50:** Synthesis of (R)-8-(3-((S)-2-((6-oxo-5-(trifluoromethyl)-1, 6-dihydropyridazin-4-yl)amino)propoxy)propionyl)-3-(trifluoromethyl)-6, 6a, 7, 8, 9, 10-hexahydropyrazino[1, 2-d]pyrido[3, 2-b][1, 4]oxazinane -4-carbonitrile **(Compound 51)**

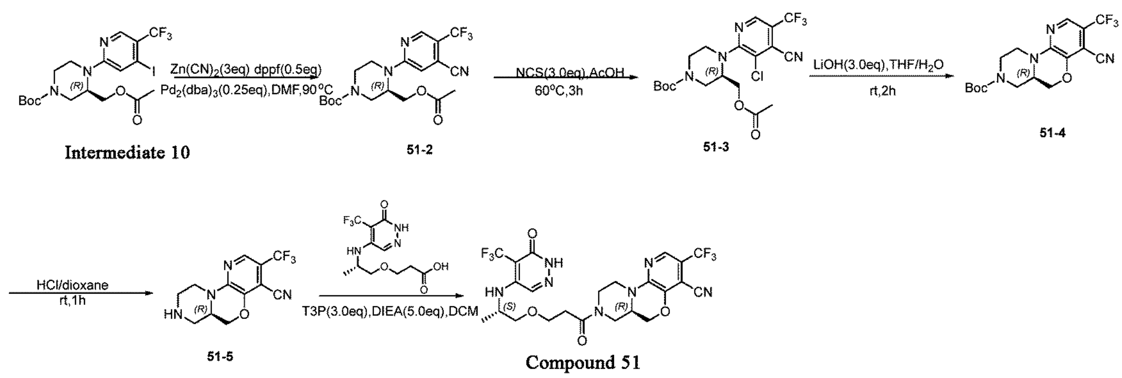


Intermediate 10 51-2 51-3 51-4

51-5 Compound 51

Step I: Synthesis of t-butyl (R)-3-(acetyloxymethyl)-4-(4-cyano-5-(trifluoromethyl)pyridin-2-yl)piperazin-1-carboxylate

T-butyl (R)-3-(acetyloxymethyl)-4-(4-iodo-5-(trifluoromethyl)pyridin-2-yl)piperazin-1-carboxylate (**Intermediate 10,** 1.00 g, 1.89 mmol) were dissolved in N,N-dimethylformamide (20.00 ml), and then zinc cyanide (0.65 g, 5.56 mmol), tris(dibenzylideneacetone) dipalladium (0.05 g, 0.50 mmol) and 1,1'-bis(diphenylphosphino)ferrocene (0.05 g, 0.90 mmol) were added. The reaction system was purged three times with nitrogen. The mixture was stirred for 2 hrs under nitrogen atmosphere at 90°C. After complete reaction, the reaction solution was cooled to room temperature, added with water (50 ml), and then extracted with ethyl acetate (25 ml*3). The organic layer was collected, washed with saturated brine, dried over anhydrous sodium sulfate, and filtered. The filtrate was concentrated, and the resulting crude product was purified by column chromatography on silica gel (petroleum ether: ethyl acetate =80:20) to obtain t-butyl (R)-3-(acetyloxymethyl)-4-(4-cyano-5-(trifluoromethyl)pyridin-2-yl)piperazin-1-carboxylate as a yellow oil (0.70 g, 1.63 mmol, yield: 87.5%). LCMS(ESI)m/z: 429[M+H]+

Step II: Synthesis of t-butyl (R)-3-(acetyloxymethyl)-4-(3-chloro-4-cyano-5-(trifluoromethyl)pyridin-2-yl)piperazin-1-carboxylate

T-butyl (R)-3-(acetyloxymethyl)-4-(4-cyano-5-(trifluoromethyl)pyridin-2-yl)piperazin-1-carboxylate (0.60 g, 1.40 mmol) was added to glacial acetic acid (20.00 ml), and then N-chlorosuccinimide (0.57 g, 4.28 mmol) was added. The mixture was stirred for 3 hrs at 60°C. After complete reaction, the reaction solution was cooled to room temperature, rotary dried to remove the solvent, added with water (20 ml), and then extracted with ethyl acetate (30 ml*3). The organic layer was collected, washed with saturated brine, dried over anhydrous sodium sulfate, and filtered. The filtrate was concentrated, and the crude product was purified by column chromatography on silica gel(petroleum ether: ethyl acetate =80:20) to obtain t-butyl (R)-3-(acetyloxymethyl)-4-(3-chloro-4-cyano-5-(trifluoromethyl)pyridin-2-yl)piperazin-1-carboxylate as a yellow oil (0.45 g, 0.97 mmol , yield 70%). LCMS(ESI)m/z: 463[M+H]+

Step III: Synthesis of t-butyl (R)-4-cyano-3-(trifluoromethyl)-6a, 7, 9, 10-tetrahydropyrazino[1, 2-d]pyrido[3, 2-b][1, 4]oxazinane -8(6H)-carboxylate

T-butyl (R)-3-(acetyloxymethyl)-4-(3-chloro-4-cyano-5-(trifluoromethyl)pyridin-2-yl)piperazin-1-carboxylate(200.00mg, 0.43mmol) was added to a mixed solution of tetrahydrofuran (5.00 ml) and water (5.00 ml). Lithium hydroxide monohydrate (54.60 mg, 1.29 mmol) was added. The mixture was stirred at room temperature for 2 hrs. After complete reaction, the reaction solution was extracted with ethyl acetate (5 ml*3). The organic layer was collected, washed with saturated brine, dried over anhydrous sodium sulfate, and filtered. The filtrate was concentrated to obtain crude t-butyl (R)-4-cyano-3-(trifluoromethyl)-6a, 7, 9, 10-tetrahydropyrazino[1, 2-d]pyrido[3, 2-b][1, 4]oxazinane -8(6H)-carboxylate as a white solid (180.00 mg). LCMS(ESI)m/z: 385[M+H]+

Step IV: Synthesis of (R)-3-(trifluoromethyl)-6, 6a, 7, 8, 9, 10-hexahydropyrazino[1, 2-d]pyrido[3, 2-b][1, 4]oxazinane -4-carbonitrile

T-butyl (R)-4-cyano-3-(trifluoromethyl)-6a, 7, 9, 10-tetrahydropyrazino[1, 2-d]pyrido[3, 2-b][1, 4] oxazinane -8(6H)-carboxylate (170.00mg, 0.44mmol) was dissolved in hydrogen chloride/dioxane solution (4 mol/l, 5.00 ml). The mixture was stirred at room temperature for 1 hrs. After complete reaction, the reaction solution was concentrated under reduced pressure to obtain crude (R)-3-(trifluoromethyl)-6, 6a, 7, 8, 9, 10-hexahydropyrazino[1, 2-d]pyrido[3, 2-b][1, 4]oxazinane -4-carbonitrile as a yellow solid (150.00 mg). The crude product was used in the next reaction without purification. LCMS(ESI)m/z: 285[M+H]+

Step V: Synthesis of (R)-8-(3-((S)-2-((6-oxo-5-(trifluoromethyl)-1, 6-dihydropyridazin-4-yl)amino)propoxy)propionyl)-3-(trifluoromethyl)-6, 6a, 7, 8, 9, 10-hexahydropyrazino[1, 2-d]pyrido[3, 2-b][1, 4]oxazinane -4-carbonitrile

(R)-3-(trifluoromethyl)-6, 6a, 7, 8, 9, 10-hexahydropyrazino[1, 2-d]pyrido[3, 2-b][1, 4] oxazinane -4-carbonitrile (50.00 mg, 0.17 mmol) was added to dichloromethane (5.00 ml), and then (S)-3-(2-((6-oxo-5-(trifluoromethyl)-1, 6-dihydropyridazin-4-yl)amino)propoxy)propionic acid (43.00mg, 0.14mmol), 1-propylphosphonic anhydride (340.00mg, 0.53mmol, 50% solution in ethyl acetate) and N,N-diisopropylethyl amine (113.00 mg,0.88 mmol) were added. The mixture was stirred at room temperature for 2 hrs. Then the reaction solution was added with water (10 ml), and extracted with dichloromethane (10 ml x 3). The organic layer was collected, washed with saturated brine, dried over anhydrous sodium sulfate, and filtered. The filtrate was concentrated, to obtain a crude product, which was purified by high performance liquid chromatography (chromatographic column Xsele CSH C18 OBD column, 30 x 150 mm, 5 μm; mobile phase A: 10 mmol/l ammonium bicarbonate aqueous solution, mobile phase B: acetonitrile; flow rate: 60 ml/ min; gradient: 20% B to 58% B over 8 min; wavelength: 220 nm; retention time: 7.87 min) to obtain (R)-8-(3-((S)-2-((6-oxo-5-(trifluoromethyl)-1, 6-dihydropyridazin-4-yl)amino)propoxy)propionyl)-3-(trifluoromethyl)-6, 6a, 7, 8, 9, 10-hexahydropyrazino[1, 2-d]pyrido[3, 2-b][1, 4]oxazinane -4-carbonitrile as a white solid (5.54 mg, 0.01 mmol, yield: 8%).

LCMS(ESI)m/z: 576.00 [M+H]+. HNMR (400 MHz, DMSO-d6): δ ppm 12.44 (s, 1H), 8.18(s, 1H),7.90(s, 1H), 6.38-6.15(m, 1H), 4.78-4.56 (m, 1H),4.56-4.31(m, 2H), 4.22-4.16(m, 1H), 4.16-3.93 (m, 2H),3.75-3.66(m, 2H),3.66-3.53(m, 1H), 3.53-3.44 (m, 2H), 3.27-3.08 (m, 1H), 3.03-2.71 (m, 2H),2.67-2.57(m,2H), 1.20-1.07(m, 3H).

**Example 51:** Synthesis of 5-(((S)-1-(3-((R)-4-methyl-3-(trifluoromethyl)-6a, 7, 9, 10-tetrahydropyrazino[1, 2-d]pyrido[3, 2-b][1, 4]oxazinane -8(6H)-yl)-3-oxopropoxy)prop-2-yl)amino)-4-(trifluoromethyl)pyridazin-3(2H)-one **(Compound 52)**

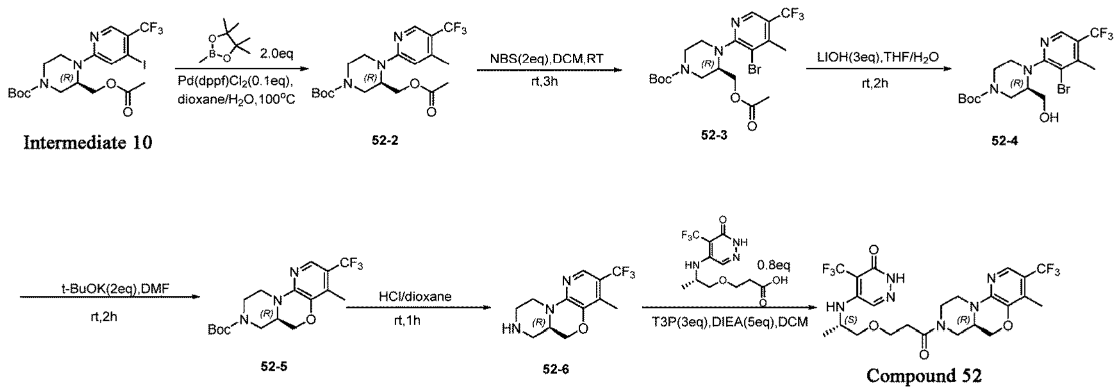

Intermediate 10
52-2
52-3
52-4
52-5
52-6
Compound 52

Step I: Synthesis of t-butyl (R)-3-(acetyloxymethyl)-4-(4-methyl-5-(trifluoromethyl)pyridin-2-yl)piperazin-1-carboxylate

T-butyl (R)-3-(acetyloxymethyl)-4-(4-iodo-5-(trifluoromethyl)pyridin-2-yl)piperazin-1-carboxylate (**Intermediate 10,** 2.00 g, 3.78 mmol) was dissolved in a mixed solution of dioxane (20.00 ml) and water (20.00 ml), and then 2, 4, 4, 5, 5-pentamethyl-1, 3, 2-dioxolane (1.06 g, 7.56 mmol), [1,1'-bi(diphenylphosphino) ferrocene]palladium dichloride (0.73 g, 0.37 mmol) and potassium carbonate (1.04 g, 7.54 mmol) were added. The reaction system was purged 3 times with nitrogen. The mixture was stirred for 16 hrs under nitrogen atmosphere at 100°C. After the reaction was completed, the reaction solution was cooled to room temperature, added with water (50 ml), and then extracted with ethyl acetate (50 ml*3). The organic layer was collected, washed with saturated brine, dried over anhydrous sodium sulfate, and filtered. The filtrate was concentrated, and the obtained crude product was purified by column chromatography on silica gel (petroleum ether: ethyl acetate =80:20) to obtain t-butyl (R)-3-(acetyloxymethyl)-4-(4-methyl-5-(trifluoromethyl)pyridin-2-yl)piperazin-1-carboxylate as a yellow oil (1.00 g, 2.40 mmol, yield: 63%). LCMS (ESI) m/z: 418[M+H]+

Step II: Synthesis of t-butyl (R)-3-(acetyloxymethyl)-4-(3-bromo-4-methyl-5-(trifluoromethyl)pyridin-2-yl)piperazin-1 -carboxylate

T-butyl (R)-3-(acetyloxymethyl)-4-(4-methyl-5-(trifluoromethyl)pyridin-2-yl)piperazin-1-carboxylate (0.80 g, 1.92 mmol) was added to dichloromethane (20.00 ml), and then N-bromosuccinimide (0.68 g, 3.84 mmol) was added. The mixture was stirred for 3 hrs at room temperature. After the reaction was completed, the reaction solution was added with water (30 ml), and then extracted with dichloromethane (30 ml * 3). The organic layer was collected, washed with

saturated brine, dried over anhydrous sodium sulfate, and filtered. The filtrate was concentrated, and the obtained crude product was purified by column chromatography on silica gel (petroleum ether: ethyl acetate =75:25) to obtain t-butyl (R)-3-(acetyloxymethyl)-4-(3-bromo-4-methyl-5-(trifluoromethyl)pyridin-2-yl)piperazin-1-carboxylate as a colorless oil (0.30 g, 0.60 mmol, yield 31%). LCMS (ESI) m/z: 496[M+H]+

Step III: Synthesis of t-butyl4-(3-bromo-4-methyl-5-(trifluoromethyl)pyridin-2-yl)-3-(hydroxymethyl)piperazin-1 -carboxylate

T-butyl (R)-3-(acetyloxymethyl)-4-(3-bromo-4-methyl-5-(trifluoromethyl)pyridin-2-yl)piperazin-1-carboxylate (300.00 mg, 0.60 mmol) was added to a mixed solution of tetrahydrofuran (5 ml) and water (5 ml), and then lithium hydroxide monohydrate (75.60 mg, 1.80 mmol) was added. The mixture was stirred for 2 hrs at room temperature. After complete reaction, the reaction solution was extracted with ethyl acetate (20 ml*3). The organic layer was collected, washed with saturated brine, dried over anhydrous sodium sulfate, and filtered. The filtrate was concentrated, and the obtained crude product was purified by column chromatography on silica gel (petroleum ether: ethyl acetate =60:40) to obtain t-butyl4-(3-bromo-4-methyl-5-(trifluoromethyl)pyridin-2-yl)-3-(hydroxymethyl)piperazin-1-carboxylate as a yellow oil (230.00 mg, 0.50 mmol, yield: 83%). LCMS (ESI) m/z: 454[M+H]+

Step IV: Synthesis of t-butyl (R)-4-methyl-3-(trifluoromethyl)-6a, 7, 9, 10-tetrahydropyrazino[1, 2-d]pyrido[3, 2-b][1, 4]oxazinane -8(6H)-carboxylate

T-butyl 4-(3-bromo-4-methyl-5-(trifluoromethyl)pyridin-2-yl)-3-(hydroxymethyl)piperazin-1-carboxylate (230.00 mg, 0.50 mmol) was dissolved in N,N-dimethylformamide (10.00 ml). Potassium tert-butoxide (113.40 mg, 1.00 mmol) was added to the mixed solution. The mixture was stirred at room temperature for 2 hrs. After the reaction was completed, the reaction solution was added with water (30 ml), and then extracted with ethyl acetate (20 ml*3). The organic layer was collected, washed with saturated brine, dried over anhydrous sodium sulfate, and filtered. The filtrate was concentrated, and the obtained crude product was purified by column chromatography on silica gel (petroleum ether: ethyl acetate =80:20) to obtain t-butyl (R)-4-methyl-3-(trifluoromethyl)-6a, 7, 9, 10-tetrahydropyrazino[1, 2-d]pyrido[3, 2-b][1, 4] oxazinane -8(6H)-carboxylate as a yellow oil (120.00 mg, 0.32 mmol, yield: 64%). LCMS (ESI) m/z: 374[M+H]+

Step V: Synthesis of (R)-4-methyl-3-(trifluoromethyl)-6, 6a, 7, 8, 9, 10-hexahydropyrazino[1, 2-d]pyrido[3, 2-b][1, 4]oxazinane

T-butyl (R)-4-methyl-3-(trifluoromethyl)-6a, 7, 9, 10-tetrahydropyrazino[1, 2-d]pyrido[3, 2-b][1, 4]oxazinane -8(6H)-carboxylate (120.00 mg, 0.32 mmol) was dissolved in a hydrogen chloride/dioxane solution (4 mol/l, 5.00 ml). The mixture was stirred at room temperature for 1 hrs. After complete reaction, the reaction solution was concentrated under reduced pressure to obtain crude (R)-4-methyl-3-(trifluoromethyl)-6, 6a, 7, 8, 9, 10-hexahydropyrazino[1, 2-d]pyrido[3, 2-b][1, 4] oxazinane as a white solid (80.00 mg). The crude product was used in the next reaction without purification. LCMS (ESI) m/z: 274[M+H]+

Step VI: Synthesis of 5-(((S)-1-(3-((R)-4-methyl-3-(trifluoromethyl)-6a, 7, 9, 10-tetrahydropyrazino[1, 2-d]pyrido[3, 2-b][1, 4]oxazinane -8(6H)-yl)-3-oxopropoxy)prop-2-yl)amino)-4-(trifluoromethyl)pyridazin-3(2H)-one

(R)-4-methyl-3-(trifluoromethyl)-6, 6a, 7, 8, 9, 10-hexahydropyrazino[1, 2-d]pyrido[3, 2-b][1, 4]oxazinane (50.00 mg, 0.18 mmol) was added to dichloromethane (5.00 ml). Then, (S)-3-(2-((6-oxo-5-(trifluoromethyl)-1, 6-dihydropyridazin-4-yl)amino)propoxy)propionic acid (45.00mg, 0.15mmol), 1-propylphosphonic anhydride (343.00mg, 0.54mmol, 50% solution in ethyl acetate) and N,N-diisopropylethyl amine (116.00 mg, 0.90 mmol) were added. The mixture was stirred at room temperature for 2 hrs. After the reaction was completed, the reaction solution was added with water (10 ml), and extracted with dichloromethane (5 ml x 3). The organic layer was collected, washed with saturated brine, dried over anhydrous sodium sulfate, and filtered. The filtrate was concentrated, and the obtained crude product was purified by high performance liquid chromatography (chromatographic column: XBridge Prep OBD C18 column, 30 * 150 mm, 5 μm; mobile phase A: 10 mmol/l ammonium bicarbonate aqueous solution, mobile phase B: acetonitrile; flow rate: 60 ml/ min; gradient:15% B to 61% B over 10 min; wavelength: 220 nm; retention time: 9.70 min) to obtain 5-(((S)-1-(3-((R)-4-methyl-3-(trifluoromethyl)-6a, 7, 9, 10-tetrahydropyrazino[1, 2-d]pyrido[3, 2-b][1, 4]oxazinane -8(6H)-yl)-3-oxopropoxy)prop-2-yl)amino)-4-(trifluoromethyl)pyridazin-3(2H)-one as a white solid (5.89 mg, 0.01 mmol, yield: 6%).

LCMS (ESI) m/z: 565.15 [M+H]+. HNMR (400 MHz, DMSO-d6): δ ppm 12.45(s, 1H), 7.99(s, 1H),7.90(s, 1H), 6.27(s, 1H), 4.56-4.37 (m, 3H),4.22-4.08(m, 1H), 4.08-3.89(m, 2H), 3.79-3.55(m, 2H),3.53-3.45(m, 2H),3.21-3.02 (m, 1H), 2.92-2.88 (m, 1H), 2.88-2.68 (m, 1H), 2.64-2.57 (m, 2H),2.45-2.40(m,1H),2.20-2.15(m, 3H), 1.18-1.10(m, 3H).

**Example 52:** Synthesis of (S)-5-methyl-6-oxo-8-(3-(S)-2-(6-oxo-5-(trifluoromethyl)-1, 6-dihydropyridazin-4-yl)amino)propoxy)propionyl)-6, 6a, 7, 8, 9, 10-hexahydro-5H-pyrazino[1, 2-a]pyrido[3, 2-e]pyrazin-3-carbonitrile (**Compound 53**)

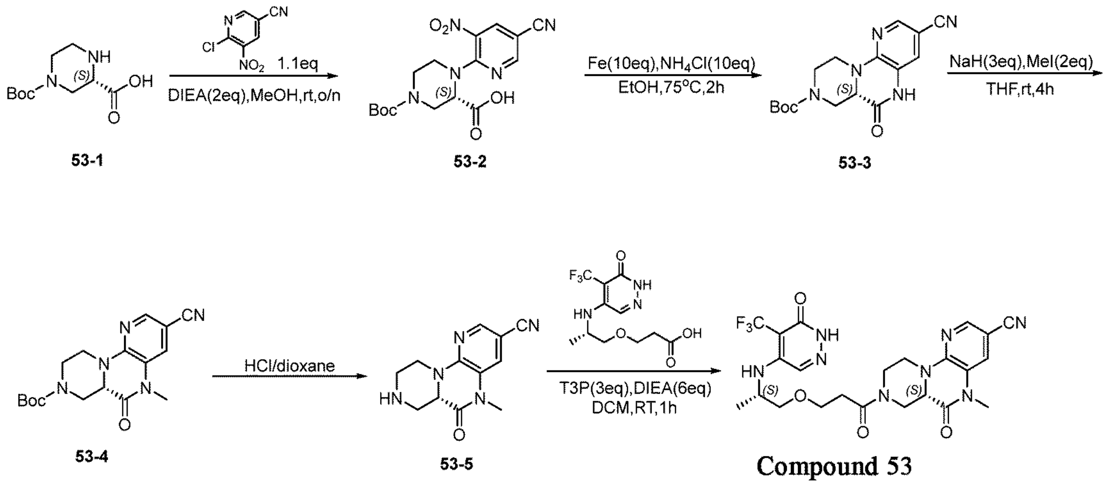


53-1 53-2 53-3 53-4 53-5 Compound 53

Step I: Synthesis of (S)-4-(t-butoxycarbonyl)-1-(5-cyano-3-nitropyridin-2-yl)piperazin-2-carboxylic acid

(S)-4-(t-butoxycarbonyl)piperazin-2-carboxylic acid (1 g, 4.35 mmol) and 6-chloro-5-nitronicotinonitrile (877 mg, 4.79 mmol) were dissolved in methanol (10 ml), and then N,N-diisopropylethyl amine (1.12 g, 8.70 mmol) was added and reacted overnight at room temperature. After the reaction was completed, the reaction solution was poured into water (30 ml), adjusted to pH 6 with formic acid, and then extracted with ethyl acetate (30 ml x 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate :petroleum ether=50: 50) to obtain (S)-4-(t-butoxycarbonyl)-1-(5-cyano-3-nitropyridin-2-yl)piperazin-2-carboxylic acid as a yellow oil (508 mg, 1.35 mmol, yield 27%). LCMS (ESI): 378 [M+H]+

Step II: Synthesis of t-butyl (S)-3-cyano-6-oxo-5, 6, 6a, 7, 9, 10-hexahydro-8H-pyrazino[1, 2-a]pyrido[3, 2-e]pyrazin-8-carboxylate

(S)-4-(t-butoxycarbonyl)-1-(5-cyano-3-nitropyridin-2-yl)piperazin-2-carboxylic acid (508 mg, 1.35 mmol) and ammonium chloride (716 mg, 13.5 mmol) were dissolved in ethanol (5 ml), and then iron powder (756 mg, 13.5 mmol) was added and reacted at 75°C for 2 hrs. After complete reaction, the reaction solution was cooled to room temperature, poured into water (20 ml), and then extracted with ethyl acetate (20 ml x 3). The organic phases were combined, washed with saturated brine, and dried over anhydrous sodium sulfate, Anhydrous sodium sulfate was removed by filtration, and the filtrate was concentrated. The crude product was purified by column chromatography on silica gel (ethyl acetate :petroleum ether=40: 60) to obtain t-butyl (S)-3-cyano-6-oxo-5, 6, 6a, 7, 9, 10-hexahydro-8H-pyrazino[1, 2-a]pyrido[3, 2-e]pyrazin-8-carboxylate as a yellow oil (300 mg, 0.91 mmol, yield 67%). LCMS (ESI): 330 [M+H]+

Step III: Synthesis of t-butyl (S)-3-cyano-5-methyl-6-oxo-5, 6, 6a, 7, 9, 10-hexahydro-8H-pyrazino[1, 2-a]pyrido[3, 2-e]pyrazin-8-carboxylate

T-butyl (S)-3-cyano-6-oxo-5, 6, 6a, 7, 9, 10-hexahydro-8H-pyrazino[1, 2-a]pyrido[3, 2-e]pyrazin-8-carboxylate (300 mg, 0.91 mmol) was dissolved in tetrahydrofuran (10 ml), cooled to 0°C, and then added with sodium hydride (109 mg, 2.73 mmol, 60%). After stirring for 1 hr, iodomethane (258 mg, 1.82 mmol) was added, and further reacted for 3 hrs at room temperature. After the reaction was completed, the reaction solution was poured into ice water (20 ml), and then the aqueous phase was extracted with ethyl acetate (20 ml x 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate :petroleum ether=30: 70) to obtain t-butyl (S)-3-cyano-5-methyl-6-oxo-5, 6, 6a, 7, 9, 10-hexahydro-8H-pyrazino[1, 2-a]pyrido[3, 2-e]pyrazin-8-carboxylate as a yellow oil (150 mg, 0.44 mmol, yield 48%). LCMS (ESI): 344 [M+H]+

Step IV: Synthesis of (S)-5-methyl-6-oxo-6, 6a, 7, 8, 9, 10-hexahydro-5H-pyrazino[1, 2-a]pyrido[3, 2-e]pyrazin-3-carbonitrile

T-butyl (S)-3-cyano-5-methyl-6-oxo-5, 6, 6a, 7, 9, 10-hexahydro-8H-pyrazino[1, 2-a]pyrido[3, 2-e]pyrazin-8-carboxylate (150 mg, 0.44 mmol) was dissolved in ice-cold (0°C) hydrogen chloride/1, 4-dioxane solution (4 mol/l, 8 ml), slowly warmed to room temperature and reacted for 4 hrs with stirring. After complete reaction, the reaction solution was concentrated under reduced pressure, to obtain crude (S)-5-methyl-6-oxo-6, 6a, 7, 8, 9, 10-hexahydro-5H-pyrazino[1, 2-a]pyrido[3, 2-e]pyrazin-3-carbonitrile as a yellow solid (100 mg). The crude product was used in the next reaction without purification. LCMS (ESI): 244 [M+H]+

Step V: Synthesis of (S)-5-methyl-6-oxo-8-(3-(S)-2-(6-oxo-5-(trifluoromethyl)-1, 6-dihydropyridazin-4-yl)amino)propoxy)propionyl)-6, 6a, 7, 8, 9, 10-hexahydro-5H-pyrazino[1, 2-a]pyrido[3, 2-e]pyrazin-3-carbonitrile

(S)-5-methyl-6-oxo-6, 6a, 7, 8, 9, 10-hexahydro-5H-pyrazino[1, 2-a]pyrido[3, 2-e]pyrazin-3-carbonitrile (100 mg, 0 .41 mmol) and (S)-3-(2-((6-oxo-5-(trifluoromethyl)-1, 6-dihydropyridazin-4-yl)amino)propoxy)propionic acid (127 mg, 0.41 mmol) were dissolved in dichloromethane (10 ml), and then diisopropylethyl amine (317 mg, 2.46 mmol) and propylphosphonic anhydride (782 mg, 1.23 mmol, 50% solution in ethyl acetate) were added. The reaction mixture was stirred at room temperature for 1 hr. The reaction solution was poured into ice water (20 ml), and extracted with dichloromethane (20 ml x 3). The organic phases were combined, washed with saturated brine, and dried over anhydrous sodium sulfate. Anhydrous sodium sulfate was removed by filtration, and the filtrate was concentrated. The crude product was purified by high performance liquid chromatography (chromatographic column: XBridge Prep OBD C18, 30 x 150 mm 5 um, mobile phase A: 10 mmol/l ammonium bicarbonate aqueous solution, mobile phase B: acetonitrile, flow rate: 60 ml/ min, elution gradient: mobile phase B from 5% to 50% over 8 min, detection wavelength: 220 nm, retention time: 6.89 min) to obtain (S)-5-methyl-6-oxo-8-(3-(S)-2-(6-oxo-5-(trifluoromethyl)-1, 6-dihydropyridazin-4-yl)amino)propoxy)propionyl)-6, 6a, 7, 8, 9, 10-hexahydro-5H-pyrazino[1, 2-a]pyrido[3, 2-e]pyrazin-3-carbonitrile as a white solid (12.36 mg, 0.023 mmol, yield 5.6%).

LCMS (ESI) m/z: 535.15 $[M+H]^+$, HNMR (400 MHz, DMSO-d6): $\delta$ ppm 12.44 (s, 1H), 8.28 (s, 1H), 7.90-7.87 (m, 1H), 7.64 (s, 1H), 6.29-6.27 (m, 1H), 4.86-4.47 (m, 2H), 4.28-3.99 (m, 3H), 3.70-3.67 (m, 2H), 3.49-3.45 (m, 2H), 3.26-3.23 (m, 3H), 3.08-3.05 (m, 1H), 2.83-2.71 (m, 1H), 2.67-2.57 (m, 3H), 1.15 (d, J = 6.4 Hz, 3H).

**Example 53:** Synthesis of (R)-5-methyl-8-(3-((S)-2-((6-oxo-5-(trifluoromethyl)-1, 6-dihydropyridazin-4-yl)amino)propoxy)propionyl)-3-(trifluoromethyl)-7, 8, 9, 10-tetrahydro-5H-pyrazino[1, 2-a]pyrido[3, 2-e]pyrazin-6(6aH)-one (**Compound 54**)

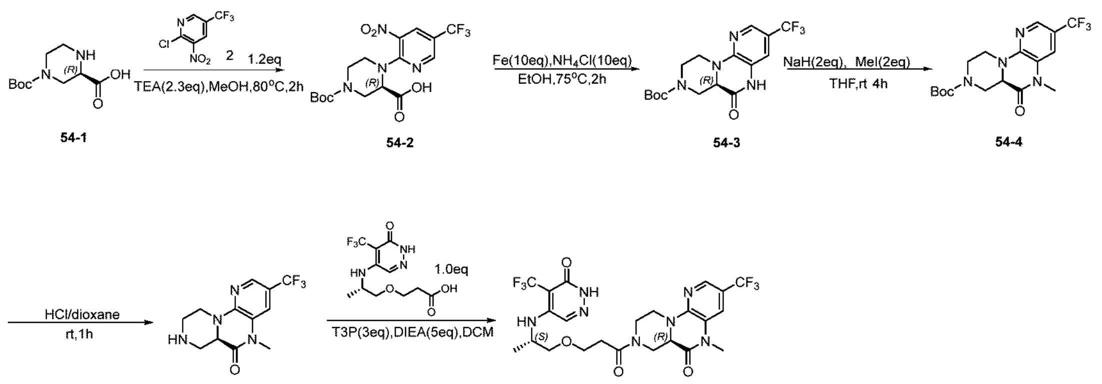


54-1 54-2 54-3 54-4 54-5

Compound 54

Step I: Synthesis of (R)-4-(t-butoxycarbonyl)-1-(3-nitro-5-(trifluoromethyl)pyridin-2-yl)piperazin-2-carboxylic acid

(R)-4-(t-butoxycarbonyl)piperazin-2-carboxylic acid (2.00 g, 8.69 mmol) was dissolved in methanol (40.00 ml), and then 2-chloro-3-nitro-5-(trifluoromethyl)pyridine (2.40 g, 10.62 mmol) and triethyl amine (2.0 g, 19.80 mmol) were added. The mixture was stirred for 2 hrs at 80°C. After the reaction was completed, the reaction solution was cooled to room temperature, added with water (50 ml), adjusted to pH 6 with formic acid, and then extracted with ethyl acetate (50 ml*3). The organic layer was collected, washed with saturated brine, dried over anhydrous sodium sulfate, and

filtered. The filtrate was concentrated, and the resulting crude product was purified by column chromatography on silica gel (dichloromethane : methanol=80:20) to obtain (R)-4-(t-butoxycarbonyl)-1-(3-nitro-5-(trifluoromethyl)pyridin-2-yl)piperazin-2-carboxylic acid as a yellow solid (3.30 g, 7.86 mmol, yield 90%). LCMS (ESI) m/z: 421[M+H]+

Step II: Synthesis of t-butyl (R)-6-oxo-3-(trifluoromethyl)-5, 6, 6a, 7, 9, 10-hexahydro-8H-pyrazino[1, 2-a]pyrido[3, 2-e]pyrazin-8-carboxylate

(R)-4-(t-butoxycarbonyl)-1-(3-nitro-5-(trifluoromethyl)pyridin-2-yl)piperazin-2-carboxylic acid (2.00 g, 4.76 mmol) was added to ethanol (40.00 ml), and then iron powder (2.67 g, 47.68 mmol) and ammonium chloride (2.52 g, 47.54 mmol) were added. The mixture was stirred for 2 hrs at 75°C. The reaction solution was cooled, and filtered through diatomaceous earth. The filtrate was concentrated under reduced pressure, added with water (50 ml), and then extracted with ethyl acetate (30 ml*3). The organic layer was collected, washed with saturated brine, dried over anhydrous sodium sulfate, and filtered. The filtrate was concentrated, and the obtained crude product was purified by column chromatography on silica gel (petroleum ether: ethyl acetate =50:50) to obtain t-butyl (R)-6-oxo-3-(trifluoromethyl)-5, 6, 6a, 7, 9, 10-hexahydro-8H-pyrazino[1, 2-a]pyrido[3, 2-e]pyrazin-8-carboxylate as a yellow solid (1.60 g, 4.30 mmol, yield 90%). LCMS (ESI) m/z: 373[M+H]+

Step III: Synthesis of t-butyl (R)-5-methyl-6-oxo-3-(trifluoromethyl)-5, 6, 6a, 7, 9, 10-hexahydro-8H-pyrazino[1, 2-a]pyrido[3, 2-e]pyrazin-8-carboxylate

T-butyl (R)-6-oxo-3-(trifluoromethyl)-5, 6, 6a, 7, 9, 10-hexahydro-8H-pyrazino[1, 2-a]pyrido[3, 2-e]pyrazin-8-carboxylate (1.00 g, 2.68 mmol) was added to tetrahydrofuran (20.00 ml). The mixed solution was cooled to 0°C, added with sodium hydride (0.21 g, 5.25 mmol, 60%), warmed to room temperature, and stirred for one hour. The mixed solution was then cooled to 0°C, added with iodomethane (0.76 g, 5.36 mmol), and reacted for 3 hrs. After the reaction was completed, the reaction solution was added with ice water (40 ml), and extracted with ethyl acetate (20 ml*3). The organic layer was collected, washed with saturated brine, dried over anhydrous sodium sulfate, and filtered. The filtrate was concentrated, and the obtained crude product was purified by column chromatography on silica gel (petroleum ether: ethyl acetate =80:20) to obtain t-butyl (R)-5-methyl-6-oxo-3-(trifluoromethyl)-5, 6, 6a, 7, 9, 10-hexahydro-8H-pyrazino[1, 2-a]pyrido[3, 2-e]pyrazin-8-carboxylate as a yellow solid (0.40 g, 1.03 mmol, yield 38%). LCMS (ESI) m/z: 387 M+H]+

Step IV: Synthesis of (R)-5-methyl-3-(trifluoromethyl)-7, 8, 9, 10-tetrahydro-5H-pyrazino[1, 2-a]pyrido[3, 2-e]pyrazin-6(6aH)-one

T-butyl (R)-5-methyl-6-oxo-3-(trifluoromethyl)-5, 6, 6a, 7, 9, 10-hexahydro-8H-pyrazino[1, 2-a]pyrido[3, 2-e]pyrazin-8-carboxylate (100.00 mg, 0.26 mmol) was dissolved in hydrogen chloride/dioxane solution (4 mol/l, 5.00 ml). The mixture was stirred at room temperature for 1 hrs. After the reaction was completed, the reaction solution was concentrated under reduced pressure to obtain crude (R)-5-methyl-3-(trifluoromethyl)-7, 8, 9, 10-tetrahydro-5H-pyrazino[1, 2-a]pyrido[3, 2-e]pyrazin-6(6aH)-one as a green solid (90.00 mg). The crude product was used in the next reaction without purification. LCMS (ESI) m/z: 287[M+H]+

Step V: Synthesis of (R)-5-methyl-8-(3-((S)-2-((6-oxo-5-(trifluoromethyl)-1, 6-dihydropyridazin-4-yl)amino)propoxy)propionyl)-3-(trifluoromethyl)-7, 8, 9, 10-tetrahydro-5H-pyrazino[1, 2-a]pyrido[3, 2-e]pyrazin-6(6aH)-one

(R)-5-methyl-3-(trifluoromethyl)-7, 8, 9, 10-tetrahydro-5H-pyrazino[1, 2-a]pyrido[3, 2-e]pyrazin-6(6aH)-one (50.00 mg, 0.17 mmol) was added to dichloromethane (5.00 ml), and then (S)-3-(2-((6-oxo-5-(trifluoromethyl)-1, 6-dihydropyridazin-4-yl)amino)propoxy)propionic acid (54.08 mg, 0.18 mmol), 1-propylphosphonic anhydride (333.90 mg, 0.53 mmol, 50% solution in ethyl acetate) and N,N-diisopropylethyl amine (112.88 mg, 0.88 mmol) were added. The mixture was stirred at room temperature for 2 hrs. After the reaction was completed, the reaction solution was added with water (10ml), and extracted with dichloromethane (5 ml x 3). The organic layer was then collected, washed with saturated brine, dried over anhydrous sodium sulfate, and filtered. The filtrate was concentrated, and the obtained crude product was purified by high performance liquid chromatography (chromatographic column: YMC-Actus Triart C18, 30*150 mm, 5 μm; mobile phase A: 10 mmol/l ammonium bicarbonate aqueous solution, mobile phase B: acetonitrile; flow rate: 60 ml/ min; gradient: 20% B to 60% B over 10 min; wavelength: 220 nm; retention time: 8.82 min) to obtain (R)-5-methyl-8-(3-((S)-2-((6-oxo-5-(trifluoromethyl)-1, 6-dihydropyridazin-4-yl)amino)propoxy)propionyl)-3-(trifluoromethyl)-7, 8, 9, 10-tetrahydro-5H-pyrazino[1, 2-a]pyrido[3, 2-e]pyrazin-6(6aH)-one as a white solid (6.01 mg, 0.01 mmol, yield6%). LCMS (ESI) m/z: 578.10 [M+H]+. HNMR (400 MHz, DMSO-d6): δ ppm 12.42 (s, 1H), 8.19(s, 1H), 8.02-7.75(m, 1H), 7.59-7.33(m, 1H), 6.41-6.12(m, 1H), 4.99-4.37(m, 2H), 4.32-3.87 (m, 3H),3.82-3.57(m, 2H),3.57-3.42 (m, 2H), 3.33-3.32 (m, 3H), 3.25-3.20 (m, 1H), 3.17-3.02 (m, 1H),2.86-2.70(m,1H), 2.68-2.60(m, 2H), 1.28-0.92(m, 3H).

**Example 54**: Synthesis of 5-(((S)-1-((S)-2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-4-(trifluoromethyl)pyridazin-3(2H)-one (**Compound 55**) and 5-(((S)-1-((R)-2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-4-(trifluoromethyl)pyridazin-3(2H)-one (**Compound 56**)

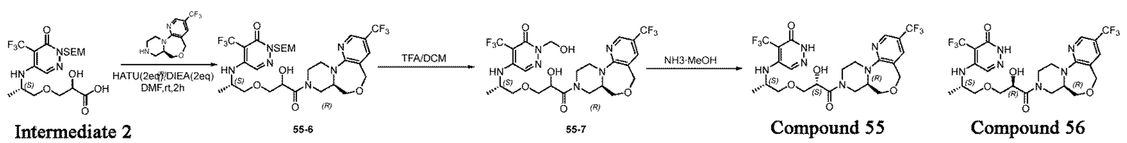


Intermediate 2 55-6 55-7 Compound 55 Compound 56

Step I: Synthesis of 5-(((2S)-1-(2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-4-(trifluoromethyl)-2-(2-(trimethylsilyl)ethoxy)methyl)pyridazin-3(2H)-one

hydroxy-3-((S)-2-((6-oxo-5-(trifluoromethyl)-1-((2-(trimethylsilyl)ethoxy)methyl)-1, 6-dihydropyridazin-4-yl)amino)propoxy)propionic acid (**Intermediate 2,** 400 mg, 0.88 mmol) and (R)-3-(trifluoromethyl)-7, 7a, 8, 9, 10, 11-hexahydro-SH-pyrazino[2, 1-c]pyrido[2, 3-e][1, 4]oxazepane (240 mg, 0.88 mmol) were dissolved in N,N-dimethylformamide (5 ml), and then N,N,N',N'-tetramethyl-O-(7-azabenzotriazole -1-yl)uronium hexafluorophosphate (669 mg, 1.76 mmol) and N,N-diisopropylethyl amine (114 mg, 1.76 mmol) were added and reacted for 2 hrs at room temperature. After the reaction was completed, the reaction solution was poured into water (10 ml) and extracted with ethyl acetate (10 ml x 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (dichloromethane : methanol=70: 30) to obtain 5-(((2S)-1-(2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-4-(trifluoromethyl)-2-(2-(trimethylsilyl)ethoxy)methyl)pyridazin-3(2H)-one as a yellow oil (300 mg, 0.42 mmol, yield 47%). LCMS (ESI) m/z: 711 $[M+H]^+$

Step II: Synthesis of 5-(((2S)-1-(2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-2-(hydroxymethyl)-4-(trifluoromethyl)pyridazin-3(2H)-one

5-(((2S)-1-(2-hydroxy-3-oxo-3-((R)-3-(trifluoromethyl)-7a, 8, 10, 11-tetrahydro-SH-pyrazino[2, 1-c]pyrido[2, 3-e][1, 4]oxazepane-9(7H)-yl)propoxy)prop-2-yl)amino)-4-(trifluoromethyl)-2-(2-(trimethylsilyl)ethoxy)methyl)pyridazin-3(2H)-one (300 mg, 0.42 mmol) was dissolved in ice-cold (0°C) trifluoroacetic acid (2 ml) and dichloromethane (8 ml), slowly warmed to room temperature and reacted for 4 hrs with stirring. After complete reaction, the reaction solution was concentrated under reduced pressure, to obtain crude 5-(((2S)-1-(2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-2-(hydroxymethyl)-4-(trifluoromethyl)pyridazin-3(2H)-one as a yellow solid (160 mg). The crude product was used in the next reaction without purification. LCMS (ESI) m/z: 611 $[M+H]^+$

Step III: Synthesis of 5-(((S)-1-((S)-2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-4-(trifluoromethyl)pyridazin-3(2H)-one and 5-(((S)-1-((R)-2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-4-(trifluoromethyl)pyridazin-3(2H)-one

Crude 5-(((2S)-1-(2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-2-(hydroxymethyl)-4-(trifluoromethyl)pyridazin-3(2H)-one (160 mg) was dissolved in an aminomethanol solution (7 mol/l, 5 ml), and reacted for 3 hrs at room temperature. After the reaction was completed, the reaction solution was concentrated under reduced pressure, added with water (20 ml), and extracted with ethyl acetate (20 ml x 3). The organic phases were combined, washed with saturated brine, and dried over anhydrous sodium sulfate. Anhydrous sodium sulfate was removed by filtration, and the filtrate was concentrated. The crude product was purified by high performance liquid chromatography (chromatographic column: XBridge Prep OBD C18, 30 x 150 mm 5 um, mobile phase A: 10 mmol/l ammonium bicarbonate aqueous solution, mobile phase B: acetonitrile, flow rate: 60 ml/ min, elution gradient: mobile phase B from 20% to 47% over 10 min, detection wavelength:

220 nm, retention time: 9.23/9.68 min) to obtain the first peak 5-(((S)-1-((S)-2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-4-(trifluoromethyl)pyridazin-3(2H)-one as a white solid (30.13 mg, 0.052 mmol, yield 11.24%) and the second peak 5-(((S)-1-((R)-2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-4-(trifluoromethyl)pyridazin-3(2H)-one as a white solid (32.23 mg, 0.056 mmol, yield 12.40%).

First peak (**Compound 55**): 5-(((S)-1-((S)-2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-4-(trifluoromethyl)pyridazin-3(2H)-one

LCMS (ESI) m/z: 581.10[M+H]$^+$

LCMS: chromatographic column: Express C18 HPLC 5 cm * 3.0 mm, 2.7 $\mu$m; mobile phase A: water (0.05% trifluoroacetic acid), mobile phase B: acetonitrile (0.05% trifluoroacetic acid); flow rate: 1.2 ml/ min; gradient: 5% B to 60% B over 0-3 min, 60% B to 100% B over 3-3.8 min, 100% B to 100% B over 3.8-4.8 min, and 100% B to 5% B over 4.8-4.9 min; wavelength: 220 nm; retention time: 2.14 min.

HNMR (400 MHz, DMSO-$d_6$): $\delta$ ppm 8.40 (s,1H), 7.94-7.86 (m,1H), 7.79 (s,1H), 6.32-6.24(m,1H), 5.42-5.15 (m,1H), 4.95-4.84 (m,1H), 4.62-4.51 (m,1H), 4.48-4.38 (m,1H), 4.17-4.08 (m,1H), 4.08-3.96 (m,2H), 3.94-3.85 (m,2H), 3.83-3.57 (m,5H), 3.57-3.48 (m,3H), 3.46-3.35 (m,1H), 1.12-1.10 (m, 3H).

Second peak (**Compound 56**): 5-(((S)-1-((R)-2-hydroxy-3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)-4-(trifluoromethyl)pyridazin-3(2H)-one

LCMS (ESI) m/z: 581.15[M+H]$^+$

LCMS: chromatographic column: Express C18 HPLC 5 cm * 3.0 mm, 2.7 $\mu$m; mobile phase A: water (0.05% trifluoroacetic acid), mobile phase B: acetonitrile (0.05% trifluoroacetic acid); flow rate: 1.2 ml/ min; gradient: 5% B to 60% B over 0-3 min, 60% B to 100% B over 3-3.8 min, 100% B to 100% B over 3.8-4.8 min, and 100% B to 5% B over 4.8-4.9 min; wavelength: 220 nm; retention time: 2.20 min.

HNMR (400 MHz, DMSO-$d_6$): $\delta$ ppm 12.10(brs,1H), 8.44-8.36 (m,1H), 7.96-7.90 (m,1H), 7.82-7.77 (m,1H), 6.32-6.20 (m,1H), 5.39-5.19 (m,1H), 5.01-4.81 (m,1H), 4.68-4.51 (m,1H), 4.49-4.44 (m,1H), 4.24-4.08 (m,1H), 4.08-3.89 (m,3H), 3.86-3.63 (m,5H), 3.62-3.58 (m,1H), 3.56-3.50 (m,3H), 3.31-3.18 (m,1H), 1.13-1.10 (m, 3H).

**Example 55:** Synthesis of (R)-6-methyl-9-(3-((S)-2-((6-oxo-5-(trifluoromethyl)-1, 6-dihydropyridazin-4-yl)amino)propoxy)propionyl)-3-(trifluoromethyl)-5, 6, 8, 9, 10, 11-hexahydropyrazino[1, 2-a]pyrido[3, 2-f][1, 4]diazepan-7(7aH)-one (**Compound 57**)

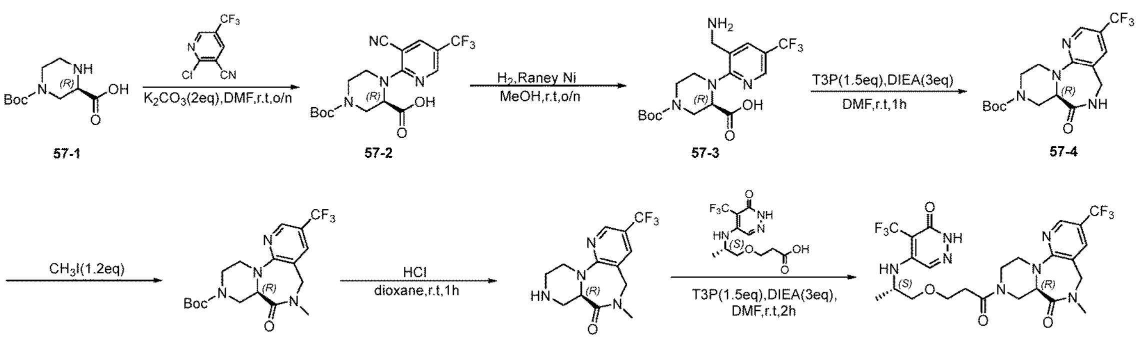


57-1 57-2 57-3 57-4 57-5 57-6 Compound 57

Step I: Synthesis of (R)-4-(t-butoxycarbonyl)-1-(3-cyano-5-(trifluoromethyl)pyridin-2-yl)piperazin-2-carboxylic acid

(R)-4-(t-butoxycarbonyl)piperazin-2-carboxylic acid (2.00 g, 8.7 mmol) was dissolved in N,N-dimethylformamide (20 ml), and then potassium carbonate (2.40 g, 17.4 mmol) and 2-chloro-5-(trifluoromethyl)nicotinonitrile (2.68 g, 13.05 mmol) were added to the mixture. The mixture was reacted overnight at room temperature. After the reaction was completed, the reaction solution was added with water (50 ml), and then extracted with ethyl acetate (20 ml*2). The organic phases were combined, washed with saturated brine, dried over anhydrous sodium sulfate, and filtered. The filtrate was concentrated under reduced pressure. The crude product was purified by column chromatography on silica

gel (petroleum ether: ethyl acetate =60:40) to obtain (R)-4-(t-butoxycarbonyl)-1-(3-cyano-5-(trifluoromethyl)pyridin-2-yl)piperazin-2-carboxylic acid as a yellow solid (1.58 g, 3.95 mmol, yield 45%). LCMS (ESI) m/z: 401[M+H]$^+$

Step II: Synthesis of (R)-1-(3-(aminomethyl)-5-(trifluoromethyl)pyridin-2-yl)-4-(t-butoxycarbonyl)piperazin-2-carboxylic acid

(R)-4-(t-butoxycarbonyl)-1-(3-cyano-5-(trifluoromethyl)pyridin-2-yl)piperazin-2-carboxylic acid (1.58 g, 3.95 mmol) was dissolved in methanol (40 ml), and added with Raney nickel (200 mg, 50%). The mixture was stirred overnight under hydrogen atmosphere at room temperature. After the reaction was completed, the reaction solution was filtered. The filter cake was washed with methanol (20 ml*2), and the filtrate was concentrated under reduced pressure to obtain crude (R)-1-(3-(aminomethyl)-5-(trifluoromethyl)pyridin-2-yl)-4-(t-butoxycarbonyl)piperazin-2-carboxylic acid as a yellow solid (930 mg). The crude product was used in the next reaction without purification. LCMS (ESI) m/z: 405[M+H]$^+$

Step III: Synthesis of t-butyl (R)-7-oxo-3-(trifluoromethyl)-6, 7, 7a, 8, 10, 11-hexahydropyrazino[1, 2-a]pyrido[3, 2-f][1, 4]diazepan-9(5H)-carboxylate

Crude (R)-1-(3-(aminomethyl)-5-(trifluoromethyl)pyridin-2-yl)-4-(t-butoxycarbonyl)piperazin-2-carboxylic acid (930 mg) was dissolved in N,N-dimethylformamide (20 ml). 1-propylphosphonic anhydride (2.24 g, 3.5 mmol, 50% solution in ethyl acetate) and N,N-diisopropylethyl amine (909mg, 7.05mmol) were added to the mixture. The mixture was stirred at room temperature for 1 hr. After the reaction was completed, the reaction solution was poured into water (40 ml), and then extracted with ethyl acetate (20 ml*2). The organic phases were combined, washed with saturated brine, dried over anhydrous sodium sulfate, and filtered. The filtrate was concentrated under reduced pressure. The crude product was purified by column chromatography on silica gel (petroleum ether: ethyl acetate =40:60) to obtain t-butyl (R)-7-oxo-3-(trifluoromethyl)-6, 7, 7a, 8, 10, 11-hexahydropyrazino[1, 2-a]pyrido[3, 2-f][1, 4]diazepan-9(5H)-carboxylate as a white solid (280 mg, 0.73 mmol, yield 31%). LCMS (ESI) m/z: 387[M+H]$^+$

Step IV: Synthesis of t-butyl (R)-6-methyl-7-oxo-3-(trifluoromethyl)-6, 7, 7a, 8, 10, 11-hexahydropyrazino[1, 2-a]pyrido[3, 2-f][1, 4]diazepan-9(5H)-carboxylate

T-butyl (R)-7-oxo-3-(trifluoromethyl)-6, 7, 7a, 8, 10, 11-hexahydropyrazino[1, 2-a]pyrido[3, 2-f][1, 4]diazepan-9(5H)-carboxylate (280 mg, 0.73 mmol) was dissolved in N,N-dimethylformamide (5 ml). Sodium hydride (21 mg, 0.88 mmol, 60%) was added to the mixture at 0°C. After stirring for 1 hr at 0°C, iodomethane (124 mg, 0.88 mmol) was added, and the mixture was further stirred for 1 hr at room temperature. After the reaction was completed, the mixture was poured into ice water (10 ml), and then extracted with ethyl acetate (10 ml*2). The organic phases were combined, washed with saturated brine, dried over anhydrous sodium sulfate, and filtered. The filtrate was concentrated under reduced pressure. The crude product was purified by column chromatography on silica gel (petroleum ether: ethyl acetate =60:40) to obtain t-butyl (R)-6-methyl-7-oxo-3-(trifluoromethyl)-6, 7, 7a, 8, 10, 11-hexahydropyrazino[1, 2-a]pyrido[3, 2-f][1, 4]diazepan-9(5H)-carboxylate as a white solid (78 mg, 0.19 mmol, yield 26%). LCMS (ESI) m/z: 401[M+H]$^+$

Step V: Synthesis of (R)-6-methyl-3-(trifluoromethyl)-5, 6, 8, 9, 10, 11-hexahydropyrazino[1, 2-a]pyrido[3, 2-f][1, 4]diazepan-7(7aH)-one

T-butyl (R)-6-methyl-7-oxo-3-(trifluoromethyl)-6, 7, 7a, 8, 10, 11-hexahydropyrazino[1, 2-a]pyrido[3, 2-f][1, 4]diazepan-9(5H)-carboxylate (78 mg, 0.19 mmol) was dissolved in a hydrogen chloride/dioxane solution (4 mol/l, 2 ml). The mixture was stirred at room temperature for 1 hr. After the reaction was completed, the reaction solution was concentrated under reduced pressure to obtain crude (R)-6-methyl-3-(trifluoromethyl)-5, 6, 8, 9, 10, 11-hexahydropyrazino[1, 2-a]pyrido[3, 2-f][1, 4]diazepan-7(7aH)-one as a yellow solid (43 mg). The crude product was used in the next reaction without purification. LCMS (ESI) m/z: 301[M+H]$^+$

Step VI: Synthesis of (R)-6-methyl-9-(3-((S)-2-((6-oxo-5-(trifluoromethyl)-1, 6-dihydropyridazin-4-yl)amino)propoxy)propionyl)-3-(trifluoromethyl)-5, 6, 8, 9, 10, 11-hexahydropyrazino[1, 2-a]pyrido[3, 2-f][1, 4]diazepan-7(7aH)-one

To a solution of crude (R)-6-methyl-3-(trifluoromethyl)-5, 6, 8, 9, 10, 11-hexahydropyrazino[1, 2-a]pyrido[3, 2-f][1, 4]diazepan-7(7aH)-one (43 mg) in dichloromethane (4 ml), (S)-3-(2-((6-oxo-5-(trifluoromethyl)-1, 6-dihydropyridazin-4-yl)amino)propoxy)propionic acid (68 mg, 0.2 mmol), 1-propylphosphonic anhydride (127 mg, 0.2 mmol, 50% solution in ethyl acetate) and diisopropylethyl amine (77 mg, 0.6 mmol) were added, and the mixture was stirred at room temperature for 2 hrs. After complete reaction, the reaction solution was added with water (10 ml), and extracted with dichloromethane (10 ml*2). The organic phases were combined, washed with saturated brine, dried over anhydrous

sodium sulfate, and filtered. The filtrate was concentrated under reduced pressure. The crude product was purified by high performance liquid chromatography (chromatographic column: XBridge Prep OBD C18, 30 x 150 mm 5 um, mobile phase A: 10 mmol/l ammonium bicarbonate aqueous solution, mobile phase B: acetonitrile, flow rate: 60 ml/ min, elution gradient: mobile phase B from 15% to 54% over 8 min, detection wavelength: 220 nm, retention time: 7.23 min) to obtain (R)-6-methyl-9-(3-((S)-2-((6-oxo-5-(trifluoromethyl)-1, 6-dihydropyridazin-4-yl)amino)propoxy)propionyl)-3-(trifluoromethyl)-5, 6, 8, 9, 10, 11-hexahydropyrazino[1, 2-a]pyrido[3, 2-f][1, 4]diazepan-7(7aH)-one as a white solid (4.56 mg, 0. 007 mmol, yield 5%).

LCMS (ESI) m/z: 592.25 $[M+H]^+$. HNMR (400 MHz, DMSO-$d_6$): δ ppm 12.45(s,1H), 8.44-8.25(m,1H), 7.99-7.84(m,1H), 7.84-7.68(m,1H), 6.38-6.22(m,1H), 5.37-5.26(m,1H), 5.26-5.15(m,1H), 4.55-4.30(m,1H), 4.25-4.08(m,2H), 4.08-3.92(m,1H), 3.78-3.63(m,4H), 3.63-3.53(m,2H), 3.50-3.46(m,2H), 2.94(s,3H), 2.73-2.66(m,2H), 1.20-1.10(m,3H).

**Example 56:** Synthesis of (S)-5-methyl-8-(3-((S)-2-((6-oxo-5-(trifluoromethyl)-1, 6-dihydropyridazin-4-yl)amino)propoxy)propionyl)-3-(trifluoromethyl)-7, 8, 9, 10-tetrahydro-5H-bipyrazino[1, 2-a:2 ', 3 '-e]pyrazin-6(6aH)-one (**Compound 58**)

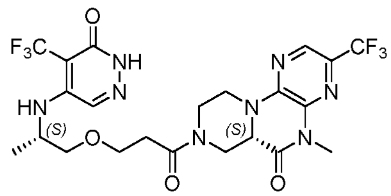

(S)-4-(t-butoxycarbonyl)piperazin-2-carboxylic acid was used as a raw material, and following the same method and steps for the synthesis of **Compound 48, Compound 58 was synthesized.**

LCMS(ESI) m/z: 579.15 $[M+H]^+$. HNMR (400 MHz, DMSO-d6): δ ppm 12.44 (s, 1H), 8.19 (s, 1H), 7.98-7.88 (m, 1H), 6.32-6.21 (m, 1H), 4.90-4.80 (m, 0.5H), 4.68-4.36 (m, 2H), 4.35-4.22 (m, 1H), 4.22-4.06 (m, 1H), 4.06-4.01 (m, 0.5H), 3.76-3.62 (m, 2H), 3.55-3.46 (m, 2H), 3.42-3.35 (m, 1H), 3.31-3.28 (m, 3H), 3.19-3.02 (m, 1H), 2.91-2.83 (m, 1H), 2.70-2.62 (m, 2H), 1.15 (d, J = 6.4 Hz, 3H).

**Example 57:** Synthesis of (S)-2-methoxy-5-methyl-8-(3-((S)-2-((6-oxo-5-(trifluoromethyl)-1, 6-dihydropyridazin-4-yl)amino)propoxy)propionyl)-3-(trifluoromethyl)-7, 8, 9, 10-tetrahydro-5H-pyrazino[1, 2-a]pyrido[3, 2-e]pyrazin-6(6aH)-one (**Compound 59**)

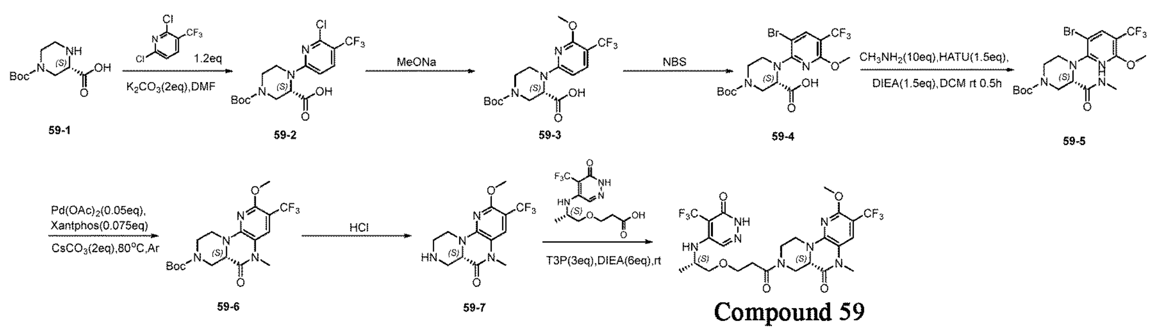


Step I: Synthesis of (S)-4-(t-butoxycarbonyl)-1-(6-chloro-S-(trifluoromethyl)pyridin-2-yl)piperazin-2-carboxylic acid

(S)-4-(t-butoxycarbonyl)piperazin-2-carboxylic acid (2.00 g, 8.69 mmol), 2, 6-dichloro-3-(trifluoromethyl)pyridine (2.25 g, 10.42 mmol) and potassium carbonate (2.40 g, 17.37 mmol) were added to N,N-dimethylformamide (20 ml). The mixture was stirred overnight at 100°C. After complete reaction, the reaction solution was cooled to room temperature, poured into water (50 ml), and adjusted to pH 3-4 with formic acid. A solid was precipitated out, and filtered. The filter cake was dried to obtain (S)-4-(t-butoxycarbonyl)-1-(6-chloro-5-(trifluoromethyl)pyridin-2-yl)piperazin-2-carboxylic acid

as a grey solid (1.49 g, 3.66 mmol, yield 42%). LCMS (ESI) m/z: 410[M+H] +

Step II: Synthesis of (S)-4-(t-butoxycarbonyl)-1-(6-methoxy-5-(trifluoromethyl)pyridin-2-yl)piperazin-2-carboxylic acid

(S)-4-(t-butoxycarbonyl)-1-(6-chloro-5-(trifluoromethyl)pyridin-2-yl)piperazin-2-carboxylic acid (1.49 g, 3.66 mmol) and sodium methoxide (1.98 g, 36.6 mmol) were added to N,N-dimethylformamide (20 ml). The mixture was stirred overnight at 120°C. After complete reaction, the reaction solution was cooled to room temperature, poured into water (50 ml), adjusted to pH 6 with formic acid, and then extracted with ethyl acetate (20 ml*3). The organic phases were combined, washed with saturated brine, dried over anhydrous sodium sulfate, and filtered. The filtrate was concentrated, and the obtained crude product was purified by medium-pressure liquid chromatography (Angela C18 OBD column, water: acetonitrile=60: 40) to obtain (S)-4-(t-butoxycarbonyl)-1-(6-methoxy-5-(trifluoromethyl)pyridin-2-yl)piperazin-2-carboxylic acid as a yellow oil (500 mg, 1.23 mmol, yield 33%). LCMS (ESI) m/z: 406[M+H] +

Step III: Synthesis of (S)-1-(3-bromo-6-methoxy-5-(trifluoromethyl)pyridin-2-yl)-4-(t-butoxycarbonyl)piperazin-2-carboxylic acid

(S)-4-(t-butoxycarbonyl)-1-(6-methoxy-5-(trifluoromethyl)pyridin-2-yl)piperazin-2-carboxylic acid (500 mg, 1.23 mmol) was added to dichloromethane (5 ml), and then N-bromosuccinimide (505 mg, 2.84 mmol) was added batchwise. The mixture was stirred at room temperature for 1 hr. After the reaction was completed, the reaction solution was poured into water (20 ml), and extracted with dichloromethane (10 ml*2). The organic phases were combined, washed with saturated brine, dried over anhydrous sodium sulfate, and filtered. The filtrate was concentrated. The crude product was purified by column chromatography on silica gel (dichloromethane : methanol=80: 20) to obtain (S)-1-(3-bromo-6-methoxy-5-(trifluoromethyl)pyridin-2-yl)-4-(t-butoxycarbonyl)piperazin-2-carboxylic acid as a yellow oil (300 mg, 0.62 mmol, yield 50%). LCMS (ESI) m/z: 484[M+H] +

Step IV: Synthesis of t-butyl (S)-4-(3-bromo-6-methoxy-5-(trifluoromethyl)pyridin-2-yl)-3-(methylaminoformyl)piperazin-1-carboxylate

(S)-1-(3-bromo-6-methoxy-5-(trifluoromethyl)pyridin-2-yl)-4-(t-butoxycarbonyl)piperazin-2-carboxylic acid (300 mg, 0.62 mmol), methylamine hydrochloride (418 mg, 6.2 mmol,), N,N,N',N'-tetramethyl-O-(7-azabenzotriazole -1-yl)uronium hexafluorophosphate (353 mg, 0.93 mmol) and N,N-diisopropylethyl amine (160 mg, 1.24 mmol) were added to dichloromethane (5 ml). The mixture was stirred at room temperature for 0.5 hr. After complete reaction, the mixture was poured into water (10 ml), and extracted with dichloromethane (5 ml*2). The organic phases were combined, washed with saturated brine, dried over anhydrous sodium sulfate, and filtered. The filtrate was concentrated. The crude product was purified by column chromatography on silica gel (petroleum ether: ethyl acetate =85: 15) to obtain t-butyl (S)-4-(3-bromo-6-methoxy-5-(trifluoromethyl)pyridin-2-yl)-3-(methylaminoformyl)piperazin-1-carboxylate as a green oil (220 mg, 0.44 mmol, yield 70%). LCMS (ESI) m/z: 497[M+H] +

Step V: Synthesis of t-butyl (S)-2-methoxy-5-methyl-6-oxo-3-(trifluoromethyl)-5, 6, 6a, 7, 9, 10-hexahydro-8H-pyrazino[1, 2-a]pyrido[3, 2-e]pyrazin-8-carboxylate

T-butyl (S)-4-(3-bromo-6-methoxy-5-(trifluoromethyl)pyridin-2-yl)-3-(methylaminoformyl)piperazin-1-carboxylate (220 mg, 0.44 mmol), palladium acetate (5 mg, 0.022 mmol), 4,5-bis(diphenylphosphino)-9,9-dimethylxanthene (19 mg, 0.033 mmol), and cesium carbonate (288 mg, 0.88 mmol) were added to dioxane (5 ml). The mixture was reacted for 0.5 hr under argon atmosphere at 80°C. After the reaction was completed, the reaction solution was cooled to room temperature. The mixture was poured into water (10 ml), and then extracted with ethyl acetate (10 ml*3). The organic phases were combined, washed with saturated brine, dried over anhydrous sodium sulfate, and filtered. The filtrate was concentrated. The crude product was purified by column chromatography on silica gel (petroleum ether: ethyl acetate =85: 15) to obtain t-butyl (S)-2-methoxy-5-methyl-6-oxo-3-(trifluoromethyl)-5, 6, 6a, 7, 9, 10-hexahydro-8H-pyrazino[1, 2-a]pyrido[3, 2-e]pyrazin-8-carboxylate as a yellow oil (120 mg, 0.29 mmol, yield 65%). LCMS (ESI) m/z:417[M+H] +

Step VI: Synthesis of (S)-2-methoxy-5-methyl-3-(trifluoromethyl)-7, 8, 9, 10-tetrahydro-5H-pyrazino[1, 2-a]pyrido[3, 2-e]pyrazin-6(6aH)-one

T-butyl (S)-2-methoxy-5-methyl-6-oxo-3-(trifluoromethyl)-5, 6, 6a, 7, 9, 10-hexahydro-8H-pyrazino[1, 2-a]pyrido[3, 2-e]pyrazin-8-carboxylate (120 mg, 0.29 mmol) was added to a hydrogen chloride/dioxane solution (2 ml), and the mixture was reacted for 0.5 hr at room temperature. After the reaction was completed, the mixture was concentrated under reduced pressure to obtain crude (S)-2-methoxy-5-methyl-3-(trifluoromethyl)-7, 8, 9, 10-tetrahydro-5H-pyrazino[1,

2-a]pyrido[3, 2-e]pyrazin-6(6aH)-one as a yellow solid (80 mg). LCMS (ESI) m/z:317[M+H] +

Step VII: Synthesis of (S)-2-methoxy-5-methyl-8-(3-((S)-2-((6-oxo-5-(trifluoromethyl)-1, 6-dihydropyridazin-4-yl)amino)propoxy)propionyl)-3-(trifluoromethyl)-7, 8, 9, 10-tetrahydro-5H-pyrazino[1, 2-a]pyrido[3, 2-e]pyrazin-6(6aH)-one

Crude (S)-2-methoxy-5-methyl-3-(trifluoromethyl)-7, 8, 9, 10-tetrahydro-5H-pyrazino[1, 2-a]pyrido[3, 2-e]pyrazin-6(6aH)-one (60 mg), (S)-3-(2-((6-oxo-5-(trifluoromethyl)-1, 6-dihydropyridazin-4-yl)amino)propoxy)propionic acid (59 mg, 0.19 mmol) and N,N-diisopropylethyl amine (147 mg, 1.14 mmol) was sequentially added to N,N-dimethylformamide (2 ml). Then, 1-propylphosphonic anhydride (262 mg, 0.57 mmol, 50% solution in ethyl acetate) was added. The mixture was stirred at room temperature for 0.5 hr. After the reaction was completed, the mixture was poured into water (10 ml), and then extracted with ethyl acetate (5 ml*3). The organic phases were combined, washed with saturated brine, dried over anhydrous sodium sulfate, and filtered. The filtrate was concentrated, and the obtained crude product was purified by high performance liquid chromatography (chromatographic column: CSH C18 OBD column 30 x 150 mm 5 μm; mobile phase A: 10 mmol/l ammonium bicarbonate aqueous solution, mobile phase B: acetonitrile; flow rate: 60 ml/ min; gradient: 25% B to 55% B over 10 min; wavelength: 220 nm; retention time: 8.32 min) to obtain (S)-2-methoxy-5-methyl-8-(3-((S)-2-((6-oxo-5-(trifluoromethyl)-1, 6-dihydropyridazin-4-yl)amino)propoxy)propionyl)-3-(trifluoromethyl)-7, 8, 9, 10-tetrahydro-5H-pyrazino[1, 2-a]pyrido[3, 2-e]pyrazin-6(6aH)-one as a white solid (10.22 mg, 0.016 mmol, yield 8%).

LCMS (ESI) m/z: 608.15[M+H] +. HNMR (400 MHz, DMSO-$d_6$): δ ppm 12.60-12.25 (m, 1H), 8.07-7.75 (m, 1H), 7.40 (s, 1H), 6.27 (s, 1H), 4.96-4.41 (m, 2H), 4.34-3.99(m, 3H), 3.92 (s, 3H), 3.77-3.65 (m, 2H), 3.54-3.47 (m, 2H), 3.27 (s, 3H), 3.25-3.14 (m, 1H), 3.15-2.83 (m, 1H), 2.75-2.61 (m, 3H), 1.44-0.94 (m, 3H).

**Example 58:** Synthesis of 5-((S)-1-(3-oxo-3-((S)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one (**Compound 60**) and 5-(((S)-1-(3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one **(Compound 61)**

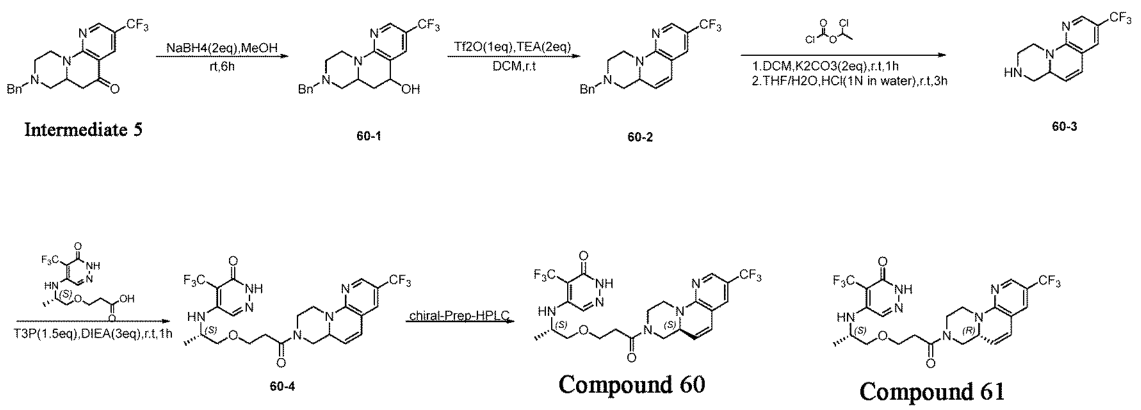


Intermediate 5 60-1 60-2 60-3

60-4 Compound 60 Compound 61

Step I: Synthesis of 8-benzyl-3-(trifluoromethyl)-6, 6a, 7, 8, 9, 10-hexahydro-5H-pyrazino[1, 2-a][1, 8]naphthyridin-5-ol

8-benzyl-3-(trifluoromethyl)-6, 6a, 7, 8, 9, 10-hexahydro-5H-pyrazino[1, 2-a][1, 8]naphthyridin-5-one (**Intermediate 5,** 660 mg, 1.83 mmol) was dissolved in anhydrous methanol (2 ml), and sodium borohydride (138 mg, 3.66 mmol) was added at 0°C. The mixture was stirred at room temperature for 6 hrs. After the reaction was completed, the reaction solution was quenched with a sodium bicarbonate aqueous solution (10 ml), and then extracted with dichloromethane (15 ml*2). The organic phases were combined, washed with saturated brine (15 ml*2), dried over anhydrous sodium sulfate and then filtered. The filtrate was concentrated under reduced pressure to obtain a crude product. The crude product was purified by column chromatography on silica gel (petroleum ether: ethyl acetate =40:60) to obtain 8-benzyl-3-(trifluoromethyl)-6, 6a, 7, 8, 9, 10-hexahydro-SH-pyrazino[1, 2-a][1, 8]naphthyridin-5-ol as a yellow oil (580 mg, 1.59 mmol, yield 86%). LCMS(ESI)m/z: 364[M+H]+

Step II: Synthesis of 8-benzyl-3-(trifluoromethyl)-6a, 7, 9, 10-tetrahydro-8H-pyrazino[1, 2-a][1, 8]naphthyridine

8-benzyl-3-(trifluoromethyl)-6, 6a, 7, 8, 9, 10-hexahydro-5H-pyrazino[1, 2-a][1, 8]naphthyridin-5-ol (580 mg,

1.59 mmol) was dissolved in dichloromethane (2 ml), and triethyl amine (0.44 ml, 3.18 mmol) and trifluoromethanesulfonic anhydride (448 mg, 1.59 mmol) were added dropwise to the solution. The mixture was stirred overnight at room temperature. After the reaction was completed, the reaction solution was quenched with water (10 ml*2) and dichloromethane (15 ml*2). The organic phases were combined, washed with saturated brine (10 ml*2), dried over anhydrous sodium sulfate and filtered. The filtrate was concentrated under reduced pressure to obtain a crude product. The crude product was purified by column chromatography on silica gel (petroleum ether: ethyl acetate =40:60) to obtain 8-benzyl-3-(trifluoromethyl)-6a, 7, 9, 10-tetrahydro-8H-pyrazino[1, 2-a][1, 8]naphthyridine as a yellow oil (200 mg, 0.58 mmol, yield 36%). LCMS (ESI) m/z: 346[M+H]+

Step III: Synthesis of 3-(trifluoromethyl)-6a, 7, 9, 10-tetrahydro-8H-pyrazino[1, 2-a][1, 8]naphthyridine

8-benzyl-3-(trifluoromethyl)-6a, 7, 9, 10-tetrahydro-8H-pyrazino[1, 2-a][1, 8]naphthyridine (200 mg, 0.58 mmol) was dissolved in dichloromethane (5 ml), and then potassium carbonate (160 mg, 1.16 mmol) and 1-chloroethyl chloroformate (249 mg, 1.74 mmol) were added. The mixture was stirred at room temperature for 1 hr, and then tetrahydrofuran (2 ml) and 1 mol/l dilute hydrochloric acid (2 ml) were added. The mixture was further stirred for 3 hrs at room temperature. After the reaction was completed, the reaction solution was extracted with water (10 ml*2) and dichloromethane (15 ml*2). The organic layers were combined, and washed with saturated brine (10 ml*2). The organic phase was dried over anhydrous sodium sulfate and then filtered. The filtrate was concentrated under reduced pressure to obtain a crude product. The crude product was purified by column chromatography on silica gel (petroleum ether: ethyl acetate =30:70) to obtain 3-(trifluoromethyl)-6a, 7, 9, 10-tetrahydro-8H-pyrazino[1, 2-a][1, 8]naphthyridine as a yellow oil (140 mg, 0.55 mmol, yield 94%). LCMS (ESI) m/z: 256[M+H]+

Step IV: Synthesis of 5-(((2S)-1-(3-oxo-3-(3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin- 3 (2H) -one

3-(trifluoromethyl)-6a, 7, 9, 10-tetrahydro-8H-pyrazino[1, 2-a][1, 8]naphthyridine (140 mg, 0.55 mmol) and (S)-3-(2-((6-oxo-5-(trifluoromethyl)-1, 6-dihydropyridazin-4-yl)amino)propoxy)propionic acid (168 mg, 0.55 mmol) were dissolved in dichloromethane (2 ml), and then 1-propylphosphonic anhydride (516 mg, 0.31 mmol, 50% solution in ethyl acetate) and N,N-diisopropylethyl amine (212 mg, 1.65 mmol) were added. The mixture was stirred at room temperature for 2 hrs, and then the reaction solution was added with water (10 ml), and extracted with dichloromethane (5 ml x 3). The organic layer was collected, washed with saturated brine, dried over anhydrous sodium sulfate, and filtered. The filtrate was concentrated, and the obtained crude product was purified by high performance liquid chromatography (chromatographic column: CSH C18 OBD column 30 x 150 mm 5 μm, n; mobile phase A: 10 mmol/l ammonium bicarbonate aqueous solution, mobile phase B: acetonitrile; flow rate: 60 ml/ min; gradient: 8% B to 66% B over 8 min; wavelength: 220 nm; retention time: 7.56 min) to obtain 5-(((2S)-1-(3-oxo-3-(3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one as a white solid (80 mg, 0.15 mmol, yield 27%). LCMS (ESI) m/z: 547[M+H]+

Step V: Synthesis of 5-((S)-1-(3-oxo-3-((S)-3-(trifluoromethyl)-6a, 7, 9, 10-tetrahydro-8H-pyrazino[1, 2-a] [1, 8]naphthyridin-8-yl)propoxy)prop-2-yl)amino)-4-(trifluoromethyl)pyridazin-3(2H)-one (**Compound 60**) and 5-(((S)-1-(3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one (**Compound 61**)

5-(((2S)-1-(3-oxo-3-(3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one (80 mg, 0.15 mmol) was purified by chiral preparative chromatography (chromatographic column: CHIRAL ART Cellulose-SB, 2*25 cm, 5 μm, mobile phase A: methyl t-butyl ether (0.1% diethyl amine), mobile phase B: methanol, flow rate: 20 ml/ min, elution gradient: mobile phase B from 10% to 10% over 10 min, detection wavelength: 220 nm) to obtain the first peak 5-((S)-1-(3-oxo-3-((S)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one as a yellow solid (9.6 mg, 0.017 mmol, yield 11.51%) and the second peak 5-(((S)-1-(3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one as a yellow solid (11.98 mg, 0.021 mmol, yield 14.7%).

First peak (**Compound 60**): 5-((S)-1-(3-oxo-3-((S)-3-(trifluoromethyl)-6a, 7, 9, 10-tetrahydro-8H-pyrazino[1, 2-a] [1, 8]naphthyridin-8-yl)propoxy)prop-2-yl)amino)-4-(trifluoromethyl)pyridazin- 3 (2H) -one

LCMS (ESI) m/z: 547.10[M+H]+

HNMR (400 MHz, DMSO-$d_6$): δ ppm 12.42 (s,1H), 8.19-8.03 (m,1H), 7.90 (s,1H), 7.48-7.30 (m,1H), 6.54-6.43

(m,1H), 6.36-6.17 (m,1H), 5.82-5.63 (m,1H), 4.83-4.69 (m,1H), 4.62-4.32 (m,2H), 4.21-4.06 (m,1H), 3.97-3.84 (m,1H), 3.74-3.62 (m,2H), 3.51-3.47 (m,2H), 3.23-3.15 (m,1H), 3.13-3.01 (m,1H), 2.81-2.70 (m,1H), 2.65-2.61 (m,1H), 2.58-2.55 (m,1H), 1.17 (s,3H).

Chiral HPLC: chromatographic column CHIRAL ART Cellulose-SB 4.6*100 mm, 3 um; mobile phase A: methyl t-butyl ether (0.1% diethyl amine), mobile phase B: methanol, mobile phase A: mobile phase B=90: 10; flow rate: 1.0 ml/ min; temperature: 25°C; retention time: 2.90 min.

Second peak (**Compound 61**): 5-((S)-1-(3-oxo-3-((S)-3-(trifluoromethyl)-6a, 7, 9, 10-tetrahydro-8H-pyrazino[1, 2-a] [1, 8]naphthyridin-8-yl)propoxy)prop-2-yl)amino)-4-(trifluoromethyl)pyridazin- 3 (2H) -one

LCMS (ESI) m/z: 547.15[M+H]+

HNMR (400 MHz, DMSO-$d_6$): δ ppm 12.42 (s,1H), 8.21-8.01 (m,1H), 7.90 (s,1H), 7.55-7.28 (m,1H), 6.66-6.35 (m,1H), 6.35-6.05 (m,1H), 5.82-5.62 (m,1H), 4.86-4.67 (m,1H), 4.63-4.27 (m,2H), 4.21-4.04 (m,1H), 3.98-3.83 (m,1H), 3.78-3.61 (m,2H), 3.56-3.45 (m,2H), 3.25-3.14 (m,1H), 3.14-2.96 (m,1H), 2.81-2.71 (m,1H), 2.64-2.60 (m,1H), 2.58-2.55 (m,1H) ,1.14 (s,3H).

Chiral HPLC: chromatographic column CHIRAL ART Cellulose-SB 4.6*100 mm, 3 um; mobile phase A: methyl t-butyl ether (0.1% diethyl amine), mobile phase B: methanol, mobile phase A: mobile phase B=90: 10; flow rate: 1.0 ml/ min; temperature: 25°C; retention time: 3.39 min.

**Example 59**: Synthesis of 5-(((S)-1-(3-((S)-4-(difluoromethyl)-3-(trifluoromethyl)-6, 7, 7a, 8, 10, 11-hexahydro-9H-pyrazino[1, 2-d]pyrido[3, 2-b][1, 4]oxazepane-9-yl)-3-oxypropoxy)prop-2-yl)amino)-4-(trifluoromethyl)pyridazin-3(2H)-one (**Compound 62**)

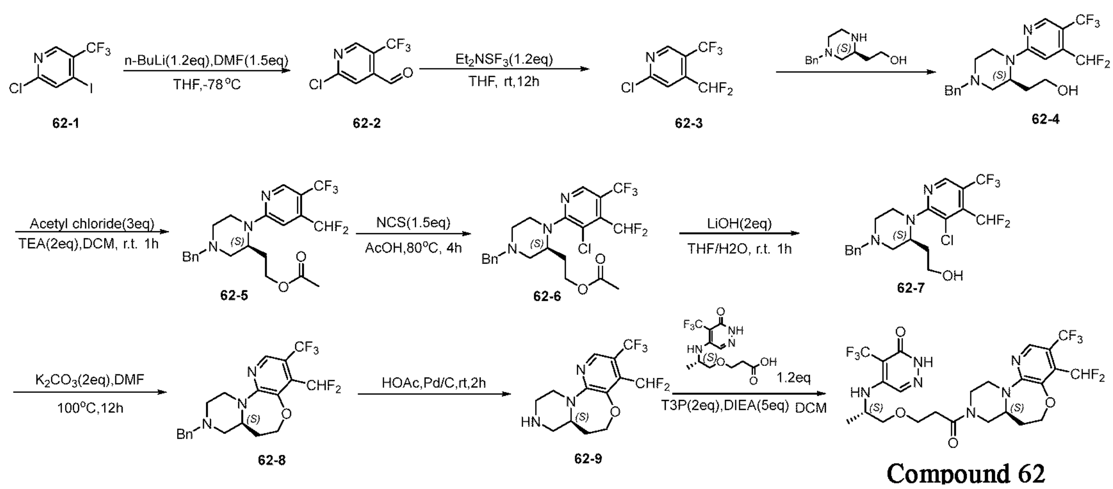


Step I: Synthesis of 2-chloro-5-(trifluoromethyl)isonicotinaldehyde

Chloro-4-iodo-5-(trifluoromethyl)pyridine (10.00 g, 32.53 mmol) was dissolved in tetrahydrofuran (100.00 ml). The solution was cooled to -78°C, and then n-butyl lithium (15.61 ml, 39.03 mmol, 2.5 mol/l solution in n-hexane) was added. The mixture was stirred at -78°C for 1 hr. At -78°C, dimethyl formamide (3.56 g, 48.79 mmol) was added to the mixture. The reaction was stirred at -78°C for 6 hours. The reaction was quenched with saturated ammonium chloride solution, and extracted with ethyl acetate (50 ml*3). The organic layers were combined, dried over anhydrous sodium sulfate, and filtered. The filtrate was concentrated, and the crude product was purified by column chromatography on silica gel (petroleum ether: ethyl acetate =70:30) to obtain 2-chloro-5-(trifluoromethyl)isonicotinaldehyde as a yellow solid (3.90 g, 32.527 mmol, yield 57%). LCMS(ESI)m/z: 210[M+H]+

Step II: Synthesis of 2-chloro-4-(difluoromethyl)-5-(trifluoromethyl)pyridine

(2-Chloro-5-(trifluoromethyl)isonicotinaldehyde (3.40 g, 16.23 mmol) was added to dichloromethane (40.00 ml), the reaction solution was cooled to 0°C, and then diethylaminosulfur trifluoride (7.85 g, 48.68 mmol) was added. The mixture was stirred at room temperature for 16 hrs, and the reaction was quenched with a saturated sodium bicarbonate aqueous solution and then extracted with dichloromethane (50 ml*3). The organic layers were combined, dried over anhydrous sodium sulfate, and filtered. The filtrate was concentrated, to obtain crude 2-chloro-4-(difluoromethyl)-5-(trifluoromethyl)pyridine as a yellow oil (3.60 g). The crude product was directly used in the next step without purification. LCMS (ESI) m/z: **232**$[M+H]^+$

Step III: Synthesis of (S)-2-(4-benzyl-1-(4-(difluoromethyl)-5-(trifluoromethyl)pyridin-2-yl)piperazin-2-yl)ethyl-1 -ol

Chloro-4-(difluoromethyl)-5-(trifluoromethyl)pyridine (2.00 g, 8.64 mmol) was added to dimethylformamide (20.00 ml), and then (S)-2-(4-benzylpiperazin-2-yl)ethyl-1-ol (1.90 g, 8.64 mmol) and N,N-diisopropylethyl amine (2.23 g, 17.27 mmol) were added to the solution. The reaction was stirred for 16 hrs at 80°C. After the reaction was completed, the system was cooled to room temperature, and the reaction solution was poured into water (40 ml), and then extracted with ethyl acetate (20 ml*3). The organic layers were combined, dried over anhydrous sodium sulfate, and filtered. The filtrate was concentrated, and the crude product was purified by column chromatography on silica gel (petroleum ether: ethyl acetate =60:40) to obtain (S)-2-(4-benzyl-1-(4-(difluoromethyl)-5-(trifluoromethyl)pyridin-2-yl)piperazin-2-yl)ethyl-1-ol as a yellow solid (1.20g, 8.63mmol, yield: 33%). LCMS: MS(ESI)m/z: 416 M+H]+

Step IV: Synthesis of (S)-2-(4-benzyl-1-(4-(difluoromethyl)-5-(trifluoromethyl)pyridin-2-yl)piperazin-2-yl)ethyl acetate

(S)-2-(4-benzyl-1-(4-(difluoromethyl)-5-(trifluoromethyl)pyridin-2-yl)piperazin-2-yl)ethyl-1-ol (220 mg, 0.53 mmol) was dissolved in dichloromethane (3.00 ml). Acetyl chloride (124 mg, 1.59 mmol) and triethyl amine (106 mg, 1.06 mmol) were added to the mixed solution. The reaction was stirred at room temperature for 1 hr. The was quenched with a saturated ammonium chloride aqueous solution, and then extracted with ethyl acetate (20 ml*3). The organic layers were combined, dried over anhydrous sodium sulfate, and filtered. The filtrate was concentrated to obtain crude (S)-2-(4-benzyl-1-(4-(difluoromethyl)-5-(trifluoromethyl)pyridin-2-yl)piperazin-2-yl)ethyl acetate as a yellow oil (180 mg). LCMS: MS(ESI)m/z: 458 M+H]+

Step V: Synthesis of (S)-2-(4-benzyl-1-(3-chloro-4-(difluoromethyl)-5-(trifluoromethyl)pyridin-2-yl)piperazin-2-yl)ethyl acetate

(S)-2-(4-benzyl-1-(4-(difluoromethyl)-5-(trifluoromethyl)pyridin-2-yl)piperazin-2-yl)ethyl acetate (750 mg, 1.64 mmol) was added to acetic acid (7.00 ml), and then N-chlorosuccinimide (545 mg, 4.10 mmol) was added to the solution. The reaction was stirred at room temperature for 16 hr. The mixture was poured into water (10 ml), and then extracted with ethyl acetate (20 ml*3). The organic layers were combined, dried over anhydrous sodium sulfate, and filtered. The filtrate was concentrated, and the crude product was purified by column chromatography on silica gel (petroleum ether: ethyl acetate =80:20) to obtain (S)-2-(4-benzyl-1-(3-chloro-4-(difluoromethyl)-5-(trifluoromethyl)pyridin-2-yl)piperazin-2-yl)ethyl acetate as a yellow oil (220 mg, 1.64 mmol, yield: 27%). LCMS: MS(ESI)m/z: 492 M+H]+

Step VI: Synthesis of (S)-2-(4-benzyl-1-(3-chloro-4-(difluoromethyl)-5-(trifluoromethyl)pyridin-2-yl)piperazin-2-yl)ethyl-1 -ol

(S)-2-(4-benzyl-1-(3-chloro-4-(difluoromethyl)-5-(trifluoromethyl)pyridin-2-yl)piperazin-2-yl)ethyl acetate (220 mg, 0.45 mmol) were added to a mixed solution of tetrahydrofuran (3.00 ml) and water (1.50 ml), and then lithium hydroxide monohydrate (38 mg, 0.89 mmol) were added to the mixed solution. The reaction was stirred at room temperature for 1 hr. The mixture was poured into water (5.00 ml), and then adjusted to pH 6 with 1 mol/l hydrochloric acid. The reaction solution was then extracted with ethyl acetate (20 ml*3). The organic layers were combined, dried over anhydrous sodium sulfate, and filtered. The filtrate was concentrated, and the crude product was purified by column chromatography on silica gel (petroleum ether: ethyl acetate =60:40) to obtain (S)-2-(4-benzyl-1-(3-chloro-4-(difluoromethyl)-5-(trifluoromethyl)pyridin-2-yl)piperazin-2-yl)ethyl-1-ol as a yellow oil (100 mg, 0.45 mmol, yield: 50%). LCMS: MS(ESI)m/z: 450 M+H]+

Step VII: Synthesis of (S)-9-benzyl-4-(difluoromethyl)-3-(trifluoromethyl)-7, 7a, 8, 9, 10, 11-hexahydro-6H-pyrazino[1, 2-d]pyrido[3, 2-b][1, 4]oxazepane

(S)-2-(4-benzyl-1-(3-chloro-4-(difluoromethyl)-5-(trifluoromethyl)pyridin-2-yl)piperazin-2-yl)ethyl-1-ol (95 mg, 0.21 mmol) was added to dimethylformamide (10.00 ml), and then potassium carbonate (60 mg, 0.43 mmol) was added to the mixed solution. The reaction was stirred for 16 hrs at 80°C, and the mixture was poured into water (20 ml), and then extracted with ethyl acetate (20 ml*3). The organic layers were combined, dried over anhydrous sodium sulfate, and filtered. The filtrate was concentrated and the obtained crude product was purified by column chromatography on

silica gel (petroleum ether: ethyl acetate =75:25) to obtain (S)-9-benzyl-4-(difluoromethyl)-3-(trifluoromethyl)-7, 7a, 8, 9, 10, 11-hexahydro-6H-pyrazino[1, 2-d]pyrido[3, 2-b][1, 4] oxazepane as a yellow oil (80 mg, 0.21mmol, yield: 92%). LCMS: MS(ESI) m/z: 414 M+H]+

Step VIII: Synthesis of (S)-4-(difluoromethyl)-3-(trifluoromethyl)-7, 7a, 8, 9, 10, 11-hexahydro-6H-pyrazino[1, 2-d]pyrido[3, 2-b][1, 4]oxazepane

(S)-9-benzyl-4-(difluoromethyl)-3-(trifluoromethyl)-7, 7a, 8, 9, 10, 11-hexahydro-6H-pyrazino[1, 2-d]pyrido[3, 2-b][1, 4] oxazepane (75 mg, 0.18 mmol) was added to acetic acid (75.00 ml), and then aqueous palladium on carbon (15 mg, 20 wt%, 10%) was added to the mixed solution. The reaction solution was stirred for 2 hrs at room temperature, and filtered to remove palladium on carbon. The filtrate was concentrated under reduced pressure to obtain a crude product, and the obtained crude product was purified by reverse phase chromatography (water: acetonitrile=25:75) to obtain (S)-4-(difluoromethyl)-3-(trifluoromethyl)-7, 7a, 8, 9, 10, 11-hexahydro-6H-pyrazino[1, 2-d]pyrido[3, 2-b][1, 4] oxazepane as a yellow oil (40 mg, 0.12 mmol, yield: 68%). LCMS: MS(ESI) m/z: 324 M+H]+

Step IX: Synthesis of 5-(((S)-1-(3-(S)-4-(difluoromethyl)-3-(trifluoromethyl)-6, 7, 7a, 8, 10, 11-hexahydro-9H-pyrazino[1, 2-d]pyrido[3, 2-b][1, 4]oxazepane-9-yl)-3-oxypropoxy)prop-2-yl)amino)-4-(trifluoromethyl)pyridazin-3(2H)-one

(S)-4-(difluoromethyl)-3-(trifluoromethyl)-7, 7a, 8, 9, 10, 11-hexahydro-6H-pyrazino[1, 2-d]pyrido[3, 2-b][1, 4] oxazepane (35 mg, 0.11 mmol) was added to dichloromethane (5.00 ml), and then (S)-3-(2-((6-oxo-5-(trifluoromethyl)-1, 6-dihydropyridazin-4-yl)amino)propoxy)propionic acid (41 mg, 0.14 mmol), propylphosphonic anhydride (140 mg, 0.22 mmol), and N,N-diisopropylethyl amine (28 mg, 0.22 mmol) were added. The mixture was stirred at room temperature for 2 hrs, and then the reaction solution was added with water (10 ml), and extraced with dichloromethane (5 ml x 3). The organic layer was collected, dried over anhydrous sodium sulfate and filtered. The filtrate was concentrated, and the obtained crude product was purified by high performance liquid chromatographic column (chromatographic column: CSH OBD column 30 * 150 mm 5 μm; mobile phase A: water (containing 0.05% formic acid), mobile phase B: acetonitrile; flow rate: 60 ml/ min; gradient: 5% B to 35% B over 8 min; wavelength: 220 nm; retention time: 7.3 min) to obtain 5-(((S)-1-(3-(S)-4-(difluoromethyl)-3-(trifluoromethyl)-6, 7, 7a, 8, 10, 11-hexahydro-9H-pyrazino[1, 2-d]pyrido[3, 2-b][1, 4]oxazepane-9-yl)-3-oxypropoxy)prop-2-yl)amino)-4-(trifluoromethyl)pyridazin-3(2H)-one as a white solid (5.64 mg, 0.1 mmol, yield: 8%).

LCMS (ESI) m/z: 615.10 [M+H]+. HNMR (400 MHz, DMSO-$d_6$): δ ppm 12.25 (s, 1H), 8.22 (s, 1H), 7.83 (s, 1H), 7.38-6.95 (m, 1H), 6.12-5.81 (m, 1H), 4.42-4.36 (m, 1H), 4.36-4.27 (m, 1H), 4.15-4.05 (m, 2H), 3.97-3.85 (m, 2H), 3.79-3.65 (m, 4H), 3.57-3.40 (m, 4H), 2.58-2.54 (m, 2H), 2.21-2.08 (m, 1H), 2.04-1.93 (m, 1H), 1.22-1.13 (m, 3H).

**Example 60:** Synthesis of 5-(((S)-1-(3-((S)-5, 5-difluoro-3-(trifluoromethyl)-5, 6, 6a, 7, 9, 10-hexahydro-8H-pyrazino[1, 2-a][1, 8]naphthyridin-8-yl)-3-oxopropoxy)prop-2-yl)amino)-4-(trifluoromethyl)pyridazin-3(2H)-one **(Compound 63)** and 5-(((S)-1-(3-((R)-5, 5-difluoro-3-(trifluoromethyl)-5, 6, 6a, 7, 9, 10-hexahydro-8H-pyrazino[1, 2-a][1, 8]naphthyridin-8-yl)-3-oxopropoxy)prop-2-yl)amino)-4-(trifluoromethyl)pyridazin-3(2H)-one **(Compound 64)**

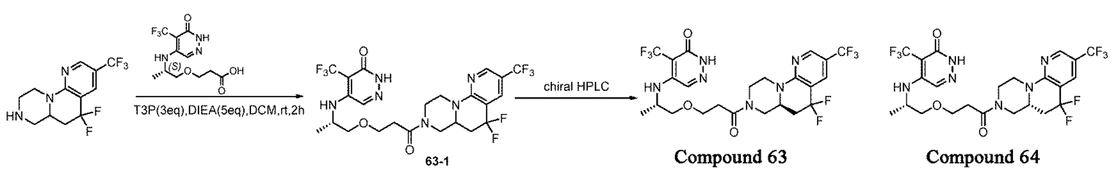


(S)-3-(2-((6-oxo-5-(trifluoromethyl)-1, 6-dihydropyridazin-4-yl)amino)propoxy)propionic acid **(Intermediate 1,** 105 mg, 0.34 mmol) and 5, 5-difluoro-3-(trifluoromethyl)-6, 6a, 7, 8, 9, 10-hexahydro-5H-pyrazino[1, 2-a][1, 8]naphthyridine (100 mg, 0.34 mmol) were dissolved in dichloromethane (5.0 ml), and then N,N-diisopropylethyl amine (131 mg, 1.02 mmol) and 1-propylphosphonic anhydride (324 mg, 0.51mmol, 50% solution in ethyl acetate) were added. The reaction mixture was stirred at room temperature for 2 hrs. After complete reaction, the reaction solution was poured into ice water (20 ml), and then the aqueous phase was extracted with ethyl acetate (10 ml x 3). The organic phases were combined, and dried over anhydrous sodium sulfate. Anhydrous sodium sulfate was removed by filtration, and the filtrate was rotary dried to obtain a crude product. The crude product was purified by high performance liquid chromatography to obtain 40 mg of a diastereomeric mixture **63-1,** which was resolved by chiral preparative HPLC (CHIRAL ART Cellulose-SB, 2*25 cm, 5 μm; mobile phase A: methyl t-butyl ether (containing 0.1% diethyl amine), mobile phase

B: methanol; flow rate: 20 ml/min; gradient: 10% B to 10% B over 12.5 min; wavelength: 220/254 nm; retention time: 6.59 min, 7.56 min) to obtain 5-(((S)-1-(3-((S)-5, 5-difluoro-3-(trifluoromethyl)-5, 6, 6a, 7, 9, 10-hexahydro-8H-pyrazino[1, 2-a][1, 8]naphthyridin-8-yl)-3-oxopropoxy)prop-2-yl)amino)-4-(trifluoromethyl)pyridazin-3(2H)-one as a white solid (11.63 mg, 0.020 mmol, yield 5.9%), and 5-(((S)-1-(3-((R)-5, 5-difluoro-3-(trifluoromethyl)-5, 6, 6a, 7, 9, 10-hexahydro-8H-pyrazino[1, 2-a][1, 8]naphthyridin-8-yl)-3-oxopropoxy)prop-2-yl)amino)-4-(trifluoromethyl)pyridazin-3(2H)-one as a white solid (7.77 mg, 0.013 mmol, yield 3.8%).

First peak **(Compound 63):** 5-(((S)-1-(3-((S)-5, 5-difluoro-3-(trifluoromethyl)-5, 6, 6a, 7, 9, 10-hexahydro-8H-pyrazino[1, 2-a][1, 8]naphthyridin-8-yl)-3-oxopropoxy)prop-2-yl)amino)-4-(trifluoromethyl)pyridazin-3(2H)-one

LCMS (ESI) m/z: 585.10[M+H]$^+$

HNMR (400 MHz, DMSO-$d_6$): δ ppm 12.45 (s, 1H), 8.61 (s, 1H), 8.32 (brs, 1H), 8.05 (s, 1H), 7.91 (s, 1H), 6.26-6.25 (m, 1H), 4.69-4.44 (m, 2H), 4.19-4.02 (m, 2H), 3.72-3.67 (m, 1H), 3.56-3.52 (m, 1H), 3.50-3.49 (m, 2H), 3.23-3.17 (m, 1H), 2.94-2.85 (m, 2H), 2.82-2.77 (m, 1H), 2.73-2.65 (m, 1H), 2.63-2.61 (m, 1H), 2.35-2.24 (m, 1H), 1.18-1.15 (m, 3H).

Chiral chromatography: chromatographic column: CHIRAL ART Cellulose-SB, 4.6*100 cm, 3 μm; mobile phase A: methyl t-butyl ether (0.1% diethyl amine), mobile phase B: methanol; mobile phase A: mobile phase B= 90: 10; flow rate: 1.0 ml/ min; temperature: 25°C; wavelength: 220/254 nm; retention time: 4.19 min.

Second peak **(Compound 64):** 5-(((S)-1-(3-((R)-5, 5-difluoro-3-(trifluoromethyl)-5, 6, 6a, 7, 9, 10-hexahydro-8H-pyrazino[1, 2-a][1, 8]naphthyridin-8-yl)-3-oxopropoxy)prop-2-yl)amino)-4-(trifluoromethyl)pyridazin-3(2H)-one

LCMS (ESI) m/z: 585.10[M+H]$^+$

HNMR (400 MHz, DMSO-$d_6$): δ ppm 12.45 (s, 1H), 8.60 (s, 1H), 8.15 (s, 1H), 7.89 (s, 1H), 6.28-6.25 (m, 1H), 4.69-4.44 (m, 2H), 4.19-4.03 (m, 2H), 3.73-3.64 (m, 2H), 3.56-3.53 (m, 1H), 3.49-3.42 (m, 2H), 3.22-3.07 (m, 1H), 3.04-2.85 (m, 1H), 2.82-2.72 (m, 1H), 2.67-2.61 (m, 3H), 2.34-2.28 (m, 1H), 1.15 (d, J = 8Hz, 3H).

Chiral chromatography: chromatographic column: CHIRAL ART Cellulose-SB, 4.6*100 cm, 3 μm; mobile phase A: methyl t-butyl ether (0.1% diethyl amine), mobile phase B: methanol;mobile phaseA: mobile phase B=90:10; flow rate: 1.0 ml/ min; temperature: 25°C; wavelength: 220/254 nm; retention time: 4.64 min.

**Example 61:** Synthesis of (S)-8-((R)-2-fluoro-3-((S)-2-((6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one **(Compound 65)** and (S)-8-((S)-2-fluoro-3-((S)-2-((6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one **(Compound** 66)

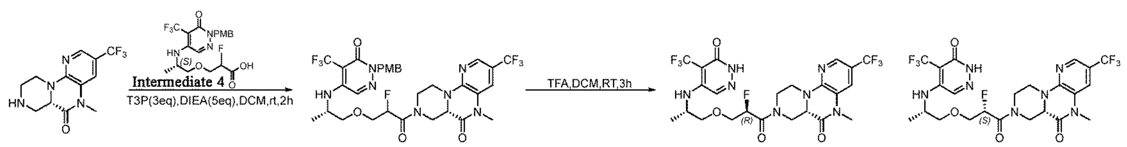


7-4 65-1 **Compound 65** **Compound 66**

Step I: Synthesis of (6aS)-8-(2-fluoro-3-((S)-2-((1-(4-methoxybenzyl)-6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one

(S)-5-methyl-3-(trifluoromethyl)-7, 8, 9, 10-tetrahydro-5H-pyrazino[1, 2-a]pyrido[3, 2-e]pyrazin-6(6aH)-one (100 mg, 0.35 mmol) and 2-fluoro-3-((S)-2-((1-(4-methoxybenzyl)-6-oxo-5-(trifluoromethyl)-1, 6-dihydropyridazin-4-yl)amino)propoxy)propionic acid **(Intermediate 4,** 156 mg, 0.35 mmol) were dissolved in dichloromethane (2ml), and then N,N-diisopropylethyl amine (135 mg, 1.05 mmol) and 1-propylphosphonic anhydride (337 mg, 0.53 mmol, 50% solution in ethyl acetate) were added. The reaction mixture was stirred at room temperature for 2 hrs. The reaction solution was poured into ice water (20 ml), and the aqueous phase was extracted with dichloromethane (20 ml × 3). The organic phases were combined, and dried over anhydrous sodium sulfate. Anhydrous sodium sulfate was removed by filtration, and the filtrate was rotary dried to obtain a crude product. The crude product was purified by column chromatography on silica gel (petroleum ether: ethyl acetate =40: 60) to obtain (6aS)-8-(2-fluoro-3-((S)-2-((1-(4-methoxybenzyl)-6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one as a yellow oil (200 mg, 0.28 mmol, yield 80%). LCMS (ESI): 716 $[M+H]^+$

Step II: Synthesis of (S)-8-((R)-2-fluoro-3-((S)-2-((6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one and (S)-8-((S)-2-fluoro-3-((S)-2-((6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one

(6aS)-8-(2-fluoro-3-((S)-2-((1-(4-methoxybenzyl)-6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one (200 mg, 0.28 mmol) was dissolved in an ice-cold (0°C) mixed solution of trifluoroacetic acid (2 ml), trifluoromethanesulfonic acid (1 ml) and dichloromethane (5 ml), slowly warmed to room temperature, and reacted for 3 hrs with stirring. After complete reaction, the reaction solution was concentrated under reduced pressure, to obtain a crude product. The crude product was purified by high performance liquid chromatography to obtain 150 mg of a mixture, which was resolved by chiral preparative chromatography (CHIRALPAK ID, 2*25 cm, 5 μm; mobile phase A: methyl t-butyl ether (containing 0.1% diethyl amine), mobile phase B: ethanol; flow rate: 20 ml/ min; gradient: 40% B to 40% B over 12.5 min; wavelength: 220/254 nm; retention time 1: 7.15 min; retention time 2: 10.33 min), to obtain the first peak (S)-8-((R)-2-fluoro-3-((S)-2-((6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one as a white solid (52.08 mg, 0.088 mmol, yield 31%), and the second peak (S)-8-((S)-2-fluoro-3-((S)-2-((6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one as a white solid (38.65 mg, 0.065 mmol, yield 23%).

First peak **(Compound 65):** (S)-8-((R)-2-fluoro-3-((S)-2-((6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one

LCMS(ESI) m/z: 596.05$[M+H]^+$

HNMR (400 MHz, DMSO-$d_6$): δ ppm 12.45 (s, 1H), 8.20 (s, 1H), 7.92 (s, 1H), 7.48 (s, 1H), 6.32-6.29 (m, 1H), 5.60 (dt, J = 48.0, 4.8 Hz, 1H), 4.79-4.06 (m, 5H), 3.91-3.88 (m, 1H), 3.87-3.80 (m, 1H), 3.61-3.57 (m, 3H), 3.30-3.21 (m, 2H), 3.18-3.16 (m, 1H), 2.90-2.67 (m, 2H), 1.18-1.11 (m, 3H).

Chiral chromatography: chromatographic column: CHIRALPAK ID, 4.6*50 cm, 3 μm; mobile phase A: methyl t-butyl ether (0.1% diethyl amine), mobile phase B: ethanol; mobile phase A: mobile phase B = 60:40; flow rate: 1.0 ml/ min; temperature: 25°C; wavelength: 220/254 nm; retention time: 3.02 min.

Second peak **(Compound 66):** (S)-8-((S)-2-fluoro-3-((S)-2-((6-oxo-5-(trifluoromethyl)-1, 6-dihydropyridazin-4-yl)amino)propoxy)propionyl)-5-methyl-3-(trifluoromethyl)-7, 8, 9, 10-tetrahydro-5H-pyrazino[1, 2-a]pyrido[3, 2-e]pyrazin-6(6aH)-one

LCMS(ESI)m/z: 596.05$[M+H]^+$

HNMR (400 MHz, DMSO-$d_6$): δ ppm 12.47 (s, 1H), 8.55 (brs, 1H), 8.20 (s, 1H), 7.92 (s, 1H), 7.49 (d, J = 1.6 Hz, 1H), 6.30 (s, 1H), 5.62 (dt, J = 48.4, 4.8 Hz, 1H), 4.81-4.47 (m, 2H), 4.42-4.17 (m, 2H), 4.17-4.02 (m, 1H), 3.95-3.79 (m, 2H), 3.65-3.62 (m, 1H), 3.57-3.54 (m, 1H), 3.20-3.13 (m, 1H), 2.93-2.51 (m, 4H), 1.19-1.15 (m, 3H).

Chiral chromatography: chromatographic column: CHIRALPAK ID, 4.6*50 cm, 3 μm; mobile phase A: methyl t-butyl ether (0.1% diethyl amine), mobile phase B: ethanol; mobile phase A: mobile phase B = 60:40; flow rate: 1.0 ml/min; temperature: 25°C; wavelength: 220/254 nm; retention time: 5.22 min.

**Example** 62: Synthesis of 5-((S)-1-((S)-2-hydroxy-3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one **(Compound 67)**

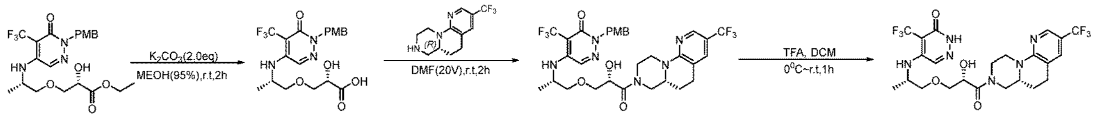


67-1

67-2

Compound 67

Step I: Synthesis of (S)-2-hydroxy-3-((S)-2-((1-(4-methoxybenzyl)-6-oxo-5-(trifluoromethyl)-1, 6-dihydropyridazin-4-yl)amino)propoxy)propionic acid

Ethyl (S)-2-hydroxy-3-((S)-2-((1-(4-methoxybenzyl)-6-oxo-5-(trifluoromethyl)-1, 6-dihydropyridazin-4-yl)amino)propoxy) propionate (100 mg, 0.211 mmol) was dissolved in a mixed solution of methanol (1.8 ml) and water (0.2 ml), and then potassium carbonate (58 mg, 0.422 mmol) was added. The mixture was stirred at room temperature for 1 hr. After the reaction was completed, the reaction solution was adjusted to pH 3 with 10% dilute hydrochloric acid, and extracted with ethyl acetate (10 ml*3). The organic layers were combined, washed with saturated brine, dried over anhydrous sodium sulfate, and filtered. The filtrate was concentrated, to obtain crude (S)-2-hydroxy-3-((S)-2-((1-(4-methoxybenzyl)-6-oxo-5-(trifluoromethyl)-1, 6-dihydropyridazin-4-yl)amino)propoxy)propionic acid as a yellow oil (90 mg). The product was directly used in the next step without purification. LCMS (ESI): 446 [M+H]+

Step II: Synthesis of 5-((S)-1-((S)-2-hydroxy-3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-2-(4-methoxybenzyl)-4-(trifluoromethyl)pyridazin-3(2H)-one

(R)-3-(trifluoromethyl)-6, 6a, 7, 8, 9, 10-hexahydro-5H-pyrazino[1, 2-a][1, 8]naphthyridine (52 mg, 0.202 mmol) was dissolved in N,N-dimethylformamide (2.00 ml), and then (S)-2-hydroxy-3-((S)-2-((1-(4-methoxybenzyl)-6-oxo-5-(trifluoromethyl)-1, 6-dihydropyridazin-4-yl)amino)propoxy)propionic acid (90 mg, 0.202 mmol), N,N,N',N'-tetramethyl-O-(7-azabenzotriazole -1-yl)uronium hexafluorophosphate (92 mg, 0.244 mmol) and N,N-diisopropylethyl amine (78 mg, 0.606 mmol) were added. The mixture was stirred at room temperature for 1 hr. After the reaction was completed, the reaction solution was poured into water (10 ml), and then extracted with ethyl acetate (10 ml*3). The organic layers were combined, washed with saturated brine, dried over anhydrous sodium sulfate, and filtered. The filtrate was concentrated, and the residue was purified by column chromatography on silica gel (dichloromethane:methanol=80:20) to obtain 5-((S)-1-((S)-2-hydroxy-3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-2-(4-methoxybenzyl)-4-(trifluoromethyl)pyridazin-3(2H)-one as a yellow oil (100 mg, 0.145 mmol, yield 72%). LCMS (ESI): 685[M+H]+

Step III: Synthesis of 5-((S)-1-((S)-2-hydroxy-3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one

5-((S)-1-((S)-2-hydroxy-3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-2-(4-methoxybenzyl)-4-(trifluoromethyl)pyridazin-3(2H)-one (100 mg, 0.15 mmol) was added to a mixed solution of dichloromethane (2 ml) and trifluoroacetic acid (0.4 ml). The mixture was stirred at room temperature for 2 hrs. After the reaction was completed, the system was directly concentrated under reduced pressure, and the crude product was purified by high performance liquid chromatography (chromatographic column: CSH OBD column 30 * 150 mm 5 μm; mobile phase A: water (containing 0.05% formic acid), mobile phase B: acetonitrile; flow rate: 60 ml/ min; gradient: 10% B to 50% B over 10 min; wavelength: 220 nm; retention time: 9.77 min) to obtain 5-(((S)-1-((S)-2-hydroxy-3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one as a white solid (10.01mg, 0.018 mmol, yield 12%).

LCMS (ESI): 565.20[M+H]+

HNMR (400 MHz, DMSO-d6): δ ppm 12.45 (s, 1H), 8.23(s, 1H), 7.90(s,1H), 7.53(s, 1H), 6.29-6.28(m, 1H), 5.46-4.96(m, 1H), 4.76-4.66 (m,1H), 4.51-4.48(m, 1H),4.38-4.36(m, 1H), 4.19-4.11(m, 2H), 3.81-3.77 (m, 1H),3.59-3.49 (m, 4H), 2.98-2.82(m,2H), 2.78-2.71(m, 3H), 2.04-2.01(m, 1H), 1.69-1.60(m, 1H), 1.21-1.11(m, 3H).

**Example 63:** Synthesis of 5-((S)-1-((R)-2-hydroxy-3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one **(Compound 68)**

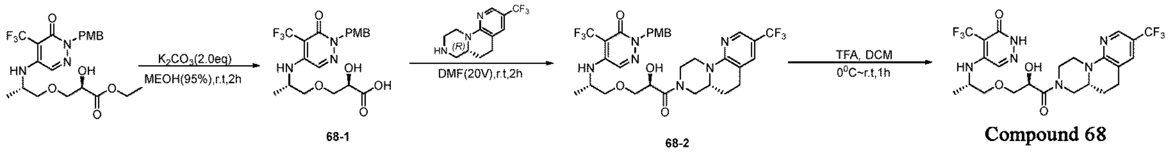


68-1 68-2 Compound 68

Ethyl (R)-2-hydroxy-3-((S)-2-((1-(4-methoxybenzyl)-6-oxo-5-(trifluoromethyl)-1, 6-dihydropyridazin-4-yl)amino)propoxy)propionate was used as a starting material, and the synthesis method was the same as that for 5-((S)-1-((S)-2-hydroxy-3-oxo-3-((R)-3-(trifluoromethyl)-5, 6, 6a, 7, 9, 10-hexahydro-8H-pyrazino[1, 2-a][1, 8]naphthyridin-8-yl)propoxy)prop-2-yl)amino)-4-(trifluoromethyl)pyridazin-3(2H)-one **(Compound 6).**

LCMS(ESI) m/z: 565.15 $[M+H]^+$

HNMR (400 MHz, DMSO-d6): δ ppm 12.45 (s, 1H), 8.24(s, 1H), 7.91(s,1H), 7.53(s, 1H), 6.27(s, 1H),5.34-5.20(m, 1H), 4.77-4.66 (m,1H),4.55-4.45(m, 1H),4.45-4.32 (m, 1H), 4.22-4.07(m, 2H), 3.72-3.47(m, 5H),3.13-3.08(m,1H), 2.99-2.72(m,4H), 2.14-1.98(m, 1H), 1.68-1.55(m, 1H), 1.18-1.10(m, 3H).

**Example 64:** Synthesis of 5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)oxy)-4-(trifluoromethyl)pyridazin-3(2H)-one **(Compound 69)**

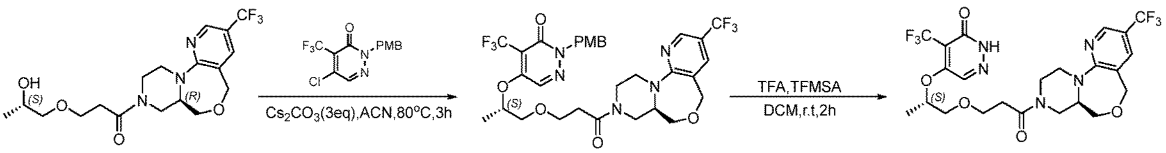


Intermediate 7 69-1 Compound 69

Step I: Synthesis of 2-(4-methoxybenzyl)-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)oxy)-4-(trifluoromethyl)pyridazin-3 (2H)-one

3-((S)-1-hydroxyprop-2-yl)oxy)-1-((R)-3-(trifluoromethyl)-7a, 8, 10, 11-tetrahydro-5H-pyrazino[2, 1-c]pyrido[2, 3-e][1, 4]oxazepane-9(7H)-yl)prop-1-one **(Intermediate 7,** 250 mg, 0.62 mmol) was dissolved in acetonitrile (3 ml), and then 5-chloro-2-(4-methoxybenzyl)-4-(trifluoromethyl)pyridazin-3(2H)-one (200 mg, 0.62 mmol) and cesium carbonate (605 mg, 1.86 mmol) were added and reacted for 3 hrs at 80°C. After the reaction was completed, the system was cooled to room temperature, the reaction solution was poured into water (10 ml), and then extracted with ethyl acetate (5 ml*3). The organic phases were combined, washed with saturated brine, dried over anhydrous sodium sulfate, and filtered. The filtrate was concentrated, and the residue was purified by column chromatography on silica gel (petroleum ether: ethyl acetate =50:50) to obtain 2-(4-methoxybenzyl)-5-((S)-2-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)propoxy)-4-(trifluoromethyl)pyridazin-3(2H)-one as a yellow solid (170 mg, 0.25 mmol, yield 43.48%). LCMS (ESI) m/z: 686 $[M+H]^+$

Step II: Synthesis of 5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)oxy)-4-(trifluoromethyl)pyridazin-3(2H)-one

2-(4-methoxybenzyl)-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)oxy)-4-(trifluoromethyl)pyridazin-3(2H)-one (100.00 mg, 0.15 mmol) was dissolved in dichloromethane (2 ml), and then trifluoroacetic acid (0.1 ml) and trifluoromethanesulfonic acid (0.2 ml) were added and reacted for 1 hr at room temperature. After the reaction was completed, the reaction solution was concentrated. The crude product was purified by high performance liquid chromatography (chromatographic column: XBridge Prep OBD C18 column, 30*150 mm, 5 μm; mobile phase A: 10 mmol/l ammonium bicarbonate aqueous solution, mobile phase B: acetonitrile; flow rate: 60 ml/ min; gradient: 20% B to 70% B over 10 min; wavelength: 220 nm; retention time: 10.20 min) to obtain 5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)oxy)-4-(trifluoromethyl)pyridazin-3(2H)-one as a white solid (27.29 mg, 0.048 mmol, yield 33.50%).

LCMS (ESI) m/z: 566 .15$[M+H]^+$

HNMR (400 MHz, DMSO-d6): δ ppm 12.94 (s, 1H), 8.38 (s, 1H), 8.17 (s, 1H), 7.72 (d, *J* = 2.4 Hz, 1H), 5.12-5.06 (m, 1H), 4.93-4.80 (m, 1H), 4.56-4.41 (m, 1H), 4.04-4.00 (m, 1H), 3.94 - 3.63 (m, 8H), 3.60-3.52 (m, 2H), 3.42-3.37 (m, 2H), 2.53-2.50 (m, 2H), 1.27 (d, *J* = 6.2 Hz, 3H).

**Example 65:** Synthesis of 5-((S)-2-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)propoxy)-4-(trifluoromethyl)pyridazin-3(2H)-one **(Compound 70)**

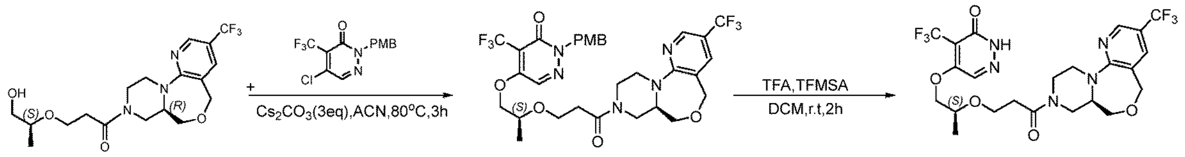


Intermediate 6 70-1 Compound 70

3-((S)-1-hydroxyprop-2-yl)oxy)-1-((R)-3-(trifluoromethyl)-7a, 8, 10, 11-tetrahydro-5H-pyrazino[2, 1-c]pyrido[2, 3-e][1, 4]oxazepane-9(7H)-yl)prop-1-one **(Intermediate 6)** was used as a starting material, and the synthesis method was the same as that for 5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)oxy)-4-(trifluoromethyl)pyridazin-3 (2H)-one **(Compound** 69) .

LCMS (ESI) m/z: 566 .10[M+H]$^+$

HNMR (400 MHz, DMSO-d6): δ ppm 13.04 (s, 1H), 8.37 (s, 1H), 8.16 (s, 1H), 7.72 (s, 1H), 4.94-4.82 (m, 1H), 4.58-4.46 (m, 1H), 4.33 (d, J = 5.0 Hz, 2H), 4.09-4.00 (m, 1H), 3.96-3.89 (m, 1H), 3.89 - 3.53(m, 8H), 3.44-3.36 (m, 2H), 2.59-2.50(m, 2H), 1.14 (d, J = 6.3 Hz, 3H).

**Example 66** Synthesis of 5-((S)-1-((S)-2-hydroxy-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one **(Compound 71)**

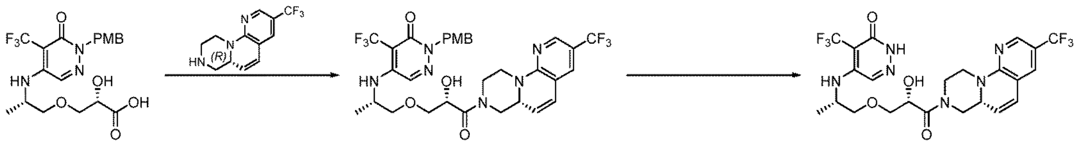

67-1 71-1 Compound 71

Step I: Synthesis of 5-((S)-1-((S)-2-hydroxy-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-2-(4-methoxybenzyl)-4-(trifluoromethyl)pyridazin-3 (2H)-one

(S)-2-hydroxy-3-((S)-2-((1-(4-methoxybenzyl)-6-oxo-5-(trifluoromethyl)-1, 6-dihydropyridazin-4-yl)amino)propoxy)propionic acid (100 mg, 0.22 mmol) and (R)-3-(trifluoromethyl)-6a, 7, 9, 10-tetrahydro-8H-pyrazino[1, 2-a][1, 8]naphthyridine (56 mg, 0.22 mmol) were dissolved in dichloromethane (5 ml). Then 2-(7-azobenzotriazole)-N,N,N',N'-tetramethyluronium hexafluorophosphate (168 mg, 0.44 mmol) and N,N-diisopropylethyl amine (85 mg, 0.66 mmol) were added, and reacted for 1 hr at room temperature. After the reaction was completed, the reaction solution was poured into water (10 ml), and extracted with dichloromethane (10 ml × 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate:petroleum ether=50: 50) to obtain 5-((S)-1-((S)-2-hydroxy-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-2-(4-methoxybenzyl)-4-(trifluoromethyl)pyridazin-3(2H)-one as a yellow oil (50 mg, 0.073 mmol, yield 33%).

LCMS (ESI): 683 [M+H]$^+$

Step II: Synthesis of 5-((S)-1-((S)-2-hydroxy-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin- 3 (2H) -one

5-((S)-1-((S)-2-hydroxy-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-2-(4-methoxybenzyl)-4-(trifluoromethyl)pyridazin-3(2H)-one (50 mg, 0.073 mmol) was dissolved in dichloromethane (5 ml). Then trifluoroacetic acid (2 ml) and trifluoromethanesulfonic acid (2 ml) were added, and reacted for 1 hr at room temperature. After the reaction was completed, the reaction solution was poured into a saturated sodium bicarbonate aqueous solution (10 ml), and extracted with dichloromethane (10 ml × 3).

The organic phases were combined, washed with saturated brine, and dried over anhydrous sodium sulfate. Anhydrous sodium sulfate was removed by filtration, and the filtrate was concentrated to obtain a crude product. The crude product was purified by high performance liquid chromatography (chromatographic column: XBridge Prep OBD C18, 30 × 150 mm 5 um, mobile phase A: 10 mmol/l ammonium bicarbonate aqueous solution, mobile phase B: acetonitrile, flow rate: 60 ml/ min, elution gradient: mobile phase B from 15% to 43% over 8 min, detection wavelength: 220 nm, retention time: 7.47 min) to obtain 5-((S)-1-((S)-2-hydroxy-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one as a yellow solid (10.52 mg, 0.019 mmol, yield 25%).

LCMS (ESI) m/z: 562.95[M+H]+

HNMR (400 MHz, DMSO-$d_6$): δ ppm 12.45 (s, 1H), 8.12 (s, 1H), 7.90 (s, 1H), 7.42 (s, 1H), 6.48 (d, J = 9.2Hz, 1H), 6.29 (s, 1H), 5.72-5.68 (m, 1H), 5.27 (s, 1H), 4.81-4.72 (m, 1H), 4.46-4.32 (m, 3H), 4.14-4.02 (m, 2H), 3.60 (s, 1H), 3.52 (s, 3H), 3.22-3.09 (m, 1H), 2.64-2.61 (m, 1H), 1.14 (s, 3H).

**Example 67** Synthesis of 5-((S)-1-((R)-2-hydroxy-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one **(Compound 72)**

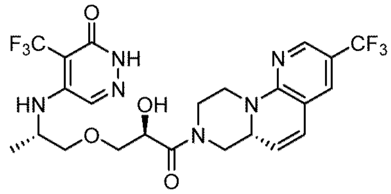

Compound 72

(R)-2-hydroxy-3-((S)-2-((1-(4-methoxybenzyl)-6-oxo-5-(trifluoromethyl)-1, 6-dihydropyridazin-4-yl)amino)propoxy)propionic acid **68-1** was used as a starting material, and the synthesis method was the same as that for 5-((S)-1-((S)-2-hydroxy-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one **(Compound 71).**

LCMS(ESI) m/z: 562.95 [M+H]+

HNMR (400 MHz, DMSO-d6): δ ppm 12.45 (s, 1H), 8.12 (s, 1H), 7.91 (s, 1H), 7.42 (s, 1H), 6.48 (d, J = 9.2Hz, 1H), 6.28 (s, 1H), 5.76-5.67 (m, 1H), 5.31 (s, 1H), 4.81-4.73 (m, 1H), 4.60-4.42 (m, 2H), 4.41-4.30 (m, 1H), 4.13-4.09 (m, 2H), 3.60-3.52 (m, 3H), 3.24-3.09 (m, 1H), 2.78-2.66 (m, 1H), 1.15 (s, 3H).

**Example 68:** Synthesis of 5-((S)-1-((R)-2-fluoro-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one **(Compound** 73)

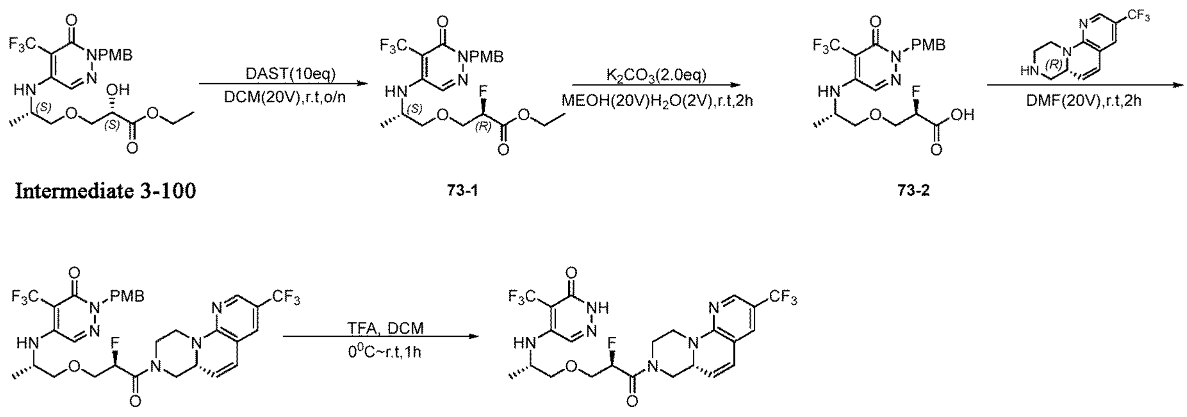


Intermediate 3-100 73-1 73-2

73-3 Compound 73

Step I: Synthesis of ethyl (R)-2-fluoro-3-((S)-2-((1-(4-methoxybenzyl)-6-oxo-5-(trifluoromethyl)-1, 6-dihydropyridazin-4-yl)amino)propoxy)propionate

Ethyl (S)-2-hydroxy-3-((S)-2-((1-(4-methoxybenzyl)-6-oxo-5-(trifluoromethyl)-1, 6-dihydropyridazin-4-yl)amino)propoxy)propionate **(Intermediate 3-100,** 200 mg, 0.42 mmol) and diethylaminosulfur trifluoride (680 mg, 4.22 mmol) were dissolved in dichloromethane (4.00 ml), and stirred for 3 hrs under nitrogen atmosphere at room temperature. After the reaction was completed, the reaction solution was poured into water (20 ml), and extracted with dichloromethane (10 ml*2). The organic layers were combined, washed with saturated brine, dried over anhydrous sodium sulfate, and filtered. The filtrate was concentrated under vacuum, and the residue was purified by column chromatography on silica gel (dichloromethane : methanol=90:10) to obtain ethyl (R)-2-fluoro-3-((S)-2-((1-(4-methoxybenzyl)-6-oxo-5-(trifluoromethyl)-1, 6-dihydropyridazin-4-yl)amino)propoxy)propionate as a yellow solid (190 mg, 0.39 mmol, yield 95%). LCMS (ESI) m/z: 476$[M+H]^+$

Step II: Synthesis of (R)-2-fluoro-3-((S)-2-((1-(4-methoxybenzyl)-6-oxo-5-(trifluoromethyl)-1, 6-dihydropyridazin-4-yl)amino)propoxy)propionic acid

Ethyl (R)-2-fluoro-3-((S)-2-((1-(4-methoxybenzyl)-6-oxo-5-(trifluoromethyl)-1, 6-dihydropyridazin-4-yl)amino)propoxy)propionate (200 mg, 0.42 mmol) were dissolved in a mixed solution of methanol (1.8 ml) and water (0.2 ml), and then potassium carbonate (116 mg, 0.84 mmol) were added. The mixture was stirred at room temperature for 1 hrs. After the reaction was completed, the reaction system was adjusted to pH 3 with 10% dilute hydrochloric acid, and, and extracted with ethyl acetate (10 ml*3). The organic layers were combined, washed with saturated brine, dried over anhydrous sodium sulfate, and filtered. The filtrate was concentrated under vacuum to obtain crude (R)-2-fluoro-3-((S)-2-((1-(4-methoxybenzyl)-6-oxo-5-(trifluoromethyl)-1, 6-dihydropyridazin-4-yl)amino)propoxy)propionic acid as a yellow oil (180 mg). The crude product was directly used in the next step without purification. LCMS (ESI): 448 [M+H]+

Step III: Synthesis of 5-((S)-1-((R)-2-fluoro-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-2-(4-methoxybenzyl)-4-(trifluoromethyl)pyridazin-3(2H)-one

(R)-3-(trifluoromethyl)-6a, 7, 9, 10-tetrahydro-8H-pyrazino[1, 2-a][1, 8]naphthyridine (103 mg, 0.40 mmol) was dissolved in N,N-dimethylformamide (4.00 ml), and then (R)-2-fluoro-3-((S)-2-((1-(4-methoxybenzyl)-6-oxo-5-(trifluoromethyl)-1, 6-dihydropyridazin-4-yl)amino)propoxy)propionic acid (180 mg, 0.40 mmol), N,N,N',N'-tetramethyl-O-(7-azabenzotriazole -1-yl)uronium hexafluorophosphate (183 mg, 0.48 mmol) and N,N-diisopropylethyl amine (123 mg, 0.96 mmol) were added. The mixture was stirred at room temperature for 1 hrs. After complete reaction, the reaction solution was poured into water (10 ml), and then extracted with ethyl acetate (10 ml*2). The organic layers were combined, washed with saturated brine, dried over anhydrous sodium sulfate, and filtered. The filtrate was concentrated under vacuum, and the residue was purified by reverse phase chromatography (0.5% ammonium bicarbonate aqueous solution: acetonitrile=50:50) to obtain 5-((S)-1-((R)-2-fluoro-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-2-(4-methoxybenzyl)-4-(trifluoromethyl)pyridazin-3(2H)-one as a brown yellow oil (90 mg, 0.13 mmol, yield 32%). LCMS (ESI): 685[M+H]+

Step IV: Synthesis of 5-((S)-1-((R)-2-fluoro-3-oxo-3-((R)-3-(trifluoromethyl)-6a, 7, 9, 10-tetrahydro-8H-pyrazino[1, 2-a] [1, 8]naphthyridin-8-yl)propoxy)prop-2-yl)amino)-4-(trifluoromethyl)pyridazin- 3 (2H) -one

5-((S)-1-((R)-2-fluoro-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-2-(4-methoxybenzyl)-4-(trifluoromethyl)pyridazin-3(2H)-one (90 mg, 0.13 mmol) was dissolved in dichloromethane (3.00 ml), and then trifluoroacetic acid (0.5 ml) and trifluoromethanesulfonic acid (0.5 ml) was added. The mixture was stirred at room temperature for 0.5 hr. After complete reaction, the system was directly concentrated under reduced pressure. The crude product was purified by high performance liquid chromatography (chromatographic column: CSH C18 OBD column, 19*250 nm, 5 micrometers; mobile phase A: 10 mmol/l ammonium bicarbonate aqueous solution, mobile phase B: acetonitrile; flow rate: 60 ml/ min; elution gradient: mobile phase B from 25% to 65% over 8 min; detection wavelength: 220 nm; retention time 7.42 min) to obtain 5-((S)-1-((R)-2-fluoro-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one as a white solid (12.01 mg, 0.022 mmol, yield 17%).

LCMS (ESI): 564.95[M+H]+

HNMR (400 MHz, $CDCl_3$): $\delta$ ppm 10.04 (s, 1H), 8.11(s, 1H), 7.65(s,1H), 7.15(s, 1H), 6.40-6.30(m, 1H), 5.82-5.70(m, 1H), 5.62-5.51 (m,1H),5.42-5.10(m, 1H),5.10-5.01 (m, 1H), 4.64-4.43(m, 2H), 4.20-3.82 (m, 4H),3.80-3.69 (m, 1H), 3.65-3.52(m,1H), 3.42-3.20(m, 1H), 3.01-2.62(m, 2H), 1.43-1.28(m, 3H).

**Example 69:** Synthesis of 5-((S)-1-((S)-2-fluoro-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one **(Compound 74)**

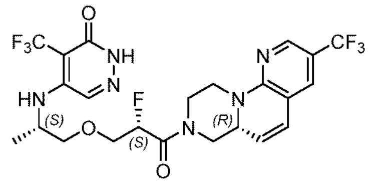

Compound 74

Ethyl (R)-2-hydroxy-3-((S)-2-((1-(4-methoxybenzyl)-6-oxo-5-(trifluoromethyl)-1, 6-dihydropyridazin-4-yl)amino)propoxy)propionate **(Intermediate3-200)** was used as a starting material, and the synthesis method was the same as that for 5-((S)-1-((R)-2-fluoro-3-oxo-3-((R)-3-(trifluoromethyl)-6a, 7, 9, 10-tetrahydro-8H-pyrazino[1, 2-a][1, 8]naphthyridin-8-yl)propoxy)prop-2-yl)amino)-4-(trifluoromethyl)pyridazin-3(2H)-one **(Compound** 73).

LCMS (ESI): 564.95[M+H]+

HNMR (400 MHz, $CDCl_3$): δ ppm 10.04 (s, 1H), 8.11(s, 1H), 7.65(s,1H), 7.15(s, 1H), 6.40-6.30(m, 1H), 5.82-5.70(m, 1H), 5.62-5.51 (m,1H),5.42-5.10(m, 1H),5.10-5.01 (m, 1H), 4.64-4.43(m, 2H), 4.15-3.89 (m, 4H),3.80-3.69 (m, 1H), 3.65-3.52(m,1H), 3.42-3.20(m, 1H), 3.01-2.62(m, 2H), 1.43-1.28(m, 3H).

**Example 70:** Synthesis of 4-(dimethylphosphoryl)-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)pyridazin-3(2H)-one **(Compound** 75)

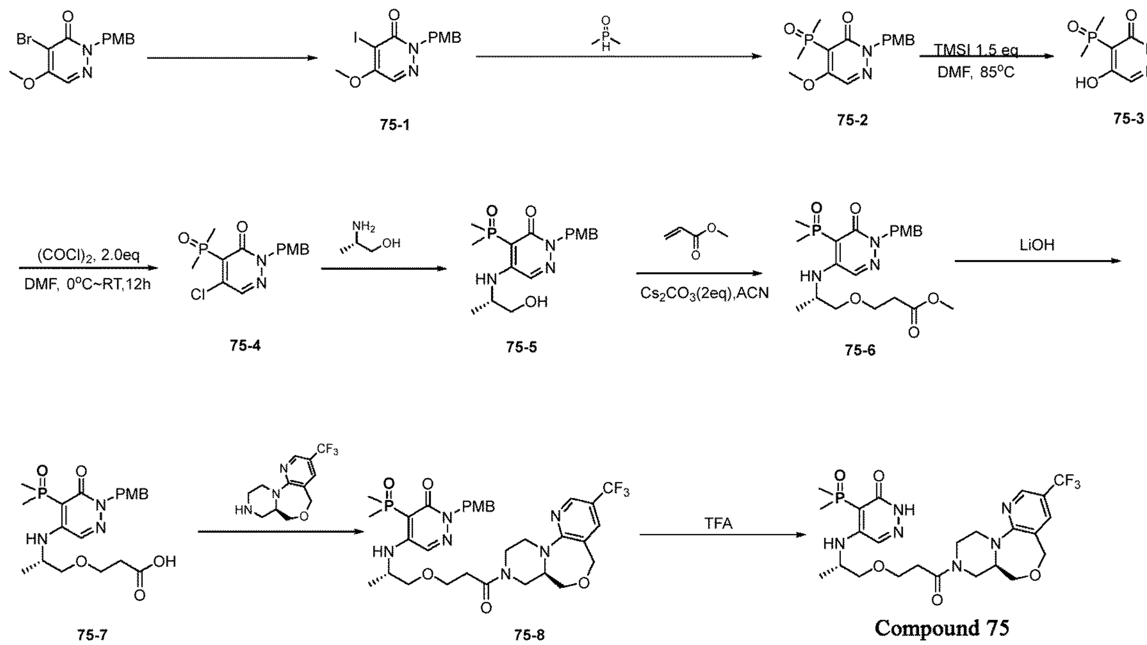


75-1 75-2 75-3 75-4 75-5 75-6 75-7 75-8 Compound 75

Step I: Synthesis of 4-iodo-5-methoxy-2-(4-methoxybenzyl)pyridazin-3(2H)-one

To a solution of 4-bromo-5-methoxy-2-(4-methoxybenzyl)pyridazin-3(2H)-one (5.00 g, 15.377 mmol) in dioxane(150ml), N, N-dimethylethylene diamine (135mg, 1.538mmol), cuprous iodide (438 mg, 2.307 mmol) and potassium iodide (5.10 g, 30.754 mmol) were added. The mixture was reacted for 12 hrs at 110°C. After the reaction was completed, the reaction solution was cooled to room temperature, and filtered. The filtrate was concentrated, and the residue was purified by column chromatography on silica gel (petroleum ether: ethyl acetate =70:30) to obtain 4-iodo-5-methoxy-2-(4-methoxybenzyl)pyridazin-3(2H)-one as a white solid (2.00 g, 5.3 mmol, yield 35.0%). LCMS (ESI) m/z: 373[M+H]$^+$

Step II: Synthesis of 4-(dimethylphosphoryl)-5-methoxy-2-(4-methoxybenzyl)pyridazin-3(2H)-one

To a solution of 4-iodo-5-methoxy-2-(4-methoxybenzyl)pyridazin-3(2H)-one (500 mg, 1.34 mmol) in N,N-dimethylformamide (15 ml), palladium acetate (30 mg, 0.134 mmol), 4,5-bis(diphenylphosphine)-9,9-dimethylxanthene (92 mg, 0.160 mmol), cesium carbonate (874 mg, 2.69 mmol), and dimethylphosphine oxide (209 mg, 2.69 mmol) were added. The mixture was reacted for 2 hrs under argon atmosphere at 60°C. After the reaction was completed, the system was cooled to room temperature. The reaction solution was poured into saturated sodium bicarbonate aqueous solution (30 ml), and then extracted with ethyl acetate (10 ml × 3). The organic phases were combined, washed with saturated brine, dried over anhydrous sodium sulfate, and filtered. The filtrate was concentrated, and the crude product was purified by reverse phase chromatography (acetonitrile: ammonium bicarbonate aqueous solution=30:70) to obtain 4-(dimethylphosphoryl)-5-methoxy-2-(4-methoxybenzyl)pyridazin-3(2H)-one as a yellow oil (253 mg, 0.78 mmol, yield 58%). LCMS (ESI) m/z: 323[M+H]+

Step III: Synthesis of 4-(dimethylphosphoryl)-5-hydroxy-2-(4-methoxybenzyl)pyridazin-3(2H)-one

To a solution of 4-(dimethylphosphoryl)-5-methoxy-2-(4-methoxybenzyl)pyridazin-3(2H)-one (253 mg, 0.785 mmol) in N,N-dimethylformamide (10 ml), trimethyliodosilane (235 mg, 1.177 mmol) was added. The mixture was reacted for 2 hrs at 85°C. After the reaction was completed, the system was cooled to room temperature. The reaction solution was poured into water (20 ml), and then extracted with ethyl acetate (10 ml × 3). The organic phases were combined, washed with saturated brine, dried over anhydrous sodium sulfate, and filtered. The filtrate was concentrated, and the crude product was purified by reverse phase chromatography (acetonitrile: ammonium bicarbonate aqueous solution=20: 80) to obtain 4-(dimethylphosphoryl)-5-hydroxy-2-(4-methoxybenzyl)pyridazin-3(2H)-one as a yellow oil (243 mg, 0.78 mmol, yield 96%). LCMS (ESI) m/z: 309[M+H]$^+$

Step IV: Synthesis of 5-chloro-4-(dimethylphosphoryl)-2-(4-methoxybenzyl)pyridazin-3(2H)-one

At 0°C, oxalyl chloride (200 mg, 1.577 mmol) was added to a solution of 4-(dimethylphosphoryl)-5-hydroxy-2-(4-methoxybenzyl)pyridazin-3(2H)-one (243 mg, 0.788 mmol) in N,N-dimethylformamide (10 ml). The mixture was then reacted for 12 hr at room temperature. After the reaction was completed, the system was concentrated, to remove excessive oxalyl chloride. The remaining reaction solution was slowly poured into a saturated sodium bicarbonate aqueous solution (20 ml). The reaction solution was then extracted with ethyl acetate (10 ml × 3). The organic phases were combined, washed with saturated brine, dried over anhydrous sodium sulfate, and filtered. The filtrate was concentrated, and the crude product was purified by column chromatography on silica gel (petroleum ether: ethyl acetate =70:30) to obtain 5-chloro-4-(dimethylphosphoryl)-2-(4-methoxybenzyl)pyridazin-3(2H)-one as a yellow oil (200 mg, 0.61 mmol, yield 78%). LCMS (ESI) m/z: 327[M+H]+

Step V: Synthesis of (S)-4-(dimethylphosphoryl)-5-(1-hydroxypropyl-2-yl)amino)-2-(4-methoxybenzyl)pyridazin-3(2H)-one

chloro-4-(dimethylphosphoryl)-2-(4-methoxybenzyl)pyridazin-3(2H)-one (1.00 g, 3.06 mmol), (S)-2-aminopropyl-1-ol (0.23 g, 3.06 mmol) and triethyl amine (0.61 g, 6.12 mmol) were added to ethanol (10.00 ml). The mixture was stirred for 3 hrs at 60°C. After complete reaction, the system was directly concentrated under reduced pressure, to obtain crude (S)-4-(dimethylphosphoryl)-5-(1-hydroxypropyl-2-yl)amino)-2-(4-methoxybenzyl)pyridazin-3(2H)-one as a white solid (0.80 g). The crude product was directly used in the next step without purification. LCMS (ESI) m/z: 366[M+H]+

Step VI: Synthesis of methyl (S)-3-(2-((5-(dimethylphosphoryl)-1-(4-methoxybenzyl)-6-oxo-1, 6-dihydropyridazin-4-yl)amino)propoxy)propionate

(S)-4-(dimethylphosphoryl)-5-(1-hydroxypropyl-2-yl)amino)-2-(4-methoxybenzyl)pyridazin-3(2H)-one (1.00 g, 2.74 mmol), methyl acrylate (0.24 g, 2.74 mmol) and cesium carbonate (1.78 g, 5.47 mmol) were added to acetonitrile (10.00 ml). The mixture was stirred for 2 hrs at room temperature. After complete reaction, the reaction solution was filtered. The filtrate was concentrated. The crude product was purified by column chromatography on silica gel (petroleum ether: ethyl acetate =80:20) to obtain methyl (S)-3-(2-((5-(dimethylphosphoryl)-1-(4-methoxybenzyl)-6-oxo-1, 6-dihydropyridazin-4-yl)amino)propoxy)propionate as a yellow oil (0.70 g, 1.52 mmol, yield 57%). LCMS (ESI) m/z: 452[M+H]+

Step VII: Synthesis of (S)-3-(2-((5-(dimethylphosphoryl)-1-(4-methoxybenzyl)-6-oxo-1, 6-dihydropyridazin-4-yl)amino)propoxy)propionic acid

Methyl (S)-3-(2-((5-(dimethylphosphoryl)-1-(4-methoxybenzyl)-6-oxo-1, 6-dihydropyridazin-4-yl)amino)propoxy)propionate (1.00 g, 2.22 mmol) and lithium hydroxide monohydrate (0.18 g, 4.43 mmol) were added to a mixed solution of methanol (10.00 ml) and water (2.00 ml). The mixture was stirred at room temperature for 5 hrs. After complete reaction, the reaction solution was poured into water (10 ml). The system was adjusted to pH 6 with formic acid, and extracted with ethyl acetate (10 ml*3). The organic phases were combined, washed with saturated brine, dried over anhydrous sodium sulfate, and filtered. The filtrate was concentrated to obtain crude (S)-3-(2-((5-(dimethylphosphoryl)-1-(4-methoxybenzyl)-6-oxo-1, 6-dihydropyridazin-4-yl)amino)propoxy)propionic acid as a yellow oil (0.70 g). LCMS (ESI) m/z: 438[M+H]+

Step VIII: Synthesis of 4-(dimethylphosphoryl)-2-(4-methoxybenzyl)-5-((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)pyridazin-3(2H)-one

(S)-3-(2-((5-(dimethylphosphoryl)-1-(4-methoxybenzyl)-6-oxo-1, 6-dihydropyridazin-4-yl)amino)propoxy)propionic acid (0.50 g, 1.14 mmol), (R)-3-(trifluoromethyl)-7, 7a, 8, 9, 10, 11-hexahydro-5H-pyrazino[2, 1-c]pyrido[2, 3-e][1, 4]oxazepane (0.31 g, 1.14 mmol), 1-propylphosphonic anhydride (1.45 g, 2.29 mmol, 50% solution in ethyl acetate) and N,N-diisopropylethyl amine (0.44 g, 3.43 mmol) were added to dichloromethane (5.00 ml), and stirred at room temperature for 2 hrs. After complete reaction, the reaction solution was poured into water (10 ml), and extracted with dichloromethane (10 ml * 3). The organic phases were combined, washed with saturated brine, dried over anhydrous sodium sulfate, and filtered. The filtrate was concentrated. The crude product was purified by column chromatography on silica gel (petroleum ether: ethyl acetate =40:60) to obtain 4-(dimethylphosphoryl)-2-(4-methoxybenzyl)-5-((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)pyridazin-3(2H)-one as a yellow oil (0.50 g, 0.72 mmol, yield 63%). LCMS (ESI) m/z: 693[M+H]+

Step IX: Synthesis of 4-(dimethylphosphoryl)-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)pyridazin-3(2H)-one

4-(dimethylphosphoryl)-2-(4-methoxybenzyl)-5-((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)pyridazin-3(2H)-one (0.10 g, 0.14 mmol) was added to a mixed solution of trifluoroacetic acid (1 ml) and dichloromethane (5 ml). The mixture was reacted for 2 hrs at room temperature. After complete reaction, the system was concentrated, and the crude product was purified by high performance liquid chromatography (chromatographic column: XBridge Shield RP18 OBD column, 19*150 mm, 5 μm; mobile phase A: 0.05% formic acid aqueous solution, mobile phase B: acetonitrile; flow rate: 60 ml; elution gradient: mobile phase B from 40% to 65% over 8 min; detection wavelength: 220 nm; retention time: 7.25 min) to obtain 4-(dimethylphosphoryl)-5-(((S)-1-(3-oxo-3-((R)-3-(trifluoromethyl)-7a, 8, 10, 11-tetrahydro-5H-pyrazino[2, 1-c]pyrido[2, 3-e][1, 4]oxazepane-9(7H)-yl)propoxy)prop-2-yl)amino)pyridazin-3(2H)-one as a white solid (11.23 mg, 0.019 mmol, yield 14%).

LCMS (ESI) m/z: 573.30 [M+H] +

HNMR (400 MHz, DMSO-$d_6$): δ ppm 12.23 (s, 1H), 9.32 (d, J = 8.4 Hz, 1H), 8.42-8.37 (m, 1H), 7.84 - 7.75 (m, 2H), 4.91 (t, J = 16.0 Hz, 1H), 4.56-4.44 (m, 1H), 4.07 - 3.88 (m, 4H), 3.87- 3.52 (m, 7H), 3.51-3.39 (m, 3H), 2.62- 2.58 (m, 2H), 1.73 - 1.63 (m, 6H), 1.12-1.09 (m, 3H).

**Example** 71 5-(((S)-1-(3-((R)-6,6-dimethyl-3-(trifluoromethyl)-5,6,6a,7,9,10-hexahydro-8H-pyrazino[1,2-a]pyrido[3,2-e]pyrazin-8-yl)-3-oxopropoxy)prop-2-yl)amino)-4-(trifluoromethyl)pyridazin-3(2H)-one **(Compound** 76)

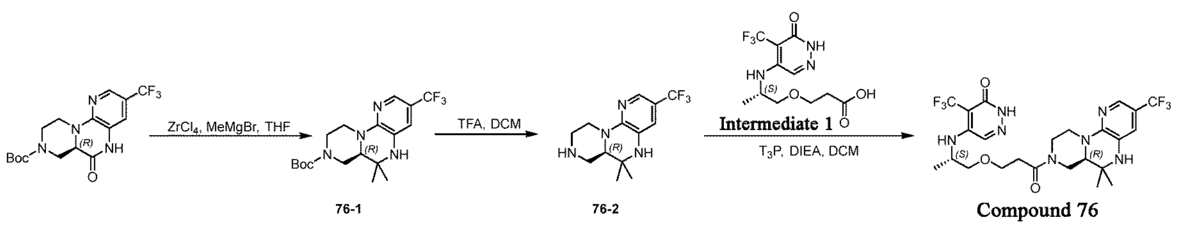

Step I: Synthesis of t-butyl (R)-6,6-dimethyl-3-(trifluoromethyl)-5,6,6a,7,9,10-hexahydro-8H-pyrazino[1,2-a]pyrido[3,2-e]pyrazin-8-carboxylate

At -10 °C and under nitrogen atmosphere, zirconium tetrachloride (0.45 g, 1.93 mmol) was added to a solution of the compound t-butyl (R)-6-oxo-3-(trifluoromethyl)-5,6,6a,7,9,10-hexahydro-8H-pyrazino[1,2-a]pyrido[3,2-e]pyrazin-8-carboxylate (0.30 g, 0.81 mmol) in THF (8 mL). The mixture was stirred for 30 min at -10 °C, and then methylmagnesium bromide (3 M solution in tetrahydrofuran, 3.5 mL, 10.50 mmol) was added. The reaction solution was stirred for 2 hrs at 0 °C, then heated to 25 °C, and stirred for 12 hrs. The reaction solution was quenched with saturated ammonium chloride (20 mL), and then extracted with ethyl acetate (20 mL * 3). The organic layer was concentrated, and the residue was purified by flash column chromatography (silica gel, methanol/dichloromethane over 0-5% gradient), to obtain crude compound t-butyl (R)-6,6-dimethyl-3-(trifluoromethyl)-5,6,6a,7,9,10-hexahydro-8H-pyrazino[1,2-a]pyrido[3,2-e]pyrazin-8-carboxylate as a yellow oil (40% purity, 100 mg, 0.10 mmol, yield 13%). LCMS (ESI) m/z: 387.1 $[M+H]^+$

Step II: Synthesis of (R)-6,6-dimethyl-3-(trifluoromethyl)-6,6a,7,8,9,10-hexahydro-5H-pyrazino[1,2-a]pyrido[3,2-e]pyrazine

To a solution of the compound t-butyl (R)-6,6-dimethyl-3-(trifluoromethyl)-5,6,6a,7,9,10-hexahydro-8H-pyrazino[1,2-a]pyrido[3,2-e]pyrazin-8-carboxylate (40% purity, 70 mg, 0.07 mmol) in dichloromethane (1 mL), trifluoroacetic acid (0.4 mL, 5.43 mmol) was added, and the reaction solution was stirred for 1 hr at 25 °C. The reaction solution was concentrated to obtain crude compound (R)-6,6-dimethyl-3-(trifluoromethyl)-6,6a,7,8,9,10-hexahydro-5H-pyrazino[1,2-a]pyrido[3,2-e]pyrazine as a yellow oil (60 mg). LCMS (ESI) m/z: 187.1 $[M+H]^+$

Step III: Synthesis of 5-(((S)-1-(3-((R)-6,6-dimethyl-3-(trifluoromethyl)-5,6,6a,7,9,10-hexahydro-8H-pyrazino[1,2-a]pyrido[3,2-e]pyrazin-8-yl)-3-oxopropoxy)prop-2-yl)amino)-4-(trifluoromethyl)pyridazin-3(2H)-one

To a solution of the compound (R)-6,6-dimethyl-3-(trifluoromethyl)-6,6a,7,8,9,10-hexahydro-5H-pyrazino[1,2-a]pyrido[3,2-e]pyrazine (60 mg, 0.10 mmol) in dichloromethane (800 uL), (S)-3-(2-((6-oxo-5-(trifluoromethyl)-1,6-dihydropyridazin-4-yl)amino)propoxy)propionic acid **(Intermediate 1,** 42 mg, 0.15 mmol), diisopropylethyl amine (128 uL, 0.78 mmol) and 1-propylphosphonic anhydride (50% solution in ethyl acetate, 123 mg, 0.19 mmol) were added. The reaction solution was stirred for 16 hrs at 25 °C. The reaction solution was concentrated, and the residue was purified by preparative HPLC to obtain compound 5-(((S)-1-(3-((R)-6,6-dimethyl-3-(trifluoromethyl)-5,6,6a,7,9,10-hexahydro-8H-pyrazino[1,2-a]pyrido[3,2-e]pyrazin-8-yl)-3-oxopropoxy)prop-2-yl)amino)-4-(trifluoromethyl)pyridazin-3(2H)-one as a yellow solid (1.96 mg, 3.39 umol, yield 3%).

LCMS (ESI) m/z: 578.2 $[M+H]^+$

HNMR (400 MHz, MeOH-d4): δ ppm 7.93 (s, 1H), 7.71 (s, 1H), 6.83 (s, 1H), 4.78-4.63 (m, 1H), 4.14 (br s, 1H), 4.07-3.93 (m, 1H), 3.85-3.72 (m, 2H), 3.65-3.57 (m, 1H), 3.50 (dd, *J* = 9.7, 6.6 Hz, 1H), 3.28-3.11 (m, 2H), 3.06-2.76 (m, 2H), 2.69 (m, 2H), 2.57 (t, *J* = 12.1 Hz, 1H), 1.33-1.16 (m, 9H).

**Example 72:** Synthesis of 5-(((S)-1-(3-((R)-2-amino-3-(trifluoromethyl)-7a, 8, 10, 11-tetrahydro-5H-pyrazino[2, 1-c]pyrido[2, 3-e][1, 4]oxazepane-9(7H)-yl)-3-oxypropoxy)prop-2-yl)amino)-4-(trifluoromethyl)pyridazin-3(2H)-one **(Compound 77)**

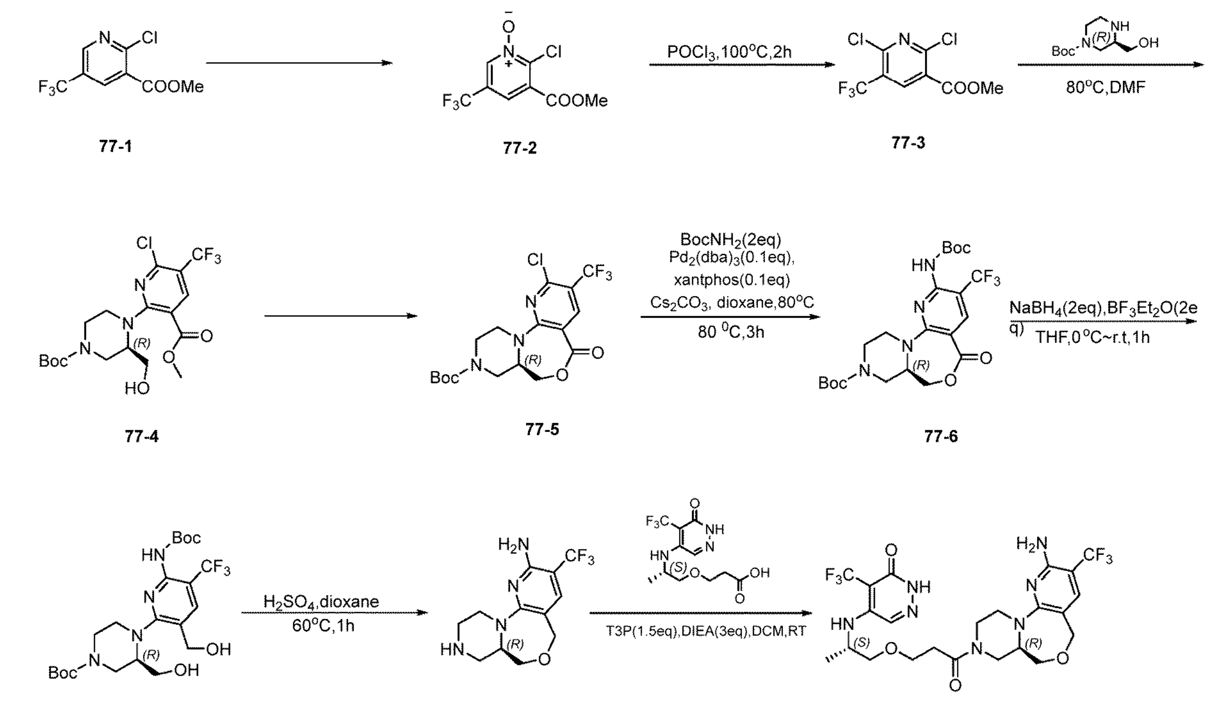


77-1 77-2 77-3

77-4 77-5 77-6

77-7 77-8 **Compound 77**

Step I: Synthesis of 2-chloro-3-(methoxycarbonyl)-5-(trifluoromethyl)pyridine 1-oxide

Methyl 2-chloro-5-(trifluoromethyl)nicotinate (5.02 g, 20.9 mmol) was dissolved in hydrogen peroxide (100 ml), and trifluoroacetic acid (4.76 g, 41.8 mmol) was added to the solution. The mixture was stirred for 2 hrs at 60°C. After the reaction was completed, the system was cooled to room temperature, poured into ice water, and extracted with dichloromethane (100 ml*2). The organic phases were combined, washed with saturated brine, dried over anhydrous sodium sulfate and then filtered. The filtrate was concentrated under reduced pressure to obtain a crude product. The crude product was purified by column chromatography on silica gel (petroleum ether: ethyl acetate =80:20) to obtain 2-chloro-3-(methoxycarbonyl)-5-(trifluoromethyl)pyridine 1-oxide as a yellow solid (2.52 g, 9.8 mmol, yield 46%). LCMS (ESI) m/z: 256[M+H]+

Step II: Synthesis of methyl 2, 6-dichloro-5-(trifluoromethyl)nicotinate

Chloro-3-(methoxycarbonyl)-5-(trifluoromethyl)pyridine 1-oxide (2.52 g, 9.8 mmol) was dissolved in phosphorus oxychloride (30 ml). The mixture was stirred for 2 hrs at 100°C. After the reaction was completed, the reaction solution was concentrated under reduced pressure, and the residue was added with water (100 ml) and extracted with dichloromethane (50 ml*2). The organic layers were combined, washed with saturated brine, dried over anhydrous sodium sulfate, and then filtered. The filtrate was concentrated under reduced pressure to obtain a crude product. The crude product was purified by column chromatography on silica gel (petroleum ether: ethyl acetate =50:50) to obtain methyl 2, 6-dichloro-5-(trifluoromethyl)nicotinate as a yellow oil (1.43 g, 5.2 mmol, yield 53%). LCMS (ESI) m/z: 274$[M+H]^+$

Step III: Synthesis of t-butyl (R)-4-(6-chloro-3-(methoxycarbonyl)-5-(trifluoromethyl)pyridin-2-yl)-3-(hydroxymethyl)piperazin-1-carboxylate

Methyl 2, 6-dichloro-5-(trifluoromethyl)nicotinate (1.43 g, 5.2 mmol) was added to a reaction flask, and then N,N-dimethylformamide (30 ml) was added. At room temperature, triethyl amine (1.81 g, 10.4 mmol) and t-butyl (R)3-(hydroxymethyl)piperazin-1-carboxylate (1.68 g, 7.8 mmol) were added. The mixture was stirred for 16 hrs at 80°C. After the reaction was completed, the system was cooled to room temperature. The reaction solution was poured into water (60 ml), and then extracted with ethyl acetate (30 ml * 2). The organic layers were combined, washed with saturated brine, dried over anhydrous sodium sulfate and then filtered. The filtrate was concentrated under reduced pressure to obtain a crude product. The crude product was purified by column chromatography on silica gel (petroleum ether: ethyl acetate =40:60) to obtain t-butyl (R)-4-(6-chloro-3-(methoxycarbonyl)-5-(trifluoromethyl)pyridin-2-yl)-3-(hydroxymethyl)piperazin-1-carboxylate as a yellow solid (1.31 g, 5.9 mmol, yield 40%). LCMS (ESI) m/z: **454**$[M+H]^+$

Step IV: Synthesis of t-butyl (R)-2-chloro-5-oxo-3-(trifluoromethyl)-7a, 8, 10, 11-tetrahydro-5H-pyrazino[2, 1-c]pyrido[2, 3-e][1, 4]oxazepane-9(7H)-carboxylate

T-butyl (R)-4-(6-chloro-3-(methoxycarbonyl)-5-(trifluoromethyl)pyridin-2-yl)-3-(hydroxymethyl)piperazin-1-carboxylate (1.52 g, 3.3 mmol) was dissolved in acetonitrile (20 ml), N,N-diisopropylethyl amine (851 mg, 6.7 mmol) was added, and reacted for 12 hrs with stirring under nitrogen atmosphere at 80°C. After the reaction was completed, the system was concentrated under reduced pressure.. The residue was purified by column chromatography on silica gel (petroleum ether: ethyl acetate =50:50) to obtain t-butyl (R)-2-chloro-5-oxo-3-(trifluoromethyl)-7a, 8, 10, 11-tetrahydro-5H-pyrazino[2, 1-c]pyrido[2, 3-e][1, 4]oxazepane-9(7H)-carboxylate as a white solid (1.21 g, 2.8 mmol, 85% yield). LCMS (ESI) m/z: **422**$[M+H]^+$

Step V: Synthesis of t-butyl (R)-2-((t-butoxycarbonyl)amino)-5-oxo-3-(trifluoromethyl)-7a, 8, 10, 11-tetrahydro-5H-pyrazino[2, 1-c]pyrido[2, 3-e][1, 4]oxazepane-9(7H)-carboxylate

T-butyl (R)-2-chloro-5-oxo-3-(trifluoromethyl)-7a, 8, 10, 11-tetrahydro-5H-pyrazino[2, 1-c]pyrido[2, 3-e][1, 4]oxazepane-9(7H)-carboxylate (520 mg, 1.2 mmol) was added to dioxane (10 ml), and then t-butyl aminocarboxylate (280 mg, 2.4 mmol), tris(dibenzylideneacetone) dipalladium (68 mg, 0.12 mmol), 4,5-bis(diphenylphosphine)-9,9-dimethylxanthene (69 mg, 0.28 mmol), and cesium carbonate (0.77g, 2.4 mmol) were added. The mixture was stirred for 3 hrs under nitrogen atmosphere at 80°C. After the reaction was completed, the system was cooled to room temperature. The reaction solution was poured into water (20 ml), and then extracted with ethyl acetate (20 ml*2). The organic layers were combined, washed with saturated brine, dried over anhydrous sodium sulfate and then filtered. The filtrate was concentrated under reduced pressure to obtain a crude product. The crude product was purified by column chromatography on silica gel (petroleum ether: ethyl acetate =50:50) to obtain t-butyl (R)-2-((t-butoxycarbonyl)amino)-5-oxo-3-(trifluoromethyl)-7a, 8, 10, 11-tetrahydro-5H-pyrazino[2, 1-c]pyrido[2, 3-e][1, 4]oxazepane-9(7H)-carboxylate as a white solid (374 mg, 0.74 mmol, yield 61%). LCMS (ESI) m/z: **503**$[M+H]^+$

Step VI: Synthesis of t-butyl (R)-4-(6-(t-butoxycarbonyl)amino)-3-(hydroxymethyl)-5-(trifluoromethyl)pyridin-2-yl)-3-(hydroxymethyl)piperazin-1-carboxylate

T-butyl (R)-2-((t-butoxycarbonyl)amino)-5-oxo-3-(trifluoromethyl)-7a, 8, 10, 11-tetrahydro-5H-pyrazino[2, 1-c]pyrido[2, 3-e][1, 4]oxazepane-9(7H)-carboxylate (374 mg, 0.74 mmol) was dissolved in tetrahydrofuran (5 ml), and sodium borohydride (55 mg, 1.48 mmol) and a boron trifluoride-diethyl ether solution (105mg, 0.74mmol) were added at 0°C. The mixture was stirred at room temperature for 1 hr. After the reaction was completed, the system was quenched by pouring into ice water, and then extracted with ethyl acetate (10 ml*2). The organic layers were combined, washed with saturated brine, dried over anhydrous sodium sulfate, and then filtered. The filtrate was concentrated under reduced pressure, to obtain crude t-butyl (R)-4-(6-(t-butoxycarbonyl)amino)-3-(hydroxymethyl)-5-(trifluoromethyl)pyridin-2-yl)-3-(hydroxymethyl)piperazin-1-carboxylate as a yellow oil (210 mg). The crude product was used directly in the next reaction without purification. LCMS (ESI) m/z: **507**$[M+H]^+$

Step VII: Synthesis of (R)-3-(trifluoromethyl)-7, 7a, 8, 9, 10, 11-hexahydro-5H-pyrazino[2, 1-c]pyrido[2, 3-e][1, 4]oxazepane-2-amine

T-butyl (R)-4-(6-(t-butoxycarbonyl)amino)-3-(hydroxymethyl)-5-(trifluoromethyl)pyridin-2-yl)-3-(hydroxymethyl)piperazin-1-carboxylate (210 mg, 0.41 mmol) was dissolved in dioxane (10 ml), and concentrated sulfuric acid (2 ml) was added dropwise to the system. The mixture was stirred for 1 hr at 60°C. After the reaction was completed, the reaction system was poured into ice water, adjusted to a neutral pH with 2 mol/l sodium carbonate aqueous solution, and then extracted with ethyl acetate (20 ml*2). The organic layers were combined, washed with saturated brine, dried over anhydrous sodium sulfate and then filtered. The filtrate was concentrated under reduced pressure to obtain a crude product. The crude product was purified by column chromatography on silica gel (petroleum ether: ethyl acetate =30:70) to obtain (R)-3-(trifluoromethyl)-7, 7a, 8, 9, 10, 11-hexahydro-5H-pyrazino[2, 1-c]pyrido[2, 3-e][1, 4]oxazepane-2-amine as a white solid (51 mg, 0.17 mmol, yield 41%). LCMS (ESI) m/z: 289[M+H]+

Step VIII: Synthesis of 5-(((S)-1-(3-((R)-2-amino-3-(trifluoromethyl)-7a, 8, 10, 11-tetrahydro-5H-pyrazino[2, 1-c]pyrido[2, 3-e][1, 4]oxazepane-9(7H)-yl)-3-oxypropoxy)prop-2-yl)amino)-4-(trifluoromethyl)pyridazin-3(2H)-one

(R)-3-(trifluoromethyl)-7, 7a, 8, 9, 10, 11-hexahydro-5H-pyrazino[2, 1-c]pyrido[2, 3-e][1, 4]oxazepane-2-amine (33 mg, 0.14 mmol) was dissolved in dichloromethane (5 ml), and then N,N-diisopropylethyl amine (54 mg, 0.42 mmol), 1-propylphosphonic anhydride (133 mg, 0.21 mmol, 50% solution in ethyl acetate) and (S)-3-(2-((6-oxo-5-(trifluoromethyl)-1, 6-dihydropyridazin-4-yl)amino)propoxy)propionic acid (345 mg, 0.112 mmol) were added. The mixture was stirred at room temperature for 3 hrs. After the reaction was completed, the reaction solution was poured into water (10 ml), and extracted with dichloromethane (10 ml*2). The organic layers were combined, washed with saturated brine, dried over anhydrous sodium sulfate and then filtered. The filtrate was concentrated under reduced pressure to obtain a crude product. The crude product was purified by high performance liquid chromatography (chromatographic column: Xsele CSH C18 OBD column 30*150 mm 5 μm; mobile phase A: 0.05% formic acid aqueous solution, mobile phase B: acetonitrile; flow rate: 60 ml/ min; gradient: 3% B to 3% B over 2 min, 20% B to 60% B over 8 min; wavelength: 220 nm; retention time: 7.33 min) to obtain 5-(((S)-1-(3-((R)-2-amino-3-(trifluoromethyl)-7a, 8, 10, 11-tetrahydro-5H-pyrazino[2, 1-c]pyrido[2, 3-e][1, 4]oxazepane-9(7H)-yl)-3-oxypropoxy)prop-2-yl)amino)-4-(trifluoromethyl)pyridazin-3(2H)-one as a white solid (2.28 mg, 0.003 mmol, yield 2.8%).

LCMS (ESI) m/z: 580.20 [M+H] $^+$

HNMR (400 MHz, DMSO-$d_6$): δ ppm 12.46 (s, 1H), 7.91 (s, 1H), 7.46-7.38 (m, 1H), 6.32-6.24 (m, 1H), 6.15-5.99 (m, 2H), 4.72-4.56 (m, 1H), 4.42-4.25 (m, 1H), 4.22-4.07 (m, 1H), 3.98-3.90 (m, 1H), 3.89-3.79 (m, 2H), 3.78-3.54 (m, 6H) ,3.52-3.46 (m, 3H) , 3.27-3.09 (m, 1H), 2.61-2.54 (m, 2H), 1.21-1.11 (m, 3H).

**Example 73:** Synthesis of 5-(((S)-1-(3-oxo-3-((R)-3-(trifluoromethyl)-6a, 7, 9, 10-tetrahydropyrazino[1, 2-d]pyrido[3, 2-b][1, 4]oxathian-8(6H)-yl)propoxy)prop-2-yl)amino)-4-(trifluoromethyl)pyridazin-3(2H)-one **(Compound 78)**

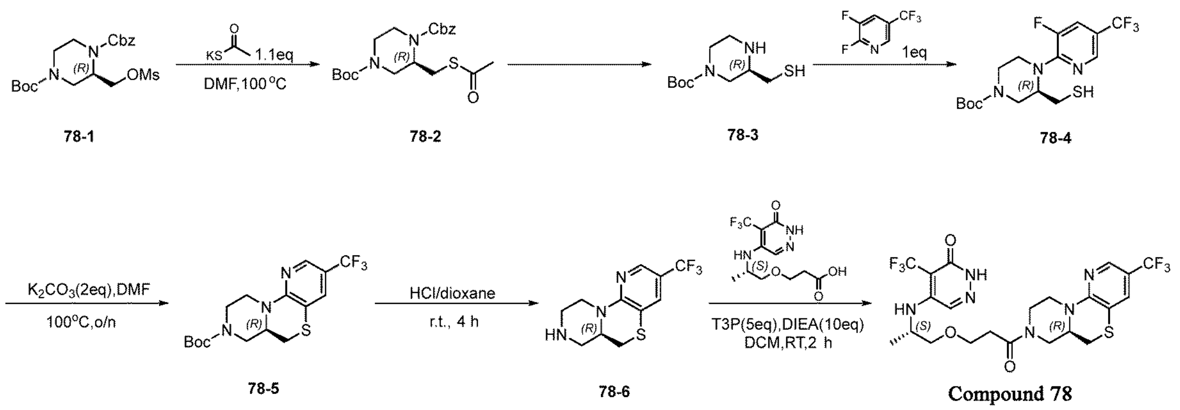


78-1 78-2 78-3 78-4 78-5 78-6 Compound 78

Step I: Synthesis of 1-benzyl 4-(t-butyl) (R)-2-(acetylthio)methyl)piperazin-1, 4-dicarboxylate

Benzyl 4-(t-butyl) (R)-2-(mesyl)oxy)methyl)piperazin-1, 4-dicarboxylate (8.11 g, 19 mmol) and potassium thioacetate (2.36 g, 20.9 mmol) were dissolved in N, N-dimethylformamide (100 ml), and the mixture was reacted for 5 hrs with stirring at 100°C under nitrogen atmosphere. After complete reaction, the system was cooled to room temperature, and filtered. The filtrate was concentrated under reduced pressure, added with water (50 ml), and then extracted with ethyl acetate (25ml*2). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=15:85) to obtain 1-benzyl 4-(t-butyl) (R)-2-(acetylthio)methyl)piperazin-1, 4-dicarboxylate as a yellow solid (4.13 g, 10 mmol, yield 53%). LCMS (ESI) m/z: 409 [M+H]$^+$

Step II: Synthesis of t-butyl (R)-3-(sulfydrylmethyl)piperazin-1-carboxylate

Benzyl 4-(t-butyl) (R)-2-(acetylthio)methyl)piperazin-1, 4-dicarboxylate (4.13 g, 10 mmol) and palladium dichloride (177 mg, 1 mmol) were dissolved in dichloromethane (50 ml), and then triethylsilane (2.32 g, 20 mmol) was added. The mixture was reacted for 12 hrs at room temperature. After the reaction was completed, the mixture was filtered. The filtrate was concentrated under reduced pressure, added with water (30 ml), and extracted with ethyl acetate (15 ml*3). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=40:60) to obtain t-butyl (R)-3-(sulfydrylmethyl)piperazin-1-carboxylate as a yellow oil (1.72 g, 7.3 mmol, yield73%). LCMS (ESI) m/z: 233 [M+H]$^+$

Step III: Synthesis of t-butyl (R)-4-(3-fluoro-5-(trifluoromethyl)pyridin-2-yl)-3-(sulfydrylmethyl)piperazin-1-carboxylate

t-butyl (R)-3-(sulfydrylmethyl)piperazin-1-carboxylate (1.72 g, 7.3 mmol) and 2, 3-difluoro-5-(trifluoromethyl)pyridine(1.41 g, 7.3 mmol) were dissolved in N, N-dimethylformamide (20 ml), and then N,N-diisopropylethyl amine (1.96 g, 15.2 mmol) was added. The mixture was reacted overnight at 80°C. After complete reaction, the system was cooled to room temperature. The reaction solution was poured into water (50 ml), and then extracted with ethyl acetate (25 ml * 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=30:70) to obtain t-butyl(R)-4-(3-fluoro-5-(trifluoromethyl)pyridin-2-yl)-3-(sulfydrylmethyl)piperazin-1-carboxylate as a yellow oil (723 mg, 1.83 mmol, yield 24%). LCMS (ESI) m/z: 396 $[M+H]^+$

Step IV: Synthesis of t-butyl (R)-3-(trifluoromethyl)-6a, 7, 9, 10-tetrahydropyrazino[1, 2-d]pyrido[3, 2-b][1, 4]oxathian-8(6H)-carboxylate

t-butyl (R)-4-(3-fluoro-5-(trifluoromethyl)pyridin-2-yl)-3-(sulfydrylmethyl)piperazin-1-carboxylate (723 mg, 1.83 mmol) was dissolved in N, N-dimethylformamide (10 ml), and then potassium carbonate (505 mg, 3.66 mmol) was added. The mixture was reacted overnight at 100°C. After complete reaction, the system was cooled to room temperature. The reaction solution was poured into water (25 ml), and extracted with ethyl acetate (10 ml × 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=20:80) to obtain t-butyl (R)-3-(trifluoromethyl)-6a,7, 9, 10-tetrahydropyrazino[1, 2-d]pyrido[3, 2-b][1, 4]oxathian-8(6H)-carboxylate as a yellow solid (240 mg, 0.64 mmol, yield 35%). LCMS (ESI) m/z: 376 $[M+H]^+$

Step V: Synthesis of (R)-3-(trifluoromethyl)-6, 6a, 7, 8, 9, 10-hexahydropyrazino[1, 2-d]pyrido[3, 2-b][1, 4]oxathiane

t-butyl (R)-3-(trifluoromethyl)-6a, 7, 9, 10tetrahydropyrazino[1, 2-d]pyrido[3, 2-b][1, 4]oxathian-8(6H)-carboxylate (240 mg, 0.64 mmol) was dissolved in ice-cold (0°C) hydrogen chloride solution in 1, 4-dioxane (5 ml, 4 mol/l). The mixture was slowly warmed to room temperature and then stirred for 2 hrs. After complete reaction, the reaction solution was concentrated under reduced pressure. The crude product was diluted in water (30 ml), adjusted to pH 8-9 with sodium bicarbonate, and then extracted with dichloromethane (15 ml × 3). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=40:60) to obtain (R)-3-(trifluoromethyl)-6, 6a, 7, 8, 9, 10-hexahydropyrazino[1, 2-d]pyrido[3, 2-b][1, 4]oxathiane as a yellow solid (150 mg, 0.55 mmol, yield91%). LCMS (ESI) m/z: 276 $[M+H]^+$

Step VI: Synthesis of 5-(((S)-1-(3-oxo-3-((R)-3-(trifluoromethyl)-6a, 7, 9, 10-tetrahydropyrazino[1, 2-d]pyrido[3, 2-b][1, 4]oxathian-8(6H)-yl)propoxy)prop-2-yl)amino)-4-(trifluoromethyl)pyridazin- 3 (2H) -one

(S)-3-(2-((6-oxo-5-(trifluoromethyl)-1, 6-dihydropyridazin-4-yl)amino)propoxy)propionic acid (170 mg, 0.55 mmol) and (R)-3-(trifluoromethyl)-6, 6a, 7, 8, 9, 10-hexahydropyrazino[1, 2-d]pyrido[3, 2-b][1, 4]oxathiane (150 mg, 0.55 mmol) were dissolved in dichloromethane (5 .0 ml), and then N,N-diisopropylethyl amine (213 mg, 1.65 mmol) and 1-propylphosphonic anhydride (525 mg, 0.825 mmol, 50% solution in ethyl acetate) were added. The mixture was stirred at room temperature for 2 hrs. After complete reaction, the reaction solution was poured into ice water (20 ml), and then extracted with ethyl acetate (10 ml × 3). The organic phases were combined, washed with saturated brine, and dried over anhydrous sodium sulfate. Anhydrous sodium sulfate was removed by filtration, and the filtrate was concentrated. The crude product was purified by high performance liquid chromatography (chromatographic column: YMC-Actus Triart C18, 30*150 mm, 5 μm; mobile phase A: 10 mol/l ammonium bicarbonate aqueous solution, mobile phase B: acetonitrile, flow rate: 60 ml/ min, elution gradient: mobile phase B from 20% to 60% in 8 min, detection wavelength: 220 nm, retention time: 7.57 min) to obtain 5-(((S)-1-(3-oxo-3-((R)-3-(trifluoromethyl)-6a, 7, 9, 10-tetrahydropyrazino[1, 2-d]pyrido[3, 2-b][1, 4]oxathian-8(6H)-yl)propoxy)prop-2-yl)amino)-4-(trifluoromethyl)pyridazin-3(2H)-one as a white solid (77.21 mg, 0.13 mmol, yield23%).

LCMS(ESI)m/z: 567.10$[M+H]^+$

HNMR (400 MHz, DMSO-$d_6$): δ ppm 12.46 (s, 1H), 8.08(s, 1H), 7.92 (s, 1H), 7.66-7.32 (m, 1H), 6.46-6.10 (m, 1H), 4.44-4.21 (m, 1H), 4.21-4.10 (m, 1H), 4.10-3.83 (m, 2H), 3.76-3.57 (m, 2H), 3.55-3.38 (m, 3H), 3.30-3.17 (m, 2H), 3.17-2.96 (m, 2H), 2.95-2.79 (m, 1H), 2.73-2.57 (m, 2H), 1.15-1.09 (m, 3H).

**Example 74:** Synthesis of (R)-9-(3-((S)-2-((6-oxo-5-(trifluoromethyl)-1, 6-dihydropyridazin-4-yl)amino)propoxy)propionyl)-3-(trifluoromethyl)-5, 6, 8, 9, 10, 11-hexahydropyrazino[1, 2-a]pyrido[3, 2-f][1, 4]diazepin-7(7aH)-one **(Compound 79)**

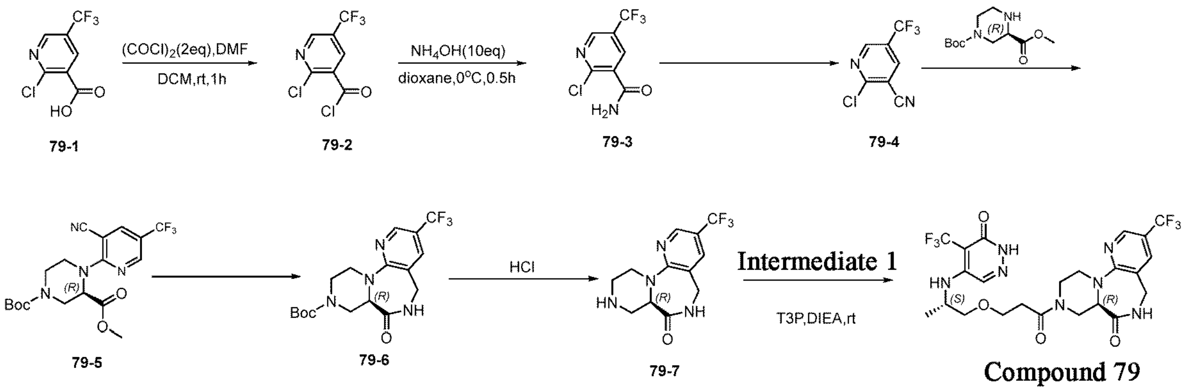


Step I: Synthesis of 2-chloro-5-(trifluoromethyl)nicotinyl chloride

chloro-5-(trifluoromethyl)nicotinic acid (4.01 g, 17.73 mmol) was added to dichloromethane (100.00 ml), and cooled to 0°C. N,N-dimethylformamide (65 mg, 0.89 mmol) and oxalyl chloride (4.52 g, 35.47 mmol) were added, and then warmed to room temperature. The reaction was stirred at room temperature for 1 hr. After the reaction was completed, the system was directly concentrated under reduced pressure to obtain crude 2-chloro-5-(trifluoromethyl)nicotinyl chloride as a yellow solid (4.81 g). LCMS (ESI) m/z: 244 $[M+H]^+$

Step II: Synthesis of 2-chloro-5-(trifluoromethyl)nicotinamide

chloro-5-(trifluoromethyl)nicotinyl chloride (4.81 g, 19.67 mmol) was dissolved in 1,4-dioxane (11 ml), and then aqueous ammonia (10 ml, 28%) was added. The reaction was stirred at 0°C for 0.5 hr. After the reaction was completed, the reaction solution was poured into water (20 ml), and then extracted with ethyl acetate (10 ml*3). The organic phases were combined, washed with saturated brine, and dried over anhydrous sodium sulfate. Anhydrous sodium sulfate was removed by filtration, and the filtrate was concentrated to obtain crude 2-chloro-5-(trifluoromethyl)nicotinamide as a white solid (3.71 g). LCMS (ESI) m/z: 225 $[M+H]^+$

Step III: 2Synthesis of -chloro-5-(trifluoromethyl)nicotinonitrile

Chloro-5-(trifluoromethyl)nicotinamide (2.01 g, 8.91 mmol) was dissolved in phosphorus oxychloride (20.00 ml), and the mixture was stirred for 2 hrs at 100°C. After the reaction was completed, the system was directly concentrated under reduced pressure to obtain crude 2-chloro-5-(trifluoromethyl)nicotinonitrile as a yellow solid (1.37 g). LCMS (ESI) m/z: 207 $[M+H]^+$

Step IV: Synthesis of 1-(t-butyl) 3-methyl (R)-4-(3-cyano-5-(trifluoromethyl)pyridin-2-yl)piperazin-1, 3-dicarboxylate

1-(T-butyl) 3-methyl (R)-piperazin-1, 3-dicarboxylate (709 mg, 2.90 mmol) was dissolved in N-methylpyrrolidone (40.00 ml), and then potassium fluoride (337 mg, 5.81 mmol) and 2-chloro-5-(trifluoromethyl)nicotinonitrile (400 mg, 1.94 mmol) were added. The reaction was stirred at 95°C for 3 hrs. After the reaction was completed, the system was cooled to room temperature. The reaction solution was poured into water (100 ml), and then extracted with ethyl acetate (50 ml * 2). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=20:80) to obtain 1-(t-butyl) 3-methyl (R)-4-(3-cyano-5-(trifluoromethyl)pyridin-2-yl)piperazin-1, 3-dicarboxylate as a yellow oil (210 mg, 0.504mmol, yield26%). LCMS (ESI) m/z: 415 $[M+H]^+$

Step V: Synthesis of t-butyl (R)-7-oxo-3-(trifluoromethyl)-6, 7, 7a, 8, 10, 11-hexahydropyrazino[1, 2-a]pyrido[3, 2-f][1, 4]diazepin-9(5H)-carboxylate

1-(T-butyl) 3-methyl (R)-4-(3-cyano-5-(trifluoromethyl)pyridin-2-yl)piperazin-1, 3-dicarboxylate (430 mg, 1.04 mmol) was dissolved in methanol (20.00 ml), and then sodium borohydride (392 mg, 10.38 mmol) and cobalt chloride (202 mg, 1.56 mmol) were added. The mixture was stirred overnight at 60°C. After the reaction was completed, the system was cooled to room temperature. The reaction solution was poured into water (20 ml), and extracted with ethyl acetate (10 ml * 2). The organic phases were combined, washed with saturated brine, and dried over anhydrous sodium sulfate. Anhydrous sodium sulfate was removed by filtration, and the filtrate was concentrated to obtain the crude product t-butyl (R)-7-oxo-3-(trifluoromethyl)-6, 7, 7a, 8, 10, 11-hexahydropyrazino[1, 2-a]pyrido[3, 2-f][1, 4]diazepin-9(5H)-carboxylate as a yellow oil (250 mg). LCMS (ESI) m/z: 387 $[M+H]^+$

Step VI: Synthesis of (R)-3-(trifluoromethyl)-5, 6, 8, 9, 10, 11-hexahydropyrazino[1, 2-a]pyrido[3, 2-f][1, 4]diazepin-7(7aH)-one

t-butyl (R)-7-oxo-3-(trifluoromethyl)-6, 7, 7a, 8, 10, 11-hexahydropyrazino[1, 2-a]pyrido[3, 2-f][1, 4]diazepin-9(5H)-carboxylate (240 mg, 0.62 mmol) was dissolved in a hydrogen chloride solution in 1,4-dioxane (3.00 ml, 4 mol/l). The reaction was stirred at room temperature for 0.5 hr. After the reaction was completed, the system was directly concentrated under reduced pressure to obtain crude (R)-3-(trifluoromethyl)-5, 6, 8, 9, 10, 11-hexahydropyrazino[1, 2-a]pyrido[3, 2-f][1, 4]diazepan-7(7aH)-one as a yellow solid (230 mg). LCMS (ESI) m/z: 287 $[M+H]^+$

Step VII: Synthesis of (R)-9-(3-((S)-2-((6-oxo-5-(trifluoromethyl)-1, 6-dihydropyridazin-4-yl)amino)propoxy)propionyl)-3-(trifluoromethyl)-5, 6, 8, 9, 10, 11-hexahydropyrazino[1, 2-a]pyrido[3, 2-f][1, 4]diazepin-7(7aH)-one

(R)-3-(trifluoromethyl)-5, 6, 8, 9, 10, 11-hexahydropyrazino[1, 2-a]pyrido[3, 2-f][1, 4]diazepin-7(7aH)-one (60 mg, 0.19 mmol) was dissolved in dichloromethane (3.00 ml), and then N,N-diisopropylethyl amine (0.25 g, 1.94 mmol), (S)-3-(2-((6-oxo-5-(trifluoromethyl)-1, 6-dihydropyridazin-4-yl)amino)propoxy)propionic acid(58mg, 0.19 mmol), and 1-propylphosphonic anhydride (0.62g, 0.97 mmol, 50% solution in ethyl acetate) were added. The reaction was stirred at room temperature for 0.5 hr. After the reaction was completed, the reaction solution was poured into water (10 ml), and extracted with dichloromethane (10 ml*2). The organic phases were combined, washed with saturated brine, and dried over anhydrous sodium sulfate. Anhydrous sodium sulfate was removed by filtration, and the filtrate was concentrated. The crude product was purified by high performance liquid chromatography (chromatographic column: YMC-Actus Triart C18, 30*150 mm, 5 μm; mobile phase A: 10 mmol/l ammonium bicarbonate aqueous solution, mobile phase B: acetonitrile; flow rate: 60 ml/ min; gradient: 20% B to 65% B in 8 min; detection wavelength: 220 nm; retention time: 7.57 min) to obtain (R)-9-(3-((S)-2-((6-oxo-5-(trifluoromethyl)-1, 6-dihydropyridazin-4-yl)amino)propoxy)propionyl)-3-(trifluoromethyl)-5, 6, 8, 9, 10, 11-hexahydropyrazino[1, 2-a]pyrido[3, 2-f][1, 4]diazepin-7(7aH)-one as a white solid (10.33 mg, 0.0179 mmol, yield: 9%).

LCMS (ESI)m/z: 578.10$[M+H]^+$

$^1$HNMR (400 MHz, DMSO-$d_6$) δ (ppm):: 12.45 (s, 1H), 8.40-8.31 (m, 1H), 8.31-8.20 (s, 1H), 7.98-7.88 (m, 1H), 7.88-7.69 (m, 1H),6.36-6.18 (m, 1H), 5.11-4.95 (m, 1H), 4.95-4.77 (m, 1H), 4.49-4.26 (m, 1H), 4.26-4.08 (m, 1H), 4.08-3.90(m,2H), 3.78-3.62 (m, 4H), 3.62-3.35 (m, 5H), 2.70-2.64 (m, 1H), 1.25-1.05 (m, 3H).

**Example 75:** Synthesis of 5-(((S)-1-(3-((7aR, 10S)-10-methyl-3-(trifluoromethyl)-7a, 8, 10, 11-tetrahydro-5H-pyrazino[2, 1-c]pyrido[2, 3-e][1, 4]oxazepane-9(7H)-yl)-3-oxypropoxy)prop-2-yl)amino)-4-(trifluoromethyl)pyridazin-3(2H)-one **(Compound 80)**

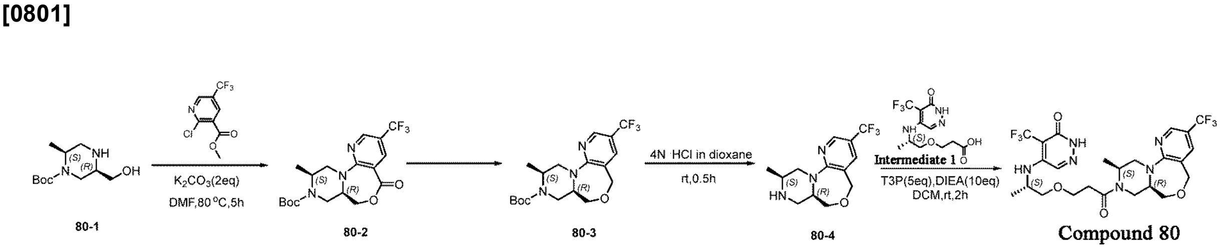


80-1 80-2 80-3 80-4 Compound 80

Step I: Synthesis oft-butyl (7aR, 10S)-10-methyl-5-oxo-3-(trifluoromethyl)-7a, 8, 10, 11-tetrahydro-SH-pyrazino[2, 1-c]pyrido[2, 3-e][1, 4]oxazepane-9(7H)-carboxylate

t-butyl (2S, SR)-5-(hydroxymethyl)-2-methylpiperazin-1-carboxylate (500 mg, 2.17 mmol) was dissolved in N,N-dimethylformamide (20.00 ml), and then potassium carbonate (600 mg, 4.34 mmol) and methyl 2-chloro-5-(trifluoromethyl)nicotinate (624 mg, 2.61 mmol) were added. The reaction was stirred at 80°C for 5 hrs. After the reaction was completed, the system was cooled to room temperature. The reaction solution was poured into water (50 ml), and then extracted with ethyl acetate (20 ml * 2). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=20:80) to obtain t-butyl (7aR, 10S)-10-methyl-5-oxo-3-(trifluoromethyl)-7a, 8, 10, 11-tetrahydro-5H-pyrazino[2, 1-c]pyrido[2, 3-e][1, 4]oxazepane-9(7H)-carboxylate as a yellow solid (240 mg, 0.607 mmol, yield 28%). LCMS (ESI) m/z: 402 $[M+H]^+$

Step II: Synthesis of t-butyl (7aR, 10S)-10-methyl-3-(trifluoromethyl)-7a, 8, 10, 11-tetrahydro-5H-pyrazino[2, 1-c]pyrido[2, 3-e][1, 4]oxazepane-9(7H)-carboxylate

t-butyl (7aR, 10S)-10-methyl-5-oxo-3-(trifluoromethyl)-7a, 8, 10, 11-tetrahydro-5H-pyrazino[2, 1-c]pyrido[2, 3-e][1, 4]oxazepane-9(7H)-carboxylate (840 mg, 2.09 mmol) was dissolved in tetrahydrofuran (6.00 ml) , and cooled to 0°C. Boron trifluoride-diethyl etherate (4.45g, 31.39 mmol) and sodium borohydride (3.17 g, 83.71 mmol) were added, and then warmed to room temperature. The mixture was stirred at room temperature for 0.5 hr. After the reaction was completed, the reaction solution was poured into water (20 ml), and then extracted with ethyl acetate (10 ml * 2). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=20:80) to obtain t-butyl (7aR, 10S)-10-methyl-3-(trifluoromethyl)-7a, 8, 10, 11-tetrahydro-5H-pyrazino[2, 1-c]pyrido[2, 3-e][1, 4]oxazepane-9(7H)-carboxylate as a yellow oil (180 mg, 0.459mmol, yield22%). LCMS (ESI) m/z: 388 $[M+H]^+$

Step III: Synthesis of (7aR, 10S)-10-methyl-3-(trifluoromethyl)-7, 7a, 8, 9, 10, 11-hexahydro-5H-pyrazino[2, 1-c]pyrido[2, 3-e][1, 4]oxazepane

t-butyl (7aR, 10S)-10-methyl-3-(trifluoromethyl)-7a, 8, 10, 11-tetrahydro-5H-pyrazino[2, 1-c]pyrido[2, 3-e][1, 4]oxazepane-9(7H)-carboxylate (210 mg, 0.54 mmol) was dissolved in a hydrogen chloride solution in 1,4-dioxane (3.00 ml, 4 mol/l). The mixture was stirred at room temperature for 0.5 hr. After the reaction was completed, the system was directly concentrated under reduced pressure to obtain crude (7aR, 10S)-10-methyl-3-(trifluoromethyl)-7, 7a, 8, 9, 10, 11-hexahydro-5H-pyrazino[2, 1-c]pyrido[2, 3-e][1, 4]oxazepane as a yellow oil (180 mg). LCMS (ESI) m/z: 288 $[M+H]^+$

Step IV: Synthesis of 5-(((S)-1-(3-((7aR, 10S)-10-methyl-3-(trifluoromethyl)-7a, 8, 10, 11-tetrahydro-5H-pyrazino[2, 1-c]pyrido[2, 3-e][1, 4]oxazepane-9(7H)-yl)-3-oxypropoxy)prop-2-yl)amino)-4-(trifluoromethyl)pyridazin-3(2H)-one

(7aR, 10S)-10-methyl-3-(trifluoromethyl)-7, 7a, 8, 9, 10, 11-hexahydro-5H-pyrazino[2, 1-c]pyrido[2, 3-e][1, 4]oxazepane (60mg, 0.21 mmol) was dissolved in dichloromethane (3.00 ml), and then N,N-diisopropylethyl amine (270 mg, 2.09 mmol), (S)-3-(2-((6-oxo-5-(trifluoromethyl)-1, 6-dihydropyridazin-4-yl)amino)propoxy)propionic acid (57mg, 0.21 mmol) and 1-propylphosphonic anhydride (660 mg, 1.04 mmol, 50% solution in ethyl acetate) were added. The reaction was stirred at room temperature for 0.5 hr. After the reaction was completed, the reaction solution was poured into water (10 ml), and extracted with dichloromethane (10 ml * 2). The organic phases were combined, washed with saturated brine, and dried over anhydrous sodium sulfate. Anhydrous sodium sulfate was removed by filtration, and the filtrate was concentrated. The crude product was purified by high performance liquid chromatography (chromatographic column: YMC-Actus Triart C18, 30*150 mm, 5 μm; mobile phase A: 10 mmol/l ammonium bicarbonate aqueous solution, mobile phase B: acetonitrile; flow rate: 60 ml/ min; gradient: 20% B to 50% B in 8 min; detection wavelength: 220 nm; retention time: 7.15 min) to obtain 5-(((S)-1-(3-((7aR, 10S)-10-methyl-3-(trifluoromethyl)-7a, 8, 10, 11-tetrahydro-5H-pyrazino[2, 1-c]pyrido[2, 3-e][1, 4]oxazepane-9(7H)-yl)-3-oxypropoxy)prop-2-yl)amino)-4-(trifluoromethyl)pyridazin-3(2H)-one as a white solid (15.61 mg, 0.027 mmol, yield 13%).

LCMS (ESI)m/z: 579.15$[M+H]^+$

$^1$HNMR(400 MHz, DMSO-$d_6$)$\delta$ (ppm): 12.46 (s, 1H), 8.45-8.27 (m,1H), 7.94-7.78 (m, 1H), 7.78-7.69 (m, 1H), 6.30-6.17 (m, 1H), 5.01-4.91 (m, 1H), 4.59-4.43 (m, 1H), 4.32-4.19 (m, 1H), 4.19-3.96 (m, 3H), 3.91-3.73 (m, 3H), 3.73-3.53 (m, 3H), 3.48-3.42 (m,2H), 3.31-3.28 (m, 1H), 2.63-2.54 (m, 2H), 1.20-1.01 (m, 6H).

**Example 76** Synthesis of 5-(((S)-1-(3-oxo-3-((S)-3-(trifluoromethyl)-6a, 7, 9, 10-tetrahydropyrazino[1, 2-d]pyrido[3, 2-b][1, 4]oxathianyl-8(6H)-yl)propoxy)prop-2-yl)amino)-4-(trifluoromethyl)pyridazin-3(2H)-one **(Compound 81)**

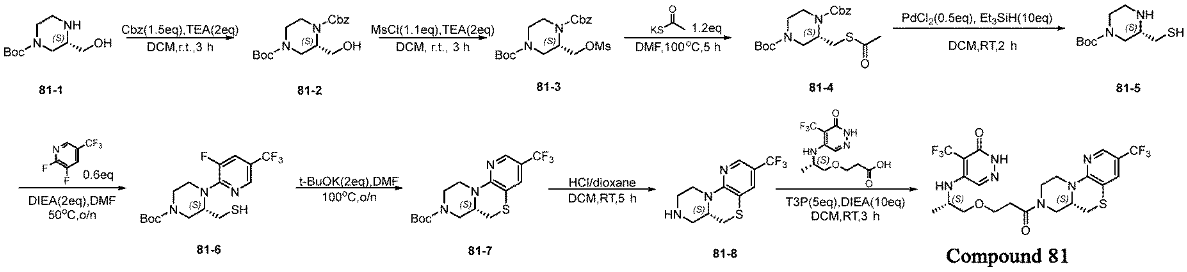


81-1 81-2 81-3 81-4 81-5

81-6 81-7 81-8 **Compound 81**

Step I: Synthesis of 1-benzyl4-(t-butyl)(S)-2-(hydroxymethyl)piperazin-1, 4-dicarboxylate

t-butyl (S)-3-(hydroxymethyl)piperazin-1-carboxylate (5.02 g, 23 mmol) and triethyl amine (4.65 g, 46 mmol) were dissolved in dichloromethane (50 ml), and benzyl chloroformate (7.82 g, 46 mmol) was added dropwise to the mixed solution. The mixture was reacted for 3 hrs with stirring at room temperature. After complete reaction, the mixture was filtered. The filtrate was concentrated under reduced pressure, and the residue was added with water (30 ml), and extracted with ethyl acetate (15 ml * 2). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=30:70) to obtain 1-benzyl 4-(t-butyl) (S)-2-(hydroxymethyl)piperazin-1, 4-dicarboxylate as a yellow oil (7.76 g, 22 mmol, yield 96%).

LCMS (ESI) m/z: 351 $[M+H]^+$

Step II: Synthesis of 1-benzyl 4-(t-butyl)(S)-2-(mesyl)oxy)methyl)piperazin-1,4-dicarboxylate

benzyl 4-(t-butyl)(S)-2-(hydroxymethyl)piperazin-1, 4-dicarboxylate (7.76 g, 22 mmol) and triethyl amine (4.41 g, 44 mmol) were dissolved in dichloromethane (70 ml), and then methanesulfonyl chloride (5.02 g, 44 mmol) was added dropwise to the mixed solution. The mixture was reacted with stirring at room temperature for 3 hrs. After complete reaction, the reaction solution was poured into water (100 ml) and extracted with dichloromethane (50 ml * 2). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=20:80) to obtain 1-benzyl 4-(t-butyl) (S)-2-(mesyl)oxy)methyl)piperazin-1, 4-dicarboxylate as a yellow oil (8.61 g, 20 mmol, yield 91%). LCMS (ESI) m/z: 429 $[M+H]^+$

Step III: Synthesis of 1-benzyl 4-(t-butyl) (S)-2-(acetylthio)methyl)piperazin-1, 4-dicarboxylate

Benzyl 4-(t-butyl) (S)-2-(mesyl)oxy)methyl)piperazin-1, 4-dicarboxylate (8.61 g, 20 mmol) and potassium thioacetate (2.62 g, 22 mmol) were dissolved in N,N-dimethylformamide (100 ml), and the mixture was reacted for 5 hrs with stirring at 100°C. After complete reaction, the system was cooled to room temperature. The reaction solution was poured into water (200 ml), and extracted with ethyl acetate (50 ml*3). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=20:80) to obtain 1-benzyl 4-(t-butyl) (S)-2-(acetylthio)methyl)piperazin-1, 4-dicarboxylate as a yellow solid (5.23 g, 13 mmol, yield 63%). LCMS (ESI) m/z: 409 $[M+H]^+$

Step IV: Synthesis of t-butyl (S)-3-(sulfydrylmethyl)piperazin-1-carboxylate

Benzyl 4-(t-butyl) (S)-2-(acetylthio)methyl)piperazin-1, 4-dicarboxylate (2.02 g, 5 mmol) and palladium dichloride (88 mg, 0. 5 mmol) were dissolved in dichloromethane (20 ml), and then triethylsilane (1.16 g, 10 mmol) was added. The mixture was reacted for 2 hrs with stirring at room temperature. After complete reaction, the system was directly concentrated under reduced pressure. The residue was purified by column chromatography on silica gel (ethyl acetate : petroleum ether=40:60) to obtain t-butyl (S)-3-(sulfydrylmethyl)piperazin-1-carboxylate as a yellow oil (900 mg, 3.9 mmol, yield78%). LCMS (ESI) m/z: 233 $[M+H]^+$

Step V: Synthesis of t-butyl (S)-4-(3-fluoro-5-(trifluoromethyl)pyridin-2-yl)-3-(sulfydrylmethyl)piperazin-1-carboxylate

T-butyl (S)-3-(sulfydrylmethyl)piperazin-1-carboxylate (900 mg, 3.9 mmol) and 2,3-difluoro-5-(trifluoromethyl)pyridine (714 mg, 3.9 mmol) were dissolved in N,N-dimethylformamide (10 ml), and then N,N-diisopropylethyl amine (1.01 g, 7.8 mmol) was added. The mixture was reacted overnight with stirring at 80°C. After complete reaction, the system was cooled to room temperature. The reaction solution was poured into water (20 ml), and then extracted with ethyl acetate (10 ml*2). The reaction mixture was cooled and filtered. The filtrate was concentrated under reduced pressure, added with water (30 ml), and extracted with ethyl acetate. The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=30:70) to obtain t-butyl (S)-4-(3-fluoro-5-(trifluoromethyl)pyridin-2-yl)-3-(sulfydrylmethyl)piperazin-1-carboxylate as a yellow oil (308 mg, 0.78 mmol, yield20%). LCMS (ESI) m/z: 396 $[M+H]^+$

Step VI: Synthesis of t-butyl (S)-3-(trifluoromethyl)-6a, 7, 9, 10-tetrahydropyrazino[1, 2-d]pyrido[3, 2-b][1, 4]oxathianyl-8(6H)-carboxylate

t-butyl (S)-4-(3-fluoro-5-(trifluoromethyl)pyridin-2-yl)-3-(sulfydrylmethyl)piperazin-1-carboxylate (308 mg, 0.78 mmol) was dissolved in N, N-dimethylformamide (5 ml), and then potassium carbonate (215 mg, 1.56 mmol) was added. The mixture was reacted for 12 hrs with stirring at 100°C. After complete reaction, the system was cooled to room temperature. The reaction solution was poured into water (25 ml), and then extracted with ethyl acetate (10 ml *2). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=20:80) to obtain t-butyl (S)-3-(trifluoromethyl)-6a, 7, 9, 10-tetrahydropyrazino[1, 2-d]pyrido[3, 2-b][1, 4]oxathianyl-8(6H)-carboxylate as a yellow oil (100 mg, 0.27 mmol, yield 34%). LCMS (ESI) m/z: 376 $[M+H]^+$

Step VII: Synthesis of (S)-3-(trifluoromethyl)-6, 6a, 7, 8, 9, 10-hexahydropyrazino[1, 2-d]pyrido[3, 2-b][1, 4]oxathiane

T-butyl (S)-3-(trifluoromethyl)-6a, 7, 9, 10-tetrahydropyrazino[1, 2-d]pyrido[3, 2-b][1, 4]oxathian-8(6H)-carboxylate (100 mg, 0.27 mmol) was dissolved in ice-cold (0°C) hydrogen chloride/1, 4-dioxane solution (5 ml, 4 mol/l). The reaction mixture was slowly warmed to room temperature and reacted for 5 hrs with stirring. After complete reaction, the reaction solution was concentrated under reduced pressure to obtain crude (S)-3-(trifluoromethyl)-6, 6a, 7, 8, 9, 10-hexahydropyrazino[1, 2-d]pyrido[3, 2-b][1, 4]oxathiane as a yellow solid (60 mg). LCMS (ESI) m/z: 276 $[M+H]^+$

Step VIII: Synthesis of 5-(((S)-1-(3-oxo-3-((S)-3-(trifluoromethyl)-6a, 7, 9, 10-tetrahydropyrazino[1, 2-d]pyrido[3, 2-b][1, 4]oxathianyl-8(6H)-yl)propoxy)prop-2-yl)amino)-4-(trifluoromethyl)pyridazin-3(2H)-one

(S)-3-(2-((6-oxo-5-(trifluoromethyl)-1, 6-dihydropyridazin-4-yl)amino)propoxy)propionic acid (68 mg, 0.22 mmol) and (S)-3-(trifluoromethyl)-6, 6a, 7, 8, 9, 10-hexahydropyrazino[1, 2-d]pyrido[3, 2-b][1, 4]oxathianyl (60 mg, 0.22 mmol) were dissolved in dichloromethane (5 ml), and then N,N-diisopropylethyl amine (85 mg, 0.66 mmol) and 1-propylphosphonic anhydride (210 mg, 0.33 mmol, 50% solution in ethyl acetate) were added. The mixture was stirred at room temperature for 3 hrs. After complete reaction, the reaction solution was poured into water (20 ml), and then extracted with dichloromethane (10 ml × 2). The organic phases were combined, washed with saturated brine, and dried over anhydrous sodium sulfate. Anhydrous sodium sulfate was removed by filtration, and the filtrate was concentrated. The crude product was purified by high performance liquid chromatography (chromatographic column: YMC-Actus Triart C18, 30*150 mm, 5 μm; mobile phase A: 10 mol/l ammonium bicarbonate aqueous solution, mobile phase B: acetonitrile, flow rate: 60 ml/ min, elution gradient: mobile phase B from 25% to 55% over 8 min, detection wavelength: 220 nm, retention time: 7.68 min) to obtain 5-(((S)-1-(3-oxo-3-((S)-3-(trifluoromethyl)-6a, 7, 9, 10-tetrahydropyrazino[1, 2-d]pyrido[3, 2-b][1, 4]oxathianyl-8(6H)-yl)propoxy)prop-2-yl)amino)-4-(trifluoromethyl)pyridazin-3(2H)-one as a white solid (1.54 mg, 0.003 mmol, yield 1.23%).

LCMS (ESI)m/z: 567.10$[M+H]^+$

$^1$HNMR(400 MHz, DMSO-$d_6$)δ (ppm): 12.45 (s, 1H), 8.08 (s, 1H), 7.91 (s, 1H), 7.47 (d, J = 6.9 Hz, 1H), 6.27 (d, J = 4.4 Hz, 1H), 4.41-4.26 (m, 1H), 4.22-4.01 (m, 2H), 4.00-3.85 (m, 2H), 3.71-3.66 (m, 2H), 3.50-3.48 (m, 2H), 3.26-3.22 (m, 1H), 3.16-3.10 (m, 1H), 3.09-3.01 (m, 1H), 2.94-2.82 (m, 1H) , 2.72-2.66 (m, 1H) , 2.64-2.53 (m, 2H)1.15(d, J = 4.0 Hz, 3H).

**Example 76:** Synthesis of 5-(((S)-1-(3-oxo-3-((S)-3-(trifluoromethyl)-7a, 8, 10, 11-tetrahydro-5H-pyrazino[2, 1-c]pyrido[2, 3-e][1, 4]oxazepanyl -9(7H)-yl)propoxy)prop-2-yl)amino)-4-(trifluoromethyl)pyridazin-3(2H)-one **(Compound 82)**

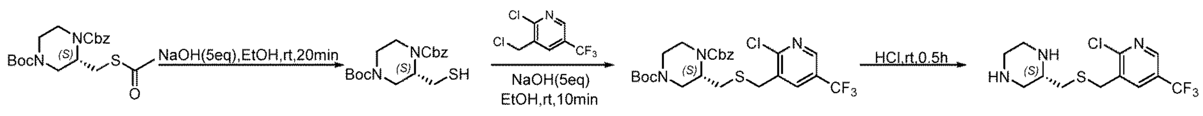


82-1 82-2 82-3 82-4

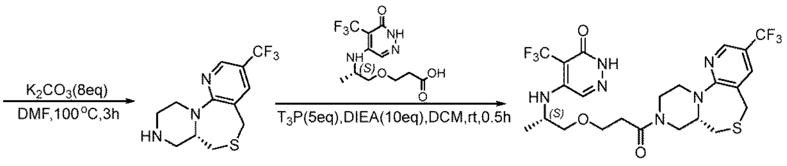


82-5 Compound 82

Step I: Synthesis of 1-benzyl 4-(t-butyl) (S)-2-(sulfydrylmethyl)piperazin-1, 4-dicarboxylate

Benzyl 4-(t-butyl) (S)-2-(acetylthio)methyl)piperazin-1, 4-dicarboxylate (1.10 g, 2.69 mmol) was dissolved in ethanol (5.00 ml), and then sodium hydroxide (538 mg, 13.46 mmol) was added. The reaction was stirred at room temperature for 20 min. After the reaction was completed, the reaction solution was poured into water (25 ml), and then extracted with ethyl acetate (10 ml * 2). The organic phases were combined, washed with saturated brine, and dried over anhydrous sodium sulfate. Anhydrous sodium sulfate was removed by filtration, and the filtrate was concentrated, to obtain crude 1-benzyl 4-(t-butyl) (S)-2-(sulfydrylmethyl)piperazin-1, 4-dicarboxylate as a yellow oil (1.42 g). LCMS (ESI) m/z: 367 $[M+H]^+$

Step II: Synthesis of 1-benzyl 4-(t-butyl) (S)-2-((((2-chloro-5-(trifluoromethyl)pyridin-3-yl)methyl)thio)methyl)piperazin-1, 4-dicarboxylate

Benzyl 4-(t-butyl) (S)-2-(sulfydrylmethyl)piperazin-1, 4-dicarboxylate (1.21 g, 3.27 mmol) was dissolved in ethanol (20.00 ml), and then sodium hydroxide (654 mg, 16.37 mmol) and 2-chloro-3-(chloromethyl)-5-(trifluoromethyl)pyridine (903 mg, 3.93 mmol) were added. The reaction was stirred at room temperature for 10 min. After the reaction was completed, the reaction solution was poured into water (25 ml), and then extracted with ethyl acetate (10 ml * 2). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=20:80) to obtain 1-benzyl 4-(t-butyl) (S)-2-((((2-chloro-5-(trifluoromethyl)pyridin-3-yl)methyl)thio)methyl)piperazin-1,4-dicarboxylate as a yellow oil (1.82 g, 3.20 mmol, yield 98%). LCMS (ESI) m/z: 560 $[M+H]^+$

Step III: Synthesis of (S)-2-((((2-chloro-5-(trifluoromethyl)pyridin-3-yl)methyl)thio)methyl)piperazine

Benzyl 4-(t-butyl) (S)-2-((((2-chloro-5-(trifluoromethyl)pyridin-3-yl)methyl)thio)methyl)piperazin-1, 4-dicarboxylate (0.20 g, 0.36 mmol) was dissolved in concentrated hydrochloric acid (2.00 ml), and the reaction was stirred for 0.5 hr at 50°C. After the reaction was completed, the system was directly concentrated under reduced pressure, to obtain crude (S)-2-((((2-chloro-5-(trifluoromethyl)pyridin-3-yl)methyl)thio)methyl)piperazine as a yellow solid (0.21 g). LCMS (ESI) m/z: 326 $[M+H]^+$

Step IV: Synthesis of (S)-3-(trifluoromethyl)-7, 7a, 8, 9, 10, 11-hexahydro-5H-pyrazino[2, 1-c]pyrido[2, 3-e][1, 4]oxazepane

(S)-2-((((2-chloro-5-(trifluoromethyl)pyridin-3-yl)methyl)thio)methyl)piperazine (200 mg, 0.61 mmol) was dissolved in N,N-dimethylformamide (10.00 ml), and then potassium carbonate (678 mg, 4.91 mmol) was added. The mixture was reacted for 3 hrs at 100°C. After the reaction was completed, the system was cooled to room temperature. The reaction solution was poured into water (25 ml), and then extracted with ethyl acetate (10 ml * 2). The organic phases were combined, washed with saturated brine, and dried over anhydrous sodium sulfate. Anhydrous sodium sulfate was removed by filtration, and the filtrate was concentrated. The crude product was purified by column chromatography on silica gel (ethyl acetate : petroleum ether=30:70) to obtain (S)-3-(trifluoromethyl)-7, 7a, 8, 9, 10, 11-hexahydro-5H-pyrazino[2, 1-c]pyrido[2, 3-e][1, 4]oxazepane (132 mg, 0.45mmol, yield74%), as a yellow oil. LCMS (ESI) m/z: 326 $[M+H]^+$

Step V: Synthesis of 5-(((S)-1-(3-oxo-3-((S)-3-(trifluoromethyl)-7a, 8, 10, 11-tetrahydro-5H-pyrazino[2, 1-c]pyrido[2, 3-e][1, 4]oxazepanyl -9(7H)-yl)propoxy)prop-2-yl)amino)-4-(trifluoromethyl)pyridazin-3(2H)-one

(S)-3-(trifluoromethyl)-7, 7a, 8, 9, 10, 11-hexahydro-5H-pyrazino[2, 1-c]pyrido[2, 3-e][1, 4]oxazepane (56 mg, 0.19 mmol) was dissolved in dichloromethane (2.00 ml), and then N,N-diisopropylethyl amine (250 mg, 1.94 mmol), (S)-3-(2-((6-oxo-5-(trifluoromethyl)-1, 6-dihydropyridazin-4-yl)amino)propoxy)propionic acid (60 mg, 0.19 mmol) and 1-propylphosphonic anhydride (617 mg, 0.97 mmol, 50% solution in ethyl acetate) were added. The mixture was reacted for 0.5 hr at room temperature. After the reaction was completed, the system was cooled to room temperature. The reaction solution was poured into water (25 ml), and extracted with dichloromethane (10 ml*2). The organic phases were combined, washed with saturated brine, and dried over anhydrous sodium sulfate. Anhydrous sodium sulfate was removed by filtration, and the filtrate was concentrated. The residue was purified by high performance liquid chromatography (chromatographic column: Xselect CSH OBD Column 30*150mm 5um; mobile phase A: 0.05% formic acid aqueous solution, mobile phase B: acetonitrile; flow rate: 60 ml/ min; gradient: 25% B to 55% B in 8 min; detection wavelength: 220 nm; retention time: 7.38 min) to obtian 5-(((S)-1-(3-oxo-3-((S)-3-(trifluoromethyl)-7a, 8, 10, 11-tetrahydro-5H-pyrazino[2, 1-c]pyrido[2, 3-e][1, 4]oxazepanyl -9(7H)-yl)propoxy)prop-2-yl)amino)-4-(trifluoromethyl)pyridazin-3(2H)-one as a white solid (28.45 mg, 0.045 mmol, yield 24%).
LCMS (ESI)m/z: 581.10$[M+H]^+$
$^1$HNMR(400 MHz, DMSO-$d_6$)$\delta$ (ppm): 12.45 (s, 1H), 8.38-8.33 (m, 1H), 7.91 (s, 1H), 7.77-7.71 (m, 1H), 6.30-6.22 (m, 1H), 4.36-4.18 (m, 2H), 4.18-4.09 (m, 1H), 3.94-3.85 (m, 1H), 3.84-3.76 (m, 1H), 3.74-3.52(m,7H), 3.51-3.47(m, 2H), 3.02-2.89 (m, 1H), 2.89-2.77(m,1H), 2.60-2.54(m,2H), 1.20-1.10 (m, 3H).

**Example 77:** (S)-9-(3-((S)-2-((6-oxo-5-(trifluoromethyl)-1,6-dihydropyridazin-4-yl)amino)propoxy)propionyl)-3-(trifluoromethyl)-7,7a,8,9,10,11-hexahydropyrazino[1,2-d]pyrido[3,2-b][1,4]diazepin-6(5H)-one **(Compound 83)** and (R)-9-(3-((S)-2-((6-oxo-5-(trifluoromethyl)-1,6-dihydropyridazin-4-yl)amino)propoxy)propionyl)-3-(trifluoromethyl)-7,7a,8,9,10,11-hexahydropyrazino[1,2-d]pyrido[3,2-b][1,4]diazepin-6(5H)-one **(Compound 84)**

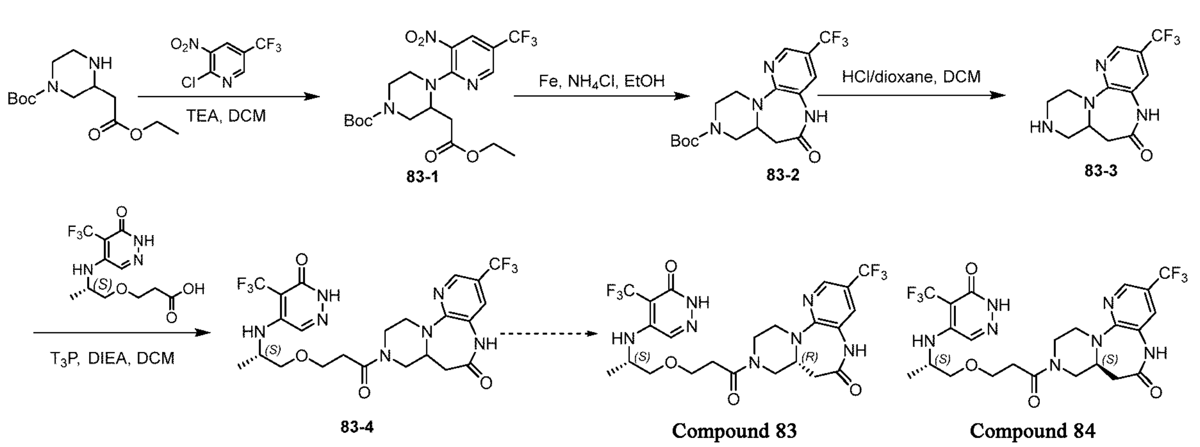

83-1 83-2 83-3 83-4 Compound 83 Compound 84

Step I: t-butyl 3-(2-ethoxy-2-oxyethyl)-4-(3-nitro-5-(trifluoromethyl)pyridin-2-yl)piperazin-1-carboxylate

To a solution of 2-chloro-3-nitro-5-(trifluoromethyl)pyridine (3.50 g, 15.45 mmol) and triethyl amine (6.5 mL, 46.35 mmol) in dichloromethane (52 mL), t-butyl 3-(2-ethoxy-2-oxyethyl)piperazin-1-carboxylate (4.63 g, 16.99 mmol) was added. The reaction system was stirred for 12 hrs under nitrogen atmosphere at 25 °C. The reaction solution was added with water (30 mL), and extracted with dichloromethane (30 mL * 3). The organic phase was dried over anhydrous sodium sulfate, filtered, and rotary dried. The crude product was purified by flash column chromatography (silica gel, ethyl acetate/petroleum ether over 0-50% gradient) to obtain the compound t-butyl 3-(2-ethoxy-2-oxyethyl)-4-(3-nitro-5-(trifluoromethyl)pyridin-2-yl)piperazin-1-carboxylate as a white solid (7.00 g, 15.10 mmol, yield 98%). LCMS (ESI) m/z: 485.2 $[M+Na]^+$

Step II: t-butyl 6-oxo-3-(trifluoromethyl)-6,7,7a,8,10,11-hexahydropyrazino[1,2-d]pyrido[3,2-b][1,4]diazepin-9(5H)-carboxylate

To a solution of t-butyl 3-(2-ethoxy-2-oxyethyl)-4-(3-nitro-5-(trifluoromethyl)pyridin-2-yl)piperazin-1-carboxylate (7.00 g, 15.10 mmol) and acetic acid (17.3 mL, 302.74 mmol) in ethanol (140 mL), reduced iron powder (8.45 g, 151.38 mmol) was added. The reaction system was stirred for 12 hrs at 80 °C under nitrogen atmosphere. The reaction solution was filtered. The filtrate was concentrated to half of the volume, added with water (200 mL), and extracted with dichloromethane (200 mL * 3). The organic phases were combined, washed sequentially with saturated sodium bicarbonate (200 mL) and saturated brine (200 mL), dried over anhydrous sodium sulfate, filtered, and rotary dried, to obtain crude compound t-butyl 6-oxo-3-(trifluoromethyl)-6,7,7a,8,10,11-hexahydropyrazino[1,2-d]pyrido[3,2-b][1,4]diazepin-9(5H)-carboxylate as a white solid (5.90 g). LCMS (ESI) m/z: 409.1 $[M+Na]^+$

Step III: 3-(trifluoromethyl)-7,7a,8,9,10,11-hexahydropyrazino[1,2-d]pyrido[3,2-b][1,4]diazepin-6(5H)-one

At 0 °C, to a solution of t-butyl 6-oxo-3-(trifluoromethyl)-6,7,7a,8,10,11-hexahydropyrazino[1,2-d]pyrido[3,2-b][1,4]diazepin-9(5H)-carboxylate (500 mg, 1.29 mmol) in dichloromethane (3 mL), hydrogen chloride (4 M solution in dioxane, 3.24 mL, 12.96 mmol) was added, and the reaction mixture was stirred for 12 hrs at 25 °C. The reaction solution was rotary dried, to obtain crude compound 3-(trifluoromethyl)-7,7a,8,9,10,11-hexahydropyrazino[1,2-d]pyrido[3,2-b][1,4]diazepin-6(5H)-one as a white solid (500 mg). LCMS (ESI) m/z: 287.1 $[M+H]^+$

Step IV: (S)-9-(3-((S)-2-((6-oxo-5-(trifluoromethyl)-1,6-dihydropyridazin-4-yl)amino)propoxy)propionyl)-3-(trifluoromethyl)-7,7a,8,9,10,11-hexahydropyrazino[1,2-d]pyrido[3,2-b][1,4]diazepin-6(5H)-one and (R)-9-(3-((S)-2-((6-oxo-5-(trifluoromethyl)-1,6-dihydropyridazin-4-yl)amino)propoxy)propionyl)-3-(trifluoromethyl)-7,7a, 8,9,10,11-hexahydropyrazino[1,2-d]pyrido[3,2-b][1,4]diazepin-6(5H)-one

To a solution of (S)-3-(2-((6-oxo-5-(trifluoromethyl)-1,6-dihydropyridazin-4-yl)amino)propoxy)propionic acid (200 mg, 0.65 mmol), diisopropylethyl amine (642 uL, 3.88 mmol) and 3-(trifluoromethyl)-7,7a,8,9,10,11-hexahydropyrazino[1,2-d]pyrido[3,2-b][1,4]diazepin-6(5H)-one (313 mg, 1.09 mmol) in dichloromethane (2 mL), 1-propylphosphonic anhydride (50% solution in ethyl acetate, 2.06 g, 3.23 mmol) were added, and the reaction system was stirred for 2 hrs at 25 °C. The reaction solution was diluted in water (10 mL), and extracted with dichloromethane (10 mL * 3). The organic phases were combined, dried over anhydrous sodium sulfate, filtered and rotary dried. The crude product was purified by preparative HPLC, to obtain the compound 9-(3-((S)-2-((6-oxo-5-(trifluoromethyl)-1,6-dihydropyridazin-4-yl)amino)propoxy)propionyl)-3-(trifluoromethyl)-7,7a, 8,9,10,11-hexahydropyrazino [1,2-d]pyrido [3,2-b] [1,4] diazepin-6(5H)-one (90 mg).

The compound was separated by SFC (column: DAICEL CHIRALPAK AD (250 mm*30 mm, 10 um); mobile phase: phase A: carbon dioxide, and phase B: 0.1% aqueous ammonia/ethanol, with B being maintained at 40%; flow rate: 80 ml/ min), to obtain the target compounds PH-APRN-D-02-98-100 and PH-APRN-D-02-98-200.

First peak **(Compound 83):** A chiral compound (16.42 mg, white solid) with a short peak time after the racemic compound was resolved. SFC analysis (column: Chiralpak AD-3 50*4.6mm I.D., 3um;mobile phase: phase A: carbon dioxide, phase B: 0.05% diethyl amine/ethanol; gradient: phase B from 5% to 40% over 5 min, 40% phase B over 1.2 min, and then 5% phase B over 0.8 min, flow rate: 4 ml/min): RT = 1.862 min, ee = 99.86%;
LCMS (ESI) m/z: 578.3 $[M+H]^+$
$^1$HNMR (400 MHz, MeOH-$d_4$) δ (ppm): 8.41 (s, 1H), 7.97 (d, J = 5.5 Hz, 1H), 7.54 (d, J = 2.0 Hz, 1H), 4.60-4.48 (m, 1H), 4.24-4.11 (m, 1H), 4.09-3.92 (m, 2H), 3.88-3.67 (m, 3H), 3.67-3.60 (m, 1H), 3.53 (dd, J = 6.9, 9.7 Hz, 1H), 3.43-3.02 (m, 2H), 2.99-2.61 (m, 4H), 2.53-2.41 (m, 1H), 1.28 (d, J = 6.5 Hz, 3H).
Second peak **(Compound 84):** A chiral compound (17.72 mg, white solid) with a long peak time after the racemic compound was resolved. SFC analysis (column: Chiralpak AD-3 50*4.6mm I.D., 3um;mobile phase: phase A: carbon dioxide, phase B: 0.05% diethyl amine/ethanol; gradient: phase B from 5% to 40% over 5 min, 40% phase B over 1.2 min, and then 5% phase B over 0.8 min, flow rate: 4 ml/min): RT = 2.067 min, ee = 99.20%;
LCMS (ESI) m/z: 578.3 $[M+H]^+$
$^1$HNMR (400 MHz, MeOH-$d_4$) δ (ppm): 8.41 (d, J = 4.5 Hz, 1H), 7.97 (s, 1H), 7.54 (s, 1H), 4.61-4.50 (m, 1H), 4.18 (d, J = 4.3 Hz, 1H), 4.09-3.92 (m, 2H), 3.87-3.59 (m, 4H), 3.52 (ddd, J = 3.5, 6.7, 9.9 Hz, 1H), 3.43-3.36 (m, 1H), 3.28-3.03 (m, 1H), 2.98-2.62 (m, 4H), 2.53-2.41 (m, 1H), 1.28 (d, J = 6.5 Hz, 3H).

**Example 78:** 5-methyl-9-(3-((S)-2-((6-oxo-5-(trifluoromethyl)-1,6-dihydropyridazin-4-yl)amino)propoxy)propionyl)-3-(trifluoromethyl)-7, 7a, 8,9,10,11-hexahydropyrazino[1,2-d]pyrido[3,2-b][1,4]diazepin-6(5H)-one **(Compound 85)**

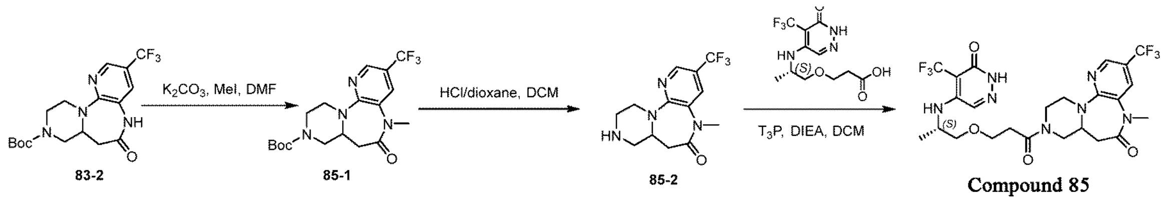


83-2 85-1 85-2 Compound 85

Step I: t-butyl5-methyl-6-oxo-3-(trifluoromethyl)-6,7,7a,8,10,11-hexahydropyrazino[1,2-d]pyrido[3,2-b] [1,4]diazepin-9(SH)-carboxylate**(85-1)**

At 0 °C, to a solution of t-butyl 6-oxo-3-(trifluoromethyl)-6,7,7a,8,10,11-hexahydropyrazino[1,2-d]pyrido[3,2-b][1,4]diazepin-9(5H)-carboxylate (600 mg, 1.55 mmol) in dimethylformamide (12 mL), potassium carbonate (429 mg, 3.11 mmol) was added. Then iodomethane (441 mg, 3.11 mmol) was added dropwise. The reaction system was stirred for 2 hrs under nitrogen atmosphere at 25 °C. The reaction mixture was filtered, and the filtrate was diluted in water (36 mL) and then extracted with ethyl acetate (40 mL * 3). The organic phases were combined, washed with brine (30 mL), dried over anhydrous sodium sulfate, filtered, and rotary dried, to obtain the crude compound t-butyl 5-methyl-6-oxo-3-(trifluoromethyl)-6,7,7a,8,10,11-hexahydropyrazino[1,2-d]pyrido[3,2-b][1,4]diazepin-9(5H)-carboxylate as a white solid (610 mg).

LCMS (ESI) m/z: 345.1 $[M+H]^+$

Step II and Step III: 5-methyl-9-(3-((S)-2-((6-oxo-5-(trifluoromethyl)-1,6-dihydropyridazin-4-yl)amino)propoxy)propionyl)-3-(trifluoromethyl)-7,7a,8,9,10,11-hexahydropyrazino[1,2-d]pyrido[3,2-b][1,4]diazepin-6(5H)-one

T-butyl 5-methyl-6-oxo-3-(trifluoromethyl)-6,7,7a,8,10,11-hexahydropyrazino[1,2-d]pyrido[3,2-b][1,4]diazepin-9(5H)-carboxylate **(85-1)** was used as the raw material, and the synthesis method was the same as that in Step III and Step IV in **Example 77,** to obtain 5-methyl-9-(3-((S)-2-((6-oxo-5-(trifluoromethyl)-1,6-dihydropyridazin-4-yl)amino)propoxy)propionyl)-3-(trifluoromethyl)-7,7a,8,9,10,11-hexahydropyrazino[1,2-d]pyrido [3,2-b][1,4] diazepin-6(5H)-one.

LCMS (ESI) m/z: 592.3$[M+H]^+$

1H NMR (400 MHz, CD3OD) δ (ppm): 8.47 (s, 1H), 8.07-7.84 (m, 2H), 4.60-4.49 (m, 1H), 4.17 (s, 1H), 4.10-3.96 (m, 1H), 3.92-3.74 (m, 3H), 3.72-3.47 (m, 3H), 3.34 (s, 3H), 3.25-2.89 (m, 2H), 2.87-2.58 (m, 4H), 2.52-2.37 (m, 1H), 1.28 (d, J = 6.4 Hz, 3H).

**Test of inhibitory activity against PARP7 enzyme**

Enzymatic reaction

Enzymatic reaction buffer: 20 mM HEPES PH = 8, 100 mM NaCl, 0.1% BSA, 2 mM DTT and 0.002% Tween 20.

Enzyme reagent: PARP7 FLAG-tag (BPS 80527) diluted into the enzyme reaction butter at 30 nM.

Probe A: diluted into the enzyme reaction butter at 5 nM.

Compounds: 4-fold diluted into the enzyme reaction butter by using DMSO starting from 10 mM, to give 8 concentrations.

The enzyme reagent, Probe A and the compound to be tested were added into a PerkinElmer Opti-Plate at a ratio of 4: 4: 2, and the total reaction volume was 10 ul. The mixture was uniformly mixed and reacted at room temperature for 1 hr. The final concentration of PARP7 was 12 nM and the final concentration of Probe A was 2 nm.

2. Time-resolved fluorescence spectroscopy

LANCE Eu-W1024 streptavidin, diluted into 1×Lance buffer at 8 nM.

ULight-anti FLAG, diluted into 1×Lance buffer at 0.5 nM.

Each 5 ul of LANCE Eu-W1024 streptavidin and ULight-anti FLAG was added to a reaction plate, and reacted for 1 hr at room temperature. The final concentration of LANCE Eu-W1024 streptavidin and ULight-anti FLAG were respectively 4 nM and 0.25 nM.

3. The signal was read on Envision Instrument, where the excitation wavelength was 320 nm, and the emission wavelength was 615 nm and 665nm.

4. Data analysis

IC50 was calculated by GraphPad Prism 8 software, and IC50 (half maximal inhibitory concentration) of the compound was obtained by using a nonlinear fitting formula below:

$$Y=Bottom + (Top-Bottom)/(1+10^((LogIC50-X)*HillSlope))$$

X: log concentration of compound, Y: inhibition rate (% inhibition)

**Cell proliferation inhibition assay**

Cell plating

NCI-H1373 cells were cultured to a cell confluence of 80%-90%, and the cells were collected.
The medium in the flask was removed and the cells were washed twice with PBS.
The cells were digested with trypsin, and then terminated with the medium. Then, the suspension was transferred to a 15 ml centrifuge tube and centrifuged at 1000 rpm for 4 min.
The cells were re-suspended in corresponding medium, counted, and formulated to a cell suspension with an appropriate density.
100 μL of the cell suspension was inoculated into a 96-well plate at 2000/well.
The cells were incubated overnight in an incubator at 37°C in 5% CO2.

2. Preparation of compound

Starting from 10 mM, the compound was 4-fold diluted with DMSO to give 10 concentrations.

3. Treatment of cells with compound

24 hrs of cell plating, 100 nL of diluted compound was added to the 96-well cell culture plate.
The cell plate was incubated in an incubator for 6 days.

4. Detection by CTG method

100 ul supernatant was pipetted from each well, and then 100 μL CTG reagent (CelltiterGlo kit) was added to each well. The plate was shaken for 2 min on a fast shaker at 300 rpm and left to stand for 30 min in the dark at room temperature.
The chemiluminescence signal was read on Envision Instrument.

5. Data analysis

$IC_{50}$ was calculated by GraphPad Prism 8 software, and $IC_{50}$ (half maximal inhibitory concentration) of the compound was obtained by using a nonlinear fitting formula below:

$$Y=Bottom + (Top-Bottom)/(1+10^((LogIC_{50}-X)*HillSlope))$$

X: log concentration of compound, Y: inhibition rate (% inhibition)

Table 1

| Compound No. | $IC_{50}$ (nM) for NCI-H1373 cell viability | Inhibitory activity against PARP7 enzyme $IC_{50}$ (nM) |
|---|---|---|
| Compound 1 | A | A |

(continued)

| Compound No. | $IC_{50}$ (nM) for NCI-H1373 cell viability | Inhibitory activity against PARP7 enzyme $IC_{50}$ (nM) |
|---|---|---|
| Compound 2 | A | A |
| Compound 3 | D | A |
| Compound 4 | D | A |
| Compound 5 | A | A |
| Compound 6 | C | A |
| Compound 7 | C | A |
| Compound 8 | B | A |
| Compound 9 | C | A |
| Compound 10 | B | A |
| Compound 11 | C | A |
| Compound 12 | A | A |
| Compound 13 | B | A |
| Compound 14 | B | A |
| Compound 15 | C | A |
| Compound 16 | D | B |
| Compound 17 | D | B |
| Compound 18 | D | B |
| Compound 19 | D | C |
| Compound 20 | D | D |
| Compound 22 | C | A |
| Compound 23 | B | A |
| Compound 24 | B | A |
| Compound 25 | B | A |
| Compound 27 | C | A |
| Compound 29 | A | A |
| Compound 30 | B | A |
| Compound 31 | C | A |
| Compound 32 | A | A |
| Compound 33 | A | A |
| Compound 34 | C | A |
| Compound 35 | A | A |
| Compound 36 | C | A |
| Compound 37 | B | A |
| Compound 38 | B | A |
| Compound 39 | A | A |
| Compound 40 | A | A |
| Compound 41 | C | A |
| Compound 42 | C | A |

(continued)

| Compound No. | $IC_{50}$ (nM) for NCI-H1373 cell viability | Inhibitory activity against PARP7 enzyme $IC_{50}$ (nM) |
|---|---|---|
| Compound 43 | B | A |
| Compound 45 | A | A |
| Compound 46 | B | A |
| Compound 47 | A | A |
| Compound 48 | A | A |
| Compound 49 | D | A |
| Compound 50 | B | A |
| Compound 51 | B | A |
| Compound 52 | A | A |
| Compound 53 | B | A |
| Compound 54 | A | A |
| Compound 55 | A | A |
| Compound 56 | B | A |
| Compound 57 | C | A |
| Compound 58 | A | A |
| Compound 60 | A | A |
| Compound 61 | A | A |
| Compound 62 | B | A |
| Compound 63 | C | A |
| Compound 64 | C | A |
| Compound 65 | A | A |
| Compound 66 | C | A |
| Compound 67 | A | A |
| Compound 68 | A | A |
| Compound 69 | A | A |
| Compound 70 | NT | A |
| Compound 71 | A | A |
| Compound 72 | A | A |
| Compound 73 | A | A |
| Compound 74 | A | A |
| Compound 76 | NT | A |
| Compound 77 | D | A |
| Compound 79 | C | A |
| Compound 82 | C | A |
| Compound 84 | D | A |
| NCI-H1373 cell viability: A: $IC_{50}$ < 100 nM; B: 100<$IC_{50}$<500 nM; C: 500<$IC_{50}$<5000 nM; D: $IC_{50}$ >5000 nM; NT: not tested<br>Inhibitory activity against PARP7 enzyme: A: $IC_{50}$ < 10 nM; B: 10<$IC_{50}$<100 nM; C: 100<$IC_{50}$<500 nM; D: $IC_{50}$>500 nM. | | |

As can be seen from Table 1 above, the compound of the present invention has better inhibitory activity against PARP7 and can significantly inhibit the growth of NCI-H1373 human lung adenocarcinoma cells. The $IC_{50}$ for anti-proliferation activity of most compounds is <500 nM; the $IC_{50}$ of some compounds is < 100 nM; and the $IC_{50}$ of some compounds is <50 nM.

Although specific embodiments of the present invention has been described above, it should be understood by those skilled in the art that they are only exemplary, and various changes or modifications can be made to these embodiments without departing from the principle and essence of the present invention. Therefore, the scope of protection of the present invention is defined by the appended claims.

## Claims

**1.** A fused triheterocyclic compound of Formula IA or a pharmaceutically acceptable salt thereof,

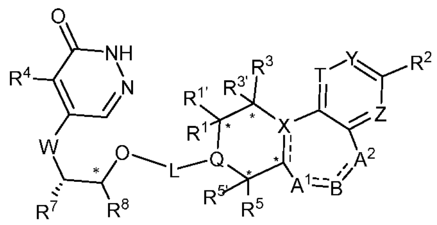

**IA**

wherein W is NH or O;
L is

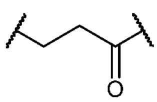 , 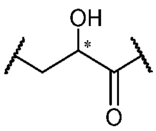 , 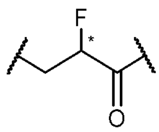 , or 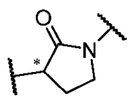 ;

$R^1$, $R^{1'}$, $R^3$, $R^{3'}$, $R^5$, and $R^{5'}$ are independently H, $C_{1-6}$ alkyl, or $C_{1-6}$ alkyl substituted with one or more $R^{1a}$; or $R^1$ and $R^{1'}$, $R^3$ and $R^{3'}$, and $R^5$ and $R^{5'}$ are independently taken together to form O=, where when more than one substituent is present, they are the same or different, and
$R^{1a}$ is independently halo, CN, or OH;
$R^2$ and $R^4$ are independently halo, CN, $SF_5$, $C_{1-6}$ alkyl, $C_{1-6}$ alkyl substituted with one or more $R^{2a}$, or $C_{1-6}$ alkyl-S- substituted with one or more $R^{2b}$, where when more than one substituent is present, they are the same or different, and
$R^{2a}$ and $R^{2b}$ are independently halo;
X is independently $C(R^{3a})$, C or N;
Q is independently $C(R^{3a})$ or N, where
when Q is $C(R^{3a})$, the carbon in $C(R^{3a})$ is a chiral carbon atom, and is in R configuration, S configuration or a combination thereof;
T is independently $C(R^{3b})$ or N; Y is independently $C(R^{3c})$ or N; and Z is independently $C(R^{3d})$ or N;
in which $R^{3a}$, $R^{3b}$, $R^{3c}$, and $R^{3d}$ are independently H, halo, CN, OH, $NH_2$, $C_{1-6}$ alkyl, $C_{1-6}$ alkyl substituted with one or more $R^{a4}$, or $C_{1-6}$ alkoxy;
$A^1$ is independently $C(R^{4a})$, $C(R^{4b}R^{4c})$ or C(=O), where
$R^{4a}$, $R^{4b}$, and $R^{4c}$ are independently H, $C_{1-6}$ alkyl, or $C_{1-6}$ alkyl substituted with one or more $R^{a1}$, where when more than one substituent is present, they are the same or different;
when B is independently $C(R^{6a})$, or $C(R^{6b}R^{6c})$, $A^2$ is independently $C(R^{5a})$, $C(R^{5b}R^{5c})$, O, S, $N(R^{5d})$, N or a linkage;
when B is independently O, S, S(=O), $S(=O)_2$, N, or $N(R^{6d})$, $A^2$ is independently $C(R^{5a})$, $C(R^{5b}R^{5c})$, or a linkage;
in which $R^{5a}$, $R^{5b}$, $R^{5c}$, and $R^{5d}$ are independently H, $C_{1-6}$ alkyl, or $C_{1-6}$ alkyl substituted with one or more $R^{a2}$, or $R^{5b}$ and $R^{5c}$ are taken together to form =O;
$R^{6a}$, $R^{6b}$, and $R^{6c}$ are independently H, halo, $C_{1-6}$ alkyl, $C_{1-6}$ alkyl substituted with one or more $R^{a3}$, $C_{3-6}$ cycloalkyl, or OH, or $R^{6b}$ and $R^{6c}$ are taken together to form =O;

$R^{6d}$ is independently H, $C_{1-6}$ alkyl, $C_{1-6}$ alkyl substituted with one or more $R^{a3}$, $C_{3-6}$ cycloalkyl or -CN, where when more than one substituent is present, they are the same or different; and
$R^{a1}$, $R^{a2}$, $R^{a3}$, and $R^{a4}$ are independently deuterium, halo or OH;
$R^7$ and $R^8$ are independently
H, C1-6 alkyl or $C_{1-6}$ alkyl substituted with $OR^{7a}$, where
$R^{7a}$ is selected from H, $C_{1-6}$ alkyl and $C_{1-6}$ haloalkyl; and
- - - - represents a double bond or a single bond;* indicates a chiral carbon atom that is in R configuration, S configuration or a combination thereof.

**2.** The fused triheterocyclic compound of Formula IA or the pharmaceutically acceptable salt thereof according to claim 1, wherein the fused triheterocyclic compound of Formula IA is represented by Formula I':

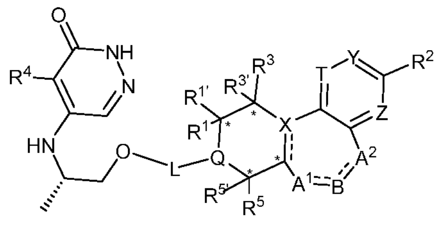

I'

wherein L is

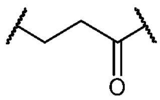 , 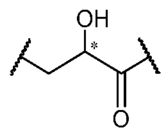 , or 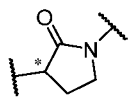 ;

$R^1$, $R^{1'}$, $R^3$, $R^{3'}$, $R^5$, and $R^{5'}$ are independently H, $C_{1-6}$ alkyl, or $C_{1-6}$ alkyl substituted with one or more $R^{1a}$; or $R^1$ and $R^{1'}$, $R^3$ and $R^{3'}$, and $R^5$ and $R^{5'}$ are independently taken together to form O=, where when more than one substituent is present, they are the same or different, and
$R^{1a}$ is independently halo, CN, or OH;
$R^2$ and $R^4$ are independently halo, CN, $C_{1-6}$ alkyl, $C_{1-6}$ alkyl substituted with one or more $R^{2a}$, or $C_{1-6}$ alkyl-S- substituted with one or more $R^{2b}$, where when more than one substituent is present, they are the same or different, and
$R^{2a}$ and $R^{2b}$ are independently halo;
X is independently $C(R^{3a})$, C or N;
Q is independently $C(R^{3a})$ or N, where
when Q is $C(R^{3a})$, the carbon in $C(R^{3a})$ is a chiral carbon atom, and is in R configuration, S configuration or a mixture thereof;
T is independently $C(R^{3b})$ or N; Y is independently $C(R^{3c})$ or N; and Z is independently $C(R^{3d})$ or N;
in which $R^{3a}$, $R^{3b}$, $R^{3c}$, and $R^{3d}$ are independently H, halo, CN, $NH_2$, $C_{1-6}$ alkyl, $C_{1-6}$ alkyl substituted with one or more $R^{a4}$, or $C_{1-6}$ alkoxy;
$A^1$ is independently $C(R^{4a})$, $C(R^{4b}R^{4c})$ or C(=O), where
$R^{4a}$, $R^{4b}$, and $R^{4c}$ are independently H, $C_{1-6}$ alkyl, or $C_{1-6}$ alkyl substituted with one or more $R^{a1}$, where when more than one substituent is present, they are the same or different;
when B is independently $C(R^{6a})$, or $C(R^{6b}R^{6c})$, $A^2$ is independently $C(R^{5a})$, $C(R^{5b}R^{5c})$, O, S, $N(R^{5d})$, N or a linkage;
when B is independently O, S, S(=O), $S(=O)_2$, N, or $N(R^{6d})$, $A^2$ is independently $C(R^{5a})$, $C(R^{5b}R^{5c})$, or a linkage;
in which $R^{5a}$, $R^{5b}$, $R^{5c}$, and $R^{5d}$ are independently H, $C_{1-6}$ alkyl, or $C_{1-6}$ alkyl substituted with one or more $R^{a2}$, or $R^{5b}$ and $R^{5c}$ are taken together to form =O;
$R^{6a}$, $R^{6b}$, and $R^{6c}$ are independently H, halo, $C_{1-6}$ alkyl, $C_{1-6}$ alkyl substituted with one or more $R^{a3}$, $C_{3-6}$ cycloalkyl, or OH, or $R^{6b}$ and $R^{6c}$ are taken together to form =O;
$R^{6d}$ is independently H, $C_{1-6}$ alkyl, $C_{1-6}$ alkyl substituted with one or more $R^{a3}$, $C_{3-6}$ cycloalkyl or -CN, where when more than one substituent is present, they are the same or different; and
$R^{a1}$, $R^{a2}$, $R^{a3}$, and $R^{a4}$ are independently deuterium, halo or OH; and

- - - - represents a double bond or a single bond;* indicates a chiral carbon atom that is in R configuration, S configuration or a combination thereof.

**3.** The fused triheterocyclic compound of Formula IA or the pharmaceutically acceptable salt thereof according to claim 1 or 2, wherein the fused triheterocyclic compound of Formula IA is a fused triheterocyclic compound of Formula I:

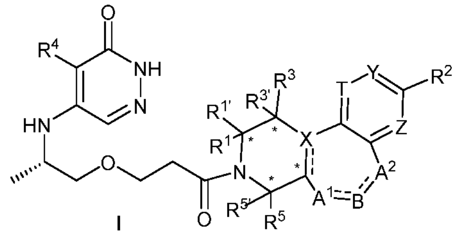

I

wherein $R^1$, $R^{1'}$, $R^3$, $R^{3'}$, $R^5$, and $R^{5'}$ are independently H, $C_{1-6}$ alkyl or $C_{1-6}$ alkyl substituted with one or more $R^{1a}$; or $R^1$ and $R^{1'}$, $R^3$ and $R^{3'}$, and $R^5$ and $R^{5'}$ are independently taken together to form O=, where when more than one substituent is present, they are the same or different, and
$R^{1a}$ is independently halo, CN, or OH;
$R^2$ and $R^4$ are independently halo, CN, $C_{1-6}$ alkyl, $C_{1-6}$ alkyl substituted with one or more $R^{2a}$, or $C_{1-6}$ alkyl-S- substituted with one or more $R^{2b}$, where when more than one substituent is present, they are the same or different, and
$R^{2a}$ and $R^{2b}$ are independently halo;
X is independently $C(R^{3a})$, C or N;
T is independently $C(R^{3b})$ or N; Y is independently $C(R^{3c})$ or N; and Z is independently $C(R^{3d})$ or N;
in which $R^{3a}$, $R^{3b}$, $R^{3c}$, and $R^{3d}$ are independently H, halo, CN, $NH_2$ or $C_{1-6}$ alkyl;
$A^1$ is independently $C(R^{4a})$, $C(R^{4b}R^{4c})$ or C(=O), where
$R^{4a}$, $R^{4b}$, and $R^{4c}$ are independently H, $C_{1-6}$ alkyl, or $C_{1-6}$ alkyl substituted with one or more $R^{a1}$, where when more than one substituent is present, they are the same or different;
when B is independently $C(R^{6a})$, or $C(R^{6b}R^{6c})$, $A^2$ is independently $C(R^{5a})$, $C(R^{5b}R^{5c})$, O, S, $N(R^{5d})$, N or a linkage;
when B is independently O, S, N or $N(R^{6d})$, $A^2$ is independently $C(R^{5a})$, $C(R^{5b}R^{5c})$ or a linkage;
in which $R^{5a}$, $R^{5b}$, $R^{5c}$, and $R^{5d}$ are independently H, $C_{1-6}$ alkyl or $C_{1-6}$ alkyl substituted with one or more $R^{a2}$;
$R^{6a}$, $R^{6b}$, and $R^{6c}$ are independently H, halo, $C_{1-6}$ alkyl, $C_{1-6}$ alkyl substituted with one or more $R^{a3}$, or $C_{3-6}$ cycloalkyl;
$R^{6d}$ is independently H, $C_{1-6}$ alkyl, $C_{1-6}$ alkyl substituted with one or more $R^{a3}$, or $C_{3-6}$ cycloalkyl, where when more than one substituent is present, they are the same or different; and
$R^{a1}$, $R^{a2}$, and $R^{a3}$ are independently deuterium, or halo; and
- - - - represents a double bond or a single bond; * indicates a chiral carbon atom that is in R configuration, S configuration or a combination thereof.

**4.** The fused triheterocyclic compound of Formula IA or the pharmaceutically acceptable salt thereof according to claim 1 or 2, wherein

one of $R^1$ and $R^{1'}$ is H, and the other one is H, $C_{1-6}$ alkyl, or $C_{1-6}$ alkyl substituted with one or more $R^{1a}$; or $R^1$ and $R^{1'}$ are taken together to form O=, for example, one of $R^1$ and $R^{1'}$ is H, and the other one is H or $C_{1-6}$ alkyl;
and/or, one of $R^3$ and $R^{3'}$ is H, and the other one is H, $C_{1-6}$ alkyl, or $C_{1-6}$ alkyl substituted with one or more $R^{1a}$; or $R^3$ and $R^{3'}$ are taken together to form O=, for example, one of $R^3$ and $R^{3'}$ is H, and the other one is H or $C_{1-6}$ alkyl;
and/or, one of $R^5$ and $R^{5'}$ is H, and the other one is H, $C_{1-6}$ alkyl, or $C_{1-6}$ alkyl substituted with one or more $R^{1a}$; or $R^5$ and $R^{5'}$ are taken together to form O=, for example, one of $R^5$ and $R^{5'}$ is H, and the other one is H or $C_{1-6}$ alkyl;
and/or, when the carbon to which $R^1$ and $R^{1'}$ are attached is a chiral carbon, it is in S configuration or R configuration, for example, R configuration;
and/or, when the carbon to which $R^3$ and $R^{3'}$ are attached is a chiral carbon, it is in S configuration or R configuration;
and/or, when the carbon to which $R^5$ and $R^{5'}$ are attached is a chiral carbon, it is in S configuration or R configuration;
and/or, $R^2$ is independently halo, CN, $C_{1-6}$ alkyl substituted with one or more $R^{2a}$, or $C_{1-6}$ alkyl-S- substituted with one or more $R^{2b}$, for example, halo, or $C_{1-6}$ alkyl substituted with one or more $R^{2a}$;

and/or, $R^4$ is independently halo, CN, or $C_{1-6}$ alkyl substituted with one or more $R^{2a}$, for example, halo, or $C_{1-6}$ alkyl substituted with one or more $R^{2a}$;
and/or, X is independently N;
and/or, T is independently N;
and/or, $R^{3c}$ is H, CN, $NH_2$ or $C_{1-6}$ alkyl, for example, H;
and/or, Y is independently $C(R^{3c})$ or N;
and/or, $R^{3d}$ is H, halo, CN or $C_{1-6}$ alkyl, for example, H;
and/or, Z is independently $C(R^{3d})$ or N;
and/or, $R^{4a}$, $R^{4b}$, and $R^{4c}$ are independently H or $C_{1-6}$ alkyl, for example, one of $R^{4b}$ and $R^{4c}$ is H, and the other one is H or $C_{1-6}$ alkyl;
and/or, when $A^1$ is $C(R^{4b}R^{4c})$ and the carbon is a chiral carbon, it is in S configuration, R configuration or a combination thereof;
and/or, $A^1$ is independently $C(R^{4b}R^{4c})$ or C(=O), for example, $C(R^{4b}R^{4c})$;
and/or, $R^{5a}$, $R^{5b}$, $R^{5c}$, and $R^{5d}$ are independently H or $C_{1-6}$ alkyl, for example, one of $R^{5b}$ and $R^{5c}$ is H, and the other one is H or $C_{1-6}$ alkyl;
and/or, when $A^2$ is $C(R^{5b}R^{5c})$ and the carbon is a chiral carbon, it is in S configuration, R configuration or a combination thereof;
and/or, $A^2$ is independently $C(R^{5b}R^{5c})$ or a linkage;
and/or, $R^{6a}$, $R^{6b}$, and $R^{6c}$ are independently H or halo, for example, $R^{6b}$ and $R^{6c}$ are independently H;
and/or, $R^{6d}$ is independently H, $C_{1-6}$ alkyl, $C_{1-6}$ alkyl substituted with one or more $R^{a3}$, or $C_{3-6}$ cycloalkyl, for example, H or $C_{1-6}$ alkyl;
and/or, B is independently $C(R^{6b}R^{6c})$, O, S or $N(R^{6d})$, for example, -O-, -S- or $N(R^{6d})$;
and/or,

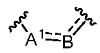

is independently $-C(R^{4b}R^{4c})-O-$, $-C(R^{4b}R^{4c})-S-$, $-C(R^{4b}R^{4c})-N(R^{6d})-$, - $C(R^{4b}R^{4c})-C(R^{6b}R^{6c})-$, -C(=O)-O-, or $-C(=O)-N(R^{6d})-$, for example, $-C(R^{4b}R^{4c})-O-$, $-C(R^{4b}R^{4c})-S-$, $-C(R^{4b}R^{4c})-N(R^{6d})-$, or $-C(=O)-N(R^{6d})-$;
and/or,

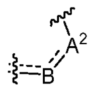

is independently -O-, -S-, $-C(R^{6b}R^{6c})-$, $-N(R^{6d})-$, $-O-C(R^{5b}R^{5c})-$, $-S-C(R^{5b}R^{5c})-$, $-N(R^{6d})-C(R^{5b}R^{5c})-$, $-C(R^{6b}R^{6c})-O-$, $-C(R^{6b}R^{6c})-S-$, or $-C(R^{6b}R^{6c})-N(R^{5d})-$, for example, -O-, $-O-C(R^{5b}R^{5c})-$, $-S-C(R^{5b}R^{5c})-$, $-N(R^{6d})-$, or $-N(R^{6d})-C(R^{5b}R^{5c})-$;
and/or,

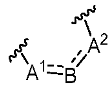

is independently

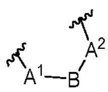 or 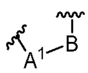 ;

and and/or,

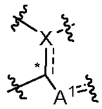

is independently

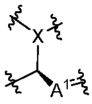 or 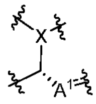.

**5.** The fused triheterocyclic compound of Formula IA or the pharmaceutically acceptable salt thereof according to claim 1 or 2, wherein

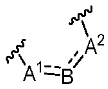

is independently

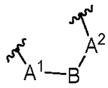, 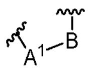 or 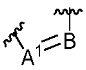;

and/or, when B is independently O, S, or $N(R^{6d})$, $A^2$ is independently $C(R^{5b}R^{5c})$ or a linkage;
and/or, when B is independently $C(R^{6b}R^{6c})$, $A^2$ is independently O, $N(R^{5d})$ or a linkage;
and/or, when B is independently $N(R^{6d})$, $R^{6d}$ is independently $C_{1-6}$ alkyl, or $C_{1-6}$ alkyl substituted with one or more $R^{a3}$, and $A^1$ is independently C(=O);
and/or, when T and Z are both N, $A^2$ is independently O, $N(R^{5d})$ or $C(R^{5b}R^{5c})$;
and/or,

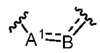

is independently $-C(R^{4b}R^{4c})-O-$, $-C(R^{4b}R^{4c})-S-$, $-C(R^{4b}R^{4c})-N(R^{6d})-$, $-C(R^{4b}R^{4c})-C(R^{6b}R^{6c})-$, -C(=O)-O-, $-C(=O)-N(R^{6d})-$, $-C(R^{4b}R^{4c})-S(=O)-$, $-C(R^{4b}R^{4c})-S(=O)_2$, or $-C(R^{4a})=C(R^{6a})-$;
and/or,

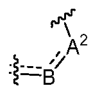

is independently -O-, -S-, $-C(R^{6b}R^{6C})-$, $-N(R^{6d})-$, $-O-C(R^{5b}R^{5c})-$, $-S-C(R^{5b}R^{5c})-$, $-N(R^{6d})-C(R^{5b}R^{5c})-$, $-C(R^{6b}R^{6c})-O-$, $-C(R^{6b}R^{6c})-S-$, $-C(R^{6b}R^{6c})-N(R^{5d})-$, $-C(R^{5b}R^{5c})-$, $-S(=O)_2-C(R^{5b}R^{5c})-$, $-O-C(R^{6b}R^{6c})-$, or $=C(R^{6a})-$;
and/or, when $R^{3a}$, $R^{3b}$, $R^{3c}$, and $R^{3d}$ are independently $C_{1-6}$ alkyl or $C_{1-6}$ alkyl substituted with one or more $R^{a4}$, the $C_{1-6}$ alkyl and $C_{1-6}$ alkyl in the $C_{1-6}$ alkyl substituted with one or more $R^{a4}$ is methyl, ethyl, n-propyl, isopropyl, n-butyl, isobutyl, s-butyl or t-butyl, for example, methyl;
and/or, when $R^{3a}$, $R^{3b}$, $R^{3c}$, and $R^{3d}$ are independently $C_{1-6}$ alkoxy, the $C_{1-6}$ alkoxy is methoxy, ethoxy, n-propoxy, iso-propoxy, n-butoxy, iso-butoxy, s-butoxy or t-butoxy, for example, methoxy;
and/or, when $R^7$ and $R^8$ are independently $C_{1-6}$ alkyl and $C_{1-6}$ haloalkyl, the $C_{1-6}$ alkyl is independently methyl, ethyl, n-propyl or isopropyl, for example, methyl;
and/or, when $R^{7a}$ is selected from $C_{1-6}$ alkyl and $C_{1-6}$ haloalkyl, the $C_{1-6}$ alkyl is independently methyl, ethyl, n-propyl or isopropyl;
and/or, when $R^{7a}$ is selected from $C_{1-6}$ haloalkyl, the halo is independently F, Cl, Br or I, for example, F;
and/or, when the carbon to which $R^8$ is attached is a chiral carbon, it is in S configuration or R configuration, for example, R configuration;
and/or, $R^7$ and $R^8$ are independently H or $C_{1-3}$ alkyl, for example, one of $R^7$ and $R^8$ is H, and the other one is methyl.

**6.** The fused triheterocyclic compound of Formula IA or the pharmaceutically acceptable salt thereof according to claim 1 or 2, wherein

one of $R^1$, $R^{1'}$, $R^3$, $R^{3'}$, $R^5$, and $R^{5'}$ is H, $C_{1-6}$ alkyl or $C_{1-6}$ alkyl substituted with one or more $R^{1a}$, and the others are H, for example, $R^1$, $R^{1'}$, $R^3$, $R^3$, $R^5$, and $R^{5'}$ are H;
and/or, when $R^1$, $R^{1'}$, $R^3$, $R^{3'}$, $R^5$, and $R^{5'}$ are independently $C_{1-6}$ alkyl or $C_{1-6}$ alkyl substituted with one or more $R^{1a}$, the $C_{1-6}$ alkyl or the $C_{1-6}$ alkyl in the $C_{1-6}$ alkyl substituted with one or more $R^{1a}$ is methyl, ethyl, n-propyl, isopropyl, n-butyl, isobutyl, s-butyl or t-butyl, for example, methyl;
and/or, when $R^{1a}$ is halo, the halo is F, Cl, Br, or I, for example, F;
and/or, when $R^2$ and $R^4$ are independently halo, the halo is F, Cl, Br, or I, for example, F;
and/or, when $R^2$ and $R^4$ are independently $C_{1-6}$ alkyl, $C_{1-6}$ alkyl substituted with one or more $R^{2a}$, or $C_{1-6}$ alkyl-S- substituted with one or more $R^{2b}$, the $C_{1-6}$ alkyl and the $C_{1-6}$ alkyl in the $C_{1-6}$ alkyl substituted with one or more $R^{2a}$ and $C_{1-6}$ alkyl-S- substituted with one or more $R^{2b}$ are methyl, ethyl, n-propyl, isopropyl, n-butyl, isobutyl, s-butyl or t-butyl, for example, methyl;
and/or, when $R^2$ and $R^4$ are independently $C_{1-6}$ alkyl substituted with one or more $R^{2a}$ or $C_{1-6}$ alkyl-S- substituted with one or more $R^{2b}$, the number of the substituent is 1, 2, or 3;
and/or, when $R^{2a}$ and $R^{2b}$ are independently halo, the halo is F, Cl, Br, or I, for example, F;
and/or, when $R^{3a}$, $R^{3b}$, $R^{3c}$, and $R^{3d}$ are independently halo, the halo is F, Cl, Br, or I, for example, F;
and/or, when $R^{3a}$, $R^{3b}$, $R^{3c}$, and $R^{3d}$ are independently $C_{1-6}$ alkyl, the $C_{1-6}$ alkyl is methyl, ethyl, n-propyl, isopropyl, n-butyl, isobutyl, s-butyl or t-butyl, for example, methyl;
and/or, when $R^{4a}$, $R^{4b}$, and $R^{4c}$ are independently $C_{1-6}$ alkyl or $C_{1-6}$ alkyl substituted with one or more $R^{a1}$, the $C_{1-6}$ alkyl and the $C_{1-6}$ alkyl in the $C_{1-6}$ alkyl substituted with one or more $R^{a1}$ is methyl, ethyl, n-propyl, isopropyl, n-butyl, isobutyl, s-butyl or t-butyl, for example, methyl;
and/or, when $R^{5a}$, $R^{5b}$, $R^{5c}$, and $R^{5d}$ are independently $C_{1-6}$ alkyl or $C_{1-6}$ alkyl substituted with one or more $R^{a2}$, the $C_{1-6}$ alkyl and the $C_{1-6}$ alkyl in the $C_{1-6}$ alkyl substituted with one or more $R^{a2}$ is methyl, ethyl, n-propyl, isopropyl, n-butyl, isobutyl, s-butyl or t-butyl, for example, methyl;
and/or, when $R^{6a}$, $R^{6b}$, and $R^{6c}$ are independently halo, the halo is F, Cl, Br, or I, for example, F;
and/or, when $R^{6a}$, $R^{6b}$, $R^{6c}$, and $R^{6d}$ are independently $C_{1-6}$ alkyl or $C_{1-6}$ alkyl substituted with one or more $R^{a3}$, the $C_{1-6}$ alkyl and the $C_{1-6}$ alkyl in the $C_{1-6}$ alkyl substituted with one or more $R^{a3}$ is methyl, ethyl, n-propyl, isopropyl, n-butyl, isobutyl, s-butyl or t-butyl, for example, methyl or ethyl;
and/or, when $R^{6a}$, $R^{6b}$, $R^{6c}$, and $R^{6d}$ are independently $C_{3-6}$ cycloalkyl, the $C_{3-6}$ cycloalkyl is cyclopropyl, cyclobutyl, cyclopentyl, or cyclohexyl, for example, cyclopropyl;
and/or, when $R^{a1}$, $R^{a2}$, and $R^{a3}$ are independently halo, the halo is F, Cl, Br, or I, for example, F.

**7.** The fused triheterocyclic compound of Formula IA or the pharmaceutically acceptable salt thereof according to claim 1 or 2, wherein

T is independently N, or CH;
Q is independently N or CH;
and/or, Y is independently CH, N, C(CN), $C(NH_2)$, $C(CHF_2)$, or C(OMe);
and/or, Z is independently CH, N, C(CN), $C(NH_2)$, $C(CHF_2)$, or $C(CH_3)$;
and/or, $A^1$ is independently $CH_2$, $C(CH_3)$, C(=O), or CH;
and/or, B is independently $CH_2$, $CF_2$, O, S, NH, $N(CH_3)$, $N(CD_3)$, N (cyclopropyl), N $(CH_2CF_3)$, $C(CH_3)$, C(=O), N(CN), S(=O), $S(=O)_2$, CH(OH), N $(CH_2CH_3)$, N $(CH_2CH_2OH)$, or CH;
and/or, $A^2$ is independently $CH_2$, O, S, NH, $N(CH_3)$, N (cyclopropyl), $CH(CH_3)$, a linkage, or C(=O);
and/or,

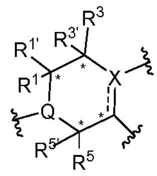

is independently

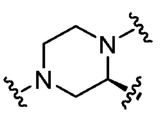, 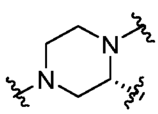, 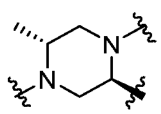,

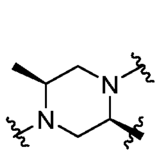, 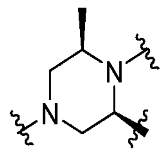, 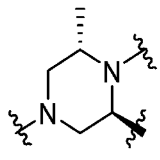, 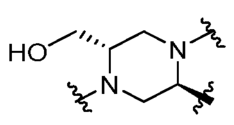, 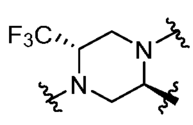,

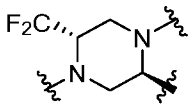, 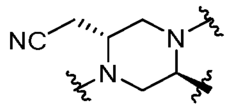, 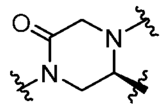, 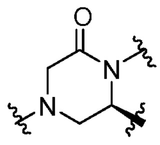, 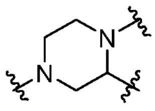, 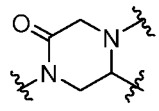,

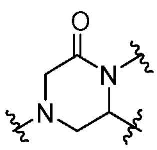, 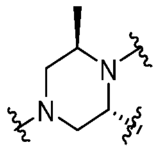, 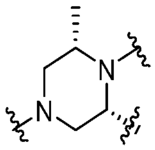, 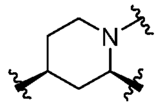 or 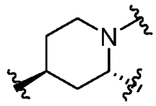;

and/or,

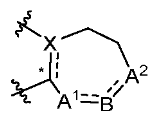

is independently

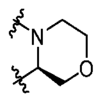, 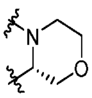, 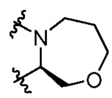, 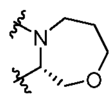, 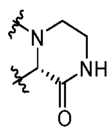,

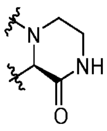, 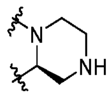, 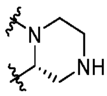, 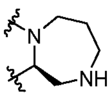, 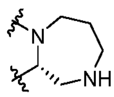, 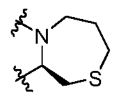, 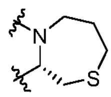, 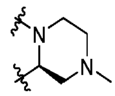, 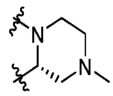,

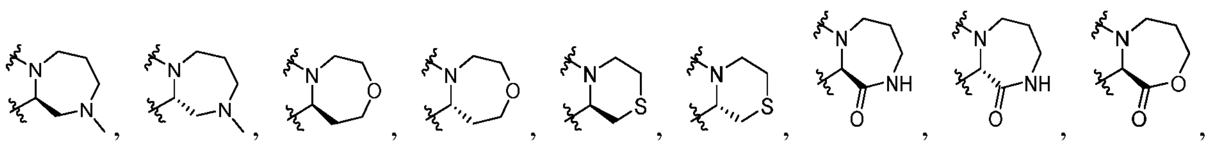

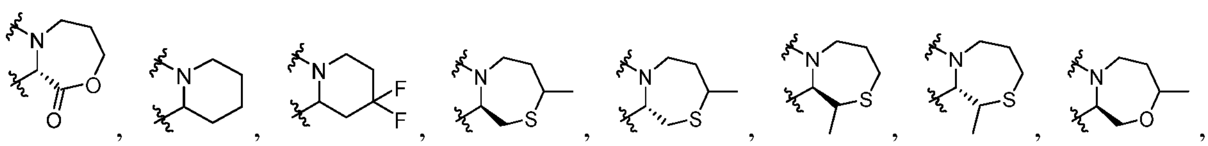

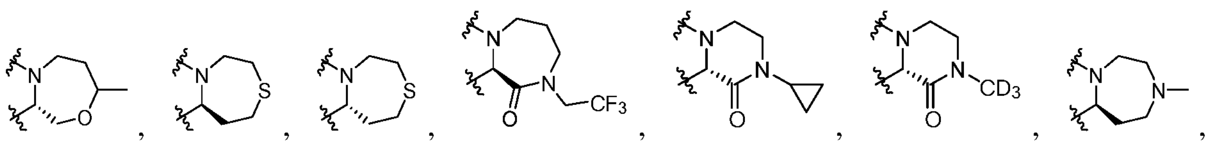

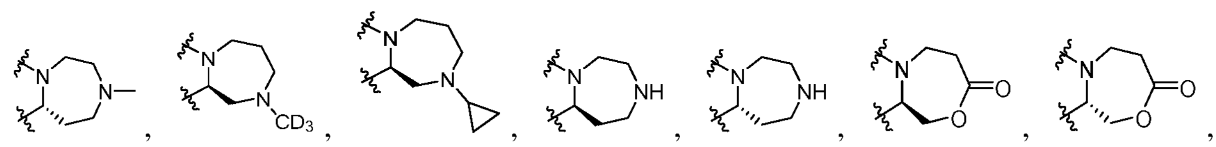

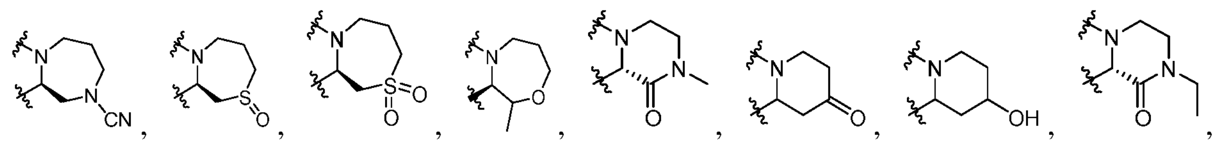

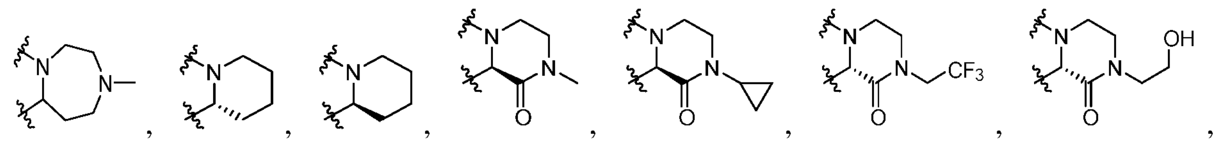

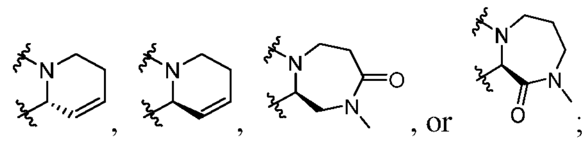

, or ;

and/or,

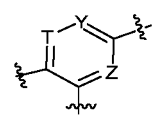

is independently

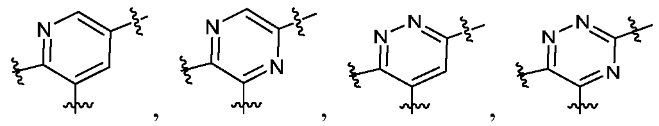

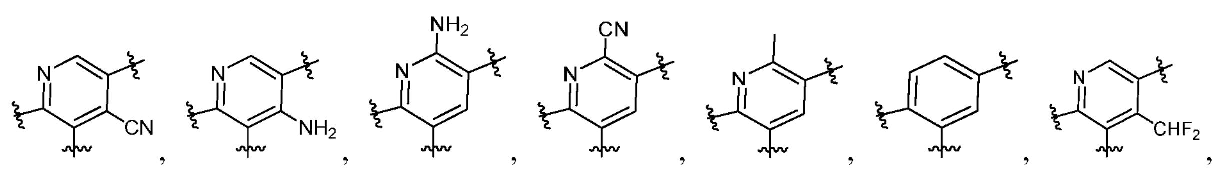

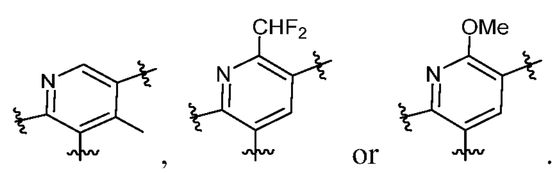

or .

**8.** The fused triheterocyclic compound of Formula IA or the pharmaceutically acceptable salt thereof according to claim 1 or 2, wherein

$R^1$, $R^{1'}$, $R^3$, $R^3$, $R^5$, and $R^{5'}$ are independently H, methyl, HO-methyl, NC-methyl, $CHF_2$, and $CF_3$; or $R^1$ and $R^{1'}$, $R^3$ and $R^{3'}$, and $R^5$ and $R^{5'}$ are independently taken together to form O=;
and/or, $R^2$, and $R^4$ are independently fluoro, bromo, chloro, CN, $CF_3$, and $CF_3$-S-;

and/or, Y is independently CH, N, C(CN), and $C(NH_2)$;
and/or, Z is independently CH, N, C(CN), and $C(NH_2)$;
and/or, $A^1$ is independently $CH_2$, $C(CH_3)$ or C(=O);
and/or, B is independently $CH_2$, $CF_2$, O, S, NH, $N(CH_3)$, $N(CD_3)$, N (cyclopropyl), N $(CH_2CF_3)$, $C(CH_3)$ or C(=O);
and/or, $A^2$ is independently $CH_2$, O, S, NH, $N(CH_3)$, N (cyclopropyl), $CH(CH_3)$ or a linkage;
and/or,

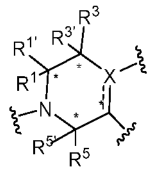

is independently

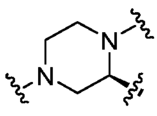 , 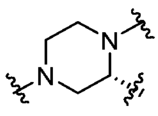 , 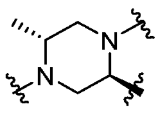 ,

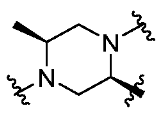 , 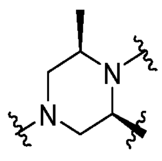 , 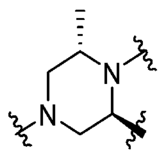 , 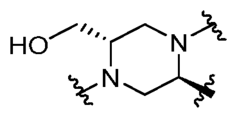 , 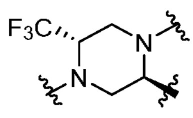 ,

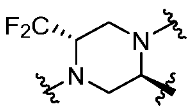 , 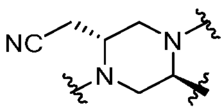 , 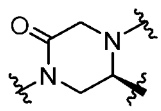 , or 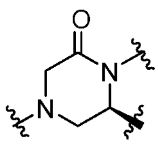 ,

for example,

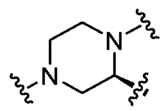 ,

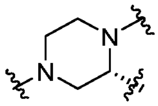 , 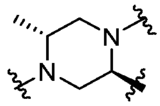 , 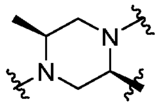 ;

and/or,

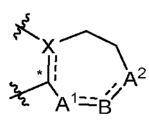

is independently

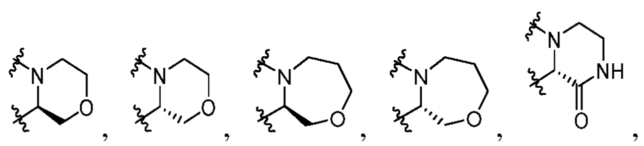

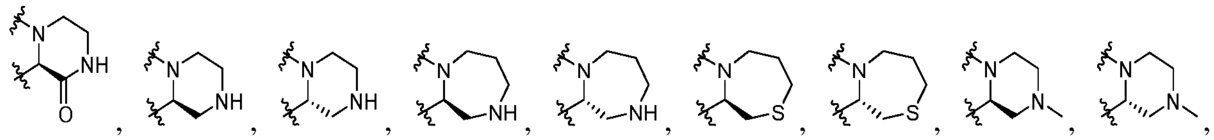

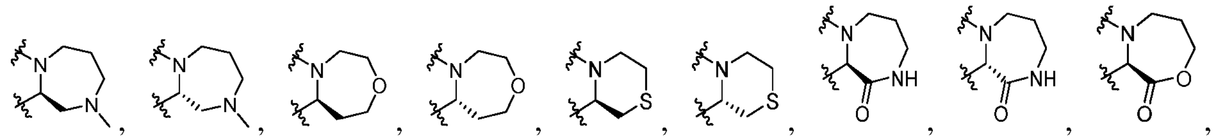

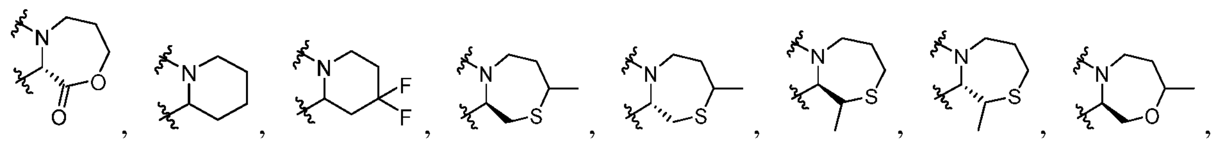

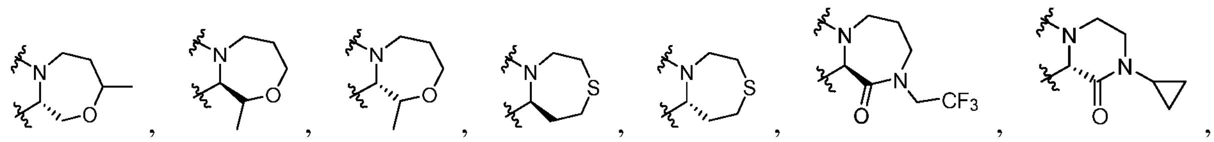

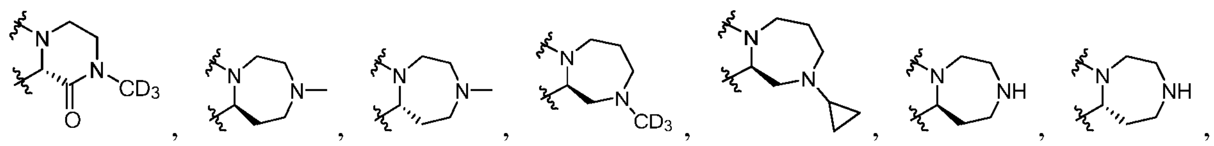

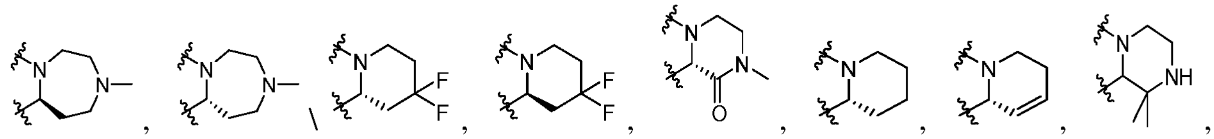

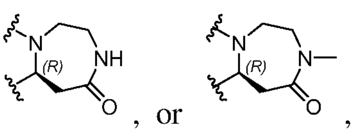

or

for example,

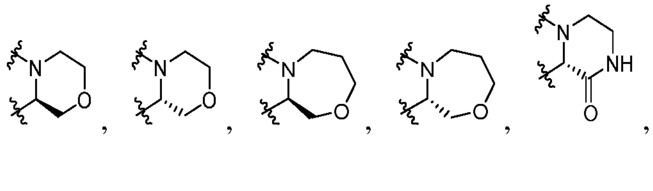

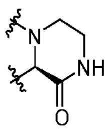, 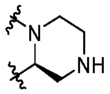, 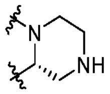, 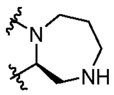, 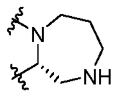, 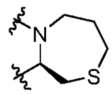, 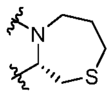, 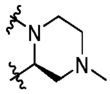, 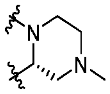,

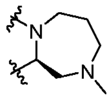, or 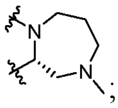;

and/or,

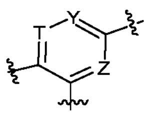

is independently

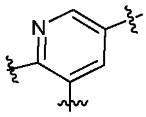, 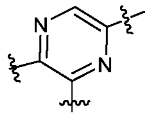, 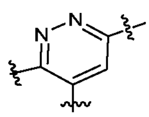, 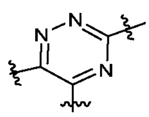

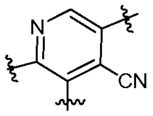, 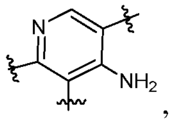, 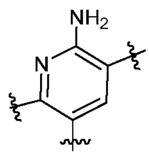, 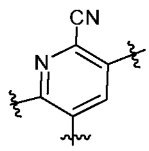, or 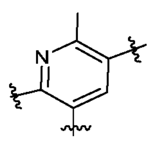;

for example,

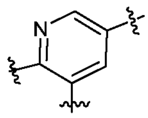, and 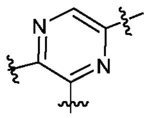.

**9.** The fused triheterocyclic compound of Formula IA or the pharmaceutically acceptable salt thereof according to claim 1, wherein

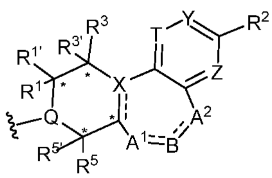

is independently

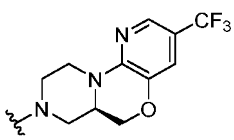 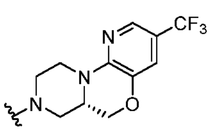

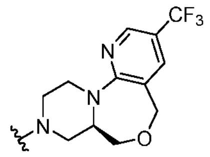 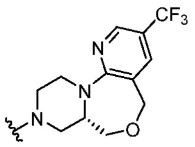 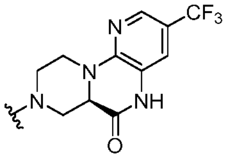 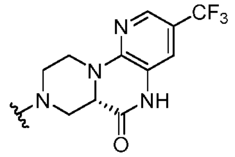 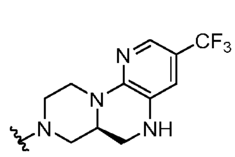

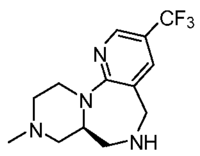 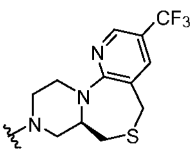 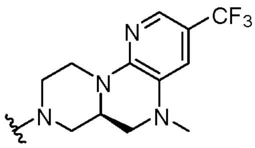 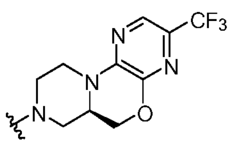 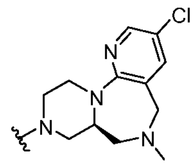

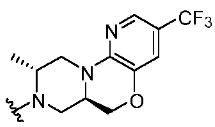 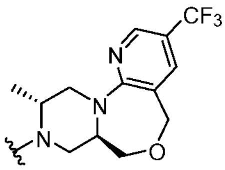 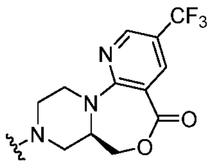 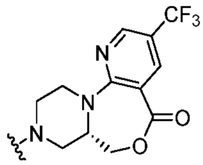 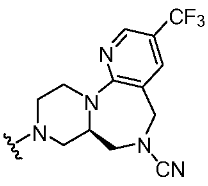

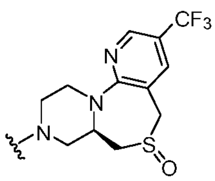 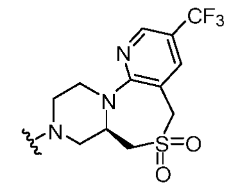 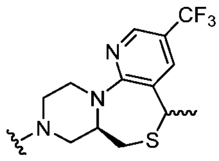 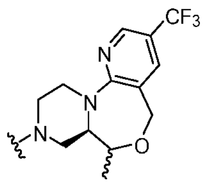 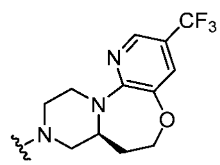

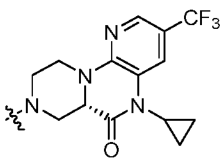 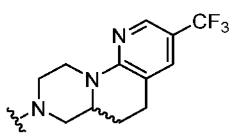 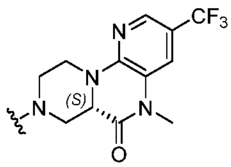 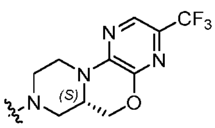 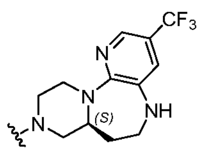

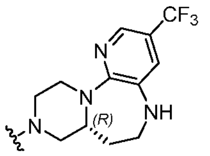 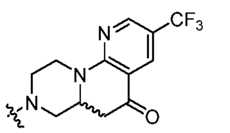 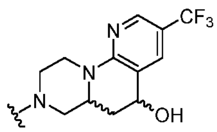 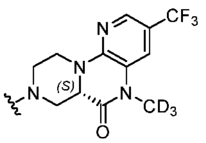 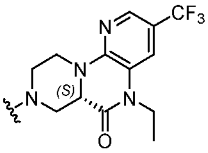

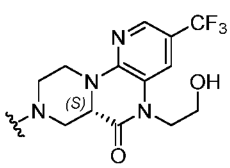 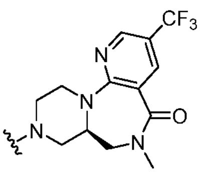 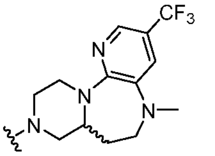 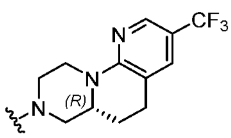 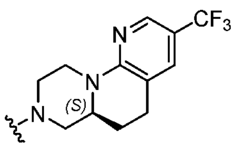

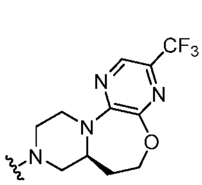 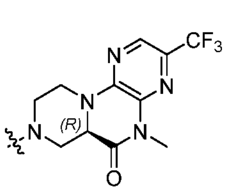 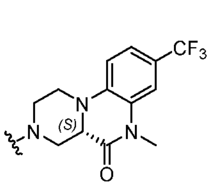 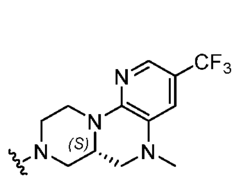 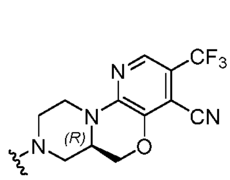

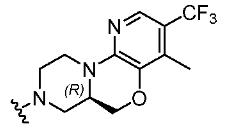 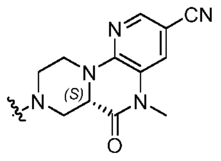 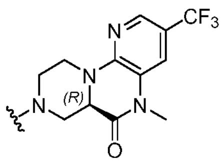 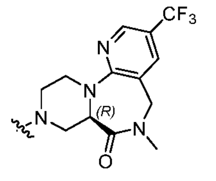 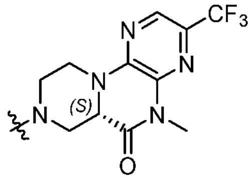

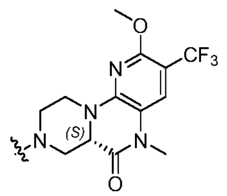 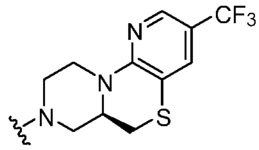 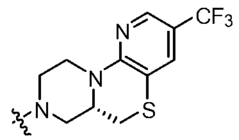 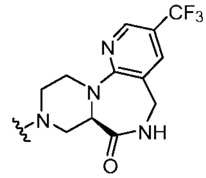 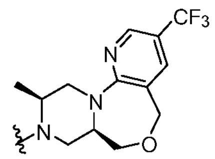

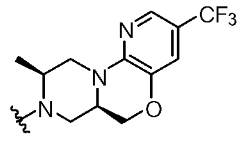 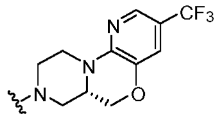 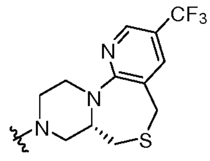 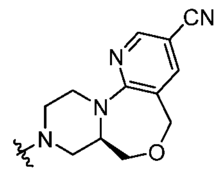 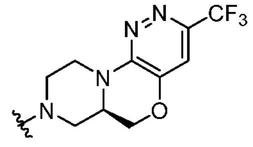

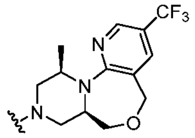 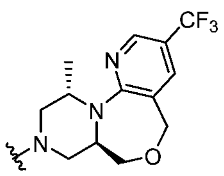 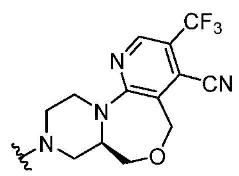 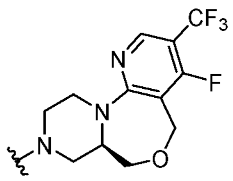 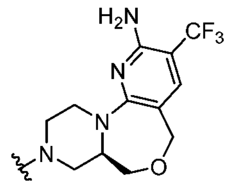

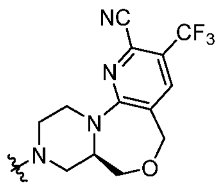 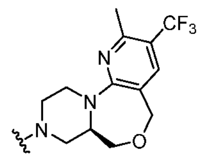 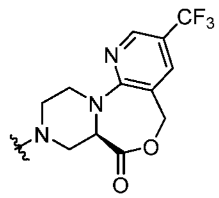 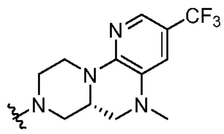 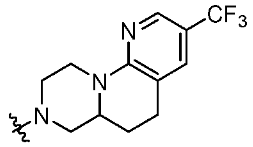

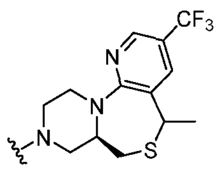 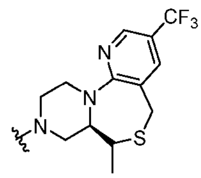 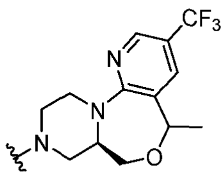 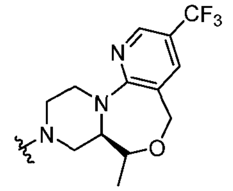 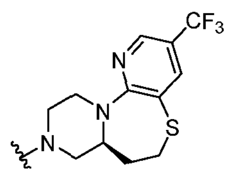

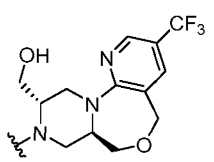 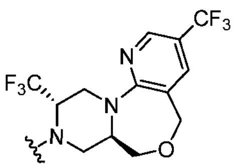 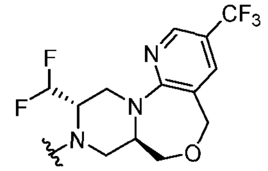 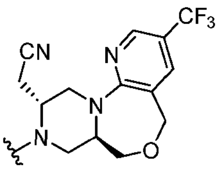 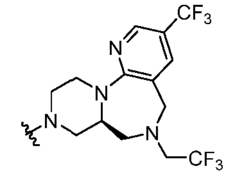

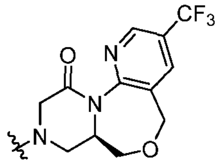 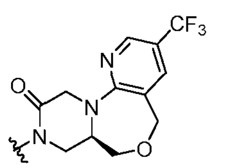 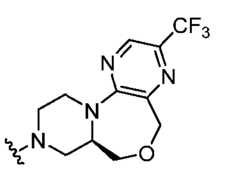 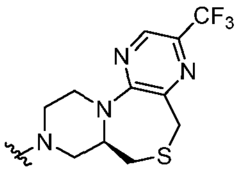 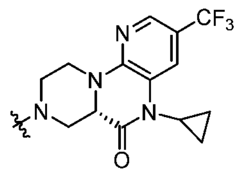

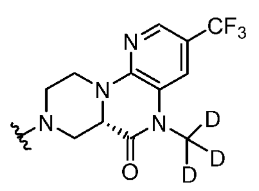 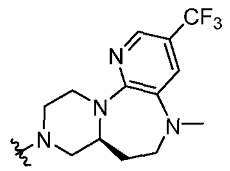 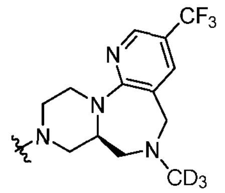 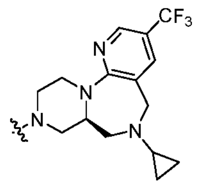 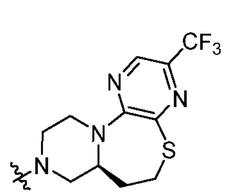

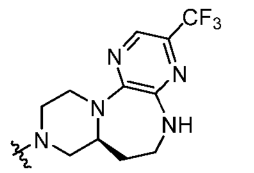 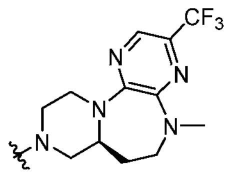 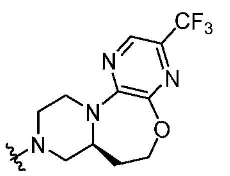 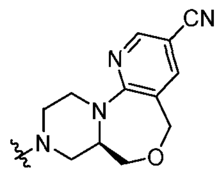 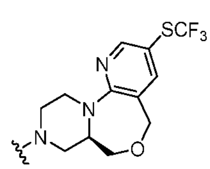

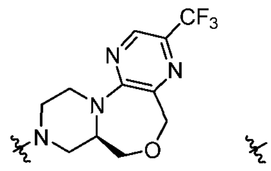 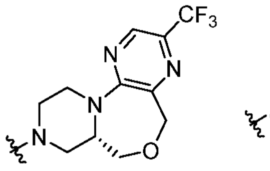 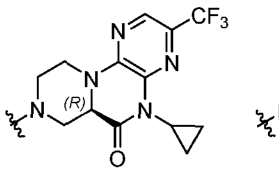 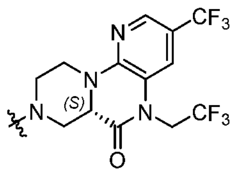 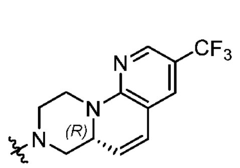

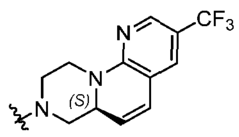 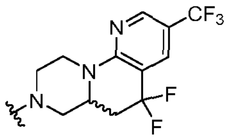 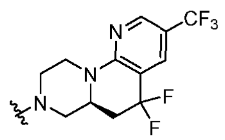 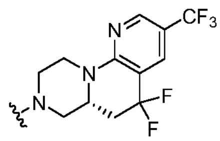 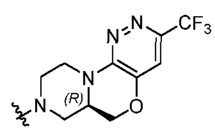

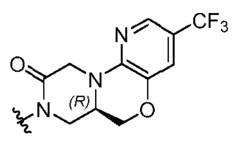 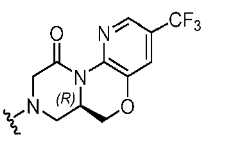 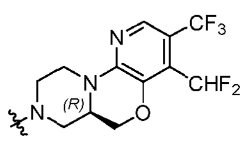 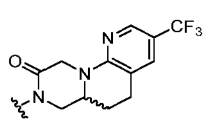 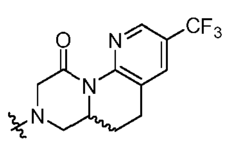

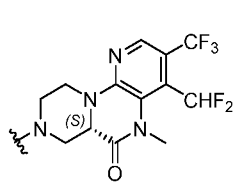 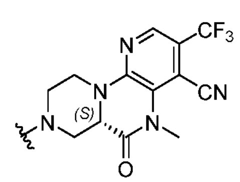 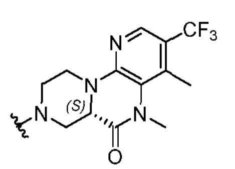 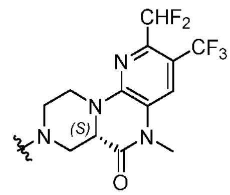 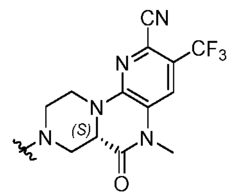

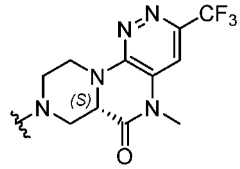 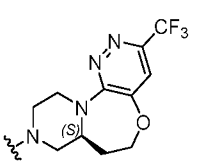 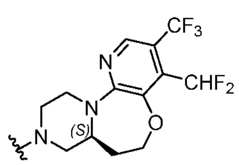 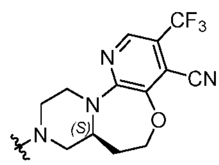 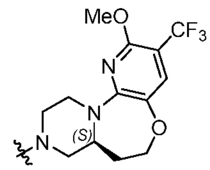

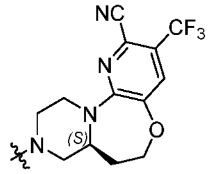 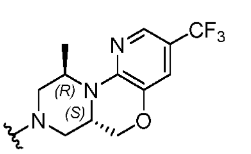 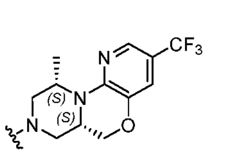 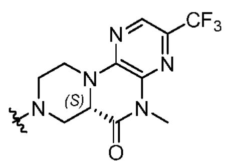 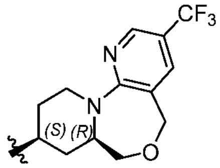

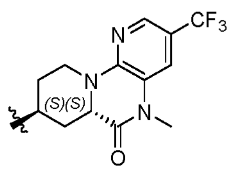 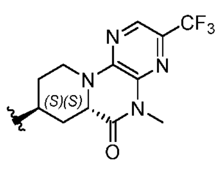 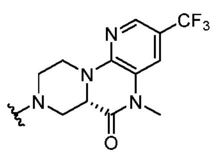 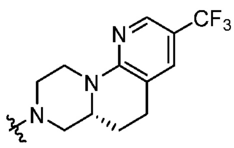 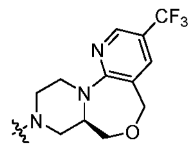

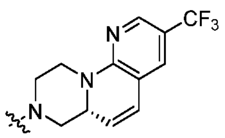 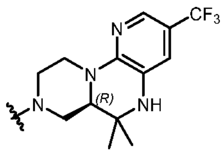 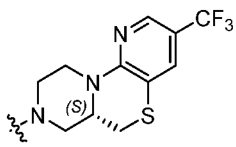 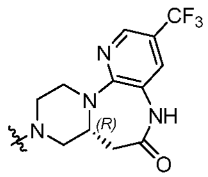 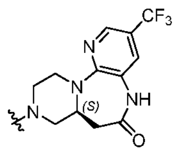 or

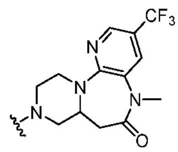 ;

and/or,

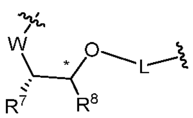

is independently

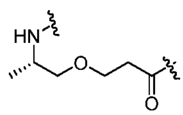 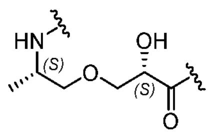

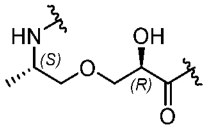 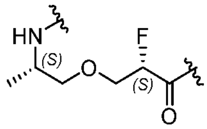 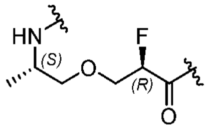 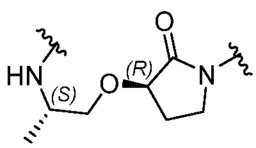 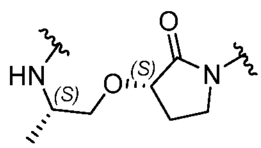

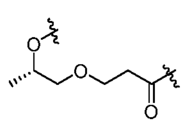 or 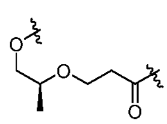 .

**10.** The fused triheterocyclic compound of Formula IA or the pharmaceutically acceptable salt thereof according to claim 1 or 2, wherein the fused triheterocyclic compound of Formula IA is any one of:

embodiment 1, in which
$R^1$, and $R^{1'}$ are independently H or $C_{1-6}$ alkyl;
$R^3$, $R^{3'}$, $R^5$, and $R^{5'}$ are independently H;
$R^2$, and $R^4$ are independently $C_{1-6}$ alkyl substituted with one or more halo;
X and T are independently N;
Y is independently $C(R^{3c})$;
Z is independently $C(R^{3d})$ or N;

$A^1$ is independently $C(R^{4b}R^{4c})$ or C(=O);
B is independently O, S, N or $N(R^{6d})$, and $A^2$ is independently $C(R^{5b}R^{5c})$ or a linkage; and
- - - -represents a single bond;
embodiment 2, in which
$R^1$, and $R^{1'}$ are independently H or methyl;
$R^3$, $R^{3'}$, $R^5$, and $R^{5'}$ are independently H;
$R^2$, and $R^4$ are independently $CF_3$;
X and T are independently N;
Y is independently CH;
Z is independently CH or N;
$A^1$ is independently $CH_2$ or C(=O);
B is independently O, S, N or NH, and $A^2$ is independently $CH_2$ or a linkage; and
- - - -represents a single bond;
embodiment 3, in which
$R^1$, $R^{1'}$, $R^3$, $R^{3'}$, $R^5$, and $R^{5'}$ are independently H;
$R^2$, and $R^4$ are independently $C_{1-6}$ alkyl substituted with one or more halo;
X and T are independently N;
Y is independently $C(R^{3c})$;
Z is independently $C(R^{3d})$;
$A^1$ is independently $C(R^{4b}R^{4c})$;
B is independently O or S;
$A^2$ is independently $C(R^{5b}R^{5c})$ or a linkage; and
- - - -represents a single bond;
embodiment 4, in which
$R^1$, $R^{1'}$, $R^3$, $R^{3'}$, $R^5$, and $R^{5'}$ are independently H;
$R^2$, and $R^4$ are independently $CF_3$;
X and T are independently N;
Y, and Z are independently CH;
$A^1$ is independently $CH_2$;
B is independently O or S;
$A^2$ is independently $CH_2$ or a linkage; and
- - - -represents a single bond;
embodiment 5, in which
L is

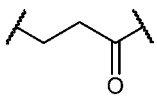,
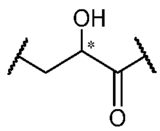, or
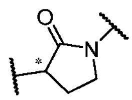;

$R^1$, and $R^{1'}$ are independently H, or $R^1$ and $R^{1'}$ are taken together to form O=;
$R^3$, and $R^{3'}$ are independently H, $C_{1-6}$ alkyl, or $R^3$ and $R^{3'}$ are taken together to form O=;
$R^5$, and $R^{5'}$ are independently H;
$R^2$, and $R^4$ are independently halo, CN, or $C_{1-6}$ alkyl substituted with one or more halo;
XisN;
T is $C(R^{3b})$ or N;
$R^{3b}$ is H;
Y is $C(R^{3c})$;
$R^{3c}$ is H, $C_{1-6}$ alkyl substituted with one or more $R^{a4}$, or $C_{1-6}$ alkoxy;
Z is $C(R^{3d})$;
$R^{3d}$ is H, $C_{1-6}$ alkyl or $C_{1-6}$ alkyl substituted with one or more $R^{a4}$;
$A^1$ is independently $C(R^{4a})$, $C(R^{4b}R^{4c})$ or C(=O), where
$R^{4a}$, $R^{4b}$, and $R^{4c}$ are independently H or $C_{1-6}$ alkyl;
when B is independently O, S(=O), $S(=O)_2$, or $N(R^{6d})$, $A^2$ is independently $C(R^{5a})$, $C(R^{5b}R^{5c})$, or a linkage;
when B is independently $C(R^{6a})$, or $C(R^{6b}R^{6c})$, $A^2$ is independently $N(R^{5d})$, or a linkage;
in which $R^{5b}$ and $R^{5c}$ are independently H, or $R^{5b}$ and $R^{5c}$ are taken together to form =O;
$R^{5d}$ is $C_{1-6}$ alkyl;

$R^{6d}$ is $C_{1-6}$ alkyl, $C_{1-6}$ alkyl substituted with one or more $R^{a3}$, $C_{3-6}$ cycloalkyl, or CN;
$R^{6a}$ is H;
$R^{6b}$ and $R^{6c}$ are independently H, -OH, or $R^{6b}$ and $R^{6c}$ are taken together to form =O; and
$R^{a3}$ is halo or -OH; and
- - - -represents a single bond;
embodiment 6, in which
L is

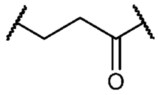 or 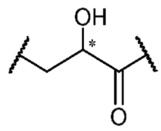 ;

$R^1$, and $R^{1'}$ are independently H or $C_{1-6}$ alkyl;
$R^3$, $R^{3'}$, $R^5$, and $R^{5'}$ are independently H;
$R^2$ is CN, or $C_{1-6}$ alkyl substituted with one or more halo;
$R^4$ is $C_{1-6}$ alkyl substituted with one or more halo;
XisN;
T is $C(R^{3b})$ or N, where $R^{3b}$ is H;
Y is independently $C(R^{3c})$, where $R^{3c}$ is H or $C_{1-6}$ alkyl;
Z is independently $C(R^{3d})$ or N, where $R^{3d}$ is H, CN or $C_{1-6}$ alkyl;
$A^1$ is independently $C(R^{4b}R^{4c})$ or C(=O), where $R^{4b}$ and $R^{4c}$ are independently H;
when B is independently O, S, S(=O), $S(=O)_2$, or $N(R^{6d})$, $A^2$ is independently $C(R^{5b}R^{5c})$ or a linkage;
when B is independently $C(R^{6b}R^{6c})$, $A^2$ is independently O, $N(R^{5d})$ or a linkage;
in which $R^{5b}$ and $R^{5c}$ are independently H, or $C_{1-6}$ alkyl, or $R^{5b}$ and $R^{5c}$ are taken together to form =O;
$R^{6d}$ is H, $C_{1-6}$ alkyl, $C_{1-6}$ alkyl substituted with one or more $R^3$, $C_{3-6}$ cycloalkyl, or CN;
$R^{5d}$ is H, or $C_{1-6}$ alkyl; and
$R^{6b}$ and $R^{6c}$ are independently H, -OH, or $R^{6b}$ and $R^{6c}$ are taken together to form =O; and
$R^{a3}$ is deuterium or -OH; and
- - - -represents a single bond;
embodiment 7, in which
L is

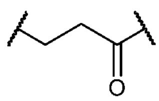 ;

$R^1$, and $R^{1'}$ are independently H or $C_{1-6}$ alkyl;
$R^3$, $R^{3'}$, $R^5$, and $R^{5'}$ are independently H;
$R^2$ is CN, or $C_{1-6}$ alkyl substituted with one or more halo;
$R^4$ is $C_{1-6}$ alkyl substituted with one or more halo;
X and T are independently N;
Y is independently $C(R^{3c})$, where $R^{3c}$ is H;
Z is independently $C(R^{3d})$ or N, where $R^{3d}$ is H;
$A^1$ is independently $C(R^{4b}R^{4c})$ or C(=O), where $R^{4b}$ and $R^{4c}$ are independently H;
when B is independently O, S, or $N(R^{6d})$, $A^2$ is independently $C(R^{5b}R^{5c})$ or a linkage;
when B is independently $C(R^{6b}R^{6c})$, $A^2$ is independently O, $N(R^{5d})$ or a linkage;
when $A^1$ is C(=O) and B is $N(R^{6d})$, $A^2$ is a linkage;
in which $R^{5b}$ and $R^{5c}$ are independently H;
$R^{6d}$ is H, $C_{1-6}$ alkyl, $C_{1-6}$ alkyl substituted with one or more $R^{a3}$, or $C_{3-6}$ cycloalkyl;
$R^{6b}$ and $R^{6c}$ are independently H, -OH, or $R^{6b}$ and $R^{6c}$ are taken together to form =O; and
$R^{5d}$ is H, or $C_{1-6}$ alkyl; and
$R^{a3}$ is deuterium; and
- - - -represents a single bond;
embodiment 8, in which
L is

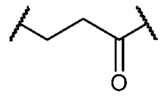 ;

$R^1$, $R^{1'}$, $R^3$, $R^{3'}$, $R^5$, and $R^{5'}$ are independently H;
$R^2$, and $R^4$ are independently $C_{1-6}$ alkyl substituted with one or more halo;
X and T are independently N;
Y is independently $C(R^{3c})$, where $R^{3c}$ is H;
Z is independently $C(R^{3d})$ or N, where $R^{3d}$ is H;
$A^1$ is independently $C(R^{4b}R^{4c})$ or C(=O), where $R^{4b}$ and $R^{4c}$ are independently H;
when B is independently O, S, or $N(R^{6d})$, $A^2$ is independently $C(R^{5b}R^{5c})$ or a linkage;
when B is independently $C(R^{6b}R^{6c})$, $A^2$ is independently O, $N(R^{5d})$ or a linkage;
when B is independently $N(R^{6d})$, $A^1$ is independently C(=O);
when $A^1$ is C(=O) and B is $N(R^{6d})$, $A^2$ is a linkage;
when T and Z are both N, $A^2$ is independently O, $N(R^{5d})$ or $C(R^{5b}R^{5c})$;
in which $R^{5b}$ and $R^{5c}$ are independently H;
$R^{6d}$ are independently $C_{1-6}$ alkyl, or $C_{1-6}$ alkyl substituted with one or more $R^{a3}$;
in which $R^{6b}$ and $R^{6c}$ are independently H;
$R^{5d}$ is H; and
$R^{a3}$ is deuterium; and
- - - - represents a single bond.

**11.** The fused triheterocyclic compound of Formula IA or the pharmaceutically acceptable salt thereof according to claim 1, wherein
the fused triheterocyclic compound of Formula IA has any one of the following structures:

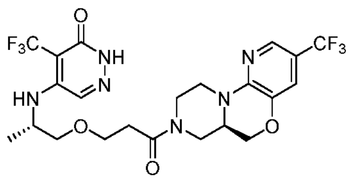 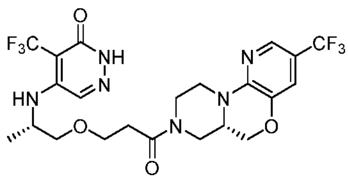 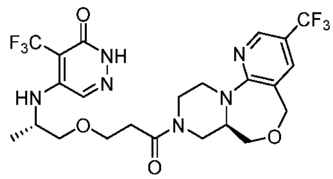

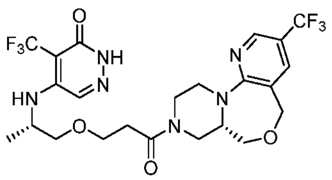 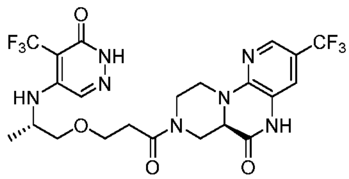 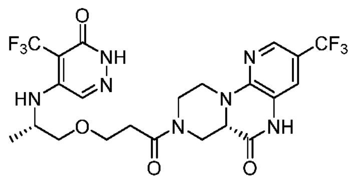

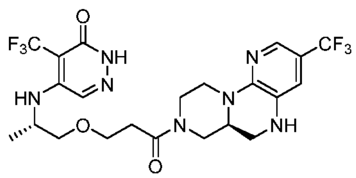 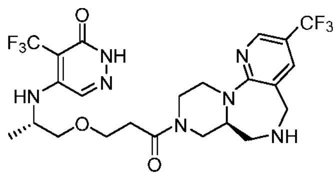 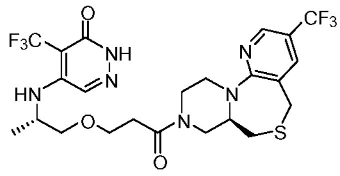

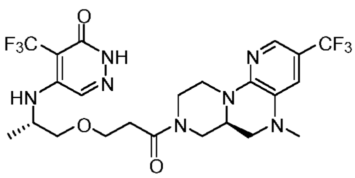 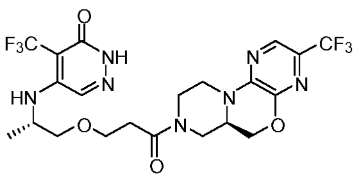 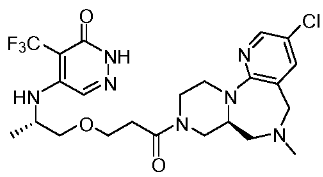

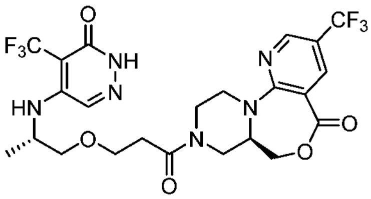
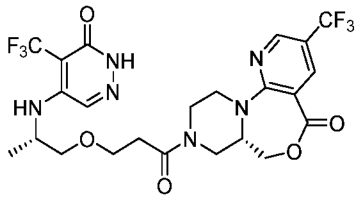
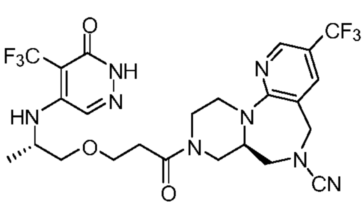

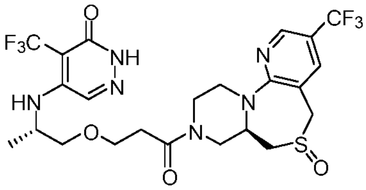
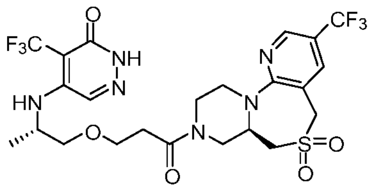
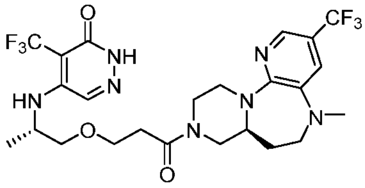

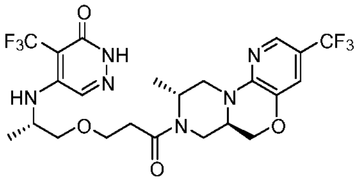
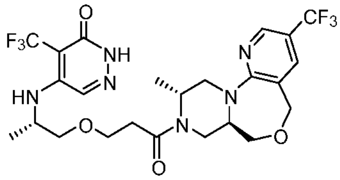
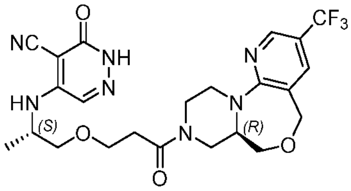

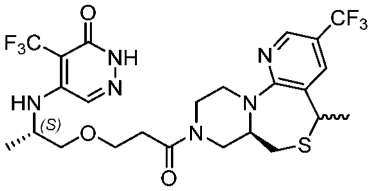
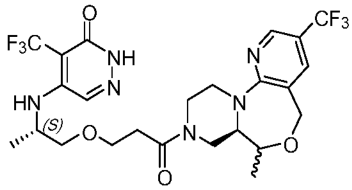
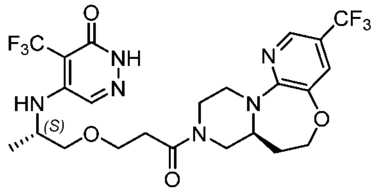

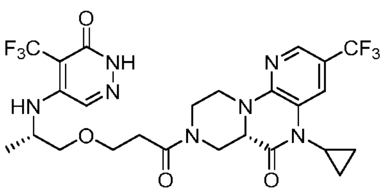
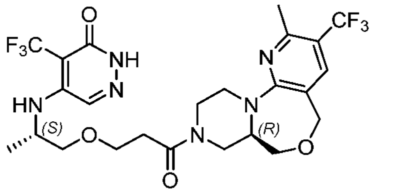
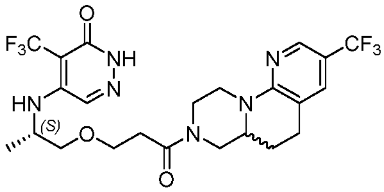

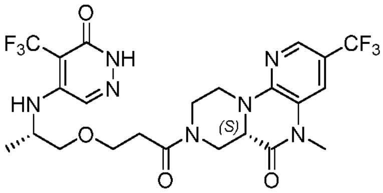
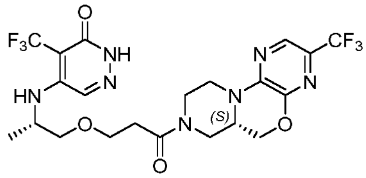
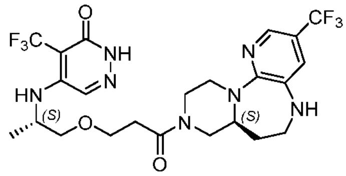

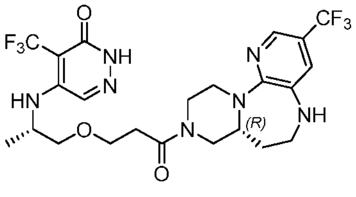
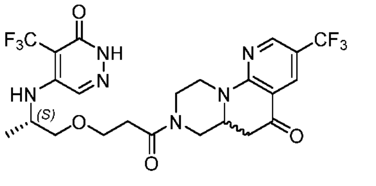
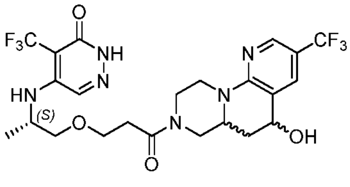

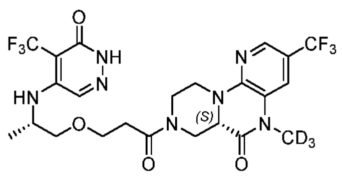
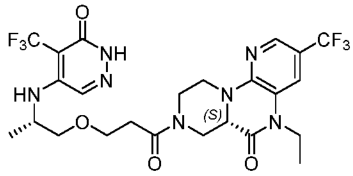
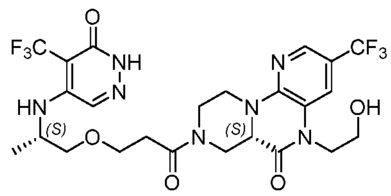

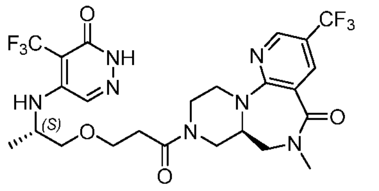
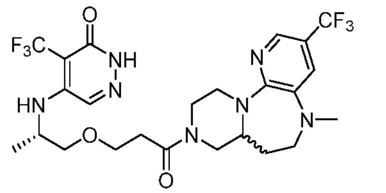
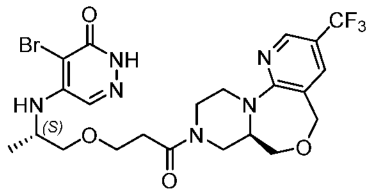

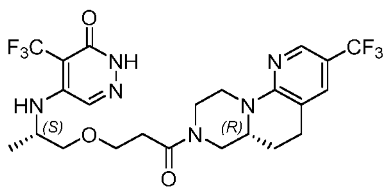
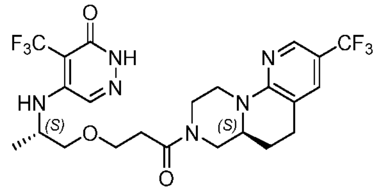
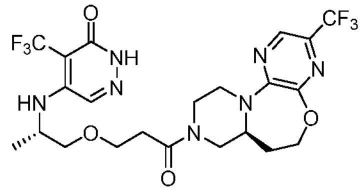

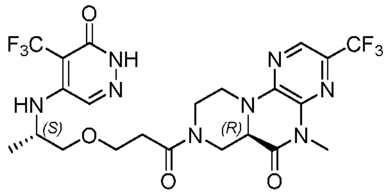
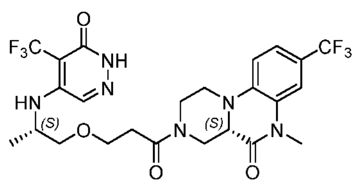
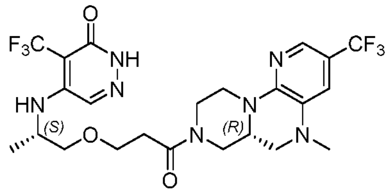

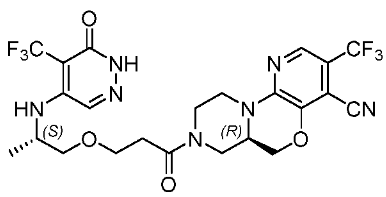
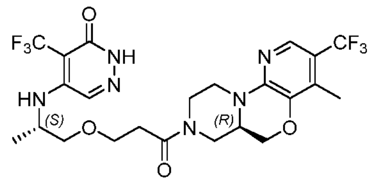
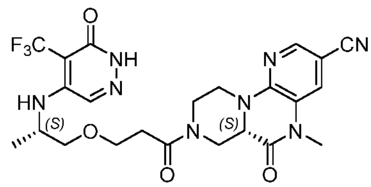

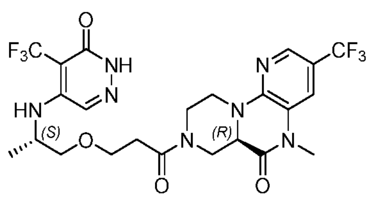
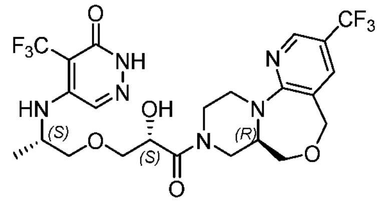
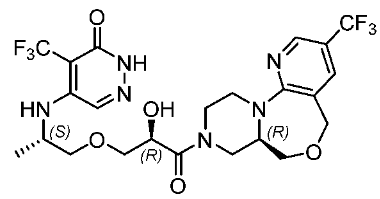

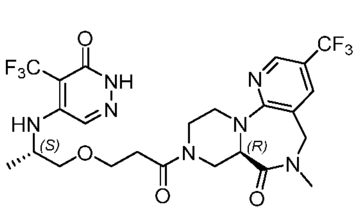
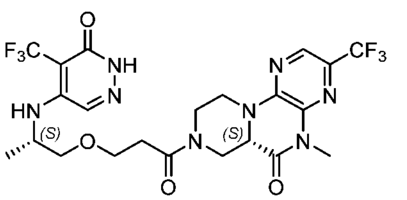
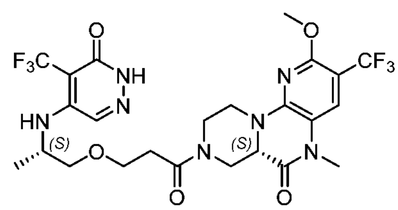

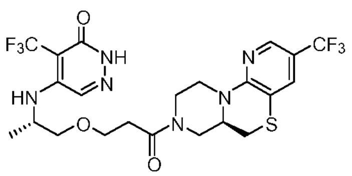
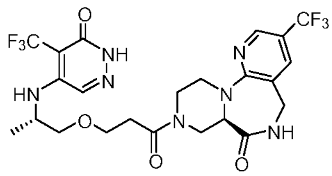
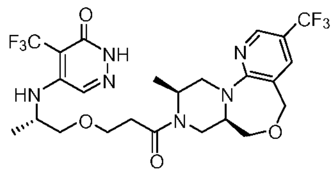

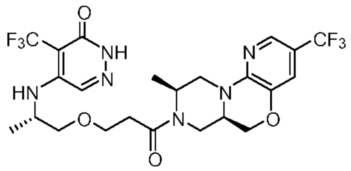
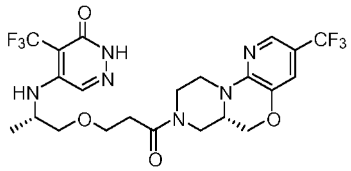

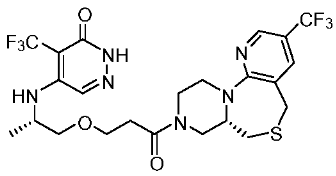
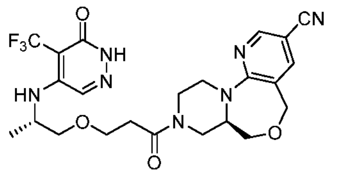
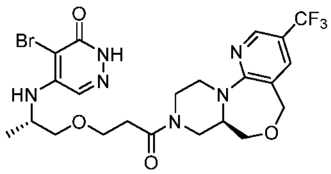

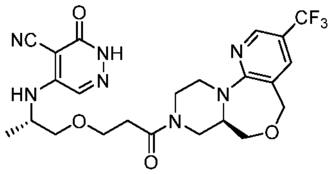
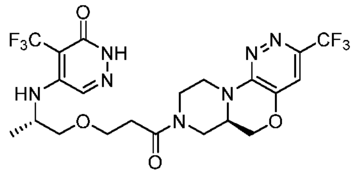
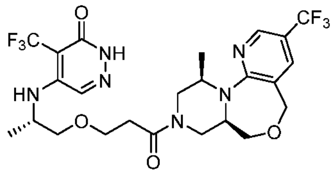

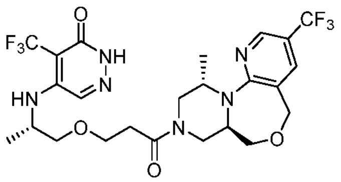
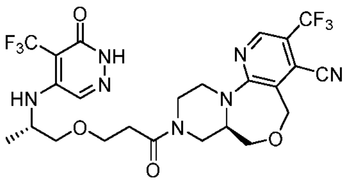
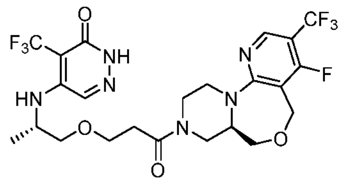

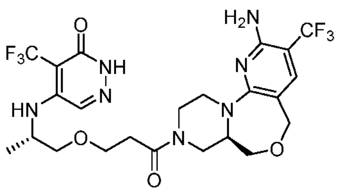
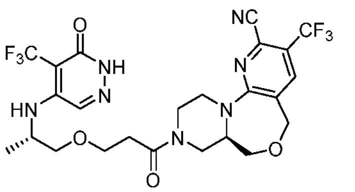
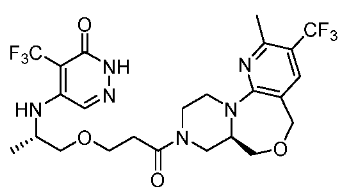

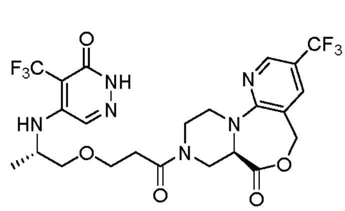
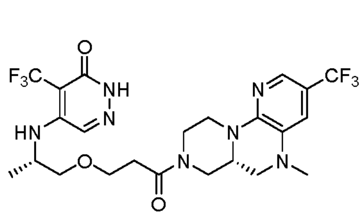

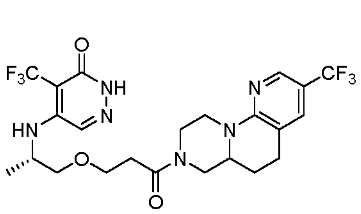
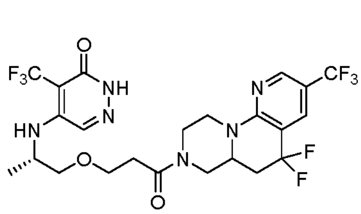
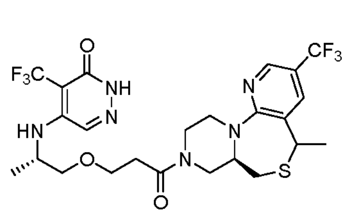

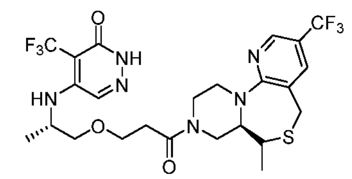
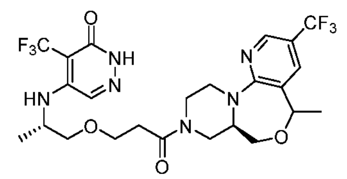
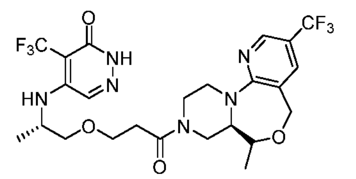

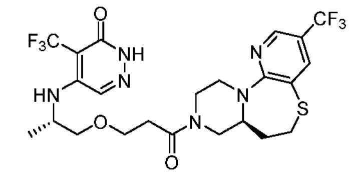
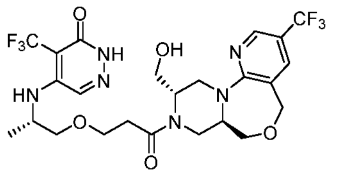
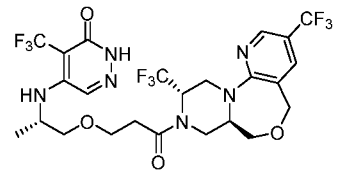

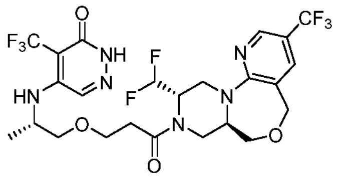
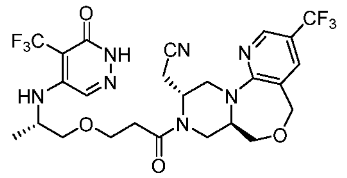
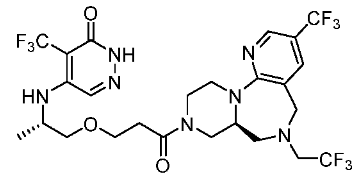

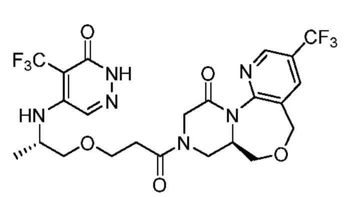
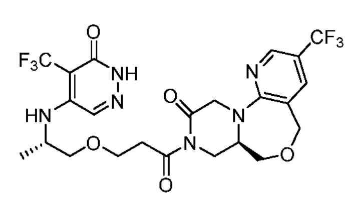
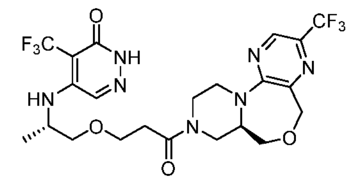

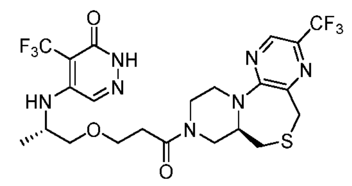
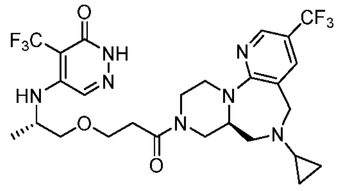
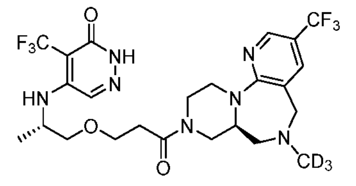

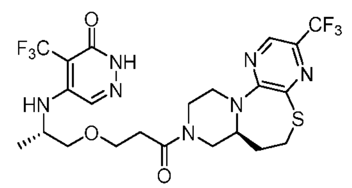
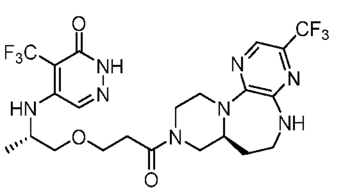
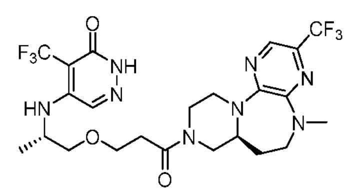

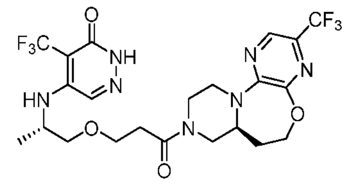
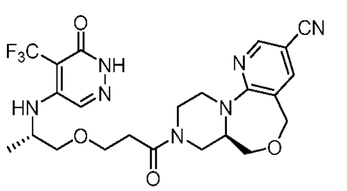
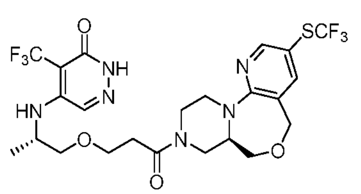

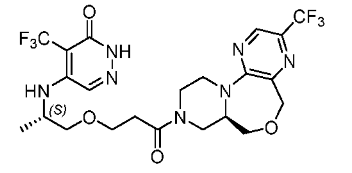
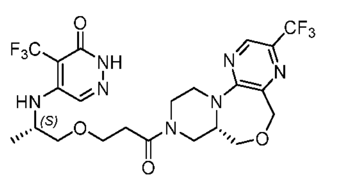
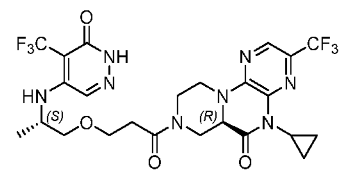

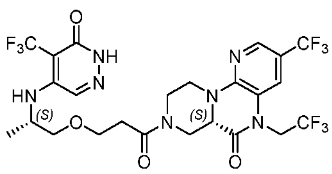 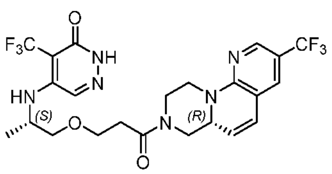 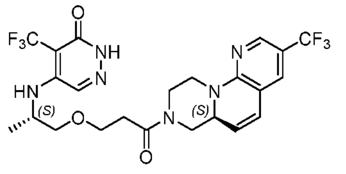

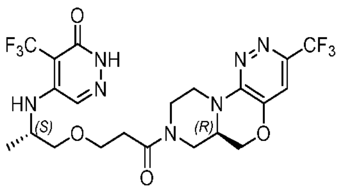 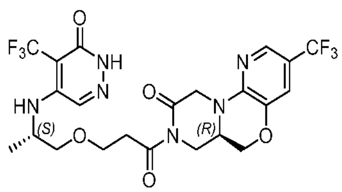 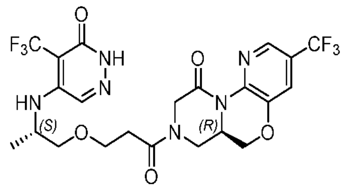

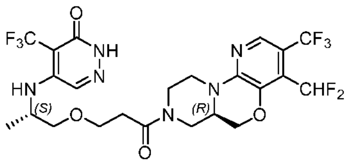 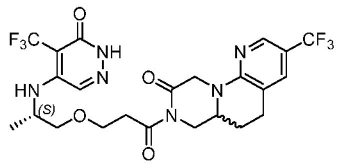

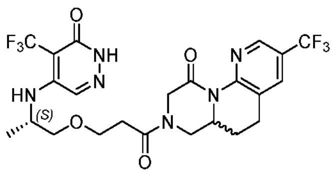 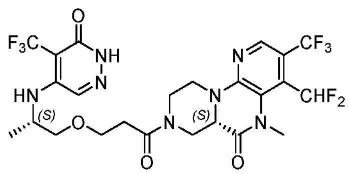 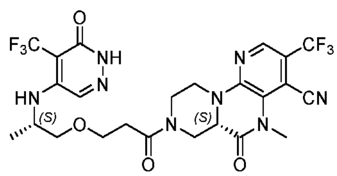

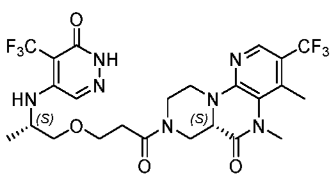 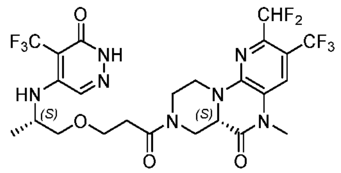 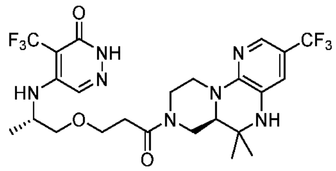

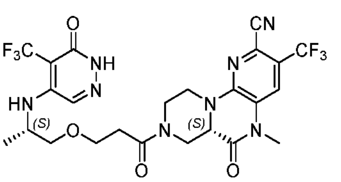 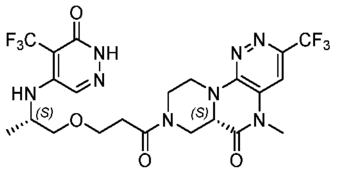 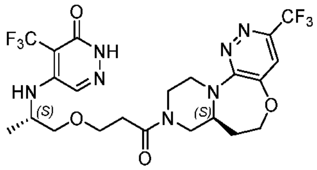

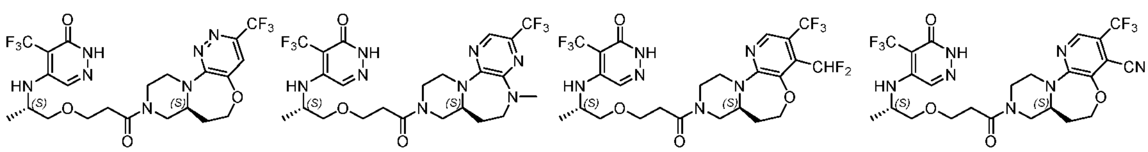

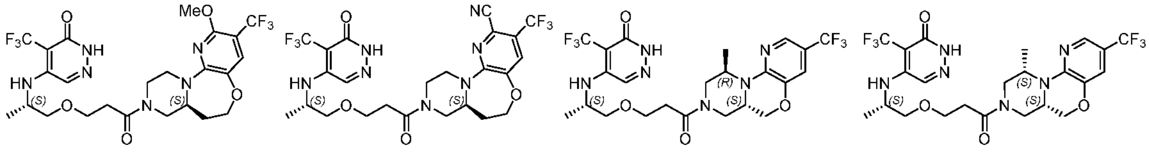

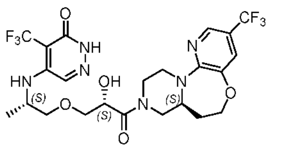
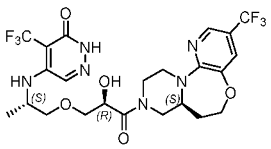
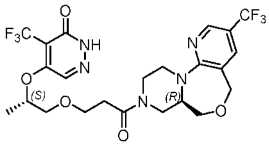

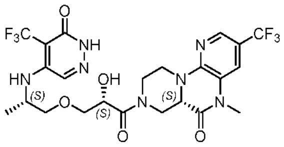
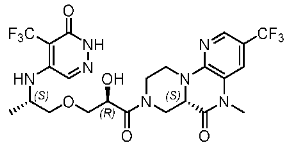
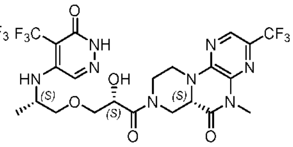
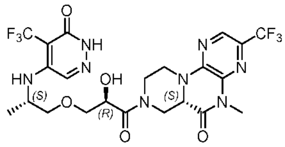

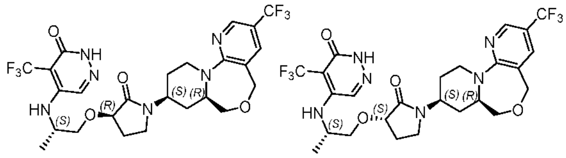
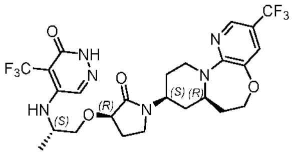
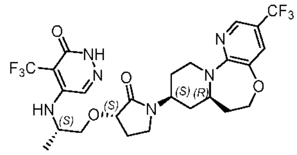

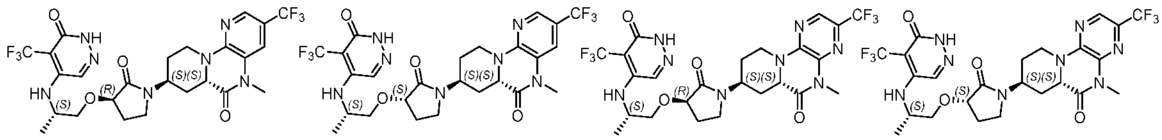

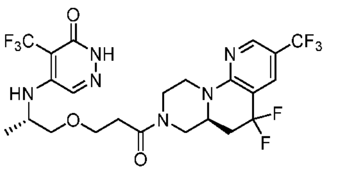
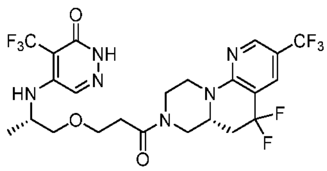
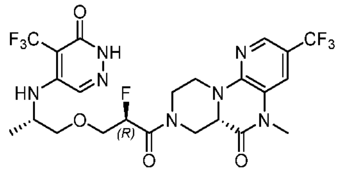

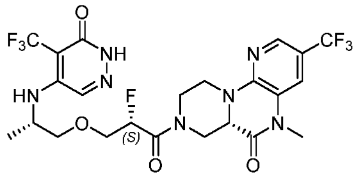
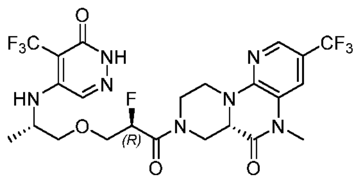
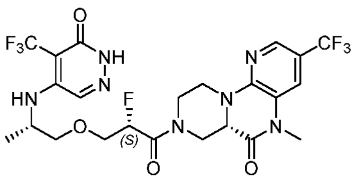

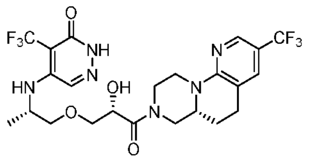
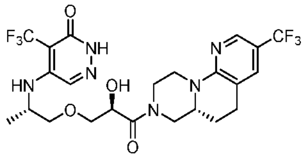
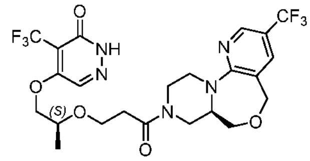

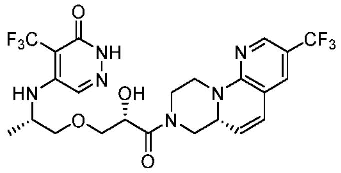
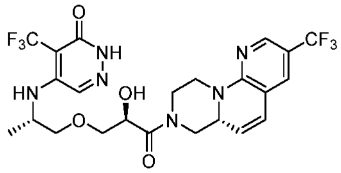
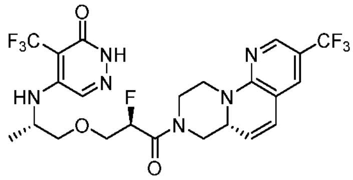

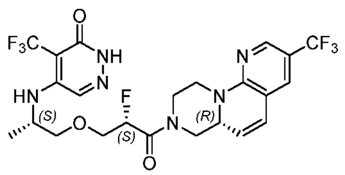 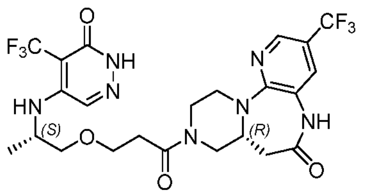 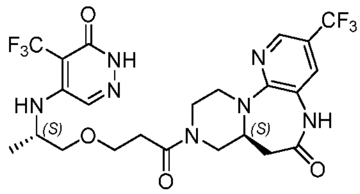

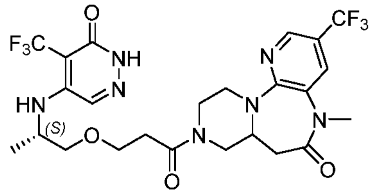,

in which " " bond represents a combination of R configuration and S configuration.

**12.** A method for preparing the fused triheterocyclic compound of Formula IA according to any one of claims 1 to 11, comprising the following steps:

subjecting Compound of Formula IIA and Compound of Formula IIIA to an amidation reaction in a solvent in the presence of a base and a condensing agent, to obtain the fused triheterocyclic compound of Formula IA:

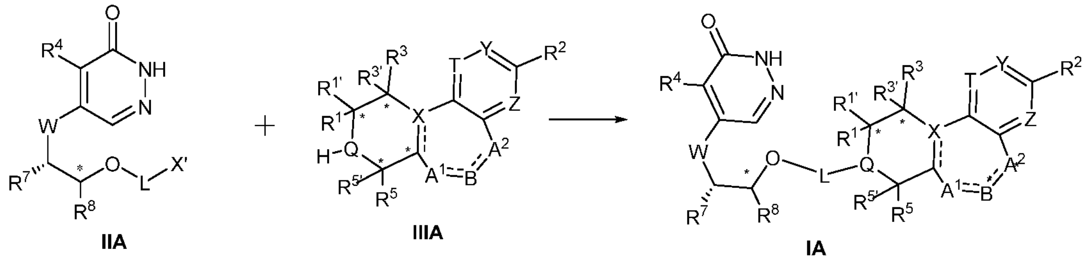

IIA IIIA IA

wherein W, L, $R^7$, $R^8$, $R^1$, $R^{1'}$, $R^3$, $R^{3'}$, $R^5$, $R^{5'}$, $R^2$, $R^4$, X, T, Y, Z, $A^1$, B, $A^2$, Q, ---- and * are as defined in any one of claims 1 to 11; and
wherein preferably,
the solvent is DMF or dichloromethane;
and/or, the base is diisopropylethyl amine or $K_2CO_3$;
and/or, the molar ratio of the base to Compound of Formula IIA is 1: 1;
and/or, the condensing agent is propylphosphonic anhydride;
and/or, the condensing agent is used as a 50% solution in ethyl acetate;
and/or, the molar ratio of the condensing agent to the compound of Formula IIA is 1: 1;
and/or, the molar ratio of Compound of Formula IIA to Compound of Formula III is 1: 1;
and/or, the amidation reaction is carried out under a nitrogen atmosphere;
and/or, the reaction temperature of the amidation reaction is from 0°C to 60°C.

**13.** A pharmaceutical composition, comprising the fused triheterocyclic compound of Formula IA or the pharmaceutically acceptable salt thereof according to any one of claims 1 to 11, and a pharmaceutically acceptable adjuvant.

**14.** Use of the fused triheterocyclic compound of Formula IA or the pharmaceutically acceptable salt thereof according to any one of claims 1 to 11 in the preparation of PARP inhibitors, wherein the PARP is PARP7.

**15.** Use of the fused triheterocyclic compound of Formula IA or the pharmaceutically acceptable salt thereof according to any one of claims 1 to 11 in the preparation of drugs for preventing and/or treating PARP-related diseases or PARP-mediated diseases, wherein

the PARP is PARP7;

and/or the PARP-related or PARP-mediated diseases comprise abnormal proliferative diseases, including, but not limited to, cancers, such as lung cancer, colon cancer, colorectal cancer, pancreatic cancer, renal cancer, gastric cancer, esophageal cancer, ovarian cancer, breast cancer, cervical cancer, head and neck cancer, bladder cancer, melanoma, fibrosarcoma and glioblastoma.

**16.** Use of the fused triheterocyclic compound of Formula IA or the pharmaceutically acceptable salt thereof according to any one of claims 1 to 11 in the preparation of drugs for preventing and/or treating abnormal proliferative diseases, including, but not limited to, cancers, such as lung cancer, colon cancer, colorectal cancer, pancreatic cancer, renal cancer, gastric cancer, esophageal cancer, ovarian cancer, breast cancer, cervical cancer, head and neck cancer, bladder cancer, melanoma, fibrosarcoma and glioblastoma.

**INTERNATIONAL SEARCH REPORT**

International application No.
**PCT/CN2022/074278**

**A. CLASSIFICATION OF SUBJECT MATTER**

C07D 471/14(2006.01)i; C07D 498/14(2006.01)i; C07D 513/14(2006.01)i; A61K 31/501(2006.01)i; A61K 31/4985(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D A61K A61P

Documentation searched other than minimum documentation to the extent that such documents are included in the fields searched

Electronic data base consulted during the international search (name of data base and, where practicable, search terms used)

WPI, EPODOC, CNPAT, CAPLUS(STN), MARPAT(STN), CNKI, 百度学术: 上海湃隆, 谷晓辉, 张浩义, 多聚ADP核糖聚合酶, 哒嗪? , 三并杂环, 哌嗪, 吡啶, pyridazin? , piperazin?, pyridin?, PARP? , structural formula search,

**C. DOCUMENTS CONSIDERED TO BE RELEVANT**

| Category* | Citation of document, with indication, where appropriate, of the relevant passages | Relevant to claim No. |
|---|---|---|
| PA | WO 2021087025 A1 (RIBON THERAPEUTICS INC.) 06 May 2021 (2021-05-06)<br>see claims 1-80 | 1-16 |
| PA | WO 2021087018 A1 (RIBON THERAPEUTICS INC.) 06 May 2021 (2021-05-06)<br>see claims 1-40 | 1-16 |
| A | WO 2019212937 A1 (RIBON THERAPEUTICS INC.) 07 November 2019 (2019-11-07)<br>see claims 1-428 and embodiment compounds | 1-16 |
| A | WO 2020223229 A1 (RIBON THERAPEUTICS INC.) 05 November 2020 (2020-11-05)<br>see claims 1-24 | 1-16 |

☐ Further documents are listed in the continuation of Box C.    ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 April 2022** | **26 April 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**
**Information on patent family members**

International application No.
**PCT/CN2022/074278**

| Patent document cited in search report | Publication date (day/month/year) | Patent family member(s) | Publication date (day/month/year) |
|---|---|---|---|
| WO 2021087025 A1 | 06 May 2021 | US 2021130342 A1 | 06 May 2021 |
| | | TW 202128653 A | 01 August 2021 |
| WO 2021087018 A1 | 06 May 2021 | None | |
| WO 2019212937 A1 | 07 November 2019 | EC SP20069404 A | 29 January 2021 |
| | | US 2019330194 A1 | 31 October 2019 |
| | | IL 278116 D0 | 30 November 2020 |
| | | MA 52486 A | 10 March 2021 |
| | | EP 3788040 A1 | 10 March 2021 |
| | | KR 20210014108 A | 08 February 2021 |
| | | BR 112020022006 A2 | 26 January 2021 |
| | | JP 2021523104 A | 02 September 2021 |
| | | SG 11202010183 X A | 27 November 2020 |
| | | CN 112424188 A | 26 February 2021 |
| | | PH 12020551760 A1 | 28 June 2021 |
| | | US 2021024502 A1 | 28 January 2021 |
| | | CR 20200518 A | 22 March 2021 |
| | | PE 20211382 A1 | 27 July 2021 |
| | | CO 2020013599 A2 | 21 December 2020 |
| | | US 2020123134 A1 | 23 April 2020 |
| | | EA 202092590 A1 | 08 April 2021 |
| | | JP 2022017221 A | 25 January 2022 |
| | | AU 2019262927 A1 | 12 November 2020 |
| | | TW 202014416 A | 16 April 2020 |
| | | CA 3098585 A1 | 07 November 2019 |
| | | CL 2020002821 A1 | 19 February 2021 |
| WO 2020223229 A1 | 05 November 2020 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2021101515791
- CN 2021107681969


### Non-patent literature cited in the description


- **S. VYAS ; M. CHESARONE- CATALDO ; T. TODOROVA ; Y H. HUANG ; P. CHANG.** A systematic analysis of the PARP protein family identifies new functions critical for cell physiology. *Nat. Common,* 2013, vol. 4, 2240
- **M. S. COHEN ; P. CHANG.** Insights into the biogenesis, function, and regulation of ADP-ribosylation. *Nat Chem Biol,* 2018, vol. 14, 236-243
- **A. OHMOTO ; S. YACHIDA.** Current status of poly(ADP-ribose)polymerase inhibitors and future directions. *Onco Targets Ther,* 2017, vol. 10, 5195-5208
- **S. FENG ; Z. CAO ; X. WANG.** Role of aryl hydrocarbon receptor in cancer. *Biochim Biophys Acta,* 2013, vol. 1836, 197-210
- **B. STOCKINGER ; P. DI MEGLIO ; M. GIALITAKIS ; J. H. DUARTE.** The aryl hydrocarbon receptor: multitasking in the immune system. *Annu Rev Immunol,* 2014, vol. 32, 403-432
- **C. A. OPITZ et al.** An endogenous tumor-promoting ligand of the human aryl hydrocarbon receptor. *Nature,* 2011, vol. 478, 197-203
- **L. MACPHERSON et al.** Aryl hydrocarbon receptor repressor and TIPARP (ARTD14) use similar, but also distinct mechanisms to repress aryl hydrocarbon receptor signaling. *Int J Mot Sci,* 2014, vol. 15, 7939-7957
- **MA, Q et al.** *Biochem,* 2001, vol. 289, 499-506
- **E. C. BOLTON et al.** Cell- and gene-specific regulation of primary target genes by the androgen receptor. *Genes Dev,* 2007, vol. 21, 2005-2017
- **J. SCHMAHL ; C. S. RAYMOND ; P. SORIANO.** PDGF signaling specificity is mediated through multiple immediate early genes. *Nat Genet,* 2007, vol. 39, 52-60
- **N. HAO et al.** Xenobiotics and loss of cell adhesion drive distinct transcriptional outcomes by aryl hydrocarbon receptor signaling. *Mot Pharmacol,* 2012, vol. 82, 1082-1093
- **L. MACPHERSON et al.** Aryl hydro- carbon receptor repressor and TIPARP (ARTD14) use similar, but also distinct mechanisms to repress aryl hydrocarbon receptor signaling. *Int J Mot Sci,* 2014, vol. 15, 7939-7957
- **L. MACPHERSON et al.** 2,3,7,8-Tetrachlorodibenzo-p-dioxin poly(ADP-ribose) polymerase (TIPARP, ARTD14) is a mono-ADP-ribosyltransferase and repressor of aryl hydro- carbon receptor transactivation. *Nucleic Acids Res,* 2013, vol. 41, 1604-1621
- **C. BINDESBOLL et al.** TCDD-inducible poly-ADP-ribose polymerase (TIPARP/PARP7) mono-ADP-ribosylates and co-activates liver X receptors. *Biochem J,* 2016, vol. 473, 899-910
- **T. KOZAKI et al.** Mitochondrial damage elicits a TCDD-inducible poly( ADP-ribose) polymerase-mediated antiviral response. *Proc Natl Acad Sci USA,* 2017, vol. 114, 2681-2686
- **T. YAMADA et al.** Constitutive aryl hydrocarbon receptor signaling constrains Type I interferon-mediated antiviral innate defense. *Nat Immunol,* 2016, vol. 17, 687-604
- **D. PAN et al.** A major chromatin regulator determines resistance of tumor cells to T cell-mediated killing. *Science,* 2018, vol. 359, 770-775
- **J. M. GOZGIT et al.** PARP7 negatively regulates the type I interferon response in cancer cells and its inhibition triggers antitumor immunity. *Cancer Cell,* 2021, vol. 39, 1214-1226
- *Inactive Ingredient Guide,* 1996
- Hand book of Pharmaceutical Additives. Synapse Information Resources, Inc, 2002
- Remington's Pharmaceutical Sciences. Mack Publishing Company
- CAS version. Handbook of Chemistry and Physics. 1994
- **THOMAS SORRELL.** Organic Chemistry. University Science Books, 1999
- **MICHAEL B. SMITH ; JERRY MARCH.** March's Advanced Organic Chemistry. John Wiley & Sons, 2007